# EUROPEAN PATENT APPLICATION

(11) **EP 3 851 457 A1**
(43) Date of publication of application: **21.07.2021**
(21) Application number: 20202658.9
(22) Date of filing: 20.01.2017
(51) Int. Cl.: C07K 16/28

(54) **MULTISPECIFIC MOLECULES TARGETING CLL-1**

(30) Priority: 21.01.2016 WO PCT/CN2016/071599
(62) Divisional of application: 17702163.1
(71) Applicant: Novartis AG, 4056 Basel (CH)
(72) Inventor: EBERSBACH, Hilmar, 4002 Basel (CH); DALEY, Michael, Cambridge, Massachusetts 02139 (US); JASCUR, Julia, 4002 Basel (CH); WU, Qilong, Shanghai, 201203 (CN); YANG, Qiumei, Shanghai, 201203 (CN); ZHANG, Jiquan, Shanghai, 201203 (CN)
(74) Representative: Didelon, Frédéric

(57) **Abstract**

The present invention is directed to multispecific/multivalent molecules comprising an anti-CLL-1 binding domain.

## Description

### Field of the Invention

The present invention relates to the field of immunology. Specifically, the invention relates to multispecific and/or multivalent molecules targeting C-type lectin-like-1 (CLL-1) and methods of making and using thereof.

### Related Applications

This application claims priority to PCT Application No. PCT/CN2016/071599, filed January 21, 2016. The entire content of this application is incorporated herein by reference.

### Background of the Invention

Multispecific molecules that are capable of binding two or more antigens are known in the art and offer significant clinical benefits, for example, for diagnostic enzyme assays, as vaccine adjuvants, for delivering thrombolytic agents, for treating diseases, for targeting immune complexes to cell surface receptors, or for delivering immunotoxins to tumor cells, etc. (Burrows, F. J. and Thorpe, P. E. (1993) Proc Natl Acad Sci USA 90:8996 9000; Zhu, Z. et al. (1996) Bio/Technology 14:192 196; Holliger, P. et al. (1996) Protein Engin. 9:299 305; Morrison et al., (1997) Nature Biotech. 15:159-163; Huang, X. et al. (1997) Science 275:547 550Alt et al. (1999) FEBS Letters 454: 90-94; Zuo et al., (2000) Protein Engineering 13:361-367; Lu et al., (2004) JBC 279:2856-2865; Lu et al., (2005) JBC 280:19665-19672; Marvin et al., (2005) Acta Pharmacologica Sinica 26:649-658; Marvin et al., (2006) Curr Opin Drug Disc Develop 9:184-193; Shen et al., (2007) J Immun Methods 218:65-74; Wu et al., (2007) Nat Biotechnol. 11:1290-1297; Dimasi et al., (2009) J Mol Biol. 393:672-692; and Michaelson et al., (2009) mAbs 1:128-141).

Most patients with acute myeloid leukemia (AML) are incurable using standard therapy (Mrozek et al, 2012, J Clin Oncol, 30:4515-23) and those with relapsed or refractory AML (RR-AML) have a particularly poor prognosis (Kern et al, 2003, Blood 2003, 101:64-70; Wheatley et al, 1999, Br J Haematol, 107:69-79). C-type lectin-like-1 (CLL-1) is also known as MICL, CLEC12A, CLEC-1, Dendritic Cell-Associated Lectin 1, and DCAL-2. CLL-1 is a glycoprotein receptor and member of the large family of C-type lectin-like receptors involved in immune regulation. CLL-1 is expressed in hematopoietic cells, primarily on innate immune cells including monocytes, DCs, pDCs, and granulocytes (Cancer Res. 2004; J Immunol 2009) and myeloid progenitor cells (Blood, 2007). CLL-1 is also found on acute myeloid leukemia (AML) blasts and leukemic stem cells (e.g., CD34+/CD38-) (Zhao et al., Haematologica. 2010, 95(1):71-78.). CLL-1 expression may also be relevant for other myeloid leukemias, such as acute myelomonocytic leukemia, acute monocytic leukemia, acute promyelocytic leukemia, chronic myeloid leukemia (CML), and myelodysplastic syndrome (MDS).

There is an unmet medical need for multispecific molecules which target CLL-1.

### Summary of the Invention

In one aspect, the disclosure provides a multispecific molecule including a first antigen binding domain and a second antigen binding domain, wherein the first antigen binding domain is an anti-CLL-1 binding domain including a heavy chain complementary determining region 1 (HC CDR1), a heavy chain complementary determining region 2 (HC CDR2), and a heavy chain complementary determining region 3 (HC CDR3) of any anti-CLL-1 binding domain amino acid sequence listed in Table 2.

In embodiments, the anti-CLL-1 binding domain further comprises a light chain complementary determining region 1 (LC CDR1), a light chain complementary determining region 2 (LC CDR2), and a light chain complementary determining region 3 (LC CDR3) of any anti-CLL-1 binding domain amino acid sequence listed in Table 2.

In embodiments, including the multispecific molecule of the preceding aspects or embodiments, the anti-CLL-1 binding domain includes LC CDR1, LC CDR2, and LC CDR3 that are the LC CDR sequences listed in Table 4, 6 or 8.

In embodiments, including in each of the preceding aspects or embodiments, the anti-CLL-1 binding domain includes HC CDR1, HC CDR2 and HC CDR3 that are the HC CDR sequences listed in Table 3, 5 or 7.

In embodiments, including in each of the preceding aspects or embodiments, the anti-CLL-1 binding domain of the multispecific molecule includes:
(i) the amino acid sequence of any light chain variable region listed in Table 2;
(ii) an amino acid sequence having at least one, two or three modifications but not more than 30, 20 or 10 modifications of the amino acid sequence of any of the light chain variable regions provided in Table 2; or
(iii) an amino acid sequence with 95-99% identity to the amino acid sequence of any of the light chain variable regions provided in Table 2.

In embodiments, including in each of the preceding aspects or embodiments, the anti-CLL-1 binding domain of the multispecific molecule includes:
(i) the amino acid sequence of any heavy chain variable region listed in Table 2;
(ii) an amino acid sequence having at least one, two or three modifications but not more than 30, 20 or 10 modifications of the amino acid sequence of any of the heavy chain variable regions provided in Table 2; or
(ii) an amino acid sequence with 95-99% identity to the amino acid sequence of any of the heavy chain variable regions provided in Table 2.

In embodiments, including in each of the preceding aspects or embodiments, the multispecific molecule includes an anti-CLL-1 binding domain that includes the amino acid sequence of any light chain variable region listed in Table 2, and the amino acid sequence of any heavy chain variable region listed in Table 2. In embodiments, the anti-CLL-1 binding domain that includes the amino acid sequence of the light chain variable region and the amino acid sequence of the heavy chain variable region of any anti-CLL-1 binding domain listed in Table 2, e.g., of any of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, or CLL-1-13.

In embodiments, including in each of the preceding aspects or embodiments, the anti-CLL-1 binding domain of the multispecific molecule includes:
(i) any amino acid sequence of any of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77;
(ii) an amino acid sequence having at least one, two or three modifications but not more than 30, 20 or 10 modifications to any of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77; or
(iii) an amino acid sequence with 95-99% identify to any of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77.

In aspects, including in any of the aforementioned aspects and embodiments, (for example in aspects that include a second antigen binding domain that binds CD3 (e.g., as described herein), for example where said anti-CD3 binding domain includes a VL sequence of SEQ ID NO: 1209 and a VH sequence of SEQ ID NO: 1236), the multispecific molecule includes an anti-CLL-1 binding domain that includes:
a) a VL sequence of SEQ ID NO: 88 and a VH sequence of SEQ ID NO: 75;
b) a VL sequence of SEQ ID NO: 89 and a VH sequence of SEQ ID NO: 76;
c) a VL sequence of SEQ ID NO: 87 and a VH sequence of SEQ ID NO: 74;
d) a VL sequence of SEQ ID NO: 85 and a VH sequence of SEQ ID NO: 72;
e) a VL sequence of SEQ ID NO: 86 and a VH sequence of SEQ ID NO: 73;
f) a VL sequence of SEQ ID NO: 83 and a VH sequence of SEQ ID NO: 70;
g) a VL sequence of SEQ ID NO: 90 and a VH sequence of SEQ ID NO: 77;
h) a VL sequence of SEQ ID NO: 79 and a VH sequence of SEQ ID NO: 66; or
i) a VL sequence of SEQ ID NO: 84 and a VH sequence of SEQ ID NO: 71.

In aspects, including in any of the aforementioned aspects and embodiments, (for example in aspects that include a second antigen binding domain that binds CD3 (e.g., as described herein), for example where said anti-CD3 binding domain includes SEQ ID NO: 506); the multispecific molecule includes an anti-CLL-1 binding domain that includes:
a) SEQ ID NO: 49;
b) SEQ ID NO: 50;
c) SEQ ID NO: 48;
d) SEQ ID NO: 46;
e) SEQ ID NO: 47;
f) SEQ ID NO: 44;
g) SEQ ID NO: 51;
h) SEQ ID NO: 40; or
i) SEQ ID NO: 45.

In aspects, including in any of the aforementioned aspects and embodiments, (for example in aspects that include a second antigen binding domain that binds CD3 (e.g., as described herein), for example where said anti-CD3 binding domain includes, e.g., consists of, SEQ ID NO: 507); the multispecific molecule includes a polypeptide comprising an anti-CLL-1 binding domain that includes, e.g., consists of:
a) SEQ ID NO: 512;
b) SEQ ID NO: 517;
c) SEQ ID NO: 522;
d) SEQ ID NO: 527;
e) SEQ ID NO: 532;
f) SEQ ID NO: 537;
g) SEQ ID NO: 542;
h) SEQ ID NO: 547; or
i) SEQ ID NO: 552.

In another aspect, the disclosure provides multispecific molecules, e.g., anti-CLL-1 multispecific molecules, including as described in each of the preceding aspects or embodiments, including a second antigen-binding domain that binds a cancer antigen other than CLL-1. In embodiments, the cancer antigen other than CLL-1 is expressed on a cell that also expresses CLL-1. In embodiments, the cancer antigen other than CLL-1 is expressed on an acute myeloid leukemia (AML), e.g., is an AML cell antigen. In embodiments, the cancer antigen is selected from the group consisting of CD123, CD33, CD34, FLT3, and folate receptor beta.

In another aspect, the disclosure provides multispecific molecules, e.g., anti-CLL-1 multispecific molecules, including as described in each of the preceding aspects or embodiments, including a second antigen-binding domain that binds an immune effector cell antigen. In embodiments, the immune effector cell antigen is an antigen expressed on a NK cell, e.g., is CD16 (Fc Receptor gamma III) or CD64 (Fc Receptor gamma I). In other embodiments, the immune effector cell antigen is an antigen expressed on a T cell, e.g., is an immune costimulatory molecule, e.g., is selected from the group consisting of an MHC class I molecule, a TNF receptor protein, an immunoglobulin-like protein, a cytokine receptor, an integrin, a signaling lymphocytic activation molecule (SLAM protein), an activating NK cell receptor, BTLA, Toll ligand receptor, OX40, CD2, CD7, CD27, CD28, CD30, CD40, CD47, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), TLR7, LTBR, LAT, GADS, SLP-76, PAG/Cbp, CD19a, and a ligand that specifically binds with CD83. In other embodiments, the immune effector cell antigen is an antigen expressed on a T cell, e.g., is an immune inhibitory molecule, e.g., is selected from the group consisting of PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta.

In other embodiments, the immune effector cell antigen is an antigen expressed on a T cell, e.g., is CD3. In embodiments, the anti-CD3 binding domain includes a heavy chain complementary determining region 1 (HC CDR1), a heavy chain complementary determining region 2 (HC CDR2), and a heavy chain complementary determining region 3 (HC CDR3) of any anti-CD3 binding domain VH amino acid sequence listed in Table 26. In embodiments, said anti-CD3 binding domain further includes a light chain complementary determining region 1 (LC CDR1), a light chain complementary determining region 2 (LC CDR2), and a light chain complementary determining region 3 (LC CDR3) of any anti-CD3 binding domain VL amino acid sequence listed in Table 26.

In embodiments, said LC CDR1, LC CDR2, and LC CDR3 are the LC CDR sequences of any anti-CD3 binding domain listed in Table 27, 28 or 29. In embodiments, said HC CDR1, HC CDR2 and HC CDR3 are the HC CDR sequences of any anti-CD3 binding domain listed in Table 27, 28 or 29.

In embodiments, the anti-CD3 binding domain includes:
(i) the amino acid sequence of any light chain variable region listed in Table 26;
(ii) an amino acid sequence having at least one, two or three modifications but not more than 30, 20 or 10 modifications of the amino acid sequence of any of the light chain variable regions provided in Table 26; or
(iii) an amino acid sequence with 95-99% identity to the amino acid sequence of any of the light chain variable regions provided in Table 26.

In embodiments, the anti-CD3 binding domain includes:
(i) the amino acid sequence of any heavy chain variable region listed in table 26;
(ii) an amino acid sequence having at least one, two or three modifications but not more than 30, 20 or 10 modifications of the amino acid sequence of any of the heavy chain variable regions provided in Table 26; or
(iii) an amino acid sequence with 95-99% identity to the amino acid sequence of any of the heavy chain variable regions provided in Table 26.

In embodiments, the anti-CD3 binding domain includes the amino acid sequence of any light chain variable region listed in Table 26, and the amino acid sequence of any heavy chain variable region listed in Table 26. In embodiments, the anti-CD3 binding domain includes the amino acid sequence of any light chain variable region and the amino acid sequence of any heavy chain variable region of any anti-CD3 binding domain listed in Table 26. In embodiments, the anti-CD3 binding domain includes a heavy chain variable region amino acid sequence selected from the group consisting of SEQ ID NO: 1200, 1202, 1204, 1206, 1208, 1210, 1212, 1214, 1216, 1218, 1220, 1222, 1224, 1226, 1228, 1230, 1232, 1234, and 1236; and a light chain variable amino acid sequence selected from the group consisting of SEQ ID NO: 1201, 1203, 1205, 1207, 1209, 1211, 1213, 1215, 1217, 1219, 1221, 1223, 1225, 1227, 1229, 1231, 1233, 1235, and 1237.

In one aspect, including in any of the preceding aspects or embodiments, the multispecific molecule is multivalent, e.g., bivalent, with respect to the first antigen binding domain or second antigen binding domain. In embodiments, the multispecific molecule is bivalent with respect to the anti-CLL-1 binding domain. In embodiments, the two or more anti-CLL-1 binding domains bind to the same epitope of CLL-1, e.g., include the same anti-CLL-1 binding domain (e.g., the same CDRs, the same VH and VL or the same scFv sequences). In embodiments, the two or more anti-CLL-1 binding domains bind to different epitopes of CLL-1.

The disclosure provides for the multispecific molecules in a variety of formats. In one aspect, the disclosure provides for the multispecific molecule of any of the preceding embodiments as a bispecific antibody. In one aspect, the disclosure provides for the multispecific molecule of any of the preceding embodiments as a dualbody. In one aspect, the disclosure provides for the multispecific molecule of any of the preceding embodiments as a scFv-Fc. In one aspect, the disclosure provides for the multispecific molecule of any of the preceding embodiments as a mixed chain multispecific molecule. In one aspect, the disclosure provides for the multispecific molecule of any of the preceding embodiments as a tandem scFv. Each of these formats is described in more detail below.

The disclosure further provides for bispecific molecules of any of the preceding embodiments.

In embodiments, including in any of the preceding aspects and embodiments that include one or more heavy chain constant domains, the heavy chain constant domain may be selected from SEQ ID NO: 500, SEQ ID NO: 501, SEQ ID NO: 504 and SEQ ID NO: 505. In a preferred embodiment, the antigen binding domains of the multispecific molecule are scFvs, and the first heavy chain constant domain includes, e.g., is, SEQ ID NO: 500, and the second heavy chain constant domain includes, e.g., is, SEQ ID NO: 501. In another preferred embodiment, the antigen binding domains are Fabs, and the first heavy chain constant domain comprises, e.g., is, SEQ ID NO: 504, and the first light chain constant domain comprises, e.g., is, SEQ ID NO: 502; and the second heavy chain constant domain comprises, e.g., is, SEQ ID NO: 505, and the first light chain constant domain comprises, e.g., is, SEQ ID NO: 503.

The disclosure further provides multispecific molecules, including of any of the preceding embodiments, further including CH3, and optionally, CH2 domains. In one aspect, including in any of the preceding embodiments and aspects, the polypeptide(s) of the multispecific molecules that include the HC CDR sequences of the first and/or second antigen binding domains further include a CH3 domain, and optionally a CH2 domain. The disclosure further provides multispecific molecules, including of any of the preceding embodiments, wherein at least one, e.g., both, of said CH3 domains include one or more modifications to enhance heterodimerization, e.g., as described herein.

In one aspect, the multispecific molecule that includes one or more modifications to enhance heterodimerization includes introduction of a knob in a first CH3 domain and a hole in a second CH3 domain, or vice versa, such that heterodimerization of the polypeptide that includes the first CH3 domain and the polypeptide that includes the second CH3 domain is favored relative to polypeptides that include only unmodified CH3 domains.

In embodiments, the knob or hole is introduced to the first CH3 domain at residue 366, 405 or 407 according to the EU numbering scheme of Kabat et al. (pp. 688-696 in Sequences of proteins of immunological interest, 5th ed., Vol. 1 (1991; NIH, Bethesda, Md.)) to create either a knob or hole, and a complimentary hole or knob is introduced to the second CH3 domain at residue 407 if residue 366 is mutated in the first CH3 domain, residue 394 if residue 405 is mutated in the first CH3 domain, or residue 366 if residue 407 is mutated in the first CH3 domain, according to the EU numbering scheme of Kabat et al. (pp. 688-696 in Sequences of proteins of immunological interest, 5th ed., Vol. 1 (1991; NIH, Bethesda, Md.)).

In embodiments, the first CH3 domain includes introduction of a knob at position 366, and the second CH3 domain includes introduction of a hole at position 366, position 368 and position 407, according to the EU numbering scheme of Kabat et al. (pp. 688-696 in Sequences of proteins of immunological interest, 5th ed., Vol. 1 (1991; NIH, Bethesda, Md.)), or vice versa.

In embodiments, the first CH3 domain includes a tyrosine or tryptophan at position 366, and the second CH3 domain includes a serine at position 366, alanine at position 368 and valine at position 407, according to the EU numbering scheme of Kabat et al. (pp. 688-696 in Sequences of proteins of immunological interest, 5th ed., Vol. 1 (1991; NIH, Bethesda, Md.)), or vice versa.

In another aspect, the one or more modifications to enhance heterodimerization include. IgG heterodimerization modifications. In embodiments, the first CH3 domain includes the mutation K409R and the second CH3 domain includes the mutation F405L, or vice versa.

In another aspect, the one or more modifications to enhance heterodimerization include polar bridge modifications. In embodiments, said one or more modifications include modifications to the first CH3 domain that are selected from a group consisting of: S364L, T366V, L368Q, D399K, F405S, K409F, T411K, and combinations thereof. In embodiments, including those that include first CH3 domain mutations, the second CH3 domain may include one or more modifications that are selected from the group consisting of Y407F, K409Q and T411D, and combinations thereof.

In another aspect, the multispecific molecule of any of the preceding aspects or embodiments may further include a first CH3 domain including a cysteine capable for forming a disulfide bond with a cysteine of the second CH3 domain. In embodiments, the first CH3 domain includes a cysteine at position 354 and the second CH3 domain includes a cysteine at position 349, or vice versa.

In another aspect, the multispecific molecule of any of the preceding aspects or embodiments may further (or alternatively) include a constant domain that comprises one or more mutations to reduce, e.g., silence, antibody-dependent cell-mediated cytotoxicity (ADCC) and/or compliment-dependent cytotoxicity (CDC). In an embodiment, the one or more mutations to reduce, e.g., silence ADCC and/or CDC include, e.g., are, the DAPA mutations (e.g., D265A and P329A, according to EU numbering). In an embodiment, the one or more mutations to reduce, e.g., silence ADCC and/or CDC include, e.g., are, the LALA mutations (e.g., L234A and L235A, according to EU numbering). In an embodiment, the one or more mutations to reduce, e.g., silence ADCC and/or CDC include, e.g., is, N279A, according to EU numbering. Constant domains comprising combinations of any of the above may also be included.

In another aspect, the disclosure provides isolated nucleic acid, e.g., one or more polynucleotides, encoding the multispecific molecule of any of the preceding aspects or embodiments. In embodiments, the isolated nucleic acid is disposed on a single continuous polynucleotide. In other embodiments, the isolated polynucleotide is disposed on two or more continuous polynucleotides.

In embodiments, the nucleic acid includes sequence encoding an anti-CD3 binding domain. In embodiments, including in any of the aforementioned nucleic acid aspects and embodiments, the nucleic acid includes SEQ ID NO: 508 and SEQ ID NO: 509.

In embodiments, including in any of the aforementioned nucleic acid aspects and embodiments, the isolated nucleic acid includes SEQ ID NO: 510.

In embodiments, including in any of the aforementioned nucleic acid aspects and embodiments, the isolated nucleic acid includes SEQ ID NO: 511.

In embodiments, the nucleic acid includes sequence encoding an anti-CLL-1 binding domain. In embodiments, including in any of the aforementioned nucleic acid aspects and embodiments, the isolated nucleic acid includes:
a) SEQ ID NO: 513 and SEQ ID NO: 514;
b) SEQ ID NO: 518 and SEQ ID NO: 519;
c) SEQ ID NO: 523 and SEQ ID NO: 524;
d) SEQ ID NO: 528 and SEQ ID NO: 529;
e) SEQ ID NO: 533 and SEQ ID NO: 534;
f) SEQ ID NO: 538 and SEQ ID NO: 539;
g) SEQ ID NO: 543 and SEQ ID NO: 544;
h) SEQ ID NO: 548 and SEQ ID NO: 549; or
i) SEQ ID NO: 553 and SEQ ID NO: 554.

In embodiments, including in any of the aforementioned nucleic acid aspects and embodiments, the isolated nucleic acid includes:
a) SEQ ID NO: 515;
b) SEQ ID NO: 520;
c) SEQ ID NO: 525;
d) SEQ ID NO: 530;
e) SEQ ID NO: 535;
f) SEQ ID NO: 540;
g) SEQ ID NO: 545;
h) SEQ ID NO: 550; or
i) SEQ ID NO: 555.

In embodiments, including in any of the aforementioned nucleic acid aspects and embodiments, the isolated nucleic acid includes:
a) SEQ ID NO: 516;
b) SEQ ID NO: 521;
c) SEQ ID NO: 526;
d) SEQ ID NO: 531;
e) SEQ ID NO: 536;
f) SEQ ID NO: 541;
g) SEQ ID NO: 546;
h) SEQ ID NO: 551; or
i) SEQ ID NO: 556.

In embodiments, the isolated nucleic acid includes sequence encoding an anti-CD3 binding domain, for example, as described herein, and sequence encoding an anti-CLL-1 binding domain, for example, as described herein. In embodiments, the sequence encoding the anti-CD3 binding domain and the sequence encoding the anti-CLL-1 binding domain are disposed on separate polynucleotides. In embodiments, the sequence encoding the anti-CD3 binding domain and the sequence encoding the anti-CLL-1 binding domain are disposed on a single polynucleotide.

In another aspect, the disclosure provides a vector e.g., one or more vectors, that includes the isolated nucleic acid described above.

In another aspect, the disclosure provides a cell including the isolated nucleic acid or vector described above.

In another aspect, the disclosure provides for methods of treatment. In one aspect, the disclosure provides a method of treating a mammal having a disease associated with expression of CLL-1 including administering to the mammal an effective amount of a multispecific molecule of any of the preceding aspects or embodiments, the isolated nucleic acid described herein, the vector described herein or the cell described herein.

In embodiments, the disease associated with CLL-1 expression is:
(i) a cancer or malignancy, or a precancerous condition chosen from one or more of a myelodysplasia, a myelodysplastic syndrome or a preleukemia, or
(ii) a non-cancer related indication associated with expression of CLL-1.

In embodiments, the disease is a hematologic cancer. In embodiments the disease is acute myeloid leukemia (AML), acute lymphoblastic B-cell leukemia (B-cell acute lymphoid leukemia, BALL), acute lymphoblastic T-cell leukemia (T-cell acute lymphoid leukemia (TALL), B-cell prolymphocytic leukemia, chronic lymphocytic leukemia, chronic myeloid leukemia (CML), myelodysplastic syndrome, plasma cell myeloma, or a combination thereof. In embodiments, the disease is acute myeloid leukemia (AML).

In another aspect, the disclosure provides for the use of the multispecific molecule of any of the preceding aspects or embodiments, the isolated nucleic acid described above, the vector described above or the cell described above, in the manufacture of a medicament.

In another aspect, the disclosure provides the multispecific molecule of any of the preceding aspects or embodiments, for use as a medicament.

In another aspect, the disclosure provides the multispecific molecule of any of the preceding aspects or embodiments, for use in a therapy.

In another aspect, the disclosure provides the multispecific molecule of any of the preceding aspects or embodiments, for use in treating a subject having a hematologic cancer.

In another aspect, the disclosure provides the multispecific molecule of any of the preceding aspects or embodiments, for use in treating a subject having acute myeloid leukemia (AML).

In another aspect, the disclosure provides for compositions including the multispecific molecule of any of the preceding aspects or embodiments. In embodiments, the composition is a pharmaceutical composition. In embodiments, the compositions may further include a pharmaceutically acceptable diluent or carrier. In embodiments, the compositions include a therapeutically effective amount of the multispecific molecule of any of the preceding aspects or embodiments.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In addition, the materials, methods, and examples are illustrative only and not intended to be limiting. Headings, subheadings or numbered or lettered elements, e.g., (a), (b), (i) etc, are presented merely for ease of reading. The use of headings or numbered or lettered elements in this document does not require the steps or elements be performed in alphabetical order or that the steps or elements are necessarily discrete from one another. Other features, objects, and advantages of the invention will be apparent from the description and drawings, and from the claims.

### Brief Description of the Drawings

FIG. 1 shows various exemplary formats for multispecific, e.g., bispecific, molecules that incorporate an anti-CLL-1 binding domain, e.g., a human or humanized anti-CLL-1 binding domain.
FIG 2. Shows *in vitro* T cell killing of CLL1 -expressing cancer cell line HL60 with CD3 x CLL1 bispecific antibodies.
FIG 3. Shows CD3 x CLL1 bispecific antibody-mediated T cell activation in vitro via engagement with CLL1-expressing cancer cell line U937, as demonstrated through an NFAT luciferase reporter gene in the Jurkat human T cell line.

### Detailed Description of the Invention

### Definitions

In order that the present invention may be more readily understood, certain terms are first defined. Additional definitions are set forth throughout the detailed description. Unless defined otherwise, all technical and scientific terms used herein have the same meaning as commonly understood by those of ordinary skill in the art to which this invention pertains.

The term "antibody" as used herein refers to a polypeptide (or set of polyptptides) of the immunoglobulin family that is capable of binding an antigen non-covalently, reversibly and specifically. For example, a naturally occurring "antibody" of the IgG type is a tetramer comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CHI, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen, which is sometimes referred to herein as the antigen binding domain. The constant regions of the antibodies may mediate the binding of the immunoglobulin to host tissues or factors, including various cells of the immune system (e.g., effector cells) and the first component (Clq) of the classical complement system. The term "antibody" includes, but is not limited to, monoclonal antibodies, human antibodies, humanized antibodies, camelised antibodies, chimeric antibodies, bispecific or multispecific antibodies and anti-idiotypic (anti-Id) antibodies (including, e.g., anti-Id antibodies to antibodies of the invention). The antibodies can be of any isotype/class (e.g., IgG, IgE, IgM, IgD, IgA and IgY) or subclass (e.g., IgG1, IgG2, IgG3, IgG4, IgA1 and IgA2).

Both the light and heavy chains are divided into regions of structural and functional homology. The terms "constant" and "variable" are used functionally. In this regard, it will be appreciated that the variable domains of both the light (VL) and heavy (VH) chain portions determine antigen recognition and specificity. Conversely, the constant domains of the light chain (CL) and the heavy chain (CH1, CH2 or CH3) confer important biological properties such as secretion, transplacental mobility, Fc receptor binding, complement binding, and the like. By convention the numbering of the constant region domains increases as they become more distal from the antigen binding site or amino-terminus of the antibody. The N-terminus is a variable region and at the C-terminus is a constant region; the CH3 and CL domains actually comprise the carboxy-terminus of the heavy and light chain, respectively.

The term "antibody fragment" as used herein refers to one or more portions of an antibody. In some embodiments, these portions are part of the contact domain(s) of an antibody. In some other embodiments, these portion(s) are antigen-binding fragments that retain the ability of binding an antigen non-covalently, reversibly and specifically, sometimes referred to herein as the antigen binding domain. Examples of binding fragments include, but are not limited to, single-chain Fvs (scFv), a Fab fragment, a monovalent fragment consisting of the VL, VH, CL and CH1 domains; a F(ab)₂ fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (Ward et al., (1989) Nature 341:544-546), which consists of a VH domain; and an isolated complementarity determining region (CDR).

Antibody fragments can also be incorporated into single domain antibodies, maxibodies, minibodies, intrabodies, diabodies, triabodies, tetrabodies, v-NAR and bis-scFv (see, *e.g*., Hollinger and Hudson, (2005) Nature Biotechnology 23: 1126-1136). Antibody fragments can be grafted into scaffolds based on polypeptides such as Fibronectin type III (Fn3) (see U.S. Pat. No. 6,703,199, which describes fibronectin polypeptide monobodies).

Antibody fragments can be incorporated into single chain molecules comprising a pair of tandem Fv segments (for example, VH-CH1-VH-CH1) which, together with complementary light chain polypeptides (for example, VL-VC-VL-VC), form a pair of antigen binding regions (Zapata et al., (1995) Protein Eng. 8:1057-1062; and U.S. Pat. No. 5,641,870).

The term "half antibody" refers to a portion of an antibody molecule, antibody fragment, antibody-like molecule or multispecific molecule that comprises a single antigen binding domain. In an embodiment, a half antibody refers to a heavy and light chain pair of, for example, an IgG antibody. In one embodiment, a half antibody refers to a polypeptide comprising a VL domain and a CL domain, and a second polypeptide comprising a VH domain, a CH1 domain, a hinge domain, a CH2 domain, and a CH3 domain, wherein said VL and VH domains comprise an antigen binding domain. In another embodiment, a half antibody refers to a polypeptide comprising a scFv domain, a CH2 domain and a CH3 domain. In some multispecific molecule embodiments, a first half antibody will associate, e.g., heterodimerize, with a second half antibody. In some multispecific molecules a first half antibody will be covalently linked to a second half antibody. In some multispecific molecules, either a first half antibody, a second half antibody, or both a first and second half antibody may comprise an additional antigen binding domain.

The term "antibody-like molecule" refers to a molecule comprising an antibody or a fragment thereof.

The terms "complementarity determining region" or "CDR," as used herein, refer to the sequences of amino acids within antibody variable regions which confer antigen specificity and binding affinity. For example, in general, there are three CDRs in each heavy chain variable region (e.g., HCDR1, HCDR2, and HCDR3) and three CDRs in each light chain variable region (LCDR1, LCDR2, and LCDR3). The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof. Under the Kabat numbering scheme, in some embodiments, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under the Chothia numbering scheme, in some embodiments, the CDR amino acids in the VH are numbered 26-32 (HCDR1), 52-56 (HCDR2), and 95-102 (HCDR3); and the CDR amino acid residues in the VL are numbered 26-32 (LCDR1), 50-52 (LCDR2), and 91-96 (LCDR3). In a combined Kabat and Chothia numbering scheme, in some embodiments, the CDRs correspond to the amino acid residues that are part of a Kabat CDR, a Chothia CDR, or both. For instance, in some embodiments, the CDRs correspond to amino acid residues 26-35 (HCDR1), 50-65 (HCDR2), and 95-102 (HCDR3) in a human VH, e.g., a mammalian VH, e.g., a human VH; and amino acid residues 24-34 (LCDR1), 50-56 (LCDR2), and 89-97 (LCDR3) in humana VL, e.g., a mammalian VL, e.g., a human VL.

The term "single-chain Fv" or "scFv" as used herein refers to antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the scFv to form the desired structure for antigen binding. For a review of scFv see Plückthun in The Pharmacology of Monoclonal Antibodies, vol. 113, Rosenburg and Moore eds., (1994) Springer-Verlag, New York, pp. 269-315.

The term "diabody" as used herein refers to small antibody fragments with two antigen-binding sites, which fragments comprise a heavy chain variable domain (VH) connected to a light chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., (1993) Proc. Natl. Acad. Sci. USA 90:6444-6448.

The term "monospecific molecule," as used herein, refers to a molecule that binds to one epitope on a target antigen. In some embodiments, a mono-specific molecule of the present invention is a monospecific antibody-like molecule. In some embodiments, a mono-specific molecule of the present invention is a monospecific antibody.

As used herein, the terms "CLL-1" and "CLL1" are used interchangeably, and refer to C-type lectin-like molecule-1, which is an antigenic determinant detectable on leukemia precursor cells and on normal immune cells. C-type lectin-like-1 (CLL-1) is also known as MICL, CLEC12A, CLEC-1, Dendritic Cell-Associated Lectin 1, and DCAL-2. The human and murine amino acid and nucleic acid sequences can be found in a public database, such as GenBank, UniProt and Swiss-Prot. For example, the amino acid sequence of human CLL-1 can be found as UniProt/Swiss-Prot Accession No. Q5QGZ9 and the nucleotide sequence encoding of the human CLL-1 can be found at Accession Nos. NM 001207010.1, NM 138337.5, NM 201623.3, and NM 201625.1. In one embodiment, the multispecific molecule, e.g., bispecific molecule, e.g., bispecific antibody or antibody-like molecule comprises at least one, e.g., one, anti-CLL-1 binding domain and binds an epitope within the extracellular domain of the CLL-1 protein or a fragment thereof. In one embodiment, the CLL-1 protein is expressed on a cancer cell.

The terms "CD3" or "CD-3," as used interchangeably herein, refer to the cluster of differentiation 3 co-receptor (or co-receptor complex, or polypeptide chain of the co-receptor complex) of the T cell receptor. The amino acid sequence of the polypeptide chains of human CD3 are provided in NCBI Accession P04234, P07766 and P09693. CD3 proteins may also include variants. CD3 proteins may also include fragments. CD3 proteins also include post-translational modifications of the CD3 amino acid sequences. Post-translational modifications include, but are not limited to, N-and O-linked glycosylation.

The term "bispecific molecule" as used herein refers to a molecule that binds to two different epitopes on one antigen (also referred to herein as "biparatopic") or two different antigens. In some embodiments, a bispecific molecule of the present invention is a bispecific antibody-like molecule. In some embodiments, a bispecific molecule of the present invention is a bispecific antibody. In some embodiments, the bispecific molecule of the present inveniton is not biparatropic.

The term "multispecific molecule" as used herein refers to a molecule that binds to two or more different epitopes on one antigen (also referred to herein as "multiparatopic") or on two or more different antigens. Recognition of each antigen is generally accomplished with an "antigen binding domain". In some embodiments, a multispecific molecule of the present invention is a multispecific antibody-like molecule. In some embodiments, a multispecific molecule of the present invention is a multispecific antibody. In some embodiments, the bispecific molecule of the present inveniton is not multiparatropic. The term "multispecific" includes "bispecific." In some aspects, the multispecific molecules consist of one polypeptide chain that comprises a plurality, e.g., two or more, e.g., two, antigen binding domains. In some aspects the multispecific molecules comprise two, three, four or more polypeptide chains that together comprise a plurality, e.g., two or more, e.g., two, antigen binding domains.

The term "a tandem of VH domains (or VHs)" as used herein refers to a string of VH domains, consisting of multiple numbers of identical VH domains of an antibody. Each of the VH domains, except the last one at the end of the tandem, has its C-terminus connected to the N-terminus of another VH domain with or without a linker. A tandem has at least 2, 3, 4, 5, 6, 7, 8, 9, 10, 11, 12, 13, 14, 15, 16, 17, 18, 19, 20, 25, 30, 50, or 100 VH domains. The tandem of VH can be produced by joining the encoding genes of each VH domain in a desired order using recombinant methods with or without a linker (e.g., a synthetic linker) that enables them to be made as a single protein. In one aspect, the VH domains in the tandem, alone or in combination with VL domains of the same antibody , retain the binding specificity of the original antibody. The N-terminus of the first VH domain in the tandem is defined as the N-terminus of the tandem, while the C-terminus of the last VH domain in the tandem is defined as the C-terminus of the tandem.

The term "a tandem of VL domains (or VLs)" as used herein refers to a string of VL domains, consisting of multiple numbers of identical VL domains of an antibody. Each of the VL domains, except the last one at the end of the tandem, has its C-terminus connected to the N-terminus of another VH with or without a linker. A tandem has at least 2, 3, 4, 5, 6, 7, 8, 9, 10, 11, 12, 13, 14, 15, 16, 17, 18, 19, 20, 25, 30, 50, or 100 VL domains. The tandem of VL can be produced by joining the encoding gene of each VL domain in a desired order using recombinant methods with or without a linker (e.g., a synthetic linker) that enables them to be made as a single protein. Preferably, the VL domains in the tandem, alone or in combination with VH domains of the same antibody, retain the binding specificity of the original antibodies. The N-terminus of the first VL domain in the tandem is defined as the N-terminus of the tandem, while the C-terminus of the last VL domain in the tandem is defined as the C-terminus of the tandem.

The term "monovalent molecule" as used herein refers to a molecule that has a single antigen binding domain. In some embodiments, a monovalent molecule of the present invention is a monovalent antibody-like molecule. In some embodiments, a monovalent molecule of the present invention is a monovalent antibody.

The term "bivalent molecule" as used herein refers to a molecule that has two antigen binding domains. In some embodiments, a bivalent molecule of the present invention is a bivalent antibody-like molecule. In some embodiments, a bivalent molecule of the present invention is a bivalent antibody.

The term "trivalent molecule" as used herein refers to a molecule that has three antigen binding domains. In some embodiments, a trivalent molecule of the present invention is a trivalent antibody-like molecule. In some embodiments, a trivalent molecule of the present invention is a trivalent antibody. In some embodiments, a trivalent molecule may consist of two antigen binding domains capable of binding to the same epitope of the same antigen, and a third antigen binding domain that binds to a distinct epitope or antigen, e.g., a distinct antigen. Such embodiments are considered trivalent bispecific molecules.

The term "tetravalent molecule" as used herein refers to a molecule that has four antigen binding domains. In some embodiments, a tetravalent molecule of the present invention is a tetravalent antibody-like molecule. In some embodiments, a tetravalent molecule of the present invention is a tetravalent antibody. In some embodiments, a tetravalent molecule may consist of two antigen binding domains capable of binding to the same epitope of the same antigen, and two additional antigen binding domains that bind to a distinct epitope or antigen, e.g., a distinct antigen. When such two additional antigen binding domains bind to the same epitope or antigen, e.g., the same distinct antigen, such embodiments are considered tetravalent bispecific molecules. When such two additional antigen binding domains bind to different epitopes or antigens, e.g., different distinct antigens, such embodiments are considered tetravalent trispecific molecules.

The term "multivalent molecule" as used herein refers to a molecule that has at least two antigen binding sites. In some embodiments, a multivalent molecule of the present invention is a multivalent antibody-like molecule. In some embodiments, a multivalent molecule of the present invention is a multivalent antibody. In some embodiments, a multivalent molecule is a bivalent molecule, trivalent molecule or a tetravalent molecule.

The term "substantially similar," or "substantially the same," as used herein refers to a sufficiently high degree of similarity between two numeric values (generally one associated with an antibody-like molecule of the invention and the other associated with a reference/comparator antibody or antibody-like molecule) such that one of skill in the art would consider the difference between the two values to be of little or no biological and/or statistical significance within the context of the biological characteristic measured by said values (e.g., Tm values or the amount of the assembled antibodies). The difference between said two values is preferably less than about 50%, preferably less than about 40%, preferably less than about 30%, preferably less than about 20%, preferably less than about 10% as a function of the value for the reference/comparator antibody.

The term "substantially same yield" as used herein refers to the amount of an assembled molecule (e.g. antibody or antibody-like molecule) of the present invention is substantially the same as that of the antibodies it derived from when being prepared under similar conditions in similar cell types. Relative yields of antibody or antibody like products can be determined using standard methods including scanning densitometry of SDS-PAGE gels and/or immunoblots and the AME5-RP assay.

The term "thermostability" as used herein refers to the ability of a protein (e.g., an antibody or an antibody-like molecule) to retain the characteristic property when heated moderately. When exposed to heat, proteins will experience denaturing/unfolding process and will expose hydrophobic residues. A protein is completely unfolded in response to heat at a characteristic temperature. The temperature at the mid-point of the protein unfolding process is defined as Tm, which is an important physical characteristic of a protein, and can be measured with the techniques known in the art, such as by monitoring the denaturing process using Sypro orange dye labeling hydrophobic residues of denatured proteins or by using differential scanning calorimetry (DSC) techniques.

The term "epitope" as used herein refers to any determinant capable of binding with high affinity to an antibody or an antibody-like molecule. An epitope is a region of an antigen that is bound by an antibody (or an antibody-like molecule) that specifically targets that antigen, and when the antigen is a protein, includes specific amino acids that directly contact the antibody or the antibody-like molecule. Most often, epitopes reside on proteins, but in some instances, may reside on other kinds of molecules, such as nucleic acids. Epitope determinants may include chemically active surface groupings of molecules such as amino acids, sugar side chains, phosphoryl or sulfonyl groups, and may have specific three dimensional structural characteristics, and/or specific charge characteristics.

Generally, antibodies or antibody-like molecules specific for a particular target antigen will preferentially recognize an epitope on the target antigen in a complex mixture of proteins and/or macromolecules.

Regions of a given polypeptide that include an epitope can be identified using any number of epitope mapping techniques, well known in the art. See, e.g., Epitope Mapping Protocols in Methods in Molecular Biology, Vol. 66 (Glenn E.Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al., (1984) Proc. Natl. Acad. Sci. USA 8:3998-4002; Geysen et al., (1985) Proc. Natl. Acad. Sci. USA 82:78-182; Geysen et al., (1986) Mol. Immunol. 23:709-715. Similarly, conformational epitopes are readily identified by determining spatial conformation of amino acids such as by, e.g., x-ray crystallography and two-dimensional nuclear magnetic resonance. See, e.g., Epitope Mapping Protocols, supra. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al., (1981) Proc. Natl. Acad. Sci USA 78:3824-3828; for determining antigenicity profiles, and the Kyte-Doolittle technique, Kyte et al., (1982) J.MoI. Biol. 157:105-132; for hydropathy plots.

Specific binding between two entities means a binding with an equilibrium constant (K_{A}) (kₒₙ/k_{off}) of at least 10²M⁻¹, at least 5x10²M⁻¹, at least 10³M⁻¹, at least 5x10³M⁻¹, at least 10⁴M⁻¹ at least 5x10⁴M⁻¹, at least 10⁵M⁻¹, at least 5x10⁵M⁻¹, at least 10⁶M⁻¹, at least 5x10⁶M⁻¹, at least 10⁷M⁻¹, at least 5x10⁷M⁻¹, at least 10⁸M⁻¹, at least 5x10⁸M⁻¹, at least 10⁹M⁻¹, at least 5x10⁹M⁻¹, at least 10¹⁰M⁻¹, at least 5x10¹⁰M⁻¹, at least 10¹¹M⁻¹ , at least 5x10¹¹M⁻¹, at least 10¹²M⁻¹, at least 5x10¹²M⁻¹, at least 10¹³M⁻¹, at least 5x10¹³ M⁻¹, at least 10¹⁴M⁻¹, at least 5x10¹⁴M⁻¹, at least 10¹⁵M⁻¹, or at least 5x10¹⁵M⁻¹.

The term "specifically (or selectively) binds" to an antigen or an epitope refers to a binding reaction that is determinative of the presence of a cognate antigen or an epitope in a heterogeneous population of proteins and other biologics. In addition to the equilibrium constant (K_{A}) noted above, an antibody or antibody-like molecule of the invention typically also has a dissociation rate constant (K_{D}) (k_{off}/kₒₙ) of less than 5x10⁻²M, less than 10⁻²M, less than 5x10⁻³M, less than 10⁻³M, less than 5x10⁻⁴M, less than 10⁻⁴M, less than 5x10⁻⁵M, less than 10⁻⁵M, less than 5x10⁻⁶M, less than 10⁻⁶M, less than 5x10⁻⁷M, less than 10⁻⁷M, less than 5x10⁻⁸M, less than 10⁻⁸M, less than 5x10⁻⁹M, less than 10⁻⁹M, less than 5x10⁻¹⁰M, less than 10⁻¹⁰M, less than 5x10⁻¹¹M, less than 10⁻¹¹M, less than 5x10⁻¹²M, less than 10⁻¹²M, less than 5x10⁻¹³M, less than 10⁻¹³M, less than 5x10⁻¹⁴M, less than 10⁻¹⁴M, less than 5x10⁻¹⁵M, or less than 10⁻15M or lower, and binds to the target antigen with an affinity that is at least two-fold greater than its affinity for binding to a non-specific antigen (*e.g.*, HSA).

The terms "a molecule (e.g. an antibody or an antibody-like molecule) recognizing an antigen (or an epitope)" and "a molecule specific for an antigen (or an epitope)" are used interchangeably herein with the term "a molecule which binds specifically to an antigen (or an epitope)". In one embodiment, the molecule (e.g. antibody or antibody-like molecule) or fragment thereof has dissociation constant (K_{d}) of less than 3000 pM, less than 2500 pM, less than 2000 pM, less than 1500 pM, less than 1000 pM, less than 750 pM, less than 500 pM, less than 250 pM, less than 200 pM, less than 150 pM, less than 100 pM, less than 75 pM, less than 10 pM, less than 1 pM as assessed using a method described herein or known to one of skill in the art (e.g., a BIAcore assay, ELISA, FACS, SET) (Biacore International AB, Uppsala, Sweden). The term "K_{assoc}" or "Kₐ", as used herein, refers to the association rate of a particular antibody-antigen interaction, whereas the term "K_{dis}" or "K_{d}," as used herein, refers to the dissociation rate of a particular antibody-antigen interaction. The term "K_{D}", as used herein, refers to the dissociation constant, which is obtained from the ratio of Kd to Ka (i.e. K_{d}/Kₐ) and is expressed as a molar concentration (M). K_{D} values for antibodies can be determined using methods well established in the art. A method for determining the K_{D} of an antibody is by using surface plasmon resonance, or using a biosensor system such as a Biacore® system.

The term "multiple binding specificities" as used herein refers to that a molecule of the present invention (e.g., an antibody or an antibody like molecule) is capable of specifically binding at least two, three, four, five, six, seven, eight, nine, or ten different epitopes either on the same antigen or on at least two, three, four, five, seven, eight, nine or ten different antigens.

The term "dual binding specificity" as used herein refers to that a molecule of the present invention (e.g., an antibody or an antibody like molecule) is capable of binding two different epitopes either on the same antigen or on two different antigens.

The term "isolated antibody" or "isolated antibody-like molecule" as used herein refers to an antibody or an antibody-like molecule that is substantially free of other antibodies or antibody-like molecules having different antigenic specificities. Moreover, an isolated antibody or antibody-like molecule may be substantially free of other cellular material and/or chemicals.

The term "monoclonal antibody" or "monoclonal antibody composition" as used herein refers to polypeptides, including antibodies, antibody fragments, molecules, etc. that have substantially identical to amino acid sequence or are derived from the same genetic source. This term also includes preparations of antibody molecules of single molecular composition. A monoclonal antibody composition displays a single binding specificity and affinity for a particular epitope.

The term "humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin lo sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1: 105-115 (1998); Harris, Biochem. Soc. Transactions 23:1035-1038 (1995); Hurle and Gross, Curr. Op. Biotech. 5:428-433 (1994).

The term "human antibody" as used herein includes antibodies having variable regions in which both the framework and CDR regions are derived from sequences of human origin. Furthermore, if the antibody contains a constant region, the constant region also is derived from such human sequences, e.g., human germline sequences, or mutated versions of human germline sequences or antibody containing consensus framework sequences derived from human framework sequences analysis, for example, as described in Knappik, et al. (2000. J Mol Biol 296, 57-86). The structures and locations of immunoglobulin variable domains, e.g., CDRs, may be defined using well known numbering schemes, e.g., the Kabat numbering scheme, the Chothia numbering scheme, or a combination of Kabat and Chothia (see, e.g., Sequences of Proteins of Immunological Interest, U.S. Department of Health and Human Services (1991), eds. Kabat *et al.;* Al Lazikani et al., (1997) J. Mol. Bio. 273:927 948); Kabat et al., (1991) Sequences of Proteins of Immunological Interest, 5th edit., NIH Publication no. 91-3242 U.S. Department of Health and Human Services; Chothia et al., (1987) J. Mol. Biol. 196:901-917; Chothia et al, (1989) Nature 342:877-883; and Al-Lazikani et al., (1997) J. Mol. Biol. 273:927-948.

The human antibodies of the invention may include amino acid residues not encoded by human sequences (e.g., mutations introduced by random or site-specific mutagenesis in vitro or by somatic mutation in vivo, or a conservative substitution to promote stability or manufacturing). However, the term "human antibody", as used herein, is not intended to include antibodies in which CDR sequences derived from the germline of another mammalian species, such as a mouse, have been grafted onto human framework sequences.

A "modification" or "mutation" of an amino acid residue/position, as used herein, refers to a change of a primary amino acid sequence as compared to a starting amino acid sequence, wherein the change results from a sequence alteration involving said amino acid residue/positions. For example, typical modifications include substitution of the residue (or at said position) with another amino acid (e.g., a conservative or non-conservative substitution), insertion of one or more amino acids adjacent to said residue/position, and deletion of said residue/position. An "amino acid substitution," or variation thereof, refers to the replacement of an existing amino acid residue in a predetermined (starting) amino acid sequence with a different amino acid residue. Generally and preferably, the modification results in alteration in at least one physicobiochemical activity of the variant polypeptide compared to a polypeptide comprising the starting (or "wild type") amino acid sequence. For example, in the case of an antibody, a physicobiochemical activity that is altered can be binding affinity, binding capability and/or binding effect upon a target molecule.

The term "conservatively modified variant" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" include individual substitutions, deletions or additions to a polypeptide sequence which result in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, *e.g.*, Creighton, Proteins (1984)). In some embodiments, the phrase "conservative sequence modifications" are used to refer to amino acid modifications that do not significantly affect or alter the binding characteristics of the antibody or the antibody-like molecule containing the amino acid sequence.

The terms "percent identical" or "percent identity," in the context of two or more nucleic acids or polypeptide sequences, refers to two or more sequences or subsequences that are the same. Two sequences are "substantially identical" if two sequences have a specified percentage of amino acid residues or nucleotides that are the same (*i.e.,* 60% identity, optionally 65%, 70%, 75%, 80%, 85%, 90%, 95%, or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

The term "comparison window" as used herein includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, *e.g.*, by the local homology algorithm of Smith and Waterman (1970) Adv. Appl. Math. 2:482c, by the homology alignment algorithm of Needleman and Wunsch (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, *e.g.,* Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al*., *supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, (1989) Proc. Natl. Acad. Sci. USA 89:10915) alignments (B) of 50, expectation (E) of 10, M=5, N=-4, and a comparison of both strands.

The BLAST algorithm also performs a statistical analysis of the similarity between two sequences (see, *e.g*., Karlin and Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The percent identity between two amino acid sequences can also be determined using the algorithm of E. Meyers and W. Miller (Comput. Appl. Biosci. 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol, Biol. 48:444-453 (1970)) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

Other than percentage of sequence identity noted above, another indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

The term "nucleic acid" is used herein interchangeably with the term "polynucleotide" and refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (*e.g.*, degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., (1991) Nucleic Acid Res. 19:5081; Ohtsuka et al., (1985) J. Biol. Chem. 260:2605-2608; and Rossolini et al., (1994) Mol. Cell. Probes 8:91-98).

The term "operably linked" or functionally linked, as used herein, refers to a functional relationship between two or more polynucleotide (*e.g.*, DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, *i.e*., they are cis-acting. However, some transcriptional regulatory sequences, such as enhancers, need not be physically contiguous or located in close proximity to the coding sequences whose transcription they enhance.

The terms "polypeptide" and "protein" are used interchangeably herein to refer to a polymer of amino acid residues. The phrases also apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

The term "*in vivo* half life", as used herein, refers to the half-life of the molecule of interest or variants thereof circulating in the blood of a given mammal.

The term "subject" includes human and non-human animals. Non-human animals include all vertebrates, *e.g.*, mammals and non-mammals, such as non-human primates, sheep, dog, cow, chickens, amphibians, and reptiles. Except when noted, the terms "patient" or "subject" are used herein interchangeably.

The term "cancer" refers to a disease characterized by the rapid and uncontrolled growth of aberrant cells. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. Preferred cancers treated by the methods described herein include multiple myeloma, Hodgkin's lymphoma or non-Hodgkin's lymphoma.

The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

The terms "cancer antigen," "cancer associated antigen" or "tumor antigen" interchangeably refer to a molecule (typically a protein, carbohydrate or lipid) that is expressed on the surface of a cancer cell, either entirely or as a fragment (e.g., MHC/peptide), and which is useful for the preferential targeting of a pharmacological agent to the cancer cell. In some embodiments, a tumor antigen is a marker expressed by both normal cells and cancer cells, e.g., a lineage marker, e.g., CD19 on B cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some embodiments, a tumor antigen is a cell surface molecule that is inappropriately synthesized in the cancer cell, for instance, a molecule that contains deletions, additions or mutations in comparison to the molecule expressed on a normal cell. In some embodiments, a tumor antigen will be expressed exclusively on the cell surface of a cancer cell, entirely or as a fragment (e.g., MHC/peptide), and not synthesized or expressed on the surface of a normal cell. In some embodiments, the multispecific molecules of the present invention includes multispecific molecules comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5): 1935-1942; Sergeeva et al., Blood, 2011 117(16):4262-4272; Verma et al., J Immunol 2010 184(4):2156-2165; Willemsen et al., Gene Ther 2001 8(21) : 1601-1608 ; Dao et al., Sci Transl Med 2013 5(176) :176ra33 ; Tassev et al., Cancer Gene Ther 2012 19(2):84-100). For example, TCR-like antibody can be identified from screening a library, such as a human scFv phage displayed library.

As used herein, the terms "treat", "treatment" and "treating" refer to the reduction or amelioration of the progression, severity and/or duration of a proliferative disorder, or the amelioration of one or more symptoms (preferably, one or more discernible symptoms) of a proliferative disorder resulting from the administration of one or more therapies (e.g., one or more therapeutic agents such as a multispecific molecule, e.g., bispecific molecule, e.g., bispecific antibody or bispecific antibody-like molecule, of the invention). In specific embodiments, the terms "treat", "treatment" and "treating" refer to the amelioration of at least one measurable physical parameter of a proliferative disorder, such as growth of a tumor, not necessarily discernible by the patient. In other embodiments the terms "treat", "treatment" and "treating" -refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

Various aspects of the invention are described in further detail in the following sections and subsections.

### I. Anti-CLL-1 Binding Domains

In one aspect, the invention provides a number of multispecific molecules, e.g., bispecific molecules, e.g., bispecific antibodies, comprising an antibody or antibody fragment engineered for enhanced binding to a CLL-1 protein.

In one aspect the anti-CLL-1 binding domain of the multispecific molecule, e.g., a bispecific molecule, e.g., a bispecific antibody, comprises more than one polypeptide. By way of example, the anti-CLL-1 binding domain of a multispecific molecule, e.g., a bispecific molecule, e.g., a bispecific antibody, may comprise a light chain variable region, e.g., as described herein, disposed on a first polypeptide and a heavy chain variable region, e.g., as described herein, disposed on a second polypeptide. In one aspect the polypeptide comprising the light chain variable region and the polypeptide comprising the heavy chain polypeptide form a half antibody. In another aspect, the anti-CLL-1 binding domain consists of one polypeptide, e.g., a polypeptide comprising both a light chain variable region and a heavy chain variable region of an anti-CLL-1 binding domain, e.g., as described herein. In one aspect, the anti-CLL-1 antigen binding portion of the multispecific molecule, e.g., of the bispecific molecule, e.g., of the bispecific antibody or bispecific antibody-like molecule, is a scFv antibody fragment. In one aspect such antibody fragments are functional in that they retain the equivalent binding affinity, e.g., they bind the same antigen with comparable efficacy, as the IgG antibody from which it is derived. In other embodiments, the antibody fragment has a lower binding affinity, e.g., it binds the same antigen with a lower binding affinity than the antibody from which it is derived, but is functional in that it provides a biological response described herein. In one embodiment, the multispecific molecule, e.g., of the bispecific molecule, e.g., of the bispecific antibody or bispecific antibody-like molecule, molecule comprises an antibody fragment that has a binding affinity KD of 10-4 M to 10-8 M, e.g., 10-5 M to 10-7 M, e.g., 10-6 M or 10-7 M, for the target antigen. In one embodiment, the antibody fragment has a binding affinity that is at least five-fold, 10-fold, 20-fold, 30-fold, 50-fold, 100-fold or 1,000-fold less than a reference antibody, e.g., an antibody described herein.

In one aspect such antibody fragments are functional in that they provide a biological response that can include, but is not limited to, activation of an immune response, inhibition of signal-transduction origination from its target antigen, inhibition of kinase activity, and the like, as will be understood by a skilled artisan. In one aspect, the anti-CLL-1 antigen binding domain of the multispecific molecule, e.g., of the bispecific molecule, e.g., of the bispecific antibody or bispecific antibody-like molecule, is a scFv antibody fragment that is humanized compared to the murine sequence of the scFv from which it is derived. In one embodiment, the anti-CLL-1 antigen binding domain is a human anti-CLL-1 antigen binding domain. In one embodiment, the anti-CLL-1 antigen binding domain is a humanized anti-CLL-1 antigen binding domain.

In some aspects, the anti-CLL-1 binding domains of the invention are incorporated into a multispecific molecule, e.g., of the bispecific molecule, e.g., of the bispecific antibody or bispecific antibody-like molecule. In one aspect, the multispecific molecule, e.g., of the bispecific molecule, e.g., of the bispecific antibody or bispecific antibody-like molecule, comprises a CLL-1 binding domain comprising a sequence of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, or SEQ ID NO: 51. In one aspect, the scFv domains are human.

In one aspect, the anti-CLL-1 binding domain, e.g., human or humanized scFv, portion of a multispecific molecule, e.g., of a bispecific molecule, e.g., of a bispecific antibody, of the invention is encoded by a transgene whose sequence has been codon optimized for expression in a mammalian cell. In one aspect, the chains, e.g., the entire construct, of the multispecific molecule of the invention is encoded by a transgene whose entire sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least US Patent Numbers 5,786,464 and 6,114,148.

In one aspect, the human anti-CLL-1 binding domain comprises the scFv portion provided in SEQ ID NO: 39. In one embodiment, the human anti- CLL-1 binding domain comprises the scFv portion provided in SEQ ID NO:40. In one embodiment, the human anti-CLL-1 binding domain comprises the scFv portion provided in SEQ ID NO:41. In one embodiment, the human anti-CLL-1 binding domain comprises the scFv portion provided in SEQ ID NO:42. In one embodiment, the human anti-CLL-1 binding domain comprises the scFv portion provided in SEQ ID NO:43. In one embodiment, the human anti-CLL-1 binding domain comprises the scFv portion provided in SEQ ID NO:44. In one embodiment, the human anti-CLL-1 binding domain comprises the scFv portion provided in SEQ ID NO:45. In one embodiment, the human anti-CLL-1 binding domain comprises the scFv portion provided in SEQ ID NO:46. In one embodiment, the human anti-CLL-1 binding domain comprises the scFv portion provided in SEQ ID NO:47. In one embodiment, the human anti-CLL-1 binding domain comprises the scFv portion provided in SEQ ID NO:48. In one embodiment, the human anti-CLL-1 binding domain comprises the scFv portion provided in SEQ ID NO:49. In one embodiment, the human anti-CLL-1 binding domain comprises the scFv portion provided in SEQ ID NO:50. In one embodiment, the human anti-CLL-1 binding domain comprises the scFv portion provided in SEQ ID NO:51.

Furthermore, the present invention provides CLL-1 multispecific molecule, e.g., bispecific molecule, e.g., bispecific antibody or bispecific antibody-like molecule, compositions and their use in medicaments or methods for treating, among other diseases, cancer or any malignancy or autoimmune diseases involving cells or tissues which express CLL-1.

In one aspect, the multispecific molecule, e.g., bispecific molecule, e.g., bispecific antibody or bispecific antibody-like molecule, of the invention can be used to eradicate CLL-1-expressing normal cells, and is thereby applicable for use as a conditioning therapy prior to cell transplantation. In one aspect, the CLL-1-expressing normal cell is a CLL-1-expressing normal stem cell and the cell transplantation is a stem cell transplantation.

The present invention includes a multispecific molecule, e.g., bispecific molecule, e.g., bispecific antibody or bispecific antibody-like molecule, that comprises an anti-CLL-1 binding domain (e.g., human or humanized CLL-1 binding domain as described herein), and at least a second antigen binding domain, and wherein said anti-CLL-1 binding domain comprises a heavy chain complementary determining region 1 (HC CDR1), a heavy chain complementary determining region 2 (HC CDR2), and a heavy chain complementary determining region 3 (HC CDR3) of any anti-CLL-1 heavy chain binding domain amino acid sequence listed in Table 2. The anti-CLL-1 binding domain of the multispecific molecule, e.g., bispecific molecule, e.g., bispecific antibody or bispecific antibody-like molecule, can further comprise a light chain complementary determining region 1 (LC CDR1), a light chain complementary determining region 2 (LC CDR2), and a light chain complementary determining region 3 (LC CDR3) of any anti-CLL-1 light chain binding domain amino acid sequence listed in Table 2.

The present invention also provides nucleic acid molecules encoding the multispecific molecule, e.g., bispecific molecule, e.g., bispecific antibody or bispecific antibody-like molecule, e.g., as described herein, e.g., encoding a multispecific molecule, e.g., bispecific molecule, e.g., bispecific antibody or bispecific antibody-like molecule that comprises an anti-CLL-1 binding domain (e.g., human or humanized CLL-1 binding domain as described herein), wherein said anti-CLL-1 binding domain comprises a heavy chain complementary determining region 1 (HC CDR1), a heavy chain complementary determining region 2 (HC CDR2), and a heavy chain complementary determining region 3 (HC CDR3) of any anti-CLL-1 heavy chain binding domain amino acid sequence listed in Table 2. In embodiments, the encoded anti-CLL-1 binding domain of the multispecific molecule, e.g., bispecific molecule, e.g., bispecific antibody or bispecific antibody-like molecule can further comprise a light chain complementary determining region 1 (LC CDR1), a light chain complementary determining region 2 (LC CDR2), and a light chain complementary determining region 3 (LC CDR3) of any anti-CLL-1 light chain binding domain amino acid sequence listed in Table 2.

In specific aspects, a multispecific molecule, e.g., bispecific molecule, e.g., bispecific antibody or bispecific antibody-like molecule, construct of the invention comprises a scFv domain selected from the group consisting of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51, wherein the scFv may be preceded by an optional leader sequence such as provided in SEQ ID NO: 1. Also included in the invention is a nucleotide sequence that encodes the polypeptide of any of the scFv fragments selected from the group consisting of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51.

Also included in the invention is nucleic acid molecule comprising a nucleotide sequence that encodes the polypeptide of any of the scFv fragments selected from the group consisting of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51, and each of the domains of SEQ ID NO: 1, plus any of the additional domains of the CLL-1 multispecific molecule, e.g., bispecific molecule, e.g., bispecific antibody or bispecific antibody-like molecule, of the invention.

In one aspect, an exemplary CLL-1 multispecific molecule, e.g., bispecific molecule, e.g., bispecific antibody or bispecific antibody-like molecule, construct comprises an optional leader sequence. Specific CLL-1 multispecific molecule, e.g., bispecific molecule, e.g., bispecific antibody or bispecific antibody-like molecule, constructs containing human scFv domains of the invention are provided as SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51.

An exemplary leader sequence is provided as SEQ ID NO: 1.

In one aspect, the present invention encompasses a recombinant nucleic acid construct comprising a nucleic acid molecule encoding a multispecific molecule, e.g., bispecific molecule, e.g., bispecific antibody or bispecific antibody-like molecule, wherein the nucleic acid molecule comprises the nucleic acid sequence encoding an anti-CLL-1 binding domain, or fragment thereof (e.g., a VL or VH domain). In one aspect, the nucleic acid encoding the anti-CLL-1 binding domain is selected from one or more of SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63 and SEQ ID NO: 64. In one aspect, the nucleic acid encoding the anti-CLL-1 binding domain comprises SEQ ID NO: 52. In one aspect, the nucleic acid encoding the anti-CLL-1 binding domain comprises SEQ ID NO: 53. In one aspect, the nucleic acid encoding the anti-CLL-1 binding domain comprises SEQ ID NO: 54. In one aspect, the nucleic acid encoding the anti-CLL-1 binding domain comprises SEQ ID NO: 55. In one aspect, the nucleic acid encoding the anti-CLL-1 binding domain comprises SEQ ID NO: 56. In one aspect, the nucleic acid encoding the anti-CLL-1 binding domain comprises SEQ ID NO: 57. In one aspect, the nucleic acid encoding the anti-CLL-1 binding domain comprises SEQ ID NO: 58. In one aspect, the nucleic acid encoding the anti-CLL-1 binding domain comprises SEQ ID NO: 59. In one aspect, the nucleic acid encoding the anti-CLL-1 binding domain comprises SEQ ID NO: 60. In one aspect, the nucleic acid encoding the anti-CLL-1 binding domain comprises SEQ ID NO: 61. In one aspect, the nucleic acid encoding the anti-CLL-1 binding domain comprises SEQ ID NO: 62. In one aspect, the nucleic acid encoding the anti-CLL-1 binding domain comprises SEQ ID NO: 63. In one aspect, the nucleic acid encoding the anti-CLL-1 binding domain comprises SEQ ID NO: 64.

In one aspect, the present invention encompasses a recombinant nucleic acid construct comprising a nucleic acid molecule encoding a domain, e.g., a VH or a VL domain, of a multispecific molecule, e.g., bispecific molecule, e.g., bispecific antibody or bispecific antibody-like molecule, wherein the nucleic acid molecule comprises a nucleic acid sequence encoding a domain (e.g., a VH or VL) of an anti-CLL-1 binding domain selected from one or more of a VH or VL of any of SEQ ID NO: 39-51.

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the nucleic acid of interest can be produced synthetically, rather than cloned.

The present invention includes vector constructs, e.g., plasmid, retroviral and lentiviral vector constructs expressing a multispecific molecule, e.g., bispecific molecule, e.g., bispecific antibody or bispecific antibody-like molecule, that can be directly transduced into a cell.

The present invention also includes an RNA construct that can be directly transfected into a cell. A method for generating mRNA for use in transfection involves in vitro transcription (IVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-2000 bases in length (SEQ ID NO:35). RNA so produced can efficiently transfect different kinds of cells. In one embodiment, the template includes sequences for the polypeptides of the multispecific molecule, e.g., bispecific molecule, e.g., bispecific antibody or bispecific antibody-like molecule. In an embodiment, an RNA vector is transduced into a cell by electroporation.

The multispecific molecules, e.g., bispecific molecules, e.g., bispecific antibodies, of the present invention comprise a target-specific binding domain. The choice of moiety depends upon the type and number of ligands that define the surface of a target cell. For example, the antigen binding domain may be chosen to recognize an antigen that acts as a cell surface marker on target cells associated with a particular disease state.

In one aspect, the multispecific molecule, e.g., bispecific molecule, e.g., bispecific antibody or bispecific antibody-like molecule, of the present invention comprises a binding domain that specifically binds CLL-1. In one aspect, the multispecific molecule, e.g., bispecific molecule, e.g., bispecific antibody or bispecific antibody-like molecule, of the present invention comprises an antigen binding domain that specifically binds human CLL-1.

Each of the antigen binding domains, e.g., the anti-CLL-1 binding domain, can be any protein that binds to the antigen including but not limited to a monoclonal antibody, a polyclonal antibody, a recombinant antibody, a human antibody, a humanized antibody, and a functional fragment thereof, including but not limited to a single-domain antibody such as a heavy chain variable domain (VH), a light chain variable domain (VL) and a variable domain (VHH) of camelid derived nanobody, a Fab, a scFv, and to an alternative scaffold known in the art to function as antigen binding domain, such as a recombinant fibronectin domain, and the like. In some instances, it is beneficial for the antigen binding domain to be derived from the same species in which the multispecific molecule, e.g., bispecific molecule, e.g., bispecific antibody or bispecific antibody-like molecule, will ultimately be used in. For example, for use in humans, it may be beneficial for the antigen binding domain of the multispecific molecule, e.g., bispecific molecule, e.g., bispecific antibody or bispecific antibody-like molecule, to comprise human or humanized residues for the antigen binding domain of an antibody or antibody fragment.

Thus, in one aspect, the antigen binding domain comprises a human or a humanized antibody or an antibody fragment. In one embodiment, the human anti-CLL-1 binding domain comprises one or more (e.g., all three) light chain complementary determining region 1 (LC CDR1), light chain complementary determining region 2 (LC CDR2), and light chain complementary determining region 3 (LC CDR3) of a human anti-CLL-1 binding domain described herein (e.g., in Table 2), and/or one or more (e.g., all three) heavy chain complementary determining region 1 (HC CDR1), heavy chain complementary determining region 2 (HC CDR2), and heavy chain complementary determining region 3 (HC CDR3) of a human anti-CLL-1 binding domain described herein (e.g., in Table 2), e.g., a human anti-CLL-1 binding domain comprising one or more, e.g., all three, LC CDRs and one or more, e.g., all three, HC CDRs. In one embodiment, the human anti-CLL-1 binding domain comprises one or more (e.g., all three) heavy chain complementary determining region 1 (HC CDR1), heavy chain complementary determining region 2 (HC CDR2), and heavy chain complementary determining region 3 (HC CDR3) of a human anti-CLL-1 binding domain described herein (e.g., in Table 2), e.g., the human anti-CLL-1 binding domain has two variable heavy chain regions, each comprising a HC CDR1, a HC CDR2 and a HC CDR3 described herein. In one embodiment, the human anti-CLL-1 binding domain comprises a human light chain variable region described herein (e.g., in Table 2) and/or a human heavy chain variable region described herein (e.g., in Table 2). In one embodiment, the human anti-CLL-1 binding domain comprises a human heavy chain variable region described herein (e.g., in Table 2), e.g., at least two human heavy chain variable regions described herein (e.g., in Table 2). In one embodiment, the anti-CLL-1 binding domain is a scFv comprising a light chain and a heavy chain of an amino acid sequence of Table 2. In an embodiment, the anti-CLL-1 binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 2, or a sequence with 95-99% identity with an amino acid sequence of Table 2; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 2, or a sequence with 95-99% identity to an amino acid sequence of Table 2.

In one embodiment, the human anti-CLL-1 binding domain comprises a sequence selected from a group consisting of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51, or a sequence with 95-99% identify thereof. In one embodiment, the nucleic acid sequence encoding the human anti-CLL-1 binding domain comprises a sequence selected from a group consisting of SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, and SEQ ID NO: 64, or a sequence with 95-99% identify thereof. In one embodiment, the human anti-CLL-1 binding domain is a scFv, and a light chain variable region comprising an amino acid sequence described herein, e.g., in Table 2, is attached to a heavy chain variable region comprising an amino acid sequence described herein, e.g., in Table 2, via a linker, e.g., a linker described herein. In one embodiment, the human anti-CLL-1 binding domain includes a (Gly₄-Ser)n linker, wherein n is 1, 2, 3, 4, 5, or 6, preferably 3 or 4 (SEQ ID NO:26). The light chain variable region and heavy chain variable region of a scFv can be, e.g., in any of the following orientations: light chain variable region-linker-heavy chain variable region or heavy chain variable region-linker-light chain variable region. In one aspect, the anti-CLL-1 antigen binding domain portion comprises one or more sequence selected from SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51.

In one embodiment, the anti- CLL-1 binding domain comprises a light chain variable region described herein (e.g., in Table 2) and/or a heavy chain variable region described herein (e.g., in Table 2). In one embodiment, the anti-CLL-1 binding domain comprises a polypeptide comprising a light chain variable region described herein (e.g., in Table 2) and a polypeptide comprising a heavy chain variable region described herein (e.g., in Table 2). In some embodiments, the polypeptide comprising the light chain variable region described herein (e.g., in Table 2) and the polypeptide comprising a heavy chain variable region described herein (e.g., in Table 2) form a half antibody, or antibody fragment thereof (e.g., noncovalently associate or covalently associate to form a half antibody, or antibody fragment thereof), comprising an anti-CLL-1 binding domain.

In one embodiment, the anti-CLL-1 binding domain comprises a light chain variable region provided in SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, or SEQ ID NO: 196, and/or a heavy chain variable region provided in SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, or SEQ ID NO: 195. In one embodiment, the encoded anti- CLL-1 binding domain is a scFv comprising a light chain and a heavy chain of an amino acid sequence of Table 2. In an embodiment, the human or humanized anti-CLL-1 binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, or SEQ ID NO: 196, or a sequence with 95-99% identity thereof; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, or SEQ ID NO: 195, or a sequence with 95-99% identity thereof. In one embodiment, the encoded anti-CLL-1 binding domain includes a (Gly4-Ser)n linker, wherein n is 1, 2, 3, 4, 5, or 6, preferably 3 or 4 (SEQ ID NO:26). The light chain variable region and heavy chain variable region of a scFv can be, e.g., in any of the following orientations: light chain variable region-linker-heavy chain variable region or heavy chain variable region-linker-light chain variable region.

In embodiments, the anti-CLL-1 binding domain comprises the light chain variable region provided in SEQ ID NO: 78 and the heavy chain variable region provided in SEQ ID NO: 65. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 78, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 65, or a sequence with 95-99% identity thereof.

In embodiments, the anti-CLL-1 binding domain comprises the light chain variable region provided in SEQ ID NO: 79 and the heavy chain variable region provided in SEQ ID NO: 66. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 79, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 66, or a sequence with 95-99% identity thereof.

In embodiments, the anti-CLL-1 binding domain comprises the light chain variable region provided in SEQ ID NO: 80 and the heavy chain variable region provided in SEQ ID NO: 67. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 80, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 67, or a sequence with 95-99% identity thereof.

In embodiments, the anti-CLL-1 binding domain comprises the light chain variable region provided in SEQ ID NO: 81 and the heavy chain variable region provided in SEQ ID NO: 68. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 81, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 68, or a sequence with 95-99% identity thereof.

In embodiments, the anti-CLL-1 binding domain comprises the light chain variable region provided in SEQ ID NO: 82 and the heavy chain variable region provided in SEQ ID NO: 69. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 82, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 69, or a sequence with 95-99% identity thereof.

In embodiments, the anti-CLL-1 binding domain comprises the light chain variable region provided in SEQ ID NO: 83 and the heavy chain variable region provided in SEQ ID NO: 70. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 83, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 70, or a sequence with 95-99% identity thereof.

In embodiments, the anti-CLL-1 binding domain comprises the light chain variable region provided in SEQ ID NO: 84 and the heavy chain variable region provided in SEQ ID NO: 71. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 84, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 71, or a sequence with 95-99% identity thereof.

In embodiments, the anti-CLL-1 binding domain comprises the light chain variable region provided in SEQ ID NO: 85 and the heavy chain variable region provided in SEQ ID NO: 72. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 85, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 72, or a sequence with 95-99% identity thereof.

In embodiments, the anti-CLL-1 binding domain comprises the light chain variable region provided in SEQ ID NO: 86 and the heavy chain variable region provided in SEQ ID NO: 73. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 86, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 73, or a sequence with 95-99% identity thereof.

In embodiments, the anti-CLL-1 binding domain comprises the light chain variable region provided in SEQ ID NO: 87 and the heavy chain variable region provided in SEQ ID NO: 74. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 87, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 74, or a sequence with 95-99% identity thereof.

In embodiments, the anti-CLL-1 binding domain comprises the light chain variable region provided in SEQ ID NO: 88 and the heavy chain variable region provided in SEQ ID NO: 75. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 88, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 75, or a sequence with 95-99% identity thereof.

In embodiments, the anti-CLL-1 binding domain comprises the light chain variable region provided in SEQ ID NO: 89 and the heavy chain variable region provided in SEQ ID NO: 76. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 89, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 76, or a sequence with 95-99% identity thereof.

In embodiments, the anti-CLL-1 binding domain comprises the light chain variable region provided in SEQ ID NO: 90 and the heavy chain variable region provided in SEQ ID NO: 77. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 90, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 77, or a sequence with 95-99% identity thereof.

In embodiments, the anti-CLL-1 binding domain comprises the light chain variable region provided in SEQ ID NO: 196 and the heavy chain variable region provided in SEQ ID NO: 195. In embodiments, the anti-CLL-1 binding domain comprises a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the light chain variable region amino acid sequence provided in SEQ ID NO: 196, or a sequence with 95-99% identity thereof; and a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of the heavy chain variable region amino acid sequence provided in SEQ ID NO: 195, or a sequence with 95-99% identity thereof.

In embodiments, the anti-CLL-1 binding domain comprises a light chain variable region and a heavy chain variable region of any anti-CLL-1 binding domain of Table 2. In embodiments, the anti-CLL-1 binding domain comprises a light chain variable region amino acid sequence selected from the group consisting of SEQ ID NO: 78-90, and a heavy chain variable region amino acid sequence selected from the group consisting of SEQ ID NO: 65-77.

In some aspects, a non-human anti-CLL-1 binding domain is humanized, where specific sequences or regions of the antibody are modified to increase similarity to an antibody naturally produced in a human or fragment thereof. In one aspect, the antigen binding domain is humanized.

A humanized antibody can be produced using a variety of techniques known in the art, including but not limited to, CDR-grafting (see, e.g., European Patent No. EP 239,400; International Publication No. WO 91/09967; and U.S. Pat. Nos. 5,225,539, 5,530,101, and 5,585,089, each of which is incorporated herein in its entirety by reference), veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973, each of which is incorporated herein by its entirety by reference), chain shuffling (see, e.g., U.S. Pat. No. 5,565,332, which is incorporated herein in its entirety by reference), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., J. Immunol., 169:1119-25 (2002), Caldas et al., Protein Eng., 13(5):353-60 (2000), Morea et al., Methods, 20(3):267-79 (2000), Baca et al., J. Biol. Chem., 272(16): 10678-84 (1997), Roguska et al., Protein Eng., 9(10):895-904 (1996), Couto et al., Cancer Res., 55 (23 Supp):5973s-5977s (1995), Couto et al., Cancer Res., 55(8):1717-22 (1995), Sandhu J S, Gene, 150(2):409-10 (1994), and Pedersen et al., J. Mol. Biol., 235(3):959-73 (1994), each of which is incorporated herein in its entirety by reference. Often, framework residues in the framework regions will be substituted with the corresponding residue from the CDR donor antibody to alter, for example improve, antigen binding. These framework substitutions, e.g., conservative substitutions are identified by methods well-known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; and Riechmann et al., 1988, Nature, 332:323, which are incorporated herein by reference in their entireties.)

A humanized antibody or antibody fragment has one or more amino acid residues remaining in it from a source which is nonhuman. These nonhuman amino acid residues are often referred to as "import" residues, which are typically taken from an "import" variable domain. As provided herein, humanized antibodies or antibody fragments comprise one or more CDRs from nonhuman immunoglobulin molecules and framework regions wherein the amino acid residues comprising the framework are derived completely or mostly from human germline. Multiple techniques for humanization of antibodies or antibody fragments are well-known in the art and can essentially be performed following the method of Winter and coworkers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567; 6,331,415; 5,225,539; 5,530,101; 5,585,089; 6,548,640, the contents of which are incorporated herein by reference herein in their entirety). In such humanized antibodies and antibody fragments, substantially less than an intact human variable domain has been substituted by the corresponding sequence from a nonhuman species. Humanized antibodies are often human antibodies in which some CDR residues and possibly some framework (FR) residues are substituted by residues from analogous sites in rodent antibodies. Humanization of antibodies and antibody fragments can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332), the contents of which are incorporated herein by reference herein in their entirety.

The choice of human variable domains, both light and heavy, to be used in making the humanized antibodies is to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987), the contents of which are incorporated herein by reference herein in their entirety). Another method uses a particular framework derived from the consensus sequence of all human antibodies of a particular subgroup of light or heavy chains. The same framework may be used for several different humanized antibodies (see, e.g., Nicholson et al. Mol. Immun. 34 (16-17): 1157-1165 (1997); Carter et al., Proc. Natl. Acad. Sci. USA, 89:4285 (1992); Presta et al., J. Immunol., 151:2623 (1993), the contents of which are incorporated herein by reference herein in their entirety). In some embodiments, the framework region, e.g., all four framework regions, of the heavy chain variable region are derived from a VH4_4-59 germline sequence. In one embodiment, the framework region can comprise, one, two, three, four or five modifications, e.g., substitutions, e.g., conservative substitutions, e.g., from the amino acid at the corresponding murine sequence. In one embodiment, the framework region, e.g., all four framework regions of the light chain variable region are derived from a VK3_1.25 germline sequence. In one embodiment, the framework region can comprise, one, two, three, four or five modifications, e.g., substitutions, e.g., conservative substitutions, e.g., from the amino acid at the corresponding murine sequence.

In some aspects, the portion of a multispecific molecule, e.g., bispecific molecule, e.g., bispecific antibody or bispecific antibody-like molecule, composition of the invention that comprises an antibody fragment is humanized with retention of high affinity for the target antigen and other favorable biological properties. According to one aspect of the invention, humanized antibodies and antibody fragments are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, e.g., the analysis of residues that influence the ability of the candidate immunoglobulin to bind the target antigen. In this way, FR residues can be selected and combined from the recipient and import sequences so that the desired antibody or antibody fragment characteristic, such as increased affinity for the target antigen, is achieved. In general, the CDR residues are directly and most substantially involved in influencing antigen binding.

A humanized antibody or antibody fragment may retain a similar antigenic specificity as the original antibody, e.g., in the present invention, the ability to bind human CLL-1. In some embodiments, a humanized antibody or antibody fragment may have improved affinity and/or specificity of binding to human CLL-1.

In one aspect, the anti-CLL-1 binding domain is characterized by particular functional features or properties of an antibody or antibody fragment. For example, in one aspect, the portion of a multispecific molecule, e.g., bispecific molecule, e.g., bispecific antibody or bispecific antibody-like molecule, composition of the invention that comprises an antigen binding domain to CLL-1 specifically binds human CLL-1.

In one aspect, the anti-CLL-1 antigen binding domain has the same or a similar binding specificity to human CLL-1 as mouse CLL-1. In one aspect, the invention relates to an antigen binding domain comprising an antibody or antibody fragment, wherein the antibody binding domain specifically binds to a CLL-1 protein or fragment thereof, wherein the antibody or antibody fragment comprises a variable light chain and/or a variable heavy chain that includes an amino acid sequence of SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 195, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, or SEQ ID NO: 196. In one aspect, the antigen binding domain comprises an amino acid sequence of an scFv selected from SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, and SEQ ID NO: 51. In certain aspects, the scFv is contiguous with and in the same reading frame as a leader sequence. In one aspect the leader sequence is the polypeptide sequence provided as SEQ ID NO:1.

In one aspect, the anti-CLL-1 binding domain is a fragment, e.g., a single chain variable fragment (scFv). In one aspect, the anti-CLL-1 binding domain is a Fv, a Fab, a (Fab')2, or a bi-functional (e.g. bi-specific) hybrid antibody (e.g., Lanzavecchia et al., Eur. J. Immunol. 17, 105 (1987)). In one aspect, the antibodies and fragments thereof of the invention binds a CLL-1 protein with wild-type or enhanced affinity.

In some instances, scFvs can be prepared according to method known in the art (see, for example, Bird et al., (1988) Science 242:423-426 and Huston et al., (1988) Proc. Natl. Acad. Sci. USA 85:5879-5883). ScFv molecules can be produced by linking VH and VL regions together using flexible polypeptide linkers. The scFv molecules comprise a linker (e.g., a Ser-Gly linker) with an optimized length and/or amino acid composition. The linker length can greatly affect how the variable regions of a scFv fold and interact. In fact, if a short polypeptide linker is employed (e.g., between 5-10 amino acids) intrachain folding is prevented. Interchain folding is also required to bring the two variable regions together to form a functional epitope binding site. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715, is incorporated herein by reference.

An scFv can comprise a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its VL and VH regions. The linker sequence may comprise any naturally occurring amino acid. In some embodiments, the linker sequence comprises amino acids glycine and serine. In another embodiment, the linker sequence comprises sets of glycine and serine repeats such as (Gly₄Ser)n, where n is a positive integer equal to or greater than 1 (SEQ ID NO:25). In one embodiment, the linker can be (Gly₄Ser)₄ (SEQ ID NO:27) or (Gly₄Ser)₃(SEQ ID NO:28). Variation in the linker length may retain or enhance activity, giving rise to superior efficacy in activity studies.

### Exemplary Anti-CLL-1 Binding Domains

Exemplary CLL-1 multispecific molecule, e.g., bispecific molecule, e.g., bispecific antibody or bispecific antibody-like molecule, constructs disclosed herein comprise an scFv (e.g., a scFv as disclosed in Table 2, optionally preceded with an optional leader sequence (e.g., SEQ ID NO:1 and SEQ ID NO:12 for exemplary leader amino acid and nucleotide sequences, respectively)). The sequences of the scFv fragments (SEQ ID NOs: 39-51, not including the optional leader sequence) are provided herein in Table 2 and the description below. The CLL-1 multispecific molecule, e.g., bispecific molecule, e.g., bispecific antibody or bispecific antibody-like molecule, construct can further comprise one or more additional antibody domains, e.g., one or more LCs, one or more CH1s, one or more CH2s, one or more CH3s, one or more hinge domains, and/or one or more additional VL and/or VH domains, e.g., as described herein. In certain embodiments, the domains are contiguous with and in the same reading frame to form single polypeptide. In other embodiments, the domain are in separate polypeptides, e.g., as in a multispecific antibody or antibody-like molecule described herein.

In certain embodiments, the CLL-1 multispecific molecule includes an amino acid sequence (e.g., a VL, a VH and/or a scFv sequence) of, or includes an amino acid sequence ((e.g., a VL, a VH and/or a scFv sequence) encoded by the nucleotide sequence of, CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 2, or a sequence substantially (e.g., 95-99%) identical thereto.

In certain embodiments, the CLL-1 multispecific molecule, or the anti-CLL-1 antigen binding domain, includes the scFv amino acid sequence of, CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264 provided in Table 2 (with or without the leader sequence), or a sequence substantially identical (e.g., 95-99% identical, or up to 20, 15, 10, 8, 6, 5, 4, 3, 2, or 1 amino acid changes, e.g., substitutions (e.g., conservative substitutions)) to any of the aforesaid sequences.

In certain embodiments, the CLL-1 multispecific molecule, or the anti-CLL-1 antigen binding domain, includes the heavy chain variable region and/or the light chain variable region of, CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264 provided in Table 2, or a sequence substantially identical (e.g., 95-99% identical, or up to 20, 15, 10, 8, 6, 5, 4, 3, 2, or 1 amino acid changes, e.g., substitutions (e.g., conservative substitutions)) to any of the aforesaid sequences.

In certain embodiments, the CLL-1 multispecific molecule, or the anti-CLL-1 antigen binding domain, includes one, two or three CDRs from the heavy chain variable region (e.g., HCDR1, HCDR2 and/or HCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264 provided in Table 3; and/or one, two or three CDRs from the light chain variable region (e.g., LCDR1, LCDR2 and/or LCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 4; or a sequence substantially identical (e.g., 95-99% identical, or up to 20, 15, 10, 8, 6, 5, 4, 3, 2, or 1 amino acid changes, e.g., substitutions (e.g., conservative substitutions)) to any of the aforesaid sequences.

In certain embodiments, the CLL-1 multispecific molecule, or the anti-CLL-1 antigen binding domain, includes one, two or three CDRs from the heavy chain variable region (e.g., HCDR1, HCDR2 and/or HCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 5; and/or one, two or three CDRs from the light chain variable region (e.g., LCDR1, LCDR2 and/or LCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 6; or a sequence substantially identical (e.g., 95-99% identical, or up to 20, 15, 10, 8, 6, 5, 4, 3, 2, or 1 amino acid changes, e.g., substitutions (e.g., conservative substitutions)) to any of the aforesaid sequences.

In certain embodiments, the CLL-1 multispecific molecule, or the anti-CLL-1 antigen binding domain, includes one, two or three CDRs from the heavy chain variable region (e.g., HCDR1, HCDR2 and/or HCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 7; and/or one, two or three CDRs from the light chain variable region (e.g., LCDR1, LCDR2 and/or LCDR3) of CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, CLL-1-13, 139115, 139116, 139117, 139118, 139119, 139120, 139121, 139122, 146259, 146261, 146262, 146263, or 146264, provided in Table 8; or a sequence substantially identical (e.g., 95-99% identical, or up to 20, 15, 10, 8, 6, 5, 4, 3, 2, or 1 amino acid changes, e.g., substitutions (e.g., conservative substitutions)) to any of the aforesaid sequences.

The amino acid and nucleic acid sequences of exemplary CLL-1 scFv domains and exemplary CLL-1 VL and VH domains are provided in Table 2.

Table 1 below designates the nicknames for the CLL-1 constructs with respect to the DNA ID number, also listed in Table 1.

**Table 1: Names of exemplary anti-CLL-1 constructs.**

| Construct ID | Nickname |
|---|---|
| 139115 | CLL-1-1 |
| 139116 | CLL-1-2 |
| 139117 | CLL-1-3 |
| 139118 | CLL-1-4 |
| 139119 | CLL-1-5 |
| 139120 | CLL-1-6 |
| 139121 | CLL-1-7 |
| 139122 | CLL-1-8 |
| 146259 | CLL-1-9 |
| 146261 | CLL-1-10 |
| 146262 | CLL-1-11 |
| 146263 | CLL-1-12 |
| 146264 | CLL-1-13 |

Table 2: Human CLL-1 binding domain sequences

**Table 2: Amino Acid and Nucleic Acid Sequences of anti-CLL-1 binding domains**

| **Name/ Description** | **SEQ ID NO:** | **Sequence** |
|---|---|---|
| **139115** | | |
| **139115- aa ScFv domain CLL-1-1** | 39 | EVQLQQSGAEVKKPGSSVKVSCKASGGTFSSYAISWVRQAPGQGLEWMGG IIPIFGTANYAQKFQGRVTITADESTSTAYMELSSLRSEDTAVYYCARDL EMATIMGGYWGQGTLVTVSSGGGGSGGGGSGGGGSQSALTQPASVSGSPG QSITISCTGTSSDVGGYNYVSWYQQHPGKAPKLMIYDVSNRPSGVSNRFS GSKSGNTASLTISGLQAEDEADYYCSSYTSSSTLDVVFGGGTKLTVL |
| **139115- nt ScFv domain CLL-1-1** | 52 | GAAGTGCAACTCCAACAGTCAGGCGCAGAAGTCAAGAAGCCCGGATCGTC AGTGAAAGTGTCCTGCAAAGCCTCCGGCGGAACCTTCAGCTCCTACGCAA TCAGCTGGGTGCGGCAGGCGCCCGGACAGGGACTGGAGTGGATGGGCGGT ATCATTCCGATCTTTGGCACCGCCAATTACGCCCAGAAGTTCCAGGGACG CGTCACAATCACCGCCGACGAATCGACTTCCACCGCCTACATGGAGCTGT CGTCCTTGAGGAGCGAAGATACCGCCGTGTACTACTGCGCTCGGGATCTG GAGATGGCCACTATCATGGGGGGTTACTGGGGCCAGGGGACCCTGGTCAC TGTGTCCTCGGGAGGAGGGGGATCAGGCGGCGGCGGTTCCGGGGGAGGAG GAAGCCAGTCCGCGCTGACTCAGCCAGCTTCCGTGTCTGGTTCGCCGGGA CAGTCCATCACTATTAGCTGTACCGGCACCAGCAGCGACGTGGGCGGCTA CAACTATGTGTCATGGTACCAGCAGCACCCGGGGAAGGCGCCTAAGCTGA TGATCTACGACGTGTCCAACCGCCCTAGCGGAGTGTCCAACAGATTCTCC GGTTCGAAGTCAGGGAACACTGCCTCCCTCACGATTAGCGGGCTGCAAGC CGAGGATGAAGCCGACTACTACTGCTCCTCCTATACCTCCTCCTCGACCC TGGACGTGGTGTTCGGAGGAGGCACCAAGCTCACCGTCCTT |
| **139115- aa VH of ScFv CLL-1-1** | 65 | EVQLQQSGAEVKKPGSSVKVSCKASGGTFSSYAISWVRQAPGQGLEWMGG IIPIFGTANYAQKFQGRVTITADESTSTAYMELSSLRSEDTAVYYCARDL EMATIMGGYWGQGTLVTVSS |
| **139115- aa VL of ScFv CLL-1-1** | 78 | QSALTQPASVSGSPGQSITISCTGTSSDVGGYNYVSWYQQHPGKAPKLMI YDVSNRPSGVSNRFSGSKSGNTASLTISGLQAEDEADYYCSSYTSSSTLD VVFGGGTKLTVL |

| **139116** | | |
|---|---|---|
| **139116- aa ScFv domain CLL-1-2** | 40 | EVQLVESGGGVVQPGGSLRLSCAASGFTFDDYAMHWVRQAPGKGLEWVSL ISGDGGSTYYADSVKGRFTISRDNSKNTLYLQMNSLRVEDTAVYYCARVF DSYYMDVWGKGTTVTVSSGGGGSGGGGSGSGGSEIVLTQSPLSLPVTPGQ PASISCRSSQSLVYTDGNTYLNWFQQRPGQSPRRLIYKVSNRDSGVPDRF SGSGSDTDFTLKISRVEAEDVGIYYCMQGTHWSFTFGQGTRLEIK |
| **139116- nt ScFv domain CLL-1-2** | 53 | GAAGTGCAATTGGTGGAAAGCGGAGGAGGAGTGGTGCAACCTGGAGGAAG CCTGAGACTGTCATGTGCCGCCTCGGGATTCACTTTCGATGACTACGCAA TGCACTGGGTCCGCCAGGCCCCCGGAAAGGGTCTGGAATGGGTGTCCCTC ATCTCCGGCGATGGGGGTTCCACTTACTATGCGGATTCTGTGAAGGGCCG CTTCACAATCTCCCGGGACAATTCCAAGAACACTCTGTACCTTCAAATGA ACTCCCTGAGGGTGGAGGACACCGCTGTGTACTACTGCGCGAGAGTGTTT GACTCGTACTATATGGACGTCTGGGGAAAGGGCACCACCGTGACCGTGTC CAGCGGTGGCGGTGGATCGGGGGGCGGCGGCTCCGGGAGCGGAGGTTCCG AGATTGTGCTGACTCAGTCGCCGTTGTCACTGCCTGTCACCCCCGGGCAG CCGGCCTCCATTTCATGCCGGTCCAGCCAGTCCCTGGTCTACACCGATGG GAACACTTACCTCAACTGGTTCCAGCAGCGCCCAGGACAGTCCCCGCGGA GGCTGATCTACAAAGTGTCAAACCGGGACTCCGGCGTCCCCGATCGGTTC TCGGGAAGCGGCAGCGACACCGACTTCACGCTGAAGATTTCCCGCGTGGA AGCCGAGGACGTGGGCATCTACTACTGTATGCAGGGCACCCACTGGTCGT TTACCTTCGGACAAGGAACTAGGCTCGAGATCAAG |
| **139116- aa VH of ScFv CLL-1-2** | 66 | EVQLVESGGGVVQPGGSLRLSCAASGFTFDDYAMHWVRQAPGKGLEWVSL ISGDGGSTYYADSVKGRFTISRDNSKNTLYLQMNSLRVEDTAVYYCARVF DSYYMDVWGKGTTVTVSS |
| **139116- aa VL of ScFv CLL-1-2** | 79 | EIVLTQSPLSLPVTPGQPASISCRSSQSLVYTDGNTYLNWFQQRPGQSPR RLIYKVSNRDSGVPDRFSGSGSDTDFTLKISRVEAEDVGIYYCMQGTHWS FTFGQGTRLEIK |

| **139118** | | |
|---|---|---|
| **139118- aa ScFv domain CLL-1-3** | 41 | QVQLQESGPGLVKPSETLSLTCTVSGGSISSSSYYWGWIRQPPGKGLEWI GSIYYSGSTYYNPSLKSRVSISVDTSKNQFSLKLKYVTAADTAVYYCATP GTYYDFLSGYYPFYWGQGTLVTVSSGGGGSGGGGSGGGGSDIVMTQSPSS LSASVGDRVTITCRASQGISSYLAWYQQKPGKAPKLLIYAASTLQSGVPS RFSGSGSGTDFTLTISSLQPEDFATYYCQQLNSYPYTFGQGTKLEIK |
| **139118- nt ScFv domain CLL-1-3** | 54 | CAAGTGCAGCTTCAAGAAAGCGGTCCAGGACTCGTCAAGCCATCAGAAAC TCTTTCCCTCACTTGTACCGTGTCGGGAGGCAGCATCTCCTCGAGCTCCT ACTACTGGGGTTGGATTAGACAGCCCCCGGGAAAGGGGTTGGAGTGGATC GGTTCCATCTACTACTCCGGGTCGACCTACTACAACCCTTCCCTGAAATC TCGGGTGTCCATCTCCGTCGACACCTCCAAGAACCAGTTCAGCCTGAAGC TGAAATATGTGACCGCGGCCGATACTGCCGTGTACTATTGCGCCACCCCG GGAACCTACTACGACTTCCTCTCGGGGTACTACCCGTTTTACTGGGGACA GGGGACTCTCGTGACCGTGTCCTCGGGCGGCGGAGGTTCAGGCGGTGGCG GATCGGGGGGAGGAGGCTCAGACATTGTGATGACCCAGAGCCCGTCCAGC CTGAGCGCCTCCGTGGGCGATAGGGTCACGATTACTTGCCGGGCGTCCCA GGGAATCTCAAGCTACCTGGCCTGGTACCAACAGAAGCCCGGAAAGGCAC CCAAGTTGCTGATCTATGCCGCTAGCACTCTGCAGTCCGGGGTGCCTTCC CGCTTCTCCGGCTCCGGCTCGGGCACCGACTTCACCCTGACCATTTCCTC ACTGCAACCCGAGGACTTCGCCACTTACTACTGCCAGCAGCTGAACTCCT ACCCTTACACATTCGGACAGGGAACCAAGCTGGAAATCAAG |
| **139118- aa VH of ScFv CLL-1-3** | 67 | QVQLQESGPGLVKPSETLSLTCTVSGGSISSSSYYWGWIRQPPGKGLEWI GSIYYSGSTYYNPSLKSRVSISVDTSKNQFSLKLKYVTAADTAVYYCATP GTYYDFLSGYYPFYWGQGTLVTVSS |
| **139118- aa VL of ScFv CLL-1-3** | 80 | DIVMTQSPSSLSASVGDRVTITCRASQGISSYLAWYQQKPGKAPKLLIYA ASTLQSGVPSRFSGSGSGTDFTLTISSLQPEDFATYYCQQLNSYPYTFGQ GTKLEIK |

| **139122** | | |
|---|---|---|
| **139122- aa ScFv domain CLL-1-4** | 42 | QVQLVESGGGLVQPGGSLRLSCAASGFTFSSYWMSWVRQAPGKGLEWVAN INEDGSAKFYVDSVKGRFTISRDNAKNSLYLQMNSLRAEDTAVYFCARDL RSGRYWGQGTLVTVSSGGGGSGGGGSGGGGSEIVLTQSPGTLSLSPGGRA TLSCRASQSISGSFLAWYQQKPGQAPRLLIYGASSRATGIPDRFSGSGSG TDFTLTISRLEPEDFAVYYCQQYGSSPPTFGLGTKLEIK |
| **139122- nt ScFv domain CLL-1-4** | 55 | CAAGTGCAACTCGTGGAATCTGGTGGAGGACTCGTGCAACCCGGAGGATC ATTGCGACTCTCGTGTGCGGCATCCGGCTTTACCTTTTCATCCTACTGGA TGTCCTGGGTCAGACAGGCCCCCGGGAAGGGACTGGAATGGGTCGCGAAC ATCAACGAGGACGGCTCGGCCAAGTTCTACGTGGACTCCGTGAAGGGCCG CTTCACGATCTCACGGGATAACGCCAAGAATTCCCTGTATCTGCAAATGA ACAGCCTGAGGGCCGAGGACACTGCGGTGTACTTCTGCGCACGCGACCTG AGGTCCGGGAGATACTGGGGACAGGGCACCCTCGTGACCGTGTCGAGCGG AGGAGGGGGGTCGGGCGGCGGCGGTTCCGGTGGCGGCGGTAGCGAAATTG TGTTGACCCAGTCCCCTGGAACCCTGAGCCTGTCACCTGGAGGACGCGCC ACCCTGTCCTGCCGGGCCAGCCAGAGCATCTCAGGGTCCTTCCTGGCTTG GTACCAGCAGAAGCCGGGACAGGCTCCGAGACTTCTGATCTACGGCGCCT CCTCGCGGGCGACCGGAATCCCGGATCGGTTCTCCGGCTCGGGAAGCGGA ACTGACTTCACTCTTACCATTTCCCGCCTGGAGCCGGAAGATTTCGCCGT GTACTACTGCCAGCAGTACGGGTCATCCCCTCCAACCTTCGGCCTGGGAA CTAAGCTGGAAATCAAA |
| **139122- aa VH of ScFv CLL-1-4** | 68 | QVQLVESGGGLVQPGGSLRLSCAASGFTFSSYWMSWVRQAPGKGLEWVAN INEDGSAKFYVDSVKGRFTISRDNAKNSLYLQMNSLRAEDTAVYFCARDL RSGRYWGQGTLVTVSS |
| **139122- aa VL of ScFv CLL-1-4** | 81 | EIVLTQSPGTLSLSPGGRATLSCRASQSISGSFLAWYQQKPGQAPRLLIY GASSRATGIPDRFSGSGSGTDFTLTISRLEPEDFAVYYCQQYGSSPPTFG LGTKLEIK |

| **139117** | | |
|---|---|---|
| **139117- aa ScFv domain CLL-1-5** | 43 | EVQLQQSGPGLVRPSETLSLTCTVSGGPVRSGSHYWNWIRQPPGRGLEWI GYIYYSGSTNYNPSLENRVTISIDTSNNHFSLKLSSVTAADTALYFCARG TATFDWNFPFDSWGQGTLVTVSSGGGGSGGGGSGSGGSDIQMTQSPSSLS ASIGDRVTITCRASQSISSYLNWYQQKPGKAPKLLIYAASSLQSGVPSRF SGSGSGTDFTLTISSLQPEDFATYYCQQSYSTPWTFGQGTKLEIK |
| **139117-nt ScFv domain CLL-1-5** | 56 | GAAGTGCAACTCCAACAATCCGGTCCAGGACTCGTCAGACCCTCCGAAAC TCTCTCGCTTACATGCACTGTGTCCGGCGGCCCTGTGCGGTCCGGCTCTC ATTACTGGAACTGGATTCGCCAGCCCCCGGGACGCGGACTGGAGTGGATC GGCTACATCTATTACTCGGGGTCGACTAACTACAACCCGAGCCTGGAAAA TAGAGTGACCATCTCAATCGACACGTCCAACAACCACTTCTCGCTGAAGT TGTCCTCCGTGACTGCCGCCGATACTGCCCTGTACTTCTGTGCTCGCGGA ACCGCCACCTTCGACTGGAACTTCCCTTTTGACTCATGGGGCCAGGGAC CCTTGTGACCGTGTCCAGCGGAGGAGGAGGCTCCGGTGGTGGCGGAGCG GTAGCGGCGGAAGCGACATCCAGATGACCCAGTCACCGTCCTCGCTGTCC GCATCCATTGGGGATCGGGTCACTATTACTTGCCGGGCGTCCCAGTCCAT CTCGTCCTACCTGAACTGGTATCAGCAGAAGCCAGGGAAAGCCCCCAAGC TGCTGATCTACGCGGCCAGCAGCCTGCAGTCAGGAGTGCCTTCAAGGTTT AGCGGCAGCGGATCGGGAACCGACTTCACCCTGACCATTTCCTCCCTCCA ACCCGAGGATTTCGCCACCTACTACTGCCAGCAGTCCTACTCCACCCCGT GGACCTTCGGACAGGGAACCAAGCTGGAGATCAAG |
| **139117- aa VH of ScFv CLL-1-5** | 69 | EVQLQQSGPGLVRPSETLSLTCTVSGGPVRSGSHYWNWIRQPPGRGLEWI GYIYYSGSTNYNPSLENRVTISIDTSNNHFSLKLSSVTAADTALYFCARG TATFDWNFPFDSWGQGTLVTVSS |
| **139117- aa VL of ScFv CLL-1-5** | 82 | DIQMTQSPSSLSASIGDRVTITCRASQSISSYLNWYQQKPGKAPKLLIYA ASSLQSGVPSRFSGSGSGTDFTLTISSLQPEDFATYYCQQSYSTPWTFGQ GTKLEIK |

| **139119** | | |
|---|---|---|
| **139119- aa ScFv domain CLL-1-6** | 44 | QVQLQESGAGLLKPSETLSLTCAVYGGSFSGYYWSWIRQPPGKGLEWVGE INHSGSTNYNPSLKSRVTISVDTSKNQFSLKLSSVTAADTAVYYCARGSG LVVYAIRVGSGWFDYWGQGTLVTVSSGGGGSGGGDSGGGGSDIQMTQSPS SLSASVGDRVTITCRASQSISSYLNWYQQKPGKAPKLLMYAASSLQSGVP SRFSGSGSGTDFTLTISSLQPEDFATYYCQQSYSTPPWTFGQGTKVDIK |
| **139119- nt ScFv domain CLL-1-6** | 57 | CAAGTGCAACTTCAAGAATCAGGCGCAGGACTTCTCAAGCCATCCGAAAC ACTCTCCCTCACTTGCGCGGTGTACGGGGAAGCTTCTCGGATACTACT GGTCCTGGATTAGGCAGCCTCCCGGCAAAGGCCTGGAATGGGTCGGGGAG ATCAACCACTCCGGTTCAACCAACTACAACCCGTCGCTGAAGTCCCGCGT GACCATTTCCGTGGACACCTCTAAGAATCAGTTCAGCCTGAAGCTCTCGT CCGTGACCGCGGCGGACACCGCCGTCTACTACTGCGCTCGGGGATCAGGA CTGGTGGTGTACGCCATCCGCGTGGGCTCGGGCTGGTTCGATTACTGGGG CCAGGGAACCCTGGTCACTGTGTCGTCCGGCGGAGGAGGTTCGGGGGGCG GAGACAGCGGTGGAGGGGGTAGCGACATCCAGATGACCCAGTCCCCGTCC TCGCTGTCCGCCTCCGTGGGAGATAGAGTGACCATCACCTGTCGGGCATC CCAGAGCATTTCCAGCTACCTGAACTGGTATCAGCAGAAGCCCGGAAAGG CCCCTAAGCTGTTGATGTACGCCGCCAGCAGCTTGCAGTCGGGCGTGCCG AGCCGGTTTTCCGGTTCCGGCTCCGGGACTGACTTCACCCTGACTATCTC ATCCCTGCAACCCGAGGACTTCGCCACTTATTACTGCCAGCAGTCCTACT CAACCCCTCCCTGGACGTTCGGACAGGGCACCAAGGTCGATATCAAG |
| **139119- aa VH of ScFv CLL-1-6** | 70 | QVQLQESGAGLLKPSETLSLTCAVYGGSFSGYYWSWIRQPPGKGLEWVGE INHSGSTNYNPSLKSRVTISVDTSKNQFSLKLSSVTAADTAVYYCARGSG LVVYAIRVGSGWFDYWGQGTLVTVSS |
| **139119- aa VL of ScFv CLL-1-6** | 83 | DIQMTQSPSSLSASVGDRVTITCRASQSISSYLNWYQQKPGKAPKLLMYA ASSLQSGVPSRFSGSGSGTDFTLTISSLQPEDFATYYCQQSYSTPPWTFG QGTKVDIK |

| **139120** | | |
|---|---|---|
| **139120- aa ScFv domain CLL-1-7** | 45 | EVQLVESGGGLVKPGGSLRLSCAASGFTFSSYSMNWVRQAPGKGLEWVSS ISSSSSYIYYADSVKGRFTISRDNAKNSLYLQMNSLRAEDTAVYYCARDP SSSGSYYMEDSYYYGMDVWGQGTTVTVSSGGGGSGGGGSGGGGSNFMLTQ PHSVSESPGKTVTISCTGSSGSIASNYVQWYQQRPGSAPTTVIYEDNQRP SGVPDRFSGSIDSSSNSASLTISGLKTEDEADYYCQSYDSSNQVVFGGGT KLTVL |
| **139120- nt ScFv domain CLL-1-7** | 58 | GAAGTGCAATTGGTGGAATCTGGAGGAGGACTTGTGAAACCTGGTGGAAG CCTGAGACTTTCCTGTGCGGCCTCGGGATTCACTTTCTCCTCCTACTCCA TGAACTGGGTCAGACAGGCCCCTGGGAAGGGACTGGAATGGGTGTCATCC ATCTCCTCCTCATCGTCGTACATCTACTACGCCGATAGCGTGAAGGGGCG GTTCACCATTTCCCGGGACAACGCTAAGAACAGCCTCTATCTGCAAATGA ATTCCCTCCGCGCCGAGGACACTGCCGTGTACTACTGCGCGAGGGACCCC TCATCAAGCGGCAGCTACTACATGGAGGACTCGTATTACTACGGAATGGA CGTCTGGGGCCAGGGAACCACTGTGACGGTGTCCTCCGGTGGAGGGGGCT CCGGGGGCGGGGGATCTGGCGGAGGAGGCTCCAACTTCATGCTGACCCAG CCGCACTCCGTGTCCGAAAGCCCCGGAAAGACCGTGACAATTTCCTGCAC CGGGTCCTCCGGCTCGATCGCATCAAACTACGTGCAGTGGTACCAGCAGC GCCCGGGCAGCGCCCCCACCACTGTCATCTACGAGGATAACCAGCGGCCG TCGGGTGTCCCAGACCGGTTTTCCGGTTCGATCGATAGCAGCAGCAACAG CGCCTCCCTGACCATTTCCGGCCTCAAGACCGAGGATGAGGCTGACTACT |
| | | ACTGCCAGTCGTATGACTCCTCGAACCAAGTGGTGTTCGGTGGCGGCACC AAGCTGACTGTGCTG |
| **139120- aa VH of ScFv CLL-1-7** | 71 | EVQLVESGGGLVKPGGSLRLSCAASGFTFSSYSMNWVRQAPGKGLEWVSS ISSSSSYIYYADSVKGRFTISRDNAKNSLYLQMNSLRAEDTAVYYCARDP SSSGSYYMEDSYYYGMDVWGQGTTVTVSS |
| **139120- aa VL of ScFv CLL-1-7** | 84 | NFMLTQPHSVSESPGKTVTISCTGSSGSIASNYVQWYQQRPGSAPTTVIY EDNQRPSGVPDRFSGSIDSSSNSASLTISGLKTEDEADYYCQSYDSSNQV VFGGGTKLTVL |

| **139121** | | |
|---|---|---|
| **139121- aa ScFv domain CLL-1-8** | 46 | QVNLRESGGGLVQPGGSLRLSCAASGFTFSSYEMNWVRQAPGKGLEWVSY ISSSGSTIYYADSVKGRFTISRDNAKNSLYLQMNSLRAEDTAVYYCAREA LGSSWEWGQGTTVTVSSGGGGSGGGGSGGGGSDIQMTQSPSSLSASVGDR VTITCQASQDISNYLNWYQQKPGKAPKLLIYDASNLETGVPSRFSGSGSG TDFTFTISSLQPEDIATYYCQQYDNLPLTFGGGTKLEIK |
| **139121- nt ScFv domain CLL-1-8** | 59 | CAAGTGAACCTGAGAGAAAGCGGCGGAGGACTTGTGCAACCTGGAGGAAG CCTGAGACTGTCATGTGCCGCGTCCGGCTTCACCTTCTCGTCCTACGAGA TGAACTGGGTCCGCCAGGCACCGGGCAAAGGACTGGAATGGGTGTCCTAC ATTTCCTCGTCCGGGTCCACCATCTATTACGCCGACTCCGTGAAGGGACG GTTCACCATCTCCCGGGACAACGCCAAGAACTCCCTCTACCTCCAAATGA ACTCACTGAGGGCAGAGGACACTGCGGTCTACTACTGCGCCCGCGAAGCT TTGGGTAGCTCCTGGGAGTGGGGCCAGGGAACCACTGTGACCGTGTCCTC GGGTGGAGGGGGCTCCGGTGGCGGGGGTTCAGGGGGTGGCGGAAGCGATA TCCAGATGACTCAGTCACCAAGCTCCCTGAGCGCCTCAGTGGGAGATCGG GTCACAATCACGTGCCAGGCGTCCCAGGACATTTCTAACTACCTCAATTG GTACCAGCAGAAGCCGGGGAAGGCCCCCAAGCTTCTGATCTACGATGCCT CCAACCTGGAAACCGGCGTGCCCTCCCGCTTCTCGGGATCGGGCAGCGGC ACTGACTTCACCTTTACCATCTCGTCCCTGCAACCTGAGGACATCGCCAC CTATTACTGCCAGCAGTACGATAACCTCCCGCTGACTTTCGGAGGCGGAA CTAAGCTGGAGATTAAG |
| **139121- aa VH of ScFv CLL-1-8** | 72 | QVNLRESGGGLVQPGGSLRLSCAASGFTFSSYEMNWVRQAPGKGLEWVSY ISSSGSTIYYADSVKGRFTISRDNAKNSLYLQMNSLRAEDTAVYYCAREA LGSSWEWGQGTTVTVSS |
| **139121- aa VL of ScFv CLL-1-8** | 85 | DIQMTQSPSSLSASVGDRVTITCQASQDISNYLNWYQQKPGKAPKLLIYD ASNLETGVPSRFSGSGSGTDFTFTISSLQPEDIATYYCQQYDNLPLTFGG GTKLEIK |

| **146259** | | |
|---|---|---|
| **146259- aa ScFv domain CLL-1-9** | 47 | QVQLVQSGAEVKEPGASVKVSCKAPANTFSDHVMHWVRQAPGQRFEWMGY IHAANGGTHYSQKFQDRVTITRDTSANTVYMDLSSLRSEDTAVYYCARGG YNSDAFDIWGQGTMVTVSSGGGGSGGGGSGGGGSGGGGSDIVMTQSPSSV SASVGDRVTITCRASQDISSWLAWYQQKPGKAPKLLIYAASSLQSGVPSR FNGSGSGTDFTLTISSLQPEDFATYYCQQSYSTPLTFGGGTKVEIK |
| **146259- nt ScFv domain CLL-1-9** | 60 | CAAGTGCAACTCGTCCAGTCCGGTGCAGAAGTCAAGGAACCCGGAGCCTC CGTGAAAGTGTCCTGCAAAGCTCCTGCCAACACTTTCTCGGACCACGTGA TGCACTGGGTGCGCCAGGCGCCGGGCCAGCGCTTCGAATGGATGGGATAC ATTCATGCCGCCAATGGCGGTACCCACTACTCCCAAAAGTTCCAGGATAG AGTCACCATCACCCGGGACACCAGCGCCAACACCGTGTATATGGATCTGT CCAGCCTGAGGTCCGAGGATACCGCCGTGTACTACTGCGCCCGGGGCGGA TACAACTCAGACGCGTTCGACATTTGGGGACAGGGTACTATGGTCACCGT GTCATCCGGGGGCGGTGGCAGCGGGGGCGGAGGCTCTGGCGGAGGCGGAT CAGGGGGAGGAGGGTCCGACATCGTGATGACCCAGTCCCCGTCATCGGTG TCCGCGTCCGTGGGAGACAGAGTGACCATCACGTGTCGCGCCAGCCAGGA CATCTCCTCGTGGTTGGCATGGTACCAGCAGAAGCCTGGAAAGGCCCCGA AGCTGCTCATCTACGCCGCCTCCTCCCTTCAATCGGGAGTGCCCTCGCGG TTCAACGGAAGCGGAAGCGGGACAGATTTTACCCTGACTATTAGCTCGCT |
| | | GCAGCCCGAGGACTTCGCTACTTACTACTGCCAACAGAGCTACTCCACCC CACTGACTTTCGGCGGGGGTACCAAGGTCGAGATCAAG |
| **146259- aa VH of ScFv CLL-1-9** | 73 | QVQLVQSGAEVKEPGASVKVSCKAPANTFSDHVMHWVRQAPGQRFEWMGY IHAANGGTHYSQKFQDRVTITRDTSANTVYMDLSSLRSEDTAVYYCARGG YNSDAFDIWGQGTMVTVSS |
| **146259- aa VL of ScFv CLL-1-9** | 86 | DIVMTQSPSSVSASVGDRVTITCRASQDISSWLAWYQQKPGKAPKLLIYA ASSLQSGVPSRFNGSGSGTDFTLTISSLQPEDFATYYCQQSYSTPLTFGG GTKVEIK |

| **146261** | | |
|---|---|---|
| **146261- aa ScFv domain CLL-1-10** | 48 | QVQLVQSGGGLVQPGGSLRLSCAASGFTFSSYSMNWVRQAPGKGLEWVSY ISSSSSTIYYADSVKGRFTISRDNAKNSLYLQMNSLRAEDTAVYYCARDL SVRAIDAFDIWGQGTMVTVSSGGGGSGGGGSGGGGSGGGGSDIVLTQSPS SLSASVGDRVTITCQASQDISNYLNWYQQKPGKAPKLLIYDASNLETGVP SRFSGSGSGTDFTFTISSLQPEDFATYYCQQAYSTPFTFGPGTKVEIK |
| **146261- nt ScFv domain CLL-1-10** | 61 | CAAGTGCAACTTGTTCAATCCGGTGGAGGTCTTGTGCAGCCCGGAGGATC ACTCAGACTGTCGTGCGCCGCCTCTGGGTTCACTTTCTCCTCATACTCGA TGAACTGGGTGCGCCAGGCGCCGGGAAAGGGCCTGGAATGGGTGTCATAC ATCTCCTCCTCATCCTCCACCATCTACTACGCCGATTCCGTGAAGGGCCG CTTCACTATTTCCCGGGACAACGCGAAAAACTCGCTCTATCTGCAAATGA ACTCCCTGCGCGCCGAGGACACCGCCGTGTACTACTGCGCCCGGGACCTG AGCGTGCGGGCTATTGATGCGTTCGACATCTGGGGACAGGGCACCATGGT CACAGTGTCCAGCGGAGGCGGCGGCAGCGGTGGAGGAGGATCAGGGGGAG GAGGTTCGGGGGGCGGTGGCTCCGATATCGTGCTGACCCAGAGCCCGTCG AGCCTCTCCGCCTCCGTCGGCGACAGAGTGACCATCACGTGTCAGGCATC CCAGGACATTAGCAACTACCTGAATTGGTACCAGCAGAAGCCTGGAAAGG CACCCAAGTTGCTGATCTACGACGCCTCCAACCTGGAAACCGGAGTGCCA TCCAGGTTCTCGGGCAGCGGCTCGGGAACCGACTTCACTTTTACTATCTC CTCCCTGCAACCCGAGGATTTCGCGACCTACTACTGCCAGCAGGCCTACA GCACCCCTTTCACCTTCGGGCCGGGAACTAAGGTCGAAATCAAG |
| **146261- aa VH of ScFv CLL-1-10** | 74 | QVQLVQSGGGLVQPGGSLRLSCAASGFTFSSYSMNWVRQAPGKGLEWVSY ISSSSSTIYYADSVKGRFTISRDNAKNSLYLQMNSLRAEDTAVYYCARDL SVRAIDAFDIWGQGTMVTVSS |
| **146261- aa VL of ScFv CLL-1-10** | 87 | DIVLTQSPSSLSASVGDRVTITCQASQDISNYLNWYQQKPGKAPKLLIYD ASNLETGVPSRFSGSGSGTDFTFTISSLQPEDFATYYCQQAYSTPFTFGP GTKVEIK |

| **146262** | | |
|---|---|---|
| **146262- aa ScFv domain CLL-1-11** | 49 | EVQLVQSGGGVVRSGRSLRLSCAASGFTFNSYGLHWVRQAPGKGLEWVAL IEYDGSNKYYGDSVKGRFTISRDKSKSTLYLQMDNLRAEDTAVYYCAREG NEDLAFDIWGQGTLVTVSSGGGGSGGGGSGGGGSGGGGSEIVLTQSPSSL SASVGDRVTITCQASQFIKKNLNWYQHKPGKAPKLLIYDASSLQTGVPSR FSGNRSGTTFSFTISSLQPEDVATYYCQQHDNLPLTFGGGTKVEIK |
| **146262- nt ScFv domain CLL-1-11** | 62 | GAAGTGCAATTGGTGCAATCAGGAGGAGGAGTGGTCAGATCTGGAAGAAG CCTGAGACTGTCATGCGCGGCTTCGGGCTTTACCTTCAACTCCTACGGCC TCCACTGGGTGCGCCAGGCCCCCGGAAAAGGCCTCGAATGGGTCGCACTG ATTGAGTACGACGGGTCCAACAAGTACTACGGAGATAGCGTGAAGGGCCG CTTCACCATCTCACGGGACAAGTCCAAGTCCACCCTGTATCTGCAAATGG ACAACCTGAGGGCCGAGGATACTGCCGTGTACTACTGCGCCCGCGAAGGA AACGAAGATCTGGCCTTCGATATTTGGGGCCAGGGTACTCTTGTGACCGT GTCGAGCGGAGGCGGAGGCTCCGGTGGAGGAGGATCGGGGGGTGGTGGTT CCGGCGGCGGGGGGAGCGAAATCGTGCTGACCCAGTCGCCTTCCTCCCTC TCCGCTTCCGTGGGGGACCGGGTCACTATTACGTGTCAGGCGTCCCAATT CATCAAGAAGAATCTGAACTGGTACCAGCACAAGCCGGGAAAGGCCCCCA AACTGCTCATCTACGACGCCAGCTCGCTGCAGACTGGCGTGCCTTCCCGG TTTTCCGGGAACCGGTCGGGAACCACCTTCTCATTCACCATCAGCAGCCT |
| | | CCAGCCGGAGGACGTGGCGACCTACTACTGCCAGCAGCATGACAACCTTC CACTGACTTTCGGCGGGGGCACCAAGGTCGAGATTAAG |
| **146262- aa VH of ScFv CLL-1-11** | 75 | EVQLVQSGGGVVRSGRSLRLSCAASGFTFNSYGLHWVRQAPGKGLEWVAL IEYDGSNKYYGDSVKGRFTISRDKSKSTLYLQMDNLRAEDTAVYYCAREG NEDLAFDIWGQGTLVTVSS |
| **146262- aa VL of ScFv CLL-1-11** | 88 | EIVLTQSPSSLSASVGDRVTITCQASQFIKKNLNWYQHKPGKAPKLLIYD ASSLQTGVPSRFSGNRSGTTFSFTISSLQPEDVATYYCQQHDNLPLTFGG GTKVEIK |

| **146263** | | |
|---|---|---|
| **146263- aa ScFv domain CLL-1-12** | 50 | QVQLVESGGGLVQPGGSLRLSCAASGFNVSSNYMTWVRQAPGKGLEWVSV IYSGGATYYGDSVKGRFTVSRDNSKNTVYLQMNRLTAEDTAVYYCARDRL YCGNNCYLYYYYGMDVWGQGTLVTVSSGGGGSGGGGSGGGGSGGGGSDIQ VTQSPSSLSASVGDRVTITCRASQSISSYLNWYQQKPGKAPKLLIYAASS LQSGVPSRFSGSGSGTDFTLTISSLQPEDFATYYCQQSYSTPPLTFGQGT KVEIK |
| **146263- nt ScFv domain CLL-1-12** | 63 | CAAGTGCAACTCGTGGAATCAGGCGGAGGACTCGTGCAACCCGGAGGTTC CCTTAGACTGTCATGTGCCGCTTCCGGGTTCAATGTGTCCAGCAACTACA TGACCTGGGTCAGACAGGCGCCGGGAAAGGACTTGAATGGGTGTCCGTG ATCTACTCCGGTGGAGCAACATACTACGGAGACTCCGTGAAAGGCCGCTT TACCGTGTCCCGCGATAACTCGAAGAACACCGTGTACTTGCAGATGAACA GGCTGACTGCCGAGGACACCGCCGTGTATTATTGCGCCCGGGACAGGCTG TACTGTGGAAACAACTGCTACCTGTACTACTACTACGGGATGGACGTGTG GGGACAGGGCACTCTCGTCACTGTGTCATCCGGGGGGGGCGGTAGCGGTG GCGGAGGGTCCGGCGGAGGAGGCTCAGGGGGAGGCGGAAGCGATATCCAG GTCACCCAGTCTCCCTCCTCGCTGTCCGCCTCCGTGGGCGACCGCGTCAC CATTACTTGCCGGGCGTCGCAGTCGATCAGCTCCTACCTGAACTGGTACC AGCAGAAGCCTGGAAAGGCCCCGAAGCTGCTGATCTACGCGGCCTCGTCC CTGCAAAGCGGCGTCCCGTCGCGGTTCAGCGGTTCCGGTTCGGAACCGA CTTCACCCTGACTATTTCCTCCCTGCAACCCGAGGATTTCGCCACTTACT ACTGCCAGCAGTCCTACTCCACCCCACCTCTGACCTTCGGCCAAGGAACC AAGGTCGAAATCAAG |
| **146263- aa VH of ScFv CLL-1-12** | 76 | QVQLVESGGGLVQPGGSLRLSCAASGFNVSSNYMTWVRQAPGKGLEWVSV IYSGGATYYGDSVKGRFTVSRDNSKNTVYLQMNRLTAEDTAVYYCARDRL YCGNNCYLYYYYGMDVWGQGTLVTVSS |
| **146263- aa VL of ScFv CLL-1-12** | 89 | DIQVTQSPSSLSASVGDRVTITCRASQSISSYLNWYQQKPGKAPKLLIYA ASSLQSGVPSRFSGSGSGTDFTLTISSLQPEDFATYYCQQSYSTPPLTFG QGTKVEIK |

| **146264** | | |
|---|---|---|
| **146264- aa ScFv domain CLL-1-13** | 51 | QVQLVQSGAEVKKSGASVKVSCKASGYPFTGYYIQWVRQAPGQGLEWMGW IDPNSGNTGYAQKFQGRVTMTRNTSISTAYMELSSLRSEDTAVYYCASDS YGYYYGMDVWGQGTLVTVSSGGGGSGGGGSGGGGSGGGGSDIQMTQSPSS LSASVGDRVTFTCRASQGISSALAWYQQKPGKPPKLLIYDASSLESGVPS RFSGSGSGTDFTLTISSLQPEDFATYYCQQFNNYPLTFGGGTKVEIK |
| **146264- nt ScFv domain CLL-1-13** | 64 | CAAGTGCAACTCGTCCAGTCCGGTGCAGAAGTGAAAAAGAGCGGAGCCTC AGTGAAAGTGTCCTGCAAGGCCTCCGGTTACCCCTTCACTGGATACTACA TTCAGTGGGTCCGCCAAGCCCCGGGACAGGGTCTGGAGTGGATGGGGTGG ATTGACCCTAACTCGGGAAATACGGGATACGCGCAGAAGTTCCAGGGCCG CGTGACCATGACCAGGAACACCTCGATCAGCACCGCCTACATGGAACTGT CCTCCCTGCGGTCGGAGGATACTGCCGTGTACTACTGCGCCTCCGATTCC TATGGGTACTACTACGGAATGGACGTCTGGGGACAGGGCACCCTCGTGAC CGTGTCCTCGGGAGGCGGAGGGAGCGGCGGGGGTGGATCGGGAGGAGGCG GCTCCGGCGGCGGCGGTAGCGACATCCAGATGACCCAGTCACCATCAAGC CTTAGCGCCTCCGTGGGCGACAGAGTGACATTCACTTGTCGGGCGTCCCA GGGAATCTCCTCCGCTCTGGCTTGGTATCAGCAGAAGCCTGGAAGCCTC |
| | | CGAAGCTGTTGATCTACGACGCGAGCAGCCTGGAATCAGGGGTGCCCTCC CGGTTTTCCGGGTCCGGTTCTGGCACCGATTTCACCCTGACCATTTCGTC CCTCCAACCCGAGGACTTCGCCACTTACTACTGCCAGCAGTTCAACAACT ACCCGCTGACCTTCGGAGGAGGCACTAAGGTCGAGATCAAG |
| **146264- aa VH of ScFv CLL-1-13** | 77 | QVQLVQSGAEVKKSGASVKVSCKASGYPFTGYYIQWVRQAPGQGLEWMGW IDPNSGNTGYAQKFQGRVTMTRNTSISTAYMELSSLRSEDTAVYYCASDS YGYYYGMDVWGQGTLVTVSS |
| **146264- aa VL of ScFv CLL-1-13** | 90 | DIQMTQSPSSLSASVGDRVTFTCRASQGISSALAWYQQKPGKPPKLLIYD ASSLESGVPSRFSGSGSGTDFTLTISSLQPEDFATYYCQQFNNYPLTFGG GTKVEIK |

| **181268** | | |
|---|---|---|
| **181268- aa VH of ScFv** | 195 | EVQLVESGGGLVQPGGSLRLSCAASGFTFSSYEMNWVRQAPGKGLEWVSY ISSSGSTIYYADSVKGRFTISRDNAKNSLYLQMNSLRAEDTAVYYCARDP YSSSWHDAFDIWGQGTMVTVSS |
| **181268- aa VL of ScFv** | 196 | EIVLTQSPGTLSLSPGERATLSCRASQSVSSSYLAWYQQKPGQAPRLLIY GASSRATGIPDRFSGSGSGTDFTLTISRLEPEDFAVYYCQQYGSSPLTFG GGTKVDIK |

In embodiments, additional exemplary CLL-1 multispecific molecule, e.g., bispecific molecule, e.g., bispecific antibody or bispecific antibody-like molecule, constructs comprise an antigen binding domain comprising one or more, e.g., one, two, or three, CDRs of the heavy chain variable domain and/or one or more, e.g., one, two, or three, CDRs of the light chain variable domain, or the VH and/or VL, or the scFv sequence, of the scFv sequence the anti-CLL-1 (CLEC12A) antibody disclosed in PCT Publication WO2014/051433, the entire contents of which are hereby incorporated by reference.

In embodiments, the VL of the CLL-1 binding domain precedes the VH ("L2H"). In other embodiments, the VH of the CLL-1 binding domain precedes the VL ("H2L").

The antigen binding domain of the multispecific molecule, e.g., bispecific molecule, e.g., bispecific antibody or bispecific antibody-like molecule, construct can include a Gly/Ser linker having one or more of the following sequences: GGGGS (SEQ ID NO:25); encompassing 1-6 "Gly Gly Gly Gly Ser" repeating units, e.g., GGGGSGGGGS GGGGSGGGGS GGGGSGGGGS (SEQ ID NO:26); GGGGSGGGGS GGGGSGGGGS (SEQ ID NO:27); GGGGSGGGGS GGGGS (SEQ ID NO:28); GGGS (SEQ ID NO:29); or encompassing 1-10 "Gly Gly Gly Ser" repeating units, e.g., GGGSGGGSGG GSGGGSGGGS GGGSGGGSGG GSGGGSGGGS (SEQ ID NO:38). Alternatively, the antigen binding domain of the multispecific molecule, e.g., bispecific molecule, e.g., bispecific antibody or bispecific antibody-like molecule, construct can include, for example, a linker including the sequence GSTSGSGKPGSGEGSTKG (SEQ ID NO: 1108)

In embodiments, the nucleic acid construct encoding a multispecific molecule, e.g., bispecific molecule, e.g., bispecific antibody or bispecific antibody-like molecule, includes a poly A sequence, e.g., a sequence encompassing 50-5000 or 100-5000 adenines (e.g., SEQ ID NO:30, SEQ ID NO:33, SEQ ID NO:34 or SEQ ID NO:35), or a sequence encompassing 50-5000 thymines (e.g., SEQ ID NO:31, SEQ ID NO:32).

The human CDR sequences of the scFv domains are shown in Tables 3, 5, and 7 for the heavy chain variable domains and in Tables 4, 6, and 8 for the light chain variable domains. "ID" stands for the respective SEQ ID NO for each CDR.

**Table 3. Heavy Chain Variable Domain CDRs according to a combination of the Kabat and Chothia numbering scheme.**

| **Candidate** | **HCDR1** | **ID** | **HCDR2** | **ID** | **HCDR3** | **ID** |
|---|---|---|---|---|---|---|
| CLL-1-9 | ANTFSDHVMH | 125 | YIHAANGGTHYSQK FQD | 138 | GGYNSDAFDI | 151 |
| CLL-1-6 | GGSFSGYYWS | 122 | EINHSGSTNYNPSLK S | 135 | GSGLVVYAIRVGSGWFDY | 148 |
| CLL-1-10 | GFTFSSYSMN | 126 | YISSSSSTIYYADSVK G | 139 | DLSVRAIDAFDI | 152 |
| CLL-1-11 | GFTFNSYGLH | 127 | LIEYDGSNKYYGDSV KG | 140 | EGNEDLAFDI | 153 |
| CLL-1-12 | GFNVSSNYMT | 128 | VIYSGGATYYGDSV KG | 141 | DRLYCGNNCYLYYYYGM DV | 154 |
| CLL-1-1 | GGTFSSYAIS | 117 | GIIPIFGTANYAQKFQ | 130 | DLEMATIMGGY | 143 |
| CLL-1-2 | GFTFDDYAMH | 118 | LISGDGGSTYYADSV KG | 131 | VFDSYYMDV | 144 |
| CLL-1-3 | GGSISSSSYYWG | 119 | SIYYSGSTYYNPSLK S | 132 | PGTYYDFLSGYYPFY | 145 |
| CLL-1-4 | GFTFSSYWMS | 120 | NINEDGSAKFYVDSV KG | 133 | DLRSGRY | 146 |
| CLL-1-5 | GGPVRSGSHYW N | 121 | YIYYSGSTNYNPSLE N | 134 | GTATFDWNFPFDS | 147 |
| CLL-1-7 | GFTFSSYSMN | 123 | SISSSSSYIYYADSVK G | 136 | DPSSSGSYYMEDSYYYG MDV | 149 |
| CLL-1-8 | GFTFSSYEMN | 124 | YISSSGSTIYYADSVK G | 137 | EALGSSWE | 150 |
| CLL-1-13 | GYPFTGYYIQ | 129 | WIDPNSGNTGYAQK FQG | 142 | DSYGYYYGMDV | 155 |
| 181268 | GFTFSSYEMN | 199 | YISSSGSTIYYADSVK G | 200 | DPYSSSWHDAFDI | 201 |

**Table 4. Light Chain Variable Domain CDRs according to a combination of the Kabat and Chothia numbering scheme.**

| **Candidate** | **LCDR1** | **ID** | **LCDR2** | **ID** | **LCDR3** | **ID** |
|---|---|---|---|---|---|---|
| CLL-1-9 | RASQDISSWLA | 164 | AASSLQS | 177 | QQSYSTPLT | 190 |
| CLL-1-6 | RASQSISSYLN | 161 | AASSLQS | 174 | QQSYSTPPWT | 187 |
| CLL-1-10 | QASQDISNYLN | 165 | DASNLET | 178 | QQAYSTPFT | 191 |
| CLL-1-11 | QASQFIKKNLN | 166 | DASSLQT | 179 | QQHDNLPLT | 192 |
| CLL-1-12 | RASQSISSYLN | 167 | AASSLQS | 180 | QQSYSTPPLT | 193 |
| CLL-1-1 | TGTSSDVGGYNYVS | 156 | DVSNRPS | 169 | SSYTSSSTLDVV | 182 |
| CLL-1-2 | RSSQSLVYTDGNTYLN | 157 | KVSNRDS | 170 | MQGTHWSFT | 183 |
| CLL-1-3 | RASQGISSYLA | 158 | AASTLQS | 171 | QQLNSYPYT | 184 |
| CLL-1-4 | RASQSISGSFLA | 159 | GASSRAT | 172 | QQYGSSPPT | 185 |
| CLL-1-5 | RASQSISSYLN | 160 | AASSLQS | 173 | QQSYSTPWT | 186 |
| CLL-1-7 | TGSSGSIASNYVQ | 162 | EDNQRPS | 175 | QSYDSSNQVV | 188 |
| CLL-1-8 | QASQDISNYLN | 163 | DASNLET | 176 | QQYDNLPLT | 189 |
| CLL-1-13 | RASQGISSALA | 168 | DASSLES | 181 | QQFNNYPLT | 194 |
| 181268 | RASQSVSSSYLA | 202 | GASSRAT | 203 | QQYGSSPLT | 204 |

**Table 5. Heavy Chain Variable Domain CDRs according to the Kabat numbering scheme (Kabat et al. (1991), "Sequences of Proteins of Immunological Interest," 5th Ed. Public Health Service, National Institutes of Health, Bethesda, MD)**

| **Candidate** | **HCDR1** | **ID** | **HCDR2** | **ID** | **HCDR3** | **ID** |
|---|---|---|---|---|---|---|
| 146259-CLL-1-9 | DHVMH | 322 | YIHAANGGTHYSQKF OD | 336 | GGYNSDAFDI | 350 |
| 139119-CLL-1-6 | GYYWS | 319 | EINHSGSTNYNPSLKS | 333 | GSGLVVYAIRVGSG WFDY | 347 |
| 146261-CLL-1-10 | SYSMN | 323 | YISSSSSTIYYADSVK G | 337 | DLSVRAIDAFDI | 351 |
| 146262-CLL-1-11 | SYGLH | 324 | LIEYDGSNKYYGDSV KG | 338 | EGNEDLAFDI | 352 |
| 146263-CLL-1-12 | SNYMT | 325 | VIYSGGATYYGDSVK G | 339 | DRLYCGNNCYLYY YYGMDV | 353 |
| 139115-CLL-1-1 | SYAIS | 314 | GIIPIFGTANYAQKFQ G | 328 | DLEMATIMGGY | 342 |
| 139116-CLL-1-2 | DYAMH | 315 | LISGDGGSTYYADSV KG | 329 | VFDSYYMDV | 343 |
| 139118-CLL-1-3 | SSSYYWG | 316 | SIYYSGSTYYNPSLKS | 330 | PGTYYDFLSGYYPF Y | 344 |
| 139122-CLL-1-4 | SYWMS | 317 | NINEDGSAKFYVDSV KG | 331 | DLRSGRY | 345 |
| 139117-CLL-1-5 | SGSHYWN | 318 | YIYYSGSTNYNPSLEN | 332 | GTATFDWNFPFDS | 346 |
| 139120-CLL-1-7 | SYSMN | 320 | SISSSSSYIYYADSVK G | 334 | DPSSSGSYYMEDSY YYGMDV | 348 |
| 139121-CLL-1-8 | SYEMN | 321 | YISSSGSTIYYADSVK G | 335 | EALGSSWE | 349 |
| 146264-CLL-1-13 | GYYIQ | 326 | WIDPNSGNTGYAQKF QG | 340 | DSYGYYYGMDV | 354 |
| 181268 | SYEMN | 327 | YISSSGSTIYYADSVK G | 341 | DPYSSSWHDAFDI | 355 |

**Table 6. Light Chain Variable Domain CDRs according to the Kabat numbering scheme (Kabat et al. (1991), "Sequences of Proteins of Immunological Interest," 5th Ed. Public Health Service, National Institutes of Health, Bethesda, MD)**

| **Candidate** | **LCDR1** | **ID** | **LCDR2** | **ID** | **LCDR3** | **ID** |
|---|---|---|---|---|---|---|
| 146259-CLL-1-9 | RASQDISSWLA | 364 | AASSLQS | 378 | QQSYSTPLT | 392 |
| 139119-CLL-1-6 | RASQSISSYLN | 361 | AASSLQS | 375 | QQSYSTPPWT | 389 |
| 146261-CLL-1-10 | QASQDISNYLN | 365 | DASNLET | 379 | QQAYSTPFT | 393 |
| 146262-CLL-1-11 | QASQFIKKNLN | 366 | DASSLQT | 380 | QQHDNLPLT | 394 |
| 146263-CLL-1-12 | RASQSISSYLN | 367 | AASSLQS | 381 | QQSYSTPPLT | 395 |
| 139115-CLL-1-1 | TGTSSDVGGYNYVS | 356 | DVSNRPS | 370 | SSYTSSSTLDVV | 384 |
| 139116-CLL-1-2 | RSSQSLVYTDGNTYLN | 357 | KVSNRDS | 371 | MQGTHWSFT | 385 |
| 139118-CLL-1-3 | RASQGISSYLA | 358 | AASTLQS | 372 | QQLNSYPYT | 386 |
| 139122-CLL-1-4 | RASQSISGSFLA | 359 | GASSRAT | 373 | QQYGSSPPT | 387 |
| 139117-CLL-1-5 | RASQSISSYLN | 360 | AASSLQS | 374 | QQSYSTPWT | 388 |
| 139120-CLL-1-7 | TGSSGSIASNYVQ | 362 | EDNQRPS | 376 | QSYDSSNQVV | 390 |
| 139121-CLL-1-8 | QASQDISNYLN | 363 | DASNLET | 377 | QQYDNLPLT | 391 |
| 146264-CLL-1-13 | RASQGISSALA | 368 | DASSLES | 382 | QQFNNYPLT | 396 |
| 181268 | RASQSVSSSYLA | 369 | GASSRAT | 383 | QQYGSSPLT | 397 |

**Table 7. Heavy Chain Variable Domain CDRs according to the Chothia numbering scheme (Al-Lazikani et al., (1997) JMB 273,927-948)**

| **Candidate** | **HCDR1** | **ID** | **HCDR2** | **ID** | **HCDR3** | **ID** |
|---|---|---|---|---|---|---|
| 146259-CLL-1-9 | ANTFSDH | 406 | HAANGG | 420 | GGYNSDAFDI | 434 |
| 139119-CLL-1-6 | GGSFSGY | 403 | NHSGS | 417 | GSGLVVYAIRVGSGWFDY | 431 |
| 146261-CLL-1-10 | GFTFSSY | 407 | SSSSST | 421 | DLSVRAIDAFDI | 435 |
| 146262-CLL-1-11 | GFTFNSY | 408 | EYDGSN | 422 | EGNEDLAFDI | 436 |
| 146263-CLL-1-12 | GFNVSSN | 409 | YSGGA | 423 | DRLYCGNNCYLYYYYGMDV | 437 |
| 139115-CLL-1-1 | GGTFSSY | 398 | IPIFGT | 412 | DLEMATIMGGY | 426 |
| 139116-CLL-1-2 | GFTFDDY | 399 | SGDGGS | 413 | VFDSYYMDV | 427 |
| 139118-CLL-1-3 | GGSISSSSY | 400 | YYSGS | 414 | PGTYYDFLSGYYPFY | 428 |
| 139122-CLL-1-4 | GFTFSSY | 401 | NEDGSA | 415 | DLRSGRY | 429 |
| 139117-CLL-1-5 | GGPVRSGSH | 402 | YYSGS | 416 | GTATFDWNFPFDS | 430 |
| 139120-CLL-1-7 | GFTFSSY | 404 | SSSSSY | 418 | DPSSSGSYYMEDSYYYGMDV | 432 |
| 139121-CLL-1-8 | GFTFSSY | 405 | SSSGST | 419 | EALGSSWE | 433 |
| 146264-CLL-1-13 | GYPFTGY | 410 | DPNSGN | 424 | DSYGYYYGMDV | 438 |
| 181268 | GFTFSSY | 411 | SSSGST | 425 | DPYSSSWHDAFDI | 439 |

**Table 8. Light Chain Variable Domain CDRs according to the Chothia numbering scheme (Al-Lazikani et al., (1997) JMB 273,927-948)**

| **Candidate** | **LCDR1** | **ID** | **LCDR2** | **ID** | **LCDR3** | **ID** |
|---|---|---|---|---|---|---|
| 146259-CLL-1-9 | SQDISSW | 448 | AAS | 462 | SYSTPL | 476 |
| 139119-CLL-1-6 | SQSISSY | 445 | AAS | 459 | SYSTPPW | 473 |
| 146261-CLL-1-10 | SQDISNY | 449 | DAS | 463 | AYSTPF | 477 |
| 146262-CLL-1-11 | SQFIKKN | 450 | DAS | 464 | HDNLPL | 478 |
| 146263-CLL-1-12 | SQSISSY | 451 | AAS | 465 | SYSTPPL | 479 |
| 139115-CLL-1-1 | TSSDVGGYNY | 440 | DVS | 454 | YTSSSTLDV | 468 |
| 139116-CLL-1-2 | SQSLVYTDGNTY | 441 | KVS | 455 | GTHWSF | 469 |
| 139118-CLL-1-3 | SQGISSY | 442 | AAS | 456 | LNSYPY | 470 |
| 139122-CLL-1-4 | SQSISGSF | 443 | GAS | 457 | YGSSPP | 471 |
| 139117-CLL-1-5 | SQSISSY | 444 | AAS | 458 | SYSTPW | 472 |
| 139120-CLL-1-7 | SSGSIASNY | 446 | EDN | 460 | YDSSNQV | 474 |
| 139121-CLL-1-8 | SQDISNY | 447 | DAS | 461 | YDNLPL | 475 |
| 146264-CLL-1-13 | SQGISSA | 452 | DAS | 466 | FNNYPL | 480 |
| 181268 | SQSVSSSY | 453 | GAS | 467 | YGSSPL | 481 |

In certain embodiments, the multispecific molecule, e.g., bispecific molecule, e.g., bispecific antibody or bispecific antibody-like molecule, described herein (*e.g.,* the multispecific molecule, e.g., bispecific molecule, e.g., bispecific antibody or bispecific antibody-like molecule, nucleic acid or a multispecific molecule, e.g., bispecific molecule, e.g., bispecific antibody or bispecific antibody-like molecule, polypeptide) or a CLL-1 binding domain includes a CLL-1 binding domain that includes:
(1) one, two, or three light chain (LC) CDRs chosen from one of the following:
   (i) a LC CDR1 of SEQ ID NO: 156, LC CDR2 of SEQ ID NO: 169 and LC CDR3 of SEQ ID NO: 182 of CLL-1-1;
   (ii) a LC CDR1 of SEQ ID NO: 157, LC CDR2 of SEQ ID NO: 170 and LC CDR3 of SEQ ID NO: 183 of CLL-1-2;
   (iii) a LC CDR1 of SEQ ID NO: 158, LC CDR2 of SEQ ID NO: 171 and LC CDR3 of SEQ ID NO: 184 of CLL-1-3;
   (iv) a LC CDR1 of SEQ ID NO: 159, LC CDR2 of SEQ ID NO: 172 and LC CDR3 of SEQ ID NO: 185 of CLL-1-4;
   (v) a LC CDR1 of SEQ ID NO: 160, LC CDR2 of SEQ ID NO: 173 and LC CDR3 of SEQ ID NO: 186 of CLL-1-5;
   (vi) a LC CDR1 of SEQ ID NO: 161, LC CDR2 of SEQ ID NO: 174 and LC CDR3 of SEQ ID NO: 187 of CLL-1-6;
   (vii) a LC CDR1 of SEQ ID NO: 162, LC CDR2 of SEQ ID NO: 175 and LC CDR3 of SEQ ID NO: 188 of CLL-1-7;
   (viii) a LC CDR1 of SEQ ID NO: 163, LC CDR2 of SEQ ID NO: 176 and LC CDR3 of SEQ ID NO: 189 of CLL-1-8; or
   (ix) a LC CDR1 of SEQ ID NO: 164, LC CDR2 of SEQ ID NO: 177 and LC CDR3 of SEQ ID NO: 190 of CLL-1-9;
   (x) a LC CDR1 of SEQ ID NO: 165, LC CDR2 of SEQ ID NO: 178 and LC CDR3 of SEQ ID NO: 191 of CLL-1-10;
   (xi) a LC CDR1 of SEQ ID NO: 166, LC CDR2 of SEQ ID NO: 179 and LC CDR3 of SEQ ID NO: 192 of CLL-1-11;
   (xii) a LC CDR1 of SEQ ID NO: 167, LC CDR2 of SEQ ID NO: 180 and LC CDR3 of SEQ ID NO: 193 of CLL-1-12;
   (xiii) a LC CDR1 of SEQ ID NO: 168, LC CDR2 of SEQ ID NO: 181 and LC CDR3 of SEQ ID NO: 194 of CLL-1-13;
   (xiv) a LC CDR1 of SEQ ID NO: 202, LC CDR2 of SEQ ID NO: 203 and LC CDR3 of SEQ ID NO: 204 of 181286; and/or
(2) one, two, or three heavy chain (HC) CDRs from one of the following:
   (i) a HC CDR1 of SEQ ID NO: 117, HC CDR2 of SEQ ID NO: 130 and HC CDR3 of SEQ ID NO: 143 of CLL-1-1;
   (ii) a HC CDR1 of SEQ ID NO: 118, HC CDR2 of SEQ ID NO: 131 and HC CDR3 of SEQ ID NO: 144 of CLL-1-2;
   (iii) a HC CDR1 of SEQ ID NO: 119, HC CDR2 of SEQ ID NO: 132 and HC CDR3 of SEQ ID NO: 145 of CLL-1-3;
   (iv) a HC CDR1 of SEQ ID NO: 120, HC CDR2 of SEQ ID NO: 133 and HC CDR3 of SEQ ID NO: 146 of CLL-1-4;
   (v) a HC CDR1 of SEQ ID NO: 121, HC CDR2 of SEQ ID NO: 134 and HC CDR3 of SEQ ID NO: 147 of CLL-1-5;
   (vi) a HC CDR1 of SEQ ID NO: 122, HC CDR2 of SEQ ID NO: 135 and HC CDR3 of SEQ ID NO: 148 of CLL-1-6;
   (vii) a HC CDR1 of SEQ ID NO: 123, HC CDR2 of SEQ ID NO: 136 and HC CDR3 of SEQ ID NO: 149 of CLL-1-7;
   (viii) a HC CDR1 of SEQ ID NO: 124, HC CDR2 of SEQ ID NO: 137 and HC CDR3 of SEQ ID NO: 150 of CLL-1-8; or
   (ix) a HC CDR1 of SEQ ID NO: 125, HC CDR2 of SEQ ID NO: 138 and HC CDR3 of SEQ ID NO: 151 of CLL-1-9;
   (x) a HC CDR1 of SEQ ID NO: 126, HC CDR2 of SEQ ID NO: 139 and HC CDR3 of SEQ ID NO: 152 of CLL-1-10;
   (xi) a HC CDR1 of SEQ ID NO: 127, HC CDR2 of SEQ ID NO: 140 and HC CDR3 of SEQ ID NO: 153 of CLL-1-11;
   (xii) a HC CDR1 of SEQ ID NO: 128, HC CDR2 of SEQ ID NO: 141 and HC CDR3 of SEQ ID NO: 154 of CLL-1-12;
   (xiii) a HC CDR1 of SEQ ID NO: 129, HC CDR2 of SEQ ID NO: 142 and HC CDR3 of SEQ ID NO: 155 of CLL-1-13;
   (xiv) a HC CDR1 of SEQ ID NO: 199, HC CDR2 of SEQ ID NO: 200 and HC CDR3 of SEQ ID NO: 201 of 181286.

In certain embodiments, the multispecific molecule, e.g., bispecific molecule, e.g., bispecific antibody or bispecific antibody-like molecule, described herein (*e.g.,* the multispecific molecule, e.g., bispecific molecule, e.g., bispecific antibody or bispecific antibody-like molecule, nucleic acid or a multispecific molecule, e.g., bispecific molecule, e.g., bispecific antibody or bispecific antibody-like molecule, polypeptide) or a CLL-1 binding domain includes a CLL-1 binding domain that includes:
(1) one, two, or three light chain (LC) CDRs chosen from one of the following:
   (i) a LC CDR1 of SEQ ID NO: 356, LC CDR2 of SEQ ID NO: 370 and LC CDR3 of SEQ ID NO: 384 of CLL-1-1;
   (ii) a LC CDR1 of SEQ ID NO: 357, LC CDR2 of SEQ ID NO: 371 and LC CDR3 of SEQ ID NO: 385 of CLL-1-2;
   (iii) a LC CDR1 of SEQ ID NO: 358, LC CDR2 of SEQ ID NO: 372 and LC CDR3 of SEQ ID NO: 386 of CLL-1-3;
   (iv) a LC CDR1 of SEQ ID NO: 359, LC CDR2 of SEQ ID NO: 373 and LC CDR3 of SEQ ID NO: 387 of CLL-1-4;
   (v) a LC CDR1 of SEQ ID NO: 360, LC CDR2 of SEQ ID NO: 374 and LC CDR3 of SEQ ID NO: 388 of CLL-1-5;
   (vi) a LC CDR1 of SEQ ID NO: 361, LC CDR2 of SEQ ID NO: 375 and LC CDR3 of SEQ ID NO: 389 of CLL-1-6;
   (vii) a LC CDR1 of SEQ ID NO: 362, LC CDR2 of SEQ ID NO: 376 and LC CDR3 of SEQ ID NO: 390 of CLL-1-7;
   (viii) a LC CDR1 of SEQ ID NO: 363, LC CDR2 of SEQ ID NO: 377 and LC CDR3 of SEQ ID NO: 391 of CLL-1-8; or
   (ix) a LC CDR1 of SEQ ID NO: 364, LC CDR2 of SEQ ID NO: 378 and LC CDR3 of SEQ ID NO: 392 of CLL-1-9;
   (x) a LC CDR1 of SEQ ID NO: 365, LC CDR2 of SEQ ID NO: 379 and LC CDR3 of SEQ ID NO: 393 of CLL-1-10;
   (xi) a LC CDR1 of SEQ ID NO: 366, LC CDR2 of SEQ ID NO: 380 and LC CDR3 of SEQ ID NO: 394 of CLL-1-11;
   (xii) a LC CDR1 of SEQ ID NO: 367, LC CDR2 of SEQ ID NO: 381 and LC CDR3 of SEQ ID NO: 395 of CLL-1-12;
   (xiii) a LC CDR1 of SEQ ID NO: 368, LC CDR2 of SEQ ID NO: 382 and LC CDR3 of SEQ ID NO: 396 of CLL-1-13;
   (xiv) a LC CDR1 of SEQ ID NO: 369, LC CDR2 of SEQ ID NO: 383 and LC CDR3 of SEQ ID NO: 397 of 181286; and/or
(2) one, two, or three heavy chain (HC) CDRs from one of the following:
   (i) a HC CDR1 of SEQ ID NO: 314, HC CDR2 of SEQ ID NO: 328 and HC CDR3 of SEQ ID NO: 342 of CLL-1-1;
   (ii) a HC CDR1 of SEQ ID NO: 315, HC CDR2 of SEQ ID NO: 329 and HC CDR3 of SEQ ID NO: 343 of CLL-1-2;
   (iii) a HC CDR1 of SEQ ID NO: 316, HC CDR2 of SEQ ID NO: 330 and HC CDR3 of SEQ ID NO: 344 of CLL-1-3;
   (iv) a HC CDR1 of SEQ ID NO: 317, HC CDR2 of SEQ ID NO: 331 and HC CDR3 of SEQ ID NO: 345 of CLL-1-4;
   (v) a HC CDR1 of SEQ ID NO: 318, HC CDR2 of SEQ ID NO: 332 and HC CDR3 of SEQ ID NO: 346 of CLL-1-5;
   (vi) a HC CDR1 of SEQ ID NO: 319, HC CDR2 of SEQ ID NO: 333 and HC CDR3 of SEQ ID NO: 347 of CLL-1-6;
   (vii) a HC CDR1 of SEQ ID NO: 320, HC CDR2 of SEQ ID NO: 334 and HC CDR3 of SEQ ID NO: 348 of CLL-1-7;
   (viii) a HC CDR1 of SEQ ID NO: 321, HC CDR2 of SEQ ID NO: 335 and HC CDR3 of SEQ ID NO: 349 of CLL-1-8; or
   (ix) a HC CDR1 of SEQ ID NO: 322, HC CDR2 of SEQ ID NO: 336 and HC CDR3 of SEQ ID NO: 350 of CLL-1-9;
   (x) a HC CDR1 of SEQ ID NO: 323, HC CDR2 of SEQ ID NO: 337 and HC CDR3 of SEQ ID NO: 351 of CLL-1-10;
   (xi) a HC CDR1 of SEQ ID NO: 324, HC CDR2 of SEQ ID NO: 338 and HC CDR3 of SEQ ID NO: 352 of CLL-1-11;
   (xii) a HC CDR1 of SEQ ID NO: 325, HC CDR2 of SEQ ID NO: 339 and HC CDR3 of SEQ ID NO: 353 of CLL-1-12;
   (xiii) a HC CDR1 of SEQ ID NO: 326, HC CDR2 of SEQ ID NO: 340 and HC CDR3 of SEQ ID NO: 354 of CLL-1-13;
   (xiv) a HC CDR1 of SEQ ID NO: 327, HC CDR2 of SEQ ID NO: 341 and HC CDR3 of SEQ ID NO: 355 of 181286.

In certain embodiments, the multispecific molecule, e.g., bispecific molecule, e.g., bispecific antibody or bispecific antibody-like molecule, described herein (*e.g.,* the multispecific molecule, e.g., bispecific molecule, e.g., bispecific antibody or bispecific antibody-like molecule, nucleic acid or a multispecific molecule, e.g., bispecific molecule, e.g., bispecific antibody or bispecific antibody-like molecule, polypeptide) or a CLL-1 binding domain includes a CLL-1 binding domain that includes:
(1) one, two, or three light chain (LC) CDRs chosen from one of the following:
   (i) a LC CDR1 of SEQ ID NO: 440, LC CDR2 of SEQ ID NO: 454 and LC CDR3 of SEQ ID NO: 468 of CLL-1-1;
   (ii) a LC CDR1 of SEQ ID NO: 441, LC CDR2 of SEQ ID NO: 455 and LC CDR3 of SEQ ID NO: 469 of CLL-1-2;
   (iii) a LC CDR1 of SEQ ID NO: 442, LC CDR2 of SEQ ID NO: 456 and LC CDR3 of SEQ ID NO: 470 of CLL-1-3;
   (iv) a LC CDR1 of SEQ ID NO: 443, LC CDR2 of SEQ ID NO: 457 and LC CDR3 of SEQ ID NO: 471 of CLL-1-4;
   (v) a LC CDR1 of SEQ ID NO: 444, LC CDR2 of SEQ ID NO: 458 and LC CDR3 of SEQ ID NO: 472 of CLL-1-5;
   (vi) a LC CDR1 of SEQ ID NO: 445, LC CDR2 of SEQ ID NO: 459 and LC CDR3 of SEQ ID NO: 473 of CLL-1-6;
   (vii) a LC CDR1 of SEQ ID NO: 446, LC CDR2 of SEQ ID NO: 460 and LC CDR3 of SEQ ID NO: 474 of CLL-1-7;
   (viii) a LC CDR1 of SEQ ID NO: 447, LC CDR2 of SEQ ID NO: 461 and LC CDR3 of SEQ ID NO: 475 of CLL-1-8; or
   (ix) a LC CDR1 of SEQ ID NO: 448, LC CDR2 of SEQ ID NO: 462 and LC CDR3 of SEQ ID NO: 476 of CLL-1-9;
   (x) a LC CDR1 of SEQ ID NO: 449, LC CDR2 of SEQ ID NO: 463 and LC CDR3 of SEQ ID NO: 477 of CLL-1-10;
   (xi) a LC CDR1 of SEQ ID NO: 450, LC CDR2 of SEQ ID NO: 464 and LC CDR3 of SEQ ID NO: 478 of CLL-1-11;
   (xii) a LC CDR1 of SEQ ID NO: 451, LC CDR2 of SEQ ID NO: 465 and LC CDR3 of SEQ ID NO: 479 of CLL-1-12;
   (xiii) a LC CDR1 of SEQ ID NO: 452, LC CDR2 of SEQ ID NO: 466 and LC CDR3 of SEQ ID NO: 480 of CLL-1-13;
   (xiv) a LC CDR1 of SEQ ID NO: 453, LC CDR2 of SEQ ID NO: 467 and LC CDR3 of SEQ ID NO: 481 of 181286; and/or
(2) one, two, or three heavy chain (HC) CDRs from one of the following:
   (i) a HC CDR1 of SEQ ID NO: 398, HC CDR2 of SEQ ID NO: 412 and HC CDR3 of SEQ ID NO: 426 of CLL-1-1;
   (ii) a HC CDR1 of SEQ ID NO: 399, HC CDR2 of SEQ ID NO: 413 and HC CDR3 of SEQ ID NO: 427 of CLL-1-2;
   (iii) a HC CDR1 of SEQ ID NO: 400, HC CDR2 of SEQ ID NO: 414 and HC CDR3 of SEQ ID NO: 428 of CLL-1-3;
   (iv) a HC CDR1 of SEQ ID NO: 401, HC CDR2 of SEQ ID NO: 415 and HC CDR3 of SEQ ID NO: 429 of CLL-1-4;
   (v) a HC CDR1 of SEQ ID NO: 402, HC CDR2 of SEQ ID NO: 416 and HC CDR3 of SEQ ID NO: 430 of CLL-1-5;
   (vi) a HC CDR1 of SEQ ID NO: 403, HC CDR2 of SEQ ID NO: 417 and HC CDR3 of SEQ ID NO: 431 of CLL-1-6;
   (vii) a HC CDR1 of SEQ ID NO: 404, HC CDR2 of SEQ ID NO: 418 and HC CDR3 of SEQ ID NO: 432 of CLL-1-7;
   (viii) a HC CDR1 of SEQ ID NO: 405, HC CDR2 of SEQ ID NO: 419 and HC CDR3 of SEQ ID NO: 433 of CLL-1-8; or
   (ix) a HC CDR1 of SEQ ID NO: 406, HC CDR2 of SEQ ID NO: 420 and HC CDR3 of SEQ ID NO: 434 of CLL-1-9;
   (x) a HC CDR1 of SEQ ID NO: 407, HC CDR2 of SEQ ID NO: 421 and HC CDR3 of SEQ ID NO: 435 of CLL-1-10;
   (xi) a HC CDR1 of SEQ ID NO: 408, HC CDR2 of SEQ ID NO: 422 and HC CDR3 of SEQ ID NO: 436 of CLL-1-11;
   (xii) a HC CDR1 of SEQ ID NO: 409, HC CDR2 of SEQ ID NO: 423 and HC CDR3 of SEQ ID NO: 437 of CLL-1-12;
   (xiii) a HC CDR1 of SEQ ID NO: 410, HC CDR2 of SEQ ID NO: 424 and HC CDR3 of SEQ ID NO: 438 of CLL-1-13;
   (xiv) a HC CDR1 of SEQ ID NO: 411, HC CDR2 of SEQ ID NO: 425 and HC CDR3 of SEQ ID NO: 439 of 181286.

Exemplary anti-CLL-1 scFvs include but are not limited to CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12 and CLL-1-13. The sequences of human anti-CLL-1 scFv fragments (SEQ ID NOS: 39-51), are provided in Table 2 (and the name designations are provided in Table 1).

In embodiments multispecific molecule, e.g., bispecific molecule, e.g., bispecific antibody or bispecific antibody-like molecule, constructs are generated using scFv fragments, e.g., the human scFv fragments (e.g., SEQ ID NOs: 39-51), in combination with additional sequences, such as those shown below.

It is noted that the scFv, VH and VL fragments described herein, e.g., in Table 2 or in SEQ ID NOS: 39-51, 65-77 or 78-90, without a leader sequence (e.g., without the amino acid sequence of SEQ ID NO: 1 or the nucleotide sequence of SEQ ID NO:12), are encompassed by the present invention. In other embodiments, scFv, VH and VL fragments described herein, e.g., in Table 2 or in SEQ ID NOS: 39-51, 65-77 or 78-90, with an optional leader sequence (e.g., without the amino acid sequence of SEQ ID NO: 1 or the nucleotide sequence of SEQ ID NO:12), are also encompassed by the present invention.
**Exemplary leader (amino acid sequence) (SEQ ID NO: 1)**
   MALPVTALLLPLALLLHAARP
**Exemplary leader (nucleic acid sequence) (SEQ ID NO: 12)** 
ATGGCCCTGCCTGTGACAGCCCTGCTGCTGCCTCTGGCTCTGCTGCTGCATGCCG
CTAGACCC
**Gly/Ser (SEQ ID NO:25)**
   GGGGS
**Gly/Ser (SEQ ID NO:26): This sequence may encompass 1-6 "Gly Gly Gly Gly Ser" repeating units**
   GGGGSGGGGS GGGGSGGGGS GGGGSGGGGS
**Gly/Ser (SEQ ID NO:27)**
   GGGGSGGGGS GGGGSGGGGS
**Gly/Ser (SEQ ID NO:28)**
   GGGGSGGGGS GGGGS
**Gly/Ser (SEQ ID NO:29)**
   **GGGS**
**Gly/Ser (SEQ ID NO:38): This sequence may encompass 1-10 "Gly Gly Gly Ser" repeating units**
   GGGSGGGSGG GSGGGSGGGS GGGSGGGSGG GSGGGSGGGS

### II. Additional Antigen Binding Domains

In one aspect, the invention provides multispecific molecules comprising an anti-CLL-1 binding domain, e.g., as described herein, and a domain that binds one or more, e.g., a second, additional antigen(s) or epitope(s). Various additional antigens or epitopes are contemplated by the present disclosure, and are described more fully below. In one aspect, the additional antigen or epitope is a unique (e.g., not recognized by the first anti-CLL-1 binding domain) epitope on CLL-1. In one aspect, the additional antigen or epitope is an antigen of a target (e.g., a protein) other than CLL-1. In one aspect, the additional antigen or epitope is a cancer antigen or tumor antigen. In one aspect, the additional antigen or epitope is an antigen or epitope of an immune effector cell, e.g., a T cell or NK cell.

### a. Immune Effector Antigen Binding Domains

In one aspect, the present invention provides multispecific molecules comprising an anti-CLL-1 binding domain, e.g., as described herein, and an antigen binding domain that binds an antigen or epitope of an immune effector cell, e.g., a T cell or NK cell. As the term is used herein, an "immune effector cell" refers to a cell that is involved in an immune response, e.g., in the promotion of an immune effector response.

In one aspect the antigen or epitope of an immune effector cell is an epitope of a T cell. In one aspect the antigen is CD3.

Various anti-CD3 binding domains known in the art are suitable for use in a multispecific molecule, e.g., a bispecific antibody or antibody-like molecule, comprising an anti-CLL-1 binding domain described herein. Anti-CD3 binding domains that may be incorporated into the multispecific constructs of the present invention include those described in, for example, US2015/011825, WO2010/037835, WO2015/026894, WO2015/026892, US2014/0302064, US9029508, US2015/0118252, WO2014/051433 and WO2010/037835, the contents of which are hereby incorporated by reference in their entirety.
Exemplary anti-CD3 binding domains are provided in Table 26.

**Table 26. Sequences of VL and VH domains of anti-CD3 binding domains.**

| **Bindin g Domai n** | **Chain** | **Sequence** | **SEQ ID NO:** |
|---|---|---|---|
| CD3-1 | VH | | 1200 |
| | VL | | 1201 |
| CD3-2 | VH | EVQLVESGGGLVQPKGSLKLSCAASGFTFNTYAMNWVRQAPGKGLEWVARIRSKYN NYATYYADSVKDRFTISRDDSQSILYLQMNNLKTEDTAMYYCVRHGNFGNSYVSWFA YWGQGTLVTVSA | 1202 |
| | VL | QAVVTQESALTTSPGETVTLTCRSSTGAVTTSNYANWVQEKPDHLFTGLIGGTNKRAP GVPARFSGSLIGDKAALTITGAQTEDEAIYFCALWYSNLWVFGGGTKLTVL | 1203 |
| CD3-3 | VH | QVQLQQSGAELARPGASVKMSCKASGYTFTSYTMHWVKQRPGQGLEWIGYINPSSG YTKYNQKFKDKATLTADKSSSTAYMQLSSLTSEDSAVYYCARWQDYDVYFDYWGQGT TLTVSS | 1204 |
| | VL | QIVLSQSPAILSASPGEKVTMTCRASSSVSYMHWYQQKPGSSPKPWIYATSNLASGVP ARFSGSGSGTSYSLTISRVEAEDAATYYCQQWSSNPPTFGGGTKLETK | 1205 |
| CD3-5 | VH | | 1206 |
| | VL | QIVLTQSPAIMSASPGEKVTMTCRASSSVSYMNWYQQKSGTSPKRWIYDTSKVASGV PYRFSGSGSGTSYSLTISSMEAEDAATYYCQQWSSNPLTFGSGTKLEIN | 1207 |
| CD3-6 | VH | | 1208 |
| | VL | | 1209 |
| CD3-7 | VH | | 1210 |
| | VL | | 1211 |
| CD3-8 | VH | EVQLVESGGGLVQPGGSLRLSCAASGFTFSTYAMNWVRQAPGKGLEWVGRIRSKYN NYATYYADSVKDRFISRDDSKNSLYLQMNSLKTEDTAVYYCVRHGNFGNSYVSWFAY WGQGTLVTVSS | 1212 |
| | VL | QAVVTQEPSLTVSPGGTVTLTCRSSTGAVTTSNYANWVQQKPGQAPRGLIGGTNKRA PWTPARFSGSLLGGKAALIGAQAEDEADYYCALWYSNLWVFGGGTKLTVL | 1213 |
| CD3-9 | VH | DIKLQQSGAELARPGASVKMSCKTSGYTFTRYTMHWVKQRPGQGLEWIGYINPSRGY TNYNQKFKDKATLTTDKSSSTAYMQLSSLTSEDSAVYYCARYYDDHYCLDYWGQGTTL TVSS | 1214 |
| | VL | DIQLTQSPAIMSASPGEKVTMTCRASSSVSYMNWYQQKSGTSPKRWIYDTSKVASGV PYRFSGSGSGTSYSLISSMEAEDAATYYCQQWSSNPLTFGAGTKLELK | 1215 |
| CD3-10 | VH | EVQLVESGGGLVQPGGSLRLSCAASGFTFNTYAMNWVRQAPGKGLEWVARIRSKYN NYATYYADSVKDRFISRDDSKNSLYLQMNSLKTEDTAVYYCVRHGNFGNSYVSWFAY WGQGTLVTVSS | 1216 |
| | VL | QAVVTQEPSLTVSPGGTVTLTCRSSTGAVTTSNYANWVQQKPGQAPRGLIGGTNKRA PWTPARFSGSLLGGKAALIGAQAEDEADYYCALWYSNLWVFGGGTKLTVL | 1217 |
| CD3-11 | VH | EVKLLESGGGLVQPKGSLKLSCAASGFTFNTYAMNWVRQAPGKGLEWVARIRSKYNN YATYYADSVKDRFTISRDDSQSILYLQMNNLKTEDTAMYYCVRHGNFGNSYVSWFAY WGQGTLVTVSA | 1218 |
| | VL | QAVVTQESALTTSPGETVTLTCRSSTGAVTTSNYANWVQEKPDHLFTGLIGGTNKRAP GVPARFSGSLIGDKAALTITGAQTEDEAIYFCALWYSNLWVFGGGTKLTVL | 1219 |
| CD3-12 | VH | EVQLVESGGGLVQPGGSLKLSCAASGFTFNSYAMNWVRQAPGKGLEWVARIRSKYN NYATYYADSVKGRFTISRDDSKNTAYLQMNNLKTEDTAVYYCVRHGNFGNSYVSWW AYWGQGTLVTVSS | 1220 |
| | VL | QTVVTQEPSLTVSPGGTVTLTCGSSTGAVTSGNYPNWVQQKPGQAPRGLIGGTKFLA PGTPQRFSGSLLGGKAALTLSGVQPEDEAEYYCVLWYSNRWVFGGGTKLTVL | 1221 |
| CD3-13 | VH | EVQLVESGGGLVQPGGSLKLSCAASGFTFNKYAMNWVRQAPGKGLEWVARIRSKYN NYATYYADSVKDRFTISRDDSKNTAYLQMNNLKTEDTAVYYCVRHGNFGNSYISYWAY WGQGTLVTVSS | 1222 |
| | VL | QTVVTQEPSLTVSPGGTVTLTCGSSTGAVTSGNYPNWVQQKPGQAPRGLIGGTKFLA PGTPARFSGSLLGGKAALTLSGVQPEDEAEYYCVLWYSNRWVFGGGTKLTVL | 1223 |
| CD3-14 | VH | QVQLVQSGAEVKKPGASVKVSCKASGYTFTRYTMHWVRQAPGQGLEWMGYINPSR GYTNYNQKFKDRVTMTTDTSISTAYMELSRLRSDDTAVYYCARYYDDHYCLDYWGQG TLVTVSS | 1224 |
| | VL | EIVLTQSPATLSLSPGERATLSCSASSSVSYMNWYQQKPGQAPRLLIYDTSKLASGVPA HFRGSGSGTDFTLTISSLEPEDFAVYYCQQWSSNPFTFGQGTKVEIK | 1225 |
| CD3-15 | VH | EVQLVESGGGLVQPGGSLRLSCAASGFTFSTYAMNWVRQAPGKGLEWVSRIRSKYNN YATYYADSVKDRFTISRDDSKNTLYLQMNSLRAEDTAVYYCARHGNFGNSYVSWFAY WGQGTMVTVSS | 1226 |
| | VL | QAVVTQEPSLTVSPGGTVTLTCRSSTGAVTTSNYANWVQQKPGQAPRGLIGGTNKRA PGTPARFSGSLLGGKAALTLSGVQPEDEAEYYCALWYSNLWVFGGGTKLTVL | 1227 |
| CD3-19 | VH | EVQLVESGGGLVQPGGSLRLSCAASGFTFNTYAMNWVRQAPGKGLEWVGRIRSKYN NYATYYADSVKDRFTISRDDSKNSLYLQMNSLKTEDTAVYYCVRHGNFGNSYVSWFAY WGQGTLVTVSS | 1228 |
| | VL | QAVVTQEPSLTVSPGGTVTLTCRSSTGAVTTSNYANWVQEKPGQAPRGLIGGTNKRA PWTPARFSGSLLGGKAALTITGAQAEDEADYYCALWYSNLWVFGGGTKLTVL | 1229 |
| CD3-21 | VH | EVQLVESGGGLVQPGGSLRLSCAASGFTFNTYAMNWVRQAPGKGLEWVGRIRSKYN NYATYYADSVKGRFTISRDDSKNTLYLQMNSLRAEDTAVYYCVRHGNFGNSYVSWFA YWGQGTLVTVSS | 1230 |
| | VL | QAVVTQEPSLTVSPGGTVTLTCGSSTGAVTTSNYANWVQQKPGQAPRGLIGGTNKRA PGVPARFSGSLLGGKAALTLSGAQPEDEAEYYCALWYSNLWVFGGGTKLTVL | 1231 |
| CD3-22 | VH | EVQLVESGGGLVQPGGSLRLSCAASGFTFSTYAMNWVRQAPGKGLEWVGRIRSKYN NYATYYADSVKGRFTISRDDSKNTLYLQMNSLRAEDTAVYYCVRHGNFGDSYVSWFA YWGQGTLVTVSS | 1232 |
| | VL | QAVVTQEPSLTVSPGGTVTLTCGSSTGAVTTSNYANWVQQKPGKSPRGLIGGTNKRA PGVPARFSGSLLGGKAALTISGAQPEDEADYYCALWYSNHWVFGGGTKLTVL | 1233 |
| CD3-23 | VH | | 1234 |
| | VL | DIQMTQSPSSLSASVGDRVTITCSASSSVSYMNWYQQTPGKAPKRWIYDTSKLASGVP SRFSGSGSGTDYTFTISSLQPEDIATYYCQQWSSNPFTFGQGT | 1235 |
| CD3-24 | VH | QVQLVQSGGGVVQPGRSLRLSCKASGYTFTRYTMHWVRQAPGKGLEWIGYINPSRG YTNYNQKVKDRFTISRDNSKNTAFLQMDSLRPEDTGVYFCARYYDDHYSLDYWGQGT PVTVSS | 1236 |
| | VL | DIQMTQSPSSLSASVGDRVTITCSASSSVSYMNWYQQTPGKAPKRWIYDTSKLASGVP SRFSGSGSGTDYTFTISSLQPEDIATYYCQQWSSNPFTFGQGT | 1237 |
| CD3-25 | VH | QVQLVQSGGGVVQPGRSLRLSCKASGYTFTRYTMHWVRQAPGKGLEWIGYINPSRG YTNYNQKVKDRFTISRDNSKNTAFLQMDSLRPEDTGVYFCARYYDDHYSLDYWGQGT PVTVSS | 1236 |
| | VL | | 1209 |

**Table 27. HCDR and LCDR sequences of anti-CD3 binding domains (CDR1, CDR2 and CDR3 associated with a VH chain refer to HCDR1, HCDR2 and HCDR3, respectively (also referred to herein as HC CDR1, HC CDR2 and HC CDR3, respectively); CDR1, CDR2 and CDR3 associated with a VL chain refer to LCDR1, LCDR2 and LCDR3, respectively (also referred to herein as LC CDR1, LC CDR2 and LC CDR3, respectively)), according to the Kabat numbering scheme (Kabat et al. (1991), "Sequences of Proteins of Immunological Interest," 5th Ed. Public Health Service, National Institutes of Health, Bethesda, MD).**

| **Binding Domain** | **Chain** | **CDR1** | **SEQ ID NO:** | **CDR2** | **SEQ ID NO:** | **CDR3** | **SEQ ID NO:** |
|---|---|---|---|---|---|---|---|
| CD3-1 | VH | RYTMH | 1700 | YINPSRGYTNYNQKFKD | 1738 | YYDDHYCLDY | 1776 |
| | VL | SASSSVSYM N | 1701 | DTSKLAS | 1739 | QQWSSNPFT | 1777 |
| CD3-2 | VH | TYAMN | 1702 | RIRSKYNNYATYYADSVK D | 1740 | HGNFGNSYVSWFA Y | 1778 |
| | VL | RSSTGAVTTSN YAN | 1703 | GTNKRAP | 1741 | ALWYSNLWV | 1779 |
| CD3-3 | VH | SYTM H | 1704 | YINPSSGYTKYNQKFKD | 1742 | WQDYDVYFDY | 1780 |
| | VL | RASSSVSYMH | 1705 | ATSNLAS | 1743 | QQWSSNPPT | 1781 |
| CD3-5 | VH | RYTMH | 1706 | YINPSRGYTNYNQKFKD | 1744 | YYDDHYCLDY | 1782 |
| | VL | RASSSVSYMN | 1707 | DTSKVAS | 1745 | QQWSSNPLT | 1783 |
| CD3-6 | VH | RYTMH | 1708 | YINPSRGYTNYNQKVKD | 1746 | YYDDHYCLDY | 1784 |
| | VL | SASSSVSYM N | 1709 | DTSKLAS | 1747 | QQWSSNPFT | 1785 |
| CD3-7 | VH | GYGMH | 1710 | VIWYDGSKKYYVDSVKG | 1748 | QMGYWHFDL | 1786 |
| | VL | RASQSVSSYLA | 1711 | DASNRAT | 1749 | QQRSNWPPLT | 1787 |
| CD3-8 | VH | TYAMN | 1712 | RIRSKYNNYATYYAD | 1750 | VRHGNFGNSYVSW FAY | 1788 |
| | VL | RSSTGAVTTSN YAN | 1713 | GTNKRAP | 1751 | ALWYSNLWV | 1789 |
| CD3-9 | VH | RYTMH | 1714 | YINPSRGYTNYNQKFKD | 1752 | YYDDHYCLDY | 1790 |
| | VL | RASSSVSYMN | 1715 | DTSKVAS | 1753 | QQWSSNPLT | 1791 |
| CD3-10 | VH | TYAMN | 1716 | RIRSKYNNYATYYAD | 1754 | VRHGNFGNSYVSW FAY | 1792 |
| | VL | RSSTGAVTTSN YAN | 1717 | GTNKRAP | 1755 | ALWYSNLWV | 1793 |
| CD3-11 | VH | TYAMN | 1718 | RIRSKYNNYATYYADSVK D | 1756 | HGNFGNSYVSWFA Y | 1794 |
| | VL | RSSTGAVTTSN YAN | 1719 | GTNKRAP | 1757 | ALWYSNLWV | 1795 |
| CD3-12 | VH | SYAM N | 1720 | RIRSKYNNYATYYADSVK G | 1758 | HGNFGNSYVSWWA Y | 1796 |
| | VL | GSSTGAVTSG NYPN | 1721 | GTKFLAP | 1759 | VLWYSNRWV | 1797 |
| CD3-13 | VH | KYAMN | 1722 | RIRSKYNNYATYYADSVK D | 1760 | HGNFGNSYISYWAY | 1798 |
| | VL | GSSTGAVTSG NYPN | 1723 | GTKFLAP | 1761 | VLWYSNRWV | 1799 |
| CD3-14 | VH | RYTMH | 1724 | YINPSRGYTNYNQKFKD | 1762 | YYDDHYCLDY | 1800 |
| | VL | SASSSVSYM N | 1725 | DTSKLAS | 1763 | QQWSSNPFT | 1801 |
| CD3-15 | VH | TYAMN | 1726 | RIRSKYNNYATYYADSVK D | 1764 | HGNFGNSYVSWFA Y | 1802 |
| | VL | RSSTGAVTTSN YAN | 1727 | GTNKRAP | 1765 | ALWYSNLWV | 1803 |
| CD3-19 | VH | TYAMN | 1728 | RIRSKYNNYATYYADSVK D | 1766 | HGNFGNSYVSWFA Y | 1804 |
| | VL | RSSTGAVTTSN YAN | 1729 | GTNKRAP | 1767 | ALWYSNLWV | 1805 |
| CD3-21 | VH | TYAMN | 1730 | RIRSKYNNYATYYADSVK G | 1768 | HGNFGNSYVSWFA Y | 1806 |
| | VL | GSSTGAVTTSN YAN | 1731 | GTNKRAP | 1769 | ALWYSNLWV | 1807 |
| CD3-22 | VH | TYAMN | 1732 | RIRSKYNNYATYYADSVK G | 1770 | HGNFGDSYVSWFAY | 1808 |
| | VL | GSSTGAVTTSN YAN | 1733 | GTNKRAP | 1771 | ALWYSNHWV | 1809 |
| CD3-23 | VH | RYTM H | 1734 | YINPSRGYTNYNQKVKD | 1772 | YYDDHYCLDY | 1810 |
| | VL | SASSSVSYM N | 1735 | DTSKLAS | 1773 | QQWSSNPFT | 1811 |
| CD3-24 | VH | RYTM H | 1736 | YINPSRGYTNYNQKVKD | 1774 | YYDDHYSLDY | 1812 |
| | VL | SASSSVSYM N | 1737 | DTSKLAS | 1775 | QQWSSNPFT | 1813 |

**Table 28. HCDR and LCDR sequences of anti-CD3 binding domains (CDR1, CDR2 and CDR3 associated with a VH chain refer to HCDR1, HCDR2 and HCDR3, respectively (also referred to herein as HC CDR1, HC CDR2 and HC CDR3, respectively); CDR1, CDR2 and CDR3 associated with a VL chain refer to LCDR1, LCDR2 and LCDR3, respectively (also referred to herein as LC CDR1, LC CDR2 and LC CDR3, respectively)), according to the Chothia numbering scheme (Al-Lazikani et al., (1997) JMB 273,927-948).**

| Binding Domain | Chain | CDR1 | SEQ ID NO: | CDR2 | SEQ ID NO: | CDR3 | SEQ ID NO: |
|---|---|---|---|---|---|---|---|
| CD3-1 | VH | GYTFTRY | 1500 | NPSRGY | 1538 | YYDDHYCLDY | 1576 |
| | VL | SSSVSY | 1501 | DTS | 1539 | WSSNPF | 1577 |
| CD3-2 | VH | GFTFNTY | 1502 | RSKYNNYA | 1540 | HGNFGNSYVSWFAY | 1578 |
| | VL | STGAVTTSNY | 1503 | GTN | 1541 | WYSNLW | 1579 |
| CD3-3 | VH | GYTFTSY | 1504 | NPSSGY | 1542 | WQDYDVYFDY | 1580 |
| | VL | SSSVSY | 1505 | ATS | 1543 | WSSNPP | 1581 |
| CD3-5 | VH | GYTFTRY | 1506 | NPSRGY | 1544 | YYDDHYCLDY | 1582 |
| | VL | SSSVSY | 1507 | DTS | 1545 | WSSNPL | 1583 |
| CD3-6 | VH | GYTFTRY | 1508 | NPSRGY | 1546 | YYDDHYCLDY | 1584 |
| | VL | SSSVSY | 1509 | DTS | 1547 | WSSNPF | 1585 |
| CD3-7 | VH | GFKFSGY | 1510 | WYDGSK | 1548 | QMGYWHFDL | 1586 |
| | VL | SQSVSSY | 1511 | DAS | 1549 | RSNWPPL | 1587 |
| CD3-8 | VH | GFTFSTY | 1512 | RSKYNNYAT | 1550 | HGNFGNSYVSWFA | 1588 |
| | VL | STGAVTTSNY | 1513 | GTN | 1551 | WYSNLW | 1589 |
| CD3-9 | VH | GYTFTRY | 1514 | NPSRGY | 1552 | YYDDHYCLDY | 1590 |
| | VL | SSSVSY | 1515 | DTS | 1553 | WSSNPL | 1591 |
| CD3-10 | VH | GFTFNTY | 1516 | RSKYNNYAT | 1554 | HGNFGNSYVSWFA | 1592 |
| | VL | STGAVTTSNY | 1517 | GTN | 1555 | WYSNLW | 1593 |
| CD3-11 | VH | GFTFNTY | 1518 | RSKYNNYA | 1556 | HGNFGNSYVSWFAY | 1594 |
| | VL | STGAVTTSNY | 1519 | GTN | 1557 | WYSNLW | 1595 |
| CD3-12 | VH | GFTFNSY | 1520 | RSKYNNYA | 1558 | HGNFGNSYVSWWAY | 1596 |
| | VL | STGAVTSGNY | 1521 | GTK | 1559 | WYSNRW | 1597 |
| CD3-13 | VH | GFTFNKY | 1522 | RSKYNNYA | 1560 | HGNFGNSYISYWAY | 1598 |
| | VL | STGAVTSGNY | 1523 | GTK | 1561 | WYSNRW | 1599 |
| CD3-14 | VH | GYTFTRY | 1524 | NPSRGY | 1562 | YYDDHYCLDY | 1600 |
| | VL | SSSVSY | 1525 | DTS | 1563 | WSSNPF | 1601 |
| CD3-15 | VH | G FTFSTY | 1526 | RSKYNNYA | 1564 | HGNFGNSYVSWFAY | 1602 |
| | VL | STGAVTTSNY | 1527 | GTN | 1565 | WYSNLW | 1603 |
| CD3-19 | VH | GFTFNTY | 1528 | RSKYNNYA | 1566 | HGNFGNSYVSWFAY | 1604 |
| | VL | STGAVTTSNY | 1529 | GTN | 1567 | WYSNLW | 1605 |
| CD3-21 | VH | GFTFNTY | 1530 | RSKYNNYA | 1568 | HGNFGNSYVSWFAY | 1606 |
| | VL | STGAVTTSNY | 1531 | GTN | 1569 | WYSNLW | 1607 |
| CD3-22 | VH | G FTFSTY | 1532 | RSKYNNYA | 1570 | HGNFGDSYVSWFAY | 1608 |
| | VL | STGAVTTSNY | 1533 | GTN | 1571 | WYSNHW | 1609 |
| CD3-23 | VH | GYTFTRY | 1534 | NPSRGY | 1572 | YYDDHYCLDY | 1610 |
| | VL | SSSVSY | 1535 | DTS | 1573 | WSSNPF | 1611 |
| CD3-24 | VH | GYTFTRY | 1536 | NPSRGY | 1574 | YYDDHYSLDY | 1612 |
| | VL | SSSVSY | 1537 | DTS | 1575 | WSSNPF | 1613 |

**Table 29. HCDR and LCDR sequences of anti-CD3 binding domains (CDR1, CDR2 and CDR3 associated with a VH chain refer to HCDR1, HCDR2 and HCDR3, respectively (also referred to herein as HC CDR1, HC CDR2 and HC CDR3, respectively); CDR1, CDR2 and CDR3 associated with a VL chain refer to LCDR1, LCDR2 and LCDR3, respectively (also referred to herein as LC CDR1, LC CDR2 and LC CDR3, respectively)), according to a combination of the Kabat numbering scheme (Kabat et al. (1991), "Sequences of Proteins of Immunological Interest," 5th Ed. Public Health Service, National Institutes of Health, Bethesda, MD) and the Chothia numbering scheme (Al-Lazikani et al., (1997) JMB 273,927-948).**

| Binding Domain | Chain | CDR1 | SEQ ID NO: | CDR2 | SEQ ID NO: | CDR3 | SEQ ID NO: |
|---|---|---|---|---|---|---|---|
| CD3-1 | VH | GYTFTRYTM H | 1300 | YINPSRGYTNYNQKFKD | 1338 | YYDDHYCLDY | 1376 |
| | VL | SASSSVSYM N | 1301 | DTSKLAS | 1339 | QQWSSNPFT | 1377 |
| CD3-2 | VH | GFTFNTYAMN | 1302 | RIRSKYNNYATYYADSV KD | 1340 | HGNFGNSYVSWFA Y | 1378 |
| | VL | RSSTGAVTTSNY AN | 1303 | GTNKRAP | 1341 | ALWYSNLWV | 1379 |
| CD3-3 | VH | GYTFTSYTM H | 1304 | YINPSSGYTKYNQKFKD | 1342 | WQDYDVYFDY | 1380 |
| | VL | RASSSVSYM H | 1305 | ATSNLAS | 1343 | QQWSSNPPT | 1381 |
| CD3-5 | VH | GYTFTRYTM H | 1306 | YINPSRGYTNYNQKFKD | 1344 | YYDDHYCLDY | 1382 |
| | VL | RASSSVSYM N | 1307 | DTSKVAS | 1345 | QQWSSNPLT | 1383 |
| CD3-6 | VH | GYTFTRYTM H | 1308 | YINPSRGYTNYNQKVKD | 1346 | YYDDHYCLDY | 1384 |
| | VL | SASSSVSYM N | 1309 | DTSKLAS | 1347 | QQWSSNPFT | 1385 |
| CD3-7 | VH | GFKFSGYGMH | 1310 | VIWYDGSKKYYVDSVK G | 1348 | QMGYWHFDL | 1386 |
| | VL | RASQSVSSYLA | 1311 | DASNRAT | 1349 | QQRSNWPPLT | 1387 |
| CD3-8 | VH | GFTFSTYAMN | 1312 | RIRSKYNNYATYYADSV K | 1350 | HGNFGNSYVSWFA Y | 1388 |
| | VL | RSSTGAVTTSNY AN | 1313 | GTNKRAP | 1351 | ALWYSNLWV | 1389 |
| CD3-9 | VH | GYTFTRYTM H | 1314 | YINPSRGYTNYNQKFKD | 1352 | YYDDHYCLDY | 1390 |
| | VL | RASSSVSYM N | 1315 | DTSKVAS | 1353 | QQWSSNPLT | 1391 |
| CD3-10 | VH | GFTFNTYAMN | 1316 | RIRSKYNNYATYYADSV K | 1354 | HGNFGNSYVSWFA Y | 1392 |
| | VL | RSSTGAVTTSNY AN | 1317 | GTNKRAP | 1355 | ALWYSNLWV | 1393 |
| CD3-11 | VH | GFTFNTYAMN | 1318 | RIRSKYNNYATYYADSV KD | 1356 | HGNFGNSYVSWFA Y | 1394 |
| | VL | RSSTGAVTTSNY AN | 1319 | GTNKRAP | 1357 | ALWYSNLWV | 1395 |
| CD3-12 | VH | GFTFNSYAMN | 1320 | RIRSKYNNYATYYADSV KG | 1358 | HGNFGNSYVSWW AY | 1396 |
| | VL | GSSTGAVTSGNY PN | 1321 | GTKFLAP | 1359 | VLWYSNRWV | 1397 |
| CD3-13 | VH | GFTFNKYAMN | 1322 | RIRSKYNNYATYYADSV KD | 1360 | HGNFGNSYISYWA Y | 1398 |
| | VL | GSSTGAVTSGNY PN | 1323 | GTKFLAP | 1361 | VLWYSNRWV | 1399 |
| CD3-14 | VH | GYTFTRYTM H | 1324 | YINPSRGYTNYNQKFKD | 1362 | YYDDHYCLDY | 1400 |
| | VL | SASSSVSYM N | 1325 | DTSKLAS | 1363 | QQWSSNPFT | 1401 |
| CD3-15 | VH | G FTFSTYAM N | 1326 | RIRSKYNNYATYYADSV KD | 1364 | HGNFGNSYVSWFA Y | 1402 |
| | VL | RSSTGAVTTSNY AN | 1327 | GTNKRAP | 1365 | ALWYSNLWV | 1403 |
| CD3-19 | VH | GFTFNTYAMN | 1328 | RIRSKYNNYATYYADSV KD | 1366 | HGNFGNSYVSWFA Y | 1404 |
| | VL | RSSTGAVTTSNY AN | 1329 | GTNKRAP | 1367 | ALWYSNLWV | 1405 |
| CD3-21 | VH | GFTFNTYAMN | 1330 | RIRSKYNNYATYYADSV KG | 1368 | HGNFGNSYVSWFA Y | 1406 |
| | VL | GSSTGAVTTSNY AN | 1331 | GTNKRAP | 1369 | ALWYSNLWV | 1407 |
| CD3-22 | VH | G FTFSTYAM N | 1332 | RIRSKYNNYATYYADSV KG | 1370 | HGNFGDSYVSWFA Y | 1408 |
| | VL | GSSTGAVTTSNY AN | 1333 | GTNKRAP | 1371 | ALWYSNHWV | 1409 |
| CD3-23 | VH | GYTFTRYTM H | 1334 | YINPSRGYTNYNQKVKD | 1372 | YYDDHYCLDY | 1410 |
| | VL | SASSSVSYM N | 1335 | DTSKLAS | 1373 | QQWSSNPFT | 1411 |
| CD3-24 | VH | GYTFTRYTM H | 1336 | YINPSRGYTNYNQKVKD | 1374 | YYDDHYSLDY | 1412 |
| | VL | SASSSVSYM N | 1337 | DTSKLAS | 1375 | QQWSSNPFT | 1413 |

In one aspect, the anti-CD3 binding domain comprises the VL of SEQ ID NO: [1900] (QVQLQQSGAELARPGASVKMSCKASGYTFTRYTMHWVKQRPGQGLEWIGYINPSR GYTNYNQKFKDKATLTTDKSSSTAYMQLSSLTSEDSAVYYCARYYDDHYCLDYWG QGTTLTVSS) and the VH of SEQ ID NO: [1901] (QIVLTQSPAIMSASPGEKVTMTCSASSSVSYMNWYQQKSGTSPKRWIYDTSKLASG VPAHFRGSGSGTSYSLTISGMEAEDAATYYCQQWSSNPFTFGSGTKLEIN). In one aspect, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1900] and a VH having at least 75%, at least 80%, at least 85%, at least 90%, at least 95%, at least 96%, at least 97%, at least 98%, or at least 99% sequence identity to SEQ ID NO: [1901]. In one aspect, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1900], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1901].

In one aspect, the anti-CD3 binding domain comprises the VL of SEQ ID NO: [1902] (DIQLTQSPAIMSASPGEKVTMTCRASSSVSYMNWYQQKSGTSPKRWIYDTSKVASG VPYRFSGSGSGTSYSLISSMEAEDAATYYCQQWSSNPLTFGAGTKLELK) and the VH of SEQ ID NO: [1903] (DIKLQQSGAELARPGASVKMSCKTSGYTFTRYTMHWVKQRPGQGLEWIGYINPSRG YTNYNQKFKDKATLTTDKSSSTAYMQLSSLTSEDSAVYYCARYYDDHYCLDYWGQ GTTLTVSS). In one aspect, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1902] and a VH having at least 75%, at least 80%, at least 85%, at least 90%, at least 95%, at least 96%, at least 97%, at least 98%, or at least 99% sequence identity to SEQ ID NO: [1903]. In one aspect, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1902], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1903].

In one aspect, the anti-CD3 binding domain comprises the VL of SEQ ID NO: [1904] (QAVVTQESALTTSPGETVTLTCRSSTGAVTTSNYANWVQEKPDHLFTGLIGGTNKR APGVPARFSGSLIGDKAALTITGAQTEDEAIYFCALWYSNLWVFGGGTKLTVL) and the VH of SEQ ID NO: [1905] (EVQLVESGGGLVQPKGSLKLSCAASGFTFNTYAMNWVRQAPGKGLEWVARIRSKY NNYATYYADSVKDRFTISRDDSQSILYLQMNNLKTEDTAMYYCVRHGNFGNSYVS WFAYWGQGTLVTVSA). In one aspect, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1904] and a VH having at least 75%, at least 80%, at least 85%, at least 90%, at least 95%, at least 96%, at least 97%, at least 98%, or at least 99% sequence identity to SEQ ID NO: [1905]. In one aspect, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1904], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1905].

In one aspect, the anti-CD3 binding domain comprises the VL of SEQ ID NO: [1906] (QAVVTQEPSLTVSPGGTVTLTCRSSTGAVTTSNYANWVQQKPGQAPRGLIGGTNKR APWTPARFSGSLLGGKAALIGAQAEDEADYYCALWYSNLWVFGGGTKLTVL) and the VH of SEQ ID NO: [1907] (EVQLVESGGGLVQPGGSLRLSCAASGFTFSTYAMNWVRQAPGKGLEWVGRIRSKY NNYATYYADSVKDRFISRDDSKNSLYLQMNSLKTEDTAVYYCVRHGNFGNSYVSW FAYWGQGTLVTVSS). In one aspect, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1906] and a VH having at least 75%, at least 80%, at least 85%, at least 90%, at least 95%, at least 96%, at least 97%, at least 98%, or at least 99% sequence identity to SEQ ID NO: [1907]. In one aspect, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1906], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1907].

In one aspect, the anti-CD3 binding domain comprises the VL of SEQ ID NO: [1908] (QAVVTQEPSLTVSPGGTVTLTCRSSTGAVTTSNYANWVQQKPGQAPRGLIGGTNKR APWTPARFSGSLLGGKAALIGAQAEDEADYYCALWYSNLWVFGGGTKLTVL) and the VH of SEQ ID NO: [1909] (EVQLVESGGGLVQPGGSLRLSCAASGFTFNTYAMNWVRQAPGKGLEWVARIRSKY NNYATYYADSVKDRFISRDDSKNSLYLQMNSLKTEDTAVYYCVRHGNFGNSYVSW FAYWGQGTLVTVSS). In one aspect, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1908] and a VH having at least 75%, at least 80%, at least 85%, at least 90%, at least 95%, at least 96%, at least 97%, at least 98%, or at least 99% sequence identity to SEQ ID NO: [1909]. In one aspect, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1908], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1909].

In one aspect, the anti-CD3 binding domain comprises the VL of SEQ ID NO: [1910] (DIQMTQSPSSLSASVGDRVTITCRASQSISSYLNWYQQKPGKAPKLLIYAASSLQSGV PSR FSGSGSGTDFTLTISSLQPEDFATYYCQQSYSTPPTFGQGTKVEIK) and the VH of SEQ ID NO: [1911] (QVQLVESGGGVVQPGRSLRLSCAASGFTFRSYGMHWVRQAPGKGLEWVAIIWYSG SK KNYADSVKGRFTISRDNSKNTLYLQMNSLRAEDTAVYYCARGTGYNWFDPWGQGT LV TVSS). In one aspect, the anti-CD3 binding domain comprises a VL having at least 75%, at least 80%, at least 85%, at least 90%, at least 95%, at least 96%, at least 97%, at least 98%, or at least 99% sequence identity to SEQ ID NO: [1910] and a VH having at least 75%, at least 80%, at least 85%, at least 90%, at least 95%, at least 96%, at least 97%, at least 98%, or at least 99% sequence identity to SEQ ID NO: [1911]. In one aspect, the anti-CD3 binding domain comprises a VL having at least 1, 2, 3, 4, 5, 6, 7, 8, 9, or 10, but no more than 50, 60, 70, 80, 90 or 100 amino acid substitutions relative to the amino acids of SEQ ID NO: [1910], and a VH having at least 1, 2, 3, 4, 5, 6, 7, 8, 9, or 10, but no more than 50, 60, 70, 80, 90 or 100 amino acid substitutions relative to the amino acids of SEQ ID NO: [1911].

Virtually any other T cell antigen or epitope is useful in the present invention. By way of example, the T cell antigen or epitope may be an immune costimulatory molecule (or epitope thereof). As the terms is used herein, an "immune costimulatory molecule" or "costimulatory molecule" (used interchangeably herein) refer to the cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are required for an efficient immune response. Costimulatory molecules include, but are not limited to an MHC class I molecule, TNF receptor proteins, Immunoglobulin-like proteins, cytokine receptors, integrins, signalling lymphocytic activation molecules (SLAM proteins), activating NK cell receptors, BTLA, Toll ligand receptor, OX40, CD2, CD7, CD27, CD28, CD30, CD40, CD47, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), TLR7, LTBR, LAT, GADS, SLP-76, PAG/Cbp, CD19a, and a ligand that specifically binds with CD83.

By way of example, the T cell antigen or epitope may be an immune inhibitory molecule, e.g., a checkpoint protein. As the term is used herein, an "immune inhibitory molecule" refers to a molecule expressed on the surface of a cell that, when bound by its cognate binding partner, serves causes a reduction in an immune response. Examples of immune inhibitory (e.g., checkpoint) molecules include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta.

In another aspect of the invention, the antigen or epitope of an immune effector cell is an epitope of a NK cell. In one aspect the antigen is CD16 (Fc Receptor gamma III). Any anti-CD 16 binding domain, including those known in the art, may be useful in the multispecific molecules of the present invention. In embodiments, the anti-CD 16 binding domain comprises an anti-CD 16 binding domain described in, for example, WO2005/0089519 or US2015/0218275, the contents of which are hereby incorporated by reference in their entirety. In one aspect the antigen is CD64 (Fc Receptor gamma I). Any anti-CD64 binding domain, including those known in the art, may be useful in the multispecific molecules of the present invention. In embodiments, the anti-CD64 binding domain comprises an anti-CD64 binding domain described in, for example, WO2006/002438, the contents of which is hereby incorporated by reference in its entirety.

### b. Cancer Cell Antigen Binding Domains

In one aspect, the multispecific molecule, e.g., bispecific molecule, e.g., bispecific antibody or bispecific antibody-like molecule, is characterized by a first anti-CLL-1 binding domain, e.g., comprises a scFv as described herein, e.g., as described in Table 2, or comprises the light chain CDRs and/or heavy chain CDRs from a CLL-1 scFv described herein, and a second antigen binding domain that has binding specificity for a cancer antigen, e.g., a cancer antigen expressed on a cell that also expresses CLL-1. In some aspects the second antigen binding domain has specificity for an antigen expressed on AML cells, e.g., an antigen other than CLL-1. In some aspects the second antigen binding domain has binding specificity for an antigen expressed on multiple myeloma (MM) cells. For example, the second antigen binding domain has binding specificity for CD123. As another example, the second antigen binding domain has binding specificity for CD33. As another example, the second antigen binding domain has binding specificity for CD34. As another example, the second antigen binding domain has binding specificity for FLT3. For example, the second antigen binding domain has binding specificity for folate receptor beta. As another example, the second antigen binding domain has binding specificity for BCMA. In some aspects, the second antigen binding domain has binding specificity for an antigen expressed on B-cells, for example, CD10, CD19, CD20, CD22, CD34, CD123, FLT-3, ROR1, CD79b, CD179b, or CD79a.

### III. Formats for Multispecific Molecules

In some aspects a multispecific molecule is a bispecific antibody or bispecific antibody-like molecule. In some aspects, the bispecific antibody or antibody-like molecule may be multivalent, e.g., bivalent, with respect to one antigen and monovalent with respect to the other antigen. An exemplary bispecific antibody molecule or bispecific antibody-like molecule is characterized by a first antigen binding domain (e.g., comprising a first VL disposed on a first polypeptide and a first VH disposed on a second polypeptide) which has binding specificity for a first antigen or epitope (e.g., CLL-1) and a second antigen binding domain (e.g., comprising a second VL disposed on a third polypeptide and a second VH disposed on a fourth polypeptide) that has binding specificity for a second epitope (e.g., an epitope of an immune effector cell or of a tumor or malignant cell, e.g., as described herein). In embodiments the first and second epitopes are on the same antigen, e.g., the same protein (or subunit of a multimeric protein). In embodiments the first and second epitopes overlap. In embodiments the first and second epitopes do not overlap. In embodiments the first and second epitopes are on different antigens, e.g., different proteins (or different subunits of a multimeric protein). In embodiments a bispecific antibody molecule or bispecific antibody-like molecule comprises a heavy chain variable domain sequence and a light chain variable domain sequence which have binding specificity for a first epitope or antigen, and a heavy chain variable domain sequence and a light chain variable domain sequence which have binding specificity for a second epitope or antigen. In embodiments a bispecific antibody molecule or antibody-like molecule comprises a half antibody having binding specificity for a first epitope or antigen, and a half antibody having binding specificity for a second epitope or antigen. In an embodiment a bispecific antibody molecule or antibody-like molecule comprises a half antibody, or fragment thereof, having binding specificity for a first epitope or antigen, and a half antibody, or fragment thereof, having binding specificity for a second epitope or antigen. In embodiments a bispecific antibody molecule or bispecific antibody-like molecule comprises a scFv, or fragment thereof, having binding specificity for a first epitope or antigen and a scFv, or fragment thereof, have binding specificity for a second epitope or antigen.

In certain embodiments, the antibody or antibody-like molecule is a multispecific (*e.g.,* a bispecific or a trispecific) antibody or antibody-like molecule. Protocols for *g*enerating bispecific or heterodimeric antibody or antibody-like molecules are known in the art; including but not limited to, for example, the "knob in a hole" approach described in, *e.g.,* US 5731168; the electrostatic steering Fc pairing as described in, *e.g.,* WO 09/089004, WO 06/106905 and WO 2010/129304; Strand Exchange Engineered Domains (SEED) heterodimer formation as described in, *e.g.,* WO 07/110205; Fab arm exchange as described in, *e.g.,* WO 08/119353, WO 2011/131746, and WO 2013/060867; double antibody conjugate, *e.g.*, by antibody cross-linking to generate a bi-specific structure using a heterobifunctional reagent having an amine-reactive group and a sulfhydryl reactive group as described in, *e.g.,* US 4433059; bispecific antibody or antibody-like molecule determinants generated by recombining half antibodies (heavy-light chain pairs or Fabs) from different antibodies or antibody-like molecules through cycle of reduction and oxidation of disulfide bonds between the two heavy chains, as described in, *e.g.,* US 4444878; trifunctional antibodies, *e.g.*, three Fab' fragments cross-linked through sulfhdryl reactive groups, as described in, *e.g.,* US5273743; biosynthetic binding proteins, *e.g.,* pair of scFvs cross-linked through C-terminal tails preferably through disulfide or amine-reactive chemical cross-linking, as described in, *e.g.,* US5534254; bifunctional antibodies, *e.g.,* Fab fragments with different binding specificities dimerized through leucine zippers (*e.g.,* c-fos and c-jun) that have replaced the constant domain, as described in, *e.g.*, US5582996; bispecific and oligospecific mono-and oligovalent receptors, *e.g.*, VH-CH1 regions of two antibodies (two Fab fragments) linked through a polypeptide spacer between the CH1 region of one antibody and the VH region of the other antibody typically with associated light chains, as described in, *e.g.,* US5591828; bispecific DNA-antibody conjugates, *e.g.,* crosslinking of antibodies or Fab fragments through a double stranded piece of DNA, as described in, *e.g.,* US5635602; bispecific fusion proteins, *e.g.*, an expression construct containing two scFvs with a hydrophilic helical peptide linker between them and a full constant region, as described in, *e.g.,* US5637481; multivalent and multispecific binding proteins, *e.g.,* dimer of polypeptides having first domain with binding region of Ig heavy chain variable region, and second domain with binding region of Ig light chain variable region, generally termed diabodies (higher order structures are also encompassed creating for bispecific, trispecific, or tetraspecific molecules, as described in, *e.g.*, US5837242; minibody constructs with linked VL and VH chains further connected with peptide spacers to an antibody hinge region and CH3 region, which can be dimerized to form bispecific/multivalent molecules, as described in, *e.g.,* US5837821; VH and VL domains linked with a short peptide linker (*e.g.,* 5 or 10 amino acids) or no linker at all in either orientation, which can form dimers to form bispecific diabodies; trimers and tetramers, as described in, *e.g.,* US5844094; String of VH domains (or VL domains in family members) connected by peptide linkages with crosslinkable groups at the C-terminus further associated with VL domains to form a series of FVs (or scFvs), as described in, *e.g.,* US5864019; VL and VH domains, scFvs, or Fabs wherein one of the antigens is bound monovalently and one of the antigens is bound bivalently, optionally comprising heterodimeric Fc regions, as described in, e.g., WO2011/028952; and single chain binding polypeptides with both a VH and a VL domain linked through a peptide linker are combined into multivalent structures through non-covalent or chemical crosslinking to form, *e.g.*, homobivalent, heterobivalent, trivalent, and tetravalent structures using both scFv or diabody type format, as described in, *e.g.*, US5869620. Additional exemplary multispecific and bispecific molecules and methods of making the same are found, for example, in US5910573, US5932448, US5959083, US5989830, US6005079, US6239259, US6294353, US6333396, US6476198, US6511663, US6670453, US6743896, US6809185, US6833441, US7129330, US7183076, US7521056, US7527787, US7534866, US7612181, US2002004587A1, US2002076406A1, US2002103345A1, US2003207346A1, US2003211078A1, US2004219643A1, US2004220388A1, US2004242847A1, US2005003403A1, US2005004352A1, US2005069552A1, US2005079170A1, US2005100543A1, US2005136049A1, US2005136051A1, US2005163782A1, US2005266425A1, US2006083747A1, US2006120960A1, US2006204493A1, US2006263367A1, US2007004909A1, US2007087381A1, US2007128150A1, US2007141049A1, US2007154901A1, US2007274985A1, US2008050370A1, US2008069820A1, US2008152645A1, US2008171855A1, US2008241884A1, US2008254512A1, US2008260738A1, US2009130106A1, US2009148905A1, US2009155275A1, US2009162359A1, US2009162360A1, US2009175851A1, US2009175867A1, US2009232811A1, US2009234105A1, US2009263392A1, US2009274649A1, EP346087A2, WO0006605A2, WO02072635A2, WO04081051A1, WO06020258A2, WO2007044887A2, WO2007095338A2, WO2007137760A2, WO2008119353A1, WO2009021754A2, WO2009068630A1, WO9103493A1, WO9323537A1, WO9409131A1, WO9412625A2, WO9509917A1, WO9637621A2, WO9964460A1. The contents of the above-referenced applications are incorporated herein by reference in their entireties. Accordingly, in some embodiments, the anti-CLL-1 multispecific molecules of the present invention comprises an anti-CLL-1 binding domain in any one of the multispecific or bispecific formats known in the art and described above. Additional formats contemplated herein are described in more detail below.

Within each antibody or antibody fragment (e.g., scFv) of a bispecific antibody or antibody-like molecule, the VH can be upstream or downstream of the VL. In some embodiments, the upstream antibody or antibody fragment (e.g., scFv) is arranged with its VH (VH₁) upstream of its VL (VL₁) and the downstream antibody or antibody fragment (e.g., scFv) is arranged with its VL (VL₂) upstream of its VH (VH₂), such that the overall bispecific antibody or antibody-like molecule has the arrangement VH₁-VL₁-VL₂-VH₂. In other embodiments, the upstream antibody or antibody fragment (e.g., scFv) is arranged with its VL (VLi) upstream of its VH (VHi) and the downstream antibody or antibody fragment (e.g., scFv) is arranged with its VH (VH₂) upstream of its VL (VL₂), such that the overall bispecific antibody or antibody-like molecule has the arrangement VL₁-VH₁-VH₂-VL₂. Optionally, a linker is disposed between the two antibodies or antibody fragments (e.g., scFvs), e.g., between VL₁ and VL₂ if the construct is arranged as VH₁-VL₁-VL₂-VH₂, or between VH₁ and VH₂ if the construct is arranged as VL₁-VH₁-VH₂-VL₂. The linker may be a linker as described herein, e.g., a (Gly₄-Ser)n linker, wherein n is 1, 2, 3, 4, 5, or 6, preferably 4 (SEQ ID NO: 26). In general, the linker between the two scFvs should be long enough to avoid mispairing between the domains of the two scFvs. Optionally, a linker is disposed between the VL and VH of the first scFv. Optionally, a linker is disposed between the VL and VH of the second scFv. In constructs that have multiple linkers, any two or more of the linkers can be the same or different. Accordingly, in some embodiments, the anti-CLL-1 bispecific molecule of the present invention comprises VLs, VHs, (e.g., VLs and/or VHs from any of the anti-CLL-1 binding domains described herein) and optionally one or more linkers in an arrangement as described herein, together with, for example, VLs, VHs targeting a second epitope or antigen (e.g., a second epitope or antigen described herein, e.g., CD3 or CD16).

The present invention includes multispecific molecules, comprising an anti-CLL-1 binding domain, of various formats. Preferred formats for the multispecific molecules of the present invention are described in more detail below.

### Format 1: Dualbody

In one aspect, the present invention includes a multispecific molecule comprising a first heavy chain and a first light chain that together comprise a first antigen binding domain, and a second heavy chain and a second light chain that together comprise a second antigen binding domain. In an aspect, the dualbody is formed from a half antibody having specificity for a first antigen or epitope and a half antibody have specificity for a second antigen or epitope.

This molecule comprises a first polypeptide comprising a VL, and optionally a CL, and a second polypeptide comprising a VH, CH1, hinge, CH2 and CH3 domain, wherein the first and second polypeptide comprise a first antigen binding domain; and a third polypeptide comprising a VL, and optionally a CL, a fourth polypeptide comprising a VH, CH1, hinge, CH2 and CH3 domain, wherein the third and fourth polypeptides comprise a second antigen binding domain. In one embodiment the dualbody comprises (preferably from N-terminus to C-terminus; numbers following the domain name refer to, e.g., a first ("1" or "-1") or second ("2" or "-2") copy or version of that domain in the dualbody)):
Polypeptide 1: VL1-CL1
Polypeptide 2: VH1-CH1-1-hinge1-CH2-1-CH3-1
Polypeptide 3: VL2-CL2
Polypeptide 4: VH2-CH1-2-hinge2-CH2-2-CH3-2

In embodiments, the CL1 and CL2 domains are derived from the same antibody type or class e.g., comprise the same sequence. In other embodiments, the CL1 and CL2 domains are derived from different antibody or chain types or classes and/or comprise different sequences, for example CL1 may be derived from a lamba antibody and CL2 may be derived from a kappa antibody chain. Without being bound by theory, it is believed that deriving CL domains from different antibody types favors correct pairing of VL and VH domains (e.g., VH1 with VL1 and VH2 with VL2) to form functional antigen binding domains. In embodiments, the CH1-1 and CH1-2 domains are derived from the same antibody type or class and/or comprise the same sequence. In embodiments, the CH1-1 and CH1-2 domains are derived from different antibody types or classes and/or comprise different sequences. In embodiments, the hinge-1 and hinge-2 domains are derived from the same antibody type or class and/or comprise the same sequence. In embodiments, the hinge-1 and hinge-2 domains are derived from different antibody types or classes and/or comprise different sequences. In embodiments, the CH2-1 and CH2-2 domains are derived from the same antibody type or class and/or comprise the same sequence. In embodiments, the CH2-1 and CH2-2 domains are derived from different antibody types or classes and/or comprise different sequences. In some aspects, the CH2 domains that comprise different sequences comprise one or more mutations to favor heterodimerization of the two polypeptide chains comprising the CH2 domains, relative to unmodified polypeptide chains. Mutations for favoring heterodimerization are described in detail below. In embodiments, the CH2-1 and CH2-2 domains are derived from the same antibody type or class and/or comprise the same sequence. In embodiments, the CH3-1 and CH3-2 domains are derived from different antibody types or classes and/or comprise different sequences. In some aspects, the CH3 domains that comprise different sequences comprise one or more mutations to favor heterodimerization of the two polypeptide chains comprising the CH3 domains, relative to unmodified polypeptide chains. Mutations for favoring heterodimerization are described in detail below.

In embodiments, the polypeptides of the dualbody may additionally comprise one or more additional antibody fragments, e.g., one or more additional variable or constant domains, to form an "extended" dualbody. In one aspect, each polypeptide of the dualbody may additionally comprise a second variable domain (preferably of the same type as the first variable domain of the chain), preferably disposed between the first variable domain and the first constant domain of said polypeptide (if present), or C-terminal to the first variable domain. For example, the first polypeptide of the dualbody may comprise, from N-terminus to C-terminus: VL1-VL1-CL1. The one or more additional variable domains may be the same or different than the first variable domain. In other embodiments, the "extended" dualbody comprises one or more additional antibody constant domains, e.g., one or more copies or versions of the constant domain already present in the polypeptide chain being extended. For example, the first polypeptide of the dualbody may comprise, from N-terminus to C-terminus: VL1-CL1-CL1.

In embodiments, the dualbody has the structure depicted in Figure 1A. In embodiments, the dualbody comprises a first polypeptide chain having a VL and CL domains, a second polypeptide chain having a VH, CH1, hinge, CH2 and CH3 domains (said first and second chains making up the first half antibody that comprises the first antigen-binding domain, e.g., an anti-CLL-1 binding domain, e.g., as described herein), a third chain having a VL and CL domains, and a fourth chain having a VH, CH1, hinge, CH2 and CH3 domains (said third and fourth chains making up the second half antibody that comprises the second antigen-binding domain, e.g., an anti-CD3 binding domain, e.g., as described herein). Heterodimerization of the first and second half antibodies (shown here, favored by optional heterodimerization mutations to the CH3 domains, e.g., as described herein, as well as a stabilizing cysteine bond, e.g., as described herein) yields the multispecific molecule.

One or more chains of the multispecific molecules of the dualbody format described above may further comprise another antigen recognition domain, e.g., an scFv. In one embodiment, the scFv is disposed at the C-terminus of the chain. In one embodiment, the scFv is disposed C-terminal to the most C-terminal domain of a polypeptide comprising a heavy chain constant domain, e.g., a CH2 domain or CH3 domain. In one embodiment, the scFv is disposed C-terminal to the CH3 domain of a one or more polypeptides of the dualbody or extended dualbody. The one or more scFvs may recognize the same or different antigen or epitope as that recognized by either the first or second antigen binding domain of the dualbody or extended dualbody. Where the one or more antigen binding domains, e.g., scFvs, recognizes an epitope or antigen identical to one of the first or second antigen binding domains, the molecule is multivalent, e.g., bivalent, with respect to that antigen or epitope. In one embodiment, the antigen recognition domains of the dualbody recognize one or more cancer antigens, e.g., CLL-1, and the additional antigen binding domain, e.g., scFv, recognizes an antigen or epitope of an immune effector cell, e.g., a T cell or NK cell, e.g., CD3, CD64 or CD16.

It will be appreciated by the skilled artisan that the dualbody or extended dualbody molecules of the present invention may be stabilized by covalently linking one or more of the polypeptide chains of the molecule. Linkers are known in the art at may be chemical or peptidic. The linking may be along the polypeptide backbone (e.g., terminus to terminus), amino acid side chain to side chain, or amino acid side chain to terminus.

In embodiments, CL1 and CL2 may be independently selected from :
(a) 

 (SEQ ID
NO: 502); and
(b) 
GQPKAAPSVTLFPPSSEELQANKATLVCLISDFYPGAVTVAWKADSSPVKAGVETTT
PSKQSNNKYAASSYLSLTPEQWKSHRSYSCQVTHEGSTVEKTVAPTECS (SEQ ID
NO: 503).
In a preferred embodiment, CL1 and CL2 are different (e.g., CL1 comprises SEQ ID NO: 502 and CL2 comprises SEQ ID NO: 503, or vice versa).

In embodiments, the heavy chain constant region of peptide 2 and peptide 4 comprise an amino acid sequence independently selected from:
(a) 
ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAP
ELLGGPSVFLFPPKPKDTLMISRTPEVTCVVVAVSHEDPEVKFNWYVDGVEVHNAK
TKPREEQYNSTYRVVSVLTVLHQDWLNGKEYKCKVSNKALAAPIEKTISKAKGQPR
EPQVCTLPPSREEMTKNQVSLSCAVKGFYPSDIAVEWESNGQPENNYKTTPPVLDSD
GSFFLVSKLTVDKSRWQQGNVFSCSVMHEALHNHYTQKSLSLSPGK (SEQ ID NO:
504); and
(b) ASTKGPSVFPLAPSSKSTSGGTAALGCLVKDYFPEPVTVSWNSGALTSGVHTFPAVL 
QSSGLYSLSSVVTVPSSSLGTQTYICNVNHKPSNTKVDKRVEPKSCDKTHTCPPCPAP
ELLGGPSVFLFPPKPKDTLMISRTPEVTCVVVAVSHEDPEVKFNWYVDGVEVHNAK
TKPREEQYNSTYRVVSVLTVLHQDWLNGKEYKCKVSNKALAAPIEKTISKAKGQPR
EPQVYTLPPCREEMTKNQVSLWCLVKGFYPSDIAVEWESNGQPENNYKTTPPVLDS
DGSFFLYSKLTVDKSRWQQGNVFSCSVMHEALHNHYTQKSLSLSPGK (SEQ ID NO:
505).
In embodiments, peptide 2 (e.g., the heavy chain constant region of peptide 2) comprises SEQ ID NO: 504, and peptide 4 (e.g., the heavy chain constant region of peptide 4) comprises SEQ ID NO: 505, or vice versa.

Dualbody formats are known in the art, and are additionally described in, for example, WO2008/119353 and WO2011/131746, the contents of which are hereby incorporated by reference in their entirety. Examples of the dualbody format, including the "extended" dualbody format, are shown in Figure 1.

### Format 2: scFv-Fc

In one aspect, the present invention includes a multispecific molecule comprising a first polypeptide comprising a first scFv, a hinge domain, a CH2 domain and preferably, a CH3 domain; and a second polypeptide comprising a second scFv (preferably recognizing a different antigen or epitope than the first scFv), a hinge domain, a CH2 domain, and preferably, a CH3 domain. In one embodiment, the scFv is disposed N-terminal to the hinge and constant domains. In another embodiment, the scFv domains are disposed C-terminal to the hinge and constant domains. In another embodiment, the first scFv domain is disposed N-terminal to the hinge and constant domains of the first polypeptide, and the second scFv domain is disposed C-terminal to the hinge and constant domains of the second polypeptide. In embodiments, regardless of the orientation between the scFv domain and the constant domains, the hinge domain is disposed between the scFv domain and the constant domains.

In some aspects, the CH2 domains and/or CH3 domains of the scFv-Fc multispecific molecule comprise one or more mutations to favor heterodimerization of the two polypeptide chains comprising the CH2 and or CH3 domains of the scFv-Fc, relative to unmodified polypeptide chains. Mutations for favoring heterodimerization are described in detail below.

In embodiments, the dualbody has the structure depicted in Figure IB. In embodiments, the scFv-Fc multispecific molecule comprises a first polypeptide chain comprising a scFv (e.g., the first antigen binding domain, e.g., an anti-CLL-1 binding domain, e.g., as described herein), a hinge, CH2 and CH3 domain (i.e., the first half antibody), and a second polypeptide chain consisting of a scFv (e.g., the second antigen binding domain, e.g., an anti-CD3 binding domain, e.g., as described herein), a hinge, a CH2 and a CH3 domain (e.g., the second half antibody). Heterodimerization of the first and second half antibodies (shown here, favored by optional heterodimerization mutations to the CH3 domains, e.g., as described herein, as well as a stabilizing cysteine bond, e.g., as described herein) yields the multispecific molecule.

One or more chains of the multispecific molecules of the scFv-Fc format described above may further comprise another antigen recognition domain, e.g., an scFv. In one embodiment, the scFv is disposed at the C-terminus of the chain. In one embodiment, the scFv is disposed C-terminal to the most C-terminal domain of a polypeptide comprising a heavy chain constant domain, e.g., a CH2 domain or CH3 domain. In one embodiment, the scFv is disposed C-terminal to the CH3 domain of a one or more polypeptides of the scFv-Fc. The one or more scFvs may recognize the same or different antigen or epitope as that recognized by either the first or second antigen binding domain of the scFv-Fc. Where the one or more antigen binding domains, e.g., scFvs, recognizes an epitope or antigen identical to one of the first or second antigen binding domains, the molecule is multivalent, e.g., bivalent, with respect to that antigen or epitope. In one embodiment, the antigen recognition domains of the scFv-Fc recognize one or more cancer antigens, e.g., CLL-1, and the additional antigen binding domain, e.g., scFv, recognizes an antigen or epitope of an immune effector cell, e.g., a T cell or NK cell, e.g., CD3, CD64 or CD16.

It will be appreciated by the skilled artisan that the scFv-Fc molecules of the present invention may be stabilized by covalently linking one or more of the polypeptide chains of the molecule. Linkers are known in the art at may be chemical or peptidic. The linking may be along the polypeptide backbone (e.g., terminus to terminus), amino acid side chain to side chain, or amino acid side chain to terminus.

In embodiments, the first polypeptide chain may include a first antigen-binding domain, e.g., an scFv, and a first heavy chain constant region having the following sequence: 
GGGGSDKTHTCPPCPAPELLGGPSVFLFPPKPKDTLMISRTPEVTCVVVAVSHEDPEV
KFNWYVDGVEVHNAKTKPREEQYNSTYRVVSVLTVLHQDWLNGKEYKCKVSNKA
LAAPIEKTISKAKGQPREPQVCTLPPSRDELTKNQVSLSCAVKGFYPSDIAVEWESNG
QPENNYKTTPPVLDSDGSFFLVSKLTVDKSRWQQGNVFSCSVMHEALHNHYTQKSL
SLSPGK (SEQ ID NO: 500);
 and the second polypeptide may include a second antigen-binding domain, e.g., an scFv, and a second heavy chain constant region having the following sequence:

GGGGSDKTHTCPPCPAPELLGGPSVFLFPPKPKDTLMISRTPEVTCVVVAVSHEDPEV
KFNWYVDGVEVHNAKTKPREEQYNSTYRVVSVLTVLHQDWLNGKEYKCKVSNKA
LAAPIEKTISKAKGQPREPQVYTLPPCRDELTKNQVSLWCLVKGFYPSDIAVEWESN
GQPENNYKTTPPVLDSDGSFFLYSKLTVDKSRWQQGNVFSCSVMHEALHNHYTQKS
LSLSPGK (SEQ ID NO: 501).

Each of the first and/or second polypeptides may comprise additional amino acid residues disposed between the antigen-binding domain and the constant region, e.g., a hinge. Alternatively, the amino acids of the antigen-binding domain may be directly connected to the amino acids of the constant domain (e.g., SEQ ID NO: 500 and/or SEQ ID NO: 501) without intervening amino acids.

Alternatively, the attachment of the constant domains may be reversed: first polypeptide may comprise SEQ ID NO: 501 and the second polypeptide may comprise SEQ ID NO: 500.

Examples of the scFv-Fc format are shown in Figure 1.

### Format 3: Mixed Chain Multispecific Molecules

In one aspect, the present invention includes a multispecific molecule comprising a first half antibody comprising a first polypeptide comprising a first scFv, a hinge domain, a CH2 domain and preferably, a CH3 domain; and a second half antibody comprising two polypeptides, the first comprising a VL domain and optionally, a CL domain, and the second comprising a VH domain, a CH1 domain, a hinge domain, and a CH2 domain and/or a CH3 domain. Thus, the mixed chain multispecific molecular format includes a half antibody derived from a typical antibody structure heterodimerized with a half antibody of the scFv-Fc format, described above.

In embodiments, the mixed format multispecific molecule has the structure depicted in Figure 1C. In embodiments, the mixed format multispecific molecule comprises a first half antibody comprising a first polypeptide chain having a VL and CL domains and a second polypeptide chain having a VH, CH1, hinge, CH2 and CH3 domains (said VL and VH domains of the first and second polypeptide chains, respectively, making up the first antigen binding domain, e.g., an anti-CLL-1 binding domain, e.g., as described herein), and a second half antibody comprising a third polypeptide chain having a scFv (comprising the second antigen binding domain, e.g., an anti-CD3 binding domain, e.g., as described herein), hinge, CH2 and CH3 domain. Heterodimerization of the first and second half antibodies (shown here, favored by optional heterodimerization mutations to the CH3 domains, e.g., as described herein, as well as a stabilizing cysteine bond, e.g., as described herein) yields the multispecific molecule.

In some aspects, the CH2 domains and/or CH3 domains of the mixed chain multispecific molecule comprise one or more mutations to favor heterodimerization of the two polypeptide chains comprising the CH2 and or CH3 domains of the mixed chain multispecific molecule, relative to unmodified polypeptide chains. Mutations for favoring heterodimerization are described in detail below.

One or more chains of the multispecific molecules of the mixed chain multispecific format described above may further comprise another antigen recognition domain, e.g., an scFv. In one embodiment, the scFv is disposed at the C-terminus of the chain. In one embodiment, the scFv is disposed C-terminal to the most C-terminal domain of a polypeptide comprising a heavy chain constant domain, e.g., a CH2 domain or CH3 domain. In one embodiment, the scFv is disposed C-terminal to the CH3 domain of a one or more polypeptides of the mixed chain multispecific molecule. The one or more scFvs may recognize the same or different antigen or epitope as that recognized by either the first or second antigen binding domain of the mixed chain multispecific molecule. Where the one or more antigen binding domains, e.g., scFvs, recognizes an epitope or antigen identical to one of the first or second antigen binding domains, the molecule is multivalent, e.g., bivalent, with respect to that antigen or epitope. In one embodiment, the antigen recognition domains of the mixed chain multispecific molecule recognize one or more cancer antigens, e.g., CLL-1, and the additional antigen binding domain, e.g., scFv, recognizes an antigen or epitope of an immune effector cell, e.g., a T cell or NK cell, e.g., CD3, CD64 or CD16.

It will be appreciated by the skilled artisan that the mixed chain multispecific molecules of the present invention may be stabilized by covalently linking one or more of the polypeptide chains of the molecule. Linkers are known in the art at may be chemical or peptidic. The linking may be along the polypeptide backbone (e.g., terminus to terminus), amino acid side chain to side chain, or amino acid side chain to terminus.

In embodiments, either half of the mixed format molecule may comprise one or more of the constant region sequences described above in the sections on scFv-Fc or dualbodies. In a preferred embodiment, the half of the mixed format molecule comprising the scFv comprises a constant region sequence described above in the section on scFv-Fc, and the half of the mixed format molecule comprising separate light chain and heavy chain polypeptides comprises constant regions sequences described above in the section on dualbodies.

Examples of the mixed chain multispecific molecule format are shown in Figure 1.

### Format 4: Tandem scFv

In one aspect, the present invention includes a multispecific molecule comprising single polypeptide comprising two or more scFv domains, linked by a suitable linker. By way of example, the polypeptide of the tandem scFv comprises, from N-terminus to C-terminus: VL1-linker-VH1-linker2-VH2-linker3-VL2. By way of example, the polypeptide of the tandem scFv comprises, from N-terminus to C-terminus: VL1-linker-VH1-linker2-VL2-linker3-VH2. The linkers may be the same or different.

The scFv domains of the tandem scFv may additionally be separated by a molecule which adds improved stability to the construct, for example, a human serum albumin protein or fragment thereof.

In embodiments, the multispecific Tandem scFv has the structure depicted in Figure ID. In embodiments, the tandem scFv multispecific molecule comprises a first scFv (comprising the first antigen-binding domain, e.g., an anti-CLL-1 binding domain, e.g., as described herein) and a second scFv (comprising the second antigen-binding domain, e.g., an anti-CD3 binding domain, e.g., as described herein), connected by a linker.

### Examples of CD3 x CLL-1 Multispecific (bispecific) Antibodies

The antibodies in the table below are examples of CD3 x CLL-1 bispecific antibodies. These bispecific antibodies were generated in a scFv-Fc format, with a CD3 binding arm in the scFv-Fc format pairing with a CLL-1 binding arm in scFv-Fc format via knob and hole mutations to favor heterodimerization of the Fc domains.

**Table 11. Examplary CD3 x CLL-1 bispecific antibodies. For each of the CLL-1xCD3 bispecific molecules, the recited CLL-1 scFv-Fc was paired with the anti-CD3-Fc (SEQ ID NO: 507) to form the bispecific molecule.**

| **Bispecific Antibody Chain** | **SEQ ID NO:** | |
|---|---|---|
| **CD3 Binding Domain, and Exemplary scFv-Fc Chain** | | |
| CD3 VL | 1209 | |
| CD3 VH | 1236 | QVQLVQSGGGVVQPGRSLRLSCKASGYTFTRYTMHWVR QAPGKGLEWIGYINPSRGYTNYNQKVKDRFTISRDNSKN TAFLQMDSLRPEDTGVYFCARYYDDHYSLDYWGQGTPV TVSS |
| CD3 scFv | 506 | QVQLVQSGGGVVQPGRSLRLSCKASGYTFTRYTMHWVR QAPGKGLEWIGYINPSRGYTNYNQKVKDRFTISRDNSKN TAFLQMDSLRPEDTGVYFCARYYDDHYSLDYWGQGTPV TVSSGGGGSGGGKSKKGGSGGGGSDIQMTQSPSSLSASV GDRVTITCSASSSVSYMNWYQQTPGKAPKRWIYDTSKLA SGVPSRFSGSGSGTDYTFTISSLQPEDIATYYCQQWSSNPF TFGQGTKLQIT |
| CD3 scFv-Fc | 507 | QVQLVQSGGGVVQPGRSLRLSCKASGYTFTRYTMHWVR QAPGKGLEWIGYINPSRGYTNYNQKVKDRFTISRDNSKN TAFLQMDSLRPEDTGVYFCARYYDDHYSLDYWGQGTPV TVSSGGGGSGGGKSKKGGSGGGGSDIQMTQSPSSLSASV GDRVTITCSASSSVSYMNWYQQTPGKAPKRWIYDTSKLA SGVPSRFSGSGSGTDYTFTISSLQPEDIATYYCQQWSSNPF TFGQGTKLQITGGGGSDKTHTCPPCPAPELLGGPSVFLFPP KPKDTLMISRTPEVTCVVVAVSHEDPEVKFNWYVDGVE VHNAKTKPREEQYNSTYRVVSVLTVLHQDWLNGKEYKC KVSNKALAAPIEKTISKAKGQPREPQVCTLPPSRDELTKN QVSLSCAVKGFYPSDIAVEWESNGQPENNYKTTPPVLDS DGSFFLVSKLTVDKSRWQQGNVFSCSVMHEALHNHYTQ KSLSLSPGK |
| CD3 VL DNA | 508 | gacattcagatgactcagagcccaagctccctctccgcctccgtgggtgatcgcgtgacca ttacttgctccgcctcgtcatccgtgtcatacatgaactggtatcagcagacccccggaaag gccccgaagcgctggatctacgacacctccaagctggcttccggcgtgcctagccggttc agcggaagcggttccgggaccgactacacttttaccatttcctccctgcaacccgaggaca tcgcgacgtattactgccagcagtggtcctccaaccccttcaccttcggacagggtacaaa gctgcagatcacc |
| CD3 VH DNA | 509 | caagtgcagttggtgcagtccggtggtggagtggtccagccgggcagatcactgaggctt agctgcaaggcatccgggtacaccttcacccggtacactatgcactgggtccgccaagcc ccgggaaaaggactggaatggatcggctacatcaacccatcgagagggtacaccaacta caatcagaaggtcaaggaccggttcactatctcgagggacaactcaaagaacaccgcgtt cctgcaaatggattcgctgcggccggaggacaccggggtgtacttctgtgcccggtactac gatgaccactactctctggactactggggccagggcactcctgtgaccgtgtcc |
| CD3 scFv DNA | 510 | caagtgcagttggtgcagtccggtggtggagtggtccagccgggcagatcactgaggctt agctgcaaggcatccgggtacaccttcacccggtacactatgcactgggtccgccaagcc ccgggaaaaggactggaatggatcggctacatcaacccatcgagagggtacaccaacta caatcagaaggtcaaggaccggttcactatctcgagggacaactcaaagaacaccgcgtt cctgcaaatggattcgctgcggccggaggacaccggggtgtacttctgtgcccggtactac gatgaccactactctctggactactggggccagggcactcctgtgaccgtgtcctcgggg gaggaggaagcggcggaggaaaatccaagaagggcggcagcgggggcggaggctc ggacattcagatgactcagagcccaagctccctctccgcctccgtgggtgatcgcgtgacc attacttgctccgcctcgtcatccgtgtcatacatgaactggtatcagcagacccccggaaa ggccccgaagcgctggatctacgacacctccaagctggcttccggcgtgcctagccggtt cagcggaagcggttccgggaccgactacacttttaccatttcctccctgcaacccgaggac atcgcgacgtattactgccagcagtggtcctccaaccccttcaccttcggacagggtacaaa gctgcagatcacc |
| CD3 scFv-Fc DNA | 511 | caagtgcagttggtgcagtccggtggtggagtggtccagccgggcagatcactgaggctt agctgcaaggcatccgggtacaccttcacccggtacactatgcactgggtccgccaagcc ccgggaaaaggactggaatggatcggctacatcaacccatcgagagggtacaccaacta caatcagaaggtcaaggaccggttcactatctcgagggacaactcaaagaacaccgcgtt cctgcaaatggattcgctgcggccggaggacaccggggtgtacttctgtgcccggtactac gatgaccactactctctggactactggggccagggcactcctgtgaccgtgtcctcggggg gaggaggaagcggcggaggaaaatccaagaagggcggcagcgggggcggaggctc ggacattcagatgactcagagcccaagctccctctccgcctccgtgggtgatcgcgtgacc attacttgctccgcctcgtcatccgtgtcatacatgaactggtatcagcagacccccggaaa ggccccgaagcgctggatctacgacacctccaagctggcttccggcgtgcctagccggtt cagcggaagcggttccgggaccgactacactttaccatttcctccctgcaacccgaggac atcgcgacgtattactgccagcagtggtcctccaaccccttcaccttcggacagggtacaaa gctgcagatcaccggagggggcggatccgacaagacccacacctgtcctccttgtcctgc cccggaactgctgggcggccccagcgtgttcctgttcccgccgaagcctaaggatactctc atgatcagcaggacgcctgaagtgacctgtgtcgtggtggccgtgtcccatgaagatccag aagtcaagttcaattggtacgtggacggcgtggaggtgcacaacgccaagacaaagccta gagaggaacagtacaacagcacctaccgcgtcgtgtccgtgctgaccgtgctgcaccag gactggctgaacgggaaggagtacaagtgcaaagtgtccaacaaggccctggccgcccc aattgaaaagactatctccaaggccaagggccagccccgcgagccccaggtgtgcactct gccgccgtcaagagatgaactgactaagaaccaggtgtcactgtcctgcgccgtgaaagg gttctacccctccgacatcgccgtggagtgggaaagcaacggacagcctgaaaacaacta caaaacgactcccccctgtgctcgactccgatggctcgttcttcttggtgtcgaagctcaccgt ggataagagccggtggcaacagggaaacgtgttttcctgctccgtgatgcatgaggccctc cacaaccactacacccagaaatccctctccctgtcgccggggaag |

| **CD3 x CLL1_11** | | |
|---|---|---|
| CLL1_ 11 VL | 88 | EIVLTQSPSSLSASVGDRVTITCQASQFIKKNLNWYQHKP GKAPKLLIYDASSLQTGVPSRFSGNRSGTTFSFTISSLQPE DVATYYCQQHDNLPLTFGGGTKVEIK |
| CLL1_ 11 VH | 75 | EVQLVQSGGGVVRSGRSLRLSCAASGFTFNSYGLHWVR QAPGKGLEWVALIEYDGSNKYYGDSVKGRFTISRDKSKS TLYLQMDNLRAEDTAVYYCAREGNEDLAFDIWGQGTLV TVSS |
| CLL1_11 scFv | 49 | EVQLVQSGGGVVRSGRSLRLSCAASGFTFNSYGLHWVR QAPGKGLEWVALIEYDGSNKYYGDSVKGRFTISRDKSKS TLYLQMDNLRAEDTAVYYCAREGNEDLAFDIWGQGTLV TVSSGGGGSGGGGSGGGGSGGGGSEIVLTQSPSSLSASVG DRVTITCQASQFIKKNLNWYQHKPGKAPKLLIYDASSLQT GVPSRFSGNRSGTTFSFTISSLQPEDVATYYCQQHDNLPLT FGGGTKVEIK |
| CLL1_11 scFv-Fc | 512 | EVQLVQSGGGVVRSGRSLRLSCAASGFTFNSYGLHWVR QAPGKGLEWVALIEYDGSNKYYGDSVKGRFTISRDKSKS TLYLQMDNLRAEDTAVYYCAREGNEDLAFDIWGQGTLV TVSSGGGGSGGGGSGGGGSGGGGSEIVLTQSPSSLSASVG DRVTITCQASQFIKKNLNWYQHKPGKAPKLLIYDASSLQT GVPSRFSGNRSGTTFSFTISSLQPEDVATYYCQQHDNLPLT FGGGTKVEIKGGGGSGSDKTHTCPPCPAPELLGGPSVFLF PPKPKDTLMISRTPEVTCVVVAVSHEDPEVKFNWYVDGV EVHNAKTKPREEQYNSTYRVVSVLTVLHQDWLNGKEYK CKVSNKALAAPIEKTISKAKGQPREPQVYTLPPCRDELTK NQVSLWCLVKGFYPSDIAVEWESNGQPENNYKTTPPVLD SDGSFFLYSKLTVDKSRWQQGNVFSCSVMHEALHNHYT QKSLSLSPGK |
| CLL1_11 VL DNA | 513 | gaaattgtgctgacccagagcccatcgtcactgtccgcatccgtcggcgaccgcgtgacta tcacttgccaagcgtcccagttcatcaagaaaaatctgaactggtaccagcataagccggg gaaggcgcccaagctgctgatctacgacgcctcatccctccaaactggagtgcccagcag attcagcggaaaccggtccggaaccaccttctcgtttactatttcgagcctgcaacccgagg atgtggccacctactactgtcagcagcacgacaacttgcctctcaccttcggtggtggaacc aaagtggagattaag |
| CLL1_11 VH DNA | 514 | gaagtgcagctggtgcagtcgggtggcggagtggtccggtccgggcggtccctgcgcct gtcgtgcgctgcctccggcttcactttcaactcatacggactgcactgggtcagacaggccc cggggaagggactggaatgggtcgcgctcatcgaatacgatgggtccaacaagtattacg gcgacagcgtgaagggccggttcaccatctcccgcgacaagtccaagtcaaccctgtacc tccaaatggataacctgagggccgaggacaccgccgtgtactactgcgctcgggaaggg aacgaggacctggccttcgatatctggggccagggaaccctcgtgacggtgtccagc |
| CLL1_11 scFv DNA | 515 | gaagtgcagctggtgcagtcgggtggcggagtggtccggtccgggcggtccctgcgcct gtcgtgcgctgcctccggcttcactttcaactcatacggactgcactgggtcagacaggccc cggggaagggactggaatgggtcgcgctcatcgaatacgatgggtccaacaagtattacg gcgacagcgtgaagggccggttcaccatctcccgcgacaagtccaagtcaaccctgtacc tccaaatggataacctgagggccgaggacaccgccgtgtactactgcgctcgggaaggg aacgaggacctggccttcgatatctggggccagggaaccctcgtgacggtgtccagcgg cggcggtggaagcggcggtggcgggagcgggggaggaggatctggaggcggaggct ccgaaattgtgctgacccagagcccatcgtcactgtccgcatccgtcggcgaccgcgtga ctatcacttgccaagcgtcccagttcatcaagaaaaatctgaactggtaccagcataagccg gggaaggcgcccaagctgctgatctacgacgcctcatccctccaaactggagtgcccagc agattcagcggaaaccggtccggaaccaccttctcgtttactatttcgagcctgcaacccga ggatgtggccacctactactgtcagcagcacgacaacttgcctctcaccttcggtggtggaa ccaaagtggagattaag |
| CLL1_11 scFv-Fc DNA | 516 | gaagtgcagctggtgcagtcgggtggcggagtggtccggtccgggcggtccctgcgcct gtcgtgcgctgcctccggcttcactttcaactcatacggactgcactgggtcagacaggccc cgggggaagggactggaatgggtcgcgctcatcgaatacgatgggtccaacaagtattacg gcgacagcgtgaagggccggttcaccatctcccgcgacaagtccaagtcaaccctgtacc tccaaatggataacctgagggccgaggacaccgccgtgtactactgcgctcgggaaggg |
| | | aacgaggacctggccttcgatatctggggccagggaaccctcgtgacggtgtccagcgg cggcggtggaagcggcggtggcgggagcgggggaggaggatctggaggcggaggct ccgaaattgtgctgacccagagcccatcgtcactgtccgcatccgtcggcgaccgcgtga ctatcacttgccaagcgtcccagttcatcaagaaaaatctgaactggtaccagcataagccg gggaaggcgcccaagctgctgatctacgacgcctcatccctccaaactggagtgcccagc agattcagcggaaaccggtccggaaccaccttctcgtttactatttcgagcctgcaacccga ggatgtggccacctactactgtcagcagcacgacaacttgcctctcaccttcggtggtggaa ccaaagtggagattaagggtggcgggggatccggatccgataagacccacacctgtcca ccctgccctgcccccgaactgcttggtggtccgtccgtgtttctgttcccgcccaagcccaa ggacaccctcatgatctcacggactcctgaagtgacctgtgtggtggtcgctgtgtcccacg aggaccccgaagtcaagttcaattggtacgtggacggagtggaagtgcacaacgctaaga ccaagccccgcgaggaacagtacaactccacttaccgcgtcgtgtcggtgctgaccgtgc tgcatcaggattggctgaacggaaaggagtacaagtgcaaggtgtccaacaaggctctgg cggcacccatcgaaaagaccatcagcaaggccaaagggcaacctagagaaccacaagt ctacaccctgcctccttgccgggatgagctcaccaagaaccaggtgtccctgtggtgcctc gtgaagggcttctacccctctgacatcgcggtggaatgggagtcaaacggccagccagag aacaactacaagacaaccccccctgtcctggacagcgacggctccttcttcctgtactcgaa gctgactgtggataagagccggtggcaacagggcaacgtgttctcatgttcggtcatgcac gaggccctgcataaccactacactcagaagtccctgagcctgtcccctggaaag |

| **CD3 x CLL1_12** | | |
|---|---|---|
| CLL1 12 VL | 89 | DIQVTQSPSSLSASVGDRVTITCRASQSISSYLNWYQQKP GKAPKLLIYAASSLQSGVPSRFSGSGSGTDFTLTISSLQPE DFATYYCQQSYSTPPLTFGQGTKVEIK |
| CLL1 12 VH | 76 | QVQLVESGGGLVQPGGSLRLSCAASGFNVSSNYMTWVR QAPGKGLEWVSVIYSGGATYYGDSVKGRFTVSRDNSKN TVYLQMNRLTAEDTAVYYCARDRLYCGNNCYLYYYYG MDVWGQGTLVTVSS |
| CLL1_12 scFv | 50 | QVQLVESGGGLVQPGGSLRLSCAASGFNVSSNYMTWVR QAPGKGLEWVSVIYSGGATYYGDSVKGRFTVSRDNSKN TVYLQMNRLTAEDTAVYYCARDRLYCGNNCYLYYYYG MDVWGQGTLVTVSSGGGGSGGGGSGGGGSGGGGSDIQ VTQSPSSLSASVGDRVTITCRASQSISSYLNWYQQKPGKA PKLLIYAASSLQSGVPSRFSGSGSGTDFTLTISSLQPEDFAT YYCQQSYSTPPLTFGQGTKVEIK |
| CLL1 scFv-Fc | 517 | QVQLVESGGGLVQPGGSLRLSCAASGFNVSSNYMTWVR QAPGKGLEWVSVIYSGGATYYGDSVKGRFTVSRDNSKN TVYLQMNRLTAEDTAVYYCARDRLYCGNNCYLYYYYG MDVWGQGTLVTVSSGGGGSGGGGSGGGGSGGGGSDIQ VTQSPSSLSASVGDRVTITCRASQSISSYLNWYQQKPGKA PKLLIYAASSLQSGVPSRFSGSGSGTDFTLTISSLQPEDFAT YYCQQSYSTPPLTFGQGTKVEIKGGGGSGSDKTHTCPPCP APELLGGPSVFLFPPKPKDTLMISRTPEVTCVVVAVSHED PEVKFNWYVDGVEVHNAKTKPREEQYNSTYRVVSVLTV LHQDWLNGKEYKCKVSNKALAAPIEKTISKAKGQPREPQ VYTLPPCRDELTKNQVSLWCLVKGFYPSDIAVEWESNGQ PENNYKTTPPVLDSDGSFFLYSKLTVDKSRWQQGNVFSC SVMHEALHNHYTQKSLSLSPGK |
| CLL1_12 VL DNA | 518 | gatattcaggtcacccaatcgccgtcctccctgagcgcctccgtgggggatcgcgtgacga ttacttgccgggccagccagagcatctcctcgtacctgaactggtaccagcagaagccgg gaaaggcccccaagctgctgatctacgctgcatcaagcctgcagtccggcgtgcctagcc ggttttccggttccggttcgggtaccgacttcacactgaccatctcctcactgcaaccagag gatttcgccacctactactgtcagcagtcatactccactccgcccctgaccttcggacaagg gaccaaagtggaaatcaag |
| CLL1_12 VH DNA | 519 | caagtgcagctggtcgaatccgggggaggcctggtgcagcccggagggtcgctgaggc tgagctgcgcggcttccggcttcaatgtgtcctccaactacatgacctgggtcagacaggc ccctggaaagggactcgaatgggtgtcggtgatctactccggtggcgcaacctactatgga gacagcgtgaaggggcgcttcactgtgtcccgcgacaactccaagaacactgtgtaccttc agatgaacaggctcaccgccgaggacaccgccgtgtactactgcgcgcgggaccggctc tactgtggaaacaactgctatctgtactactactacgggatggacgtctggggccagggca ccctcgtgactgtgtcgtct |
| CLL1_12 scFv DNA | 520 | caagtgcagctggtcgaatccgggggaggcctggtgcagcccggagggtcgctgaggc tgagctgcgcggcttccggcttcaatgtgtcctccaactacatgacctgggtcagacaggc ccctggaaagggactcgaatgggtgtcggtgatctactccggtggcgcaacctactatgga gacagcgtgaaggggcgcttcactgtgtcccgcgacaactccaagaacactgtgtaccttc agatgaacaggctcaccgccgaggacaccgccgtgtactactgcgcgcgggaccggctc tactgtggaaacaactgctatctgtactactactacgggatggacgtctggggccagggca ccctcgtgactgtgtcgtctggaggaggcggtagcggtggaggcggctccggaggcgga ggctcgggagggggaggcagcgatattcaggtcacccaatcgccgtcctccctgagcgc ctccgtgggggatcgcgtgacgattacttgccgggccagccagagcatctcctcgtacctg aactggtaccagcagaagccgggaaaggcccccaagctgctgatctacgctgcatcaag cctgcagtccggcgtgcctagccggttttccggttccggttcgggtaccgacttcacactga ccatctcctcactgcaaccagaggatttcgccacctactactgtcagcagtcatactccactc cgcccctgaccttcggacaagggaccaaagtggaaatcaag |
| CLL1_12 scFv-Fc DNA | 521 | caagtgcagctggtcgaatccgggggaggcctggtgcagcccggagggtcgctgaggc tgagctgcgcggcttccggcttcaatgtgtcctccaactacatgacctgggtcagacaggc ccctggaaaggactcgaatgggtgtcggtgatctactccggtggcgcaacctactatgga gacagcgtgaaggggcgcttcactgtgtcccgcgacaactccaagaacactgtgtaccttc agatgaacaggctcaccgccgaggacaccgccgtgtactactgcgcgcgggaccggctc tactgtggaaacaactgctatctgtactactactacgggatggacgtctggggccagggca ccctcgtgactgtgtcgtctggaggaggcggtagcggtggaggcggctccggaggcgga ggctcgggagggggaggcagcgatattcaggtcacccaatcgccgtcctccctgagcgc ctccgtgggggatcgcgtgacgattacttgccgggccagccagagcatctcctcgtacctg aactggtaccagcagaagccgggaaaggcccccaagctgctgatctacgctgcatcaag cctgcagtccggcgtgcctagccggttttccggttccggttcgggtaccgacttcacactga ccatctcctcactgcaaccagaggatttcgccacctactactgtcagcagtcatactccactc cgcccctgaccttcggacaagggaccaaagtggaaatcaagggcggcggaggatccgg atccgataagacccacacctgtccaccctgccctgcccccgaactgcttggtggtccgtcc gtgtttctgttcccgcccaagcccaaggacaccctcatgatctcacggactcctgaagtgac ctgtgtggtggtcgctgtgtcccacgaggaccccgaagtcaagttcaattggtacgtggac ggagtggaagtgcacaacgctaagaccaagccccgcgaggaacagtacaactccactta ccgcgtcgtgtcggtgctgaccgtgctgcatcaggattggctgaacggaaaggagtacaa gtgcaaggtgtccaacaaggctctggcggcacccatcgaaaagaccatcagcaaggcca aagggcaacctagagaaccacaagtctacaccctgcctccttgccgggatgagctcacca agaaccaggtgtccctgtggtgcctcgtgaagggcttctacccctctgacatcgcggtgga atgggagtcaaacggccagccagagaacaactacaagacaaccccccctgtcctggaca gcgacggctccttcttcctgtactcgaagctgactgtggataagagccggtggcaacaggg caacgtgttctcatgttcggtcatgcacgaggccctgcataaccactacactcagaagtccc tgagcctgtcccctggaaag |

| **CD3 x CLL1_10** | | |
|---|---|---|
| CLL1_10 VL | 87 | DIVLTQSPSSLSASVGDRVTITCQASQDISNYLNWYQQKP GKAPKLLIYDASNLETGVPSRFSGSGSGTDFTFTISSLQPE DFATYYCQQAYSTPFTFGPGTKVEIK |
| CLL1_10 VH | 74 | QVQLVQSGGGLVQPGGSLRLSCAASGFTFSSYSMNWVR QAPGKGLEWVSYISSSSSTIYYADSVKGRFTISRDNAKNS LYLQMNSLRAEDTAVYYCARDLSVRAIDAFDIWGQGTM VTVSS |
| CLL1_10 scFv | 48 | QVQLVQSGGGLVQPGGSLRLSCAASGFTFSSYSMNWVR QAPGKGLEWVSYISSSSSTIYYADSVKGRFTISRDNAKNS LYLQMNSLRAEDTAVYYCARDLSVRAIDAFDIWGQGTM VTVSSGGGGSGGGGSGGGGSGGGGSDIVLTQSPSSLSASV GDRVTITCQASQDISNYLNWYQQKPGKAPKLLIYDASNL ETGVPSRFSGSGSGTDFTFTISSLQPEDFATYYCQQAYSTP FTFGPGTKVEIK |
| CLL1_10 scFv-Fc | 522 | QVQLVQSGGGLVQPGGSLRLSCAASGFTFSSYSMNWVR QAPGKGLEWVSYISSSSSTIYYADSVKGRFTISRDNAKNS LYLQMNSLRAEDTAVYYCARDLSVRAIDAFDIWGQGTM VTVSSGGGGSGGGGSGGGGSGGGGSDIVLTQSPSSLSASV GDRVTITCQASQDISNYLNWYQQKPGKAPKLLIYDASNL ETGVPSRFSGSGSGTDFTFTISSLQPEDFATYYCQQAYSTP FTFGPGTKVEIKGGGGSGSDKTHTCPPCPAPELLGGPSVF LFPPKPKDTLMISRTPEVTCVVVAVSHEDPEVKFNWYVD GVEVHNAKTKPREEQYNSTYRVVSVLTVLHQDWLNGKE YKCKVSNKALAAPIEKTISKAKGQPREPQVYTLPPCRDEL TKNQVSLWCLVKGFYPSDIAVEWESNGQPENNYKTTPPV LDSDGSFFLYSKLTVDKSRWQQGNVFSCSVMHEALHNH YTQKSLSLSPGK |
| CLL1_10 VL DNA | 523 | gacatcgtgttgacccagtctccttcgtccctgagcgcttccgtgggcgaccgcgtgaccat cacttgccaagcctcacaagatatctccaactacctcaattggtaccagcagaagccggga aaggcccccaagctgctcatctacgacgcctccaacctggaaactggagtcccgtcgagg ttttccggaagcggcagcggaaccgacttcaccttcaccattagcagcctgcagccagag gattttgcgacctattactgccagcaggcttactccacccccttcaccttcggacctggcacc aaggtcgaaatcaag |
| CLL1_10 VH DNA | 524 | caagtgcagctggtgcagtccggcggagggctggtgcagccgggcggttcgctgagact gtcctgtgccgcgtcgggcttcacgttctcgtcatactccatgaactgggtccgccaggccc ccggaaaaggcttggaatgggtgtcgtacatttcgtcctcctcctcaaccatctactacgccg actcagtgaagggggcggttcactatttcccgggacaacgccaagaacagcctgtacctcca aatgaactcactgcgggccgaggacactgcggtgtactactgcgcccgggacctgtccgt gagagcaattgacgcattcgatatctggggacagggaaccatggtcaccgtgtccagc |
| CLL1_10 scFv DNA | 525 | caagtgcagctggtgcagtccggcggagggctggtgcagccgggcggttcgctgagact gtcctgtgccgcgtcgggcttcacgttctcgtcatactccatgaactgggtccgccaggccc ccggaaaaggcttggaatgggtgtcgtacatttcgtcctcctcctcaaccatctactacgccg actcagtgaaggggcggttcactatttcccgggacaacgccaagaacagcctgtacctcca aatgaactcactgcgggccgaggacactgcggtgtactactgcgcccgggacctgtccgt gagagcaattgacgcattcgatatctggggacagggaaccatggtcaccgtgtccagcgg tggaggagggtcgggcggcggcggttcaggcggtggtggaagcggcgggggggggt ccgacatcgtgttgacccagtctccttcgtccctgagcgcttccgtgggcgaccgcgtgac catcacttgccaagcctcacaagatatctccaactacctcaattggtaccagcagaagccgg gaaaggcccccaagctgctcatctacgacgcctccaacctggaaactggagtcccgtcga ggttttccggaagcggcagcggaaccgacttcaccttcaccattagcagcctgcagccag aggattttgcgacctattactgccagcaggcttactccaccccttcaccttcggacctggca ccaaggtcgaaatcaag |
| CLL1_10 scFv-Fc DNA | 526 | caagtgcagctggtgcagtccggcggagggctggtgcagccgggcggttcgctgagact gtcctgtgccgcgtcgggcttcacgttctcgtcatactccatgaactgggtccgccaggccc ccggaaaaggcttggaatgggtgtcgtacatttcgtcctcctcctcaaccatctactacgccg actcagtgaagggggcggttcactatttcccgggacaacgccaagaacagcctgtacctcca aatgaactcactgcgggccgaggacactgcggtgtactactgcgcccgggacctgtccgt gagagcaattgacgcattcgatatctggggacagggaaccatggtcaccgtgtccagcgg tggaggagggtcgggcggcggcggttcaggcggtggtggaagcggcgggggggggt ccgacatcgtgttgacccagtctccttcgtccctgagcgcttccgtgggcgaccgcgtgac catcacttgccaagcctcacaagatatctccaactacctcaattggtaccagcagaagccgg |
| | | gaaaggcccccaagctgctcatctacgacgcctccaacctggaaactggagtcccgtcga ggttttccggaagcggcagcggaaccgacttcaccttcaccattagcagcctgcagccag aggattttgcgacctattactgccagcaggcttactccaccccttcaccttcggacctggca ccaaggtcgaaatcaagggcggagggggatccggatccgataagacccacacctgtcca ccctgccctgcccccgaactgcttggtggtccgtccgtgtttctgttcccgcccaagcccaa ggacaccctcatgatctcacggactcctgaagtgacctgtgtggtggtcgctgtgtcccacg aggaccccgaagtcaagttcaattggtacgtggacggagtggaagtgcacaacgctaaga ccaagccccgcgaggaacagtacaactccacttaccgcgtcgtgtcggtgctgaccgtgc tgcatcaggattggctgaacggaaaggagtacaagtgcaaggtgtccaacaaggctctgg cggcacccatcgaaaagaccatcagcaaggccaaagggcaacctagagaaccacaagt ctacaccctgcctccttgccgggatgagctcaccaagaaccaggtgtccctgtggtgcctc gtgaagggcttctacccctctgacatcgcggtggaatgggagtcaaacggccagccagag aacaactacaagacaacccccccctgtcctggacagcgacggctccttcttcctgtactcgaa gctgactgtggataagagccggtggcaacagggcaacgtgttctcatgttcggtcatgcac gaggccctgcataaccactacactcagaagtccctgagcctgtcccctggaaag |

| **CD3 x CLL1_08** | | |
|---|---|---|
| CLL1 8 VL | 85 | EIVLTQSPGTLSLSPGGRATLSCRASQSISGSFLAWYQQKP GQAPRLLIYGASSRATGIPDRFSGSGSGTDFTLTISRLEPED FAVYYCQQYGSSPPTFGLGTKLEIK |
| CLL1 8 VH | 72 | QVQLVESGGGLVQPGGSLRLSCAASGFTFSSYWMSWVR QAPGKGLEWVANINEDGSAKFYVDSVKGRFTISRDNAKN SLYLQMNSLRAEDTAVYFCARDLRSGRYWGQGTLVTVS S |
| CLL1_8 scFv | 46 | QVQLVESGGGLVQPGGSLRLSCAASGFTFSSYWMSWVR QAPGKGLEWVANINEDGSAKFYVDSVKGRFTISRDNAKN SLYLQMNSLRAEDTAVYFCARDLRSGRYWGQGTLVTVS SGGGGSGGGGSGGGGSEIVLTQSPGTLSLSPGGRATLSCR ASQSISGSFLAWYQQKPGQAPRLLIYGASSRATGIPDRFSG SGSGTDFTLTISRLEPEDFAVYYCQQYGSSPPTFGLGTKLE IK |
| CLL1_8 scFv-Fc | 527 | QVQLVESGGGLVQPGGSLRLSCAASGFTFSSYWMSWVR QAPGKGLEWVANINEDGSAKFYVDSVKGRFTISRDNAKN SLYLQMNSLRAEDTAVYFCARDLRSGRYWGQGTLVTVS SGGGGSGGGGSGGGGSEIVLTQSPGTLSLSPGGRATLSCR ASQSISGSFLAWYQQKPGQAPRLLIYGASSRATGIPDRFSG SGSGTDFTLTISRLEPEDFAVYYCQQYGSSPPTFGLGTKLE IKGGGGSGSDKTHTCPPCPAPELLGGPSVFLFPPKPKDTL MISRTPEVTCVVVAVSHEDPEVKFNWYVDGVEVHNAKT KPREEQYNSTYRVVSVLTVLHQDWLNGKEYKCKVSNKA LAAPIEKTISKAKGQPREPQVYTLPPCRDELTKNQVSLWC LVKGFYPSDIAVEWESNGQPENNYKTTPPVLDSDGSFFLY SKLTVDKSRWQQGNVFSCSVMHEALHNHYTQKSLSLSP GK |
| CLL1_8 VL DNA | 528 | gaaattgtgctgacccaatcgcccggaactctgtccctgtcccccggtggacgcgccactc tctcttgccgggcctcacagtcgatctcgggcagctttctcgcctggtaccagcagaagcc gggacaggcgcctcgcctgctgatctacggagcgtccagcagagccaccggaatcccag acagattctccggctcgggctccggtaccgactttacgctgactattagccggctggagcc ggaggacttcgccgtgtactactgtcagcagtacggcagctcaccgcctaccttcggactc gggacaaagctggaaatcaag |
| CLL1_8 VH DNA | 529 | caagtgcagctggtcgaatccggcggagggctggtgcagccgggagggtcactccggct gtcctgcgccgcatcaggattcaccttctcctcctactggatgtcctgggtccgccaggctc ccgggaagggtctggaatgggtggccaacatcaacgaggacggctccgccaagttctac gtggatagcgtgaaaggaaggttcaccatttcccgggacaacgccaagaacagcctctat |
| | | ctgcaaatgaatagcctgagggcagaagataccgcggtgtacttctgcgctcgggacctg agatccggccgctactggggccaggggaccctggtcaccgtgtcc |
| CLL1_8 scFv DNA | 531 | caagtgcagctggtcgaatccggcggagggctggtgcagccgggagggtcactccggct gtcctgcgccgcatcaggattcaccttctcctcctactggatgtcctgggtccgccaggctc ccgggaagggtctggaatgggtggccaacatcaacgaggacggctccgccaagttctac gtggatagcgtgaaaggaaggttcaccatttcccgggacaacgccaagaacagcctctat ctgcaaatgaatagcctgagggcagaagataccgcggtgtacttctgcgctcgggacctg agatccggccgctactggggccaggggaccctggtcaccgtgtcctcgggagggggcg gctccggtggtggagggagcggcggaggagggtccgaaattgtgctgacccaatcgccc ggaactctgtccctgtcccccggtggacgcgccactctctcttgccgggcctcacagtcgat ctcgggcagctttctcgcctggtaccagcagaagccgggacaggcgcctcgcctgctgat ctacggagcgtccagcagagccaccggaatcccagacagattctccggctcgggctccg gtaccgactttacgctgactattagccggctggagccggaggacttcgccgtgtactactgt cagcagtacggcagctcaccgcctaccttcggactcgggacaaagctggaaatcaag |
| CLL1_8 scFv-Fc DNA | 531 | caagtgcagctggtcgaatccggcggagggctggtgcagccgggagggtcactccggct gtcctgcgccgcatcaggattcaccttctcctcctactggatgtcctgggtccgccaggctc ccgggaagggtctggaatgggtggccaacatcaacgaggacggctccgccaagttctac gtggatagcgtgaaaggaaggttcaccatttcccgggacaacgccaagaacagcctctat ctgcaaatgaatagcctgagggcagaagataccgcggtgtacttctgcgctcgggacctg agatccggccgctactggggccaggggaccctggtcaccgtgtcctcgggagggggcg gctccggtggtggagggagcggcggaggagggtccgaaattgtgctgacccaatcgccc ggaactctgtccctgtcccccggtggacgcgccactctctcttgccgggcctcacagtcgat ctcgggcagctttctcgcctggtaccagcag aagccgggacaggcgcctcgcctgctgat ctacggagcgtccagcagagccaccggaatcccagacagattctccggctcgggctccg gtaccgactttacgctgactattagccggctggagccggaggacttcgccgtgtactactgt cagcagtacggcagctcaccgcctaccttcggactcgggacaaagctggaaatcaaggg aggcggcggatccggatccgataagacccacacctgtccaccctgccctgcccccgaact gcttggtggtccgtccgtgtttctgttcccgcccaagcccaaggacaccctcatgatctcac ggactcctgaagtgacctgtgtggtggtcgctgtgtcccacgaggaccccgaagtcaagtt caattggtacgtggacggagtggaagtgcacaacgctaagaccaagccccgcgaggaac agtacaactccacttaccgcgtcgtgtcggtgctgaccgtgctgcatcaggattggctgaac ggaaaggagtacaagtgcaaggtgtccaacaaggctctggcggcacccatcgaaaagac catcagcaaggccaaagggcaacctagagaaccacaagtctacaccctgcctccttgccg ggatgagctcaccaagaaccaggtgtccctgtggtgcctcgtgaagggcttctacccctct gacatcgcggtggaatgggagtcaaacggccagccagagaacaactacaagacaaccc cccctgtcctggacagcgacggctccttcttcctgtactcgaagctgactgtggataagagc cggtggcaacagggcaacgtgttctcatgttcggtcatgcacgaggccctgcataaccact acactcagaagtccctgagcctgtcccctggaaag |

| CD3 x CLL1_09 | | |
|---|---|---|
| CLL1_9 VL | 86 | DIVMTQSPSSVSASVGDRVTITCRASQDISSWLAWYQQKP GKAPKLLIYAASSLQSGVPSRFNGSGSGTDFTLTISSLQPE DFATYYCQQSYSTPLTFGGGTKVEIK |
| CLL1_9 VH | 73 | QVQLVQSGAEVKEPGASVKVSCKAPANTFSDHVMHWV RQAPGQRFEWMGYIHAANGGTHYSQKFQDRVTITRDTS ANTVYMDLSSLRSEDTAVYYCARGGYNSDAFDIWGQGT MVTVSS |
| CLL1_9 scFv | 47 | QVQLVQSGAEVKEPGASVKVSCKAPANTFSDHVMHWV RQAPGQRFEWMGYIHAANGGTHYSQKFQDRVTITRDTS ANTVYMDLSSLRSEDTAVYYCARGGYNSDAFDIWGQGT MVTVSSGGGGSGGGGSGGGGSGGGGSDIVMTQSPSSVSA SVGDRVTITCRASQDISSWLAWYQQKPGKAPKLLIYAAS SLQSGVPSRFNGSGSGTDFTLTISSLQPEDFATYYCQQSYS TPLTFGGGTKVEIK |
| CLL1_9 scFv-Fc | 532 | QVQLVQSGAEVKEPGASVKVSCKAPANTFSDHVMHWV RQAPGQRFEWMGYIHAANGGTHYSQKFQDRVTITRDTS ANTVYMDLSSLRSEDTAVYYCARGGYNSDAFDIWGQGT MVTVSSGGGGSGGGGSGGGGSGGGGSDIVMTQSPSSVSA SVGDRVTITCRASQDISSWLAWYQQKPGKAPKLLIYAAS SLQSGVPSRFNGSGSGTDFTLTISSLQPEDFATYYCQQSYS TPLTFGGGTKVEIKGGGGSGSDKTHTCPPCPAPELLGGPS VFLFPPKPKDTLMISRTPEVTCVVVAVSHEDPEVKFNWY VDGVEVHNAKTKPREEQYNSTYRVVSVLTVLHQDWLNG KEYKCKVSNKALAAPIEKTISKAKGQPREPQVYTLPPCRD ELTKNQVSLWCLVKGFYPSDIAVEWESNGQPENNYKTTP PVLDSDGSFFLYSKLTVDKSRWQQGNVFSCSVMHEALH NHYTQKSLSLSPGK |
| CLL1_9 VL DNA | 533 | gatatcgtgatgactcagtccccctcgtccgtgagcgcgtccgtgggcgacagagtgacc attacgtgccgcgccagccaagacatttcttcctggctcgcctggtaccagcagaagcctg gaaaggctccgaagctgctgatctacgccgcctcatcgctccaatccggagtgccatcgc ggttcaatggctcggggtccggaactgactttaccctgactattagcagcctgcagcctgag gacttcgctacctattactgccaacagtcctactccaccccgctgaccttcgggggtggtac caaggtcgaaatcaag |
| CLL1_9 VH DNA | 534 | caagtgcagctggtgcagtccggagcagaagtcaaggaaccgggagccagcgtgaagg tgtcctgtaaagcccccgcaaacactttcagcgatcacgtcatgcactgggtccggcaggc ccccggccaacgcttcgaatggatggggtacatccatgctgccaacggcggaacccacta cagccagaagtttcaggaccgcgtgacgatcaccagggacacatccgcgaacactgtgta catggacctgtcatccctgagatcggaggacaccgcagtgtactactgcgcccgggggg gatacaactccgatgcgttcgacatctggggccagggaaccatggtcaccgtgtcatcc |
| CLL1_9 scFv DNA | 535 | caagtgcagctggtgcagtccggagcagaagtcaaggaaccgggagccagcgtgaagg tgtcctgtaaagcccccgcaaacactttcagcgatcacgtcatgcactgggtccggcaggc ccccggccaacgcttcgaatggatggggtacatccatgctgccaacggcggaacccacta cagccagaagtttcaggaccgcgtgacgatcaccagggacacatccgcgaacactgtgta catggacctgtcatccctgagatcggaggacaccgcagtgtactactgcgcccgggggg gatacaactccgatgcgttcgacatctggggccagggaaccatggtcaccgtgtcatccgg tggaggcggctcgggtggcggaggatcaggaggaggaggcagcgggggcggaggtt ccgatatcgtgatgactcagtccccctcgtccgtgagcgcgtccgtgggcgacagagtga ccattacgtgccgcgccagccaagacatttcttcctggctcgcctggtaccagcagaagcc tggaaaggctccgaagctgctgatctacgccgcctcatcgctccaatccggagtgccatcg cggttcaatggctcggggtccggaactgactttaccctgactattagcagcctgcagcctga ggacttcgctacctattactgccaacagtcctactccaccccgctgaccttcgggggtggta ccaaggtcgaaatcaag |
| CLL1_9 scFv-Fc DNA | 536 | caagtgcagctggtgcagtccggagcagaagtcaaggaaccgggagccagcgtgaagg tgtcctgtaaagcccccgcaaacactttcagcgatcacgtcatgcactgggtccggcaggc ccccggccaacgcttcgaatggatggggtacatccatgctgccaacggcggaacccacta cagccagaagtttcaggaccgcgtgacgatcaccagggacacatccgcgaacactgtgta catggacctgtcatccctgagatcggaggacaccgcagtgtactactgcgcccgggggg gatacaactccgatgcgttcgacatctggggccagggaaccatggtcaccgtgtcatccgg tggaggcggctcgggtggcggaggatcaggaggaggaggcagcgggggcggaggtt ccgatatcgtgatgactcagtccccctcgtccgtgagcgcgtccgtgggcgacagagtga ccattacgtgccgcgccagccaagacatttcttcctggctcgcctggtaccagcagaagcc tggaaaggctccgaagctgctgatctacgccgcctcatcgctccaatccggagtgccatcg cggttcaatggctcggggtccggaactgactttaccctgactattagcagcctgcagcctga ggacttcgctacctattactgccaacagtcctactccaccccgctgaccttcgggggtggta ccaaggtcgaaatcaagggagggggcggatccggatccgataagacccacacctgtcca ccctgccctgcccccgaactgcttggtggtccgtccgtgtttctgttcccgcccaagcccaa ggacaccctcatgatctcacggactcctgaagtgacctgtgtggtggtcgctgtgtcccacg aggaccccgaagtcaagttcaattggtacgtggacggagtggaagtgcacaacgctaaga |
| | | ccaagccccgcgaggaacagtacaactccacttaccgcgtcgtgtcggtgctgaccgtgc tgcatcaggattggctgaacggaaaggagtacaagtgcaaggtgtccaacaaggctctgg cggcacccatcgaaaagaccatcagcaaggccaaagggcaacctagagaaccacaagt ctacaccctgcctccttgccgggatgagctcaccaagaaccaggtgtccctgtggtgcctc gtgaagggcttctacccctctgacatcgcggtggaatgggagtcaaacggccagccagag aacaactacaagacaaccccccctgtcctggacagcgacggctccttcttcctgtactcgaa gctgactgtggataagagccggtggcaacagggcaacgtgttctcatgttcggtcatgcac gaggccctgcataaccactacactcagaagtccctgagcctgtcccctggaaag |

| CD3 x CLL1_06 | | |
|---|---|---|
| CLL1_6 VL | 83 | NFMLTQPHSVSESPGKTVTISCTGSSGSIASNYVQWYQQR PGSAPTTVIYEDNQRPSGVPDRFSGSIDSSSNSASLTISGLK TEDEADYYCQSYDSSNQVVFGGGTKLTVL |
| CLL1_6 VH | 70 | EVQLVESGGGLVKPGGSLRLSCAASGFTFSSYSMNWVRQ APGKGLEWVSSISSSSSYIYYADSVKGRFTISRDNAKNSL YLQMNSLRAEDTAVYYCARDPSSSGSYYMEDSYYYGMD VWGQGTTVTVSS |
| CLL1 6 scFv | 44 | EVQLVESGGGLVKPGGSLRLSCAASGFTFSSYSMNWVRQ APGKGLEWVSSISSSSSYIYYADSVKGRFTISRDNAKNSL YLQMNSLRAEDTAVYYCARDPSSSGSYYMEDSYYYGMD VWGQGTTVTVSSGGGGSGGGGSGGGGSNFMLTQPHSVS ESPGKTVTISCTGSSGSIASNYVQWYQQRPGSAPTTVIYE DNQRPSGVPDRFSGSIDSSSNSASLTISGLKTEDEADYYCQ SYDSSNQVVFGGGTKLTVL |
| CLL1_6 scFv-Fc | 537 | EVQLVESGGGLVKPGGSLRLSCAASGFTFSSYSMNWVRQ APGKGLEWVSSISSSSSYIYYADSVKGRFTISRDNAKNSL YLQMNSLRAEDTAVYYCARDPSSSGSYYMEDSYYYGMD VWGQGTTVTVSSGGGGSGGGGSGGGGSNFMLTQPHSVS ESPGKTVTISCTGSSGSIASNYVQWYQQRPGSAPTTVIYE DNQRPSGVPDRFSGSIDSSSNSASLTISGLKTEDEADYYCQ SYDSSNQVVFGGGTKLTVLGGGGSGSDKTHTCPPCPAPE LLGGPSVFLFPPKPKDTLMISRTPEVTCVVVAVSHEDPEV KFNWYVDGVEVHNAKTKPREEQYNSTYRVVSVLTVLHQ DWLNGKEYKCKVSNKALAAPIEKTISKAKGQPREPQVYT LPPCRDELTKNQVSLWCLVKGFYPSDIAVEWESNGQPEN NYKTTPPVLDSDGSFFLYSKLTVDKSRWQQGNVFSCSVM HEALHNHYTQKSLSLSPGK |
| CLL1_6 VL DNA | 538 | aacttcatgctgacccagcctcactccgtgtccgaatccccgggaaaaccgtgactatca gctgcaccggctccagcggctcgatcgcgtcgaactacgtgcagtggtatcaacagcgcc ccggttccgcccccaccaccgtgatctacgaagataaccagcggccttccggagtcccgg atagattctccggttccattgactcctcatccaactccgcctcgctcactattagcggcctcaa gacggaggacgaagccgattactactgtcagtcctacgactcgagcaatcaagtggtgttc ggagggggcaccaagctgaccgtgctg |
| CLL1_6 VH DNA | 539 | gaagtgcagctcgtggagtcgggcggtggattggtcaagccgggcggaagcctgcggct gtcatgcgccgcttctgggttcaccttctcctcctactccatgaactgggtcagacaggcgc ccggaaagggactggaatgggtgtcctcaatctcgtcgtcctcgtcctacatctattacgcc gactcagtgaagggggcgctttactatttcgcgggacaacgctaagaactccctgtacctcca aatgaacagcctgcgggcggaggacaccgccgtgtactactgcgcaagggacccaagc agctccggctcatactacatggaggactcctactactacggaatggacgtctggggacagg gaaccactgtgaccgtgtcatcc |
| CLL1_6 scFv DNA | 540 | gaagtgcagctcgtggagtcgggcggtggattggtcaagccgggcggaagcctgcggct gtcatgcgccgcttctgggttcaccttctcctcctactccatgaactgggtcagacaggcgc ccggaaagggactggaatgggtgtcctcaatctcgtcgtcctcgtcctacatctattacgcc gactcagtgaaggggcgctttactatttcgcgggacaacgctaagaactccctgtacctcca aatgaacagcctgcgggcggaggacaccgccgtgtactactgcgcaagggacccaagc |
| | | agctccggctcatactacatggaggactcctactactacggaatggacgtctggggacagg gaaccactgtgaccgtgtcatccggcggcggtggtagcgggggcggaggaagcgggg ggggaggctccaacttcatgctgacccagcctcactccgtgtccgaatccccggggaaaa ccgtgactatcagctgcaccggctccagcggctcgatcgcgtcgaactacgtgcagtggta tcaacagcgccccggttccgcccccaccaccgtgatctacgaagataaccagcggccttc cggagtcccggatagattctccggttccattgactcctcatccaactccgcctcgctcactatt agcggcctcaagacggaggacgaagccgattactactgtcagtcctacgactcgagcaat caagtggtgttcggagggggcaccaagctgaccgtgctg |
| CLL1_6 scFv-Fc DNA | 541 | gaagtgcagctcgtggagtcgggcggtggattggtcaagccgggcggaagcctgcggct gtcatgcgccgcttctgggttcaccttctcctcctactccatgaactgggtcagacaggcgc ccggaaagggactggaatgggtgtcctcaatctcgtcgtcctcgtcctacatctattacgcc gactcagtgaaggggcgctttactatttcgcgggacaacgctaagaactccctgtacctcca aatgaacagcctgcgggcggaggacaccgccgtgtactactgcgcaagggacccaagc agctccggctcatactacatggaggactcctactactacggaatggacgtctggggacagg gaaccactgtgaccgtgtcatccggcggcggtggtagcgggggcggaggaagcgggg ggggaggctccaacttcatgctgacccagcctcactccgtgtccgaatccccggggaaaa ccgtgactatcagctgcaccggctccagcggctcgatcgcgtcgaactacgtgcagtggta tcaacagcgccccggttccgcccccaccaccgtgatctacgaagataaccagcggccttc cggagtcccggatagattctccggttccattgactcctcatccaactccgcctcgctcactatt agcggcctcaagacggaggacgaagccgattactactgtcagtcctacgactcgagcaat caagtggtgttcggagggggcaccaagctgaccgtgctgggcggtggaggatccggatc cgataagacccacacctgtccaccctgccctgcccccgaactgcttggtggtccgtccgtg tttctgttcccgcccaagcccaaggacaccctcatgatctcacggactcctgaagtgacctg tgtggtggtcgctgtgtcccacgaggaccccgaagtcaagttcaattggtacgtggacgga gtggaagtgcacaacgctaagaccaagccccgcgaggaacagtacaactccacttaccg cgtcgtgtcggtgctgaccgtgctgcatcaggattggctgaacggaaaggagtacaagtg caaggtgtccaacaaggctctggcggcacccatcgaaaagaccatcagcaaggccaaag ggcaacctagagaaccacaagtctacaccctgcctccttgccgggatgagctcaccaaga accaggtgtccctgtggtgcctcgtgaagggcttctacccctctgacatcgcggtggaatg ggagtcaaacggccagccagagaacaactacaagacaaccccccctgtcctggacagc gacggctccttcttcctgtactcgaagctgactgtggataagagccggtggcaacagggca acgtgttctcatgttcggtcatgcacgaggccctgcataaccactacactcagaagtccctg agcctgtcccctggaaag |

| **CD3 x CLL1_13** | | |
|---|---|---|
| CLL1_13 VL | 90 | DIQMTQSPSSLSASVGDRVTFTCRASQGISSALAWYQQKP GKPPKLLIYDASSLESGVPSRFSGSGSGTDFTLTISSLQPED FATYYCQQFNNYPLTFGGGTKVEIK |
| CLL1_13 VH | 77 | QVQLVQSGAEVKKSGASVKVSCKASGYPFTGYYIQWVR QAPGQGLEWMGWIDPNSGNTGYAQKFQGRVTMTRNTSI STAYMELSSLRSEDTAVYYCASDSYGYYYGMDVWGQG TLVTVSS |
| CLL1_13 scFv | 51 | QVQLVQSGAEVKKSGASVKVSCKASGYPFTGYYIQWVR QAPGQGLEWMGWIDPNSGNTGYAQKFQGRVTMTRNTSI STAYMELSSLRSEDTAVYYCASDSYGYYYGMDVWGQG TLVTVSSGGGGSGGGGSGGGGSGGGGSDIQMTQSPSSLS ASVGDRVTFTCRASQGISSALAWYQQKPGKPPKLLIYDA SSLESGVPSRFSGSGSGTDFTLTISSLQPEDFATYYCQQFN NYPLTFGGGTKVEIK |
| CLL1_13 scFv-Fc | 542 | QVQLVQSGAEVKKSGASVKVSCKASGYPFTGYYIQWVR QAPGQGLEWMGWIDPNSGNTGYAQKFQGRVTMTRNTSI STAYMELSSLRSEDTAVYYCASDSYGYYYGMDVWGQG TLVTVSSGGGGSGGGGSGGGGSGGGGSDIQMTQSPSSLS ASVGDRVTFTCRASQGISSALAWYQQKPGKPPKLLIYDA SSLESGVPSRFSGSGSGTDFTLTISSLQPEDFATYYCQQFN |
| | | NYPLTFGGGTKVEIKGGGGSGSDKTHTCPPCPAPELLGGP SVFLFPPKPKDTLMISRTPEVTCVVVAVSHEDPEVKFNWY VDGVEVHNAKTKPREEQYNSTYRVVSVLTVLHQDWLNG KEYKCKVSNKALAAPIEKTISKAKGQPREPQVYTLPPCRD ELTKNQVSLWCLVKGFYPSDIAVEWESNGQPENNYKTTP PVLDSDGSFFLYSKLTVDKSRWQQGNVFSCSVMHEALH NHYTQKSLSLSPGK |
| CLL1_13 VL DNA | 543 | gacattcagatgacccagtcaccatcctccctgtccgcctccgtcggggatagagtgacctt cacctgtcgggcctcccaaggaatctcaagcgctctggcctggtaccagcagaagcctgg aaagccgcccaagctgttgatctacgatgcctcgagcctggaatccggcgtgccgagccg gttcagcggtagcggctcgggaaccgacttcacgctcaccatctcgtccctgcaaccgga ggacttcgcgacttactactgccagcaattcaacaactaccctctgacctttggtggtggtac taaggtcgagatcaag |
| CLL1_13 VH DNA | 544 | caagtgcagctggtgcagtccggggccgaagtgaaaaagtccggtgcatccgtgaaagt gtcgtgcaaggcctcaggctatcccttcaccggatactacattcagtgggtccgccaggctc cgggacagggcctcgaatggatgggctggatcgatcccaactccggcaatactggctacg cgcagaagttccagggacgcgtgaccatgactcggaacacctccatttccaccgcctacat ggaactgtcgtcactgaggtccgaggacaccgccgtgtattactgcgcgtcggacagcta cggatactactacgggatggacgtgtggggacagggaactctggtcaccgtgtcg |
| CLL1_13 scFv DNA | 545 | caagtgcagctggtgcagtccggggccgaagtgaaaaagtccggtgcatccgtgaaagt gtcgtgcaaggcctcaggctatcccttcaccggatactacattcagtgggtccgccaggctc cgggacagggcctcgaatggatgggctggatcgatcccaactccggcaatactggctacg cgcagaagttccagggacgcgtgaccatgactcggaacacctccatttccaccgcctacat ggaactgtcgtcactgaggtccgaggacaccgccgtgtattactgcgcgtcggacagcta cggatactactacgggatggacgtgtggggacagggaactctggtcaccgtgtcgtccgg aggcggaggcagcggcgggggcggctccgggggagggggtcgggcggaggcgg aagcgacattcagatgacccagtcaccatcctccctgtccgcctccgtcggggatagagtg accttcacctgtcgggcctcccaaggaatctcaagcgctctggcctggtaccagcagaagc ctggaaagccgcccaagctgttgatctacgatgcctcgagcctggaatccggcgtgccga gccggttcagcggtagcggctcgggaaccgacttcacgctcaccatctcgtccctgcaac cggaggacttcgcgacttactactgccagcaattcaacaactaccctctgacctttggtggtg gtactaaggtcgagatcaag |
| CLL1_13 scFv-Fc DNA | 546 | caagtgcagctggtgcagtccggggccgaagtgaaaaagtccggtgcatccgtgaaagt gtcgtgcaaggcctcaggctatcccttcaccggatactacattcagtgggtccgccaggctc cgggacagggcctcgaatggatgggctggatcgatcccaactccggcaatactggctacg cgcagaagttccagggacgcgtgaccatgactcggaacacctccatttccaccgcctacat ggaactgtcgtcactgaggtccgaggacaccgccgtgtattactgcgcgtcggacagcta cggatactactacgggatggacgtgtggggacagggaactctggtcaccgtgtcgtccgg aggcggaggcagcggcgggggcggctccgggggagggggtcgggcggaggcgg aagcgacattcagatgacccagtcaccatcctccctgtccgcctccgtcggggatagagtg accttcacctgtcgggcctcccaaggaatctcaagcgctctggcctggtaccagcagaagc ctggaaagccgcccaagctgttgatctacgatgcctcgagcctggaatccggcgtgccga gccggttcagcggtagcggctcgggaaccgacttcacgctcaccatctcgtccctgcaac cggaggacttcgcgacttactactgccagcaattcaacaactaccctctgacctttggtggtg gtactaaggtcgagatcaagggtggaggaggatccggatccgataagacccacacctgtc caccctgccctgcccccgaactgcttggtggtccgtccgtgtttctgttcccgcccaagccc aaggacaccctcatgatctcacggactcctgaagtgacctgtgtggtggtcgctgtgtccca cgaggaccccgaagtcaagttcaattggtacgtggacggagtggaagtgcacaacgctaa gaccaagccccgcgaggaacagtacaactccacttaccgcgtcgtgtcggtgctgaccgt gctgcatcaggattggctgaacggaaaggagtacaagtgcaaggtgtccaacaaggctct ggcggcacccatcgaaaagaccatcagcaaggccaaagggcaacctagagaaccacaa gtctacaccctgcctccttgccgggatgagctcaccaagaaccaggtgtccctgtggtgcct cgtgaagggcttctaccccctctgacatcgcggtggaatgggagtcaaacggccagccaga gaacaactacaagacaacccccccctgtcctggacagcgacggctccttcttcctgtactcg |
| | | aagctgactgtggataagagccggtggcaacagggcaacgtgttctcatgttcggtcatgc acgaggccctgcataaccactacactcagaagtccctgagcctgtcccctggaaag |

| **CD3 x CLL1_07** | | |
|---|---|---|
| CLL1_7 VL | 84 | EIVLTQSPGTLSLSPGGRATLSCRASQSISGSFLAWYQQKP GQAPRLLIYGASSRATGIPDRFSGSGSGTDFTLTISRLEPED FAVYYCQQYGSSPPTFGLGTKLEIK |
| CLL1_7 VH | 71 | QVQLVESGGGLVQPGGSLRLSCAASGFTFSSYWMSWVR QAPGKGLEWVANINEDGSAKFYVDSVKGRFTISRDNAKN SLYLQMNSLRAEDTAVYFCARDLRSGRYWGQGTLVTVS S |
| CLL1_7 scFv | 45 | QVQLVESGGGLVQPGGSLRLSCAASGFTFSSYWMSWVR QAPGKGLEWVANINEDGSAKFYVDSVKGRFTISRDNAKN SLYLQMNSLRAEDTAVYFCARDLRSGRYWGQGTLVTVS SGGGGSGGGGSGGGGSEIVLTQSPGTLSLSPGGRATLSCR ASQSISGSFLAWYQQKPGQAPRLLIYGASSRATGIPDRFSG SGSGTDFTLTISRLEPEDFAVYYCQQYGSSPPTFGLGTKLE IK |
| CLL1_7 scFv-Fc | 547 | QVQLVESGGGLVQPGGSLRLSCAASGFTFSSYWMSWVR QAPGKGLEWVANINEDGSAKFYVDSVKGRFTISRDNAKN SLYLQMNSLRAEDTAVYFCARDLRSGRYWGQGTLVTVS SGGGGSGGGGSGGGGSEIVLTQSPGTLSLSPGGRATLSCR ASQSISGSFLAWYQQKPGQAPRLLIYGASSRATGIPDRFSG SGSGTDFTLTISRLEPEDFAVYYCQQYGSSPPTFGLGTKLE IKGGGGSDKTHTCPPCPAPELLGGPSVFLFPPKPKDTLMIS RTPEVTCVVVAVSHEDPEVKFNWYVDGVEVHNAKTKPR EEQYNSTYRVVSVLTVLHQDWLNGKEYKCKVSNKALAA PIEKTISKAKGQPREPQVYTLPPCRDELTKNQVSLWCLVK GFYPSDIAVEWESNGQPENNYKTTPPVLDSDGSFFLYSKL TVDKSRWQQGNVFSCSVMHEALHNHYTQKSLSLSPGK |
| CLL1_7 VL DNA | 548 | gaaattgtgttgacccagtcgcctggaaccctttccctgtcgcccggcggacgggctaccc tgtcgtgccgcgctagccagtcgatctccggatcttttctcgcctggtaccagcagaagccc ggacaggcccctaggctgctgatctacggggccagctcacgcgcaaccggtattccggat cggttctccggttccgggtcgggaactgacttcaccctgactatctcccggctggaaccag aggatttcgcggtctactactgccagcagtatggaagctcaccgccgaccttcggcttggg aaccaagctggaaatcaag |
| CLL1_7 VH DNA | 549 | caagtgcagctcgtcgaaagcggtggcgggctggtgcagccgggcggctcgctgagact gtcctgcgccgcgagcggcttcaccttctcctcctactggatgtcctgggtccgccaagccc ccggaaaggggctggaatgggtggccaacattaacgaggacggttccgccaagttctac gtggattccgtgaaaggccggtttaccatctcgagggacaacgccaagaattccctctacct ccaaatgaactccctgagagcggaggacactgccgtgtacttctgtgcacgcgacctgag atcaggccggtactggggccaggggacactcgtgaccgtgtcaagc |
| CLL1_7 scFv DNA | 550 | caagtgcagctcgtcgaaagcggtggcgggctggtgcagccgggcggctcgctgagact gtcctgcgccgcgagcggcttcaccttctcctcctactggatgtcctgggtccgccaagccc ccggaaaggggctggaatgggtggccaacattaacgaggacggttccgccaagttctac gtggattccgtgaaaggccggtttaccatctcgagggacaacgccaagaattccctctacct ccaaatgaactccctgagagcggaggacactgccgtgtacttctgtgcacgcgacctgag atcaggccggtactggggccaggggacactcgtgaccgtgtcaagcggaggcggtggct ccggaggaggaggttccgggggaggaggcagcgaaattgtgttgacccagtcgcctgg aaccctttccctgtcgcccggcggacgggctaccctgtcgtgccgcgctagccagtcgatc tccggatcttttctcgcctggtaccagcagaagcccggacaggcccctaggctgctgatcta cggggccagctcacgcgcaaccggtattccggatcggttctccggttccgggtcgggaac tgacttcaccctgactatctcccggctggaaccagaggatttcgcggtctactactgccagc agtatggaagctcaccgccgaccttcggcttgggaaccaagctggaaatcaag |
| CLL1_7 scFv-Fc DNA | 551 | caagtgcagctcgtcgaaagcggtggcgggctggtgcagccgggcggctcgctgagact gtcctgcgccgcgagcggcttcaccttctcctcctactggatgtcctgggtccgccaagccc ccggaaaggggctggaatgggtggccaacattaacgaggacggttccgccaagttctac gtggattccgtgaaaggccggtttaccatctcgagggacaacgccaagaattccctctacct ccaaatgaactccctgagagcggaggacactgccgtgtacttctgtgcacgcgacctgag atcaggccggtactggggccaggggacactcgtgaccgtgtcaagcggaggcggtggct ccggaggaggaggttccgggggaggaggcagcgaaattgtgttgacccagtcgcctgg aaccctttccctgtcgcccggcggacgggctaccctgtcgtgccgcgctagccagtcgatc tccggatcttttctcgcctggtaccagcagaagcccggacaggcccctaggctgctgatcta cggggccagctcacgcgcaaccggtattccggatcggttctccggttccgggtcgggaac tgacttcaccctgactatctcccggctggaaccagaggatttcgcggtctactactgccagc agtatggaagctcaccgccgaccttcggcttgggaaccaagctggaaatcaagggggggg ggcggatccgataagacccacacctgtccaccctgccctgcccccgaactgcttggtggtc cgtccgtgtttctgttcccgcccaagcccaaggacaccctcatgatctcacggactcctgaa gtgacctgtgtggtggtcgctgtgtcccacgaggaccccgaagtcaagttcaattggtacgt ggacggagtggaagtgcacaacgctaagaccaagccccgcgaggaacagtacaactcc acttaccgcgtcgtgtcggtgctgaccgtgctgcatcaggattggctgaacggaaaggagt acaagtgcaaggtgtccaacaaggctctggcggcacccatcgaaaagaccatcagcaag gccaaagggcaacctagagaaccacaagtctacaccctgcctccttgccgggatgagctc accaagaaccaggtgtccctgtggtgcctcgtgaagggcttctacccctctgacatcgcgg tggaatgggagtcaaacggccagccagagaacaactacaagacaaccccccctgtcctg gacagcgacggctccttcttcctgtactcgaagctgactgtggataagagccggtggcaac agggcaacgtgttctcatgttcggtcatgcacgaggccctgcataaccactacactcagaa gtccctgagcctgtcccctggaaag |

| **CD3 x CLL1_02** | | |
|---|---|---|
| CLL1_2 VL | 79 | EIVLTQSPLSLPVTPGQPASISCRSSQSLVYTDGNTYLNWF QQRPGQSPRRLIYKVSNRDSGVPDRFSGSGSDTDFTLKISR VEAEDVGIYYCMQGTHWSFTFGQGTRLEIK |
| CLL1_2 VH | 66 | EVQLVESGGGVVQPGGSLRLSCAASGFTFDDYAMHWVR QAPGKGLEWVSLISGDGGSTYYADSVKGRFTISRDNSKN TLYLQMNSLRVEDTAVYYCARVFDSYYMDVWGKGTTV TVSS |
| CLL1_2 scFv | 40 | EVQLVESGGGVVQPGGSLRLSCAASGFTFDDYAMHWVR QAPGKGLEWVSLISGDGGSTYYADSVKGRFTISRDNSKN TLYLQMNSLRVEDTAVYYCARVFDSYYMDVWGKGTTV TVSSGGGGSGGGGSGSGGSEIVLTQSPLSLPVTPGQPASIS CRSSQSLVYTDGNTYLNWFQQRPGQSPRRLIYKVSNRDS GVPDRFSGSGSDTDFTLKISRVEAEDVGIYYCMQGTHWS FTFGQGTRLEIK |
| CLL1_2 scFv-Fc | 552 | EVQLVESGGGVVQPGGSLRLSCAASGFTFDDYAMHWVR QAPGKGLEWVSLISGDGGSTYYADSVKGRFTISRDNSKN TLYLQMNSLRVEDTAVYYCARVFDSYYMDVWGKGTTV TVSSGGGGSGGGGSGSGGSEIVLTQSPLSLPVTPGQPASIS CRSSQSLVYTDGNTYLNWFQQRPGQSPRRLIYKVSNRDS GVPDRFSGSGSDTDFTLKISRVEAEDVGIYYCMQGTHWS FTFGQGTRLEIKGGGGSDKTHTCPPCPAPELLGGPSVFLFP PKPKDTLMISRTPEVTCVVVAVSHEDPEVKFNWYVDGVE VHNAKTKPREEQYNSTYRVVSVLTVLHQDWLNGKEYKC KVSNKALAAPIEKTISKAKGQPREPQVYTLPPCRDELTKN QVSLWCLVKGFYPSDIAVEWESNGQPENNYKTTPPVLDS DGSFFLYSKLTVDKSRWQQGNVFSCSVMHEALHNHYTQ KSLSLSPGK |
| CLL1_2 VL DNA | 553 | gaaattgtcctcacccaatccccgctgtcactgcccgtgacccctggccagccggcatcca tcagctgccggagcagccagtccctggtgtacactgacggaaatacttacctgaactggttc |
| | | cagcaacgcccggggcagagcccacgcagactgatctacaaggtgtcaaacagggact ctggagtgcccgataggttctcgggttccgggtcggacaccgattttacactgaagatctcc cgggtggaagcggaggacgtgggcatctattactgtatgcaggggacccattggtccttca cgttcggacagggcactcggctggaaatcaag |
| CLL1_2 VH DNA | 554 | gaagtgcagttggtggagagcggaggcggcgtggtgcagcccggaggttccctgcggct gtcgtgcgcggcctcgggtttcacttttgatgactacgccatgcactgggtcagacaggccc ctggaaagggcctcgaatgggtgtcgctgatttccggagatggaggcagcacctactatgc cgattccgtcaaggggagattcaccatttcccgcgacaacagcaaaaacaccttgtacctc caaatgaactccctgcgggtggaggacaccgctgtgtactactgcgcccgcgtgttcgact catactacatggacgtctggggaaaggggaccactgtgaccgtgtccagc |
| CLL1_2 scFv DNA | 555 | gaagtgcagttggtggagagcggaggcggcgtggtgcagcccggaggttccctgcggct gtcgtgcgcggcctcgggtttcacttttgatgactacgccatgcactgggtcagacaggccc ctggaaagggcctcgaatgggtgtcgctgatttccggagatggaggcagcacctactatgc cgattccgtcaaggggagattcaccatttcccgcgacaacagcaaaaacaccttgtacctc caaatgaactccctgcgggtggaggacaccgctgtgtactactgcgcccgcgtgttcgact catactacatggacgtctggggaaaggggaccactgtgaccgtgtccagcgggggaggc ggctccggcggcggcggatcgggttcaggagggtccgaaattgtcctcacccaatcccc gctgtcactgcccgtgacccctggccagccggcatccatcagctgccggagcagccagtc cctggtgtacactgacggaaatacttacctgaactggttccagcaacgcccggggcagag cccacgcagactgatctacaaggtgtcaaacagggactctggagtgcccgataggttctcg ggttccgggtcggacaccgattttacactgaagatctcccgggtggaagcggaggacgtg ggcatctattactgtatgcaggggacccattggtccttcacgttcggacagggcactcggct ggaaatcaag |
| CLL1_2 scFv-Fc DNA | 556 | gaagtgcagttggtggagagcggaggcggcgtggtgcagcccggaggttccctgcggct gtcgtgcgcggcctcgggtttcacttttgatgactacgccatgcactgggtcagacaggccc ctggaaagggcctcgaatgggtgtcgctgatttccggagatggaggcagcacctactatgc cgattccgtcaagggggagattcaccatttcccgcgacaacagcaaaaacaccttgtacctc caaatgaactccctgcgggtggaggacaccgctgtgtactactgcgcccgcgtgttcgact catactacatggacgtctggggaaaggggaccactgtgaccgtgtccagcgggggaggc ggctccggcggcggcggatcgggttcaggagggtccgaaattgtcctcacccaatcccc gctgtcactgcccgtgacccctggccagccggcatccatcagctgccggagcagccagtc cctggtgtacactgacggaaatacttacctgaactggttccagcaacgcccggggcagag cccacgcagactgatctacaaggtgtcaaacagggactctggagtgcccgataggttctcg ggttccgggtcggacaccgattttacactgaagatctcccgggtggaagcggaggacgtg ggcatctattactgtatgcaggggacccattggtccttcacgttcggacagggcactcggct ggaaatcaagggaggaggcggatccgataagacccacacctgtccaccctgccctgccc ccgaactgcttggtggtccgtccgtgtttctgttcccgcccaagcccaaggacaccctcatg atctcacggactcctgaagtgacctgtgtggtggtcgctgtgtcccacgaggaccccgaag tcaagttcaattggtacgtggacggagtggaagtgcacaacgctaagaccaagccccgcg aggaacagtacaactccacttaccgcgtcgtgtcggtgctgaccgtgctgcatcaggattg gctgaacggaaaggagtacaagtgcaaggtgtccaacaaggctctggcggcacccatcg aaaagaccatcagcaaggccaaagggcaacctagagaaccacaagtctacaccctgcct ccttgccgggatgagctcaccaagaaccaggtgtccctgtggtgcctcgtgaagggcttct acccctctgacatcgcggtggaatgggagtcaaacggccagccagagaacaactacaag acaacccccccctgtcctggacagcgacggctccttcttcctgtactcgaagctgactgtgga taagagccggtggcaacagggcaacgtgttctcatgttcggtcatgcacgaggccctgcat aaccactacactcagaagtccctgagcctgtcccctggaaag |

In aspects, the invention provides a multispecific molecule, e.g., a bispecific molecule, comprising an anti-CD3 binding domain comprising a VL sequence of SEQ ID NO: 1209 and a VH sequence of SEQ ID NO: 1236, and an anti-CLL-1 binding domain comprising:
a) a VL sequence of SEQ ID NO: 88 and a VH sequence of SEQ ID NO: 75;
b) a VL sequence of SEQ ID NO: 89 and a VH sequence of SEQ ID NO: 76;
c) a VL sequence of SEQ ID NO: 87 and a VH sequence of SEQ ID NO: 74;
d) a VL sequence of SEQ ID NO: 85 and a VH sequence of SEQ ID NO: 72;
e) a VL sequence of SEQ ID NO: 86 and a VH sequence of SEQ ID NO: 73;
f) a VL sequence of SEQ ID NO: 83 and a VH sequence of SEQ ID NO: 70;
g) a VL sequence of SEQ ID NO: 90 and a VH sequence of SEQ ID NO: 77;
h) a VL sequence of SEQ ID NO: 79 and a VH sequence of SEQ ID NO: 66; or
i) a VL sequence of SEQ ID NO: 84 and a VH sequence of SEQ ID NO: 71.

In aspects, the invention provides a multispecific molecule, e.g., a bispecific molecule, comprising an anti-CD3 binding domain comprising SEQ ID NO: 506, and an anti-CLL-1 binding domain comprising:
a) SEQ ID NO: 49;
b) SEQ ID NO: 50;
c) SEQ ID NO: 48;
d) SEQ ID NO: 46;
e) SEQ ID NO: 47;
f) SEQ ID NO: 44;
g) SEQ ID NO: 51;
h) SEQ ID NO: 40; or
i) SEQ ID NO: 45.

In aspects, the invention provides a multispecific molecule, e.g., a bispecific molecule (e.g., a bispecific molecule having a scFv-Fc format), comprising a first polypeptide comprising an anti-CD3 binding domain, wherein said first polypeptide chain comprises, e.g., consists of, SEQ ID NO: 507, and a second polypeptide comprising an anti-CLL-1 binding domain, wherein said second polypeptide chain comprises, e.g., consists of:
a) SEQ ID NO: 512;
b) SEQ ID NO: 517;
c) SEQ ID NO: 522;
d) SEQ ID NO: 527;
e) SEQ ID NO: 532;
f) SEQ ID NO: 537;
g) SEQ ID NO: 542;
h) SEQ ID NO: 547; or
i) SEQ ID NO: 552.

### IV. Binding Specificity

The molecules of the present invention can be multivalent multispecific, multivalent monospecific, monovalent multispecific, or monovalent monospecific.

In one embodiment, the molecule is a bivalent molecule (e.g., a bivalent antibody or antibody-like molecule). In one particular aspect, the present molecule has dual binding specificities if the first antigen binding domain and second antigen binding domain recognize two different antigens or two different epitopes on the same antigen. In another particular aspect, if the first and second antigen binding domains bind to the same antigen/epitope, the molecule is a mono-specific molecule. Incorporation of additional antigen binding domains, e.g., by incorporation of one or more additional scFv antigen binding domains may form a multispecific molecule when the one or more additional scFv antigen binding domains recognize a different antigen/epitope than the first and second antigen binding domains.

Standard assays to evaluate the binding specificity of the antibodies or antibody-like molecules toward various epitopes and/or antigens are known in the art, including for example, Biacore analysis, or FACS relative affinity (Scatchard), ELISAs, western blots and RIAs. Suitable assays are described in detail in the Definition and Examples.

### V. Physical Property/Yield

In certain embodiments, the present molecule offers desirable physical properties, such as a thermo-stability substantially same as or increased relative to that of natural antibodies.

Thermostability refers to protein stability during heat stress, which is an ability of a protein to retain the characteristic property when heated moderately. When exposed to heat, proteins will experience denaturing/unfolding process and will expose hydrophobic residues. Each protein is completely unfolded in response to heat at a characteristic temperature. The temperature at the mid-point of the protein unfolding process is defined as Tm, which is an important physical characteristic for a protein, and can be measured with the techniques known in the art. A multispecific molecule having a relatively high value of Tm is usually desirable because a high value of Tm often indicates less aggregation when it is used for preparing a pharmaceutical composition. In addition, higher Tm may also result in higher expression and yield.

In one particular embodiment, the multispecific molecule of the present invention has substantially same Tm as compared to that of an IgG antibody.

In yet another embodiment, the present invention includes a method of generating a multispecific molecule having substantially the same thermostability as a reference antibody comprising 1) designing a molecule of one of the formats described herein; 2) producing the molecule in a host cell; and 3) measuring and comparing Tm of the molecule and the reference antibody.

Cell culture systems have been widely used for expressing antibody fragments, but there have been few attempts to express and recover functional completely assembled full-length antibodies in high yield. Because of the complex structure and large size of completely assembled full-length antibodies or antibody-like molecules, it is often difficult to achieve proper folding and assembly of the expressed heavy and light chains, especially in bacterial cells. This problem is especially challenging when producing multispecific molecules. Because of the random pairing of two different antibody heavy and light chains within the host cells, only small percentage of the assembled antibody or antibody-like species is the desired, functional multispecific molecules. Due to the presence of mispaired byproducts, and significantly reduced production yields, sophisticated purification procedures are required (see e.g. Morrison, S. L., Nature Biotech 25 (2007) 1233-1234).

In some embodiments, the present molecules (e.g. antibodies or antibody-like molecules), when recombinantly produced in comparable cell cultures, have substantially same yield as when producing a reference antibody. In particular, under the same culture condition, being expressed by the same type of host cells, the molecules have substantially the same expression levels as a reference antibody. The expression levels of the produced molecule can be measured with the standard techniques in the art, such as, scanning densitometry of SDS-PAGE gels and/or immunoblots and the AME5-RP assay. Antibody or antibody-like molecule yield can also be quantified by protein A sensor chip using Qctec Red (Fotrte Bio).

The present invention includes a method of generating a multispecific molecule and having substantially same yield as production of a reference antibody comprising 1) designing a molecule of the present invention described herein; 2) producing the molecule in a host cell; and 3) measuring and comparing the expression level of said molecule with said reference antibody.

### VI. Modification of the Molecules of the Present Invention

### I.Molecules with Enhanced Heterodimerization

Inadequate heterodimerization of two antibody heavy chain domains has always been an obstacle for increasing the yield of desired multispecific molecules and represents challenges for purification. A variety of approaches available in the art can be used in for enhancing dimerization of the two heavy chain domains of bispecific or multispecific antibody or antibody-like molecules, as disclosed in EP 1870459A1; U.S. Pat. No. 5,582,996; U.S. Pat. No. 5,731,168; U.S. Pat. No. 5,910,573; U.S. Pat. No. 5,932,448; U.S. Pat. No. 6,833,441; U.S. Pat. No. 7,183,076; U.S. Patent Application Publication No. 2006204493A1; and PCT Publication No. WO2009/089004A1

The present invention provides methods of enhancing dimerization (hetero-dimerization) of two interacting heterologous polypeptides and/or reducing dimerization (homo-dimerization) of two identical polypeptides. Typically, each of the two interacting polypeptides comprises a CH3 domain of an antibody. The CH3 domains are derived from the constant region of an antibody of any isotype, class or subclass, and preferably of IgG (IgG1, IgG2, IgG3 and IgG4) class.

Typically, the polypeptides comprise other antibody fragments in addition to CH3 domains, such as, CH1 domains, CH2 domains, hinge domain, VH domain(s), VL domain(s), CDR(s), and/or antigen-binding fragments described herein. These antibody fragments are derived from various types of antibodies described herein, for example, polyclonal antibody, monoclonal antibodies, chimeric antibodies, humanized antibodies, human antibodies, bispecific or multispecific antibodies, camelised antibodies, anti-idiotypic (anti-Id) antibodies and antibody conjugates. In some embodiments, the two hetero-polypeptides are two heavy chains forming a bispecific or multispecific molecules. Heterodimerzation of the two different heavy chains at CH3 domains give rise to the desired antibody or antibody-like molecule, while homodimerization of identical heavy chains will reduce yield of the desired antibody or molecule. In an exemplary embodiment, the two or more hetero-polypeptide chains comprise two chains comprising CH3 domains and forming the molecules of any of the multispecific molecule Formats described above of the present invention. In an embodiment, the two hetero-polypeptide chains comprising CH3 domains comprise modifications that favor heterodimeric association of the polypeptides, relative to unmodified chains. Various examples of modification strategies are provided below.

### Knob-in-Hole (KIH)

Multispecific molecules, e.g., multispecific antibody or antibody-like molecules, of the present invention may comprise one or more, e.g., a plurality, of mutations to one or more of the constant domains, e.g., to the CH3 domains. In one example, the multispecific molecule of the present invention comprises two polypeptides that each comprise a heavy chain constant domain of an antibody, e.g., a CH2 or CH3 domain. In an example, the two heavy chain constant domains, e.g., the CH2 or CH3 domains of the multispecific molecule comprise one or more mutations that allow for a heterodimeric association between the two chains. In one aspect, the one or more mutations are disposed on the CH2 domain of the two heavy chains of the multispecific, e.g., bispecific, antibody or antibody-like molecule. In one aspect, the one or more mutations are disposed on the CH3 domains of at least two polypeptides of the multispecific molecule. In one aspect, the one or more mutations to a first polypeptide of the multispecific molecule comprising a heavy chain constant domain creates a "knob" and the one or more mutations to a second polypeptide of the multispecific molecule comprising a heavy chain constant domain creates a "hole," such that heterodimerization of the polypeptide of the multispecific molecule comprising a heavy chain constant domain causes the "knob" to interface (e.g., interact, e.g., a CH2 domain of a first polypeptide interacting with a CH2 domain of a second polypeptide, or a CH3 domain of a first polypeptide interacting with a CH3 domain of a second polypeptide) with the "hole." As the term is used herein, a "knob" refers to at least one amino acid side chain which projects from the interface of a first polypeptide of the multispecific molecule comprising a heavy chain constant domain and is therefore positionable in a compensatory "hole" in the interface with a second polypeptide of the multispecific molecule comprising a heavy chain constant domain so as to stabilize the heteromultimer, and thereby favor heteromultimer formation over homomultimer formation, for example. The knob may exist in the original interface or may be introduced synthetically (e.g. by altering nucleic acid encoding the interface). The preferred import residues for the formation of a knob are generally naturally occurring amino acid residues and are preferably selected from arginine (R), phenylalanine (F), tyrosine (Y) and tryptophan (W). Most preferred are tryptophan and tyrosine. In the preferred embodiment, the original residue for the formation of the protuberance has a small side chain volume, such as alanine, asparagine, aspartic acid, glycine, serine, threonine or valine.

A "hole" refers to at least one amino acid side chain which is recessed from the interface of a second polypeptide of the multispecific molecule comprising a heavy chain constant domain and therefore accommodates a corresponding knob on the adjacent interfacing surface of a first polypeptide of the multispecific molecule comprising a heavy chain constant domain. The hole may exist in the original interface or may be introduced synthetically (e.g. by altering nucleic acid encoding the interface). The preferred import residues for the formation of a hole are usually naturally occurring amino acid residues and are preferably selected from alanine (A), serine (S), threonine (T) and valine (V). Most preferred are serine, alanine or threonine. In the preferred embodiment, the original residue for the formation of the hole has a large side chain volume, such as tyrosine, arginine, phenylalanine or tryptophan.

In a preferred embodiment, a first CH3 domain is mutated at residue 366, 405 or 407 according to the EU numbering scheme of Kabat et al. (pp. 688-696 in Sequences of proteins of immunological interest, 5th ed., Vol. 1 (1991; NIH, Bethesda, Md.)) to create either a "knob" or a hole" (as described above), and the second CH3 domain that heterodimerizes with the first CH3 domain is mutated at: residue 407 if residue 366 is mutated in the first CH3 domain, residue 394 if residue 405 is mutated in the first CH3 domain, or residue 366 if residue 407 is mutated in the first CH3 domain, according to the EU numbering scheme of Kabat et al. (pp. 688-696 in Sequences of proteins of immunological interest, 5th ed., Vol. 1 (1991; NIH, Bethesda, Md.)), to create a "hole" or "knob" complementary to the "knob" or "hole" of the first CH3 domain.

In another preferred embodiment, a first CH3 domain is mutated at residue 366 according to the EU numbering scheme of Kabat et al. (pp. 688-696 in Sequences of proteins of immunological interest, 5th ed., Vol. 1 (1991; NIH, Bethesda, Md.)) to create either a "knob" or a hole" (as described above), and the second CH3 domain that heterodimerizes with the first CH3 domain is mutated at residues 366, 368 and/or 407, according to the EU numbering scheme of Kabat et al. (pp. 688-696 in Sequences of proteins of immunological interest, 5th ed., Vol. 1 (1991; NIH, Bethesda, Md.)), to create a "hole" or "knob" complementary to the "knob" or "hole" of the first CH3 domain. In one embodiment, the mutation to the first CH3 domain introduces a tyrosine (Y) residue at position 366. In an embodiment, the mutation to the first CH3 is T366Y. In one embodiment, the mutation to the first CH3 domain introduces a tryptophan (W) residue at position 366. In an embodiment, the mutation to the first CH3 is T366W. In embodiments, the mutation to the second CH3 domain that heterodimerizes with the first CH3 domain mutated at position 366 (e.g., has a tyrosine (Y) or tryptophan (W) introduced at position 366, e.g., comprises the mutation T366Y or T366W), comprises a mutation at position 366, a mutation at position 368 and a mutation at position 407, according to the EU numbering scheme of Kabat et al. (pp. 688-696 in Sequences of proteins of immunological interest, 5th ed., Vol. 1 (1991; NIH, Bethesda, Md.)) In embodiments, the mutation at position 366 introduces a serine (S) residue, the mutation at position 368 introduces an alanine (A), and the mutation at position 407 introduces a valine (V). In embodiments, the mutations comprise T366S, L368A and Y407V. In one embodiment the first CH3 domain of the multispecific molecule comprises the mutation T366Y, and the second CH3 domain that heterodimerizes with the first CH3 domain comprises the mutations T366S, L368A and Y407V, or vice versa. In one embodiment the first CH3 domain of the multispecific molecule comprises the mutation T366W, and the second CH3 domain that heterodimerizes with the first CH3 domain comprises the mutations T366S, L368A and Y407V, or vice versa.

Additional knob in hole mutation pairs suitable for use in any of the multispecific molecules of the present invention are further described in, for example, WO1996/027011, and Merchant et al., Nat. Biotechnol., 16:677-681 (1998), the contents of which are hereby incorporated by reference in their entirety.

In any of the embodiments described herein, the CH3 domains may be additionally mutated to introduce a pair of cysteine residues. Without being bound by theory, it is believed that the introduction of a pair of cysteine residues capable of forming a disulfide bond provide stability to the heterodimerized multispecific molecule. In embodiments, the first CH3 domain comprises a cysteine at position 354, according to the EU numbering scheme of Kabat et al. (pp. 688-696 in Sequences of proteins of immunological interest, 5th ed., Vol. 1 (1991; NIH, Bethesda, Md.)), and the second CH3 domain that heterodimerizes with the first CH3 domain comprises a cysteine at position 349, according to the EU numbering scheme of Kabat et al. (pp. 688-696 in Sequences of proteins of immunological interest, 5th ed., Vol. 1 (1991; NIH, Bethesda, Md.)) In embodiments, the first CH3 domain of the multispecific molecule comprises a cysteine at position 354 (e.g., comprises the mutation S354C) and a tyrosine (Y) at position 366 (e.g., comprises the mutation T366Y), and the second CH3 domain that heterodimerizes with the first CH3 domain comprises a cysteine at position 349 (e.g., comprises the mutation Y349C), a serine at position 366 (e.g., comprises the mutation T366S), an alanine at position 368 (e.g., comprises the mutation L368A), and a valine at position 407 (e.g., comprises the mutation Y407V). In embodiments, the first CH3 domain of the multispecific molecule comprises a cysteine at position 354 (e.g., comprises the mutation S354C) and a tryptophan (W) at position 366 (e.g., comprises the mutation T366W), and the second CH3 domain that heterodimerizes with the first CH3 domain comprises a cysteine at position 349 (e.g., comprises the mutation Y349C), a serine at position 366 (e.g., comprises the mutation T366S), an alanine at position 368 (e.g., comprises the mutation L368A), and a valine at position 407 (e.g., comprises the mutation Y407V).

### IgG Heterodimerization

In one aspect, heterodimerization of the polypeptide chains (e.g., of the half antibodies) of the multispecific molecule is increased by introducing one or more mutations in a CH3 domain which is derived from the IgG1 antibody class. In an embodiment, the mutations comprise a K409R mutation to one CH3 domain paired with F405L mutation in the second CH3 domain, according to the EU numbering scheme of Kabat et al. (pp. 688-696 in Sequences of proteins of immunological interest, 5th ed., Vol. 1 (1991; NIH, Bethesda, Md.)). Additional mutations may also, or alternatively, be at positions 366, 368, 370, 399, 405, 407, and 409 according to the EU numbering scheme of Kabat et al. (pp. 688-696 in Sequences of proteins of immunological interest, 5th ed., Vol. 1 (1991; NIH, Bethesda, Md.)). Preferably, heterodimerization of polypeptides comprising such mutations is achieved under reducing conditions, e.g., 10-100 mM 2-MEA (e.g., 25, 50, or 100 mM 2-MEA) for 1-10, e.g., 1.5-5, e.g., 5, hours at 25-37C, e.g., 25C or 37C.

The amino acid replacements described herein are introduced into the CH3 domains using techniques which are well known in the art. Normally the DNA encoding the heavy chain(s) is genetically engineered using the techniques described in Mutagenesis: a Practical Approach. Oligonucleotide-mediated mutagenesis is a preferred method for preparing substitution variants of the DNA encoding the two hybrid heavy chains. This technique is well known in the art as described by Adelman et al., (1983) DNA, 2:183.

The IgG heterodimerization strategy is described in, for example, WO2008/119353, WO2011/131746, and WO2013/060867, the contents of which are hereby incorporated by reference in their entirety.

In any of the embodiments described herein, the CH3 domains may be additionally mutated to introduce a pair of cysteine residues. Without being bound by theory, it is believed that the introduction of a pair of cysteine residues capable of forming a disulfide bond provide stability to the heterodimerized multispecific molecule. In embodiments, the first CH3 domain comprises a cysteine at position 354, according to the EU numbering scheme of Kabat et al. (pp. 688-696 in Sequences of proteins of immunological interest, 5th ed., Vol. 1 (1991; NIH, Bethesda, Md.)), and the second CH3 domain that heterodimerizes with the first CH3 domain comprises a cysteine at position 349, according to the EU numbering scheme of Kabat et al. (pp. 688-696 in Sequences of proteins of immunological interest, 5th ed., Vol. 1 (1991; NIH, Bethesda, Md.))

### Polar Bridge

In one aspect, heterodimerization of the polypeptide chains (e.g., of the half antibodies) of the multispecific molecule is increased by introducing mutations based on the "polar-bridging" rational, which is to make residues at the binding interface of the two polypeptide chains to interact with residues of similar (or complimentary) physical property in the heterodimer configuration, while with residues of different physical property in the homodimer configuration. In particular, these mutations are designed so that, in the heterodimer formation, polar residues interact with polar residues, while hydrophobic residues interact with hydrophobic residues. In contrast, in the homodimer formation, residues are mutated so that polar residues interact with hydrophobic residues. The favorable interactions in the heterodimer configuration and the unfavorable interactions in the homodimer configuration work together to make it more likely for CH3 domains to form heterodimers than to form homodimers.

In an exemplary embodiment, the above mutations are generated at one or more positions of residues 364, 368, 399, 405, 409, and 411 of CH3 domain, amino acid numbering according to the EU numbering scheme of Kabat et al. (pp. 688-696 in Sequences of proteins of immunological interest, 5th ed., Vol. 1 (1991; NIH, Bethesda, Md.)).

In one aspect, one or more mutations selected from a group consisting of: Ser364Leu, Thr366Val, Leu368Gln, Asp399Lys, Phe405Ser, Lys409Phe and Thr411Lys are introduced into one of the two CH3 domains. (Ser364Leu: original residue of serine at position 364 is replaced by leucine; Thr366Val: original residue of threonine at position 366 is replaced by valine; Leu368Gln: original residue of leucine at position 368 is replaced by glutamine; Asp399Lys: original residue aspartic acid at position 399 is replaced by lysine; Phe405Ser: original residue phenylalanine at position 405 is replaced by serine; Lys409Phe: original residue lysine at position 409 is replaced by phenylalanine; Thr411Lys: original residue of threonine at position 411 is replaced by lysine.).

In another aspect, the other CH3 can be introduced with one or more mutations selected from a group consisting of: Tyr407Phe, Lys409Gln and Thr411Asp (Tyr407Phe: original residue tyrosine at position 407 is replaced by phenyalanine; Lys409Glu: original residue lysine at position 409 is replaced by glutamic acid; Thr411Asp: original residue of threonine at position 411 is replaced by aspartic acid).

In a further aspect, one CH3 domain has one or more mutations selected from a group consisting of: Ser364Leu, Thr366Val, Leu368Gln, Asp399Lys, Phe405Ser, Lys409Phe and Thr411Lys, while the other CH3 domain has one or more mutations selected from a group consisting of: Tyr407Phe, Lys409Gln and Thr411Asp.

In one exemplary embodiment, the original residue of threonine at position 366 of one CH3 domain is replaced by valine, while the original residue of tyrosine at position 407 of the other CH3 domain is replaced by phenylalanine.

In another exemplary embodiment, the original residue of serine at position 364 of one CH3 domain is replaced by leucine, while the original residue of leucine at position 368 of the same CH3 domain is replaced by glutamine.

In yet another exemplary embodiment, the original residue of phenylalanine at position 405 of one CH3 domain is replaced by serine and the original residue of lysine at position 409 of this CH3 domain is replaced by phenylalanine, while the original residue of lysine at position 409 of the other CH3 domain is replaced by glutamine.

In yet another exemplary embodiment, the original residue of aspartic acid at position 399 of one CH3 domain is replaced by lysine, and the original residue of threonine at position 411 of the same CH3 domain is replaced by lysine, while the original residue of threonine at position 411 of the other CH3 domain is replaced by aspartic acid.

The amino acid replacements described herein are introduced into the CH3 domains using techniques which are well known in the art. Normally the DNA encoding the heavy chain(s) is genetically engineered using the techniques described in Mutagenesis: a Practical Approach. Oligonucleotide-mediated mutagenesis is a preferred method for preparing substitution variants of the DNA encoding the two hybrid heavy chains. This technique is well known in the art as described by Adelman et al., (1983) DNA, 2:183.

The polar bridge strategy is described in, for example, WO2006/106905, WO2009/089004 and K.Gunasekaran, et al. (2010) The Journal of Biological Chemistry, 285:19637-19646, the contents of which are hereby incorporated by reference in their entirety.

In any of the embodiments described herein, the CH3 domains may be additionally mutated to introduce a pair of cysteine residues. Without being bound by theory, it is believed that the introduction of a pair of cysteine residues capable of forming a disulfide bond provide stability to the heterodimerized multispecific molecule. In embodiments, the first CH3 domain comprises a cysteine at position 354, according to the EU numbering scheme of Kabat et al. (pp. 688-696 in Sequences of proteins of immunological interest, 5th ed., Vol. 1 (1991; NIH, Bethesda, Md.)), and the second CH3 domain that heterodimerizes with the first CH3 domain comprises a cysteine at position 349, according to the EU numbering scheme of Kabat et al. (pp. 688-696 in Sequences of proteins of immunological interest, 5th ed., Vol. 1 (1991; NIH, Bethesda, Md.))

### II. Molecules With Variable Region Modifications

Each of the N-terminal VH and VL domains and C-terminal VH and VL domains of the molecule (e.g. antibody or antibody-like molecule) of the present invention comprises hypervariable regions CDR1, CDR2, and CDR3 sequences. In certain embodiments, one or more of these CDR sequences have conservative modifications of the amino acid sequences, and wherein the modified molecules retain or have enhanced binding properties as compared to the parent antibodies.

In addition, it has been found that in certain instances it is beneficial to mutate residues within the framework regions to maintain or enhance the antigen binding ability of the antibody (see *e.g.,* U.S. Patent Nos. 5,530,101; 5,585,089; 5,693,762 and 6,180,370 to Queen *et al*). The molecules (e.g. antibodies or antibody-like molecules) of the present invention can be modified by introducing such mutations to its variable region frameworks in order to improve the binding properties.

Another type of variable region modification is to mutate amino acid residues within the VH and/or VL CDR1, CDR2 and/or CDR3 domains to thereby improve one or more binding properties (*e.g.*, affinity) of the molecule (e.g. antibody or antibody-like molecule) of interest, known as "affinity maturation." Site-directed mutagenesis or PCR-mediated mutagenesis can be performed to introduce the mutation(s) and the effect on antibody binding, or other functional property of interest, can be evaluated in *in vitro* or *in vivo* assays as described herein and provided in the Examples. Conservative modifications (as discussed above) can be introduced. The mutations may be amino acid substitutions, additions or deletions. Moreover, typically no more than one, two, three, four or five residues within a CDR region are altered.

Amino acid sequence variants of the present molecules can be prepared by introducing appropriate nucleotide changes into the encoding DNAs, or by synthesis of the desired variants. Such variants include, for example, deletions from, or insertions or substitutions of, residues within the amino acid sequences of present molecules. Any combination of deletion, insertion, and substitution is made to arrive at the final construct, provided that the final construct possesses the desired antigen-binding characteristics. The amino acid changes also may alter post-translational processes of the molecules, such as changing the number or position of glycosylation sites.

The present application includes variants of the molecules described herein and/or fragments thereof having amino acid conservative modifications in variable regions and/or constant regions.

### III. Molecules with an Extended in vivo Half-life.

The present molecule can be further modified to have an extended half-life *in vivo.*

A variety of strategies can be used to extend the half life of the molecules of the present invention. For example, by chemical linkage to polyethyleneglycol (PEG), reCODE PEG, antibody scaffold, polysialic acid (PSA), hydroxyethyl starch (HES), albumin-binding ligands, and carbohydrate shields; by genetic fusion to proteins binding to serum proteins, such as albumin, IgG, FcRn, and transferring; by coupling (genetically or chemically) to other binding moieties that bind to serum proteins, such as nanobodies, Fabs, DARPins, avimers, affibodies, and anticalins; by genetic fusion to rPEG, albumin, domain of albumin, albumin-binding proteins, and Fc; or by incorporation into nanocarriers, slow release formulations, or medical devices.

The molecules of the present invention having an increased half-life *in vivo* can also be generated introducing one or more amino acid modifications (*i.e.,* substitutions, insertions or deletions) into an IgG constant domain, or FcRn binding fragment thereof (preferably a Fc or hinge Fc domain fragment). See, *e.g.*, International Publication No. WO 98/23289; International Publication No. WO 97/34631; and U.S. Patent No. 6,277,375.

Further, the molecules can be conjugated to albumin in order to make the molecules more stable *in vivo* or have a longer half life in *vivo.* The techniques are well-known in the art, see, *e.g.*, International Publication Nos. WO 93/15199, WO 93/15200, and WO 01/77137; and European Patent No. EP 413,622.

The molecules of the present invention may also be fused to one or more human serum albumin (HSA) polypeptides, or a portion thereof. The use of albumin as a component of an albumin fusion protein as a carrier for various proteins has been suggested in WO 93/15199, WO 93/15200, and EP 413 622. The use of N-terminal fragments of HSA for fusions to polypeptides has also been proposed (EP 399 666). Accordingly, by genetically or chemically fusing or conjugating the molecules to albumin, can stabilize or extend the shelf-life, and/or to retain the molecule's activity for extended periods of time in solution, in vitro and/or in vivo. Additional methods pertaining to HSA fusions can be found, for example, in WO 2001077137 and WO 200306007, incorporated herein by reference. In a specific embodiment, the expression of the fusion protein is performed in mammalian cell lines, for example, CHO cell lines.

### IV. Fc Silencing

Without being bound by theory, in embodiments that incorporate one or more constant domains, e.g., heavy chain constant regions, it may be beneficial to include one or mutations to silence, e.g., ADCC and/or CDC effector function within hFc. Activation of the immune cell occurs preferentially in the presence of crosslinking to the target cell. However, human Fc may bind to high and low affinity FcR gamma receptors. Therefore, crosslinking of receptors (e.g. CD3) on the immune cell and subsequent agonism may occur upon binding in the absence of tumor targeting. Additionally, crosslinking of Fc via gamma receptors may induce antibody dependent cellular cytotoxicity (ADCC). Human Fc when complexed at the cell surface can also bind complement proteins and induce complement dependent cytotoxicity (CDC). Mutations to residues in Fc which reduce or abrogate these interactions may thus limit these effects and focus the impact of the molecules described herein upon the tumor target cell.. In embodiments, one or more, e.g., all, of the heavy chain constant region domains of the multispecific molecule, e.g., bispecific molecule, e.g., bispecific antibody or antibody-like molecule comprise the DAPA mutation (e.g. D265A and P329A in EU numbering). See e.g., Shields RL, Namenuk AK, Hong K, Meng YG, Rae J, Briggs J, Xie D, Lai J, Stadlen A, Li B, Fox JA, Presta LG. High resolution mapping of the binding site on human IgG1 for Fc gamma RI, Fc gamma RII, Fc gamma RIII, and FcRn and design of IgG1 variants with improved binding to the Fc gamma R. J Biol Chem. 2001;276(9):6591-604; U.S. Patent Publication US2015/0320880 A1, the contents of each of which are incorporated by reference in their entireties. In embodiments, one or more, e.g., all, of the heavy chain constant region domains of the multispecific molecule, e.g., bispecific molecule, e.g., bispecific antibody or antibody-like molecule comprise the LALA mutation (e.g., L234A and L235A in EU numbering). E.g., Hezareh M, Hessell AJ, Jensen RC, van de Winkel JGJ, Parren PWHI. Effector Function Activities of a Panel of Mutants of a Broadly Neutralizing Antibody against Human Immunodeficiency Virus Type 1. Journal of Virology. 2001;75(24): 12161-12168; Shields RL, Namenuk AK, Hong K, Meng YG, Rae J, Briggs J, Xie D, Lai J, Stadlen A, Li B, Fox JA, Presta LG. High resolution mapping of the binding site on human IgG1 for Fc gamma RI, Fc gamma RII, Fc gamma RIII, and FcRn and design of IgG1 variants with improved binding to the Fc gamma R. J Biol Chem. 2001;276(9):6591-604, the contents of each of which are incorporated by reference in their entirety. In embodiments, one or more, e.g., all, of the heavy chain constant region domains of the multispecific molecule, e.g., bispecific molecule, e.g., bispecific antibody or antibody-like molecule comprise an N279A mutation (according to EU numbering). E.g., Tao MH(1), Morrison SL. Studies of aglycosylated chimeric mouse-human IgG. Role of carbohydrate in the structure and effector functions mediated by the human IgG constant region. J Immunol. 1989; 143(8):2595-601; Shields RL, Namenuk AK, Hong K, Meng YG, Rae J, Briggs J, Xie D, Lai J, Stadlen A, Li B, Fox JA, Presta LG. High resolution mapping of the binding site on human IgG1 for Fc gamma RI, Fc gamma RII, Fc gamma RIII, and FcRn and design of IgG1 variants with improved binding to the Fc gamma R. J Biol Chem. 2001;276(9):6591-604, the contents of each of which are incorporated by reference in their entirety.

### V. Conjugates

The present invention includes multispecific molecules (e.g. antibodies or antibody-like molecules) or the fragments thereof recombinantly fused or chemically conjugated (including both covalent and non-covalent conjugations) to a heterologous protein or polypeptide (or fragment thereof, preferably to a polypeptide of at least 10, at least 20, at least 30, at least 40, at least 50, at least 60, at least 70, at least 80, at least 90 or at least 100 amino acids) to generate fusion proteins. Methods for fusing or conjugating proteins, polypeptides, or peptides to an antibody or an antibody fragment are known in the art. See, *e.g.,* U.S. Patent Nos. 5,336,603, 5,622,929, 5,359,046, 5,349,053, 5,447,851, and 5,112,946; European Patent Nos. EP 307,434 and EP 367,166; International Publication Nos. WO 96/04388 and WO 91/06570; Ashkenazi et al., (1991) Proc. Natl. Acad. Sci. USA 88:10535-10539; Zheng et al., (1995) J. Immunol. 154:5590-5600; and Vil et al., (1992) Proc. Natl. Acad. Sci. USA 89:11337- 11341.

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of molecules of the invention or fragments thereof (*e.g.*, molecules or fragments thereof with higher affinities and lower dissociation rates). See, generally, U.S. Patent Nos. 5,605,793, 5,811,238, 5,830,721, 5,834,252, and 5,837,458; Patten et al., (1997) Curr. Opinion Biotechnol. 8:724-33; Harayama, (1998) Trends Biotechnol. 16(2):76-82; Hansson et al., (1999) J. Mol. Biol. 287:265-76; and Lorenzo and Blasco, (1998) Biotechniques 24(2):308- 313 (each of these patents and publications are hereby incorporated by reference in its entirety). The molecules described herein or fragments thereof may be altered by being subjected to random mutagenesis by error-prone PCR, random nucleotide insertion or other methods prior to recombination. A polynucleotide encoding a fragment of the present molecule may be recombined with one or more components, motifs, sections, parts, domains, fragments, etc. of one or more heterologous molecules.

Moreover, the present molecules or fragments thereof can be fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., (1989) Proc. Natl. Acad. Sci. USA 86:821-824, for instance, hexa-histidine provides for convenient purification of the fusion protein. Other peptide tags useful for purification include, but are not limited to, the hemagglutinin ("HA") tag, which corresponds to an epitope derived from the influenza hemagglutinin protein (Wilson et al., (1984) Cell 37:767), and the "flag" tag.

In other embodiments, the molecules of the present invention or fragments thereof are conjugated to a diagnostic or detectable agent. Such molecules can be useful for monitoring or prognosing the onset, development, progression and/or severity of a disease or disorder as part of a clinical testing procedure, such as determining the efficacy of a particular therapy. Such diagnosis and detection can accomplished by coupling the molecules to detectable substances including, but not limited to, various enzymes, such as, but not limited to, horseradish peroxidase, alkaline phosphatase, beta-galactosidase, or acetylcholinesterase; prosthetic groups, such as, but not limited to, streptavidinlbiotin and avidin/biotin; fluorescent materials, such as, but not limited to, umbelliferone, fluorescein, fluorescein isothiocynate, rhodamine, dichlorotriazinylamine fluorescein, dansyl chloride or phycoerythrin; luminescent materials, such as, but not limited to, luminol; bioluminescent materials, such as but not limited to, luciferase, luciferin, and aequorin; radioactive materials, such as, but not limited to, iodine (¹²⁵I, ¹²³I, and ¹²¹I,), carbon (¹⁴C), sulfur (³⁵S), tritium (³H), indium (¹¹⁵In, ¹¹³In, ¹¹²In, and ¹¹¹In,), technetium (⁹⁹Tc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, 47Sc, ¹¹⁶Re, ¹⁸⁸Re, ¹⁴² Pr, ¹⁰⁵Rh, ⁹⁷Ru, ⁶⁸Ge, ⁵⁷Co, ⁶⁵Zn, ⁸⁵Sr, ³²P, ¹⁵³Gd, ¹⁶⁹Yb, ⁵¹Cr, ⁵⁴Mn, ⁷⁵Se, ¹¹³Sn, and ¹¹⁷Tin; and positron emitting metals using various positron emission tomographies, and nonradioactive paramagnetic metal ions.

The present application further encompasses uses of the present molecules or fragments thereof conjugated to a therapeutic moiety. The molecules of the present invention or fragments thereof may be conjugated to a therapeutic moiety such as a cytotoxin, *e.g.*, a cytostatic or cytocidal agent, a therapeutic agent or a radioactive metal ion, *e.g.,* alpha-emitters. A cytotoxin or cytotoxic agent includes any agent that is detrimental to cells.

Further, the present molecule or fragment thereof may be conjugated to a therapeutic moiety or drug moiety that modifies a given biological response. Therapeutic moieties or drug moieties are not to be construed as limited to classical chemical therapeutic agents. For example, the drug moiety may be a protein, peptide, or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, an anti-angiogenic agent; or, a biological response modifier such as, for example, a lymphokine.

In one embodiment, the present molecule, or a fragment thereof, is conjugated to a therapeutic moiety, such as a cytotoxin, a drug (e.g., an immunosuppressant) or a radiotoxin. Such conjugates are referred to herein as "immunoconjugates". Immunoconjugates that include one or more cytotoxins are referred to as "immunotoxins." A cytotoxin or cytotoxic agent includes any agent that is detrimental to (e.g., kills) cells. Examples include taxon, cytochalasin B, gramicidin D, ethidium bromide, emetine, mitomycin, etoposide, tenoposide, vincristine, vinblastine, t. colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, for example, antimetabolites (e.g., methotrexate, 6-mercaptopurine, 6-thioguanine, cytarabine, 5-fluorouracil decarbazine), ablating agents (e.g., mechlorethamine, thioepa chloraxnbucil, meiphalan, carmustine (BSNU) and lomustine (CCNU), cyclothosphamide, busulfan, dibromomannitol, streptozotocin, mitomycin C, and cis-dichlorodiamine platinum (II) (DDP) cisplatin, anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). (See e.g., Seattle Genetics US20090304721).

Other examples of therapeutic cytotoxins that can be conjugated to the molecules of the present invention include duocarmycins, calicheamicins, maytansines and auristatins, and derivatives thereof. An example of a calicheamicin antibody conjugate is commercially available (Mylotarg™; Wyeth-Ayerst).

Cytoxins can be conjugated to the molecules of the invention using linker technology available in the art. Examples of linker types that have been used to conjugate a cytotoxin to an antibody include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumor tissue such as cathepsins (e.g., cathepsins B, C, D).

For further discussion of types of cytotoxins, linkers and methods for conjugating therapeutic agents to the molecules, see also Saito et al., (2003) Adv. Drug Deliv. Rev. 55:199-215; Trail et al., (2003) Cancer Immunol. Immunother. 52:328-337; Payne, (2003) Cancer Cell 3:207-212; Allen, (2002) Nat. Rev. Cancer 2:750-763; Pastan and Kreitman, (2002) Curr. Opin. Investig. Drugs 3:1089-1091; Senter and Springer, (2001) Adv. Drug Deliv. Rev. 53:247-264.

The molecules of the present invention also can be conjugated to a radioactive isotope to generate cytotoxic radiopharmaceuticals, also referred to as radioimmunoconjugates. Examples of radioactive isotopes that can be conjugated to molecules for use diagnostically or therapeutically include, but are not limited to, iodine¹³¹, indium¹¹¹, yttrium⁹⁰, and lutetium¹⁷⁷. Method for preparing radioimmunconjugates are established in the art. Examples of radioimmunoconjugates are commercially available, including Zevalin™ (DEC Pharmaceuticals) and Bexxar™ (Corixa Pharmaceuticals), and similar methods can be used to prepare radioimmunoconjugates using the molecules of the invention. In certain embodiments, the macrocyclic chelator is 1,4,7,10-tetraazacyclododecane-N,N',N",N"'-tetraacetic acid (DOTA) which can be attached to the antibody via a linker molecule. Such linker molecules are commonly known in the art and described in Denardo et al., (1998) Clin Cancer Res. 4(10):2483-90; Peterson et al., (1999) Bioconjug. Chem. 10(4):553-7; and Zimmerman et al., (1999) Nucl. Med. Biol. 26(8):943-50, each incorporated by reference in their entireties.

Techniques for conjugating therapeutic moieties to antibodies or antibody-like molecules are well known, see, *e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies 84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., (1982) Immunol. Rev. 62:119-58.

The molecules may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### VII. Methods of Making the Molecules of the Present Invention

Where polypeptides of the multispecific molecules of the present invention are crosslinked, these functional linkages can be accomplished using methods known in the art. A variety of coupling or cross-linking agents can be used for covalent conjugation. Examples of cross-linking agents include protein A, carbodiimide, N-succinimidyl-S-acetyl-thioacetate (SATA), 5,5'-dithiobis(2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl) cyclohexane-1-carboxylate (sulfo-SMCC) (see *e.g.,* Karpovsky et al., (1984) J. Exp. Med. 160:1686; Liu et al. (1985) Proc. Natl. Acad. Sci. USA 82:8648). Other methods include those described in Paulus (1985) Behring Ins. Mitt. No. 78:118-132; Brennan et al., (1985) Science 229:81-83), and Glennie et al., (1987) J. Immunol. 139: 2367-2375). Conjugating agents are SATA and sulfo-SMCC, both available from Pierce Chemical Co. (Rockford, IL).

Alternatively, the present molecules can be generated recombinantly by introducing DNA constructs encoding the desired molecules into expression vectors and expressing and assembling the desired molecules in the same host cells.

### A. Preparing Polypeptide Chains

The first step of producing the present molecules is preparing the half-antibodies or component polypeptides (i.e., the one or more polypeptide chains of the molecules comprising the first and second antigen-binding domains). If the molecules are produced recombinantly, the nucleic acid molecules encoding the first and second half antibodies may be prepared first.

Polypeptides and antibodies and fragments thereof (e.g., half antibodies) can be produced by a variety of techniques, including conventional monoclonal antibody methodology *e.g.*, the standard somatic cell hybridization technique of Kohler and Milstein, (1975) Nature 256: 495. Many techniques for producing monoclonal antibody can be employed *e.g.*, viral or oncogenic transformation of B lymphocytes.

An animal system for preparing hybridomas is the murine system. Hybridoma production in the mouse is a well-established procedure. Immunization protocols and techniques for isolation of immunized splenocytes for fusion are known in the art. Fusion partners (*e.g.*, murine myeloma cells) and fusion procedures are also known.

Chimeric or humanized antibodies used in the present invention can be prepared based on the sequence of a murine monoclonal antibody prepared as described above. DNA encoding the heavy and light chain immunoglobulins can be obtained from the murine hybridoma of interest and engineered to contain non-murine (*e.g.*, human) immunoglobulin sequences using standard molecular biology techniques. For example, to create a chimeric antibody, the murine variable regions can be linked to human constant regions using methods known in the art (see *e.g.,* U.S. Pat. No. 4,816,567 to Cabilly *et al*.). To create a humanized antibody, the murine CDR regions can be inserted into a human framework using methods known in the art. See *e.g.,* U.S. Pat. No. 5225539 to Winter, and U.S. Pat. Nos. 5,530,101; 5,585,089; 5,693,762 and 6,180,370 to Queen *et al.*

In a certain embodiment, the antibody or antibody-like molecules of the invention are human monoclonal antibodies. Such human monoclonal antibodies can be generated using transgenic or transchromosomic mice carrying parts of the human immune system rather than the mouse system. These transgenic and transchromosomic mice include mice referred to herein as HuMAb mice and KM mice, respectively, and are collectively referred to herein as "human Ig mice."

The HuMAb mouse® (Medarex, Inc.) contains human immunoglobulin gene miniloci that encode un-rearranged human heavy (µ and γ) and κ light chain immunoglobulin sequences, together with targeted mutations that inactivate the endogenous µ and κ chain loci (see *e.g.,* Lonberg, et al., (1994) Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGκ monoclonal (Lonberg *et al.,* (1994) supra; reviewed in Lonberg, (1994) Handbook of Experimental Pharmacology 113:49-101; Lonberg and Huszar, (1995) Intern. Rev. Immunol.13: 65-93, and Harding and Lonberg, (1995) Ann. N. Y. Acad. Sci. 764:536-546). The preparation and use of HuMAb mice, and the genomic modifications carried by such mice, is further described in Taylor et al., (1992) Nucleic Acids Research 20:6287-6295; Chen et al., (1993) International Immunology 5: 647-656; Tuaillon et al., (1993) Proc. Natl. Acad. Sci. USA 94:3720-3724; Choi et al., (1993) Nature Genetics 4:117-123; Chen et al., (1993) EMBO J. 12:821-830; Tuaillon et al., (1994) J. Immunol. 152:2912-2920; Taylor et al., (1994) International Immunology 579-591; and Fishwild et al., (1996) Nature Biotechnology 14: 845-851, the contents of all of which are hereby specifically incorporated by reference in their entirety. See further, U.S. Pat. Nos. 5,545,806; 5,569,825; 5,625,126; 5,633,425; 5,789,650; 5,877,397; 5,661,016; 5,814,318; 5,874,299; and 5,770,429; all to Lonberg and Kay; U.S. Patent No. 5,545,807 to Surani et al.; PCT Publication Nos. WO 92103918, WO 93/12227, WO 94/25585, WO 97113852, WO 98/24884 and WO 99/45962, all to Lonberg and Kay; and PCT Publication No. WO 01/14424 to Korman et al.

In another embodiment, human antibodies used in the present invention can be raised using a mouse that carries human immunoglobulin sequences on transgenes and transchomosomes such as a mouse that carries a human heavy chain transgene and a human light chain transchromosome. Such mice, referred to herein as "KM mice", are described in detail in PCT Publication WO 02/43478 to Ishida et al.

Still further, alternative transgenic animal systems expressing human immunoglobulin genes are available in the art and can be used to raise human antibodies used in the present invention. For example, an alternative transgenic system referred to as the Xenomouse (Abgenix, Inc.) can be used. Such mice are described in, *e.g.,* U.S. Pat. Nos. 5,939,598; 6,075,181; 6,114,598; 6, 150,584 and 6,162,963 to Kucherlapati et al.

Moreover, alternative transchromosomic animal systems expressing human immunoglobulin genes are available in the art and can be used to raise the human antibodies used in the invention. For example, mice carrying both a human heavy chain transchromosome and a human light chain tranchromosome, referred to as "TC mice" can be used; such mice are described in Tomizuka et al., (2000) Proc. Natl. Acad. Sci. USA 97:722-727. Furthermore, cows carrying human heavy and light chain transchromosomes have been described in the art (Kuroiwa et al., (2002) Nature Biotechnology 20:889-894) and can be used to raise human antibodies used in the present application.

Human monoclonal antibodies can also be prepared using phage display methods for screening libraries of human immunoglobulin genes. Such phage display methods for isolating human antibodies are established in the art or described in the examples below. See for example: U.S. Pat. Nos. 5,223,409; 5,403,484; and 5,571,698 to Ladner *et* al.; U.S. Patent Nos. 5,427,908 and 5,580,717 to Dower *et al*.; U.S. Patent Nos. 5,969,108 and 6,172,197 to McCafferty *et al*.; and U.S. Patent Nos. 5,885,793; 6,521,404; 6,544,731; 6,555,313; 6,582,915 and 6,593,081 to Griffiths et al.

Human monoclonal antibodies used in the invention can also be prepared using SCID mice into which human immune cells have been reconstituted such that a human antibody response can be generated upon immunization. Such mice are described in, for example, U.S. Pat. Nos. 5,476,996 and 5,698,767 to Wilson *et al.*

Methods of making bispecific antibodies are known in the art and discussed in the present application.

### B. Methods of Producing Molecules of the Present Invention Recombinantly

In one embodiment, the present application provides a method of producing the one or more polypeptide chains of the multispecific molecule recombinantly, comprising: 1) producing one or more DNA constructs comprising a nucleic acid molecule encoding each of the polypeptide chains of the multispecific molecule; 2) introducing said DNA construct(s) into one or more expression vectors; 3) co-transfecting said expression vector(s) in one or more host cells; and 4) expressing and assembling the molecule in a host cell or in solution.

In this respect, the disclosure provides isolated nucleic acid, e.g., one or more polynucleotides, encoding the multispecific molecule described herein, for example a multispecific molecule that includes an anti-CD3 binding domain, e.g., as described herein, and an anti-CLL-1 binding domain, e.g., as describe herien. In embodiments, the isolated nucleic acid is disposed on a single continuous polynucleotide. In other embodiments, the isolated polynucleotide is disposed on two or more continuous polynucleotides.

In aspects, the nucleic acid includes sequence encoding an anti-CD3 binding domain. In aspects, the nucleic acid includes SEQ ID NO: 508 and SEQ ID NO: 509.

In aspects, the isolated nucleic acid includes SEQ ID NO: 510.

In aspects, the isolated nucleic acid includes SEQ ID NO: 511.

In aspects, the nucleic acid includes sequence encoding an anti-CLL-1 binding domain. In aspects, the isolated nucleic acid includes:
a) SEQ ID NO: 513 and SEQ ID NO: 514;
b) SEQ ID NO: 518 and SEQ ID NO: 519;
c) SEQ ID NO: 523 and SEQ ID NO: 524;
d) SEQ ID NO: 528 and SEQ ID NO: 529;
e) SEQ ID NO: 533 and SEQ ID NO: 534;
f) SEQ ID NO: 538 and SEQ ID NO: 539;
g) SEQ ID NO: 543 and SEQ ID NO: 544;
h) SEQ ID NO: 548 and SEQ ID NO: 549; or
i) SEQ ID NO: 553 and SEQ ID NO: 554.

In aspects, the isolated nucleic acid includes:
a) SEQ ID NO: 515;
b) SEQ ID NO: 520;
c) SEQ ID NO: 525;
d) SEQ ID NO: 530;
e) SEQ ID NO: 535;
f) SEQ ID NO: 540;
g) SEQ ID NO: 545;
h) SEQ ID NO: 550; or
i) SEQ ID NO: 555.

In aspects, the isolated nucleic acid includes:
a) SEQ ID NO: 516;
b) SEQ ID NO: 521;
c) SEQ ID NO: 526;
d) SEQ ID NO: 531;
e) SEQ ID NO: 536;
f) SEQ ID NO: 541;
g) SEQ ID NO: 546;
h) SEQ ID NO: 551; or
i) SEQ ID NO: 556.

In aspects, the isolated nucleic acid includes sequence encoding an anti-CD3 binding domain, for example, as described herein, and sequence encoding an anti-CLL-1 binding domain, for example, as described herein. In aspects, the sequence encoding the anti-CD3 binding domain and the sequence encoding the anti-CLL-1 binding domain are disposed on separate polynucleotides. In aspects, the sequence encoding the anti-CD3 binding domain and the sequence encoding the anti-CLL-1 binding domain are disposed on a single polynucleotide.

In an exemplary embodiment, the DNA sequences encoding the light chain of the first half antibody, the DNA sequence encoding the heavy chain of the first half antibody, the DNA sequences encoding the light chain of the second half antibody, and the DNA sequence encoding the heavy chain of the second half antibody are placed in separate expression vectors. The expression vectors are then co-transfected into a host cell at a ratio giving rise to optimal assembly. The encoded heavy chains and light chains are expressed in the host cell and assemble into functional molecules.

In another exemplary embodiment, the DNA sequences encoding the heavy and light chains of the first half antibody are placed in one expression vector, and the DNA sequences encoding the heavy and light chains of the second half antibody are placed in a second expression vector. The expression vectors may then be co-transfected into a host cell at a ratio giving rise to optimal assembly. The encoded heavy chains and light chains are expressed in the host cell and assemble into functional molecules. Alternatively, the expression vectors may be transfected into different host cell populations, and the multispecific molecule assembled in solution.

Desired mutations on the variable region or the constant region of the molecule described herein, such as, for enhancing hetero-dimerization, can be introduced at this stage as described herein.

The DNA sequences can be produced by de novo solid-phase DNA synthesis or by PCR mutagenesis of an existing sequence (*e.g.*, sequences as described in the Examples below) encoding heavy or light chains of the present molecules. Direct chemical synthesis of nucleic acids can be accomplished by methods known in the art, such as the phosphotriester method of Narang et al., (1979) Meth. Enzymol. 68:90; the phosphodiester method of Brown et al., (1979) Meth. Enzymol. 68:109; the diethylphosphoramidite method of Beaucage et al., (1981) Tetra. Lett., 22:1859; and the solid support method of U.S. Patent No. 4,458,066. Introducing mutations to a polynucleotide sequence by PCR can be performed as described in, *e.g.,* PCR Technology: Principles and Applications for DNA Amplification, H.A. Erlich (Ed.), Freeman Press, NY, NY, 1992; PCR Protocols: A Guide to Methods and Applications, Innis et al. (Ed.), Academic Press, San Diego, CA, 1990; Mattila et al., (1991) Nucleic Acids Res. 19:967; and Eckert et al., (1991) PCR Methods and Applications 1:17.

Also provided in the invention are expression vectors and host cells for producing the molecules described above. Various expression vectors can be employed to express the polynucleotides encoding chains or binding domains of the molecule. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (see, *e.g.,* Harrington et al., (1997) Nat Genet 15:345). For example, nonviral vectors useful for expression of the polynucleotides and polypeptides in mammalian (*e.g.*, human) cells include pThioHis A, B & C, pcDNA3.1/His, pEBVHis A, B & C, (Invitrogen, San Diego, CA), MPSV vectors, and numerous other vectors known in the art for expressing other proteins. Useful viral vectors include vectors based on retroviruses, adenoviruses, adeno associated viruses, herpes viruses, vectors based on SV40, papilloma virus, HBP Epstein Barr virus, vaccinia virus vectors and Semliki Forest virus (SFV). See, Brent *et al.,* (1995) supra; Smith, Annu. Rev. Microbiol. 49:807; and Rosenfeld et al., (1992) Cell 68:143.

The choice of expression vector depends on the intended host cells in which the vector is to be expressed. Typically, the expression vectors contain a promoter and other regulatory sequences (*e.g*., enhancers) that are operably linked to the polynucleotides encoding an antibody chain or fragment. In some embodiments, an inducible promoter is employed to prevent expression of inserted sequences except under inducing conditions. Inducible promoters include, *e.g.*, arabinose, lacZ, metallothionein promoter or a heat shock promoter. Cultures of transformed organisms can be expanded under noninducing conditions without biasing the population for coding sequences whose expression products are better tolerated by the host cells. In addition to promoters, other regulatory elements may also be required or desired for efficient expression of the heavy chains and light chains of the multispecific molecules. These elements typically include an ATG initiation codon and adjacent ribosome binding site or other sequences. In addition, the efficiency of expression may be enhanced by the inclusion of enhancers appropriate to the cell system in use (see, *e.g*., Scharf et al., (1994) Results Probl. Cell Differ. 20:125; and Bittner et al., (1987) Meth. Enzymol., 153:516). For example, the SV40 enhancer or CMV enhancer may be used to increase expression in mammalian host cells.

The expression vectors may also provide a secretion signal sequence position to form a fusion protein with polypeptides encoded by inserting the above-described sequences of heavy chain and/or light chain or fragments thereof. More often, the inserted antibody or antibody-like molecule sequences are linked to a signal sequences before inclusion in the vector. Vectors to be used to receive sequences encoding light and heavy chain variable domains sometimes also encode constant regions or parts thereof. Such vectors allow expression of the variable regions as fusion proteins with the constant regions thereby leading to production of intact antibodies or antibody-like molecules or fragments thereof. Typically, such constant regions are human.

The host cells for harboring and expressing the present molecules can be either prokaryotic or eukaryotic. E. coli is one prokaryotic host useful for cloning and expressing the polynucleotides of the present invention. Other microbial hosts suitable for use include bacilli, such as Bacillus subtilis, and other enterobacteriaceae, such as Salmonella, Serratia, and various Pseudomonas species. In these prokaryotic hosts, one can also make expression vectors, which typically contain expression control sequences compatible with the host cell (*e.g.,* an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express the antibody of the invention. Insect cells in combination with baculovirus vectors can also be used.

In some preferred embodiments, mammalian host cells are used to express and produce the molecules of the present invention. For example, they can be either a hybridoma cell line expressing endogenous immunoglobulin genes (*e.g*., the 1D6.C9 myeloma hybridoma clone as described in the Examples) or a mammalian cell line harboring an exogenous expression vector (*e.g*., the SP2/0 myeloma cells exemplified below). These include any normal mortal or normal or abnormal immortal animal or human cell. For example, a number of suitable host cell lines capable of secreting intact immunoglobulins have been developed including the CHO cell lines, various Cos cell lines, HeLa cells, myeloma cell lines, transformed B-cells and hybridomas. The use of mammalian tissue cell culture to express polypeptides is discussed generally in, *e.g.*, Winnacker, FROM GENES TO CLONES, VCH Publishers, N.Y., N.Y., 1987. Expression vectors for mammalian host cells can include expression control sequences, such as an origin of replication, a promoter, and an enhancer (see, *e.g.,* Queen et al., (1986) Immunol. Rev. 89:49-68), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP polIII promoter, the constitutive MPSV promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook, *et al.,* supra). Other methods include, *e.g.,* electroporation, calcium phosphate treatment, liposome-mediated transformation, injection and microinjection, ballistic methods, virosomes, immunoliposomes, polycation:nucleic acid conjugates, naked DNA, artificial virions, fusion to the herpes virus structural protein VP22 (Elliot and O'Hare, (1997) Cell 88:223), agent-enhanced uptake of DNA, and ex vivo transduction. For long-term, high-yield production of recombinant proteins, stable expression will often be desired. For example, cell lines which stably express antibody chains or binding fragments can be prepared using expression vectors of the invention which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type.

The present molecule preferably is generally recovered from the culture medium as a secreted polypeptide, although it also may be recovered from host cell lysate when directly produced without a secretory signal. If the molecule is membrane-bound, it can be released from the membrane using a suitable detergent solution (e.g. Triton-X 100).

When the molecule is produced in a recombinant cell other than one of human origin, it is completely free of proteins or polypeptides of human origin. However, it is necessary to purify the molecule from recombinant cell proteins or polypeptides to obtain preparations that are substantially homogeneous as to heteromultimer. As a first step, the culture medium or lysate is normally centrifuged to remove particulate cell debris. The produced molecules can be conveniently purified by hydroxylapatite chromatography, gel electrophoresis, dialysis, or affinity chromatography, with affinity chromatography being the preferred purification technique. Other techniques for protein purification such as fractionation on an ion-exchange column, ethanol precipitation, reverse phase HPLC, chromatography on silica, chromatography on heparin Sepharose, chromatography on an anion or cation exchange resin (such as a polyaspartic acid column), chromatofocusing, SDS-PAGE, and ammonium sulfate precipitation are also available.

### XI. Use of the Molecules of the Present Invention

### A. Diagnostic and Therapeutic Use

Depending on the antigens that are recognized by the molecules of the present invention, the present molecules have many diagnostic and therapeutic applications. For instance, they can be used for enzyme immunoassay, with N-terminal arms binding a specific epitope on the enzyme and C-terminal arms binding the immobilizing matrix. The enzyme immunoassay using antibody-like molecules is discussed by Nolan *et al.* (Nolan et al., (1990) Biochem. Biophys. Acta. 1040:1-11). The multispecific molecules can also be used for diagnosis of various diseases such as cancer (Songsivilai et al., (1990) Clin. Exp. Immunol. 79:315). In particular, one antigen binding domain of the molecule can bind a cancer antigen and the other binding site can bind a detectable marker described herein, for example, a chelator which tightly binds a radionuclide. (Le Doussal et al., (1992) Int. J. Cancer Suppl. 7:58-62 and Le Doussal et al., (1993) J. Nucl. Med. 34:1662-1671; Stickney et al., (1995) Cancer Res. 51:6650-6655).

The present molecules find therapeutic uses for treating various human diseases, for example, cancer, autoimmune diseases, and infectious diseases, etc.

For instance, with at least one antigen binding domain binding a tumor target or a pathogen target and at least a second antigen binding domain binding an antigen of an immune effector cell, e.g., a T cell or NK cell, the present molecules are capable of killing tumor cells or pathogens by using the patient's immune defense system using the approach discussed in Segal et al., Chem. Immunol. 47:179 (1989) and Segal et al., Biologic Therapy of Cancer 2(4) DeVita et al. eds. J. B. Lippincott, Philadelphia (1992) p. 1.

Similarly, the present molecules can also mediate killing by T cells, for example by linking the CD3 complex on T cells to a tumor-associated antigen.

The present molecules may also be used as fibrinolytic agents or vaccine adjuvants. Furthermore, the antibodies or antibody-like molecules may be used in the treatment of infectious diseases (e.g. for targeting of effector cells to virally infected cells such as HIV or influenza virus or protozoa such as Toxoplasma gondii), used to deliver immunotoxins to tumor cells, or target immune complexes to cell surface receptors (Romet-Lemonne, Fanger and Segal Eds., Lienhart (1991) p. 249.). The present molecules may also be used to deliver immunotoxin to tumor cells.

### B. Pharmaceutical Compositions

To prepare pharmaceutical or sterile compositions including the molecule of the present invention, the molecule is mixed with a pharmaceutically acceptable carrier or excipient.

Formulations of therapeutic and diagnostic agents can be prepared by mixing with physiologically acceptable carriers, excipients, or stabilizers in the form of, e.g., lyophilized powders, slurries, aqueous solutions, lotions, or suspensions (see, e.g., Hardman, et al. (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: eral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y.).

Selecting an administration regimen for a therapeutic depends on several factors, including the serum or tissue turnover rate of the entity, the level of symptoms, the immunogenicity of the entity, and the accessibility of the target cells in the biological matrix. In certain embodiments, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate doses of antibodies, cytokines, and small molecules are available (see, e.g., Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, N.Y.; Bach (ed.) (1993) Monoclonal Antibodies and Peptide Therapy in Autoimmune Diseases, Marcel Dekker, New York, N.Y.; Baert, et al. (2003) New Engl. J. Med. 348:601-608; Milgrom, et al. (1999) New Engl. J. Med. 341:1966-1973; Slamon, et al. (2001) New Engl. J. Med. 344:783-792; Beniaminovitz, et al. (2000) New Engl. J. Med. 342:613-619; Ghosh, et al. (2003) New Engl. J. Med. 348:24-32; Lipsky, et al. (2000) New Engl. J. Med. 343:1594-1602).

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors known in the medical arts.

Compositions comprising the molecules or fragments thereof of the present application can be provided by continuous infusion, or by doses at intervals of, e.g., one day, one week, or 1-7 times per week. Doses may be provided intravenously, subcutaneously, topically, orally, nasally, rectally, intramuscular, intracerebrally, or by inhalation. A specific dose protocol is one involving the maximal dose or dose frequency that avoids significant undesirable side effects. A total weekly dose may be at least 0.05 µg/kg body weight, at least 0.2 µg/kg, at least 0.5 µg/kg, at least 1 µg/kg, at least 10 µg/kg, at least 100 µg/kg, at least 0.2 mg/kg, at least 1.0 mg/kg, at least 2.0 mg/kg, at least 10 mg/kg, at least 25 mg/kg, or at least 50 mg/kg (see, e.g., Yang, et al. (2003) New Engl. J. Med. 349:427-434; Herold, et al. (2002) New Engl. J. Med. 346:1692-1698; Liu, et al. (1999) J. Neurol. Neurosurg. Psych. 67:451-456; Portielji, et al. (2003) Cancer Immunol. Immunother. 52:133-144). The desired dose of the molecules or fragments thereof is about the same as for an antibody or polypeptide, on a moles/kg body weight basis. The desired plasma concentration of the molecules or fragments thereof is about, on a moles/kg body weight basis. The dose may be at least 15 µg at least 20 µg, at least 25 µg, at least 30 µg, at least 35 µg, at least 40 µg, at least 45 µg, at least 50 µg, at least 55 µg, at least 60 µg, at least 65 µg, at least 70 µg, at least 75 µg, at least 80 µg, at least 85 µg, at least 90 µg, at least 95 µg, or at least 100 µg. The doses administered to a subject may number at least 1, 2, 3, 4, 5, 6, 7, 8, 9, 10, 11, or 12, or more.

For the molecules or fragments thereof of the invention, the dosage administered to a patient may be 0.0001 mg/kg to 100 mg/kg of the patient's body weight. The dosage may be between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight.

The dosage of the molecules or fragments thereof of the present application may be calculated using the patient's weight in kilograms (kg) multiplied by the dose to be administered in mg/kg. The dosage of the molecules or fragments thereof, of the present application may be 150 µg/kg or less, 125 µg/kg or less, 100 µg/kg or less, 95 µg/kg or less, 90 µg/kg or less, 85 µg/kg or less, 80 µg/kg or less, 75 µg/kg or less, 70 µg/kg or less, 65 µg/kg or less, 60 µg/kg or less, 55 µg/kg or less, 50 µg/kg or less, 45 µg/kg or less, 40 µg/kg or less, 35 µg/kg or less, 30 µg/kg or less, 25 µg/kg or less, 20 µg/kg or less, 15 µg/kg or less, 10 µg/kg or less, 5 µg/kg or less, 2.5 µg/kg or less, 2 µg/kg or less, 1.5 µg/kg or less, 1 µg/kg or less, 0.5 µg/kg or less, or 0.5 µg/kg or less of a patient's body weight.

Unit dose of the molecules or fragments thereof of the present application may be 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 mg, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

The dosage of the molecules or fragments thereof of the present application may achieve a serum titer of at least 0.1 µg/ml, at least 0.5 µg/ml, at least 1 µg/ml, at least 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in a subject. Alternatively, the dosage of the molecule or fragments thereof, of the present application may achieve a serum titer of at least 0.1 µg/ml, at least 0.5 µg/ml, at least 1 µg/ml, at least, 2 µg/ml, at least 5 µg/ml, at least 6 µg/ml, at least 10 µg/ml, at least 15 µg/ml, at least 20 µg /ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in the subject.

Doses of the molecules or fragments thereof of the application may be repeated and the administrations may be separated by at least 1 day, 2 days, 3 days, 5 days, 10 days, 15 days, 30 days, 45 days, 2 months, 75 days, 3 months, or at least 6 months.

An effective amount for a particular patient may vary depending on factors such as the condition being treated, the overall health of the patient, the method route and dose of administration and the severity of side effects (see, e.g., Maynard, et al. (1996) A Handbook of SOPs for Good Clinical Practice, Interpharm Press, Boca Raton, Fla.; Dent (2001) Good Laboratory and Good Clinical Practice, Urch Publ., London, UK).

The route of administration may be by, e.g., topical or cutaneous application, injection or infusion by intravenous, intraperitoneal, intracerebral, intramuscular, intraocular, intraarterial, intracerebrospinal, intralesional, or by sustained release systems or an implant (see, e.g., Sidman et al. (1983) Biopolymers 22:547-556; Langer, et al. (1981) J. Biomed. Mater. Res. 15:167-277; Langer (1982) Chem. Tech. 12:98-105; Epstein, et al. (1985) Proc. Natl. Acad. Sci. USA 82:3688-3692; Hwang, et al. (1980) Proc. Natl. Acad. Sci. USA 77:4030-4034; U.S. Pat. Nos. 6,350,466 and 6,316,024). Where necessary, the composition may also include a solubilizing agent and a local anesthetic such as lidocaine to ease pain at the site of the injection. In addition, pulmonary administration can also be employed, e.g., by use of an inhaler or nebulizer, and formulation with an aerosolizing agent. See, e.g., U.S. Pat. Nos. 6,019,968, 5,985,320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entirety.

A composition of the present invention may also be administered via one or more routes of administration using one or more of a variety of methods known in the art. As will be appreciated by the skilled artisan, the route and/or mode of administration will vary depending upon the desired results. Selected routes of administration for molecules or fragments thereof of the invention include intravenous, intramuscular, intradermal, intraperitoneal, subcutaneous, spinal or other eral routes of administration, for example by injection or infusion. eral administration may represent modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, a composition of the present application can be administered via a non-eral route, such as a topical, epidermal or mucosal route of administration, for example, intranasally, orally, vaginally, rectally, sublingually or topically. In one embodiment, the molecules or fragments thereof of the invention is administered by infusion. In another embodiment, the multispecific epitope binding protein of the invention is administered subcutaneously.

If the molecules or fragments thereof of the invention are administered in a controlled release or sustained release system, a pump may be used to achieve controlled or sustained release (see Langer, supra; Sefton, 1987, CRC Crit. Ref Biomed. Eng. 14:20; Buchwald et al., 1980, Surgery 88:507; Saudek et al., 1989, N. Engl. J. Med. 321:574). Polymeric materials can be used to achieve controlled or sustained release of the therapies of the invention (see e.g., Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In one embodiment, the polymer used in a sustained release formulation is inert, free of leachable impurities, stable on storage, sterile, and biodegradable. A controlled or sustained release system can be placed in proximity of the prophylactic or therapeutic target, thus requiring only a fraction of the systemic dose (see, e.g., Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more molecules or fragments thereof of the present application. See, e.g., U.S. Pat. No. 4,526,938, PCT publication WO 91/05548, PCT publication WO 96/20698, Ning et al., 1996, "Intratumoral Radioimmunotheraphy of a Human Colon Cancer Xenograft Using a Sustained-Release Gel," Radiotherapy & Oncology 39:179-189, Song et al., 1995, "Antibody Mediated Lung Targeting of Long-Circulating Emulsions," PDA Journal of Pharmaceutical Science & Technology 50:372-397, Cleek et al., 1997, "Biodegradable Polymeric Carriers for a bFGF Antibody for Cardiovascular Application," Pro. Int'l. Symp. Control. Rel. Bioact. Mater. 24:853-854, and Lam et al., 1997, "Microencapsulation of Recombinant Humanized Monoclonal Antibody for Local Delivery," Proc. Int'l. Symp. Control Rel. Bioact. Mater. 24:759-760, each of which is incorporated herein by reference in their entirety.

If the molecules or fragments thereof of the invention are administered topically, they can be formulated in the form of an ointment, cream, transdermal patch, lotion, gel, shampoo, spray, aerosol, solution, emulsion, or other form well-known to one of skill in the art. See, e.g., Remington's Pharmaceutical Sciences and Introduction to Pharmaceutical Dosage Forms, 19th ed., Mack Pub. Co., Easton, Pa. (1995). For non-sprayable topical dosage forms, viscous to semi-solid or solid forms comprising a carrier or one or more excipients compatible with topical application and having a dynamic viscosity, in some instances, greater than water are typically employed. Suitable formulations include, without limitation, solutions, suspensions, emulsions, creams, ointments, powders, liniments, salves, and the like, which are, if desired, sterilized or mixed with auxiliary agents (e.g., preservatives, stabilizers, wetting agents, buffers, or salts) for influencing various properties, such as, for example, osmotic pressure. Other suitable topical dosage forms include sprayable aerosol preparations wherein the active ingredient, in some instances, in combination with a solid or liquid inert carrier, is packaged in a mixture with a pressurized volatile (e.g., a gaseous propellant, such as freon) or in a squeeze bottle. Moisturizers or humectants can also be added to pharmaceutical compositions and dosage forms if desired. Examples of such additional ingredients are well-known in the art.

If the compositions comprising the molecules or fragments thereof are administered intranasally, it can be formulated in an aerosol form, spray, mist or in the form of drops. In particular, prophylactic or therapeutic agents for use according to the present invention can be conveniently delivered in the form of an aerosol spray presentation from pressurized packs or a nebuliser, with the use of a suitable propellant (e.g., dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, e.g., gelatin) for use in an inhaler or insufflator may be formulated containing a powder mix of the compound and a suitable powder base such as lactose or starch.

Methods for co-administration or treatment with a second therapeutic agent, e.g., a cytokine, steroid, chemotherapeutic agent, antibiotic, or radiation, are known in the art (see, e.g., Hardman, et al. (eds.) (2001) Goodman and Gilman's The Pharmacological Basis of Therapeutics, 10.sup.th ed., McGraw-Hill, New York, N.Y.; Poole and Peterson (eds.) (2001) Pharmacotherapeutics for Advanced Practice:A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). An effective amount of therapeutic may decrease the symptoms by at least 10%; by at least 20%; at least about 30%; at least 40%, or at least 50%.

Additional therapies (e.g., prophylactic or therapeutic agents), which can be administered in combination with the molecules or fragments thereof of the present application may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the molecules or fragments thereof of the invention. The two or more therapies may be administered within one same patient visit.

The molecules or fragments thereof of the invention and the other therapies may be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time, optionally, followed by the administration of a third therapy (e.g., prophylactic or therapeutic agent) for a period of time and so forth, and repeating this sequential administration, i.e., the cycle in order to reduce the development of resistance to one of the therapies, to avoid or reduce the side effects of one of the therapies, and/or to improve the efficacy of the therapies.

In certain embodiments, the molecules or fragments thereof of the invention can be formulated to ensure proper distribution in vivo. For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties which are selectively transported into specific cells or organs, thus enhance targeted drug delivery (see, e.g., V. V. Ranade (1989) J. Clin. Pharmacol. 29:685). Exemplary targeting moieties include folate or biotin (see, e.g., U.S. Pat. No. 5,416,016 to Low et al); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134); p 120 (Schreier et al (1994) J. Biol. Chem. 269:9090); see also K. Keinanen; M. L. Laukkanen (1994) FEBS Lett. 346:123; J. J. Killion; I. J. Fidler (1994) Immunomethods 4:273.

The present application provides protocols for the administration of pharmaceutical composition comprising molecules or fragments thereof of the present application alone or in combination with other therapies to a subject in need thereof. The therapies (e.g., prophylactic or therapeutic agents) of the combination therapies of the present invention can be administered concomitantly or sequentially to a subject. The therapy (e.g., prophylactic or therapeutic agents) of the combination therapies of the present invention can also be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time and repeating this sequential administration, i.e., the cycle, in order to reduce the development of resistance to one of the therapies (e.g., agents) to avoid or reduce the side effects of one of the therapies (e.g., agents), and/or to improve, the efficacy of the therapies.

The therapies (e.g., prophylactic or therapeutic agents) of the combination therapies of the invention can be administered to a subject concurrently. The term "concurrently" is not limited to the administration of therapies (e.g., prophylactic or therapeutic agents) at exactly the same time, but rather it is meant that a pharmaceutical composition comprising molecules or fragments thereof of the present application are administered to a subject in a sequence and within a time interval such that the molecules of the invention can act together with the other therapy(ies) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (e.g., prophylactic or therapeutic agents) are administered to a subject less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In other embodiments, two or more therapies (e.g., prophylactic or therapeutic agents) are administered to a within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

Where a series of doses are administered, these may, for example, be administered approximately every week, approximately every 2 weeks, approximately every 3 weeks, or approximately every 4 weeks, but preferably approximately every 3 weeks. The doses may, for example, continue to be administered until disease progression, adverse event, or other time as determined by the physician. For example, from about two, three, or four, up to about 17 or more fixed doses may be administered.

Aside from the multispecific molecule and antimetabolite chemotherapeutic agent, other therapeutic regimens may be combined therewith. For example, a second (third, fourth, etc) chemotherapeutic agent(s) may be administered, wherein the second chemotherapeutic agent is either another, different antimetabolite chemotherapeutic agent, or a chemotherapeutic agent that is not an antimetabolite. For example, the second chemotherapeutic agent may be a taxane (such as paclitaxel or docetaxel), capecitabine, or platinum-based chemotherapeutic agent (such as carboplatin, cisplatin, or oxaliplatin), anthracycline (such as doxorubicin, including, liposomal doxorubicin), topotecan, pemetrexed, vinca alkaloid (such as vinorelbine), and TLK 286. "Cocktails" of different chemotherapeutic agents may be administered.

In addition to the above therapeutic regimes, the patient may be subjected to surgical removal of cancer cells and/or radiation therapy.

### C. Therapeutic Application to CLL-1 Associated Diseases and/or Disorders

The present invention provides, among other things, compositions and methods for treating cancer. In one aspect, the cancer is a hematologic cancer including but is not limited to leukemia (such as acute myelogenous leukemia (AML), chronic myelogenous leukemia (CML), acute lymphoid leukemia, chronic lymphoid leukemia, acute lymphoblastic B-cell leukemia (B-cell acute lymphoid leukemia, BALL), acute lymphoblastic T-cell leukemia (T-cell acute lymphoid leukemia (TALL), B-cell prolymphocytic leukemia, plasma cell myeloma, and myelodysplastic syndrome) and malignant lymphoproliferative conditions, including lymphoma (such as multiple myeloma, non-Hodgkin's lymphoma, Burkitt's lymphoma, and small cell- and large cell-follicular lymphoma).

In one aspect, the invention provides methods for treating a disease associated with CLL-1 expression. In one aspect, the invention provides methods for treating a disease wherein part of the tumor is negative for CLL-1 and part of the tumor is positive for CLL-1. For example, the multispecific molecule, e.g., bispecific molecule, e.g., bispecific antibody or bispecific antibody-like molecule, of the invention is useful for treating subjects that have undergone treatment for a disease associated with elevated expression of CLL-1, wherein the subject that has undergone treatment for elevated levels of CLL-1 exhibits a disease associated with elevated levels of CLL-1. In embodiments, the multispecific molecule, e.g., bispecific molecule, e.g., bispecific antibody or bispecific antibody-like molecule, of the invention is useful for treating subjects that have undergone treatment for a disease associated with expression of CLL-1, wherein the subject that has undergone treatment related to expression of CLL-1 exhibits a disease associated with expression of CLL-1.

In one embodiment, the invention provides methods for treating a disease wherein CLL-1 is expressed on both normal cells and cancers cells, but is expressed at lower levels on normal cells. In one embodiment, the method further comprises selecting a multispecific molecule, e.g., bispecific molecule, e.g., bispecific antibody or bispecific antibody-like molecule, of the invention that binds with an affinity that allows the CLL-1 multispecific molecule, e.g., bispecific molecule, e.g., bispecific antibody or bispecific antibody-like molecule, to bind and mediate the killing of the cancer cells expressing CLL-1 but less than 30%, 25%, 20%, 15%, 10%, 5% or less of the normal cells expressing CLL-1 are killed, e.g., as determined by an assay described herein. For example, a killing assay such as flow cytometry based on Cr51 CTL can be used. In one embodiment, the CLL-1 multispecific molecule, e.g., bispecific molecule, e.g., bispecific antibody or bispecific antibody-like molecule, has an antigen binding domain that has a binding affinity KD of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the CLL-1 antigen binding domain has a binding affinity that is at least five-fold, 10-fold, 20-fold, 30-fold, 50-fold, 100-fold or 1,000-fold less than a reference antibody, e.g., an antibody described herein.

In one aspect, the invention pertains to a vector comprising nucleic acid encoding the one or more polypeptide chains of a CLL-1 multispecific molecule, e.g., bispecific molecule, e.g., bispecific antibody or bispecific antibody-like molecule, operably linked to promoter for expression in mammalian cells, e.g., T cells or NK cells. In one aspect, the invention provides a recombinant immune effector cell, e.g., T cell or NK cell, expressing the CLL-1 multispecific molecule, e.g., bispecific molecule, e.g., bispecific antibody or bispecific antibody-like molecule, for use in treating CLL-1-expressing tumors, wherein the recombinant immune effector cell (e.g., T cell or NK cell) expressing the CLL-1 multispecific molecule, e.g., bispecific molecule, e.g., bispecific antibody or bispecific antibody-like molecule, is termed a CLL-1 multispecific molecule-expressing cell (e.g., CLL-1 multispecific molecule-expressing T cell, or CLL-1 multispecific molecule-expressing NK cell). In one aspect, the CLL-1 multispecific molecule, e.g., bispecific molecule, e.g., bispecific antibody or bispecific antibody-like molecule,-expressing cell of the invention is capable of contacting a tumor cell with at least one CLL-1 multispecific molecule, e.g., bispecific molecule, e.g., bispecific antibody or bispecific antibody-like molecule, of the invention expressed on its surface or secreted (and, in an embodiment, bound by an antigen-binding domain targeting an antigen expressed on such cell) such that the CLL-1 multispecific molecule-expressing cell (e.g., CLL-1 multispecific molecule-expressing T cell, or CLL-1 multispecific molecule-expressing NK cell) targets the tumor cell and growth of the tumor is inhibited.

Generally, the molecules comprising a CLL-1 binding domain described herein (e.g., the multispecific molecules described herein), and compositions comprising said molecules may be utilized in the treatment and prevention of diseases that arise in individuals who are immunocompromised. In particular, the molecules comprising a CLL-1 binding domain described herein (e.g., the multispecific molecules described herein), and compositions comprising said molecules of the invention are used in the treatment of diseases, disorders and conditions associated with expression of CLL-1. In certain aspects, the molecules and compositions of the invention are used in the treatment of patients at risk for developing diseases, disorders and conditions associated with expression of CLL-1. Thus, the present invention provides methods for the treatment or prevention of diseases, disorders and conditions associated with expression of CLL-1 comprising administering to a subject in need thereof, a therapeutically effective amount of the molecules comprising a CLL-1 binding domain described herein (e.g., the multispecific molecules described herein), or compositions comprising said molecules of the invention.

In one aspect the molecules comprising a CLL-1 binding domain described herein (e.g., the multispecific molecules described herein), and compositions comprising said molecules of the invention may be used to treat a proliferative disease such as a cancer or malignancy or is a precancerous condition such as a myelodysplasia, a myelodysplastic syndrome or a preleukemia. In one aspect, a cancer associated with expression of CLL-1 is a hematological cancer, preleukemia, hyperproliferative disorder, hyperplasia or a dysplasia, which is characterized by abnormal growth of cells.

In one aspect, the molecules comprising a CLL-1 binding domain described herein (e.g., the multispecific molecules described herein), and compositions comprising said molecules of the invention are used to treat a cancer, wherein the cancer is a hematological cancer. Hematological cancer conditions are the types of cancer such as leukemia and malignant lymphoproliferative conditions that affect blood, bone marrow and the lymphatic system. In one aspect, the hematological cancer by be, for example, leukemia (such as acute myelogenous leukemia, chronic myelogenous leukemia, acute lymphoid leukemia, chronic lymphoid leukemia and myelodysplastic syndrome) and malignant lymphoproliferative conditions, including lymphoma (such as multiple myeloma, non-Hodgkin's lymphoma, Burkitt's lymphoma, and small cell- and large cell-follicular lymphoma). In other embodiments, a hematologic cancer can include minimal residual disease, MRD, e.g., of a leukemia, e.g., of AML or MDS.

In one aspect, the molecules comprising a CLL-1 binding domain described herein (e.g., the multispecific molecules described herein), and compositions comprising said molecules of the present invention are particularly useful for treating myeloid leukemias, AML and its subtypes, chronic myeloid leukemia (CML), and myelodysplastic syndrome (MDS).

Leukemia can be classified as acute leukemia and chronic leukemia. Acute leukemia can be further classified as acute myelogenous leukemia (AML) and acute lymphoid leukemia (ALL). Chronic leukemia includes chronic myelogenous leukemia (CML) and chronic lymphoid leukemia (CLL). Other related conditions include myelodysplastic syndromes (MDS, formerly known as "preleukemia") which are a diverse collection of hematological conditions united by ineffective production (or dysplasia) of myeloid blood cells and risk of transformation to AML.

Lymphoma is a group of blood cell tumors that develop from lymphocytes. Exemplary lymphomas include non-Hodgkin lymphoma and Hodgkin lymphoma.

In AML, malignant transformation and uncontrolled proliferation of an abnormally differentiated, long-lived myeloid progenitor cell results in high circulating numbers of immature blood forms and replacement of normal marrow by malignant cells. Symptoms include fatigue, pallor, easy bruising and bleeding, fever, and infection; symptoms of leukemic infiltration are present in only about 5% of patients (often as skin manifestations). Examination of peripheral blood smear and bone marrow is diagnostic. Existing treatment includes induction chemotherapy to achieve remission and post-remission chemotherapy (with or without stem cell transplantation) to avoid relapse.

AML has a number of subtypes that are distinguished from each other by morphology, immunophenotype, and cytochemistry. Five classes are described, based on predominant cell type, including myeloid, myeloid-monocytic, monocytic, erythroid, and megakaryocytic.

Remission induction rates range from 50 to 85%. Long-term disease-free survival reportedly occurs in 20 to 40% of patients and increases to 40 to 50% in younger patients treated with stem cell transplantation.

Prognostic factors help determine treatment protocol and intensity; patients with strongly negative prognostic features are usually given more intense forms of therapy, because the potential benefits are thought to justify the increased treatment toxicity. The most important prognostic factor is the leukemia cell karyotype; favorable karyotypes include t(15;17), t(8;21), and inv16 (p13;q22). Negative factors include increasing age, a preceding myelodysplastic phase, secondary leukemia, high WBC count, and absence of Auer rods.

Initial therapy attempts to induce remission and differs most from ALL in that AML responds to fewer drugs. The basic induction regimen includes cytarabine by continuous IV infusion or high doses for 5 to 7 days; daunorubicin or idarubicin is given IV for 3 days during this time. Some regimens include 6-thioguanine, etoposide, vincristine, and prednisone, but their contribution is unclear. Treatment usually results in significant myelosuppression, with infection or bleeding; there is significant latency before marrow recovery. During this time, meticulous preventive and supportive care is vital.

Chronic myelogenous (or myeloid) leukemia (CML) is also known as chronic granulocytic leukemia, and is characterized as a cancer of the white blood cells. Common treatment regimens for CML include Bcr-Abl tyrosine kinase inhibitors, imatinib (Gleevec®), dasatinib and nilotinib. Bcr-Abl tyrosine kinase inhibitors are specifically useful for CML patients with the Philadelphia chromosome translocation.

Myelodysplastic syndromes (MDS) are hematological medical conditions characterized by disorderly and ineffective hematopoiesis, or blood production. Thus, the number and quality of blood-forming cells decline irreversibly. Some patients with MDS can develop severeanemia, while others are asymptomatic. The classification scheme for MDS is known in the art, with criteria designating the ratio or frequency of particular blood cell types, e.g., myeloblasts, monocytes, and red cell precursors. MDS includes refractory anemia, refractory anemia with ring sideroblasts, refractory anemia with excess blasts, refractory anemia with excess blasts in transformation, chronic myelomonocytic leukemia (CML).

Treatments for MDS vary with the severity of the symptoms. Aggressive forms of treatment for patients experiencing severe symptoms include bone marrow transplants and supportive care with blood product support (e.g., blood transfusions) and hematopoietic growth factors (e.g., erythropoietin). Other agents are frequently used to treat MDS: 5-azacytidine, decitabine, and lenalidomide. In some cases, iron chelators deferoxamine (Desferal®) and deferasirox (Exjade®) may also be administered.

In another embodiment, the molecules comprising a CLL-1 binding domain described herein (e.g., the multispecific molecules described herein), and compositions comprising said molecules of the present invention are used to treat cancers or leukemias with leukemia stem cells. For example, the leukemia stem cells are CD34⁺/CD38⁻ leukemia cells.

The present invention provides, among other things, compositions and methods for treating cancer. In one aspect, the cancer is a hematologic cancer including but is not limited to leukemia (such as acute myelogenous leukemia, chronic myelogenous leukemia, acute lymphoid leukemia, chronic lymphoid leukemia and myelodysplastic syndrome) and malignant lymphoproliferative conditions, including lymphoma (such as multiple myeloma, non-Hodgkin's lymphoma, Burkitt's lymphoma, and small cell- and large cell-follicular lymphoma).

In one aspect, the molecules comprising a CLL-1 binding domain described herein (e.g., the multispecific molecules described herein), and compositions comprising said molecules of the invention may be used to treat other cancers and malignancies such as, but not limited to, e.g., acute leukemias including but not limited to, e.g., B-cell acute lymphoid leukemia ("BALL"), T-cell acute lymphoid leukemia ("TALL"), acute lymphoid leukemia (ALL); one or more chronic leukemias including but not limited to, e.g., chronic myelogenous leukemia (CML), chronic lymphocytic leukemia (CLL); additional hematologic cancers or hematologic conditions including, but not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. The molecules comprising a CLL-1 binding domain described herein (e.g., the multispecific molecules described herein), and compositions comprising said molecules of the present invention may be administered either alone, or as a pharmaceutical composition in combination with diluents and/or with other components such as IL-2 or other cytokines, other molecules, or cell populations.

The present invention also provides methods for inhibiting the proliferation or reducing a CLL-1-expressing cell population, the methods comprising contacting a population of cells comprising a CLL-1-expressing cell with a molecule comprising a CLL-1 binding domain described herein (e.g., the multispecific molecules described herein), or composition comprising said molecule, of the invention that binds to the CLL-1-expressing cell. In a specific aspect, the present invention provides methods for inhibiting the proliferation or reducing the population of cancer cells expressing CLL-1, the methods comprising contacting the CLL-1-expressing cancer cell population with a molecule comprising a CLL-1 binding domain described herein (e.g., the multispecific molecules described herein), or composition comprising said molecule, of the invention that binds to the CLL-1-expressing cell. In one aspect, the present invention provides methods for inhibiting the proliferation or reducing the population of cancer cells expressing CLL-1, the methods comprising contacting the CLL-1-expressing cancer cell population with a molecule comprising a CLL-1 binding domain described herein (e.g., the multispecific molecules described herein), or composition comprising said molecule, of the invention that binds to the CLL-1-expressing cell. In certain aspects, the molecule comprising a CLL-1 binding domain described herein (e.g., the multispecific molecules described herein), or composition comprising said molecule, of the invention reduces the quantity, number, amount or percentage of cells and/or cancer cells by at least 25%, at least 30%, at least 40%, at least 50%, at least 65%, at least 75%, at least 85%, at least 95%, or at least 99% in a subject with or animal model for myeloid leukemia or another cancer associated with CLL-1-expressing cells relative to a negative control. In one aspect, the subject is a human.

The present invention also provides methods for preventing, treating and/or managing a disease associated with CLL-1-expressing cells (e.g., a hematologic cancer or atypical cancer expessing CLL-1), the methods comprising administering to a subject in need a molecule comprising a CLL-1 binding domain described herein (e.g., the multispecific molecules described herein), or composition comprising said molecule, of the invention that binds to the CLL-1-expressing cell. In one aspect, the subject is a human. Non-limiting examples of disorders associated with CLL-1-expressing cells include autoimmune disorders (such as lupus), inflammatory disorders (such as allergies and asthma) and cancers (such as hematological cancers or atypical cancers expressing CLL-1).

The present invention also provides methods for preventing, treating and/or managing a disease associated with CLL-1-expressing cells, the methods comprising administering to a subject in need a molecule comprising a CLL-1 binding domain described herein (e.g., the multispecific molecules described herein), or composition comprising said molecule, of the invention that binds to the CLL-1-expressing cell. In one aspect, the subject is a human.

The present invention provides methods for preventing relapse of cancer associated with CLL-1-expressing cells, the methods comprising administering to a subject in need thereof a molecule comprising a CLL-1 binding domain described herein (e.g., the multispecific molecules described herein), or composition comprising said molecule, of the invention that binds to the CLL-1-expressing cell. In one aspect, the methods comprise administering to the subject in need thereof an effective amount of a molecule comprising a CLL-1 binding domain described herein (e.g., the multispecific molecules described herein), or composition comprising said molecule described herein, that binds to the CLL-1-expressing cell in combination with an effective amount of another therapy.

A multispecific molecule, e.g., a bispecific molecule, e.g., a bispecific antibody or antibody-like molecule, comprising a CLL-1 binding domain, e.g., as described herein, may be used in combination with other known agents and therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

A multispecific molecule, e.g., a bispecific molecule, e.g., a bispecific antibody or antibody-like molecule, comprising a CLL-1 binding domain, e.g., as described herein, and the at least one additional therapeutic agent can be administered simultaneously, in the same or in separate compositions, or sequentially. For sequential administration, the multispecific molecule, e.g., a bispecific molecule, e.g., a bispecific antibody or antibody-like molecule, comprising a CLL-1 binding domain, e.g., as described herein, can be administered first, and the additional agent can be administered second, or the order of administration can be reversed.

The multispecific molecule, e.g., a bispecific molecule, e.g., a bispecific antibody or antibody-like molecule, comprising a CLL-1 binding domain, e.g., as described herein, and/or other therapeutic agents, procedures or modalities can be administered during periods of active disorder, or during a period of remission or less active disease. The multispecific molecule, e.g., a bispecific molecule, e.g., a bispecific antibody or antibody-like molecule, comprising a CLL-1 binding domain, e.g., as described herein, can be administered before the other treatment, concurrently with the treatment, post-treatment, or during remission of the disorder.

When administered in combination, the multispecific molecule, e.g., a bispecific molecule, e.g., a bispecific antibody or antibody-like molecule, comprising a CLL-1 binding domain, e.g., as described herein, and the additional agent (e.g., second or third agent), or all, can be administered in an amount or dose that is higher, lower or the same than the amount or dosage of each agent used individually, e.g., as a monotherapy. In certain embodiments, the administered amount or dosage of the multispecific molecule, e.g., a bispecific molecule, e.g., a bispecific antibody or antibody-like molecule, comprising a CLL-1 binding domain, e.g., as described herein, the additional agent (e.g., second or third agent), or all, is lower (e.g., at least 20%, at least 30%, at least 40%, or at least 50%) than the amount or dosage of each agent used individually, e.g., as a monotherapy. In other embodiments, the amount or dosage of the multispecific molecule, e.g., a bispecific molecule, e.g., a bispecific antibody or antibody-like molecule, comprising a CLL-1 binding domain, e.g., as described herein, the additional agent (e.g., second or third agent), or all, that results in a desired effect (e.g., treatment of cancer) is lower (e.g., at least 20%, at least 30%, at least 40%, or at least 50% lower) than the amount or dosage of each agent used individually, e.g., as a monotherapy, required to achieve the same therapeutic effect.

In further aspects, a multispecific molecule, e.g., a bispecific molecule, e.g., a bispecific antibody or antibody-like molecule, comprising a CLL-1 binding domain, e.g., as described herein, may be used in a treatment regimen in combination with surgery, chemotherapy, radiation, immunosuppressive agents, such as cyclosporin, azathioprine, methotrexate, mycophenolate, and FK506, cellular therapies (e.g., cellular immunotherapies, e.g., chimeric antigen receptor T cell therapy), antibodies, or other immunoablative agents such as CAMPATH, anti-CD3 antibodies or other antibody therapies, cytoxin, fludarabine, cyclosporin, FK506, rapamycin, mycophenolic acid, steroids, FR901228, cytokines, and irradiation. peptide vaccine, such as that described in Izumoto et al. 2008 J Neurosurg 108:963-971.

In certain instances, compounds of the present invention are combined with other therapeutic agents, such as other anti-cancer agents, anti-allergic agents, anti-nausea agents (or anti-emetics), pain relievers, cytoprotective agents, and combinations thereof.

### EXAMPLES

The following examples are provided to further illustrate the invention but not to limit its scope. Other variants of the invention will be readily apparent to one of ordinary skill in the art and are encompassed by the appended claims.

### Example 1 - Generation and binding of human anti-CLL-1 binding domains

Phage display libraries were panned against immobilized CLL-1. Bound phage were isolated and sequenced, and binding to target confirmed by ELISA and/or FACs. 13 scFvs were confirmed to bind to human CLL-1, and were designated CLL-1-1, CLL-1-2, CLL-1-3, CLL-1-4, CLL-1-5, CLL-1-6, CLL-1-7, CLL-1-8, CLL-1-9, CLL-1-10, CLL-1-11, CLL-1-12, and CLL-1-13.

After selection and confirmation, to assess the binding and biophysical characteristics antihuman CLL-1 scFvs identified by phage panning, scFv constructs were transiently produced and purified from HEK293F cells. Transient expression and purification in HEK293F cells was performed with standard methodology. Briefly, 100ml of HEK293F cells at 3x106cells/ml were transfected with 100 µg plasmid and 300 µg polyethylenimine. The cells were incubated at 37°C with 8% CO2 and rotated at 80 rpm. After six days, the cells were harvested by centrifugation at 3500g for 20 minutes. The supernatant was purified by binding the scFv to 200 µl Ni-NTA agarose beads (Qiagen) overnight at 4°C. The protein was eluted with 200 µl 300mM imidazole, and dialyzed against phosphate buffered saline.

Next, the affinity for human CLL-1 of a representative number of anti-CLL-1 scFvs was tested by Biacore. Briefly, anti-Fc antibody (Jackson ImmunoReasearch, catalog # 109-005-098) was immobilized to a CM5 sensor chip via amine coupling, and human CLL-1 Fc was then captured at a density of 120 RU. ScFv samples were serial diluted 3-fold and injected over the chip at a constant flow rate. Association and dissociation rates of the protein complex were monitored for 270 s and 400 s, respectively. Double referencing was performed against an anti-huFc coated flow cell and a buffer blank and the data was fit using a 1:1 Langmuir or steady state affinity model with the Biacore T200 evaluation software. The results are reported in Table 9. Binding of clones CLL-1-6, CLL-1-8, CLL-1-9, CLL-1-10, and CLL1-13 to human CLL-1 was confirmed. As well, CLL-1-1 was found to bind weakly under these experimental conditions, and CLL-1-11 and CLL-1-12 did not exhibit binding under these assay conditions.

**Table 9. Binding kinetics and affinity of anti-CLL-1 scFvs to human CLL-1.**

| **Sample** | **Fit** | **ka (1/Ms)** | **kd (1/s)** | **KD (nM)** |
|---|---|---|---|---|
| CLL-1-1 | Limited Binding | | | |
| CLL-1-6 | 1:1 Binding | 3.78E+04 | 1.02E-03 | 26.9 |
| CLL-1-8 | 1:1 Binding | 2.16E+05 | 9.27E-04 | 4.3 |
| CLL-1-9 | 1:1 Binding | 1.41E+05 | 1.28E-03 | 9.1 |
| CLL-1-10 | 1:1 Binding | 1.42E+05 | 2.14E-03 | 15 |
| CLL-1-11 | No Binding | | | |
| CLL-1-12 | No Binding | | | |
| CLL-1-13 | 1:1 Binding | 2.57E+04 | 1.19E-03 | 46.1 |

These data confirm the binding of anti-CLL-1 scFvs to human scFv and show the clones generated have affinity for human CLL-1 ranging from 4.3 nM to 46.1 nM.

### Example 2 - In vitro assessment of CD3xCLL1 bispecific antibodies against CLL-1-expressing cancer cells

### Materials and Methods

A panel of CD3 x CLL1 antibodies were used to demonstrate the utility of these CLL1 bispecifics as a cancer therapeutic. These bispecific antibodies were constructed in the scFv-Fc format, and are composed of an anti-CD3 scFv fused to hIgG1 CH2 and CH3 domains (Fc) (SEQ ID NO: 507), paired with an anti-CLL1 scFv-Fc (sequences are shown in Table 11). A non-CLL1-binding negative control bispecific antibody used in these assays is of a scFv-Fc format, containing an anti-CD3 scFv and an anti-GH scFv, which is specific for human cytomegalovirus envelope glycoprotein H. Antibody dose titrations for in vitro cell based assays represented here range from 1pM to 100nM.

PBMCs Peripheral blood mononuclear cells (PBMC) were isolated from the blood of a healthy human donor using a Ficoll-Paque PLUS (GE Healthcare #17-1440-02) density gradient. T-cells (pan) were isolated from the PBMC fraction by negative selection (Miltenyi #130-096-535, 130-041-407, 130-042-401). These isolated T-cells were activated for expansion with a 3X ratio of Human T-Activator CD3/CD28 Dynabeads (Gibco #11132D) for nine days, magnetically debeaded and stored as frozen aliquots in liquid nitrogen. Frozen aliquots were thawed, counted and used immediately in T-cell killing assays at an E:T ratio of 3:1 with target cancer cell line.

Target human cancer cell line HL60, expressing moderate levels of human CLL1, was transduced to constitutively express luciferase. Luciferase is used to measure cell viability/survival with the BrightGlo reagent (Promega E2650). Target cells were plated 30,000 cells per well in a 96 well plate (Costar 3904) together with 90,000 thawed T-cells, and a serial dilution of bispecific antibody, all in media containing RPMI/1640, 10% FBS, 2mM L-glutamine, 0.1mM Non-essential amino acids, 1mM Sodium pyruvate, 10mM HEPES, 0.055mM 2-mercaptoethanol (Gibco 22400089, 16140, 25030-081, 11140-050, 11360-070, 15630-080, 21985-023 respectively).

The assay was incubated at 37°C/5% CO2 for 20-24 hours, followed by measurements of target cell viability (BrightGlo, Promega # E2650), and interferon gamma cytokine levels in the culture supernatants (MSD # N05049A-1) as a measure of T-cell activation, following vendor supplied protocols.

A Jurkat NFAT luciferase (JNL) gene reporter assay (RGA) was also used to evaluate ability of CD3 bispecific antibodies to activate T-cells through engagement with CLL1 expressing target cell line U937. U937 cells were seeded in 96 well plates at a density of 20,000 cells per well. 100,000 JNL cells were added per well (E:T = 5:1), as well as serial dilutions of CD3 x CLL1 bispecific antibodies. The assay was incubated for 4 hours at 37°C, followed by luciferase activity measurements using the Promega OneGlo assay (Promega #E-6120) according to vendor protocol. Resulting luminescence values were plotted in GraphPad PRISM, and EC50 values determined by logistic regression analysis.

### Results

Figure 2 shows the *in vitro* ability of the CD3 x CLL-1 bispecific antibodies to meditate T cell killing of CLL1-expressing cancer cell line HL60. Figure 3 shows the ability of CD3 x CLL1 bispecific antibodies to mediate T cell activation *in vitro* via engagement with CLL1-expressing cancer cell line U937. Table 10 summarizes the results of these two experiments (NFAT = results of T-cell activation assay; Killing = results of T-cell mediated CLL1-positive cancer cell line killing assay).

**Table 10. Summary of in vitro results. CD3 x CLL-1 bispecific antibodies in the Table are listed in rank order according to potency.**

| | EC50 (nM) | |
|---|---|---|
| | **NFAT** | **Killing** |
| **Bispecific Antibody** | **U937** | **HL60** |
| CD3 x CLL1_11 | 0.08 | 0.03 |
| CD3 x CLL1_12 | 0.63 | 0.20 |
| CD3 x CLL1_10 | 0.85 | 0.27 |
| CD3 x CLL1_08 | 1.26 | 0.31 |
| CD3 x CLL1_09 | 1.74 | 0.36 |
| CD3 x CLL1_06 | 0.96 | 1.27 |
| CD3 x CLL1_13 | 2.16 | 4.24 |
| CD3 x CLL1_07 | - | 44.32 |
| CD3 x CLL1_02 | - | >300 |
| CD3 x GH (Ctrl) | - | >300 |

We have developed of a panel of CD3 x CLL1 bispecific antibodies and have shown many of them to potently redirect T cell killing of CLL1-expressing cells *in vitro.* Figure 2 demonstrates that CD3 x CLL1 bispecific antibodies can specifically and effectively engage T-cell killing of CLL1 -expressing tumor cell line HL60 at an E:T ratio of 3:1 in 22 hours. Anti-CD3 x Anti-CLL1 bispecific antibody clone #11 shows the greatest *in vitro* potency, with an EC50 value of 31pM. The other clones have potency ranging from 44 nM to 200 pM, as summarized in Table 10. Without being bound by theory, it is believed that these CD3 x CLL-1 bispecific antibodies are able to mediate T cell killing of CLL-1-expressing cells at good potency in part because of the combination of the highly specific CLL-1 binding domains coupled with a CD3 binding domain that binds CD3 at high affinity.

The foregoing written specification is considered to be sufficient to enable one skilled in the art to practice the invention. The present invention is not to be limited in scope by the constructs deposited, since the deposited embodiments are intended to illustrate only certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims. Indeed, various modifications of the invention in addition to those shown and described herein will become apparent to those skilled in the art from the foregoing description and fall within the scope of the appended claims.

It is understood that the application of the teachings of the present invention to a specific problem or situation will be within the capabilities of one having ordinary skill in the art in light of the teachings contained herein.

The disclosures of all citations in the specification are expressly incorporated herein by reference.

### EMBODIMENTS OF THE INVENTION

1. A multispecific molecule comprising a first antigen binding domain and a second antigen binding domain, wherein the first antigen binding domain is an anti-CLL-1 binding domain comprising a heavy chain complementary determining region 1 (HC CDR1), a heavy chain complementary determining region 2 (HC CDR2), and a heavy chain complementary determining region 3 (HC CDR3) of any anti-CLL-1 binding domain amino acid sequence listed in Table 2.
2. The multispecific molecule of embodiment 1, wherein said anti-CLL-1 binding domain further comprises a light chain complementary determining region 1 (LC CDR1), a light chain complementary determining region 2 (LC CDR2), and a light chain complementary determining region 3 (LC CDR3) of any anti-CLL-1 binding domain amino acid sequence listed in Table 2.
3. The multispecific molecule of embodiment 2, wherein said LC CDR1, LC CDR2, and LC CDR3 are the LC CDR sequences listed in Table 4, 6 or 8.
4. The multispecific molecule of any of embodiments 1-3, wherein said HC CDR1, HC CDR2 and HC CDR3 are the HC CDR sequences listed in Table 3, 5 or 7.
5. The multispecific molecule of any of embodiments 1-4, wherein the anti-CLL-1 binding domain comprises:
   (i) the amino acid sequence of any light chain variable region listed in Table 2;
   (ii) an amino acid sequence having at least one, two or three modifications but not more than 30, 20 or 10 modifications of the amino acid sequence of any of the light chain variable regions provided in Table 2; or
   (iii) an amino acid sequence with 95-99% identity to the amino acid sequence of any of the light chain variable regions provided in Table 2.
6. The multispecific molecule of any of embodiments 1-5, wherein the anti-CLL-1 binding domain comprises:
   (i) the amino acid sequence of any heavy chain variable region listed in Table 2;
   (ii) an amino acid sequence having at least one, two or three modifications but not more than 30, 20 or 10 modifications of the amino acid sequence of any of the heavy chain variable regions provided in Table 2; or
   (ii) an amino acid sequence with 95-99% identity to the amino acid sequence of any of the heavy chain variable regions provided in Table 2.
7. The multispecific molecule of any of embodiments 1-6, , wherein the anti-CLL-1 binding domain comprises the amino acid sequence of any light chain variable region listed in Table 2, and the amino acid sequence of any heavy chain variable region listed in Table 2.
8. The multispecific molecule of any of embodiments 1-7, wherein the anti-CLL-1 binding domain comprises:
   (i) any amino acid sequence of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77;
   (ii) an amino acid sequence having at least one, two or three modifications but not more than 30, 20 or 10 modifications to any of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77; or
   (iii) an amino acid sequence with 95-99% identity to any of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77.
9. The multispecific molecule of any of embodiments 1-8, wherein the second antigen-binding domain binds a cancer antigen other than CLL-1.
10. The multispecific molecule of embodiment 9, wherein the cancer antigen other than CLL-1 is expressed on a cell that also expresses CLL-1.
11. The multispecific molecule of any of embodiments 9-10, wherein the cancer antigen other than CLL-1 is expressed on an acute myeloid leukemia (AML) cell.
12. The multispecific molecule of any of embodiments 9-11, wherein the cancer antigen is selected from the group consisting of CD123, CD33, CD34, FLT3, and folate receptor beta.
13. The multispecific molecule of any of embodiments 1-8, wherein the second antigen-binding domain binds an immune effector cell antigen.
14. The multispecific molecule of embodiment 13, wherein the immune effector cell antigen is an antigen expressed on a NK cell.
15. The multispecific molecule of embodiment 14, wherein the antigen expressed on a NK cell is CD16 (Fc Receptor gamma III) or CD64 (Fc Receptor gamma I).
16. The multispecific molecule of embodiment 13, wherein the immune effector cell antigen is an antigen expressed on a T cell.
17. The multispecific molecule of embodiment 16, wherein the antigen expressed on a T cell is an immune costimulatory molecule.
18. The multispecific molecule of embodiment 17, wherein the immune costimulatory molecule is selected from the group consisting of an MHC class I molecule, a TNF receptor protein, an immunoglobulin-like protein, a cytokine receptor, an integrin, a signaling lymphocytic activation molecule (SLAM protein), an activating NK cell receptor, BTLA, Toll ligand receptor, OX40, CD2, CD7, CD27, CD28, CD30, CD40, CD47, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), TLR7, LTBR, LAT, GADS, SLP-76, PAG/Cbp, CD19a, and a ligand that specifically binds with CD83.
19. The multispecific molecule of embodiment 16, wherein the antigen expressed on a T cell is an immune inhibitory molecule.
20. The multispecific molecule of embodiment 19, wherein the immune inhibitory molecule is selected from the group consisting of PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta.
21. The multispecific molecule of embodiment 16, wherein the antigen expressed on a T cell is CD3.
22. The multispecific molecule of embodiment 21, wherein the anti-CD3 binding domain (i.e., the second antigen-binding domain) comprises a heavy chain complementary determining region 1 (HC CDR1), a heavy chain complementary determining region 2 (HC CDR2), and a heavy chain complementary determining region 3 (HC CDR3) of any anti-CD3 binding domain VH amino acid sequence listed in Table 26.
23. The multispecific molecule of embodiment 22, wherein said anti-CD3 binding domain further comprises a light chain complementary determining region 1 (LC CDR1), a light chain complementary determining region 2 (LC CDR2), and a light chain complementary determining region 3 (LC CDR3) of any anti-CD3 binding domain VL amino acid sequence listed in Table 26.
24. The multispecific molecule of embodiment 23, wherein said LC CDR1, LC CDR2, and LC CDR3 are the LC CDR sequences of any anti-CD3 binding domain listed in Table 27, 28 or 29.
25. The multispecific molecule of any of embodiments 22-24, wherein said HC CDR1, HC CDR2 and HC CDR3 are the HC CDR sequences of any anti-CD3 binding domain listed in Table 27, 28 or 29.
26. The multispecific molecule of any of embodiments 22-25, wherein the anti-CD3 binding domain (i.e., the second antigen-binding domain) comprises:
   (i) the amino acid sequence of any light chain variable region listed in Table 26;
   (ii) an amino acid sequence having at least one, two or three modifications but not more than 30, 20 or 10 modifications of the amino acid sequence of any of the light chain variable regions provided in Table 26; or
   (iii) an amino acid sequence with 95-99% identity to the amino acid sequence of any of the light chain variable regions provided in Table 26.
27. The multispecific molecule of any of embodiments 22-26, wherein the anti-CD3 binding domain (i.e., the second antigen-binding domain) comprises:
   (i) the amino acid sequence of any heavy chain variable region listed in table 26;
   (ii) an amino acid sequence having at least one, two or three modifications but not more than 30, 20 or 10 modifications of the amino acid sequence of any of the heavy chain variable regions provided in Table 26; or
   (ii) an amino acid sequence with 95-99% identity to the amino acid sequence of any of the heavy chain variable regions provided in Table 26.
28. The multispecific molecule of any of embodiments 22-27, wherein the anti-CD3 binding domain (i.e., the second antigen-binding domain) comprises the amino acid sequence of any light chain variable region listed in Table 26, and the amino acid sequence of any heavy chain variable region listed in Table 26.
29. The multispecific molecule of any of embodiments 22-28, wherein the anti-CD3 binding domain (i.e., the second antigen-binding domain) comprises the amino acid sequence of any light chain variable region and the amino acid sequence of any heavy chain variable region of any anti-CD3 binding domain listed in Table 26.
30. The multispecific molecule of any of embodiments 22-28, wherein the anti-CD3 binding domain (i.e., the second antigen-binding domain) comprises a heavy chain variable region amino acid sequence selected from the group consisting of SEQ ID NO: 1200, 1202, 1204, 1206, 1208, 1210, 1212, 1214, 1216, 1218, 1220, 1222, 1224, 1226, 1228, 1230, 1232, 1234, and 1236; and a light chain variable amino acid sequence selected from the group consisting of SEQ ID NO: 1201, 1203, 1205, 1207, 1209, 1211, 1213, 1215, 1217, 1219, 1221, 1223, 1225, 1227, 1229, 1231, 1233, 1235, and 1237.
31. The multispecific molecule of embodiment 21, wherein the second antigen binding domain binds CD3 and comprises a VL sequence of SEQ ID NO: 1209 and a VH sequence of SEQ ID NO: 1236; and wherein the anti-CLL-1 binding domain comprises:
   a) a VL sequence of SEQ ID NO: 88 and a VH sequence of SEQ ID NO: 75;
   b) a VL sequence of SEQ ID NO: 89 and a VH sequence of SEQ ID NO: 76;
   c) a VL sequence of SEQ ID NO: 87 and a VH sequence of SEQ ID NO: 74;
   d) a VL sequence of SEQ ID NO: 85 and a VH sequence of SEQ ID NO: 72;
   e) a VL sequence of SEQ ID NO: 86 and a VH sequence of SEQ ID NO: 73;
   f) a VL sequence of SEQ ID NO: 83 and a VH sequence of SEQ ID NO: 70;
   g) a VL sequence of SEQ ID NO: 90 and a VH sequence of SEQ ID NO: 77;
   h) a VL sequence of SEQ ID NO: 79 and a VH sequence of SEQ ID NO: 66; or
   i) a VL sequence of SEQ ID NO: 84 and a VH sequence of SEQ ID NO: 71.
32. The multispecific molecule of embodiment 21, wherein the second antigen binding domain binds CD3 and comprises SEQ ID NO: 506; and wherein the anti-CLL-1 binding domain comprises:
   a) SEQ ID NO: 49;
   b) SEQ ID NO: 50;
   c) SEQ ID NO: 48;
   d) SEQ ID NO: 46;
   e) SEQ ID NO: 47;
   f) SEQ ID NO: 44;
   g) SEQ ID NO: 51;
   h) SEQ ID NO: 40; or
   i) SEQ ID NO: 45.
33. The multispecific molecule of embodiment 21, wherein the polypeptide comprising the second antigen binding domain comprises, e.g., consists of, SEQ ID NO: 507; and wherein the polypeptide comprising the anti-CLL-1 binding domain comprises, e.g., consists of:
   a) SEQ ID NO: 512;
   b) SEQ ID NO: 517;
   c) SEQ ID NO: 522;
   d) SEQ ID NO: 527;
   e) SEQ ID NO: 532;
   f) SEQ ID NO: 537;
   g) SEQ ID NO: 542;
   h) SEQ ID NO: 547; or
   i) SEQ ID NO: 552.
34. The multispecific molecule of any of embodiments 1-33, wherein said molecule is multivalent, e.g., bivalent, with respect to the first antigen binding domain or second antigen binding domain.
35. The multispecific molecule of embodiment 34, wherein the molecule is bivalent with respect to the anti-CLL-1 binding domain.
36. The multispecific molecule of any of embodiments 1-35, wherein the multispecific molecule is a bispecific antibody.
37. The multispecific molecule of any of embodiments 1-31, wherein the multispecific molecule is a dualbody.
38. The multispecific molecule of any of embodiments 1-33, wherein the multispecific molecule is a scFv-Fc.
39. The multispecific molecule of any of embodiments 1-31, wherein the multispecific molecule is a mixed chain multispecific molecule.
40. The multispecific molecule of any of embodiments 36-39, wherein the molecule is bispecific.
41. The multispecific molecule of any of embodiments 1-32, wherein the multispecific molecule is a tandem scFv.
42. The multispecific molecule of embodiment 41, wherein the molecule is bispecific.
43. The multispecific molecule of any of embodiments 1-40, wherein the polypeptides comprising the HC CDR sequences of the first and/or second antigen binding domain further comprise a CH3 domain, and optionally a CH2 domain.
44. The multispecific molecule of embodiment 43, wherein at least one, e.g., both, of said CH3 domains comprise one or more modifications to enhance heterodimerization, e.g., as described herein.
45. The multispecific molecule of embodiment 44, wherein the one or more modifications to enhance heterodimerization comprises introduction of a knob in a first CH3 domain and a hole in a second CH3 domain, or vice versa, such that heterodimerization of the polypeptide comprising the first CH3 domain and the polypeptide comprising the second CH3 domain is favored relative to polypeptides comprising unmodified CH3 domains.
46. The multispecific molecule of embodiment 45, wherein the knob or hole is introduced to the first CH3 domain at residue 366, 405 or 407 according to the EU numbering scheme of Kabat et al. (pp. 688-696 in Sequences of proteins of immunological interest, 5th ed., Vol. 1 (1991; NIH, Bethesda, Md.)) to create either a knob or hole, and a complimentary hole or knob is introduced to the second CH3 domain at residue 407 if residue 366 is mutated in the first CH3 domain, residue 394 if residue 405 is mutated in the first CH3 domain, or residue 366 if residue 407 is mutated in the first CH3 domain, according to the EU numbering scheme of Kabat et al. (pp. 688-696 in Sequences of proteins of immunological interest, 5th ed., Vol. 1 (1991; NIH, Bethesda, Md.)).
47. The multispecific molecule of any of embodiments 44-45, wherein the first CH3 domain comprises introduction of a knob at position 366, and the second CH3 domain comprises introduction of a hole at position 366, position 368 and position 407, according to the EU numbering scheme of Kabat et al. (pp. 688-696 in Sequences of proteins of immunological interest, 5th ed., Vol. 1 (1991; NIH, Bethesda, Md.)), or vice versa.
48. The multispecific molecule of embodiment 47, wherein the first CH3 domain comprises a tyrosine or tryptophan at position 366, and the second CH3 domain comprises a serine at position 366, alanine at position 368 and valine at position 407, according to the EU numbering scheme of Kabat et al. (pp. 688-696 in Sequences of proteins of immunological interest, 5th ed., Vol. 1 (1991; NIH, Bethesda, Md.)), or vice versa.
49. The multispecific molecule of embodiments 44, wherein the one or more modifications comprise IgG heterodimerization modifications.
50. The multispecific molecule of embodiment 49, wherein the first CH3 domain comprises the mutation K409R and the second CH3 domain comprises the mutation F405L, or vice versa.
51. The multispecific molecule of embodiment 44, wherein the one or more modifications comprise polar bridge modifications.
52. The multispecific molecule of embodiment 51, wherein said one or more modifications to the first CH3 domain are selected from a group consisting of: S364L, T366V, L368Q, D399K, F405S, K409F, T411K, and combinations thereof.
53. The multispecific molecule of embodiment 52, comprising one more modifications to the second CH3 domain, wherein said one or more modifications are selected from the group consisting of Y407F, K409Q and T411D, and combinations thereof.
54. The multispecific molecule of any of embodiments 43-53, wherein the first CH3 domain comprises a cysteine capable for forming a disulfide bond with a cysteine of the second CH3 domain.
55. The multispecific molecule of embodiment 54, wherein the first CH3 domain comprises a cysteine at position 354 and the second CH3 domain comprises a cysteine at position 349, or vice versa.
56. The multispecific molecule of any of embodiments 1-40 or 43-55, wherein the multispecific molecule comprises an Fc domain, and wherein the Fc domain comprises one or more mutations, e.g., one mutation, that reduces, e.g., silences, an effector function, e.g., an ADCC and/or CDC function.
57. The multispecific molecule of embodiment 56, wherein the one or more mutations comprise a LALA mutation, a DAPA mutation, or combination thereof.
58. An isolated nucleic acid, e.g., one or more polynucleotides, encoding the multispecific molecule of any of embodiments 1-57.
59. The isolated nucleic acid of embodiment 58, disposed on a single continuous polynucleotide.
60. The isolated nucleic acid of embodiment 58, disposed on two or more continuous polynucleotides.
61. The isolated nucleic acid of any of embodiments 58-60, comprising SEQ ID NO: 508 and SEQ ID NO: 509.
62. The isolated nucleic acid of embodiment 61, comprising SEQ ID NO: 510.
63. The isolated nucleic acid of embodiment 61 or 62, comprising SEQ ID NO: 511.
64. The isolated nucleic acid of any of embodiments 58-63, further comprising:
   a) SEQ ID NO: 513 and SEQ ID NO: 514;
   b) SEQ ID NO: 518 and SEQ ID NO: 519;
   c) SEQ ID NO: 523 and SEQ ID NO: 524;
   d) SEQ ID NO: 528 and SEQ ID NO: 529;
   e) SEQ ID NO: 533 and SEQ ID NO: 534;
   f) SEQ ID NO: 538 and SEQ ID NO: 539;
   g) SEQ ID NO: 543 and SEQ ID NO: 544;
   h) SEQ ID NO: 548 and SEQ ID NO: 549; or
   i) SEQ ID NO: 553 and SEQ ID NO: 554.
65. The isolated nucleic acid of embodiment 58-64, comprising:
   a) SEQ ID NO: 515;
   b) SEQ ID NO: 520;
   c) SEQ ID NO: 525;
   d) SEQ ID NO: 530;
   e) SEQ ID NO: 535;
   f) SEQ ID NO: 540;
   g) SEQ ID NO: 545;
   h) SEQ ID NO: 550; or
   i) SEQ ID NO: 555.
66. The isolated nucleic acid of embodiment 58-65, comprising:
   a) SEQ ID NO: 516;
   b) SEQ ID NO: 521;
   c) SEQ ID NO: 526;
   d) SEQ ID NO: 531;
   e) SEQ ID NO: 536;
   f) SEQ ID NO: 541;
   g) SEQ ID NO: 546;
   h) SEQ ID NO: 551; or
   i) SEQ ID NO: 556.
67. A vector e.g., one or more vectors, comprising the isolated nucleic acid of any of embodiments 58-66.
68. A cell comprising the vector of embodiment 67.
69. A method of treating a mammal having a disease associated with expression of CLL-1 comprising administering to the mammal an effective amount of a multispecific molecule of any of embodiments 1-57, the isolated nucleic acid of any of embodiments 58-66, the vector of embodiment 67 or the cell of embodiment 68.
70. The method of embodiment 69, wherein the disease associated with CLL-1 expression is:
   (i) a cancer or malignancy, or a precancerous condition chosen from one or more of a myelodysplasia, a myelodysplastic syndrome or a preleukemia, or
   (ii) a non-cancer related indication associated with expression of CLL-1.
71. The method of embodiment 69 or 70, wherein the disease is a hematologic cancer.
72. The method of any of embodiments 69-71, wherein the disease is an acute leukemia chosen from one or more of acute myeloid leukemia (AML); acute lymphoblastic B-cell leukemia (B-cell acute lymphoid leukemia, BALL), acute lymphoblastic T-cell leukemia (T-cell acute lymphoid leukemia (TALL), B-cell prolymphocytic leukemia, chronic lymphocytic leukemia, chronic myeloid leukemia (CML), myelodysplastic syndrome, plasma cell myeloma, or a combination thereof.
73. the method of embodiment 72, wherein the disease is acute myeloid leukemia (AML).
74. Use of the multispecific molecule of any of embodiments 1-57, the isolated nucleic acid of any of embodiments 58-66, the vector of embodiment 67 or the cell of embodiment 58, in the manufacture of a medicament.
75. The multispecific molecule of any of embodiments 1-57, for use as a medicament.
76. The multispecific molecule of any of embodiments 1-57, for use in a therapy.
77. The multispecific molecule of any of embodiments 1-57, for use in treating a subject having a hematologic cancer.
78. The multispecific molecule of any of embodiments 1-57, for use in treating a subject having acute myeloid leukemia (AML).
79. A composition comprising the multispecific molecule of any of embodiments 1-57.
80. A pharmaceutical composition comprising the multispecific molecule of any of embodiments 1-57, and a pharmaceutically acceptable diluent or carrier.
81. A pharmaceutical composition comprising a therapeutically effective amount of the multispecific molecule of any of embodiments 1-57.

## Claims

1. A multispecific molecule comprising a first antigen binding domain and a second antigen binding domain, wherein the first antigen binding domain is an anti-CLL-1 binding domain comprising a heavy chain complementary determining region 1 (HC CDR1), a heavy chain complementary determining region 2 (HC CDR2), and a heavy chain complementary determining region 3 (HC CDR3) of any anti-CLL-1 binding domain amino acid sequence listed in Table 2 and wherein said anti-CLL-1 binding domain further comprises a light chain complementary determining region 1 (LC CDR1), a light chain complementary determining region 2 (LC CDR2), and a light chain complementary determining region 3 (LC CDR3) of any anti-CLL-1 binding domain amino acid sequence listed in Table 2.

2. The multispecific molecule of claim 1, wherein said LC CDR1, LC CDR2, and LC CDR3 are the LC CDR sequences listed in Table 4, 6 or 8 and
wherein said HC CDR1, HC CDR2 and HC CDR3 are the HC CDR sequences listed in Table 3, 5 or 7.

3. The multispecific molecule of any of claims 1 or 2, wherein the anti-CLL-1 binding domain comprises:
(i) the amino acid sequence of any light chain variable region listed in Table 2; or
(ii) an amino acid sequence having at least one, two or three modifications but not more than 30, 20 or 10 modifications of the amino acid sequence of any of the light chain variable regions provided in Table 2; or
(iii) an amino acid sequence with 95-99% identity to the amino acid sequence of any of the light chain variable regions provided in Table 2 or
(iv) the amino acid sequence of any heavy chain variable region listed in Table 2; or
(v) an amino acid sequence having at least one, two or three modifications but not more than 30, 20 or 10 modifications of the amino acid sequence of any of the heavy chain variable regions provided in Table 2; or
(vi) an amino acid sequence with 95-99% identity to the amino acid sequence of any of the heavy chain variable regions provided in Table 2.

4. The multispecific molecule of any of claims 1-3, wherein the anti-CLL-1 binding domain comprises the amino acid sequence of any light chain variable region listed in Table 2, and the amino acid sequence of any heavy chain variable region listed in Table 2 or
wherein the anti-CLL-1 binding domain comprises:
(i) any amino acid sequence of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77; or
(ii) an amino acid sequence having at least one, two or three modifications but not more than 30, 20 or 10 modifications to any of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77; or
(iii) an amino acid sequence with 95-99% identity to any of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, or SEQ ID NO: 77.

5. The multispecific molecule of any of claims 1-4, wherein the second antigen-binding domain binds a cancer antigen other than CLL-1.

6. The multispecific molecule of claim 5, wherein the cancer antigen other than CLL-1 is expressed on a cell that also expresses CLL-1 or
wherein the cancer antigen other than CLL-1 is expressed on an acute myeloid leukemia (AML) cell or
wherein the cancer antigen is selected from the group consisting of CD123, CD33, CD34, FLT3, and folate receptor beta.

7. The multispecific molecule of any of claims 1-4, wherein the second antigen-binding domain binds an immune effector cell antigen.

8. The multispecific molecule of claim 7, wherein the immune effector cell antigen is an antigen expressed on a NK cell or
wherein the immune effector cell antigen is an antigen expressed on a NK cell and wherein the antigen expressed on a NK cell is CD16 (Fc Receptor gamma III) or CD64 (Fc Receptor gamma I) or
wherein the immune effector cell antigen is an antigen expressed on a T cell or
wherein the immune effector cell antigen is an antigen expressed on a T cell and wherein the antigen expressed on a T cell is an immune costimulatory molecule or
wherein the immune effector cell antigen is an antigen expressed on a T cell and wherein the antigen expressed on a T cell is an immune costimulatory molecule, and wherein the immune costimulatory molecule is selected from the group consisting of an MHC class I molecule, a TNF receptor protein, an immunoglobulin-like protein, a cytokine receptor, an integrin, a signaling lymphocytic activation molecule (SLAM protein), an activating NK cell receptor, BTLA, Toll ligand receptor, OX40, CD2, CD7, CD27, CD28, CD30, CD40, CD47, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), TLR7, LTBR, LAT, GADS, SLP-76, PAG/Cbp, CD19a, and a ligand that specifically binds with CD83 or
wherein the immune effector cell antigen is an antigen expressed on a T cell, wherein the antigen expressed on a T cell is an immune inhibitory molecule or
wherein the immune effector cell antigen is an antigen expressed on a T cell, wherein the antigen expressed on a T cell is an immune inhibitory molecule selected from the group consisting of PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta.

9. The multispecific molecule of claim 7, wherein the immune effector cell antigen is an antigen expressed on a T cell and wherein the antigen expressed on a T cell is CD3.

10. The multispecific molecule of claim 9, wherein the anti-CD3 binding domain comprises
a heavy chain complementary determining region 1 (HC CDR1), a heavy chain complementary determining region 2 (HC CDR2), and a heavy chain complementary determining region 3 (HC CDR3) of any anti-CD3 binding domain VH amino acid sequence listed in Table 26 and
wherein said anti-CD3 binding domain further comprises a light chain complementary determining region 1 (LC CDR1), a light chain complementary determining region 2 (LC CDR2), and a light chain complementary determining region 3 (LC CDR3) of any anti-CD3 binding domain VL amino acid sequence listed in Table 26 or
wherein the LC CDR1, LC CDR2, and LC CDR3 are the LC CDR sequences of any anti-CD3 binding domain listed in Table 27, 28 or 29 and
wherein the HC CDR1, HC CDR2 and HC CDR3 are the HC CDR sequences of any anti-CD3 binding domain listed in Table 27, 28 or 29.

11. An isolated nucleic acid, e.g., one or more polynucleotides, encoding the multispecific molecule of any of claims 1-10.

12. A vector e.g., one or more vectors, comprising the isolated nucleic acid of claim 11.

13. A cell comprising the vector of claim 12.

14. The multispecific molecule of any of claims 1 to 13, for use as a medicament or for use in a therapy or for use in treating a subject having a hematologic cancer or for use in treating a subject having acute myeloid leukemia (AML).

15. A pharmaceutical composition comprising the multispecific molecule of any of claims 1-10, and a pharmaceutically acceptable diluent or carrier.


SEQUENCE LISTING

<110> NOVARTIS AG

<120> MULTISPECIFIC MOLECULES TARGETING CLL-1

<130> PAT057213-WO-PCT02

<140> PCT/IB2017/050318
<141> 2017-01-20

<150> PCT/CN2016/071599
<151> 2016-01-21

<160> 1911

<170> PatentIn version 3.5

<210> 1
<211> 21
<212> PRT
<213> Artificial Sequence

<220>
<221> source
<223> /note="Description of Artificial Sequence: Synthetic
      peptide"

<400> 1
Met Ala Leu Pro Val Thr Ala Leu Leu Leu Pro Leu Ala Leu Leu Leu
1               5                   10                  15

His Ala Ala Arg Pro
            20

<210> 2
<211> 6
<212> PRT
<213> Artificial Sequence

<220>
<221> source
<223> /note="Description of Artificial Sequence: Synthetic
      6xHis tag"

<400> 2
His His His His His His
1               5

<210> 3

<400> 3
000

<210> 4

<400> 4
000

<210> 5

<400> 5
000


<210> 6

<400> 6
000


<210> 7

<400> 7
000


<210> 8

<400> 8
000


<210> 9

<400> 9
000


<210> 10

<400> 10
000


<210> 11

<400> 11
000


<210> 12
<211> 63
<212> DNA
<213> Artificial Sequence

<220>
<221> source
<223> /note="Description of Artificial Sequence: Synthetic
      oligonucleotide"

<400> 12
atggccctgc ctgtgacagc cctgctgctg cctctggctc tgctgctgca tgccgctaga      60

ccc                                                                    63


<210> 13

<400> 13
000

<210> 14

<400> 14
000

<210> 15

<400> 15
000

<210> 16

<400> 16
000

<210> 17

<400> 17
000

<210> 18

<400> 18
000

<210> 19

<400> 19
000

<210> 20

<400> 20
000

<210> 21

<400> 21
000

<210> 22

<400> 22
000

<210> 23

<400> 23
000

<210> 24

<400> 24
000

<210> 25
<211> 5
<212> PRT
<213> Artificial Sequence

<220>
<221> source
<223> /note="Description of Artificial Sequence: Synthetic
      peptide"

<400> 25
Gly Gly Gly Gly Ser
1               5

<210> 26
<211> 30
<212> PRT
<213> Artificial Sequence

<220>
<221> source
<223> /note="Description of Artificial Sequence: Synthetic
      polypeptide"

<220>
<221> MISC_FEATURE
<222> (1)..(30)
<223> /note="This sequence may encompass 1-6 'Gly Gly Gly Gly Ser'
      repeating units"

<220>
<221> source
<223> /note="See specification as filed for detailed description of
      substitutions and preferred embodiments"

<400> 26
Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser
            20                  25                  30

<210> 27
<211> 20
<212> PRT
<213> Artificial Sequence

<220>
<221> source
<223> /note="Description of Artificial Sequence: Synthetic
      peptide"

<400> 27
Gly Gly Gly Gly Ser Gly Gly Gly Gly Ser Gly Gly Gly Gly Ser Gly

1               5                   10                  15

Gly Gly Gly Ser
            20

<210> 28
<211> 15
<212> PRT
<213> Artificial Sequence

<220>
<221> source
<223> /note="Description of Artificial Sequence: Synthetic
      peptide"

<400> 28
Gly Gly Gly Gly Ser Gly Gly Gly Gly Ser Gly Gly Gly Gly Ser
1               5                   10                  15

<210> 29
<211> 4
<212> PRT
<213> Artificial Sequence

<220>
<221> source
<223> /note="Description of Artificial Sequence: Synthetic
      peptide"

<400> 29
Gly Gly Gly Ser
1

<210> 30
<211> 5000
<212> DNA
<213> Artificial Sequence

<220>
<221> source
<223> /note="Description of Artificial Sequence: Synthetic
      polynucleotide"

<220>
<221> misc_feature
<222> (1)..(5000)
<223> /note="This sequence may encompass 50-5000 nucleotides"

<400> 30
aaaaaaaaaa aaaaaaaaaa aaaaaaaaaa aaaaaaaaaa aaaaaaaaaa aaaaaaaaaa      60

aaaaaaaaaa aaaaaaaaaa aaaaaaaaaa aaaaaaaaaa aaaaaaaaaa aaaaaaaaaa     120

aaaaaaaaaa aaaaaaaaaa aaaaaaaaaa aaaaaaaaaa aaaaaaaaaa aaaaaaaaaa     180

aaaaaaaaaa aaaaaaaaaa aaaaaaaaaa aaaaaaaaaa aaaaaaaaaa aaaaaaaaaa     240

aaaaaaaaaa aaaaaaaaaa aaaaaaaaaa aaaaaaaaaa aaaaaaaaaa aaaaaaaaaa     300

aaaaaaaaaa aaaaaaaaaa aaaaaaaaaa aaaaaaaaaa aaaaaaaaaa aaaaaaaaaa 360

aaaaaaaaaa aaaaaaaaaa aaaaaaaaaa aaaaaaaaaa aaaaaaaaaa aaaaaaaaaa 420

aaaaaaaaaa aaaaaaaaaa aaaaaaaaaa aaaaaaaaaa aaaaaaaaaa aaaaaaaaaa 480

aaaaaaaaaa aaaaaaaaaa aaaaaaaaaa aaaaaaaaaa aaaaaaaaaa aaaaaaaaaa 540

aaaaaaaaaa aaaaaaaaaa aaaaaaaaaa aaaaaaaaaa aaaaaaaaaa aaaaaaaaaa 600

aaaaaaaaaa aaaaaaaaaa aaaaaaaaaa aaaaaaaaaa aaaaaaaaaa aaaaaaaaaa 660

aaaaaaaaaa aaaaaaaaaa aaaaaaaaaa aaaaaaaaaa aaaaaaaaaa aaaaaaaaaa 720

aaaaaaaaaa aaaaaaaaaa aaaaaaaaaa aaaaaaaaaa aaaaaaaaaa aaaaaaaaaa 780

aaaaaaaaaa aaaaaaaaaa aaaaaaaaaa aaaaaaaaaa aaaaaaaaaa aaaaaaaaaa 840

aaaaaaaaaa aaaaaaaaaa aaaaaaaaaa aaaaaaaaaa aaaaaaaaaa aaaaaaaaaa 900

aaaaaaaaaa aaaaaaaaaa aaaaaaaaaa aaaaaaaaaa aaaaaaaaaa aaaaaaaaaa 960

aaaaaaaaaa aaaaaaaaaa aaaaaaaaaa aaaaaaaaaa aaaaaaaaaa aaaaaaaaaa 1020

aaaaaaaaaa aaaaaaaaaa aaaaaaaaaa aaaaaaaaaa aaaaaaaaaa aaaaaaaaaa 1080

aaaaaaaaaa aaaaaaaaaa aaaaaaaaaa aaaaaaaaaa aaaaaaaaaa aaaaaaaaaa 1140

aaaaaaaaaa aaaaaaaaaa aaaaaaaaaa aaaaaaaaaa aaaaaaaaaa aaaaaaaaaa 1200

aaaaaaaaaa aaaaaaaaaa aaaaaaaaaa aaaaaaaaaa aaaaaaaaaa aaaaaaaaaa 1260

aaaaaaaaaa aaaaaaaaaa aaaaaaaaaa aaaaaaaaaa aaaaaaaaaa aaaaaaaaaa 1320

aaaaaaaaaa aaaaaaaaaa aaaaaaaaaa aaaaaaaaaa aaaaaaaaaa aaaaaaaaaa 1380

aaaaaaaaaa aaaaaaaaaa aaaaaaaaaa aaaaaaaaaa aaaaaaaaaa aaaaaaaaaa 1440

aaaaaaaaaa aaaaaaaaaa aaaaaaaaaa aaaaaaaaaa aaaaaaaaaa aaaaaaaaaa 1500

aaaaaaaaaa aaaaaaaaaa aaaaaaaaaa aaaaaaaaaa aaaaaaaaaa aaaaaaaaaa 1560

aaaaaaaaaa aaaaaaaaaa aaaaaaaaaa aaaaaaaaaa aaaaaaaaaa aaaaaaaaaa 1620

aaaaaaaaaa aaaaaaaaaa aaaaaaaaaa aaaaaaaaaa aaaaaaaaaa aaaaaaaaaa 1680

aaaaaaaaaa aaaaaaaaaa aaaaaaaaaa aaaaaaaaaa aaaaaaaaaa aaaaaaaaaa 1740

aaaaaaaaaa aaaaaaaaaa aaaaaaaaaa aaaaaaaaaa aaaaaaaaaa aaaaaaaaaa 1800

aaaaaaaaaa aaaaaaaaaa aaaaaaaaaa aaaaaaaaaa aaaaaaaaaa aaaaaaaaaa 1860

aaaaaaaaaa aaaaaaaaaa aaaaaaaaaa aaaaaaaaaa aaaaaaaaaa aaaaaaaaaa 1920

aaaaaaaaaa aaaaaaaaaa aaaaaaaaaa aaaaaaaaaa aaaaaaaaaa aaaaaaaaaa 1980

aaaaaaaaaa aaaaaaaaaa aaaaaaaaaa aaaaaaaaaa aaaaaaaaaa aaaaaaaaaa 2040

aaaaaaaaaa aaaaaaaaaa aaaaaaaaaa aaaaaaaaaa aaaaaaaaaa aaaaaaaaaa 2100

aaaaaaaaaa aaaaaaaaaa aaaaaaaaaa aaaaaaaaaa aaaaaaaaaa aaaaaaaaaa 2160

aaaaaaaaaa aaaaaaaaaa aaaaaaaaaa aaaaaaaaaa aaaaaaaaaa aaaaaaaaaa 2220

aaaaaaaaaa aaaaaaaaaa aaaaaaaaaa aaaaaaaaaa aaaaaaaaaa aaaaaaaaaa 2280

aaaaaaaaaa aaaaaaaaaa aaaaaaaaaa aaaaaaaaaa aaaaaaaaaa aaaaaaaaaa 2340

aaaaaaaaaa aaaaaaaaaa aaaaaaaaaa aaaaaaaaaa aaaaaaaaaa aaaaaaaaaa 2400

aaaaaaaaaa aaaaaaaaaa aaaaaaaaaa aaaaaaaaaa aaaaaaaaaa aaaaaaaaaa 2460

aaaaaaaaaa aaaaaaaaaa aaaaaaaaaa aaaaaaaaaa aaaaaaaaaa aaaaaaaaaa 2520

aaaaaaaaaa aaaaaaaaaa aaaaaaaaaa aaaaaaaaaa aaaaaaaaaa aaaaaaaaaa 2580

aaaaaaaaaa aaaaaaaaaa aaaaaaaaaa aaaaaaaaaa aaaaaaaaaa aaaaaaaaaa 2640

aaaaaaaaaa aaaaaaaaaa aaaaaaaaaa aaaaaaaaaa aaaaaaaaaa aaaaaaaaaa 2700

aaaaaaaaaa aaaaaaaaaa aaaaaaaaaa aaaaaaaaaa aaaaaaaaaa aaaaaaaaaa 2760

aaaaaaaaaa aaaaaaaaaa aaaaaaaaaa aaaaaaaaaa aaaaaaaaaa aaaaaaaaaa 2820

aaaaaaaaaa aaaaaaaaaa aaaaaaaaaa aaaaaaaaaa aaaaaaaaaa aaaaaaaaaa 2880

aaaaaaaaaa aaaaaaaaaa aaaaaaaaaa aaaaaaaaaa aaaaaaaaaa aaaaaaaaaa 2940

aaaaaaaaaa aaaaaaaaaa aaaaaaaaaa aaaaaaaaaa aaaaaaaaaa aaaaaaaaaa 3000

aaaaaaaaaa aaaaaaaaaa aaaaaaaaaa aaaaaaaaaa aaaaaaaaaa aaaaaaaaaa 3060

aaaaaaaaaa aaaaaaaaaa aaaaaaaaaa aaaaaaaaaa aaaaaaaaaa aaaaaaaaaa 3120

aaaaaaaaaa aaaaaaaaaa aaaaaaaaaa aaaaaaaaaa aaaaaaaaaa aaaaaaaaaa 3180

aaaaaaaaaa aaaaaaaaaa aaaaaaaaaa aaaaaaaaaa aaaaaaaaaa aaaaaaaaaa 3240

aaaaaaaaaa aaaaaaaaaa aaaaaaaaaa aaaaaaaaaa aaaaaaaaaa aaaaaaaaaa 3300

aaaaaaaaaa aaaaaaaaaa aaaaaaaaaa aaaaaaaaaa aaaaaaaaaa aaaaaaaaaa 3360

aaaaaaaaaa aaaaaaaaaa aaaaaaaaaa aaaaaaaaaa aaaaaaaaaa aaaaaaaaaa 3420

aaaaaaaaaa aaaaaaaaaa aaaaaaaaaa aaaaaaaaaa aaaaaaaaaa aaaaaaaaaa 3480

aaaaaaaaaa aaaaaaaaaa aaaaaaaaaa aaaaaaaaaa aaaaaaaaaa aaaaaaaaaa 3540

aaaaaaaaaa aaaaaaaaaa aaaaaaaaaa aaaaaaaaaa aaaaaaaaaa aaaaaaaaaa 3600

aaaaaaaaaa aaaaaaaaaa aaaaaaaaaa aaaaaaaaaa aaaaaaaaaa aaaaaaaaaa 3660

aaaaaaaaaa aaaaaaaaaa aaaaaaaaaa aaaaaaaaaa aaaaaaaaaa aaaaaaaaaa 3720

aaaaaaaaaa aaaaaaaaaa aaaaaaaaaa aaaaaaaaaa aaaaaaaaaa aaaaaaaaaa 3780

aaaaaaaaaa aaaaaaaaaa aaaaaaaaaa aaaaaaaaaa aaaaaaaaaa aaaaaaaaaa 3840

aaaaaaaaaa aaaaaaaaaa aaaaaaaaaa aaaaaaaaaa aaaaaaaaaa aaaaaaaaaa 3900

aaaaaaaaaa aaaaaaaaaa aaaaaaaaaa aaaaaaaaaa aaaaaaaaaa aaaaaaaaaa 3960

aaaaaaaaaa aaaaaaaaaa aaaaaaaaaa aaaaaaaaaa aaaaaaaaaa aaaaaaaaaa 4020

aaaaaaaaaa aaaaaaaaaa aaaaaaaaaa aaaaaaaaaa aaaaaaaaaa aaaaaaaaaa 4080

aaaaaaaaaa aaaaaaaaaa aaaaaaaaaa aaaaaaaaaa aaaaaaaaaa aaaaaaaaaa 4140

aaaaaaaaaa aaaaaaaaaa aaaaaaaaaa aaaaaaaaaa aaaaaaaaaa aaaaaaaaaa 4200

aaaaaaaaaa aaaaaaaaaa aaaaaaaaaa aaaaaaaaaa aaaaaaaaaa aaaaaaaaaa 4260

aaaaaaaaaa aaaaaaaaaa aaaaaaaaaa aaaaaaaaaa aaaaaaaaaa aaaaaaaaaa 4320

aaaaaaaaaa aaaaaaaaaa aaaaaaaaaa aaaaaaaaaa aaaaaaaaaa aaaaaaaaaa 4380

aaaaaaaaaa aaaaaaaaaa aaaaaaaaaa aaaaaaaaaa aaaaaaaaaa aaaaaaaaaa 4440

aaaaaaaaaa aaaaaaaaaa aaaaaaaaaa aaaaaaaaaa aaaaaaaaaa aaaaaaaaaa 4500

aaaaaaaaaa aaaaaaaaaa aaaaaaaaaa aaaaaaaaaa aaaaaaaaaa aaaaaaaaaa 4560

aaaaaaaaaa aaaaaaaaaa aaaaaaaaaa aaaaaaaaaa aaaaaaaaaa aaaaaaaaaa 4620

aaaaaaaaaa aaaaaaaaaa aaaaaaaaaa aaaaaaaaaa aaaaaaaaaa aaaaaaaaaa 4680

aaaaaaaaaa aaaaaaaaaa aaaaaaaaaa aaaaaaaaaa aaaaaaaaaa aaaaaaaaaa 4740

aaaaaaaaaa aaaaaaaaaa aaaaaaaaaa aaaaaaaaaa aaaaaaaaaa aaaaaaaaaa 4800

aaaaaaaaaa aaaaaaaaaa aaaaaaaaaa aaaaaaaaaa aaaaaaaaaa aaaaaaaaaa 4860

aaaaaaaaaa aaaaaaaaaa aaaaaaaaaa aaaaaaaaaa aaaaaaaaaa aaaaaaaaaa 4920

aaaaaaaaaa aaaaaaaaaa aaaaaaaaaa aaaaaaaaaa aaaaaaaaaa aaaaaaaaaa 4980

aaaaaaaaaa aaaaaaaaaa 5000


<210> 31
<211> 5000
<212> DNA
<213> Artificial Sequence

<220>
<221> source
<223> /note="Description of Artificial Sequence: Synthetic
      polynucleotide"


<220>
<221> misc_feature
<222> (1)..(5000)
<223> /note="This sequence may encompass 50-5000 nucleotides"

<400> 31
tttttttttt tttttttttt tttttttttt tttttttttt tttttttttt tttttttttt 60

tttttttttt tttttttttt tttttttttt tttttttttt tttttttttt tttttttttt 120

tttttttttt tttttttttt tttttttttt tttttttttt tttttttttt tttttttttt 180

tttttttttt tttttttttt tttttttttt tttttttttt tttttttttt tttttttttt 240

tttttttttt tttttttttt tttttttttt tttttttttt tttttttttt tttttttttt 300

tttttttttt tttttttttt tttttttttt tttttttttt tttttttttt tttttttttt 360

tttttttttt tttttttttt tttttttttt tttttttttt tttttttttt tttttttttt 420

tttttttttt tttttttttt tttttttttt tttttttttt tttttttttt tttttttttt 480

tttttttttt tttttttttt tttttttttt tttttttttt tttttttttt tttttttttt 540

tttttttttt tttttttttt tttttttttt tttttttttt tttttttttt tttttttttt 600

tttttttttt tttttttttt tttttttttt tttttttttt tttttttttt tttttttttt 660

tttttttttt tttttttttt tttttttttt tttttttttt tttttttttt tttttttttt 720

tttttttttt tttttttttt tttttttttt tttttttttt tttttttttt tttttttttt 780

tttttttttt tttttttttt tttttttttt tttttttttt tttttttttt tttttttttt 840

tttttttttt tttttttttt tttttttttt tttttttttt tttttttttt tttttttttt 900

tttttttttt tttttttttt tttttttttt tttttttttt tttttttttt tttttttttt 960

tttttttttt tttttttttt tttttttttt tttttttttt tttttttttt tttttttttt 1020

tttttttttt tttttttttt tttttttttt tttttttttt tttttttttt tttttttttt 1080

tttttttttt tttttttttt tttttttttt tttttttttt tttttttttt tttttttttt 1140

tttttttttt tttttttttt tttttttttt tttttttttt tttttttttt tttttttttt 1200

tttttttttt tttttttttt tttttttttt tttttttttt tttttttttt tttttttttt 1260

tttttttttt tttttttttt tttttttttt tttttttttt tttttttttt tttttttttt 1320

tttttttttt tttttttttt tttttttttt tttttttttt tttttttttt tttttttttt 1380

tttttttttt tttttttttt tttttttttt tttttttttt tttttttttt tttttttttt 1440

tttttttttt tttttttttt tttttttttt tttttttttt tttttttttt tttttttttt 1500

tttttttttt tttttttttt tttttttttt tttttttttt tttttttttt tttttttttt 1560

tttttttttt tttttttttt tttttttttt tttttttttt tttttttttt tttttttttt 1620

tttttttttt tttttttttt tttttttttt tttttttttt tttttttttt tttttttttt 1680

tttttttttt tttttttttt tttttttttt tttttttttt tttttttttt tttttttttt 1740

tttttttttt tttttttttt tttttttttt tttttttttt tttttttttt tttttttttt 1800

tttttttttt tttttttttt tttttttttt tttttttttt tttttttttt tttttttttt 1860

tttttttttt tttttttttt tttttttttt tttttttttt tttttttttt tttttttttt 1920

tttttttttt tttttttttt tttttttttt tttttttttt tttttttttt tttttttttt 1980

tttttttttt tttttttttt tttttttttt tttttttttt tttttttttt tttttttttt 2040

tttttttttt tttttttttt tttttttttt tttttttttt tttttttttt tttttttttt 2100

tttttttttt tttttttttt tttttttttt tttttttttt tttttttttt tttttttttt 2160

tttttttttt tttttttttt tttttttttt tttttttttt tttttttttt tttttttttt 2220

tttttttttt tttttttttt tttttttttt tttttttttt tttttttttt tttttttttt 2280

tttttttttt tttttttttt tttttttttt tttttttttt tttttttttt tttttttttt 2340
tttttttttt tttttttttt tttttttttt tttttttttt tttttttttt tttttttttt 2400
tttttttttt tttttttttt tttttttttt tttttttttt tttttttttt tttttttttt 2460
tttttttttt tttttttttt tttttttttt tttttttttt tttttttttt tttttttttt 2520
tttttttttt tttttttttt tttttttttt tttttttttt tttttttttt tttttttttt 2580
tttttttttt tttttttttt tttttttttt tttttttttt tttttttttt tttttttttt 2640
tttttttttt tttttttttt tttttttttt tttttttttt tttttttttt tttttttttt 2700
tttttttttt tttttttttt tttttttttt tttttttttt tttttttttt tttttttttt 2760
tttttttttt tttttttttt tttttttttt tttttttttt tttttttttt tttttttttt 2820
tttttttttt tttttttttt tttttttttt tttttttttt tttttttttt tttttttttt 2880
tttttttttt tttttttttt tttttttttt tttttttttt tttttttttt tttttttttt 2940
tttttttttt tttttttttt tttttttttt tttttttttt tttttttttt tttttttttt 3000
tttttttttt tttttttttt tttttttttt tttttttttt tttttttttt tttttttttt 3060
tttttttttt tttttttttt tttttttttt tttttttttt tttttttttt tttttttttt 3120
tttttttttt tttttttttt tttttttttt tttttttttt tttttttttt tttttttttt 3180
tttttttttt tttttttttt tttttttttt tttttttttt tttttttttt tttttttttt 3240
tttttttttt tttttttttt tttttttttt tttttttttt tttttttttt tttttttttt 3300
tttttttttt tttttttttt tttttttttt tttttttttt tttttttttt tttttttttt 3360
tttttttttt tttttttttt tttttttttt tttttttttt tttttttttt tttttttttt 3420
tttttttttt tttttttttt tttttttttt tttttttttt tttttttttt tttttttttt 3480
tttttttttt tttttttttt tttttttttt tttttttttt tttttttttt tttttttttt 3540
tttttttttt tttttttttt tttttttttt tttttttttt tttttttttt tttttttttt 3600
tttttttttt tttttttttt tttttttttt tttttttttt tttttttttt tttttttttt 3660
tttttttttt tttttttttt tttttttttt tttttttttt tttttttttt tttttttttt 3720
tttttttttt tttttttttt tttttttttt tttttttttt tttttttttt tttttttttt 3780
tttttttttt tttttttttt tttttttttt tttttttttt tttttttttt tttttttttt 3840
tttttttttt tttttttttt tttttttttt tttttttttt tttttttttt tttttttttt 3900
tttttttttt tttttttttt tttttttttt tttttttttt tttttttttt tttttttttt 3960
tttttttttt tttttttttt tttttttttt tttttttttt tttttttttt tttttttttt 4020
tttttttttt tttttttttt tttttttttt tttttttttt tttttttttt tttttttttt 4080
tttttttttt tttttttttt tttttttttt tttttttttt tttttttttt tttttttttt 4140

tttttttttt tttttttttt tttttttttt tttttttttt tttttttttt tttttttttt 4200

tttttttttt tttttttttt tttttttttt tttttttttt tttttttttt tttttttttt 4260

tttttttttt tttttttttt tttttttttt tttttttttt tttttttttt tttttttttt 4320

tttttttttt tttttttttt tttttttttt tttttttttt tttttttttt tttttttttt 4380

tttttttttt tttttttttt tttttttttt tttttttttt tttttttttt tttttttttt 4440

tttttttttt tttttttttt tttttttttt tttttttttt tttttttttt tttttttttt 4500

tttttttttt tttttttttt tttttttttt tttttttttt tttttttttt tttttttttt 4560

tttttttttt tttttttttt tttttttttt tttttttttt tttttttttt tttttttttt 4620

tttttttttt tttttttttt tttttttttt tttttttttt tttttttttt tttttttttt 4680

tttttttttt tttttttttt tttttttttt tttttttttt tttttttttt tttttttttt 4740

tttttttttt tttttttttt tttttttttt tttttttttt tttttttttt tttttttttt 4800

tttttttttt tttttttttt tttttttttt tttttttttt tttttttttt tttttttttt 4860

tttttttttt tttttttttt tttttttttt tttttttttt tttttttttt tttttttttt 4920

tttttttttt tttttttttt tttttttttt tttttttttt tttttttttt tttttttttt 4980

tttttttttt tttttttttt 5000

<210> 32

<400> 32
000

<210> 33
<211> 5000
<212> DNA
<213> Artificial Sequence

<220>
<221> source
<223> /note="Description of Artificial Sequence: Synthetic
      polynucleotide"

<220>
<221> misc_feature
<222> (1)..(5000)
<223> /note="This sequence may encompass 100-5000 nucleotides"

<400> 33
aaaaaaaaaa aaaaaaaaaa aaaaaaaaaa aaaaaaaaaa aaaaaaaaaa aaaaaaaaaa 60

aaaaaaaaaa aaaaaaaaaa aaaaaaaaaa aaaaaaaaaa aaaaaaaaaa aaaaaaaaaa 120

aaaaaaaaaa aaaaaaaaaa aaaaaaaaaa aaaaaaaaaa aaaaaaaaaa aaaaaaaaaa 180

aaaaaaaaaa aaaaaaaaaa aaaaaaaaaa aaaaaaaaaa aaaaaaaaaa aaaaaaaaaa 240

aaaaaaaaaa aaaaaaaaaa aaaaaaaaaa aaaaaaaaaa aaaaaaaaaa aaaaaaaaaa 300

aaaaaaaaaa aaaaaaaaaa aaaaaaaaaa aaaaaaaaaa aaaaaaaaaa aaaaaaaaaa 360

aaaaaaaaaa aaaaaaaaaa aaaaaaaaaa aaaaaaaaaa aaaaaaaaaa aaaaaaaaaa 420

aaaaaaaaaa aaaaaaaaaa aaaaaaaaaa aaaaaaaaaa aaaaaaaaaa aaaaaaaaaa 480

aaaaaaaaaa aaaaaaaaaa aaaaaaaaaa aaaaaaaaaa aaaaaaaaaa aaaaaaaaaa 540

aaaaaaaaaa aaaaaaaaaa aaaaaaaaaa aaaaaaaaaa aaaaaaaaaa aaaaaaaaaa 600

aaaaaaaaaa aaaaaaaaaa aaaaaaaaaa aaaaaaaaaa aaaaaaaaaa aaaaaaaaaa 660

aaaaaaaaaa aaaaaaaaaa aaaaaaaaaa aaaaaaaaaa aaaaaaaaaa aaaaaaaaaa 720

aaaaaaaaaa aaaaaaaaaa aaaaaaaaaa aaaaaaaaaa aaaaaaaaaa aaaaaaaaaa 780

aaaaaaaaaa aaaaaaaaaa aaaaaaaaaa aaaaaaaaaa aaaaaaaaaa aaaaaaaaaa 840

aaaaaaaaaa aaaaaaaaaa aaaaaaaaaa aaaaaaaaaa aaaaaaaaaa aaaaaaaaaa 900

aaaaaaaaaa aaaaaaaaaa aaaaaaaaaa aaaaaaaaaa aaaaaaaaaa aaaaaaaaaa 960

aaaaaaaaaa aaaaaaaaaa aaaaaaaaaa aaaaaaaaaa aaaaaaaaaa aaaaaaaaaa 1020

aaaaaaaaaa aaaaaaaaaa aaaaaaaaaa aaaaaaaaaa aaaaaaaaaa aaaaaaaaaa 1080

aaaaaaaaaa aaaaaaaaaa aaaaaaaaaa aaaaaaaaaa aaaaaaaaaa aaaaaaaaaa 1140

aaaaaaaaaa aaaaaaaaaa aaaaaaaaaa aaaaaaaaaa aaaaaaaaaa aaaaaaaaaa 1200

aaaaaaaaaa aaaaaaaaaa aaaaaaaaaa aaaaaaaaaa aaaaaaaaaa aaaaaaaaaa 1260

aaaaaaaaaa aaaaaaaaaa aaaaaaaaaa aaaaaaaaaa aaaaaaaaaa aaaaaaaaaa 1320

aaaaaaaaaa aaaaaaaaaa aaaaaaaaaa aaaaaaaaaa aaaaaaaaaa aaaaaaaaaa 1380

aaaaaaaaaa aaaaaaaaaa aaaaaaaaaa aaaaaaaaaa aaaaaaaaaa aaaaaaaaaa 1440

aaaaaaaaaa aaaaaaaaaa aaaaaaaaaa aaaaaaaaaa aaaaaaaaaa aaaaaaaaaa 1500

aaaaaaaaaa aaaaaaaaaa aaaaaaaaaa aaaaaaaaaa aaaaaaaaaa aaaaaaaaaa 1560

aaaaaaaaaa aaaaaaaaaa aaaaaaaaaa aaaaaaaaaa aaaaaaaaaa aaaaaaaaaa 1620

aaaaaaaaaa aaaaaaaaaa aaaaaaaaaa aaaaaaaaaa aaaaaaaaaa aaaaaaaaaa 1680

aaaaaaaaaa aaaaaaaaaa aaaaaaaaaa aaaaaaaaaa aaaaaaaaaa aaaaaaaaaa 1740

aaaaaaaaaa aaaaaaaaaa aaaaaaaaaa aaaaaaaaaa aaaaaaaaaa aaaaaaaaaa 1800

aaaaaaaaaa aaaaaaaaaa aaaaaaaaaa aaaaaaaaaa aaaaaaaaaa aaaaaaaaaa 1860

aaaaaaaaaa aaaaaaaaaa aaaaaaaaaa aaaaaaaaaa aaaaaaaaaa aaaaaaaaaa 1920

aaaaaaaaaa aaaaaaaaaa aaaaaaaaaa aaaaaaaaaa aaaaaaaaaa aaaaaaaaaa 1980

aaaaaaaaaa aaaaaaaaaa aaaaaaaaaa aaaaaaaaaa aaaaaaaaaa aaaaaaaaaa 2040

aaaaaaaaaa aaaaaaaaaa aaaaaaaaaa aaaaaaaaaa aaaaaaaaaa aaaaaaaaaa 2100

aaaaaaaaaa aaaaaaaaaa aaaaaaaaaa aaaaaaaaaa aaaaaaaaaa aaaaaaaaaa 2160

aaaaaaaaaa aaaaaaaaaa aaaaaaaaaa aaaaaaaaaa aaaaaaaaaa aaaaaaaaaa 2220

aaaaaaaaaa aaaaaaaaaa aaaaaaaaaa aaaaaaaaaa aaaaaaaaaa aaaaaaaaaa 2280

aaaaaaaaaa aaaaaaaaaa aaaaaaaaaa aaaaaaaaaa aaaaaaaaaa aaaaaaaaaa 2340

aaaaaaaaaa aaaaaaaaaa aaaaaaaaaa aaaaaaaaaa aaaaaaaaaa aaaaaaaaaa 2400

aaaaaaaaaa aaaaaaaaaa aaaaaaaaaa aaaaaaaaaa aaaaaaaaaa aaaaaaaaaa 2460

aaaaaaaaaa aaaaaaaaaa aaaaaaaaaa aaaaaaaaaa aaaaaaaaaa aaaaaaaaaa 2520

aaaaaaaaaa aaaaaaaaaa aaaaaaaaaa aaaaaaaaaa aaaaaaaaaa aaaaaaaaaa 2580

aaaaaaaaaa aaaaaaaaaa aaaaaaaaaa aaaaaaaaaa aaaaaaaaaa aaaaaaaaaa 2640

aaaaaaaaaa aaaaaaaaaa aaaaaaaaaa aaaaaaaaaa aaaaaaaaaa aaaaaaaaaa 2700

aaaaaaaaaa aaaaaaaaaa aaaaaaaaaa aaaaaaaaaa aaaaaaaaaa aaaaaaaaaa 2760

aaaaaaaaaa aaaaaaaaaa aaaaaaaaaa aaaaaaaaaa aaaaaaaaaa aaaaaaaaaa 2820

aaaaaaaaaa aaaaaaaaaa aaaaaaaaaa aaaaaaaaaa aaaaaaaaaa aaaaaaaaaa 2880

aaaaaaaaaa aaaaaaaaaa aaaaaaaaaa aaaaaaaaaa aaaaaaaaaa aaaaaaaaaa 2940

aaaaaaaaaa aaaaaaaaaa aaaaaaaaaa aaaaaaaaaa aaaaaaaaaa aaaaaaaaaa 3000

aaaaaaaaaa aaaaaaaaaa aaaaaaaaaa aaaaaaaaaa aaaaaaaaaa aaaaaaaaaa 3060

aaaaaaaaaa aaaaaaaaaa aaaaaaaaaa aaaaaaaaaa aaaaaaaaaa aaaaaaaaaa 3120

aaaaaaaaaa aaaaaaaaaa aaaaaaaaaa aaaaaaaaaa aaaaaaaaaa aaaaaaaaaa 3180

aaaaaaaaaa aaaaaaaaaa aaaaaaaaaa aaaaaaaaaa aaaaaaaaaa aaaaaaaaaa 3240

aaaaaaaaaa aaaaaaaaaa aaaaaaaaaa aaaaaaaaaa aaaaaaaaaa aaaaaaaaaa 3300

aaaaaaaaaa aaaaaaaaaa aaaaaaaaaa aaaaaaaaaa aaaaaaaaaa aaaaaaaaaa 3360

aaaaaaaaaa aaaaaaaaaa aaaaaaaaaa aaaaaaaaaa aaaaaaaaaa aaaaaaaaaa 3420

aaaaaaaaaa aaaaaaaaaa aaaaaaaaaa aaaaaaaaaa aaaaaaaaaa aaaaaaaaaa 3480

aaaaaaaaaa aaaaaaaaaa aaaaaaaaaa aaaaaaaaaa aaaaaaaaaa aaaaaaaaaa 3540

aaaaaaaaaa aaaaaaaaaa aaaaaaaaaa aaaaaaaaaa aaaaaaaaaa aaaaaaaaaa 3600

aaaaaaaaaa aaaaaaaaaa aaaaaaaaaa aaaaaaaaaa aaaaaaaaaa aaaaaaaaaa 3660

aaaaaaaaaa aaaaaaaaaa aaaaaaaaaa aaaaaaaaaa aaaaaaaaaa aaaaaaaaaa 3720

aaaaaaaaaa aaaaaaaaaa aaaaaaaaaa aaaaaaaaaa aaaaaaaaaa aaaaaaaaaa 3780

aaaaaaaaaa aaaaaaaaaa aaaaaaaaaa aaaaaaaaaa aaaaaaaaaa aaaaaaaaaa 3840

aaaaaaaaaa aaaaaaaaaa aaaaaaaaaa aaaaaaaaaa aaaaaaaaaa aaaaaaaaaa 3900

aaaaaaaaaa aaaaaaaaaa aaaaaaaaaa aaaaaaaaaa aaaaaaaaaa aaaaaaaaaa 3960

aaaaaaaaaa aaaaaaaaaa aaaaaaaaaa aaaaaaaaaa aaaaaaaaaa aaaaaaaaaa 4020

aaaaaaaaaa aaaaaaaaaa aaaaaaaaaa aaaaaaaaaa aaaaaaaaaa aaaaaaaaaa 4080

aaaaaaaaaa aaaaaaaaaa aaaaaaaaaa aaaaaaaaaa aaaaaaaaaa aaaaaaaaaa 4140

aaaaaaaaaa aaaaaaaaaa aaaaaaaaaa aaaaaaaaaa aaaaaaaaaa aaaaaaaaaa 4200

aaaaaaaaaa aaaaaaaaaa aaaaaaaaaa aaaaaaaaaa aaaaaaaaaa aaaaaaaaaa 4260

aaaaaaaaaa aaaaaaaaaa aaaaaaaaaa aaaaaaaaaa aaaaaaaaaa aaaaaaaaaa 4320

aaaaaaaaaa aaaaaaaaaa aaaaaaaaaa aaaaaaaaaa aaaaaaaaaa aaaaaaaaaa 4380

aaaaaaaaaa aaaaaaaaaa aaaaaaaaaa aaaaaaaaaa aaaaaaaaaa aaaaaaaaaa 4440

aaaaaaaaaa aaaaaaaaaa aaaaaaaaaa aaaaaaaaaa aaaaaaaaaa aaaaaaaaaa 4500

aaaaaaaaaa aaaaaaaaaa aaaaaaaaaa aaaaaaaaaa aaaaaaaaaa aaaaaaaaaa 4560

aaaaaaaaaa aaaaaaaaaa aaaaaaaaaa aaaaaaaaaa aaaaaaaaaa aaaaaaaaaa 4620

aaaaaaaaaa aaaaaaaaaa aaaaaaaaaa aaaaaaaaaa aaaaaaaaaa aaaaaaaaaa 4680

aaaaaaaaaa aaaaaaaaaa aaaaaaaaaa aaaaaaaaaa aaaaaaaaaa aaaaaaaaaa 4740

aaaaaaaaaa aaaaaaaaaa aaaaaaaaaa aaaaaaaaaa aaaaaaaaaa aaaaaaaaaa 4800

aaaaaaaaaa aaaaaaaaaa aaaaaaaaaa aaaaaaaaaa aaaaaaaaaa aaaaaaaaaa 4860

aaaaaaaaaa aaaaaaaaaa aaaaaaaaaa aaaaaaaaaa aaaaaaaaaa aaaaaaaaaa 4920

aaaaaaaaaa aaaaaaaaaa aaaaaaaaaa aaaaaaaaaa aaaaaaaaaa aaaaaaaaaa 4980

aaaaaaaaaa aaaaaaaaaa 5000


<210> 34

<400> 34
000


<210> 35
<211> 2000
<212> DNA
<213> Artificial Sequence

<220>
<221> source
<223> /note="Description of Artificial Sequence: Synthetic
      polynucleotide"


<220>
<221> misc_feature
<222> (1)..(2000)
<223> /note="This sequence may encompass 50-2000 nucleotides"

<400> 35
aaaaaaaaaa aaaaaaaaaa aaaaaaaaaa aaaaaaaaaa aaaaaaaaaa aaaaaaaaaa 60

aaaaaaaaaa aaaaaaaaaa aaaaaaaaaa aaaaaaaaaa aaaaaaaaaa aaaaaaaaaa 120

aaaaaaaaaa aaaaaaaaaa aaaaaaaaaa aaaaaaaaaa aaaaaaaaaa aaaaaaaaaa 180

aaaaaaaaaa aaaaaaaaaa aaaaaaaaaa aaaaaaaaaa aaaaaaaaaa aaaaaaaaaa 240

aaaaaaaaaa aaaaaaaaaa aaaaaaaaaa aaaaaaaaaa aaaaaaaaaa aaaaaaaaaa 300

aaaaaaaaaa aaaaaaaaaa aaaaaaaaaa aaaaaaaaaa aaaaaaaaaa aaaaaaaaaa 360

aaaaaaaaaa aaaaaaaaaa aaaaaaaaaa aaaaaaaaaa aaaaaaaaaa aaaaaaaaaa 420

aaaaaaaaaa aaaaaaaaaa aaaaaaaaaa aaaaaaaaaa aaaaaaaaaa aaaaaaaaaa 480

aaaaaaaaaa aaaaaaaaaa aaaaaaaaaa aaaaaaaaaa aaaaaaaaaa aaaaaaaaaa 540

aaaaaaaaaa aaaaaaaaaa aaaaaaaaaa aaaaaaaaaa aaaaaaaaaa aaaaaaaaaa 600

aaaaaaaaaa aaaaaaaaaa aaaaaaaaaa aaaaaaaaaa aaaaaaaaaa aaaaaaaaaa 660

aaaaaaaaaa aaaaaaaaaa aaaaaaaaaa aaaaaaaaaa aaaaaaaaaa aaaaaaaaaa 720

aaaaaaaaaa aaaaaaaaaa aaaaaaaaaa aaaaaaaaaa aaaaaaaaaa aaaaaaaaaa 780

aaaaaaaaaa aaaaaaaaaa aaaaaaaaaa aaaaaaaaaa aaaaaaaaaa aaaaaaaaaa 840

aaaaaaaaaa aaaaaaaaaa aaaaaaaaaa aaaaaaaaaa aaaaaaaaaa aaaaaaaaaa 900

aaaaaaaaaa aaaaaaaaaa aaaaaaaaaa aaaaaaaaaa aaaaaaaaaa aaaaaaaaaa 960

aaaaaaaaaa aaaaaaaaaa aaaaaaaaaa aaaaaaaaaa aaaaaaaaaa aaaaaaaaaa 1020

aaaaaaaaaa aaaaaaaaaa aaaaaaaaaa aaaaaaaaaa aaaaaaaaaa aaaaaaaaaa 1080

aaaaaaaaaa aaaaaaaaaa aaaaaaaaaa aaaaaaaaaa aaaaaaaaaa aaaaaaaaaa 1140

aaaaaaaaaa aaaaaaaaaa aaaaaaaaaa aaaaaaaaaa aaaaaaaaaa aaaaaaaaaa 1200

aaaaaaaaaa aaaaaaaaaa aaaaaaaaaa aaaaaaaaaa aaaaaaaaaa aaaaaaaaaa 1260

aaaaaaaaaa aaaaaaaaaa aaaaaaaaaa aaaaaaaaaa aaaaaaaaaa aaaaaaaaaa 1320

aaaaaaaaaa aaaaaaaaaa aaaaaaaaaa aaaaaaaaaa aaaaaaaaaa aaaaaaaaaa 1380

aaaaaaaaaa aaaaaaaaaa aaaaaaaaaa aaaaaaaaaa aaaaaaaaaa aaaaaaaaaa 1440

aaaaaaaaaa aaaaaaaaaa aaaaaaaaaa aaaaaaaaaa aaaaaaaaaa aaaaaaaaaa 1500

aaaaaaaaaa aaaaaaaaaa aaaaaaaaaa aaaaaaaaaa aaaaaaaaaa aaaaaaaaaa 1560

aaaaaaaaaa aaaaaaaaaa aaaaaaaaaa aaaaaaaaaa aaaaaaaaaa aaaaaaaaaa 1620

aaaaaaaaaa aaaaaaaaaa aaaaaaaaaa aaaaaaaaaa aaaaaaaaaa aaaaaaaaaa 1680

aaaaaaaaaa aaaaaaaaaa aaaaaaaaaa aaaaaaaaaa aaaaaaaaaa aaaaaaaaaa 1740

aaaaaaaaaa aaaaaaaaaa aaaaaaaaaa aaaaaaaaaa aaaaaaaaaa aaaaaaaaaa 1800

aaaaaaaaaa aaaaaaaaaa aaaaaaaaaa aaaaaaaaaa aaaaaaaaaa aaaaaaaaaa 1860

aaaaaaaaaa aaaaaaaaaa aaaaaaaaaa aaaaaaaaaa aaaaaaaaaa aaaaaaaaaa 1920

aaaaaaaaaa aaaaaaaaaa aaaaaaaaaa aaaaaaaaaa aaaaaaaaaa aaaaaaaaaa 1980

aaaaaaaaaa aaaaaaaaaa 2000

<210> 36

<400> 36
000


<210> 37

<400> 37
000


<210> 38
<211> 40
<212> PRT
<213> Artificial Sequence

<220>
<221> source
<223> /note="Description of Artificial Sequence: Synthetic
      polypeptide"


<220>
<221> MISC_FEATURE
<222> (1)..(40)
<223> /note="This sequence may encompass 1-10 'Gly Gly Gly Ser'
      repeating units"

<400> 38
Gly Gly Gly Ser Gly Gly Gly Ser Gly Gly Gly Ser Gly Gly Gly Ser
1               5                   10                  15


Gly Gly Gly Ser Gly Gly Gly Ser Gly Gly Gly Ser Gly Gly Gly Ser
            20                  25                  30


Gly Gly Gly Ser Gly Gly Gly Ser
        35                  40


<210> 39
<211> 247
<212> PRT
<213> Artificial Sequence

<220>
<221> source
<223> /note="Description of Artificial Sequence: Synthetic
      polypeptide"

<400> 39
Glu Val Gln Leu Gln Gln Ser Gly Ala Glu Val Lys Lys Pro Gly Ser
1               5                   10                  15


Ser Val Lys Val Ser Cys Lys Ala Ser Gly Gly Thr Phe Ser Ser Tyr
            20                  25                  30


Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Leu Glu Met Ala Thr Ile Met Gly Gly Tyr Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Gln Ser Ala Leu Thr Gln Pro Ala Ser
    130                 135                 140

Val Ser Gly Ser Pro Gly Gln Ser Ile Thr Ile Ser Cys Thr Gly Thr
145                 150                 155                 160

Ser Ser Asp Val Gly Gly Tyr Asn Tyr Val Ser Trp Tyr Gln Gln His
                165                 170                 175

Pro Gly Lys Ala Pro Lys Leu Met Ile Tyr Asp Val Ser Asn Arg Pro
            180                 185                 190

Ser Gly Val Ser Asn Arg Phe Ser Gly Ser Lys Ser Gly Asn Thr Ala
        195                 200                 205

Ser Leu Thr Ile Ser Gly Leu Gln Ala Glu Asp Glu Ala Asp Tyr Tyr
    210                 215                 220

Cys Ser Ser Tyr Thr Ser Ser Ser Thr Leu Asp Val Val Phe Gly Gly
225                 230                 235                 240

Gly Thr Lys Leu Thr Val Leu
                245

<210> 40
<211> 245
<212> PRT
<213> Artificial Sequence

<220>
<221> source
<223> /note="Description of Artificial Sequence: Synthetic
      polypeptide"

<400> 40
Glu Val Gln Leu Val Glu Ser Gly Gly Gly Val Val Gln Pro Gly Gly
1               5                   10                  15

Ser Leu Arg Leu Ser Cys Ala Ala Ser Gly Phe Thr Phe Asp Asp Tyr
            20                  25                  30

Ala Met His Trp Val Arg Gln Ala Pro Gly Lys Gly Leu Glu Trp Val
        35                  40                  45

Ser Leu Ile Ser Gly Asp Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Val Glu Asp Thr Ala Val Tyr Tyr Cys
                85                  90                  95

Ala Arg Val Phe Asp Ser Tyr Tyr Met Asp Val Trp Gly Lys Gly Thr
            100                 105                 110

Thr Val Thr Val Ser Ser Gly Gly Gly Gly Ser Gly Gly Gly Gly Ser
        115                 120                 125

Gly Ser Gly Gly Ser Glu Ile Val Leu Thr Gln Ser Pro Leu Ser Leu
    130                 135                 140

Pro Val Thr Pro Gly Gln Pro Ala Ser Ile Ser Cys Arg Ser Ser Gln
145                 150                 155                 160

Ser Leu Val Tyr Thr Asp Gly Asn Thr Tyr Leu Asn Trp Phe Gln Gln
                165                 170                 175

Arg Pro Gly Gln Ser Pro Arg Arg Leu Ile Tyr Lys Val Ser Asn Arg
            180                 185                 190

Asp Ser Gly Val Pro Asp Arg Phe Ser Gly Ser Gly Ser Asp Thr Asp
        195                 200                 205

Phe Thr Leu Lys Ile Ser Arg Val Glu Ala Glu Asp Val Gly Ile Tyr
    210                 215                 220

Tyr Cys Met Gln Gly Thr His Trp Ser Phe Thr Phe Gly Gln Gly Thr
225                 230                 235                 240

Arg Leu Glu Ile Lys

                245

<210> 41
<211> 247
<212> PRT
<213> Artificial Sequence

<220>
<221> source
<223> /note="Description of Artificial Sequence: Synthetic
      polypeptide"

<400> 41
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Ser
            20                  25                  30

Ser Tyr Tyr Trp Gly Trp Ile Arg Gln Pro Pro Gly Lys Gly Leu Glu
        35                  40                  45

Trp Ile Gly Ser Ile Tyr Tyr Ser Gly Ser Thr Tyr Tyr Asn Pro Ser
    50                  55                  60

Leu Lys Ser Arg Val Ser Ile Ser Val Asp Thr Ser Lys Asn Gln Phe
65                  70                  75                  80

Ser Leu Lys Leu Lys Tyr Val Thr Ala Ala Asp Thr Ala Val Tyr Tyr
                85                  90                  95

Cys Ala Thr Pro Gly Thr Tyr Tyr Asp Phe Leu Ser Gly Tyr Tyr Pro
            100                 105                 110

Phe Tyr Trp Gly Gln Gly Thr Leu Val Thr Val Ser Ser Gly Gly Gly
        115                 120                 125

Gly Ser Gly Gly Gly Gly Ser Gly Gly Gly Gly Ser Asp Ile Val Met
    130                 135                 140

Thr Gln Ser Pro Ser Ser Leu Ser Ala Ser Val Gly Asp Arg Val Thr
145                 150                 155                 160

Ile Thr Cys Arg Ala Ser Gln Gly Ile Ser Ser Tyr Leu Ala Trp Tyr
                165                 170                 175

Gln Gln Lys Pro Gly Lys Ala Pro Lys Leu Leu Ile Tyr Ala Ala Ser
            180                 185                 190

Thr Leu Gln Ser Gly Val Pro Ser Arg Phe Ser Gly Ser Gly Ser Gly

195                 200                 205

Thr Asp Phe Thr Leu Thr Ile Ser Ser Leu Gln Pro Glu Asp Phe Ala
    210                 215                 220

Thr Tyr Tyr Cys Gln Gln Leu Asn Ser Tyr Pro Tyr Thr Phe Gly Gln
225                 230                 235                 240

Gly Thr Lys Leu Glu Ile Lys
                245

<210> 42
<211> 239
<212> PRT
<213> Artificial Sequence

<220>
<221> source
<223> /note="Description of Artificial Sequence: Synthetic
      polypeptide"

<400> 42
Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Asn Glu Asp Gly Ser Ala Lys Phe Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Phe Cys
                85                  90                  95

Ala Arg Asp Leu Arg Ser Gly Arg Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Gly Arg Ala Thr Leu Ser Cys Arg Ala Ser Gln Ser Ile

145                 150                 155                 160

Ser Gly Ser Phe Leu Ala Trp Tyr Gln Gln Lys Pro Gly Gln Ala Pro
                165                 170                 175

Arg Leu Leu Ile Tyr Gly Ala Ser Ser Arg Ala Thr Gly Ile Pro Asp
            180                 185                 190

Arg Phe Ser Gly Ser Gly Ser Gly Thr Asp Phe Thr Leu Thr Ile Ser
        195                 200                 205

Arg Leu Glu Pro Glu Asp Phe Ala Val Tyr Tyr Cys Gln Gln Tyr Gly
    210                 215                 220

Ser Ser Pro Pro Thr Phe Gly Leu Gly Thr Lys Leu Glu Ile Lys
225                 230                 235

<210> 43
<211> 245
<212> PRT
<213> Artificial Sequence

<220>
<221> source
<223> /note="Description of Artificial Sequence: Synthetic
      polypeptide"

<400> 43
Glu Val Gln Leu Gln Gln Ser Gly Pro Gly Leu Val Arg Pro Ser Glu
1               5                   10                  15

Thr Leu Ser Leu Thr Cys Thr Val Ser Gly Gly Pro Val Arg Ser Gly
            20                  25                  30

Ser His Tyr Trp Asn Trp Ile Arg Gln Pro Pro Gly Arg Gly Leu Glu
        35                  40                  45

Trp Ile Gly Tyr Ile Tyr Tyr Ser Gly Ser Thr Asn Tyr Asn Pro Ser
    50                  55                  60

Leu Glu Asn Arg Val Thr Ile Ser Ile Asp Thr Ser Asn Asn His Phe
65                  70                  75                  80

Ser Leu Lys Leu Ser Ser Val Thr Ala Ala Asp Thr Ala Leu Tyr Phe
                85                  90                  95

Cys Ala Arg Gly Thr Ala Thr Phe Asp Trp Asn Phe Pro Phe Asp Ser
            100                 105                 110

Trp Gly Gln Gly Thr Leu Val Thr Val Ser Ser Gly Gly Gly Gly Ser

115                 120                 125

Gly Gly Gly Gly Ser Gly Ser Gly Gly Ser Asp Ile Gln Met Thr Gln
    130                 135                 140

Ser Pro Ser Ser Leu Ser Ala Ser Ile Gly Asp Arg Val Thr Ile Thr
145                 150                 155                 160

Cys Arg Ala Ser Gln Ser Ile Ser Ser Tyr Leu Asn Trp Tyr Gln Gln
                165                 170                 175

Lys Pro Gly Lys Ala Pro Lys Leu Leu Ile Tyr Ala Ala Ser Ser Leu
            180                 185                 190

Gln Ser Gly Val Pro Ser Arg Phe Ser Gly Ser Gly Ser Gly Thr Asp
        195                 200                 205

Phe Thr Leu Thr Ile Ser Ser Leu Gln Pro Glu Asp Phe Ala Thr Tyr
    210                 215                 220

Tyr Cys Gln Gln Ser Tyr Ser Thr Pro Trp Thr Phe Gly Gln Gly Thr
225                 230                 235                 240

Lys Leu Glu Ile Lys
                245

<210> 44
<211> 249
<212> PRT
<213> Artificial Sequence

<220>
<221> source
<223> /note="Description of Artificial Sequence: Synthetic
      polypeptide"

<400> 44
Gln Val Gln Leu Gln Glu Ser Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Val
        35                  40                  45

Gly Glu Ile Asn His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu

65                  70                  75                  80

Lys Leu Ser Ser Val Thr Ala Ala Asp Thr Ala Val Tyr Tyr Cys Ala
                85                  90                  95

Arg Gly Ser Gly Leu Val Val Tyr Ala Ile Arg Val Gly Ser Gly Trp
            100                 105                 110

Phe Asp Tyr Trp Gly Gln Gly Thr Leu Val Thr Val Ser Ser Gly Gly
        115                 120                 125

Gly Gly Ser Gly Gly Gly Asp Ser Gly Gly Gly Gly Ser Asp Ile Gln
    130                 135                 140

Met Thr Gln Ser Pro Ser Ser Leu Ser Ala Ser Val Gly Asp Arg Val
145                 150                 155                 160

Thr Ile Thr Cys Arg Ala Ser Gln Ser Ile Ser Ser Tyr Leu Asn Trp
                165                 170                 175

Tyr Gln Gln Lys Pro Gly Lys Ala Pro Lys Leu Leu Met Tyr Ala Ala
            180                 185                 190

Ser Ser Leu Gln Ser Gly Val Pro Ser Arg Phe Ser Gly Ser Gly Ser
        195                 200                 205

Gly Thr Asp Phe Thr Leu Thr Ile Ser Ser Leu Gln Pro Glu Asp Phe
    210                 215                 220

Ala Thr Tyr Tyr Cys Gln Gln Ser Tyr Ser Thr Pro Pro Trp Thr Phe
225                 230                 235                 240

Gly Gln Gly Thr Lys Val Asp Ile Lys
                245

<210> 45
<211> 255
<212> PRT
<213> Artificial Sequence

<220>
<221> source
<223> /note="Description of Artificial Sequence: Synthetic
      polypeptide"

<400> 45
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr

            20                  25                  30

Ser Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Pro Ser Ser Ser Gly Ser Tyr Tyr Met Glu Asp Ser Tyr
            100                 105                 110

Tyr Tyr Gly Met Asp Val Trp Gly Gln Gly Thr Thr Val Thr Val Ser
        115                 120                 125

Ser Gly Gly Gly Gly Ser Gly Gly Gly Gly Ser Gly Gly Gly Gly Ser
    130                 135                 140

Asn Phe Met Leu Thr Gln Pro His Ser Val Ser Glu Ser Pro Gly Lys
145                 150                 155                 160

Thr Val Thr Ile Ser Cys Thr Gly Ser Ser Gly Ser Ile Ala Ser Asn
                165                 170                 175

Tyr Val Gln Trp Tyr Gln Gln Arg Pro Gly Ser Ala Pro Thr Thr Val
            180                 185                 190

Ile Tyr Glu Asp Asn Gln Arg Pro Ser Gly Val Pro Asp Arg Phe Ser
        195                 200                 205

Gly Ser Ile Asp Ser Ser Ser Asn Ser Ala Ser Leu Thr Ile Ser Gly
    210                 215                 220

Leu Lys Thr Glu Asp Glu Ala Asp Tyr Tyr Cys Gln Ser Tyr Asp Ser
225                 230                 235                 240

Ser Asn Gln Val Val Phe Gly Gly Gly Thr Lys Leu Thr Val Leu
                245                 250                 255

<210> 46
<211> 239
<212> PRT
<213> Artificial Sequence

<220>
<221> source
<223> /note="Description of Artificial Sequence: Synthetic
      polypeptide"

<400> 46
Gln Val Asn Leu Arg Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Glu Met Asn Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Ala Leu Gly Ser Ser Trp Glu Trp Gly Gln Gly Thr Thr
            100                 105                 110

Val Thr Val Ser Ser Gly Gly Gly Gly Ser Gly Gly Gly Gly Ser Gly
        115                 120                 125

Gly Gly Gly Ser Asp Ile Gln Met Thr Gln Ser Pro Ser Ser Leu Ser
    130                 135                 140

Ala Ser Val Gly Asp Arg Val Thr Ile Thr Cys Gln Ala Ser Gln Asp
145                 150                 155                 160

Ile Ser Asn Tyr Leu Asn Trp Tyr Gln Gln Lys Pro Gly Lys Ala Pro
                165                 170                 175

Lys Leu Leu Ile Tyr Asp Ala Ser Asn Leu Glu Thr Gly Val Pro Ser
            180                 185                 190

Arg Phe Ser Gly Ser Gly Ser Gly Thr Asp Phe Thr Phe Thr Ile Ser
        195                 200                 205

Ser Leu Gln Pro Glu Asp Ile Ala Thr Tyr Tyr Cys Gln Gln Tyr Asp
    210                 215                 220

Asn Leu Pro Leu Thr Phe Gly Gly Gly Thr Lys Leu Glu Ile Lys
225                 230                 235

<210> 47
<211> 246
<212> PRT
<213> Artificial Sequence

<220>
<221> source
<223> /note="Description of Artificial Sequence: Synthetic
      polypeptide"

<400> 47
Gln Val Gln Leu Val Gln Ser Gly Ala Glu Val Lys Glu Pro Gly Ala
1               5                   10                  15

Ser Val Lys Val Ser Cys Lys Ala Pro Ala Asn Thr Phe Ser Asp His
            20                  25                  30

Val Met His Trp Val Arg Gln Ala Pro Gly Gln Arg Phe Glu Trp Met
        35                  40                  45

Gly Tyr Ile His Ala Ala Asn Gly Gly Thr His Tyr Ser Gln Lys Phe
    50                  55                  60

Gln Asp Arg Val Thr Ile Thr Arg Asp Thr Ser Ala Asn Thr Val Tyr
65                  70                  75                  80

Met Asp Leu Ser Ser Leu Arg Ser Glu Asp Thr Ala Val Tyr Tyr Cys
                85                  90                  95

Ala Arg Gly Gly Tyr Asn Ser Asp Ala Phe Asp Ile Trp Gly Gln Gly
            100                 105                 110

Thr Met Val Thr Val Ser Ser Gly Gly Gly Gly Ser Gly Gly Gly Gly
        115                 120                 125

Ser Gly Gly Gly Gly Ser Gly Gly Gly Gly Ser Asp Ile Val Met Thr
    130                 135                 140

Gln Ser Pro Ser Ser Val Ser Ala Ser Val Gly Asp Arg Val Thr Ile
145                 150                 155                 160

Thr Cys Arg Ala Ser Gln Asp Ile Ser Ser Trp Leu Ala Trp Tyr Gln
                165                 170                 175

Gln Lys Pro Gly Lys Ala Pro Lys Leu Leu Ile Tyr Ala Ala Ser Ser
            180                 185                 190

Leu Gln Ser Gly Val Pro Ser Arg Phe Asn Gly Ser Gly Ser Gly Thr
        195                 200                 205

Asp Phe Thr Leu Thr Ile Ser Ser Leu Gln Pro Glu Asp Phe Ala Thr
    210                 215                 220

Tyr Tyr Cys Gln Gln Ser Tyr Ser Thr Pro Leu Thr Phe Gly Gly Gly
225                 230                 235                 240

Thr Lys Val Glu Ile Lys
                245

<210> 48
<211> 248
<212> PRT
<213> Artificial Sequence

<220>
<221> source
<223> /note="Description of Artificial Sequence: Synthetic
      polypeptide"

<400> 48
Gln Val Gln Leu Val Gln Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Tyr Ile Ser Ser Ser Ser Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Ser Val Arg Ala Ile Asp Ala Phe Asp Ile Trp Gly
            100                 105                 110

Gln Gly Thr Met Val Thr Val Ser Ser Gly Gly Gly Gly Ser Gly Gly
        115                 120                 125

Gly Gly Ser Gly Gly Gly Gly Ser Gly Gly Gly Gly Ser Asp Ile Val
    130                 135                 140

Leu Thr Gln Ser Pro Ser Ser Leu Ser Ala Ser Val Gly Asp Arg Val
145                 150                 155                 160

Thr Ile Thr Cys Gln Ala Ser Gln Asp Ile Ser Asn Tyr Leu Asn Trp
                165                 170                 175

Tyr Gln Gln Lys Pro Gly Lys Ala Pro Lys Leu Leu Ile Tyr Asp Ala
            180                 185                 190

Ser Asn Leu Glu Thr Gly Val Pro Ser Arg Phe Ser Gly Ser Gly Ser
        195                 200                 205

Gly Thr Asp Phe Thr Phe Thr Ile Ser Ser Leu Gln Pro Glu Asp Phe
    210                 215                 220

Ala Thr Tyr Tyr Cys Gln Gln Ala Tyr Ser Thr Pro Phe Thr Phe Gly
225                 230                 235                 240

Pro Gly Thr Lys Val Glu Ile Lys
                245

<210> 49
<211> 246
<212> PRT
<213> Artificial Sequence

<220>
<221> source
<223> /note="Description of Artificial Sequence: Synthetic
      polypeptide"

<400> 49
Glu Val Gln Leu Val Gln Ser Gly Gly Gly Val Val Arg Ser Gly Arg
1               5                   10                  15

Ser Leu Arg Leu Ser Cys Ala Ala Ser Gly Phe Thr Phe Asn Ser Tyr
            20                  25                  30

Gly Leu His Trp Val Arg Gln Ala Pro Gly Lys Gly Leu Glu Trp Val
        35                  40                  45

Ala Leu Ile Glu Tyr Asp Gly Ser Asn Lys Tyr Tyr Gly Asp Ser Val
    50                  55                  60

Lys Gly Arg Phe Thr Ile Ser Arg Asp Lys Ser Lys Ser Thr Leu Tyr
65                  70                  75                  80

Leu Gln Met Asp Asn Leu Arg Ala Glu Asp Thr Ala Val Tyr Tyr Cys
                85                  90                  95

Ala Arg Glu Gly Asn Glu Asp Leu Ala Phe Asp Ile Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Gly Gly Gly Gly Ser Glu Ile Val Leu Thr
    130                 135                 140

Gln Ser Pro Ser Ser Leu Ser Ala Ser Val Gly Asp Arg Val Thr Ile
145                 150                 155                 160

Thr Cys Gln Ala Ser Gln Phe Ile Lys Lys Asn Leu Asn Trp Tyr Gln
                165                 170                 175

His Lys Pro Gly Lys Ala Pro Lys Leu Leu Ile Tyr Asp Ala Ser Ser
            180                 185                 190

Leu Gln Thr Gly Val Pro Ser Arg Phe Ser Gly Asn Arg Ser Gly Thr
        195                 200                 205

Thr Phe Ser Phe Thr Ile Ser Ser Leu Gln Pro Glu Asp Val Ala Thr
    210                 215                 220

Tyr Tyr Cys Gln Gln His Asp Asn Leu Pro Leu Thr Phe Gly Gly Gly
225                 230                 235                 240

Thr Lys Val Glu Ile Lys
                245

<210> 50
<211> 255
<212> PRT
<213> Artificial Sequence

<220>
<221> source
<223> /note="Description of Artificial Sequence: Synthetic
      polypeptide"

<400> 50
Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Val Ser Ser Asn
            20                  25                  30

Tyr Met Thr Trp Val Arg Gln Ala Pro Gly Lys Gly Leu Glu Trp Val
        35                  40                  45

Ser Val Ile Tyr Ser Gly Gly Ala Thr Tyr Tyr Gly Asp Ser Val Lys
    50                  55                  60

Gly Arg Phe Thr Val Ser Arg Asp Asn Ser Lys Asn Thr Val Tyr Leu
65                  70                  75                  80

Gln Met Asn Arg Leu Thr Ala Glu Asp Thr Ala Val Tyr Tyr Cys Ala
                85                  90                  95

Arg Asp Arg Leu Tyr Cys Gly Asn Asn Cys Tyr Leu Tyr Tyr Tyr Tyr
            100                 105                 110

Gly Met Asp Val Trp Gly Gln Gly Thr Leu Val Thr Val Ser Ser Gly
        115                 120                 125

Gly Gly Gly Ser Gly Gly Gly Gly Ser Gly Gly Gly Gly Ser Gly Gly
    130                 135                 140

Gly Gly Ser Asp Ile Gln Val Thr Gln Ser Pro Ser Ser Leu Ser Ala
145                 150                 155                 160

Ser Val Gly Asp Arg Val Thr Ile Thr Cys Arg Ala Ser Gln Ser Ile
                165                 170                 175

Ser Ser Tyr Leu Asn Trp Tyr Gln Gln Lys Pro Gly Lys Ala Pro Lys
            180                 185                 190

Leu Leu Ile Tyr Ala Ala Ser Ser Leu Gln Ser Gly Val Pro Ser Arg
        195                 200                 205

Phe Ser Gly Ser Gly Ser Gly Thr Asp Phe Thr Leu Thr Ile Ser Ser
    210                 215                 220

Leu Gln Pro Glu Asp Phe Ala Thr Tyr Tyr Cys Gln Gln Ser Tyr Ser
225                 230                 235                 240

Thr Pro Pro Leu Thr Phe Gly Gln Gly Thr Lys Val Glu Ile Lys
                245                 250                 255

<210> 51
<211> 247
<212> PRT
<213> Artificial Sequence

<220>
<221> source
<223> /note="Description of Artificial Sequence: Synthetic
      polypeptide"

<400> 51

Gln Val Gln Leu Val Gln Ser Gly Ala Glu Val Lys Lys Ser Gly Ala
1               5                   10                  15

Ser Val Lys Val Ser Cys Lys Ala Ser Gly Tyr Pro Phe Thr Gly Tyr
            20                  25                  30

Tyr Ile Gln Trp Val Arg Gln Ala Pro Gly Gln Gly Leu Glu Trp Met
        35                  40                  45

Gly Trp Ile Asp Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Ser Asp Ser Tyr Gly Tyr Tyr Tyr Gly Met Asp Val Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Gly Gly Gly Gly Ser Asp Ile Gln Met
    130                 135                 140

Thr Gln Ser Pro Ser Ser Leu Ser Ala Ser Val Gly Asp Arg Val Thr
145                 150                 155                 160

Phe Thr Cys Arg Ala Ser Gln Gly Ile Ser Ser Ala Leu Ala Trp Tyr
                165                 170                 175

Gln Gln Lys Pro Gly Lys Pro Pro Lys Leu Leu Ile Tyr Asp Ala Ser
            180                 185                 190

Ser Leu Glu Ser Gly Val Pro Ser Arg Phe Ser Gly Ser Gly Ser Gly
        195                 200                 205

Thr Asp Phe Thr Leu Thr Ile Ser Ser Leu Gln Pro Glu Asp Phe Ala
    210                 215                 220

Thr Tyr Tyr Cys Gln Gln Phe Asn Asn Tyr Pro Leu Thr Phe Gly Gly
225                 230                 235                 240

Gly Thr Lys Val Glu Ile Lys
                245

<210> 52
<211> 741
<212> DNA
<213> Artificial Sequence

<220>
<221> source
<223> /note="Description of Artificial Sequence: Synthetic
      polynucleotide"

<400> 52
gaagtgcaac tccaacagtc aggcgcagaa gtcaagaagc ccggatcgtc agtgaaagtg      60

tcctgcaaag cctccggcgg aaccttcagc tcctacgcaa tcagctgggt gcggcaggcg     120

cccggacagg gactggagtg gatgggcggt atcattccga tctttggcac cgccaattac     180

gcccagaagt tccagggacg cgtcacaatc accgccgacg aatcgacttc caccgcctac     240

atggagctgt cgtccttgag gagcgaagat accgccgtgt actactgcgc tcgggatctg     300

gagatggcca ctatcatggg gggttactgg ggccagggga ccctggtcac tgtgtcctcg     360

ggaggagggg gatcaggcgg cggcggttcc gggggaggag gaagccagtc cgcgctgact     420

cagccagctt ccgtgtctgg ttcgccggga cagtccatca ctattagctg taccggcacc     480

agcagcgacg tgggcggcta caactatgtg tcatggtacc agcagcaccc ggggaaggcg     540

cctaagctga tgatctacga cgtgtccaac cgccctagcg gagtgtccaa cagattctcc     600

ggttcgaagt cagggaacac tgcctccctc acgattagcg ggctgcaagc cgaggatgaa     660

gccgactact actgctcctc ctatacctcc tcctcgaccc tggacgtggt gttcggagga     720

ggcaccaagc tcaccgtcct t                                               741


<210> 53
<211> 735
<212> DNA
<213> Artificial Sequence

<220>
<221> source
<223> /note="Description of Artificial Sequence: Synthetic
      polynucleotide"

<400> 53
gaagtgcaat tggtggaaag cggaggagga gtggtgcaac ctggaggaag cctgagactg      60

tcatgtgccg cctcgggatt cactttcgat gactacgcaa tgcactgggt ccgccaggcc     120

cccggaaagg gtctggaatg ggtgtccctc atctccggcg atgggggttc cacttactat     180

gcggattctg tgaagggccg cttcacaatc tcccgggaca attccaagaa cactctgtac     240

cttcaaatga actccctgag ggtggaggac accgctgtgt actactgcgc gagagtgttt     300

gactcgtact atatggacgt ctggggaaag ggcaccaccg tgaccgtgtc cagcggtggc     360

ggtggatcgg ggggcggcgg ctccgggagc ggaggttccg agattgtgct gactcagtcg     420

ccgttgtcac tgcctgtcac ccccgggcag ccggcctcca tttcatgccg gtccagccag 480

tccctggtct acaccgatgg gaacacttac ctcaactggt tccagcagcg cccaggacag 540

tccccgcgga ggctgatcta caaagtgtca aaccgggact ccggcgtccc cgatcggttc 600

tcgggaagcg gcagcgacac cgacttcacg ctgaagattt cccgcgtgga agccgaggac 660

gtgggcatct actactgtat gcagggcacc cactggtcgt ttaccttcgg acaaggaact 720

aggctcgaga tcaag 735

<210> 54
<211> 741
<212> DNA
<213> Artificial Sequence

<220>
<221> source
<223> /note="Description of Artificial Sequence: Synthetic
      polynucleotide"

<400> 54
caagtgcagc ttcaagaaag cggtccagga ctcgtcaagc catcagaaac tctttccctc 60

acttgtaccg tgtcgggagg cagcatctcc tcgagctcct actactgggg ttggattaga 120

cagcccccgg gaaaggggtt ggagtggatc ggttccatct actactccgg gtcgacctac 180

tacaaccctt ccctgaaatc tcgggtgtcc atctccgtcg acacctccaa gaaccagttc 240

agcctgaagc tgaaatatgt gaccgcggcc gatactgccg tgtactattg cgccaccccg 300

ggaacctact acgacttcct ctcggggtac tacccgtttt actggggaca ggggactctc 360

gtgaccgtgt cctcgggcgg cggaggttca ggcggtggcg gatcgggggg aggaggctca 420

gacattgtga tgacccagag cccgtccagc ctgagcgcct ccgtgggcga tagggtcacg 480

attacttgcc gggcgtccca gggaatctca agctacctgg cctggtacca acagaagccc 540

ggaaaggcac ccaagttgct gatctatgcc gctagcactc tgcagtccgg ggtgccttcc 600

cgcttctccg gctccggctc gggcaccgac ttcaccctga ccatttcctc actgcaaccc 660

gaggacttcg ccacttacta ctgccagcag ctgaactcct acccttacac attcggacag 720

ggaaccaagc tggaaatcaa g 741

<210> 55
<211> 717
<212> DNA
<213> Artificial Sequence

<220>
<221> source
<223> /note="Description of Artificial Sequence: Synthetic
      polynucleotide"

<400> 55

caagtgcaac tcgtggaatc tggtggagga ctcgtgcaac ccggaggatc attgcgactc 60

tcgtgtgcgg catccggctt taccttttca tcctactgga tgtcctgggt cagacaggcc 120

cccgggaagg gactggaatg ggtcgcgaac atcaacgagg acggctcggc caagttctac 180

gtggactccg tgaagggccg cttcacgatc tcacgggata acgccaagaa ttccctgtat 240

ctgcaaatga acagcctgag ggccgaggac actgcggtgt acttctgcgc acgcgacctg 300

aggtccggga gatactgggg acagggcacc ctcgtgaccg tgtcgagcgg aggagggggg 360

tcgggcggcg gcggttccgg tggcggcggt agcgaaattg tgttgaccca gtcccctgga 420

accctgagcc tgtcacctgg aggacgcgcc accctgtcct gccgggccag ccagagcatc 480

tcagggtcct tcctggcttg gtaccagcag aagccgggac aggctccgag acttctgatc 540

tacggcgcct cctcgcgggc gaccggaatc ccggatcggt tctccggctc gggaagcgga 600

actgacttca ctcttaccat ttcccgcctg gagccggaag atttcgccgt gtactactgc 660

cagcagtacg ggtcatcccc tccaaccttc ggcctgggaa ctaagctgga aatcaaa 717

<210> 56
<211> 735
<212> DNA
<213> Artificial Sequence

<220>
<221> source
<223> /note="Description of Artificial Sequence: Synthetic
      polynucleotide"

<400> 56
gaagtgcaac tccaacaatc cggtccagga ctcgtcagac cctccgaaac tctctcgctt 60

acatgcactg tgtccggcgg ccctgtgcgg tccggctctc attactggaa ctggattcgc 120

cagcccccgg gacgcggact ggagtggatc ggctacatct attactcggg gtcgactaac 180

tacaacccga gcctggaaaa tagagtgacc atctcaatcg acacgtccaa caaccacttc 240

tcgctgaagt tgtcctccgt gactgccgcc gatactgccc tgtacttctg tgctcgcgga 300

accgccacct tcgactggaa cttccctttt gactcatggg gccaggggac ccttgtgacc 360

gtgtccagcg gaggaggagg ctccggtggt ggcgggagcg gtagcggcgg aagcgacatc 420

cagatgaccc agtcaccgtc ctcgctgtcc gcatccattg gggatcgggt cactattact 480

tgccgggcgt cccagtccat ctcgtcctac ctgaactggt atcagcagaa gccagggaaa 540

gcccccaagc tgctgatcta cgcggccagc agcctgcagt caggagtgcc ttcaaggttt 600

agcggcagcg gatcgggaac cgacttcacc ctgaccattt cctccctcca acccgaggat 660

ttcgccacct actactgcca gcagtcctac tccaccccgt ggaccttcgg acagggaacc 720

aagctggaga tcaag 735

<210> 57
<211> 747
<212> DNA
<213> Artificial Sequence

<220>
<221> source
<223> /note="Description of Artificial Sequence: Synthetic
      polynucleotide"

<400> 57
caagtgcaac ttcaagaatc aggcgcagga cttctcaagc catccgaaac actctccctc      60

acttgcgcgg tgtacggggg aagcttctcg ggatactact ggtcctggat taggcagcct     120

cccggcaaag gcctggaatg ggtcggggag atcaaccact ccggttcaac caactacaac     180

ccgtcgctga agtcccgcgt gaccatttcc gtggacacct ctaagaatca gttcagcctg     240

aagctctcgt ccgtgaccgc ggcggacacc gccgtctact actgcgctcg gggatcagga     300

ctggtggtgt acgccatccg cgtgggctcg ggctggttcg attactgggg ccagggaacc     360

ctggtcactg tgtcgtccgg cggaggaggt tcggggggcg gagacagcgg tggagggggt     420

agcgacatcc agatgaccca gtccccgtcc tcgctgtccg cctccgtggg agatagagtg     480

accatcacct gtcgggcatc ccagagcatt tccagctacc tgaactggta tcagcagaag     540

cccggaaagg cccctaagct gttgatgtac gccgccagca gcttgcagtc gggcgtgccg     600

agccggtttt ccggttccgg ctccgggact gacttcaccc tgactatctc atccctgcaa     660

cccgaggact tcgccactta ttactgccag cagtcctact caacccctcc ctggacgttc     720

ggacagggca ccaaggtcga tatcaag                                         747


<210> 58
<211> 765
<212> DNA
<213> Artificial Sequence

<220>
<221> source
<223> /note="Description of Artificial Sequence: Synthetic
      polynucleotide"

<400> 58
gaagtgcaat tggtggaatc tggaggagga cttgtgaaac ctggtggaag cctgagactt      60

tcctgtgcgg cctcgggatt cactttctcc tcctactcca tgaactgggt cagacaggcc     120

cctgggaagg gactggaatg ggtgtcatcc atctcctcct catcgtcgta catctactac     180

gccgatagcg tgaaggggcg gttcaccatt tcccgggaca acgctaagaa cagcctctat     240

ctgcaaatga attccctccg cgccgaggac actgccgtgt actactgcgc gagggacccc     300

tcatcaagcg gcagctacta catggaggac tcgtattact acggaatgga cgtctggggc     360

cagggaacca ctgtgacggt gtcctccggt ggagggggct ccgggggcgg gggatctggc     420

ggaggaggct ccaacttcat gctgacccag ccgcactccg tgtccgaaag ccccggaaag 480

accgtgacaa tttcctgcac cgggtcctcc ggctcgatcg catcaaacta cgtgcagtgg 540

taccagcagc gcccgggcag cgcccccacc actgtcatct acgaggataa ccagcggccg 600

tcgggtgtcc cagaccggtt ttccggttcg atcgatagca gcagcaacag cgcctccctg 660

accatttccg gcctcaagac cgaggatgag gctgactact actgccagtc gtatgactcc 720

tcgaaccaag tggtgttcgg tggcggcacc aagctgactg tgctg 765

<210> 59
<211> 717
<212> DNA
<213> Artificial Sequence

<220>
<221> source
<223> /note="Description of Artificial Sequence: Synthetic
      polynucleotide"

<400> 59
caagtgaacc tgagagaaag cggcggagga cttgtgcaac ctggaggaag cctgagactg 60

tcatgtgccg cgtccggctt caccttctcg tcctacgaga tgaactgggt ccgccaggca 120

ccgggcaaag gactggaatg ggtgtcctac atttcctcgt ccgggtccac catctattac 180

gccgactccg tgaagggacg gttcaccatc tcccgggaca acgccaagaa ctccctctac 240

ctccaaatga actcactgag ggcagaggac actgcggtct actactgcgc ccgcgaagct 300

ttgggtagct cctgggagtg ggccagggga accactgtga ccgtgtcctc gggtggaggg 360

ggctccggtg gcgggggttc agggggtggc ggaagcgata tccagatgac tcagtcacca 420

agctccctga gcgcctcagt gggagatcgg gtcacaatca cgtgccaggc gtcccaggac 480

atttctaact acctcaattg gtaccagcag aagccgggga aggcccccaa gcttctgatc 540

tacgatgcct ccaacctgga aaccggcgtg ccctcccgct tctcgggatc gggcagcggc 600

actgacttca cctttaccat ctcgtccctg caacctgagg acatcgccac ctattactgc 660

cagcagtacg ataacctccc gctgactttc ggaggcggaa ctaagctgga gattaag 717

<210> 60
<211> 738
<212> DNA
<213> Artificial Sequence

<220>
<221> source
<223> /note="Description of Artificial Sequence: Synthetic
      polynucleotide"

<400> 60
caagtgcaac tcgtccagtc cggtgcagaa gtcaaggaac ccggagcctc cgtgaaagtg 60

tcctgcaaag ctcctgccaa cactttctcg gaccacgtga tgcactgggt gcgccaggcg 120

ccgggccagc gcttcgaatg gatgggatac attcatgccg ccaatggcgg tacccactac 180

tcccaaaagt tccaggatag agtcaccatc acccgggaca ccagcgccaa caccgtgtat 240

atggatctgt ccagcctgag gtccgaggat accgccgtgt actactgcgc ccggggcgga 300

tacaactcag acgcgttcga catttgggga cagggtacta tggtcaccgt gtcatccggg 360

ggcggtggca gcgggggcgg aggctctggc ggaggcggat cagggggagg agggtccgac 420

atcgtgatga cccagtcccc gtcatcggtg tccgcgtccg tgggagacag agtgaccatc 480

acgtgtcgcg ccagccagga catctcctcg tggttggcat ggtaccagca gaagcctgga 540

aaggccccga agctgctcat ctacgccgcc tcctcccttc aatcgggagt gccctcgcgg 600

ttcaacggaa gcggaagcgg gacagatttt accctgacta ttagctcgct gcagcccgag 660

gacttcgcta cttactactg ccaacagagc tactccaccc cactgacttt cggcgggggt 720

accaaggtcg agatcaag 738

<210> 61
<211> 744
<212> DNA
<213> Artificial Sequence

<220>
<221> source
<223> /note="Description of Artificial Sequence: Synthetic
      polynucleotide"

<400> 61
caagtgcaac ttgttcaatc cggtggaggt cttgtgcagc ccggaggatc actcagactg 60

tcgtgcgccg cctctgggtt cactttctcc tcatactcga tgaactgggt gcgccaggcg 120

ccgggaaagg gcctggaatg ggtgtcatac atctcctcct catcctccac catctactac 180

gccgattccg tgaagggccg cttcactatt tcccgggaca acgcgaaaaa ctcgctctat 240

ctgcaaatga actccctgcg cgccgaggac accgccgtgt actactgcgc ccgggacctg 300

agcgtgcggg ctattgatgc gttcgacatc tggggacagg gcaccatggt cacagtgtcc 360

agcggaggcg gcggcagcgg tggaggagga tcagggggag gaggttcggg gggcggtggc 420

tccgatatcg tgctgaccca gagcccgtcg agcctctccg cctccgtcgg cgacagagtg 480

accatcacgt gtcaggcatc ccaggacatt agcaactacc tgaattggta ccagcagaag 540

cctggaaagg cacccaagtt gctgatctac gacgcctcca acctggaaac cggagtgcca 600

tccaggttct cgggcagcgg ctcgggaacc gacttcactt ttactatctc ctccctgcaa 660

cccgaggatt tcgcgaccta ctactgccag caggcctaca gcaccccttt caccttcggg 720

ccgggaacta aggtcgaaat caag 744

<210> 62

<211> 738
<212> DNA
<213> Artificial Sequence

<220>
<221> source
<223> /note="Description of Artificial Sequence: Synthetic
      polynucleotide"

<400> 62
gaagtgcaat tggtgcaatc aggaggagga gtggtcagat ctggaagaag cctgagactg      60

tcatgcgcgg cttcgggctt taccttcaac tcctacggcc tccactgggt gcgccaggcc     120

cccggaaaag gcctcgaatg ggtcgcactg attgagtacg acgggtccaa caagtactac     180

ggagatagcg tgaagggccg cttcaccatc tcacgggaca agtccaagtc caccctgtat     240

ctgcaaatgg acaacctgag ggccgaggat actgccgtgt actactgcgc ccgcgaagga     300

aacgaagatc tggccttcga tatttggggc cagggtactc ttgtgaccgt gtcgagcgga     360

ggcggaggct ccggtggagg aggatcgggg ggtggtggtt ccggcggcgg ggggagcgaa     420

atcgtgctga cccagtcgcc ttcctccctc tccgcttccg tgggggaccg ggtcactatt     480

acgtgtcagg cgtcccaatt catcaagaag aatctgaact ggtaccagca caagccggga     540

aaggccccca aactgctcat ctacgacgcc agctcgctgc agactggcgt gccttcccgg     600

ttttccggga accggtcggg aaccaccttc tcattcacca tcagcagcct ccagccggag     660

gacgtggcga cctactactg ccagcagcat gacaaccttc cactgacttt cggcgggggc     720

accaaggtcg agattaag                                                   738


<210> 63
<211> 765
<212> DNA
<213> Artificial Sequence

<220>
<221> source
<223> /note="Description of Artificial Sequence: Synthetic
      polynucleotide"

<400> 63
caagtgcaac tcgtggaatc aggcggagga ctcgtgcaac ccggaggttc ccttagactg      60

tcatgtgccg cttccgggtt caatgtgtcc agcaactaca tgacctgggt cagacaggcg     120

ccgggaaagg gacttgaatg ggtgtccgtg atctactccg gtggagcaac atactacgga     180

gactccgtga aaggccgctt taccgtgtcc cgcgataact cgaagaacac cgtgtacttg     240

cagatgaaca ggctgactgc cgaggacacc gccgtgtatt attgcgcccg ggacaggctg     300

tactgtggaa acaactgcta cctgtactac tactacggga tggacgtgtg gggacagggc     360

actctcgtca ctgtgtcatc cggggggggc ggtagcggtg gcggagggtc cggcggagga     420

ggctcagggg gaggcggaag cgatatccag gtcacccagt ctccctcctc gctgtccgcc     480

tccgtgggcg accgcgtcac cattacttgc cgggcgtcgc agtcgatcag ctcctacctg 540

aactggtacc agcagaagcc tggaaaggcc ccgaagctgc tgatctacgc ggcctcgtcc 600

ctgcaaagcg gcgtcccgtc gcggttcagc ggttccggtt cgggaaccga cttcaccctg 660

actatttcct ccctgcaacc cgaggatttc gccacttact actgccagca gtcctactcc 720

accccacctc tgaccttcgg ccaaggaacc aaggtcgaaa tcaag 765

<210> 64
<211> 741
<212> DNA
<213> Artificial Sequence

<220>
<221> source
<223> /note="Description of Artificial Sequence: Synthetic
      polynucleotide"

<400> 64
caagtgcaac tcgtccagtc cggtgcagaa gtgaaaaaga gcggagcctc agtgaaagtg 60

tcctgcaagg cctccggtta ccccttcact ggatactaca ttcagtgggt ccgccaagcc 120

ccgggacagg gtctggagtg gatggggtgg attgacccta actcgggaaa tacgggatac 180

gcgcagaagt tccagggccg cgtgaccatg accaggaaca cctcgatcag caccgcctac 240

atggaactgt cctccctgcg gtcggaggat actgccgtgt actactgcgc ctccgattcc 300

tatgggtact actacggaat ggacgtctgg ggacagggca ccctcgtgac cgtgtcctcg 360

ggaggcggag ggagcggcgg gggtggatcg ggaggaggcg gctccggcgg cggcggtagc 420

gacatccaga tgacccagtc accatcaagc cttagcgcct ccgtgggcga cagagtgaca 480

ttcacttgtc gggcgtccca gggaatctcc tccgctctgg cttggtatca gcagaagcct 540

gggaagcctc cgaagctgtt gatctacgac gcgagcagcc tggaatcagg ggtgccctcc 600

cggttttccg ggtccggttc tggcaccgat ttcaccctga ccatttcgtc cctccaaccc 660

gaggacttcg ccacttacta ctgccagcag ttcaacaact acccgctgac cttcggagga 720

ggcactaagg tcgagatcaa g 741

<210> 65
<211> 120
<212> PRT
<213> Artificial Sequence

<220>
<221> source
<223> /note="Description of Artificial Sequence: Synthetic
      polypeptide"

<400> 65
Glu Val Gln Leu Gln Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Leu Glu Met Ala Thr Ile Met Gly Gly Tyr Trp Gly Gln
            100                 105                 110

Gly Thr Leu Val Thr Val Ser Ser
        115                 120

<210> 66
<211> 118
<212> PRT
<213> Artificial Sequence

<220>
<221> source
<223> /note="Description of Artificial Sequence: Synthetic
      polypeptide"

<400> 66
Glu Val Gln Leu Val Glu Ser Gly Gly Gly Val Val Gln Pro Gly Gly
1               5                   10                  15

Ser Leu Arg Leu Ser Cys Ala Ala Ser Gly Phe Thr Phe Asp Asp Tyr
            20                  25                  30

Ala Met His Trp Val Arg Gln Ala Pro Gly Lys Gly Leu Glu Trp Val
        35                  40                  45

Ser Leu Ile Ser Gly Asp Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Val Glu Asp Thr Ala Val Tyr Tyr Cys
                85                  90                  95

Ala Arg Val Phe Asp Ser Tyr Tyr Met Asp Val Trp Gly Lys Gly Thr
            100                 105                 110

Thr Val Thr Val Ser Ser
        115

<210> 67
<211> 125
<212> PRT
<213> Artificial Sequence

<220>
<221> source
<223> /note="Description of Artificial Sequence: Synthetic
      polypeptide"

<400> 67
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Ser
            20                  25                  30

Ser Tyr Tyr Trp Gly Trp Ile Arg Gln Pro Pro Gly Lys Gly Leu Glu
        35                  40                  45

Trp Ile Gly Ser Ile Tyr Tyr Ser Gly Ser Thr Tyr Tyr Asn Pro Ser
    50                  55                  60

Leu Lys Ser Arg Val Ser Ile Ser Val Asp Thr Ser Lys Asn Gln Phe
65                  70                  75                  80

Ser Leu Lys Leu Lys Tyr Val Thr Ala Ala Asp Thr Ala Val Tyr Tyr
                85                  90                  95

Cys Ala Thr Pro Gly Thr Tyr Tyr Asp Phe Leu Ser Gly Tyr Tyr Pro
            100                 105                 110

Phe Tyr Trp Gly Gln Gly Thr Leu Val Thr Val Ser Ser
        115                 120                 125

<210> 68
<211> 116
<212> PRT
<213> Artificial Sequence

<220>
<221> source
<223> /note="Description of Artificial Sequence: Synthetic
      polypeptide"

<400> 68
Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Asn Glu Asp Gly Ser Ala Lys Phe Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Phe Cys
                85                  90                  95

Ala Arg Asp Leu Arg Ser Gly Arg Tyr Trp Gly Gln Gly Thr Leu Val
            100                 105                 110

Thr Val Ser Ser
        115

<210> 69
<211> 123
<212> PRT
<213> Artificial Sequence

<220>
<221> source
<223> /note="Description of Artificial Sequence: Synthetic
      polypeptide"

<400> 69
Glu Val Gln Leu Gln Gln Ser Gly Pro Gly Leu Val Arg Pro Ser Glu
1               5                   10                  15

Thr Leu Ser Leu Thr Cys Thr Val Ser Gly Gly Pro Val Arg Ser Gly
            20                  25                  30

Ser His Tyr Trp Asn Trp Ile Arg Gln Pro Pro Gly Arg Gly Leu Glu
        35                  40                  45

Trp Ile Gly Tyr Ile Tyr Tyr Ser Gly Ser Thr Asn Tyr Asn Pro Ser
    50                  55                  60

Leu Glu Asn Arg Val Thr Ile Ser Ile Asp Thr Ser Asn Asn His Phe
65                  70                  75                  80

Ser Leu Lys Leu Ser Ser Val Thr Ala Ala Asp Thr Ala Leu Tyr Phe
                85                  90                  95

Cys Ala Arg Gly Thr Ala Thr Phe Asp Trp Asn Phe Pro Phe Asp Ser
            100                 105                 110

Trp Gly Gln Gly Thr Leu Val Thr Val Ser Ser
        115                 120

<210> 70
<211> 126
<212> PRT
<213> Artificial Sequence

<220>
<221> source
<223> /note="Description of Artificial Sequence: Synthetic
      polypeptide"

<400> 70
Gln Val Gln Leu Gln Glu Ser Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Val
        35                  40                  45

Gly Glu Ile Asn His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Gly Ser Gly Leu Val Val Tyr Ala Ile Arg Val Gly Ser Gly Trp
            100                 105                 110

Phe Asp Tyr Trp Gly Gln Gly Thr Leu Val Thr Val Ser Ser
        115                 120                 125

<210> 71
<211> 129
<212> PRT
<213> Artificial Sequence

<220>
<221> source

<223> /note="Description of Artificial Sequence: Synthetic
      polypeptide"

<400> 71
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Pro Ser Ser Ser Gly Ser Tyr Tyr Met Glu Asp Ser Tyr
            100                 105                 110

Tyr Tyr Gly Met Asp Val Trp Gly Gln Gly Thr Thr Val Thr Val Ser
        115                 120                 125

Ser

<210> 72
<211> 117
<212> PRT
<213> Artificial Sequence

<220>
<221> source
<223> /note="Description of Artificial Sequence: Synthetic
      polypeptide"

<400> 72
Gln Val Asn Leu Arg Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Glu Met Asn Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Ala Leu Gly Ser Ser Trp Glu Trp Gly Gln Gly Thr Thr
            100                 105                 110

Val Thr Val Ser Ser
        115

<210> 73
<211> 119
<212> PRT
<213> Artificial Sequence

<220>
<221> source
<223> /note="Description of Artificial Sequence: Synthetic
      polypeptide"

<400> 73
Gln Val Gln Leu Val Gln Ser Gly Ala Glu Val Lys Glu Pro Gly Ala
1               5                   10                  15

Ser Val Lys Val Ser Cys Lys Ala Pro Ala Asn Thr Phe Ser Asp His
            20                  25                  30

Val Met His Trp Val Arg Gln Ala Pro Gly Gln Arg Phe Glu Trp Met
        35                  40                  45

Gly Tyr Ile His Ala Ala Asn Gly Gly Thr His Tyr Ser Gln Lys Phe
    50                  55                  60

Gln Asp Arg Val Thr Ile Thr Arg Asp Thr Ser Ala Asn Thr Val Tyr
65                  70                  75                  80

Met Asp Leu Ser Ser Leu Arg Ser Glu Asp Thr Ala Val Tyr Tyr Cys
                85                  90                  95

Ala Arg Gly Gly Tyr Asn Ser Asp Ala Phe Asp Ile Trp Gly Gln Gly
            100                 105                 110

Thr Met Val Thr Val Ser Ser
        115

<210> 74
<211> 121
<212> PRT
<213> Artificial Sequence

<220>
<221> source
<223> /note="Description of Artificial Sequence: Synthetic
      polypeptide"

<400> 74
Gln Val Gln Leu Val Gln Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Tyr Ile Ser Ser Ser Ser Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Ser Val Arg Ala Ile Asp Ala Phe Asp Ile Trp Gly
            100                 105                 110

Gln Gly Thr Met Val Thr Val Ser Ser
        115                 120


<210> 75
<211> 119
<212> PRT
<213> Artificial Sequence

<220>
<221> source
<223> /note="Description of Artificial Sequence: Synthetic
      polypeptide"

<400> 75
Glu Val Gln Leu Val Gln Ser Gly Gly Gly Val Val Arg Ser Gly Arg
1               5                   10                  15

Ser Leu Arg Leu Ser Cys Ala Ala Ser Gly Phe Thr Phe Asn Ser Tyr
            20                  25                  30

Gly Leu His Trp Val Arg Gln Ala Pro Gly Lys Gly Leu Glu Trp Val

35                  40                  45

Ala Leu Ile Glu Tyr Asp Gly Ser Asn Lys Tyr Tyr Gly Asp Ser Val
    50                  55                  60

Lys Gly Arg Phe Thr Ile Ser Arg Asp Lys Ser Lys Ser Thr Leu Tyr
65                  70                  75                  80

Leu Gln Met Asp Asn Leu Arg Ala Glu Asp Thr Ala Val Tyr Tyr Cys
                85                  90                  95

Ala Arg Glu Gly Asn Glu Asp Leu Ala Phe Asp Ile Trp Gly Gln Gly
            100                 105                 110

Thr Leu Val Thr Val Ser Ser
        115

<210> 76
<211> 127
<212> PRT
<213> Artificial Sequence

<220>
<221> source
<223> /note="Description of Artificial Sequence: Synthetic
      polypeptide"

<400> 76
Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Val Ser Ser Asn
            20                  25                  30

Tyr Met Thr Trp Val Arg Gln Ala Pro Gly Lys Gly Leu Glu Trp Val
        35                  40                  45

Ser Val Ile Tyr Ser Gly Gly Ala Thr Tyr Tyr Gly Asp Ser Val Lys
    50                  55                  60

Gly Arg Phe Thr Val Ser Arg Asp Asn Ser Lys Asn Thr Val Tyr Leu
65                  70                  75                  80

Gln Met Asn Arg Leu Thr Ala Glu Asp Thr Ala Val Tyr Tyr Cys Ala
                85                  90                  95

Arg Asp Arg Leu Tyr Cys Gly Asn Asn Cys Tyr Leu Tyr Tyr Tyr Tyr
            100                 105                 110

Gly Met Asp Val Trp Gly Gln Gly Thr Leu Val Thr Val Ser Ser

115                 120                 125

<210> 77
<211> 120
<212> PRT
<213> Artificial Sequence

<220>
<221> source
<223> /note="Description of Artificial Sequence: Synthetic
      polypeptide"

<400> 77
Gln Val Gln Leu Val Gln Ser Gly Ala Glu Val Lys Lys Ser Gly Ala
1               5                   10                  15

Ser Val Lys Val Ser Cys Lys Ala Ser Gly Tyr Pro Phe Thr Gly Tyr
            20                  25                  30

Tyr Ile Gln Trp Val Arg Gln Ala Pro Gly Gln Gly Leu Glu Trp Met
        35                  40                  45

Gly Trp Ile Asp Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Ser Asp Ser Tyr Gly Tyr Tyr Tyr Gly Met Asp Val Trp Gly Gln
            100                 105                 110

Gly Thr Leu Val Thr Val Ser Ser
        115                 120


<210> 78
<211> 112
<212> PRT
<213> Artificial Sequence

<220>
<221> source
<223> /note="Description of Artificial Sequence: Synthetic
      polypeptide"

<400> 78
Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Leu Asp Val Val Phe Gly Gly Gly Thr Lys Leu Thr Val Leu
            100                 105                 110

<210> 79
<211> 112
<212> PRT
<213> Artificial Sequence

<220>
<221> source
<223> /note="Description of Artificial Sequence: Synthetic
      polypeptide"

<400> 79
Glu Ile Val Leu Thr Gln Ser Pro Leu Ser Leu Pro Val Thr Pro Gly
1               5                   10                  15

Gln Pro Ala Ser Ile Ser Cys Arg Ser Ser Gln Ser Leu Val Tyr Thr
            20                  25                  30

Asp Gly Asn Thr Tyr Leu Asn Trp Phe Gln Gln Arg Pro Gly Gln Ser
        35                  40                  45

Pro Arg Arg Leu Ile Tyr Lys Val Ser Asn Arg Asp Ser Gly Val Pro
    50                  55                  60

Asp Arg Phe Ser Gly Ser Gly Ser Asp Thr Asp Phe Thr Leu Lys Ile
65                  70                  75                  80

Ser Arg Val Glu Ala Glu Asp Val Gly Ile Tyr Tyr Cys Met Gln Gly
                85                  90                  95

Thr His Trp Ser Phe Thr Phe Gly Gln Gly Thr Arg Leu Glu Ile Lys
            100                 105                 110

<210> 80
<211> 107

<212> PRT
<213> Artificial Sequence

<220>
<221> source
<223> /note="Description of Artificial Sequence: Synthetic
      polypeptide"

<400> 80
Asp Ile Val Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Tyr
            20                  25                  30

Leu Ala Trp Tyr Gln Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Leu Asn Ser Tyr Pro Tyr
                85                  90                  95

Thr Phe Gly Gln Gly Thr Lys Leu Glu Ile Lys
            100                 105


<210> 81
<211> 108
<212> PRT
<213> Artificial Sequence

<220>
<221> source
<223> /note="Description of Artificial Sequence: Synthetic
      polypeptide"

<400> 81
Glu Ile Val Leu Thr Gln Ser Pro Gly Thr Leu Ser Leu Ser Pro Gly
1               5                   10                  15

Gly Arg Ala Thr Leu Ser Cys Arg Ala Ser Gln Ser Ile Ser Gly Ser
            20                  25                  30

Phe Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Pro Thr Phe Gly Leu Gly Thr Lys Leu Glu Ile Lys
            100                 105

<210> 82
<211> 107
<212> PRT
<213> Artificial Sequence

<220>
<221> source
<223> /note="Description of Artificial Sequence: Synthetic
      polypeptide"

<400> 82
Asp Ile Gln Met Thr Gln Ser Pro Ser Ser Leu Ser Ala Ser Ile Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Trp
                85                  90                  95

Thr Phe Gly Gln Gly Thr Lys Leu Glu Ile Lys
            100                 105

<210> 83
<211> 108
<212> PRT
<213> Artificial Sequence

<220>
<221> source
<223> /note="Description of Artificial Sequence: Synthetic
      polypeptide"

<400> 83

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Met
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Pro
                85                  90                  95

Trp Thr Phe Gly Gln Gly Thr Lys Val Asp Ile Lys
            100                 105

<210> 84
<211> 111
<212> PRT
<213> Artificial Sequence

<220>
<221> source
<223> /note="Description of Artificial Sequence: Synthetic
      polypeptide"

<400> 84
Asn Phe Met Leu Thr Gln Pro His Ser Val Ser Glu Ser Pro Gly Lys
1               5                   10                  15

Thr Val Thr Ile Ser Cys Thr Gly Ser Ser Gly Ser Ile Ala Ser Asn
            20                  25                  30

Tyr Val Gln Trp Tyr Gln Gln Arg Pro Gly Ser Ala Pro Thr Thr Val
        35                  40                  45

Ile Tyr Glu Asp Asn Gln Arg Pro Ser Gly Val Pro Asp Arg Phe Ser
    50                  55                  60

Gly Ser Ile Asp Ser Ser Ser Asn Ser Ala Ser Leu Thr Ile Ser Gly
65                  70                  75                  80

Leu Lys Thr Glu Asp Glu Ala Asp Tyr Tyr Cys Gln Ser Tyr Asp Ser
                85                  90                  95

Ser Asn Gln Val Val Phe Gly Gly Gly Thr Lys Leu Thr Val Leu
            100                 105                 110

<210> 85
<211> 107
<212> PRT
<213> Artificial Sequence

<220>
<221> source
<223> /note="Description of Artificial Sequence: Synthetic
      polypeptide"

<400> 85
Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Asp Asn Leu Pro Leu
                85                  90                  95

Thr Phe Gly Gly Gly Thr Lys Leu Glu Ile Lys
            100                 105

<210> 86
<211> 107
<212> PRT
<213> Artificial Sequence

<220>
<221> source
<223> /note="Description of Artificial Sequence: Synthetic
      polypeptide"

<400> 86
Asp Ile Val Met Thr Gln Ser Pro Ser Ser Val Ser Ala Ser Val Gly
1               5                   10                  15

Asp Arg Val Thr Ile Thr Cys Arg Ala Ser Gln Asp Ile Ser Ser Trp
            20                  25                  30

Leu Ala Trp Tyr Gln Gln Lys Pro Gly Lys Ala Pro Lys Leu Leu Ile

35                  40                  45

Tyr Ala Ala Ser Ser Leu Gln Ser Gly Val Pro Ser Arg Phe Asn Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Leu
                85                  90                  95

Thr Phe Gly Gly Gly Thr Lys Val Glu Ile Lys
            100                 105

<210> 87
<211> 107
<212> PRT
<213> Artificial Sequence

<220>
<221> source
<223> /note="Description of Artificial Sequence: Synthetic
      polypeptide"

<400> 87
Asp Ile Val Leu Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Phe Ala Thr Tyr Tyr Cys Gln Gln Ala Tyr Ser Thr Pro Phe
                85                  90                  95

Thr Phe Gly Pro Gly Thr Lys Val Glu Ile Lys
            100                 105

<210> 88
<211> 107
<212> PRT
<213> Artificial Sequence

<220>
<221> source
<223> /note="Description of Artificial Sequence: Synthetic
      polypeptide"

<400> 88
Glu Ile Val Leu Thr Gln Ser Pro Ser Ser Leu Ser Ala Ser Val Gly
1               5                   10                  15

Asp Arg Val Thr Ile Thr Cys Gln Ala Ser Gln Phe Ile Lys Lys Asn
            20                  25                  30

Leu Asn Trp Tyr Gln His Lys Pro Gly Lys Ala Pro Lys Leu Leu Ile
        35                  40                  45

Tyr Asp Ala Ser Ser Leu Gln Thr Gly Val Pro Ser Arg Phe Ser Gly
    50                  55                  60

Asn Arg Ser Gly Thr Thr Phe Ser Phe Thr Ile Ser Ser Leu Gln Pro
65                  70                  75                  80

Glu Asp Val Ala Thr Tyr Tyr Cys Gln Gln His Asp Asn Leu Pro Leu
                85                  90                  95

Thr Phe Gly Gly Gly Thr Lys Val Glu Ile Lys
            100                 105


<210> 89
<211> 108
<212> PRT
<213> Artificial Sequence

<220>
<221> source
<223> /note="Description of Artificial Sequence: Synthetic
      polypeptide"

<400> 89
Asp Ile Gln Val Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Pro
                85                  90                  95

Leu Thr Phe Gly Gln Gly Thr Lys Val Glu Ile Lys
            100                 105

<210> 90
<211> 107
<212> PRT
<213> Artificial Sequence

<220>
<221> source
<223> /note="Description of Artificial Sequence: Synthetic
      polypeptide"

<400> 90
Asp Ile Gln Met Thr Gln Ser Pro Ser Ser Leu Ser Ala Ser Val Gly
1               5                   10                  15

Asp Arg Val Thr Phe Thr Cys Arg Ala Ser Gln Gly Ile Ser Ser Ala
            20                  25                  30

Leu Ala Trp Tyr Gln Gln Lys Pro Gly Lys Pro Pro Lys Leu Leu Ile
        35                  40                  45

Tyr Asp Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Phe Asn Asn Tyr Pro Leu
                85                  90                  95

Thr Phe Gly Gly Gly Thr Lys Val Glu Ile Lys
            100                 105

<210> 91

<400> 91
000

<210> 92

<400> 92
000

<210> 93

<400> 93
000

<210> 94

<400> 94
000

<210> 95

<400> 95
000

<210> 96

<400> 96
000

<210> 97

<400> 97
000

<210> 98

<400> 98
000

<210> 99

<400> 99
000

<210> 100

<400> 100
000

<210> 101

<400> 101
000

<210> 102

<400> 102
000

<210> 103

<400> 103
000

<210> 104

<400> 104
000

<210> 105

<400> 105
000

<210> 106

<400> 106
000

<210> 107

<400> 107
000

<210> 108

<400> 108
000

<210> 109

<400> 109
000

<210> 110

<400> 110
000

<210> 111

<400> 111
000

<210> 112

<400> 112
000

<210> 113

<400> 113
000

<210> 114

<400> 114
000

<210> 115

<400> 115
000

<210> 116

<400> 116
000

<210> 117
<211> 10
<212> PRT
<213> Artificial Sequence

<220>
<221> source
<223> /note="Description of Artificial Sequence: Synthetic
      peptide"

<400> 117
Gly Gly Thr Phe Ser Ser Tyr Ala Ile Ser
1               5                   10

<210> 118
<211> 10
<212> PRT
<213> Artificial Sequence

<220>
<221> source
<223> /note="Description of Artificial Sequence: Synthetic
      peptide"

<400> 118
Gly Phe Thr Phe Asp Asp Tyr Ala Met His
1               5                   10

<210> 119
<211> 12
<212> PRT
<213> Artificial Sequence

<220>
<221> source
<223> /note="Description of Artificial Sequence: Synthetic
      peptide"

<400> 119
Gly Gly Ser Ile Ser Ser Ser Ser Tyr Tyr Trp Gly
1               5                   10

<210> 120
<211> 10

<212> PRT
<213> Artificial Sequence

<220>
<221> source
<223> /note="Description of Artificial Sequence: Synthetic
      peptide"

<400> 120
Gly Phe Thr Phe Ser Ser Tyr Trp Met Ser
1               5                   10


<210> 121
<211> 12
<212> PRT
<213> Artificial Sequence

<220>
<221> source
<223> /note="Description of Artificial Sequence: Synthetic
      peptide"

<400> 121
Gly Gly Pro Val Arg Ser Gly Ser His Tyr Trp Asn
1               5                   10


<210> 122
<211> 10
<212> PRT
<213> Artificial Sequence

<220>
<221> source
<223> /note="Description of Artificial Sequence: Synthetic
      peptide"

<400> 122
Gly Gly Ser Phe Ser Gly Tyr Tyr Trp Ser
1               5                   10


<210> 123
<211> 10
<212> PRT
<213> Artificial Sequence

<220>
<221> source
<223> /note="Description of Artificial Sequence: Synthetic
      peptide"

<400> 123
Gly Phe Thr Phe Ser Ser Tyr Ser Met Asn
1               5                   10


<210> 124
<211> 10
<212> PRT
<213> Artificial Sequence

<220>
<221> source
<223> /note="Description of Artificial Sequence: Synthetic
      peptide"

<400> 124
Gly Phe Thr Phe Ser Ser Tyr Glu Met Asn
1               5                   10


<210> 125
<211> 10
<212> PRT
<213> Artificial Sequence

<220>
<221> source
<223> /note="Description of Artificial Sequence: Synthetic
      peptide"

<400> 125
Ala Asn Thr Phe Ser Asp His Val Met His
1               5                   10


<210> 126
<211> 10
<212> PRT
<213> Artificial Sequence

<220>
<221> source
<223> /note="Description of Artificial Sequence: Synthetic
      peptide"

<400> 126
Gly Phe Thr Phe Ser Ser Tyr Ser Met Asn
1               5                   10


<210> 127
<211> 10
<212> PRT
<213> Artificial Sequence

<220>
<221> source
<223> /note="Description of Artificial Sequence: Synthetic
      peptide"

<400> 127
Gly Phe Thr Phe Asn Ser Tyr Gly Leu His
1               5                   10


<210> 128
<211> 10
<212> PRT
<213> Artificial Sequence

<220>
<221> source
<223> /note="Description of Artificial Sequence: Synthetic

peptide"

<400> 128
Gly Phe Asn Val Ser Ser Asn Tyr Met Thr
1               5                   10


<210> 129
<211> 10
<212> PRT
<213> Artificial Sequence

<220>
<221> source
<223> /note="Description of Artificial Sequence: Synthetic
      peptide"

<400> 129
Gly Tyr Pro Phe Thr Gly Tyr Tyr Ile Gln
1               5                   10


<210> 130
<211> 16
<212> PRT
<213> Artificial Sequence

<220>
<221> source
<223> /note="Description of Artificial Sequence: Synthetic
      peptide"

<400> 130
Gly Ile Ile Pro Ile Phe Gly Thr Ala Asn Tyr Ala Gln Lys Phe Gln
1               5                   10                  15


<210> 131
<211> 17
<212> PRT
<213> Artificial Sequence

<220>
<221> source
<223> /note="Description of Artificial Sequence: Synthetic
      peptide"

<400> 131
Leu Ile Ser Gly Asp Gly Gly Ser Thr Tyr Tyr Ala Asp Ser Val Lys
1               5                   10                  15


Gly



<210> 132
<211> 16
<212> PRT
<213> Artificial Sequence

<220>
<221> source

<223> /note="Description of Artificial Sequence: Synthetic
      peptide"

<400> 132
Ser Ile Tyr Tyr Ser Gly Ser Thr Tyr Tyr Asn Pro Ser Leu Lys Ser
1               5                   10                  15


<210> 133
<211> 17
<212> PRT
<213> Artificial Sequence

<220>
<221> source
<223> /note="Description of Artificial Sequence: Synthetic
      peptide"

<400> 133
Asn Ile Asn Glu Asp Gly Ser Ala Lys Phe Tyr Val Asp Ser Val Lys
1               5                   10                  15


Gly




<210> 134
<211> 16
<212> PRT
<213> Artificial Sequence

<220>
<221> source
<223> /note="Description of Artificial Sequence: Synthetic
      peptide"

<400> 134
Tyr Ile Tyr Tyr Ser Gly Ser Thr Asn Tyr Asn Pro Ser Leu Glu Asn
1               5                   10                  15


<210> 135
<211> 16
<212> PRT
<213> Artificial Sequence

<220>
<221> source
<223> /note="Description of Artificial Sequence: Synthetic
      peptide"

<400> 135
Glu Ile Asn His Ser Gly Ser Thr Asn Tyr Asn Pro Ser Leu Lys Ser
1               5                   10                  15


<210> 136
<211> 17
<212> PRT
<213> Artificial Sequence

<220>

<221> source
<223> /note="Description of Artificial Sequence: Synthetic
      peptide"

<400> 136
Ser Ile Ser Ser Ser Ser Ser Tyr Ile Tyr Tyr Ala Asp Ser Val Lys
1               5                   10                  15

Gly

<210> 137
<211> 17
<212> PRT
<213> Artificial Sequence

<220>
<221> source
<223> /note="Description of Artificial Sequence: Synthetic
      peptide"

<400> 137
Tyr Ile Ser Ser Ser Gly Ser Thr Ile Tyr Tyr Ala Asp Ser Val Lys
1               5                   10                  15

Gly

<210> 138
<211> 17
<212> PRT
<213> Artificial Sequence

<220>
<221> source
<223> /note="Description of Artificial Sequence: Synthetic
      peptide"

<400> 138
Tyr Ile His Ala Ala Asn Gly Gly Thr His Tyr Ser Gln Lys Phe Gln
1               5                   10                  15

Asp

<210> 139
<211> 17
<212> PRT
<213> Artificial Sequence

<220>
<221> source
<223> /note="Description of Artificial Sequence: Synthetic
      peptide"

<400> 139
Tyr Ile Ser Ser Ser Ser Ser Thr Ile Tyr Tyr Ala Asp Ser Val Lys

1               5                   10                  15

Gly

<210> 140
<211> 17
<212> PRT
<213> Artificial Sequence

<220>
<221> source
<223> /note="Description of Artificial Sequence: Synthetic
      peptide"

<400> 140
Leu Ile Glu Tyr Asp Gly Ser Asn Lys Tyr Tyr Gly Asp Ser Val Lys
1               5                   10                  15

Gly

<210> 141
<211> 16
<212> PRT
<213> Artificial Sequence

<220>
<221> source
<223> /note="Description of Artificial Sequence: Synthetic
      peptide"

<400> 141
Val Ile Tyr Ser Gly Gly Ala Thr Tyr Tyr Gly Asp Ser Val Lys Gly
1               5                   10                  15

<210> 142
<211> 17
<212> PRT
<213> Artificial Sequence

<220>
<221> source
<223> /note="Description of Artificial Sequence: Synthetic
      peptide"

<400> 142
Trp Ile Asp Pro Asn Ser Gly Asn Thr Gly Tyr Ala Gln Lys Phe Gln
1               5                   10                  15

Gly

<210> 143
<211> 11
<212> PRT

<213> Artificial Sequence

<220>
<221> source
<223> /note="Description of Artificial Sequence: Synthetic
      peptide"

<400> 143
Asp Leu Glu Met Ala Thr Ile Met Gly Gly Tyr
1               5                   10


<210> 144
<211> 9
<212> PRT
<213> Artificial Sequence

<220>
<221> source
<223> /note="Description of Artificial Sequence: Synthetic
      peptide"

<400> 144
Val Phe Asp Ser Tyr Tyr Met Asp Val
1               5


<210> 145
<211> 15
<212> PRT
<213> Artificial Sequence

<220>
<221> source
<223> /note="Description of Artificial Sequence: Synthetic
      peptide"

<400> 145
Pro Gly Thr Tyr Tyr Asp Phe Leu Ser Gly Tyr Tyr Pro Phe Tyr
1               5                   10                  15


<210> 146
<211> 7
<212> PRT
<213> Artificial Sequence

<220>
<221> source
<223> /note="Description of Artificial Sequence: Synthetic
      peptide"

<400> 146
Asp Leu Arg Ser Gly Arg Tyr
1               5


<210> 147
<211> 13
<212> PRT
<213> Artificial Sequence

<220>

<221> source
<223> /note="Description of Artificial Sequence: Synthetic
      peptide"

<400> 147
Gly Thr Ala Thr Phe Asp Trp Asn Phe Pro Phe Asp Ser
1               5                   10


<210> 148
<211> 18
<212> PRT
<213> Artificial Sequence

<220>
<221> source
<223> /note="Description of Artificial Sequence: Synthetic
      peptide"

<400> 148
Gly Ser Gly Leu Val Val Tyr Ala Ile Arg Val Gly Ser Gly Trp Phe
1               5                   10                  15


Asp Tyr



<210> 149
<211> 20
<212> PRT
<213> Artificial Sequence

<220>
<221> source
<223> /note="Description of Artificial Sequence: Synthetic
      peptide"

<400> 149
Asp Pro Ser Ser Ser Gly Ser Tyr Tyr Met Glu Asp Ser Tyr Tyr Tyr
1               5                   10                  15


Gly Met Asp Val
            20


<210> 150
<211> 8
<212> PRT
<213> Artificial Sequence

<220>
<221> source
<223> /note="Description of Artificial Sequence: Synthetic
      peptide"

<400> 150
Glu Ala Leu Gly Ser Ser Trp Glu
1               5


<210> 151

<211> 10
<212> PRT
<213> Artificial Sequence

<220>
<221> source
<223> /note="Description of Artificial Sequence: Synthetic
      peptide"

<400> 151
Gly Gly Tyr Asn Ser Asp Ala Phe Asp Ile
1               5                   10


<210> 152
<211> 12
<212> PRT
<213> Artificial Sequence

<220>
<221> source
<223> /note="Description of Artificial Sequence: Synthetic
      peptide"

<400> 152
Asp Leu Ser Val Arg Ala Ile Asp Ala Phe Asp Ile
1               5                   10


<210> 153
<211> 10
<212> PRT
<213> Artificial Sequence

<220>
<221> source
<223> /note="Description of Artificial Sequence: Synthetic
      peptide"

<400> 153
Glu Gly Asn Glu Asp Leu Ala Phe Asp Ile
1               5                   10


<210> 154
<211> 19
<212> PRT
<213> Artificial Sequence

<220>
<221> source
<223> /note="Description of Artificial Sequence: Synthetic
      peptide"

<400> 154
Asp Arg Leu Tyr Cys Gly Asn Asn Cys Tyr Leu Tyr Tyr Tyr Tyr Gly
1               5                   10                  15


Met Asp Val

<210> 155
<211> 11
<212> PRT
<213> Artificial Sequence

<220>
<221> source
<223> /note="Description of Artificial Sequence: Synthetic
      peptide"

<400> 155
Asp Ser Tyr Gly Tyr Tyr Tyr Gly Met Asp Val
1               5                   10


<210> 156
<211> 14
<212> PRT
<213> Artificial Sequence

<220>
<221> source
<223> /note="Description of Artificial Sequence: Synthetic
      peptide"

<400> 156
Thr Gly Thr Ser Ser Asp Val Gly Gly Tyr Asn Tyr Val Ser
1               5                   10


<210> 157
<211> 16
<212> PRT
<213> Artificial Sequence

<220>
<221> source
<223> /note="Description of Artificial Sequence: Synthetic
      peptide"

<400> 157
Arg Ser Ser Gln Ser Leu Val Tyr Thr Asp Gly Asn Thr Tyr Leu Asn
1               5                   10                  15


<210> 158
<211> 11
<212> PRT
<213> Artificial Sequence

<220>
<221> source
<223> /note="Description of Artificial Sequence: Synthetic
      peptide"

<400> 158
Arg Ala Ser Gln Gly Ile Ser Ser Tyr Leu Ala
1               5                   10


<210> 159
<211> 12
<212> PRT

<213> Artificial Sequence

<220>
<221> source
<223> /note="Description of Artificial Sequence: Synthetic
      peptide"

<400> 159
Arg Ala Ser Gln Ser Ile Ser Gly Ser Phe Leu Ala
1               5                   10


<210> 160
<211> 11
<212> PRT
<213> Artificial Sequence

<220>
<221> source
<223> /note="Description of Artificial Sequence: Synthetic
      peptide"

<400> 160
Arg Ala Ser Gln Ser Ile Ser Ser Tyr Leu Asn
1               5                   10


<210> 161
<211> 11
<212> PRT
<213> Artificial Sequence

<220>
<221> source
<223> /note="Description of Artificial Sequence: Synthetic
      peptide"

<400> 161
Arg Ala Ser Gln Ser Ile Ser Ser Tyr Leu Asn
1               5                   10


<210> 162
<211> 13
<212> PRT
<213> Artificial Sequence

<220>
<221> source
<223> /note="Description of Artificial Sequence: Synthetic
      peptide"

<400> 162
Thr Gly Ser Ser Gly Ser Ile Ala Ser Asn Tyr Val Gln
1               5                   10


<210> 163
<211> 11
<212> PRT
<213> Artificial Sequence

<220>

<221> source
<223> /note="Description of Artificial Sequence: Synthetic
      peptide"

<400> 163
Gln Ala Ser Gln Asp Ile Ser Asn Tyr Leu Asn
1               5                   10


<210> 164
<211> 11
<212> PRT
<213> Artificial Sequence

<220>
<221> source
<223> /note="Description of Artificial Sequence: Synthetic
      peptide"

<400> 164
Arg Ala Ser Gln Asp Ile Ser Ser Trp Leu Ala
1               5                   10


<210> 165
<211> 11
<212> PRT
<213> Artificial Sequence

<220>
<221> source
<223> /note="Description of Artificial Sequence: Synthetic
      peptide"

<400> 165
Gln Ala Ser Gln Asp Ile Ser Asn Tyr Leu Asn
1               5                   10


<210> 166
<211> 11
<212> PRT
<213> Artificial Sequence

<220>
<221> source
<223> /note="Description of Artificial Sequence: Synthetic
      peptide"

<400> 166
Gln Ala Ser Gln Phe Ile Lys Lys Asn Leu Asn
1               5                   10


<210> 167
<211> 11
<212> PRT
<213> Artificial Sequence

<220>
<221> source
<223> /note="Description of Artificial Sequence: Synthetic
      peptide"

<400> 167
Arg Ala Ser Gln Ser Ile Ser Ser Tyr Leu Asn
1               5                   10

<210> 168
<211> 11
<212> PRT
<213> Artificial Sequence

<220>
<221> source
<223> /note="Description of Artificial Sequence: Synthetic
      peptide"

<400> 168
Arg Ala Ser Gln Gly Ile Ser Ser Ala Leu Ala
1               5                   10

<210> 169
<211> 7
<212> PRT
<213> Artificial Sequence

<220>
<221> source
<223> /note="Description of Artificial Sequence: Synthetic
      peptide"

<400> 169
Asp Val Ser Asn Arg Pro Ser
1               5

<210> 170
<211> 7
<212> PRT
<213> Artificial Sequence

<220>
<221> source
<223> /note="Description of Artificial Sequence: Synthetic
      peptide"

<400> 170
Lys Val Ser Asn Arg Asp Ser
1               5

<210> 171
<211> 7
<212> PRT
<213> Artificial Sequence

<220>
<221> source
<223> /note="Description of Artificial Sequence: Synthetic
      peptide"

<400> 171
Ala Ala Ser Thr Leu Gln Ser

1               5


<210> 172
<211> 7
<212> PRT
<213> Artificial Sequence

<220>
<221> source
<223> /note="Description of Artificial Sequence: Synthetic
      peptide"

<400> 172
Gly Ala Ser Ser Arg Ala Thr
1               5


<210> 173
<211> 7
<212> PRT
<213> Artificial Sequence

<220>
<221> source
<223> /note="Description of Artificial Sequence: Synthetic
      peptide"

<400> 173
Ala Ala Ser Ser Leu Gln Ser
1               5


<210> 174
<211> 7
<212> PRT
<213> Artificial Sequence

<220>
<221> source
<223> /note="Description of Artificial Sequence: Synthetic
      peptide"

<400> 174
Ala Ala Ser Ser Leu Gln Ser
1               5


<210> 175
<211> 7
<212> PRT
<213> Artificial Sequence

<220>
<221> source
<223> /note="Description of Artificial Sequence: Synthetic
      peptide"

<400> 175
Glu Asp Asn Gln Arg Pro Ser
1               5

<210> 176
<211> 7
<212> PRT
<213> Artificial Sequence

<220>
<221> source
<223> /note="Description of Artificial Sequence: Synthetic
      peptide"

<400> 176
Asp Ala Ser Asn Leu Glu Thr
1               5


<210> 177
<211> 7
<212> PRT
<213> Artificial Sequence

<220>
<221> source
<223> /note="Description of Artificial Sequence: Synthetic
      peptide"

<400> 177
Ala Ala Ser Ser Leu Gln Ser
1               5


<210> 178
<211> 7
<212> PRT
<213> Artificial Sequence

<220>
<221> source
<223> /note="Description of Artificial Sequence: Synthetic
      peptide"

<400> 178
Asp Ala Ser Asn Leu Glu Thr
1               5


<210> 179
<211> 7
<212> PRT
<213> Artificial Sequence

<220>
<221> source
<223> /note="Description of Artificial Sequence: Synthetic
      peptide"

<400> 179
Asp Ala Ser Ser Leu Gln Thr
1               5


<210> 180
<211> 7
<212> PRT

<213> Artificial Sequence

<220>
<221> source
<223> /note="Description of Artificial Sequence: Synthetic
      peptide"

<400> 180
Ala Ala Ser Ser Leu Gln Ser
1               5


<210> 181
<211> 7
<212> PRT
<213> Artificial Sequence

<220>
<221> source
<223> /note="Description of Artificial Sequence: Synthetic
      peptide"

<400> 181
Asp Ala Ser Ser Leu Glu Ser
1               5


<210> 182
<211> 12
<212> PRT
<213> Artificial Sequence

<220>
<221> source
<223> /note="Description of Artificial Sequence: Synthetic
      peptide"

<400> 182
Ser Ser Tyr Thr Ser Ser Ser Thr Leu Asp Val Val
1               5                   10


<210> 183
<211> 9
<212> PRT
<213> Artificial Sequence

<220>
<221> source
<223> /note="Description of Artificial Sequence: Synthetic
      peptide"

<400> 183
Met Gln Gly Thr His Trp Ser Phe Thr
1               5


<210> 184
<211> 9
<212> PRT
<213> Artificial Sequence

<220>

<221> source
<223> /note="Description of Artificial Sequence: Synthetic
      peptide"

<400> 184
Gln Gln Leu Asn Ser Tyr Pro Tyr Thr
1               5


<210> 185
<211> 9
<212> PRT
<213> Artificial Sequence

<220>
<221> source
<223> /note="Description of Artificial Sequence: Synthetic
      peptide"

<400> 185
Gln Gln Tyr Gly Ser Ser Pro Pro Thr
1               5


<210> 186
<211> 9
<212> PRT
<213> Artificial Sequence

<220>
<221> source
<223> /note="Description of Artificial Sequence: Synthetic
      peptide"

<400> 186
Gln Gln Ser Tyr Ser Thr Pro Trp Thr
1               5


<210> 187
<211> 10
<212> PRT
<213> Artificial Sequence

<220>
<221> source
<223> /note="Description of Artificial Sequence: Synthetic
      peptide"

<400> 187
Gln Gln Ser Tyr Ser Thr Pro Pro Trp Thr
1               5                   10


<210> 188
<211> 10
<212> PRT
<213> Artificial Sequence

<220>
<221> source
<223> /note="Description of Artificial Sequence: Synthetic
      peptide"

<400> 188
Gln Ser Tyr Asp Ser Ser Asn Gln Val Val
1               5                   10


<210> 189
<211> 9
<212> PRT
<213> Artificial Sequence

<220>
<221> source
<223> /note="Description of Artificial Sequence: Synthetic
      peptide"

<400> 189
Gln Gln Tyr Asp Asn Leu Pro Leu Thr
1               5


<210> 190
<211> 9
<212> PRT
<213> Artificial Sequence

<220>
<221> source
<223> /note="Description of Artificial Sequence: Synthetic
      peptide"

<400> 190
Gln Gln Ser Tyr Ser Thr Pro Leu Thr
1               5


<210> 191
<211> 9
<212> PRT
<213> Artificial Sequence

<220>
<221> source
<223> /note="Description of Artificial Sequence: Synthetic
      peptide"

<400> 191
Gln Gln Ala Tyr Ser Thr Pro Phe Thr
1               5


<210> 192
<211> 9
<212> PRT
<213> Artificial Sequence

<220>
<221> source
<223> /note="Description of Artificial Sequence: Synthetic
      peptide"

<400> 192
Gln Gln His Asp Asn Leu Pro Leu Thr

1                   5


<210> 193
<211> 10
<212> PRT
<213> Artificial Sequence

<220>
<221> source
<223> /note="Description of Artificial Sequence: Synthetic
      peptide"

<400> 193
Gln Gln Ser Tyr Ser Thr Pro Pro Leu Thr
1               5                   10


<210> 194
<211> 9
<212> PRT
<213> Artificial Sequence

<220>
<221> source
<223> /note="Description of Artificial Sequence: Synthetic
      peptide"

<400> 194
Gln Gln Phe Asn Asn Tyr Pro Leu Thr
1               5


<210> 195
<211> 122
<212> PRT
<213> Artificial Sequence

<220>
<221> source
<223> /note="Description of Artificial Sequence: Synthetic
      polypeptide"

<400> 195
Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Ser Tyr
            20                  25                  30


Glu Met Asn Trp Val Arg Gln Ala Pro Gly Lys Gly Leu Glu Trp Val
        35                  40                  45


Ser Tyr Ile Ser Ser Ser Gly Ser Thr Ile Tyr Tyr Ala Asp Ser Val
    50                  55                  60


Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Pro Tyr Ser Ser Ser Trp His Asp Ala Phe Asp Ile Trp
            100                 105                 110

Gly Gln Gly Thr Met Val Thr Val Ser Ser
        115                 120

<210> 196
<211> 108
<212> PRT
<213> Artificial Sequence

<220>
<221> source
<223> /note="Description of Artificial Sequence: Synthetic
      polypeptide"

<400> 196
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Leu Thr Phe Gly Gly Gly Thr Lys Val Asp Ile Lys
            100                 105

<210> 197

<400> 197
000

<210> 198

<400> 198
000

<210> 199
<211> 10
<212> PRT
<213> Artificial Sequence

<220>
<221> source
<223> /note="Description of Artificial Sequence: Synthetic
      peptide"

<400> 199
Gly Phe Thr Phe Ser Ser Tyr Glu Met Asn
1               5                   10


<210> 200
<211> 17
<212> PRT
<213> Artificial Sequence

<220>
<221> source
<223> /note="Description of Artificial Sequence: Synthetic
      peptide"

<400> 200
Tyr Ile Ser Ser Ser Gly Ser Thr Ile Tyr Tyr Ala Asp Ser Val Lys
1               5                   10                  15


Gly



<210> 201
<211> 13
<212> PRT
<213> Artificial Sequence

<220>
<221> source
<223> /note="Description of Artificial Sequence: Synthetic
      peptide"

<400> 201
Asp Pro Tyr Ser Ser Ser Trp His Asp Ala Phe Asp Ile
1               5                   10


<210> 202
<211> 12
<212> PRT
<213> Artificial Sequence

<220>
<221> source
<223> /note="Description of Artificial Sequence: Synthetic
      peptide"

<400> 202
Arg Ala Ser Gln Ser Val Ser Ser Ser Tyr Leu Ala
1               5                   10

<210> 203
<211> 7
<212> PRT
<213> Artificial Sequence

<220>
<221> source
<223> /note="Description of Artificial Sequence: Synthetic
      peptide"

<400> 203
Gly Ala Ser Ser Arg Ala Thr
1               5


<210> 204
<211> 9
<212> PRT
<213> Artificial Sequence

<220>
<221> source
<223> /note="Description of Artificial Sequence: Synthetic
      peptide"

<400> 204
Gln Gln Tyr Gly Ser Ser Pro Leu Thr
1               5


<210> 205

<400> 205
000


<210> 206

<400> 206
000


<210> 207

<400> 207
000


<210> 208

<400> 208
000


<210> 209

<400> 209
000


<210> 210

<400> 210
000

<210> 211

<400> 211
000

<210> 212

<400> 212
000

<210> 213

<400> 213
000

<210> 214

<400> 214
000

<210> 215

<400> 215
000

<210> 216

<400> 216
000

<210> 217

<400> 217
000

<210> 218

<400> 218
000

<210> 219

<400> 219
000

<210> 220

<400> 220
000

<210> 221

<400> 221
000

<210> 222

<400> 222
000

<210> 223

<400> 223
000

<210> 224

<400> 224
000

<210> 225

<400> 225
000

<210> 226

<400> 226
000

<210> 227

<400> 227
000

<210> 228

<400> 228
000

<210> 229

<400> 229
000

<210> 230

<400> 230
000

<210> 231

<400> 231
000

<210> 232

<400> 232
000

<210> 233

<400> 233
000

<210> 234

<400> 234
000

<210> 235

<400> 235
000

<210> 236

<400> 236
000

<210> 237

<400> 237
000

<210> 238

<400> 238
000

<210> 239

<400> 239
000

<210> 240

<400> 240
000

<210> 241

<400> 241
000

<210> 242

<400> 242
000

<210> 243

<400> 243
000

<210> 244

<400> 244
000

<210> 245

<400> 245
000

<210> 246

<400> 246
000

<210> 247

<400> 247
000

<210> 248

<400> 248
000

<210> 249

<400> 249
000

<210> 250

<400> 250
000

<210> 251

<400> 251
000

<210> 252

<400> 252
000

<210> 253

<400> 253
000

<210> 254

<400> 254
000

<210> 255

<400> 255
000

<210> 256

<400> 256
000

<210> 257

<400> 257
000

<210> 258

<400> 258
000

<210> 259

<400> 259
000

<210> 260

<400> 260
000

<210> 261

<400> 261
000

<210> 262

<400> 262
000

<210> 263

<400> 263
000

<210> 264

<400> 264
000

<210> 265

<400> 265
000

<210> 266

<400> 266
000

<210> 267

<400> 267
000

<210> 268

<400> 268
000

<210> 269

<400> 269
000

<210> 270

<400> 270
000

<210> 271

<400> 271
000

<210> 272

<400> 272
000

<210> 273

<400> 273
000

<210> 274

<400> 274
000

<210> 275

<400> 275
000

<210> 276

<400> 276
000

<210> 277

<400> 277
000

<210> 278

<400> 278
000

<210> 279

<400> 279
000

<210> 280

<400> 280
000

<210> 281

<400> 281
000

<210> 282

<400> 282
000

<210> 283

<400> 283
000

<210> 284

<400> 284
000

<210> 285

<400> 285
000

<210> 286

<400> 286
000

<210> 287

<400> 287
000

<210> 288

<400> 288
000

<210> 289

<400> 289
000

<210> 290

<400> 290
000

<210> 291

<400> 291
000

<210> 292

<400> 292
000

<210> 293

<400> 293
000

<210> 294

<400> 294
000

<210> 295

<400> 295
000

<210> 296

<400> 296
000

<210> 297

<400> 297
000

<210> 298

<400> 298
000

<210> 299

<400> 299
000

<210> 300

<400> 300
000

<210> 301

<400> 301
000

<210> 302

<400> 302
000

<210> 303

<400> 303
000

<210> 304

<400> 304
000

<210> 305

<400> 305
000


<210> 306

<400> 306
000


<210> 307

<400> 307
000


<210> 308

<400> 308
000


<210> 309

<400> 309
000


<210> 310

<400> 310
000


<210> 311

<400> 311
000


<210> 312

<400> 312
000


<210> 313

<400> 313
000


<210> 314
<211> 5
<212> PRT
<213> Artificial Sequence

<220>
<221> source

<223> /note="Description of Artificial Sequence: Synthetic
      peptide"

<400> 314
Ser Tyr Ala Ile Ser
1               5


<210> 315
<211> 5
<212> PRT
<213> Artificial Sequence

<220>
<221> source
<223> /note="Description of Artificial Sequence: Synthetic
      peptide"

<400> 315
Asp Tyr Ala Met His
1               5


<210> 316
<211> 7
<212> PRT
<213> Artificial Sequence

<220>
<221> source
<223> /note="Description of Artificial Sequence: Synthetic
      peptide"

<400> 316
Ser Ser Ser Tyr Tyr Trp Gly
1               5


<210> 317
<211> 5
<212> PRT
<213> Artificial Sequence

<220>
<221> source
<223> /note="Description of Artificial Sequence: Synthetic
      peptide"

<400> 317
Ser Tyr Trp Met Ser
1               5


<210> 318
<211> 7
<212> PRT
<213> Artificial Sequence

<220>
<221> source
<223> /note="Description of Artificial Sequence: Synthetic
      peptide"

<400> 318
Ser Gly Ser His Tyr Trp Asn
1               5


<210> 319
<211> 5
<212> PRT
<213> Artificial Sequence

<220>
<221> source
<223> /note="Description of Artificial Sequence: Synthetic
      peptide"

<400> 319
Gly Tyr Tyr Trp Ser
1               5


<210> 320
<211> 5
<212> PRT
<213> Artificial Sequence

<220>
<221> source
<223> /note="Description of Artificial Sequence: Synthetic
      peptide"

<400> 320
Ser Tyr Ser Met Asn
1               5


<210> 321
<211> 5
<212> PRT
<213> Artificial Sequence

<220>
<221> source
<223> /note="Description of Artificial Sequence: Synthetic
      peptide"

<400> 321
Ser Tyr Glu Met Asn
1               5


<210> 322
<211> 5
<212> PRT
<213> Artificial Sequence

<220>
<221> source
<223> /note="Description of Artificial Sequence: Synthetic
      peptide"

<400> 322
Asp His Val Met His
1               5

<210> 323
<211> 5
<212> PRT
<213> Artificial Sequence

<220>
<221> source
<223> /note="Description of Artificial Sequence: Synthetic
      peptide"

<400> 323
Ser Tyr Ser Met Asn
1               5


<210> 324
<211> 5
<212> PRT
<213> Artificial Sequence

<220>
<221> source
<223> /note="Description of Artificial Sequence: Synthetic
      peptide"

<400> 324
Ser Tyr Gly Leu His
1               5


<210> 325
<211> 5
<212> PRT
<213> Artificial Sequence

<220>
<221> source
<223> /note="Description of Artificial Sequence: Synthetic
      peptide"

<400> 325
Ser Asn Tyr Met Thr
1               5


<210> 326
<211> 5
<212> PRT
<213> Artificial Sequence

<220>
<221> source
<223> /note="Description of Artificial Sequence: Synthetic
      peptide"

<400> 326
Gly Tyr Tyr Ile Gln
1               5


<210> 327

<211> 5
<212> PRT
<213> Artificial Sequence

<220>
<221> source
<223> /note="Description of Artificial Sequence: Synthetic
      peptide"

<400> 327
Ser Tyr Glu Met Asn
1               5


<210> 328
<211> 17
<212> PRT
<213> Artificial Sequence

<220>
<221> source
<223> /note="Description of Artificial Sequence: Synthetic
      peptide"

<400> 328
Gly Ile Ile Pro Ile Phe Gly Thr Ala Asn Tyr Ala Gln Lys Phe Gln
1               5                   10                  15


Gly




<210> 329
<211> 17
<212> PRT
<213> Artificial Sequence

<220>
<221> source
<223> /note="Description of Artificial Sequence: Synthetic
      peptide"

<400> 329
Leu Ile Ser Gly Asp Gly Gly Ser Thr Tyr Tyr Ala Asp Ser Val Lys
1               5                   10                  15


Gly




<210> 330
<211> 16
<212> PRT
<213> Artificial Sequence

<220>
<221> source
<223> /note="Description of Artificial Sequence: Synthetic
      peptide"

<400> 330

Ser Ile Tyr Tyr Ser Gly Ser Thr Tyr Tyr Asn Pro Ser Leu Lys Ser
1               5                   10                  15

<210> 331
<211> 17
<212> PRT
<213> Artificial Sequence

<220>
<221> source
<223> /note="Description of Artificial Sequence: Synthetic
      peptide"

<400> 331
Asn Ile Asn Glu Asp Gly Ser Ala Lys Phe Tyr Val Asp Ser Val Lys
1               5                   10                  15

Gly

<210> 332
<211> 16
<212> PRT
<213> Artificial Sequence

<220>
<221> source
<223> /note="Description of Artificial Sequence: Synthetic
      peptide"

<400> 332
Tyr Ile Tyr Tyr Ser Gly Ser Thr Asn Tyr Asn Pro Ser Leu Glu Asn
1               5                   10                  15

<210> 333
<211> 16
<212> PRT
<213> Artificial Sequence

<220>
<221> source
<223> /note="Description of Artificial Sequence: Synthetic
      peptide"

<400> 333
Glu Ile Asn His Ser Gly Ser Thr Asn Tyr Asn Pro Ser Leu Lys Ser
1               5                   10                  15

<210> 334
<211> 17
<212> PRT
<213> Artificial Sequence

<220>
<221> source
<223> /note="Description of Artificial Sequence: Synthetic
      peptide"

<400> 334
Ser Ile Ser Ser Ser Ser Ser Tyr Ile Tyr Tyr Ala Asp Ser Val Lys
1               5                   10                  15

Gly

<210> 335
<211> 17
<212> PRT
<213> Artificial Sequence

<220>
<221> source
<223> /note="Description of Artificial Sequence: Synthetic
      peptide"

<400> 335
Tyr Ile Ser Ser Ser Gly Ser Thr Ile Tyr Tyr Ala Asp Ser Val Lys
1               5                   10                  15

Gly

<210> 336
<211> 17
<212> PRT
<213> Artificial Sequence

<220>
<221> source
<223> /note="Description of Artificial Sequence: Synthetic
      peptide"

<400> 336
Tyr Ile His Ala Ala Asn Gly Gly Thr His Tyr Ser Gln Lys Phe Gln
1               5                   10                  15

Asp

<210> 337
<211> 17
<212> PRT
<213> Artificial Sequence

<220>
<221> source
<223> /note="Description of Artificial Sequence: Synthetic
      peptide"

<400> 337
Tyr Ile Ser Ser Ser Ser Ser Thr Ile Tyr Tyr Ala Asp Ser Val Lys
1               5                   10                  15

Gly

<210> 338
<211> 17
<212> PRT
<213> Artificial Sequence

<220>
<221> source
<223> /note="Description of Artificial Sequence: Synthetic
      peptide"

<400> 338
Leu Ile Glu Tyr Asp Gly Ser Asn Lys Tyr Tyr Gly Asp Ser Val Lys
1               5                   10                  15

Gly

<210> 339
<211> 16
<212> PRT
<213> Artificial Sequence

<220>
<221> source
<223> /note="Description of Artificial Sequence: Synthetic
      peptide"

<400> 339
Val Ile Tyr Ser Gly Gly Ala Thr Tyr Tyr Gly Asp Ser Val Lys Gly
1               5                   10                  15

<210> 340
<211> 17
<212> PRT
<213> Artificial Sequence

<220>
<221> source
<223> /note="Description of Artificial Sequence: Synthetic
      peptide"

<400> 340
Trp Ile Asp Pro Asn Ser Gly Asn Thr Gly Tyr Ala Gln Lys Phe Gln
1               5                   10                  15

Gly

<210> 341
<211> 17
<212> PRT
<213> Artificial Sequence

<220>
<221> source

<223> /note="Description of Artificial Sequence: Synthetic
      peptide"

<400> 341
Tyr Ile Ser Ser Ser Gly Ser Thr Ile Tyr Tyr Ala Asp Ser Val Lys
1               5                   10                  15

Gly

<210> 342
<211> 11
<212> PRT
<213> Artificial Sequence

<220>
<221> source
<223> /note="Description of Artificial Sequence: Synthetic
      peptide"

<400> 342
Asp Leu Glu Met Ala Thr Ile Met Gly Gly Tyr
1               5                   10

<210> 343
<211> 9
<212> PRT
<213> Artificial Sequence

<220>
<221> source
<223> /note="Description of Artificial Sequence: Synthetic
      peptide"

<400> 343
Val Phe Asp Ser Tyr Tyr Met Asp Val
1               5

<210> 344
<211> 15
<212> PRT
<213> Artificial Sequence

<220>
<221> source
<223> /note="Description of Artificial Sequence: Synthetic
      peptide"

<400> 344
Pro Gly Thr Tyr Tyr Asp Phe Leu Ser Gly Tyr Tyr Pro Phe Tyr
1               5                   10                  15

<210> 345
<211> 7
<212> PRT
<213> Artificial Sequence

<220>

<221> source
<223> /note="Description of Artificial Sequence: Synthetic
      peptide"

<400> 345
Asp Leu Arg Ser Gly Arg Tyr
1               5


<210> 346
<211> 13
<212> PRT
<213> Artificial Sequence

<220>
<221> source
<223> /note="Description of Artificial Sequence: Synthetic
      peptide"

<400> 346
Gly Thr Ala Thr Phe Asp Trp Asn Phe Pro Phe Asp Ser
1               5                   10


<210> 347
<211> 18
<212> PRT
<213> Artificial Sequence

<220>
<221> source
<223> /note="Description of Artificial Sequence: Synthetic
      peptide"

<400> 347
Gly Ser Gly Leu Val Val Tyr Ala Ile Arg Val Gly Ser Gly Trp Phe
1               5                   10                  15


Asp Tyr



<210> 348
<211> 20
<212> PRT
<213> Artificial Sequence

<220>
<221> source
<223> /note="Description of Artificial Sequence: Synthetic
      peptide"

<400> 348
Asp Pro Ser Ser Ser Gly Ser Tyr Tyr Met Glu Asp Ser Tyr Tyr Tyr
1               5                   10                  15


Gly Met Asp Val
            20


<210> 349

<211> 8
<212> PRT
<213> Artificial Sequence

<220>
<221> source
<223> /note="Description of Artificial Sequence: Synthetic
      peptide"

<400> 349
Glu Ala Leu Gly Ser Ser Trp Glu
1               5


<210> 350
<211> 10
<212> PRT
<213> Artificial Sequence

<220>
<221> source
<223> /note="Description of Artificial Sequence: Synthetic
      peptide"

<400> 350
Gly Gly Tyr Asn Ser Asp Ala Phe Asp Ile
1               5                   10


<210> 351
<211> 12
<212> PRT
<213> Artificial Sequence

<220>
<221> source
<223> /note="Description of Artificial Sequence: Synthetic
      peptide"

<400> 351
Asp Leu Ser Val Arg Ala Ile Asp Ala Phe Asp Ile
1               5                   10


<210> 352
<211> 10
<212> PRT
<213> Artificial Sequence

<220>
<221> source
<223> /note="Description of Artificial Sequence: Synthetic
      peptide"

<400> 352
Glu Gly Asn Glu Asp Leu Ala Phe Asp Ile
1               5                   10


<210> 353
<211> 19
<212> PRT
<213> Artificial Sequence

<220>
<221> source
<223> /note="Description of Artificial Sequence: Synthetic
      peptide"

<400> 353
Asp Arg Leu Tyr Cys Gly Asn Asn Cys Tyr Leu Tyr Tyr Tyr Tyr Gly
1               5                   10                  15

Met Asp Val

<210> 354
<211> 11
<212> PRT
<213> Artificial Sequence

<220>
<221> source
<223> /note="Description of Artificial Sequence: Synthetic
      peptide"

<400> 354
Asp Ser Tyr Gly Tyr Tyr Tyr Gly Met Asp Val
1               5                   10

<210> 355
<211> 13
<212> PRT
<213> Artificial Sequence

<220>
<221> source
<223> /note="Description of Artificial Sequence: Synthetic
      peptide"

<400> 355
Asp Pro Tyr Ser Ser Ser Trp His Asp Ala Phe Asp Ile
1               5                   10

<210> 356
<211> 14
<212> PRT
<213> Artificial Sequence

<220>
<221> source
<223> /note="Description of Artificial Sequence: Synthetic
      peptide"

<400> 356
Thr Gly Thr Ser Ser Asp Val Gly Gly Tyr Asn Tyr Val Ser
1               5                   10

<210> 357
<211> 16
<212> PRT

<213> Artificial Sequence

<220>
<221> source
<223> /note="Description of Artificial Sequence: Synthetic
      peptide"

<400> 357
Arg Ser Ser Gln Ser Leu Val Tyr Thr Asp Gly Asn Thr Tyr Leu Asn
1               5                   10                  15


<210> 358
<211> 11
<212> PRT
<213> Artificial Sequence

<220>
<221> source
<223> /note="Description of Artificial Sequence: Synthetic
      peptide"

<400> 358
Arg Ala Ser Gln Gly Ile Ser Ser Tyr Leu Ala
1               5                   10


<210> 359
<211> 12
<212> PRT
<213> Artificial Sequence

<220>
<221> source
<223> /note="Description of Artificial Sequence: Synthetic
      peptide"

<400> 359
Arg Ala Ser Gln Ser Ile Ser Gly Ser Phe Leu Ala
1               5                   10


<210> 360
<211> 11
<212> PRT
<213> Artificial Sequence

<220>
<221> source
<223> /note="Description of Artificial Sequence: Synthetic
      peptide"

<400> 360
Arg Ala Ser Gln Ser Ile Ser Ser Tyr Leu Asn
1               5                   10


<210> 361
<211> 11
<212> PRT
<213> Artificial Sequence

<220>

<221> source
<223> /note="Description of Artificial Sequence: Synthetic
      peptide"

<400> 361
Arg Ala Ser Gln Ser Ile Ser Ser Tyr Leu Asn
1               5                   10


<210> 362
<211> 13
<212> PRT
<213> Artificial Sequence

<220>
<221> source
<223> /note="Description of Artificial Sequence: Synthetic
      peptide"

<400> 362
Thr Gly Ser Ser Gly Ser Ile Ala Ser Asn Tyr Val Gln
1               5                   10


<210> 363
<211> 11
<212> PRT
<213> Artificial Sequence

<220>
<221> source
<223> /note="Description of Artificial Sequence: Synthetic
      peptide"

<400> 363
Gln Ala Ser Gln Asp Ile Ser Asn Tyr Leu Asn
1               5                   10


<210> 364
<211> 11
<212> PRT
<213> Artificial Sequence

<220>
<221> source
<223> /note="Description of Artificial Sequence: Synthetic
      peptide"

<400> 364
Arg Ala Ser Gln Asp Ile Ser Ser Trp Leu Ala
1               5                   10


<210> 365
<211> 11
<212> PRT
<213> Artificial Sequence

<220>
<221> source
<223> /note="Description of Artificial Sequence: Synthetic
      peptide"

<400> 365
Gln Ala Ser Gln Asp Ile Ser Asn Tyr Leu Asn
1               5                   10


<210> 366
<211> 11
<212> PRT
<213> Artificial Sequence

<220>
<221> source
<223> /note="Description of Artificial Sequence: Synthetic
      peptide"

<400> 366
Gln Ala Ser Gln Phe Ile Lys Lys Asn Leu Asn
1               5                   10


<210> 367
<211> 11
<212> PRT
<213> Artificial Sequence

<220>
<221> source
<223> /note="Description of Artificial Sequence: Synthetic
      peptide"

<400> 367
Arg Ala Ser Gln Ser Ile Ser Ser Tyr Leu Asn
1               5                   10


<210> 368
<211> 11
<212> PRT
<213> Artificial Sequence

<220>
<221> source
<223> /note="Description of Artificial Sequence: Synthetic
      peptide"

<400> 368
Arg Ala Ser Gln Gly Ile Ser Ser Ala Leu Ala
1               5                   10


<210> 369
<211> 12
<212> PRT
<213> Artificial Sequence

<220>
<221> source
<223> /note="Description of Artificial Sequence: Synthetic
      peptide"

<400> 369
Arg Ala Ser Gln Ser Val Ser Ser Ser Tyr Leu Ala

1                 5                    10


<210> 370
<211> 7
<212> PRT
<213> Artificial Sequence

<220>
<221> source
<223> /note="Description of Artificial Sequence: Synthetic
      peptide"

<400> 370
Asp Val Ser Asn Arg Pro Ser
1                 5


<210> 371
<211> 7
<212> PRT
<213> Artificial Sequence

<220>
<221> source
<223> /note="Description of Artificial Sequence: Synthetic
      peptide"

<400> 371
Lys Val Ser Asn Arg Asp Ser
1                 5


<210> 372
<211> 7
<212> PRT
<213> Artificial Sequence

<220>
<221> source
<223> /note="Description of Artificial Sequence: Synthetic
      peptide"

<400> 372
Ala Ala Ser Thr Leu Gln Ser
1                 5


<210> 373
<211> 7
<212> PRT
<213> Artificial Sequence

<220>
<221> source
<223> /note="Description of Artificial Sequence: Synthetic
      peptide"

<400> 373
Gly Ala Ser Ser Arg Ala Thr
1                 5

<210> 374
<211> 7
<212> PRT
<213> Artificial Sequence

<220>
<221> source
<223> /note="Description of Artificial Sequence: Synthetic
      peptide"

<400> 374
Ala Ala Ser Ser Leu Gln Ser
1               5


<210> 375
<211> 7
<212> PRT
<213> Artificial Sequence

<220>
<221> source
<223> /note="Description of Artificial Sequence: Synthetic
      peptide"

<400> 375
Ala Ala Ser Ser Leu Gln Ser
1               5


<210> 376
<211> 7
<212> PRT
<213> Artificial Sequence

<220>
<221> source
<223> /note="Description of Artificial Sequence: Synthetic
      peptide"

<400> 376
Glu Asp Asn Gln Arg Pro Ser
1               5


<210> 377
<211> 7
<212> PRT
<213> Artificial Sequence

<220>
<221> source
<223> /note="Description of Artificial Sequence: Synthetic
      peptide"

<400> 377
Asp Ala Ser Asn Leu Glu Thr
1               5


<210> 378
<211> 7
<212> PRT

<213> Artificial Sequence

<220>
<221> source
<223> /note="Description of Artificial Sequence: Synthetic
      peptide"

<400> 378
Ala Ala Ser Ser Leu Gln Ser
1               5


<210> 379
<211> 7
<212> PRT
<213> Artificial Sequence

<220>
<221> source
<223> /note="Description of Artificial Sequence: Synthetic
      peptide"

<400> 379
Asp Ala Ser Asn Leu Glu Thr
1               5


<210> 380
<211> 7
<212> PRT
<213> Artificial Sequence

<220>
<221> source
<223> /note="Description of Artificial Sequence: Synthetic
      peptide"

<400> 380
Asp Ala Ser Ser Leu Gln Thr
1               5


<210> 381
<211> 7
<212> PRT
<213> Artificial Sequence

<220>
<221> source
<223> /note="Description of Artificial Sequence: Synthetic
      peptide"

<400> 381
Ala Ala Ser Ser Leu Gln Ser
1               5


<210> 382
<211> 7
<212> PRT
<213> Artificial Sequence

<220>

<221> source
<223> /note="Description of Artificial Sequence: Synthetic
      peptide"

<400> 382
Asp Ala Ser Ser Leu Glu Ser
1               5


<210> 383
<211> 7
<212> PRT
<213> Artificial Sequence

<220>
<221> source
<223> /note="Description of Artificial Sequence: Synthetic
      peptide"

<400> 383
Gly Ala Ser Ser Arg Ala Thr
1               5


<210> 384
<211> 12
<212> PRT
<213> Artificial Sequence

<220>
<221> source
<223> /note="Description of Artificial Sequence: Synthetic
      peptide"

<400> 384
Ser Ser Tyr Thr Ser Ser Ser Thr Leu Asp Val Val
1               5                   10


<210> 385
<211> 9
<212> PRT
<213> Artificial Sequence

<220>
<221> source
<223> /note="Description of Artificial Sequence: Synthetic
      peptide"

<400> 385
Met Gln Gly Thr His Trp Ser Phe Thr
1               5


<210> 386
<211> 9
<212> PRT
<213> Artificial Sequence

<220>
<221> source
<223> /note="Description of Artificial Sequence: Synthetic
      peptide"

<400> 386
Gln Gln Leu Asn Ser Tyr Pro Tyr Thr
1               5

<210> 387
<211> 9
<212> PRT
<213> Artificial Sequence

<220>
<221> source
<223> /note="Description of Artificial Sequence: Synthetic
      peptide"

<400> 387
Gln Gln Tyr Gly Ser Ser Pro Pro Thr
1               5

<210> 388
<211> 9
<212> PRT
<213> Artificial Sequence

<220>
<221> source
<223> /note="Description of Artificial Sequence: Synthetic
      peptide"

<400> 388
Gln Gln Ser Tyr Ser Thr Pro Trp Thr
1               5

<210> 389
<211> 10
<212> PRT
<213> Artificial Sequence

<220>
<221> source
<223> /note="Description of Artificial Sequence: Synthetic
      peptide"

<400> 389
Gln Gln Ser Tyr Ser Thr Pro Pro Trp Thr
1               5                   10

<210> 390
<211> 10
<212> PRT
<213> Artificial Sequence

<220>
<221> source
<223> /note="Description of Artificial Sequence: Synthetic
      peptide"

<400> 390
Gln Ser Tyr Asp Ser Ser Asn Gln Val Val

1                 5                   10


<210> 391
<211> 9
<212> PRT
<213> Artificial Sequence

<220>
<221> source
<223> /note="Description of Artificial Sequence: Synthetic
      peptide"

<400> 391
Gln Gln Tyr Asp Asn Leu Pro Leu Thr
1               5


<210> 392
<211> 9
<212> PRT
<213> Artificial Sequence

<220>
<221> source
<223> /note="Description of Artificial Sequence: Synthetic
      peptide"

<400> 392
Gln Gln Ser Tyr Ser Thr Pro Leu Thr
1               5


<210> 393
<211> 9
<212> PRT
<213> Artificial Sequence

<220>
<221> source
<223> /note="Description of Artificial Sequence: Synthetic
      peptide"

<400> 393
Gln Gln Ala Tyr Ser Thr Pro Phe Thr
1               5


<210> 394
<211> 9
<212> PRT
<213> Artificial Sequence

<220>
<221> source
<223> /note="Description of Artificial Sequence: Synthetic
      peptide"

<400> 394
Gln Gln His Asp Asn Leu Pro Leu Thr
1               5

<210> 395
<211> 10
<212> PRT
<213> Artificial Sequence

<220>
<221> source
<223> /note="Description of Artificial Sequence: Synthetic
      peptide"

<400> 395
Gln Gln Ser Tyr Ser Thr Pro Pro Leu Thr
1               5                   10


<210> 396
<211> 9
<212> PRT
<213> Artificial Sequence

<220>
<221> source
<223> /note="Description of Artificial Sequence: Synthetic
      peptide"

<400> 396
Gln Gln Phe Asn Asn Tyr Pro Leu Thr
1               5


<210> 397
<211> 9
<212> PRT
<213> Artificial Sequence

<220>
<221> source
<223> /note="Description of Artificial Sequence: Synthetic
      peptide"

<400> 397
Gln Gln Tyr Gly Ser Ser Pro Leu Thr
1               5


<210> 398
<211> 7
<212> PRT
<213> Artificial Sequence

<220>
<221> source
<223> /note="Description of Artificial Sequence: Synthetic
      peptide"

<400> 398
Gly Gly Thr Phe Ser Ser Tyr
1               5


<210> 399
<211> 7
<212> PRT

<213> Artificial Sequence

<220>
<221> source
<223> /note="Description of Artificial Sequence: Synthetic
      peptide"

<400> 399
Gly Phe Thr Phe Asp Asp Tyr
1               5


<210> 400
<211> 9
<212> PRT
<213> Artificial Sequence

<220>
<221> source
<223> /note="Description of Artificial Sequence: Synthetic
      peptide"

<400> 400
Gly Gly Ser Ile Ser Ser Ser Ser Tyr
1               5


<210> 401
<211> 7
<212> PRT
<213> Artificial Sequence

<220>
<221> source
<223> /note="Description of Artificial Sequence: Synthetic
      peptide"

<400> 401
Gly Phe Thr Phe Ser Ser Tyr
1               5


<210> 402
<211> 9
<212> PRT
<213> Artificial Sequence

<220>
<221> source
<223> /note="Description of Artificial Sequence: Synthetic
      peptide"

<400> 402
Gly Gly Pro Val Arg Ser Gly Ser His
1               5


<210> 403
<211> 7
<212> PRT
<213> Artificial Sequence

<220>

<221> source
<223> /note="Description of Artificial Sequence: Synthetic
      peptide"

<400> 403
Gly Gly Ser Phe Ser Gly Tyr
1               5


<210> 404
<211> 7
<212> PRT
<213> Artificial Sequence

<220>
<221> source
<223> /note="Description of Artificial Sequence: Synthetic
      peptide"

<400> 404
Gly Phe Thr Phe Ser Ser Tyr
1               5


<210> 405
<211> 7
<212> PRT
<213> Artificial Sequence

<220>
<221> source
<223> /note="Description of Artificial Sequence: Synthetic
      peptide"

<400> 405
Gly Phe Thr Phe Ser Ser Tyr
1               5


<210> 406
<211> 7
<212> PRT
<213> Artificial Sequence

<220>
<221> source
<223> /note="Description of Artificial Sequence: Synthetic
      peptide"

<400> 406
Ala Asn Thr Phe Ser Asp His
1               5


<210> 407
<211> 7
<212> PRT
<213> Artificial Sequence

<220>
<221> source
<223> /note="Description of Artificial Sequence: Synthetic
      peptide"

<400> 407
Gly Phe Thr Phe Ser Ser Tyr
1               5


<210> 408
<211> 7
<212> PRT
<213> Artificial Sequence

<220>
<221> source
<223> /note="Description of Artificial Sequence: Synthetic
      peptide"

<400> 408
Gly Phe Thr Phe Asn Ser Tyr
1               5


<210> 409
<211> 7
<212> PRT
<213> Artificial Sequence

<220>
<221> source
<223> /note="Description of Artificial Sequence: Synthetic
      peptide"

<400> 409
Gly Phe Asn Val Ser Ser Asn
1               5


<210> 410
<211> 7
<212> PRT
<213> Artificial Sequence

<220>
<221> source
<223> /note="Description of Artificial Sequence: Synthetic
      peptide"

<400> 410
Gly Tyr Pro Phe Thr Gly Tyr
1               5


<210> 411
<211> 7
<212> PRT
<213> Artificial Sequence

<220>
<221> source
<223> /note="Description of Artificial Sequence: Synthetic
      peptide"

<400> 411
Gly Phe Thr Phe Ser Ser Tyr

1                   5


<210> 412
<211> 6
<212> PRT
<213> Artificial Sequence

<220>
<221> source
<223> /note="Description of Artificial Sequence: Synthetic
      peptide"

<400> 412
Ile Pro Ile Phe Gly Thr
1                   5


<210> 413
<211> 6
<212> PRT
<213> Artificial Sequence

<220>
<221> source
<223> /note="Description of Artificial Sequence: Synthetic
      peptide"

<400> 413
Ser Gly Asp Gly Gly Ser
1                   5


<210> 414
<211> 5
<212> PRT
<213> Artificial Sequence

<220>
<221> source
<223> /note="Description of Artificial Sequence: Synthetic
      peptide"

<400> 414
Tyr Tyr Ser Gly Ser
1                   5


<210> 415
<211> 6
<212> PRT
<213> Artificial Sequence

<220>
<221> source
<223> /note="Description of Artificial Sequence: Synthetic
      peptide"

<400> 415
Asn Glu Asp Gly Ser Ala
1                   5

<210> 416
<211> 5
<212> PRT
<213> Artificial Sequence

<220>
<221> source
<223> /note="Description of Artificial Sequence: Synthetic
      peptide"

<400> 416
Tyr Tyr Ser Gly Ser
1               5


<210> 417
<211> 5
<212> PRT
<213> Artificial Sequence

<220>
<221> source
<223> /note="Description of Artificial Sequence: Synthetic
      peptide"

<400> 417
Asn His Ser Gly Ser
1               5


<210> 418
<211> 6
<212> PRT
<213> Artificial Sequence

<220>
<221> source
<223> /note="Description of Artificial Sequence: Synthetic
      peptide"

<400> 418
Ser Ser Ser Ser Ser Tyr
1               5


<210> 419
<211> 6
<212> PRT
<213> Artificial Sequence

<220>
<221> source
<223> /note="Description of Artificial Sequence: Synthetic
      peptide"

<400> 419
Ser Ser Ser Gly Ser Thr
1               5


<210> 420
<211> 6
<212> PRT

<213> Artificial Sequence

<220>
<221> source
<223> /note="Description of Artificial Sequence: Synthetic
      peptide"

<400> 420
His Ala Ala Asn Gly Gly
1               5


<210> 421
<211> 6
<212> PRT
<213> Artificial Sequence

<220>
<221> source
<223> /note="Description of Artificial Sequence: Synthetic
      peptide"

<400> 421
Ser Ser Ser Ser Ser Thr
1               5


<210> 422
<211> 6
<212> PRT
<213> Artificial Sequence

<220>
<221> source
<223> /note="Description of Artificial Sequence: Synthetic
      peptide"

<400> 422
Glu Tyr Asp Gly Ser Asn
1               5


<210> 423
<211> 5
<212> PRT
<213> Artificial Sequence

<220>
<221> source
<223> /note="Description of Artificial Sequence: Synthetic
      peptide"

<400> 423
Tyr Ser Gly Gly Ala
1               5


<210> 424
<211> 6
<212> PRT
<213> Artificial Sequence

<220>

<221> source
<223> /note="Description of Artificial Sequence: Synthetic
      peptide"

<400> 424
Asp Pro Asn Ser Gly Asn
1               5


<210> 425
<211> 6
<212> PRT
<213> Artificial Sequence

<220>
<221> source
<223> /note="Description of Artificial Sequence: Synthetic
      peptide"

<400> 425
Ser Ser Ser Gly Ser Thr
1               5


<210> 426
<211> 11
<212> PRT
<213> Artificial Sequence

<220>
<221> source
<223> /note="Description of Artificial Sequence: Synthetic
      peptide"

<400> 426
Asp Leu Glu Met Ala Thr Ile Met Gly Gly Tyr
1               5                   10


<210> 427
<211> 9
<212> PRT
<213> Artificial Sequence

<220>
<221> source
<223> /note="Description of Artificial Sequence: Synthetic
      peptide"

<400> 427
Val Phe Asp Ser Tyr Tyr Met Asp Val
1               5


<210> 428
<211> 15
<212> PRT
<213> Artificial Sequence

<220>
<221> source
<223> /note="Description of Artificial Sequence: Synthetic
      peptide"

<400> 428
Pro Gly Thr Tyr Tyr Asp Phe Leu Ser Gly Tyr Tyr Pro Phe Tyr
1               5                   10                  15

<210> 429
<211> 7
<212> PRT
<213> Artificial Sequence

<220>
<221> source
<223> /note="Description of Artificial Sequence: Synthetic
      peptide"

<400> 429
Asp Leu Arg Ser Gly Arg Tyr
1               5

<210> 430
<211> 13
<212> PRT
<213> Artificial Sequence

<220>
<221> source
<223> /note="Description of Artificial Sequence: Synthetic
      peptide"

<400> 430
Gly Thr Ala Thr Phe Asp Trp Asn Phe Pro Phe Asp Ser
1               5                   10

<210> 431
<211> 18
<212> PRT
<213> Artificial Sequence

<220>
<221> source
<223> /note="Description of Artificial Sequence: Synthetic
      peptide"

<400> 431
Gly Ser Gly Leu Val Val Tyr Ala Ile Arg Val Gly Ser Gly Trp Phe
1               5                   10                  15

Asp Tyr

<210> 432
<211> 20
<212> PRT
<213> Artificial Sequence

<220>
<221> source
<223> /note="Description of Artificial Sequence: Synthetic

peptide"

<400> 432
Asp Pro Ser Ser Ser Gly Ser Tyr Tyr Met Glu Asp Ser Tyr Tyr Tyr
1               5                   10                  15

Gly Met Asp Val
            20

<210> 433
<211> 8
<212> PRT
<213> Artificial Sequence

<220>
<221> source
<223> /note="Description of Artificial Sequence: Synthetic
      peptide"

<400> 433
Glu Ala Leu Gly Ser Ser Trp Glu
1               5

<210> 434
<211> 10
<212> PRT
<213> Artificial Sequence

<220>
<221> source
<223> /note="Description of Artificial Sequence: Synthetic
      peptide"

<400> 434
Gly Gly Tyr Asn Ser Asp Ala Phe Asp Ile
1               5                   10

<210> 435
<211> 12
<212> PRT
<213> Artificial Sequence

<220>
<221> source
<223> /note="Description of Artificial Sequence: Synthetic
      peptide"

<400> 435
Asp Leu Ser Val Arg Ala Ile Asp Ala Phe Asp Ile
1               5                   10

<210> 436
<211> 10
<212> PRT
<213> Artificial Sequence

<220>
<221> source

<223> /note="Description of Artificial Sequence: Synthetic
      peptide"

<400> 436
Glu Gly Asn Glu Asp Leu Ala Phe Asp Ile
1               5                   10


<210> 437
<211> 19
<212> PRT
<213> Artificial Sequence

<220>
<221> source
<223> /note="Description of Artificial Sequence: Synthetic
      peptide"

<400> 437
Asp Arg Leu Tyr Cys Gly Asn Asn Cys Tyr Leu Tyr Tyr Tyr Tyr Gly
1               5                   10                  15


Met Asp Val




<210> 438
<211> 11
<212> PRT
<213> Artificial Sequence

<220>
<221> source
<223> /note="Description of Artificial Sequence: Synthetic
      peptide"

<400> 438
Asp Ser Tyr Gly Tyr Tyr Tyr Gly Met Asp Val
1               5                   10


<210> 439
<211> 13
<212> PRT
<213> Artificial Sequence

<220>
<221> source
<223> /note="Description of Artificial Sequence: Synthetic
      peptide"

<400> 439
Asp Pro Tyr Ser Ser Ser Trp His Asp Ala Phe Asp Ile
1               5                   10


<210> 440
<211> 10
<212> PRT
<213> Artificial Sequence

<220>

<221> source
<223> /note="Description of Artificial Sequence: Synthetic
      peptide"

<400> 440
Thr Ser Ser Asp Val Gly Gly Tyr Asn Tyr
1               5                   10


<210> 441
<211> 12
<212> PRT
<213> Artificial Sequence

<220>
<221> source
<223> /note="Description of Artificial Sequence: Synthetic
      peptide"

<400> 441
Ser Gln Ser Leu Val Tyr Thr Asp Gly Asn Thr Tyr
1               5                   10


<210> 442
<211> 7
<212> PRT
<213> Artificial Sequence

<220>
<221> source
<223> /note="Description of Artificial Sequence: Synthetic
      peptide"

<400> 442
Ser Gln Gly Ile Ser Ser Tyr
1               5


<210> 443
<211> 8
<212> PRT
<213> Artificial Sequence

<220>
<221> source
<223> /note="Description of Artificial Sequence: Synthetic
      peptide"

<400> 443
Ser Gln Ser Ile Ser Gly Ser Phe
1               5


<210> 444
<211> 7
<212> PRT
<213> Artificial Sequence

<220>
<221> source
<223> /note="Description of Artificial Sequence: Synthetic
      peptide"

<400> 444
Ser Gln Ser Ile Ser Ser Tyr
1               5


<210> 445
<211> 7
<212> PRT
<213> Artificial Sequence

<220>
<221> source
<223> /note="Description of Artificial Sequence: Synthetic
      peptide"

<400> 445
Ser Gln Ser Ile Ser Ser Tyr
1               5


<210> 446
<211> 9
<212> PRT
<213> Artificial Sequence

<220>
<221> source
<223> /note="Description of Artificial Sequence: Synthetic
      peptide"

<400> 446
Ser Ser Gly Ser Ile Ala Ser Asn Tyr
1               5


<210> 447
<211> 7
<212> PRT
<213> Artificial Sequence

<220>
<221> source
<223> /note="Description of Artificial Sequence: Synthetic
      peptide"

<400> 447
Ser Gln Asp Ile Ser Asn Tyr
1               5


<210> 448
<211> 7
<212> PRT
<213> Artificial Sequence

<220>
<221> source
<223> /note="Description of Artificial Sequence: Synthetic
      peptide"

<400> 448
Ser Gln Asp Ile Ser Ser Trp

1                 5


<210> 449
<211> 7
<212> PRT
<213> Artificial Sequence

<220>
<221> source
<223> /note="Description of Artificial Sequence: Synthetic
      peptide"

<400> 449
Ser Gln Asp Ile Ser Asn Tyr
1               5


<210> 450
<211> 7
<212> PRT
<213> Artificial Sequence

<220>
<221> source
<223> /note="Description of Artificial Sequence: Synthetic
      peptide"

<400> 450
Ser Gln Phe Ile Lys Lys Asn
1               5


<210> 451
<211> 7
<212> PRT
<213> Artificial Sequence

<220>
<221> source
<223> /note="Description of Artificial Sequence: Synthetic
      peptide"

<400> 451
Ser Gln Ser Ile Ser Ser Tyr
1               5


<210> 452
<211> 7
<212> PRT
<213> Artificial Sequence

<220>
<221> source
<223> /note="Description of Artificial Sequence: Synthetic
      peptide"

<400> 452
Ser Gln Gly Ile Ser Ser Ala
1               5

<210> 453
<211> 8
<212> PRT
<213> Artificial Sequence

<220>
<221> source
<223> /note="Description of Artificial Sequence: Synthetic
      peptide"

<400> 453
Ser Gln Ser Val Ser Ser Ser Tyr
1               5


<210> 454
<211> 3
<212> PRT
<213> Artificial Sequence

<220>
<221> source
<223> /note="Description of Artificial Sequence: Synthetic
      peptide"

<400> 454
Asp Val Ser
1


<210> 455
<211> 3
<212> PRT
<213> Artificial Sequence

<220>
<221> source
<223> /note="Description of Artificial Sequence: Synthetic
      peptide"

<400> 455
Lys Val Ser
1


<210> 456
<211> 3
<212> PRT
<213> Artificial Sequence

<220>
<221> source
<223> /note="Description of Artificial Sequence: Synthetic
      peptide"

<400> 456
Ala Ala Ser
1


<210> 457
<211> 3
<212> PRT

<213> Artificial Sequence

<220>
<221> source
<223> /note="Description of Artificial Sequence: Synthetic
      peptide"

<400> 457
Gly Ala Ser
1


<210> 458
<211> 3
<212> PRT
<213> Artificial Sequence

<220>
<221> source
<223> /note="Description of Artificial Sequence: Synthetic
      peptide"

<400> 458
Ala Ala Ser
1


<210> 459
<211> 3
<212> PRT
<213> Artificial Sequence

<220>
<221> source
<223> /note="Description of Artificial Sequence: Synthetic
      peptide"

<400> 459
Ala Ala Ser
1


<210> 460
<211> 3
<212> PRT
<213> Artificial Sequence

<220>
<221> source
<223> /note="Description of Artificial Sequence: Synthetic
      peptide"

<400> 460
Glu Asp Asn
1


<210> 461
<211> 3
<212> PRT
<213> Artificial Sequence

<220>

<221> source
<223> /note="Description of Artificial Sequence: Synthetic
      peptide"

<400> 461
Asp Ala Ser
1


<210> 462
<211> 3
<212> PRT
<213> Artificial Sequence

<220>
<221> source
<223> /note="Description of Artificial Sequence: Synthetic
      peptide"

<400> 462
Ala Ala Ser
1


<210> 463
<211> 3
<212> PRT
<213> Artificial Sequence

<220>
<221> source
<223> /note="Description of Artificial Sequence: Synthetic
      peptide"

<400> 463
Asp Ala Ser
1


<210> 464
<211> 3
<212> PRT
<213> Artificial Sequence

<220>
<221> source
<223> /note="Description of Artificial Sequence: Synthetic
      peptide"

<400> 464
Asp Ala Ser
1


<210> 465
<211> 3
<212> PRT
<213> Artificial Sequence

<220>
<221> source
<223> /note="Description of Artificial Sequence: Synthetic
      peptide"

<400> 465
Ala Ala Ser
1


<210> 466
<211> 3
<212> PRT
<213> Artificial Sequence

<220>
<221> source
<223> /note="Description of Artificial Sequence: Synthetic
      peptide"

<400> 466
Asp Ala Ser
1


<210> 467
<211> 3
<212> PRT
<213> Artificial Sequence

<220>
<221> source
<223> /note="Description of Artificial Sequence: Synthetic
      peptide"

<400> 467
Gly Ala Ser
1


<210> 468
<211> 9
<212> PRT
<213> Artificial Sequence

<220>
<221> source
<223> /note="Description of Artificial Sequence: Synthetic
      peptide"

<400> 468
Tyr Thr Ser Ser Ser Thr Leu Asp Val
1               5


<210> 469
<211> 6
<212> PRT
<213> Artificial Sequence

<220>
<221> source
<223> /note="Description of Artificial Sequence: Synthetic
      peptide"

<400> 469
Gly Thr His Trp Ser Phe

1               5


<210> 470
<211> 6
<212> PRT
<213> Artificial Sequence

<220>
<221> source
<223> /note="Description of Artificial Sequence: Synthetic
      peptide"

<400> 470
Leu Asn Ser Tyr Pro Tyr
1               5


<210> 471
<211> 6
<212> PRT
<213> Artificial Sequence

<220>
<221> source
<223> /note="Description of Artificial Sequence: Synthetic
      peptide"

<400> 471
Tyr Gly Ser Ser Pro Pro
1               5


<210> 472
<211> 6
<212> PRT
<213> Artificial Sequence

<220>
<221> source
<223> /note="Description of Artificial Sequence: Synthetic
      peptide"

<400> 472
Ser Tyr Ser Thr Pro Trp
1               5


<210> 473
<211> 7
<212> PRT
<213> Artificial Sequence

<220>
<221> source
<223> /note="Description of Artificial Sequence: Synthetic
      peptide"

<400> 473
Ser Tyr Ser Thr Pro Pro Trp
1               5

<210> 474
<211> 7
<212> PRT
<213> Artificial Sequence

<220>
<221> source
<223> /note="Description of Artificial Sequence: Synthetic
      peptide"

<400> 474
Tyr Asp Ser Ser Asn Gln Val
1               5


<210> 475
<211> 6
<212> PRT
<213> Artificial Sequence

<220>
<221> source
<223> /note="Description of Artificial Sequence: Synthetic
      peptide"

<400> 475
Tyr Asp Asn Leu Pro Leu
1               5


<210> 476
<211> 6
<212> PRT
<213> Artificial Sequence

<220>
<221> source
<223> /note="Description of Artificial Sequence: Synthetic
      peptide"

<400> 476
Ser Tyr Ser Thr Pro Leu
1               5


<210> 477
<211> 6
<212> PRT
<213> Artificial Sequence

<220>
<221> source
<223> /note="Description of Artificial Sequence: Synthetic
      peptide"

<400> 477
Ala Tyr Ser Thr Pro Phe
1               5


<210> 478
<211> 6
<212> PRT

<213> Artificial Sequence

<220>
<221> source
<223> /note="Description of Artificial Sequence: Synthetic
      peptide"

<400> 478
His Asp Asn Leu Pro Leu
1               5


<210> 479
<211> 7
<212> PRT
<213> Artificial Sequence

<220>
<221> source
<223> /note="Description of Artificial Sequence: Synthetic
      peptide"

<400> 479
Ser Tyr Ser Thr Pro Pro Leu
1               5


<210> 480
<211> 6
<212> PRT
<213> Artificial Sequence

<220>
<221> source
<223> /note="Description of Artificial Sequence: Synthetic
      peptide"

<400> 480
Phe Asn Asn Tyr Pro Leu
1               5


<210> 481
<211> 6
<212> PRT
<213> Artificial Sequence

<220>
<221> source
<223> /note="Description of Artificial Sequence: Synthetic
      peptide"

<400> 481
Tyr Gly Ser Ser Pro Leu
1               5


<210> 482

<400> 482
000

<210> 483

<400> 483
000

<210> 484

<400> 484
000

<210> 485

<400> 485
000

<210> 486

<400> 486
000

<210> 487

<400> 487
000

<210> 488

<400> 488
000

<210> 489

<400> 489
000

<210> 490

<400> 490
000

<210> 491

<400> 491
000

<210> 492

<400> 492
000

<210> 493

<400> 493

000

<210> 494

<400> 494
000

<210> 495

<400> 495
000

<210> 496

<400> 496
000

<210> 497

<400> 497
000

<210> 498

<400> 498
000

<210> 499

<400> 499
000

<210> 500
<211> 232
<212> PRT
<213> Artificial Sequence

<220>
<221> source
<223> /note="Description of Artificial Sequence: Synthetic
      polypeptide"

<400> 500
Gly Gly Gly Gly Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Ala Val Ser His Glu Asp Pro Glu Val Lys Phe Asn Trp Tyr Val

50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Ala Ala Pro Ile Glu Lys Thr Ile Ser Lys Ala Lys Gly Gln Pro
        115                 120                 125

Arg Glu Pro Gln Val Cys Thr Leu Pro Pro Ser Arg Asp Glu Leu Thr
    130                 135                 140

Lys Asn Gln Val Ser Leu Ser Cys Ala Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Val
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210> 501
<211> 232
<212> PRT
<213> Artificial Sequence

<220>
<221> source
<223> /note="Description of Artificial Sequence: Synthetic
      polypeptide"

<400> 501
Gly Gly Gly Gly Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro

20                  25                  30

Lys Asp Thr Leu Met Ile Ser Arg Thr Pro Glu Val Thr Cys Val Val
        35                  40                  45

Val Ala Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Ala Ala Pro Ile Glu Lys Thr Ile Ser Lys Ala Lys Gly Gln Pro
        115                 120                 125

Arg Glu Pro Gln Val Tyr Thr Leu Pro Pro Cys Arg Asp Glu Leu Thr
    130                 135                 140

Lys Asn Gln Val Ser Leu Trp Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210> 502
<211> 107
<212> PRT
<213> Artificial Sequence

<220>
<221> source
<223> /note="Description of Artificial Sequence: Synthetic

polypeptide"

<400> 502


<210> 503
<211> 106
<212> PRT
<213> Artificial Sequence

<220>
<221> source
<223> /note="Description of Artificial Sequence: Synthetic
      polypeptide"

<400> 503
Gly Gln Pro Lys Ala Ala Pro Ser Val Thr Leu Phe Pro Pro Ser Ser
1               5                   10                  15

Glu Glu Leu Gln Ala Asn Lys Ala Thr Leu Val Cys Leu Ile Ser Asp
            20                  25                  30

Phe Tyr Pro Gly Ala Val Thr Val Ala Trp Lys Ala Asp Ser Ser Pro
        35                  40                  45

Val Lys Ala Gly Val Glu Thr Thr Thr Pro Ser Lys Gln Ser Asn Asn
    50                  55                  60

Lys Tyr Ala Ala Ser Ser Tyr Leu Ser Leu Thr Pro Glu Gln Trp Lys
65                  70                  75                  80

Ser His Arg Ser Tyr Ser Cys Gln Val Thr His Glu Gly Ser Thr Val

85                  90                  95

Glu Lys Thr Val Ala Pro Thr Glu Cys Ser
            100                 105

<210> 504
<211> 330
<212> PRT
<213> Artificial Sequence

<220>
<221> source
<223> /note="Description of Artificial Sequence: Synthetic
      polypeptide"

<400> 504
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Ala Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu

180                 185                 190

His Gln Asp Trp Leu Asn Gly Lys Glu Tyr Lys Cys Lys Val Ser Asn
        195                 200                 205

Lys Ala Leu Ala Ala Pro Ile Glu Lys Thr Ile Ser Lys Ala Lys Gly
    210                 215                 220

Gln Pro Arg Glu Pro Gln Val Cys Thr Leu Pro Pro Ser Arg Glu Glu
225                 230                 235                 240

Met Thr Lys Asn Gln Val Ser Leu Ser Cys Ala Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Val Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 505
<211> 330
<212> PRT
<213> Artificial Sequence

<220>
<221> source
<223> /note="Description of Artificial Sequence: Synthetic
      polypeptide"

<400> 505
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser

50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Ala Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Ala Ala Pro Ile Glu Lys Thr Ile Ser Lys Ala Lys Gly
    210                 215                 220

Gln Pro Arg Glu Pro Gln Val Tyr Thr Leu Pro Pro Cys Arg Glu Glu
225                 230                 235                 240

Met Thr Lys Asn Gln Val Ser Leu Trp Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 506
<211> 245
<212> PRT
<213> Artificial Sequence

<220>
<221> source
<223> /note="Description of Artificial Sequence: Synthetic
      polypeptide"

<400> 506
Gln Val Gln Leu Val Gln Ser Gly Gly Gly Val Val Gln Pro Gly Arg
1               5                   10                  15

Ser Leu Arg Leu Ser Cys Lys Ala Ser Gly Tyr Thr Phe Thr Arg Tyr
            20                  25                  30

Thr Met His Trp Val Arg Gln Ala Pro Gly Lys Gly Leu Glu Trp Ile
        35                  40                  45

Gly Tyr Ile Asn Pro Ser Arg Gly Tyr Thr Asn Tyr Asn Gln Lys Val
    50                  55                  60

Lys Asp Arg Phe Thr Ile Ser Arg Asp Asn Ser Lys Asn Thr Ala Phe
65                  70                  75                  80

Leu Gln Met Asp Ser Leu Arg Pro Glu Asp Thr Gly Val Tyr Phe Cys
                85                  90                  95

Ala Arg Tyr Tyr Asp Asp His Tyr Ser Leu Asp Tyr Trp Gly Gln Gly
            100                 105                 110

Thr Pro Val Thr Val Ser Ser Gly Gly Gly Gly Ser Gly Gly Gly Lys
        115                 120                 125

Ser Lys Lys Gly Gly Ser Gly Gly Gly Gly Ser Asp Ile Gln Met Thr
    130                 135                 140

Gln Ser Pro Ser Ser Leu Ser Ala Ser Val Gly Asp Arg Val Thr Ile
145                 150                 155                 160

Thr Cys Ser Ala Ser Ser Ser Val Ser Tyr Met Asn Trp Tyr Gln Gln
                165                 170                 175

Thr Pro Gly Lys Ala Pro Lys Arg Trp Ile Tyr Asp Thr Ser Lys Leu
            180                 185                 190

Ala Ser Gly Val Pro Ser Arg Phe Ser Gly Ser Gly Ser Gly Thr Asp
        195                 200                 205

Tyr Thr Phe Thr Ile Ser Ser Leu Gln Pro Glu Asp Ile Ala Thr Tyr
    210                 215                 220

Tyr Cys Gln Gln Trp Ser Ser Asn Pro Phe Thr Phe Gly Gln Gly Thr
225                 230                 235                 240

Lys Leu Gln Ile Thr
                245

<210> 507
<211> 477
<212> PRT
<213> Artificial Sequence

<220>
<221> source
<223> /note="Description of Artificial Sequence: Synthetic
polypeptide"

<400> 507
Gln Val Gln Leu Val Gln Ser Gly Gly Gly Val Val Gln Pro Gly Arg
1               5                   10                  15

Ser Leu Arg Leu Ser Cys Lys Ala Ser Gly Tyr Thr Phe Thr Arg Tyr
            20                  25                  30

Thr Met His Trp Val Arg Gln Ala Pro Gly Lys Gly Leu Glu Trp Ile
        35                  40                  45

Gly Tyr Ile Asn Pro Ser Arg Gly Tyr Thr Asn Tyr Asn Gln Lys Val
    50                  55                  60

Lys Asp Arg Phe Thr Ile Ser Arg Asp Asn Ser Lys Asn Thr Ala Phe
65                  70                  75                  80

Leu Gln Met Asp Ser Leu Arg Pro Glu Asp Thr Gly Val Tyr Phe Cys
                85                  90                  95

Ala Arg Tyr Tyr Asp Asp His Tyr Ser Leu Asp Tyr Trp Gly Gln Gly
            100                 105                 110

Thr Pro Val Thr Val Ser Ser Gly Gly Gly Gly Ser Gly Gly Gly Lys
        115                 120                 125

Ser Lys Lys Gly Gly Ser Gly Gly Gly Gly Ser Asp Ile Gln Met Thr
    130                 135                 140

Gln Ser Pro Ser Ser Leu Ser Ala Ser Val Gly Asp Arg Val Thr Ile
145                 150                 155                 160

Thr Cys Ser Ala Ser Ser Ser Val Ser Tyr Met Asn Trp Tyr Gln Gln
                165                 170                 175

Thr Pro Gly Lys Ala Pro Lys Arg Trp Ile Tyr Asp Thr Ser Lys Leu
            180                 185                 190

Ala Ser Gly Val Pro Ser Arg Phe Ser Gly Ser Gly Ser Gly Thr Asp
        195                 200                 205

Tyr Thr Phe Thr Ile Ser Ser Leu Gln Pro Glu Asp Ile Ala Thr Tyr
    210                 215                 220

Tyr Cys Gln Gln Trp Ser Ser Asn Pro Phe Thr Phe Gly Gln Gly Thr
225                 230                 235                 240

Lys Leu Gln Ile Thr Gly Gly Gly Gly Ser Asp Lys Thr His Thr Cys
                245                 250                 255

Pro Pro Cys Pro Ala Pro Glu Leu Leu Gly Gly Pro Ser Val Phe Leu
            260                 265                 270

Phe Pro Pro Lys Pro Lys Asp Thr Leu Met Ile Ser Arg Thr Pro Glu
        275                 280                 285

Val Thr Cys Val Val Val Ala Val Ser His Glu Asp Pro Glu Val Lys
    290                 295                 300

Phe Asn Trp Tyr Val Asp Gly Val Glu Val His Asn Ala Lys Thr Lys
305                 310                 315                 320

Pro Arg Glu Glu Gln Tyr Asn Ser Thr Tyr Arg Val Val Ser Val Leu
                325                 330                 335

Thr Val Leu His Gln Asp Trp Leu Asn Gly Lys Glu Tyr Lys Cys Lys
            340                 345                 350

Val Ser Asn Lys Ala Leu Ala Ala Pro Ile Glu Lys Thr Ile Ser Lys
        355                 360                 365

Ala Lys Gly Gln Pro Arg Glu Pro Gln Val Cys Thr Leu Pro Pro Ser
    370                 375                 380

Arg Asp Glu Leu Thr Lys Asn Gln Val Ser Leu Ser Cys Ala Val Lys
385                 390                 395                 400

Gly Phe Tyr Pro Ser Asp Ile Ala Val Glu Trp Glu Ser Asn Gly Gln
                405                 410                 415

Pro Glu Asn Asn Tyr Lys Thr Thr Pro Pro Val Leu Asp Ser Asp Gly
            420                 425                 430

Ser Phe Phe Leu Val Ser Lys Leu Thr Val Asp Lys Ser Arg Trp Gln
        435                 440                 445

Gln Gly Asn Val Phe Ser Cys Ser Val Met His Glu Ala Leu His Asn
    450                 455                 460

His Tyr Thr Gln Lys Ser Leu Ser Leu Ser Pro Gly Lys
465                 470                 475

<210> 508
<211> 318
<212> DNA
<213> Artificial Sequence

<220>
<221> source
<223> /note="Description of Artificial Sequence: Synthetic
      polynucleotide"

<400> 508
gacattcaga tgactcagag cccaagctcc ctctccgcct ccgtgggtga tcgcgtgacc      60

attacttgct ccgcctcgtc atccgtgtca tacatgaact ggtatcagca gacccccgga     120

aaggccccga agcgctggat ctacgacacc tccaagctgg cttccggcgt gcctagccgg     180

ttcagcggaa gcggttccgg gaccgactac acttttacca tttcctccct gcaacccgag     240

gacatcgcga cgtattactg ccagcagtgg tcctccaacc ccttcacctt cggacagggt     300

acaaagctgc agatcacc                                                   318

<210> 509
<211> 354
<212> DNA
<213> Artificial Sequence

<220>
<221> source
<223> /note="Description of Artificial Sequence: Synthetic
      polynucleotide"

<400> 509
caagtgcagt tggtgcagtc cggtggtgga gtggtccagc cgggcagatc actgaggctt      60

agctgcaagg catccgggta caccttcacc cggtacacta tgcactgggt ccgccaagcc     120

ccgggaaaag gactggaatg gatcggctac atcaacccat cgagagggta caccaactac 180

aatcagaagg tcaaggaccg gttcactatc tcgagggaca actcaaagaa caccgcgttc 240

ctgcaaatgg attcgctgcg gccggaggac accggggtgt acttctgtgc ccggtactac 300

gatgaccact actctctgga ctactggggc cagggcactc ctgtgaccgt gtcc 354

<210> 510
<211> 735
<212> DNA
<213> Artificial Sequence

<220>
<221> source
<223> /note="Description of Artificial Sequence: Synthetic
      polynucleotide"

<400> 510
caagtgcagt tggtgcagtc cggtggtgga gtggtccagc cgggcagatc actgaggctt 60

agctgcaagg catccgggta caccttcacc cggtacacta tgcactgggt ccgccaagcc 120

ccgggaaaag gactggaatg gatcggctac atcaacccat cgagagggta caccaactac 180

aatcagaagg tcaaggaccg gttcactatc tcgagggaca actcaaagaa caccgcgttc 240

ctgcaaatgg attcgctgcg gccggaggac accggggtgt acttctgtgc ccggtactac 300

gatgaccact actctctgga ctactggggc cagggcactc ctgtgaccgt gtcctcgggg 360

ggaggaggaa gcggcggagg aaaatccaag aagggcggca gcgggggcgg aggctcggac 420

attcagatga ctcagagccc aagctccctc tccgcctccg tgggtgatcg cgtgaccatt 480

acttgctccg cctcgtcatc cgtgtcatac atgaactggt atcagcagac ccccggaaag 540

gccccgaagc gctggatcta cgacacctcc aagctggctt ccggcgtgcc tagccggttc 600

agcggaagcg gttccgggac cgactacact tttaccattt cctccctgca acccgaggac 660

atcgcgacgt attactgcca gcagtggtcc tccaacccct tcaccttcgg acagggtaca 720

aagctgcaga tcacc 735

<210> 511
<211> 1431
<212> DNA
<213> Artificial Sequence

<220>
<221> source
<223> /note="Description of Artificial Sequence: Synthetic
      polynucleotide"

<400> 511
caagtgcagt tggtgcagtc cggtggtgga gtggtccagc cgggcagatc actgaggctt 60

agctgcaagg catccgggta caccttcacc cggtacacta tgcactgggt ccgccaagcc 120

ccgggaaaag gactggaatg gatcggctac atcaacccat cgagagggta caccaactac 180

aatcagaagg tcaaggaccg gttcactatc tcgagggaca actcaaagaa caccgcgttc 240

ctgcaaatgg attcgctgcg gccggaggac accggggtgt acttctgtgc ccggtactac 300

gatgaccact actctctgga ctactggggc cagggcactc ctgtgaccgt gtcctcgggg 360

ggaggaggaa gcggcggagg aaaatccaag aagggcggca gcgggggcgg aggctcggac 420

attcagatga ctcagagccc aagctccctc tccgcctccg tgggtgatcg cgtgaccatt 480

acttgctccg cctcgtcatc cgtgtcatac atgaactggt atcagcagac ccccggaaag 540

gccccgaagc gctggatcta cgacacctcc aagctggctt ccggcgtgcc tagccggttc 600

agcggaagcg gttccgggac cgactacact tttaccattt cctccctgca acccgaggac 660

atcgcgacgt attactgcca gcagtggtcc tccaacccct tcaccttcgg acagggtaca 720

aagctgcaga tcaccggagg gggcggatcc gacaagaccc acacctgtcc tccttgtcct 780

gccccggaac tgctgggcgg ccccagcgtg ttcctgttcc cgccgaagcc taaggatact 840

ctcatgatca gcaggacgcc tgaagtgacc tgtgtcgtgg tggccgtgtc ccatgaagat 900

ccagaagtca agttcaattg gtacgtggac ggcgtggagg tgcacaacgc caagacaaag 960

cctagagagg aacagtacaa cagcacctac cgcgtcgtgt ccgtgctgac cgtgctgcac 1020

caggactggc tgaacggaaa ggagtacaag tgcaaagtgt ccaacaaggc cctggccgcc 1080

ccaattgaaa agactatctc caaggccaag ggccagcccc gcgagcccca ggtgtgcact 1140

ctgccgccgt caagagatga actgactaag aaccaggtgt cactgtcctg cgccgtgaaa 1200

gggttctacc cctccgacat cgccgtggag tgggaaagca acggacagcc tgaaaacaac 1260

tacaaaacga ctccccctgt gctcgactcc gatggctcgt tcttcttggt gtcgaagctc 1320

accgtggata agagccggtg gcaacaggga aacgtgtttt cctgctccgt gatgcatgag 1380

gccctccaca accactacac ccagaaatcc ctctccctgt cgccggggaa g 1431

<210> 512
<211> 480
<212> PRT
<213> Artificial Sequence

<220>
<221> source
<223> /note="Description of Artificial Sequence: Synthetic
      polypeptide"

<400> 512
Glu Val Gln Leu Val Gln Ser Gly Gly Gly Val Val Arg Ser Gly Arg
1               5                   10                  15

Ser Leu Arg Leu Ser Cys Ala Ala Ser Gly Phe Thr Phe Asn Ser Tyr
            20                  25                  30

Gly Leu His Trp Val Arg Gln Ala Pro Gly Lys Gly Leu Glu Trp Val
        35                  40                  45

Ala Leu Ile Glu Tyr Asp Gly Ser Asn Lys Tyr Tyr Gly Asp Ser Val
    50                  55                  60

Lys Gly Arg Phe Thr Ile Ser Arg Asp Lys Ser Lys Ser Thr Leu Tyr
65                  70                  75                  80

Leu Gln Met Asp Asn Leu Arg Ala Glu Asp Thr Ala Val Tyr Tyr Cys
                85                  90                  95

Ala Arg Glu Gly Asn Glu Asp Leu Ala Phe Asp Ile Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Gly Gly Gly Gly Ser Glu Ile Val Leu Thr
    130                 135                 140

Gln Ser Pro Ser Ser Leu Ser Ala Ser Val Gly Asp Arg Val Thr Ile
145                 150                 155                 160

Thr Cys Gln Ala Ser Gln Phe Ile Lys Lys Asn Leu Asn Trp Tyr Gln
                165                 170                 175

His Lys Pro Gly Lys Ala Pro Lys Leu Leu Ile Tyr Asp Ala Ser Ser
            180                 185                 190

Leu Gln Thr Gly Val Pro Ser Arg Phe Ser Gly Asn Arg Ser Gly Thr
        195                 200                 205

Thr Phe Ser Phe Thr Ile Ser Ser Leu Gln Pro Glu Asp Val Ala Thr
    210                 215                 220

Tyr Tyr Cys Gln Gln His Asp Asn Leu Pro Leu Thr Phe Gly Gly Gly
225                 230                 235                 240

Thr Lys Val Glu Ile Lys Gly Gly Gly Gly Ser Gly Ser Asp Lys Thr
                245                 250                 255

His Thr Cys Pro Pro Cys Pro Ala Pro Glu Leu Leu Gly Gly Pro Ser
            260                 265                 270

Val Phe Leu Phe Pro Pro Lys Pro Lys Asp Thr Leu Met Ile Ser Arg
        275                 280                 285

Thr Pro Glu Val Thr Cys Val Val Val Ala Val Ser His Glu Asp Pro
    290                 295                 300

Glu Val Lys Phe Asn Trp Tyr Val Asp Gly Val Glu Val His Asn Ala
305                 310                 315                 320

Lys Thr Lys Pro Arg Glu Glu Gln Tyr Asn Ser Thr Tyr Arg Val Val
                325                 330                 335

Ser Val Leu Thr Val Leu His Gln Asp Trp Leu Asn Gly Lys Glu Tyr
            340                 345                 350

Lys Cys Lys Val Ser Asn Lys Ala Leu Ala Ala Pro Ile Glu Lys Thr
        355                 360                 365

Ile Ser Lys Ala Lys Gly Gln Pro Arg Glu Pro Gln Val Tyr Thr Leu
    370                 375                 380

Pro Pro Cys Arg Asp Glu Leu Thr Lys Asn Gln Val Ser Leu Trp Cys
385                 390                 395                 400

Leu Val Lys Gly Phe Tyr Pro Ser Asp Ile Ala Val Glu Trp Glu Ser
                405                 410                 415

Asn Gly Gln Pro Glu Asn Asn Tyr Lys Thr Thr Pro Pro Val Leu Asp
            420                 425                 430

Ser Asp Gly Ser Phe Phe Leu Tyr Ser Lys Leu Thr Val Asp Lys Ser
        435                 440                 445

Arg Trp Gln Gln Gly Asn Val Phe Ser Cys Ser Val Met His Glu Ala
    450                 455                 460

Leu His Asn His Tyr Thr Gln Lys Ser Leu Ser Leu Ser Pro Gly Lys
465                 470                 475                 480

<210> 513
<211> 321
<212> DNA
<213> Artificial Sequence

<220>
<221> source
<223> /note="Description of Artificial Sequence: Synthetic
      polynucleotide"

<400> 513
gaaattgtgc tgacccagag cccatcgtca ctgtccgcat ccgtcggcga ccgcgtgact        60

atcacttgcc aagcgtccca gttcatcaag aaaaatctga actggtacca gcataagccg      120

gggaaggcgc ccaagctgct gatctacgac gcctcatccc tccaaactgg agtgcccagc      180

agattcagcg gaaaccggtc cggaaccacc ttctcgttta ctatttcgag cctgcaaccc      240

gaggatgtgg ccacctacta ctgtcagcag cacgacaact tgcctctcac cttcggtggt      300

ggaaccaaag tggagattaa g                                                321

<210> 514
<211> 357
<212> DNA
<213> Artificial Sequence

<220>
<221> source
<223> /note="Description of Artificial Sequence: Synthetic
      polynucleotide"

<400> 514
gaagtgcagc tggtgcagtc gggtggcgga gtggtccggt ccgggcggtc cctgcgcctg       60

tcgtgcgctg cctccggctt cactttcaac tcatacggac tgcactgggt cagacaggcc      120

ccggggaagg gactggaatg ggtcgcgctc atcgaatacg atgggtccaa caagtattac       180

ggcgacagcg tgaagggccg gttcaccatc tcccgcgaca agtccaagtc aaccctgtac      240

ctccaaatgg ataacctgag ggccgaggac accgccgtgt actactgcgc tcgggaaggg      300

aacgaggacc tggccttcga tatctggggc cagggaaccc tcgtgacggt gtccagc         357

<210> 515
<211> 738
<212> DNA
<213> Artificial Sequence

<220>
<221> source
<223> /note="Description of Artificial Sequence: Synthetic
      polynucleotide"

<400> 515
gaagtgcagc tggtgcagtc gggtggcgga gtggtccggt ccgggcggtc cctgcgcctg       60

tcgtgcgctg cctccggctt cactttcaac tcatacggac tgcactgggt cagacaggcc      120

ccggggaagg gactggaatg ggtcgcgctc atcgaatacg atgggtccaa caagtattac       180

ggcgacagcg tgaagggccg gttcaccatc tcccgcgaca agtccaagtc aaccctgtac      240

ctccaaatgg ataacctgag ggccgaggac accgccgtgt actactgcgc tcgggaaggg      300

aacgaggacc tggccttcga tatctggggc cagggaaccc tcgtgacggt gtccagcggc      360

ggcggtggaa gcggcggtgg cgggagcggg ggaggaggat ctggaggcgg aggctccgaa      420

attgtgctga cccagagccc atcgtcactg tccgcatccg tcggcgaccg cgtgactatc      480

acttgccaag cgtcccagtt catcaagaaa aatctgaact ggtaccagca taagccgggg      540

aaggcgccca agctgctgat ctacgacgcc tcatccctcc aaactggagt gcccagcaga 600

ttcagcggaa accggtccgg aaccaccttc tcgtttacta tttcgagcct gcaacccgag 660

gatgtggcca cctactactg tcagcagcac gacaacttgc ctctcacctt cggtggtgga 720

accaaagtgg agattaag 738

<210> 516
<211> 1440
<212> DNA
<213> Artificial Sequence

<220>
<221> source
<223> /note="Description of Artificial Sequence: Synthetic
      polynucleotide"

<400> 516
gaagtgcagc tggtgcagtc gggtggcgga gtggtccggt ccgggcggtc cctgcgcctg 60

tcgtgcgctg cctccggctt cactttcaac tcatacggac tgcactgggt cagacaggcc 120

ccggggaagg gactggaatg ggtcgcgctc atcgaatacg atgggtccaa caagtattac 180

ggcgacagcg tgaagggccg gttcaccatc tcccgcgaca agtccaagtc aaccctgtac 240

ctccaaatgg ataacctgag ggccgaggac accgccgtgt actactgcgc tcgggaaggg 300

aacgaggacc tggccttcga tatctggggc cagggaaccc tcgtgacggt gtccagcggc 360

ggcggtggaa gcggcggtgg cgggagcggg ggaggaggat ctggaggcgg aggctccgaa 420

attgtgctga cccagagccc atcgtcactg tccgcatccg tcggcgaccg cgtgactatc 480

acttgccaag cgtcccagtt catcaagaaa aatctgaact ggtaccagca taagccgggg 540

aaggcgccca agctgctgat ctacgacgcc tcatccctcc aaactggagt gcccagcaga 600

ttcagcggaa accggtccgg aaccaccttc tcgtttacta tttcgagcct gcaacccgag 660

gatgtggcca cctactactg tcagcagcac gacaacttgc ctctcacctt cggtggtgga 720

accaaagtgg agattaaggg tggcggggga tccggatccg ataagaccca cacctgtcca 780

ccctgccctg cccccgaact gcttggtggt ccgtccgtgt ttctgttccc gcccaagccc 840

aaggacaccc tcatgatctc acggactcct gaagtgacct gtgtggtggt cgctgtgtcc 900

cacgaggacc ccgaagtcaa gttcaattgg tacgtggacg gagtggaagt gcacaacgct 960

aagaccaagc cccgcgagga acagtacaac tccacttacc gcgtcgtgtc ggtgctgacc 1020

gtgctgcatc aggattggct gaacggaaag gagtacaagt gcaaggtgtc caacaaggct 1080

ctggcggcac ccatcgaaaa gaccatcagc aaggccaaag ggcaacctag agaaccacaa 1140

gtctacaccc tgcctccttg ccgggatgag ctcaccaaga accaggtgtc cctgtggtgc 1200

ctcgtgaagg gcttctaccc ctctgacatc gcggtggaat gggagtcaaa cggccagcca 1260

gagaacaact acaagacaac cccccctgtc ctggacagcg acggctcctt cttcctgtac 1320

tcgaagctga ctgtggataa gagccggtgg caacagggca acgtgttctc atgttcggtc 1380

atgcacgagg ccctgcataa ccactacact cagaagtccc tgagcctgtc ccctggaaag 1440

<210> 517
<211> 489
<212> PRT
<213> Artificial Sequence

<220>
<221> source
<223> /note="Description of Artificial Sequence: Synthetic
      polypeptide"

<400> 517
Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Val Ser Ser Asn
            20                  25                  30

Tyr Met Thr Trp Val Arg Gln Ala Pro Gly Lys Gly Leu Glu Trp Val
        35                  40                  45

Ser Val Ile Tyr Ser Gly Gly Ala Thr Tyr Tyr Gly Asp Ser Val Lys
    50                  55                  60

Gly Arg Phe Thr Val Ser Arg Asp Asn Ser Lys Asn Thr Val Tyr Leu
65                  70                  75                  80

Gln Met Asn Arg Leu Thr Ala Glu Asp Thr Ala Val Tyr Tyr Cys Ala
                85                  90                  95

Arg Asp Arg Leu Tyr Cys Gly Asn Asn Cys Tyr Leu Tyr Tyr Tyr Tyr
            100                 105                 110

Gly Met Asp Val Trp Gly Gln Gly Thr Leu Val Thr Val Ser Ser Gly
        115                 120                 125

Gly Gly Gly Ser Gly Gly Gly Gly Ser Gly Gly Gly Gly Ser Gly Gly
    130                 135                 140

Gly Gly Ser Asp Ile Gln Val Thr Gln Ser Pro Ser Ser Leu Ser Ala
145                 150                 155                 160

Ser Val Gly Asp Arg Val Thr Ile Thr Cys Arg Ala Ser Gln Ser Ile
                165                 170                 175

Ser Ser Tyr Leu Asn Trp Tyr Gln Gln Lys Pro Gly Lys Ala Pro Lys

            180                 185                 190

Leu Leu Ile Tyr Ala Ala Ser Ser Leu Gln Ser Gly Val Pro Ser Arg
        195                 200                 205

Phe Ser Gly Ser Gly Ser Gly Thr Asp Phe Thr Leu Thr Ile Ser Ser
    210                 215                 220

Leu Gln Pro Glu Asp Phe Ala Thr Tyr Tyr Cys Gln Gln Ser Tyr Ser
225                 230                 235                 240

Thr Pro Pro Leu Thr Phe Gly Gln Gly Thr Lys Val Glu Ile Lys Gly
                245                 250                 255

Gly Gly Gly Ser Gly Ser Asp Lys Thr His Thr Cys Pro Pro Cys Pro
            260                 265                 270

Ala Pro Glu Leu Leu Gly Gly Pro Ser Val Phe Leu Phe Pro Pro Lys
        275                 280                 285

Pro Lys Asp Thr Leu Met Ile Ser Arg Thr Pro Glu Val Thr Cys Val
    290                 295                 300

Val Val Ala Val Ser His Glu Asp Pro Glu Val Lys Phe Asn Trp Tyr
305                 310                 315                 320

Val Asp Gly Val Glu Val His Asn Ala Lys Thr Lys Pro Arg Glu Glu
                325                 330                 335

Gln Tyr Asn Ser Thr Tyr Arg Val Val Ser Val Leu Thr Val Leu His
            340                 345                 350

Gln Asp Trp Leu Asn Gly Lys Glu Tyr Lys Cys Lys Val Ser Asn Lys
        355                 360                 365

Ala Leu Ala Ala Pro Ile Glu Lys Thr Ile Ser Lys Ala Lys Gly Gln
    370                 375                 380

Pro Arg Glu Pro Gln Val Tyr Thr Leu Pro Pro Cys Arg Asp Glu Leu
385                 390                 395                 400

Thr Lys Asn Gln Val Ser Leu Trp Cys Leu Val Lys Gly Phe Tyr Pro
                405                 410                 415

Ser Asp Ile Ala Val Glu Trp Glu Ser Asn Gly Gln Pro Glu Asn Asn
            420                 425                 430

Tyr Lys Thr Thr Pro Pro Val Leu Asp Ser Asp Gly Ser Phe Phe Leu
        435                 440                 445

Tyr Ser Lys Leu Thr Val Asp Lys Ser Arg Trp Gln Gln Gly Asn Val
    450                 455                 460

Phe Ser Cys Ser Val Met His Glu Ala Leu His Asn His Tyr Thr Gln
465                 470                 475                 480

Lys Ser Leu Ser Leu Ser Pro Gly Lys
                485

<210> 518
<211> 324
<212> DNA
<213> Artificial Sequence

<220>
<221> source
<223> /note="Description of Artificial Sequence: Synthetic
      polynucleotide"

<400> 518
gatattcagg tcacccaatc gccgtcctcc ctgagcgcct ccgtggggga tcgcgtgacg      60

attacttgcc gggccagcca gagcatctcc tcgtacctga actggtacca gcagaagccg     120

ggaaaggccc ccaagctgct gatctacgct gcatcaagcc tgcagtccgg cgtgcctagc     180

cggttttccg gttccggttc gggtaccgac ttcacactga ccatctcctc actgcaacca     240

gaggatttcg ccacctacta ctgtcagcag tcatactcca ctccgcccct gaccttcgga     300

caagggacca aagtggaaat caag                                            324

<210> 519
<211> 381
<212> DNA
<213> Artificial Sequence

<220>
<221> source
<223> /note="Description of Artificial Sequence: Synthetic
      polynucleotide"

<400> 519
caagtgcagc tggtcgaatc cgggggaggc ctggtgcagc ccggagggtc gctgaggctg      60

agctgcgcgg cttccggctt caatgtgtcc tccaactaca tgacctgggt cagacaggcc     120

cctggaaagg gactcgaatg ggtgtcggtg atctactccg gtggcgcaac ctactatgga     180

gacagcgtga aggggcgctt cactgtgtcc cgcgacaact ccaagaacac tgtgtacctt     240

cagatgaaca ggctcaccgc cgaggacacc gccgtgtact actgcgcgcg ggaccggctc     300

tactgtggaa acaactgcta tctgtactac tactacggga tggacgtctg gggccagggc     360

accctcgtga ctgtgtcgtc t                                              381

<210> 520
<211> 765
<212> DNA
<213> Artificial Sequence

<220>
<221> source
<223> /note="Description of Artificial Sequence: Synthetic
      polynucleotide"

<400> 520
caagtgcagc tggtcgaatc cgggggaggc ctggtgcagc ccggagggtc gctgaggctg      60

agctgcgcgg cttccggctt caatgtgtcc tccaactaca tgacctgggt cagacaggcc     120

cctggaaagg gactcgaatg ggtgtcggtg atctactccg gtggcgcaac ctactatgga     180

gacagcgtga aggggcgctt cactgtgtcc cgcgacaact ccaagaacac tgtgtacctt     240

cagatgaaca ggctcaccgc cgaggacacc gccgtgtact actgcgcgcg ggaccggctc     300

tactgtggaa acaactgcta tctgtactac tactacggga tggacgtctg gggccagggc     360

accctcgtga ctgtgtcgtc tggaggaggc ggtagcggtg gaggcggctc cggaggcgga     420

ggctcgggag ggggaggcag cgatattcag gtcacccaat cgccgtcctc cctgagcgcc     480

tccgtggggg atcgcgtgac gattacttgc cgggccagcc agagcatctc ctcgtacctg     540

aactggtacc agcagaagcc gggaaaggcc cccaagctgc tgatctacgc tgcatcaagc     600

ctgcagtccg gcgtgcctag ccggttttcc ggttccggtt cgggtaccga cttcacactg     660

accatctcct cactgcaacc agaggatttc gccacctact actgtcagca gtcatactcc     720

actccgcccc tgaccttcgg acaagggacc aaagtggaaa tcaag                    765

<210> 521
<211> 1467
<212> DNA
<213> Artificial Sequence

<220>
<221> source
<223> /note="Description of Artificial Sequence: Synthetic
      polynucleotide"

<400> 521
caagtgcagc tggtcgaatc cgggggaggc ctggtgcagc ccggagggtc gctgaggctg      60

agctgcgcgg cttccggctt caatgtgtcc tccaactaca tgacctgggt cagacaggcc     120

cctggaaagg gactcgaatg ggtgtcggtg atctactccg gtggcgcaac ctactatgga     180

gacagcgtga aggggcgctt cactgtgtcc cgcgacaact ccaagaacac tgtgtacctt     240

cagatgaaca ggctcaccgc cgaggacacc gccgtgtact actgcgcgcg ggaccggctc     300

tactgtggaa acaactgcta tctgtactac tactacggga tggacgtctg gggccagggc     360

accctcgtga ctgtgtcgtc tggaggaggc ggtagcggtg gaggcggctc cggaggcgga 420

ggctcgggag ggggaggcag cgatattcag gtcacccaat cgccgtcctc cctgagcgcc 480

tccgtggggg atcgcgtgac gattacttgc cgggccagcc agagcatctc ctcgtacctg 540

aactggtacc agcagaagcc gggaaaggcc cccaagctgc tgatctacgc tgcatcaagc 600

ctgcagtccg gcgtgcctag ccggttttcc ggttccggtt cgggtaccga cttcacactg 660

accatctcct cactgcaacc agaggatttc gccacctact actgtcagca gtcatactcc 720

actccgcccc tgaccttcgg acaagggacc aaagtggaaa tcaagggcgg cggaggatcc 780

ggatccgata agacccacac ctgtccaccc tgccctgccc ccgaactgct tggtggtccg 840

tccgtgtttc tgttcccgcc caagcccaag gacaccctca tgatctcacg gactcctgaa 900

gtgacctgtg tggtggtcgc tgtgtcccac gaggaccccg aagtcaagtt caattggtac 960

gtggacggag tggaagtgca caacgctaag accaagcccc gcgaggaaca gtacaactcc 1020

acttaccgcg tcgtgtcggt gctgaccgtg ctgcatcagg attggctgaa cggaaaggag 1080

tacaagtgca aggtgtccaa caaggctctg gcggcaccca tcgaaaagac catcagcaag 1140

gccaaagggc aacctagaga accacaagtc tacaccctgc ctccttgccg ggatgagctc 1200

accaagaacc aggtgtccct gtggtgcctc gtgaagggct tctacccctc tgacatcgcg 1260

gtggaatggg agtcaaacgg ccagccagag aacaactaca agacaacccc ccctgtcctg 1320

gacagcgacg gctccttctt cctgtactcg aagctgactg tggataagag ccggtggcaa 1380

cagggcaacg tgttctcatg ttcggtcatg cacgaggccc tgcataacca ctacactcag 1440

aagtccctga gcctgtcccc tggaaag 1467

<210> 522
<211> 482
<212> PRT
<213> Artificial Sequence

<220>
<221> source
<223> /note="Description of Artificial Sequence: Synthetic
      polypeptide"

<400> 522
Gln Val Gln Leu Val Gln Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Tyr Ile Ser Ser Ser Ser Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Ser Val Arg Ala Ile Asp Ala Phe Asp Ile Trp Gly
            100                 105                 110

Gln Gly Thr Met Val Thr Val Ser Ser Gly Gly Gly Gly Ser Gly Gly
        115                 120                 125

Gly Gly Ser Gly Gly Gly Gly Ser Gly Gly Gly Gly Ser Asp Ile Val
    130                 135                 140

Leu Thr Gln Ser Pro Ser Ser Leu Ser Ala Ser Val Gly Asp Arg Val
145                 150                 155                 160

Thr Ile Thr Cys Gln Ala Ser Gln Asp Ile Ser Asn Tyr Leu Asn Trp
                165                 170                 175

Tyr Gln Gln Lys Pro Gly Lys Ala Pro Lys Leu Leu Ile Tyr Asp Ala
            180                 185                 190

Ser Asn Leu Glu Thr Gly Val Pro Ser Arg Phe Ser Gly Ser Gly Ser
        195                 200                 205

Gly Thr Asp Phe Thr Phe Thr Ile Ser Ser Leu Gln Pro Glu Asp Phe
    210                 215                 220

Ala Thr Tyr Tyr Cys Gln Gln Ala Tyr Ser Thr Pro Phe Thr Phe Gly
225                 230                 235                 240

Pro Gly Thr Lys Val Glu Ile Lys Gly Gly Gly Gly Ser Gly Ser Asp
                245                 250                 255

Lys Thr His Thr Cys Pro Pro Cys Pro Ala Pro Glu Leu Leu Gly Gly
            260                 265                 270

Pro Ser Val Phe Leu Phe Pro Pro Lys Pro Lys Asp Thr Leu Met Ile
        275                 280                 285

Ser Arg Thr Pro Glu Val Thr Cys Val Val Val Ala Val Ser His Glu
    290                 295                 300

Asp Pro Glu Val Lys Phe Asn Trp Tyr Val Asp Gly Val Glu Val His
305                 310                 315                 320

Asn Ala Lys Thr Lys Pro Arg Glu Glu Gln Tyr Asn Ser Thr Tyr Arg
                325                 330                 335

Val Val Ser Val Leu Thr Val Leu His Gln Asp Trp Leu Asn Gly Lys
            340                 345                 350

Glu Tyr Lys Cys Lys Val Ser Asn Lys Ala Leu Ala Ala Pro Ile Glu
        355                 360                 365

Lys Thr Ile Ser Lys Ala Lys Gly Gln Pro Arg Glu Pro Gln Val Tyr
    370                 375                 380

Thr Leu Pro Pro Cys Arg Asp Glu Leu Thr Lys Asn Gln Val Ser Leu
385                 390                 395                 400

Trp Cys Leu Val Lys Gly Phe Tyr Pro Ser Asp Ile Ala Val Glu Trp
                405                 410                 415

Glu Ser Asn Gly Gln Pro Glu Asn Asn Tyr Lys Thr Thr Pro Pro Val
            420                 425                 430

Leu Asp Ser Asp Gly Ser Phe Phe Leu Tyr Ser Lys Leu Thr Val Asp
        435                 440                 445

Lys Ser Arg Trp Gln Gln Gly Asn Val Phe Ser Cys Ser Val Met His
    450                 455                 460

Glu Ala Leu His Asn His Tyr Thr Gln Lys Ser Leu Ser Leu Ser Pro
465                 470                 475                 480

Gly Lys

<210> 523
<211> 321
<212> DNA
<213> Artificial Sequence

<220>
<221> source
<223> /note="Description of Artificial Sequence: Synthetic
      polynucleotide"

<400> 523
gacatcgtgt tgacccagtc tccttcgtcc ctgagcgctt ccgtgggcga ccgcgtgacc       60

atcacttgcc aagcctcaca agatatctcc aactacctca attggtacca gcagaagccg      120

ggaaaggccc ccaagctgct catctacgac gcctccaacc tggaaactgg agtcccgtcg 180

aggttttccg gaagcggcag cggaaccgac ttcaccttca ccattagcag cctgcagcca 240

gaggattttg cgacctatta ctgccagcag gcttactcca cccccttcac cttcggacct 300

ggcaccaagg tcgaaatcaa g 321

<210> 524
<211> 363
<212> DNA
<213> Artificial Sequence

<220>
<221> source
<223> /note="Description of Artificial Sequence: Synthetic
      polynucleotide"

<400> 524
caagtgcagc tggtgcagtc cggcggaggg ctggtgcagc cgggcggttc gctgagactg 60

tcctgtgccg cgtcgggctt cacgttctcg tcatactcca tgaactgggt ccgccaggcc 120

cccggaaaag gcttggaatg ggtgtcgtac atttcgtcct cctcctcaac catctactac 180

gccgactcag tgaaggggcg gttcactatt tcccgggaca acgccaagaa cagcctgtac 240

ctccaaatga actcactgcg ggccgaggac actgcggtgt actactgcgc ccgggacctg 300

tccgtgagag caattgacgc attcgatatc tggggacagg gaaccatggt caccgtgtcc 360

agc 363

<210> 525
<211> 744
<212> DNA
<213> Artificial Sequence

<220>
<221> source
<223> /note="Description of Artificial Sequence: Synthetic
      polynucleotide"

<400> 525
caagtgcagc tggtgcagtc cggcggaggg ctggtgcagc cgggcggttc gctgagactg 60

tcctgtgccg cgtcgggctt cacgttctcg tcatactcca tgaactgggt ccgccaggcc 120

cccggaaaag gcttggaatg ggtgtcgtac atttcgtcct cctcctcaac catctactac 180

gccgactcag tgaaggggcg gttcactatt tcccgggaca acgccaagaa cagcctgtac 240

ctccaaatga actcactgcg ggccgaggac actgcggtgt actactgcgc ccgggacctg 300

tccgtgagag caattgacgc attcgatatc tggggacagg gaaccatggt caccgtgtcc 360

agcggtggag gagggtcggg cggcggcggt tcaggcggtg gtggaagcgg cggggggggg 420

tccgacatcg tgttgaccca gtctccttcg tccctgagcg cttccgtggg cgaccgcgtg 480

accatcactt gccaagcctc acaagatatc tccaactacc tcaattggta ccagcagaag 540

ccgggaaagg cccccaagct gctcatctac gacgcctcca acctggaaac tggagtcccg 600

tcgaggtttt ccggaagcgg cagcggaacc gacttcacct tcaccattag cagcctgcag 660

ccagaggatt ttgcgaccta ttactgccag caggcttact ccaccccctt caccttcgga 720

cctggcacca aggtcgaaat caag 744

<210> 526
<211> 1446
<212> DNA
<213> Artificial Sequence

<220>
<221> source
<223> /note="Description of Artificial Sequence: Synthetic
      polynucleotide"

<400> 526
caagtgcagc tggtgcagtc cggcggaggg ctggtgcagc cgggcggttc gctgagactg 60

tcctgtgccg cgtcgggctt cacgttctcg tcatactcca tgaactgggt ccgccaggcc 120

cccggaaaag gcttggaatg ggtgtcgtac atttcgtcct cctcctcaac catctactac 180

gccgactcag tgaaggggcg gttcactatt tcccgggaca acgccaagaa cagcctgtac 240

ctccaaatga actcactgcg ggccgaggac actgcggtgt actactgcgc ccgggacctg 300

tccgtgagag caattgacgc attcgatatc tggggacagg gaaccatggt caccgtgtcc 360

agcggtggag gagggtcggg cggcggcggt tcaggcggtg gtggaagcgg cggggggggg 420

tccgacatcg tgttgaccca gtctccttcg tccctgagcg cttccgtggg cgaccgcgtg 480

accatcactt gccaagcctc acaagatatc tccaactacc tcaattggta ccagcagaag 540

ccgggaaagg cccccaagct gctcatctac gacgcctcca acctggaaac tggagtcccg 600

tcgaggtttt ccggaagcgg cagcggaacc gacttcacct tcaccattag cagcctgcag 660

ccagaggatt ttgcgaccta ttactgccag caggcttact ccaccccctt caccttcgga 720

cctggcacca aggtcgaaat caagggcgga gggggatccg gatccgataa gacccacacc 780

tgtccaccct gccctgcccc cgaactgctt ggtggtccgt ccgtgtttct gttcccgccc 840

aagcccaagg acaccctcat gatctcacgg actcctgaag tgacctgtgt ggtggtcgct 900

gtgtcccacg aggaccccga agtcaagttc aattggtacg tggacggagt ggaagtgcac 960

aacgctaaga ccaagccccg cgaggaacag tacaactcca cttaccgcgt cgtgtcggtg 1020

ctgaccgtgc tgcatcagga ttggctgaac ggaaaggagt acaagtgcaa ggtgtccaac 1080

aaggctctgg cggcacccat cgaaaagacc atcagcaagg ccaaagggca acctagagaa 1140

ccacaagtct acaccctgcc tccttgccgg gatgagctca ccaagaacca ggtgtccctg 1200

tggtgcctcg tgaagggctt ctacccctct gacatcgcgg tggaatggga gtcaaacggc 1260

cagccagaga acaactacaa gacaaccccc cctgtcctgg acagcgacgg ctccttcttc 1320

ctgtactcga agctgactgt ggataagagc cggtggcaac agggcaacgt gttctcatgt 1380

tcggtcatgc acgaggccct gcataaccac tacactcaga agtccctgag cctgtcccct 1440

ggaaag 1446

<210> 527
<211> 473
<212> PRT
<213> Artificial Sequence

<220>
<221> source
<223> /note="Description of Artificial Sequence: Synthetic
      polypeptide"

<400> 527
Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Asn Glu Asp Gly Ser Ala Lys Phe Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Phe Cys
                85                  90                  95

Ala Arg Asp Leu Arg Ser Gly Arg Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Gly Arg Ala Thr Leu Ser Cys Arg Ala Ser Gln Ser Ile
145                 150                 155                 160

Ser Gly Ser Phe Leu Ala Trp Tyr Gln Gln Lys Pro Gly Gln Ala Pro
                165                 170                 175

Arg Leu Leu Ile Tyr Gly Ala Ser Ser Arg Ala Thr Gly Ile Pro Asp
        180                 185                 190

Arg Phe Ser Gly Ser Gly Ser Gly Thr Asp Phe Thr Leu Thr Ile Ser
    195                 200                 205

Arg Leu Glu Pro Glu Asp Phe Ala Val Tyr Tyr Cys Gln Gln Tyr Gly
    210                 215                 220

Ser Ser Pro Pro Thr Phe Gly Leu Gly Thr Lys Leu Glu Ile Lys Gly
225                 230                 235                 240

Gly Gly Gly Ser Gly Ser Asp Lys Thr His Thr Cys Pro Pro Cys Pro
                245                 250                 255

Ala Pro Glu Leu Leu Gly Gly Pro Ser Val Phe Leu Phe Pro Pro Lys
            260                 265                 270

Pro Lys Asp Thr Leu Met Ile Ser Arg Thr Pro Glu Val Thr Cys Val
        275                 280                 285

Val Val Ala Val Ser His Glu Asp Pro Glu Val Lys Phe Asn Trp Tyr
    290                 295                 300

Val Asp Gly Val Glu Val His Asn Ala Lys Thr Lys Pro Arg Glu Glu
305                 310                 315                 320

Gln Tyr Asn Ser Thr Tyr Arg Val Val Ser Val Leu Thr Val Leu His
                325                 330                 335

Gln Asp Trp Leu Asn Gly Lys Glu Tyr Lys Cys Lys Val Ser Asn Lys
            340                 345                 350

Ala Leu Ala Ala Pro Ile Glu Lys Thr Ile Ser Lys Ala Lys Gly Gln
        355                 360                 365

Pro Arg Glu Pro Gln Val Tyr Thr Leu Pro Pro Cys Arg Asp Glu Leu
    370                 375                 380

Thr Lys Asn Gln Val Ser Leu Trp Cys Leu Val Lys Gly Phe Tyr Pro
385                 390                 395                 400

Ser Asp Ile Ala Val Glu Trp Glu Ser Asn Gly Gln Pro Glu Asn Asn
                405                 410                 415

Tyr Lys Thr Thr Pro Pro Val Leu Asp Ser Asp Gly Ser Phe Phe Leu

420                 425                 430

Tyr Ser Lys Leu Thr Val Asp Lys Ser Arg Trp Gln Gln Gly Asn Val
        435                 440                 445

Phe Ser Cys Ser Val Met His Glu Ala Leu His Asn His Tyr Thr Gln
    450                 455                 460

Lys Ser Leu Ser Leu Ser Pro Gly Lys
465                 470

<210> 528
<211> 324
<212> DNA
<213> Artificial Sequence

<220>
<221> source
<223> /note="Description of Artificial Sequence: Synthetic
      polynucleotide"

<400> 528
gaaattgtgc tgacccaatc gcccggaact ctgtccctgt cccccggtgg acgcgccact      60

ctctcttgcc gggcctcaca gtcgatctcg ggcagctttc tcgcctggta ccagcagaag     120

ccgggacagg cgcctcgcct gctgatctac ggagcgtcca gcagagccac cggaatccca     180

gacagattct ccggctcggg ctccggtacc gactttacgc tgactattag ccggctggag     240

ccggaggact tcgccgtgta ctactgtcag cagtacggca gctcaccgcc taccttcgga     300

ctcgggacaa agctggaaat caag                                            324

<210> 529
<211> 345
<212> DNA
<213> Artificial Sequence

<220>
<221> source
<223> /note="Description of Artificial Sequence: Synthetic
      polynucleotide"

<400> 529
caagtgcagc tggtcgaatc cggcggaggg ctggtgcagc cgggagggtc actccggctg      60

tcctgcgccg catcaggatt caccttctcc tcctactgga tgtcctgggt ccgccaggct     120

cccgggaagg gtctggaatg ggtggccaac atcaacgagg acggctccgc caagttctac     180

gtggatagcg tgaaaggaag gttcaccatt tcccgggaca acgccaagaa cagcctctat     240

ctgcaaatga atagcctgag ggcagaagat accgcggtgt acttctgcgc tcgggacctg     300

agatccggcc gctactgggg ccaggggacc ctggtcaccg tgtcc                     345

<210> 530
<211> 717
<212> DNA
<213> Artificial Sequence

<220>
<221> source
<223> /note="Description of Artificial Sequence: Synthetic
      polynucleotide"

<400> 530
caagtgcagc tggtcgaatc cggcggaggg ctggtgcagc cgggagggtc actccggctg      60

tcctgcgccg catcaggatt caccttctcc tcctactgga tgtcctgggt ccgccaggct     120

cccgggaagg gtctggaatg ggtggccaac atcaacgagg acggctccgc caagttctac     180

gtggatagcg tgaaaggaag gttcaccatt tcccgggaca acgccaagaa cagcctctat     240

ctgcaaatga atagcctgag ggcagaagat accgcggtgt acttctgcgc tcgggacctg     300

agatccggcc gctactgggg ccaggggacc ctggtcaccg tgtcctcggg agggggcggc     360

tccggtggtg gagggagcgg cggaggaggg tccgaaattg tgctgaccca atcgcccgga     420

actctgtccc tgtcccccgg tggacgcgcc actctctctt gccgggcctc acagtcgatc     480

tcgggcagct ttctcgcctg gtaccagcag aagccgggac aggcgcctcg cctgctgatc     540

tacggagcgt ccagcagagc caccggaatc ccagacagat tctccggctc gggctccggt     600

accgacttta cgctgactat tagccggctg gagccggagg acttcgccgt gtactactgt     660

cagcagtacg gcagctcacc gcctaccttc ggactcggga caaagctgga aatcaag        717


<210> 531
<211> 1419
<212> DNA
<213> Artificial Sequence

<220>
<221> source
<223> /note="Description of Artificial Sequence: Synthetic
      polynucleotide"

<400> 531
caagtgcagc tggtcgaatc cggcggaggg ctggtgcagc cgggagggtc actccggctg      60

tcctgcgccg catcaggatt caccttctcc tcctactgga tgtcctgggt ccgccaggct     120

cccgggaagg gtctggaatg ggtggccaac atcaacgagg acggctccgc caagttctac     180

gtggatagcg tgaaaggaag gttcaccatt tcccgggaca acgccaagaa cagcctctat     240

ctgcaaatga atagcctgag ggcagaagat accgcggtgt acttctgcgc tcgggacctg     300

agatccggcc gctactgggg ccaggggacc ctggtcaccg tgtcctcggg agggggcggc     360

tccggtggtg gagggagcgg cggaggaggg tccgaaattg tgctgaccca atcgcccgga     420

actctgtccc tgtcccccgg tggacgcgcc actctctctt gccgggcctc acagtcgatc     480

tcgggcagct ttctcgcctg gtaccagcag aagccgggac aggcgcctcg cctgctgatc 540

tacggagcgt ccagcagagc caccggaatc ccagacagat tctccggctc gggctccggt 600

accgacttta cgctgactat tagccggctg gagccggagg acttcgccgt gtactactgt 660

cagcagtacg gcagctcacc gcctaccttc ggactcggga caaagctgga aatcaaggga 720

ggcggcggat ccggatccga taagacccac acctgtccac cctgccctgc ccccgaactg 780

cttggtggtc cgtccgtgtt tctgttcccg cccaagccca aggacaccct catgatctca 840

cggactcctg aagtgacctg tgtggtggtc gctgtgtccc acgaggaccc cgaagtcaag 900

ttcaattggt acgtggacgg agtggaagtg cacaacgcta agaccaagcc ccgcgaggaa 960

cagtacaact ccacttaccg cgtcgtgtcg gtgctgaccg tgctgcatca ggattggctg 1020

aacggaaagg agtacaagtg caaggtgtcc aacaaggctc tggcggcacc catcgaaaag 1080

accatcagca aggccaaagg gcaacctaga gaaccacaag tctacaccct gcctccttgc 1140

cgggatgagc tcaccaagaa ccaggtgtcc ctgtggtgcc tcgtgaaggg cttctacccc 1200

tctgacatcg cggtggaatg ggagtcaaac ggccagccag agaacaacta caagacaacc 1260

ccccctgtcc tggacagcga cggctccttc ttcctgtact cgaagctgac tgtggataag 1320

agccggtggc aacagggcaa cgtgttctca tgttcggtca tgcacgaggc cctgcataac 1380

cactacactc agaagtccct gagcctgtcc cctggaaag 1419

<210> 532
<211> 480
<212> PRT
<213> Artificial Sequence

<220>
<221> source
<223> /note="Description of Artificial Sequence: Synthetic
      polypeptide"

<400> 532
   Gln Val Gln Leu Val Gln Ser Gly Ala Glu Val Lys Glu Pro Gly Ala
   1               5                   10                  15
   Ser Val Lys Val Ser Cys Lys Ala Pro Ala Asn Thr Phe Ser Asp His
               20                  25                  30
   Val Met His Trp Val Arg Gln Ala Pro Gly Gln Arg Phe Glu Trp Met
           35                  40                  45
   Gly Tyr Ile His Ala Ala Asn Gly Gly Thr His Tyr Ser Gln Lys Phe
       50                  55                  60
   Gln Asp Arg Val Thr Ile Thr Arg Asp Thr Ser Ala Asn Thr Val Tyr
   65                  70                  75                  80
   Met Asp Leu Ser Ser Leu Arg Ser Glu Asp Thr Ala Val Tyr Tyr Cys
                   85                  90                  95
   Ala Arg Gly Gly Tyr Asn Ser Asp Ala Phe Asp Ile Trp Gly Gln Gly
               100                 105                 110
   Thr Met Val Thr Val Ser Ser Gly Gly Gly Gly Ser Gly Gly Gly Gly
           115                 120                 125
   Ser Gly Gly Gly Gly Ser Gly Gly Gly Gly Ser Asp Ile Val Met Thr
       130                 135                 140
   Gln Ser Pro Ser Ser Val Ser Ala Ser Val Gly Asp Arg Val Thr Ile
   145                 150                 155                 160
   Thr Cys Arg Ala Ser Gln Asp Ile Ser Ser Trp Leu Ala Trp Tyr Gln
                   165                 170                 175
   Gln Lys Pro Gly Lys Ala Pro Lys Leu Leu Ile Tyr Ala Ala Ser Ser
               180                 185                 190
   Leu Gln Ser Gly Val Pro Ser Arg Phe Asn Gly Ser Gly Ser Gly Thr
           195                 200                 205
   Asp Phe Thr Leu Thr Ile Ser Ser Leu Gln Pro Glu Asp Phe Ala Thr
       210                 215                 220
   Tyr Tyr Cys Gln Gln Ser Tyr Ser Thr Pro Leu Thr Phe Gly Gly Gly
   225                 230                 235                 240
   Thr Lys Val Glu Ile Lys Gly Gly Gly Gly Ser Gly Ser Asp Lys Thr
                   245                 250                 255
   His Thr Cys Pro Pro Cys Pro Ala Pro Glu Leu Leu Gly Gly Pro Ser
               260                 265                 270
   Val Phe Leu Phe Pro Pro Lys Pro Lys Asp Thr Leu Met Ile Ser Arg
           275                 280                 285
   Thr Pro Glu Val Thr Cys Val Val Val Ala Val Ser His Glu Asp Pro
       290                 295                 300
   Glu Val Lys Phe Asn Trp Tyr Val Asp Gly Val Glu Val His Asn Ala
   305                 310                 315                 320
   Lys Thr Lys Pro Arg Glu Glu Gln Tyr Asn Ser Thr Tyr Arg Val Val
                   325                 330                 335
   Ser Val Leu Thr Val Leu His Gln Asp Trp Leu Asn Gly Lys Glu Tyr
               340                 345                 350
   Lys Cys Lys Val Ser Asn Lys Ala Leu Ala Ala Pro Ile Glu Lys Thr
           355                 360                 365
   Ile Ser Lys Ala Lys Gly Gln Pro Arg Glu Pro Gln Val Tyr Thr Leu
       370                 375                 380
   Pro Pro Cys Arg Asp Glu Leu Thr Lys Asn Gln Val Ser Leu Trp Cys
   385                 390                 395                 400
   Leu Val Lys Gly Phe Tyr Pro Ser Asp Ile Ala Val Glu Trp Glu Ser
                   405                 410                 415
   Asn Gly Gln Pro Glu Asn Asn Tyr Lys Thr Thr Pro Pro Val Leu Asp
               420                 425                 430
   Ser Asp Gly Ser Phe Phe Leu Tyr Ser Lys Leu Thr Val Asp Lys Ser
           435                 440                 445
   Arg Trp Gln Gln Gly Asn Val Phe Ser Cys Ser Val Met His Glu Ala
       450                 455                 460
   Leu His Asn His Tyr Thr Gln Lys Ser Leu Ser Leu Ser Pro Gly Lys
   465                 470                 475                 480

<210> 533
<211> 321
<212> DNA
<213> Artificial Sequence

<220>
<221> source
<223> /note="Description of Artificial Sequence: Synthetic
      polynucleotide"

<400> 533
gatatcgtga tgactcagtc cccctcgtcc gtgagcgcgt ccgtgggcga cagagtgacc      60

attacgtgcc gcgccagcca agacatttct tcctggctcg cctggtacca gcagaagcct     120

ggaaaggctc cgaagctgct gatctacgcc gcctcatcgc tccaatccgg agtgccatcg     180

cggttcaatg gctcggggtc cggaactgac tttaccctga ctattagcag cctgcagcct     240

gaggacttcg ctacctatta ctgccaacag tcctactcca ccccgctgac cttcgggggt     300

ggtaccaagg tcgaaatcaa g                                               321

<210> 534

<211> 357
<212> DNA
<213> Artificial Sequence

<220>
<221> source
<223> /note="Description of Artificial Sequence: Synthetic
      polynucleotide"

<400> 534
caagtgcagc tggtgcagtc cggagcagaa gtcaaggaac cgggagccag cgtgaaggtg      60

tcctgtaaag cccccgcaaa cactttcagc gatcacgtca tgcactgggt ccggcaggcc     120

cccggccaac gcttcgaatg gatggggtac atccatgctg ccaacggcgg aacccactac     180

agccagaagt ttcaggaccg cgtgacgatc accagggaca catccgcgaa cactgtgtac     240

atggacctgt catccctgag atcggaggac accgcagtgt actactgcgc ccggggggga     300

tacaactccg atgcgttcga catctggggc cagggaacca tggtcaccgt gtcatcc        357


<210> 535
<211> 738
<212> DNA
<213> Artificial Sequence

<220>
<221> source
<223> /note="Description of Artificial Sequence: Synthetic
      polynucleotide"

<400> 535
caagtgcagc tggtgcagtc cggagcagaa gtcaaggaac cgggagccag cgtgaaggtg      60

tcctgtaaag cccccgcaaa cactttcagc gatcacgtca tgcactgggt ccggcaggcc     120

cccggccaac gcttcgaatg gatggggtac atccatgctg ccaacggcgg aacccactac     180

agccagaagt ttcaggaccg cgtgacgatc accagggaca catccgcgaa cactgtgtac     240

atggacctgt catccctgag atcggaggac accgcagtgt actactgcgc ccggggggga     300

tacaactccg atgcgttcga catctggggc cagggaacca tggtcaccgt gtcatccggt     360

ggaggcggct cgggtggcgg aggatcagga ggaggaggca gcgggggcgg aggttccgat     420

atcgtgatga ctcagtcccc ctcgtccgtg agcgcgtccg tgggcgacag agtgaccatt     480

acgtgccgcg ccagccaaga catttcttcc tggctcgcct ggtaccagca gaagcctgga     540

aaggctccga agctgctgat ctacgccgcc tcatcgctcc aatccggagt gccatcgcgg     600

ttcaatggct cggggtccgg aactgacttt accctgacta ttagcagcct gcagcctgag     660

gacttcgcta cctattactg ccaacagtcc tactccaccc cgctgacctt cgggggtggt      720

accaaggtcg aaatcaag                                                   738


<210> 536
<211> 1440

<212> DNA
<213> Artificial Sequence

<220>
<221> source
<223> /note="Description of Artificial Sequence: Synthetic
      polynucleotide"

<400> 536
   caagtgcagc tggtgcagtc cggagcagaa gtcaaggaac cgggagccag cgtgaaggtg        60
   tcctgtaaag cccccgcaaa cactttcagc gatcacgtca tgcactgggt ccggcaggcc       120
   cccggccaac gcttcgaatg gatggggtac atccatgctg ccaacggcgg aacccactac       180
   agccagaagt ttcaggaccg cgtgacgatc accagggaca catccgcgaa cactgtgtac       240
   atggacctgt catccctgag atcggaggac accgcagtgt actactgcgc ccggggggga       300
   tacaactccg atgcgttcga catctggggc cagggaacca tggtcaccgt gtcatccggt       360
   ggaggcggct cgggtggcgg aggatcagga ggaggaggca gcgggggcgg aggttccgat       420
   atcgtgatga ctcagtcccc ctcgtccgtg agcgcgtccg tgggcgacag agtgaccatt       480
   acgtgccgcg ccagccaaga catttcttcc tggctcgcct ggtaccagca gaagcctgga       540
   aaggctccga agctgctgat ctacgccgcc tcatcgctcc aatccggagt gccatcgcgg       600
   ttcaatggct cggggtccgg aactgacttt accctgacta ttagcagcct gcagcctgag       660
   gacttcgcta cctattactg ccaacagtcc tactccaccc cgctgacctt cgggggtggt        720
   accaaggtcg aaatcaaggg aggggcggga tccggatccg ataagaccca cacctgtcca        780
   ccctgccctg cccccgaact gcttggtggt ccgtccgtgt ttctgttccc gcccaagccc       840
   aaggacaccc tcatgatctc acggactcct gaagtgacct gtgtggtggt cgctgtgtcc       900
   cacgaggacc ccgaagtcaa gttcaattgg tacgtggacg gagtggaagt gcacaacgct       960
   aagaccaagc cccgcgagga acagtacaac tccacttacc gcgtcgtgtc ggtgctgacc      1020
   gtgctgcatc aggattggct gaacggaaag gagtacaagt gcaaggtgtc caacaaggct      1080
   ctggcggcac ccatcgaaaa gaccatcagc aaggccaaag ggcaacctag agaaccacaa      1140
   gtctacaccc tgcctccttg ccgggatgag ctcaccaaga accaggtgtc cctgtggtgc      1200
   ctcgtgaagg gcttctaccc ctctgacatc gcggtggaat gggagtcaaa cggccagcca      1260
   gagaacaact acaagacaac cccccctgtc ctggacagcg acggctcctt cttcctgtac       1320
   tcgaagctga ctgtggataa gagccggtgg caacagggca acgtgttctc atgttcggtc      1380
   atgcacgagg ccctgcataa ccactacact cagaagtccc tgagcctgtc ccctggaaag      1440

<210> 537
<211> 489
<212> PRT
<213> Artificial Sequence

<220>
<221> source
<223> /note="Description of Artificial Sequence: Synthetic
      polypeptide"

<400> 537
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Pro Ser Ser Ser Gly Ser Tyr Tyr Met Glu Asp Ser Tyr
            100                 105                 110

Tyr Tyr Gly Met Asp Val Trp Gly Gln Gly Thr Thr Val Thr Val Ser
        115                 120                 125

Ser Gly Gly Gly Gly Ser Gly Gly Gly Gly Ser Gly Gly Gly Gly Ser
    130                 135                 140

Asn Phe Met Leu Thr Gln Pro His Ser Val Ser Glu Ser Pro Gly Lys
145                 150                 155                 160

Thr Val Thr Ile Ser Cys Thr Gly Ser Ser Gly Ser Ile Ala Ser Asn
                165                 170                 175

Tyr Val Gln Trp Tyr Gln Gln Arg Pro Gly Ser Ala Pro Thr Thr Val
            180                 185                 190

Ile Tyr Glu Asp Asn Gln Arg Pro Ser Gly Val Pro Asp Arg Phe Ser
        195                 200                 205

Gly Ser Ile Asp Ser Ser Ser Asn Ser Ala Ser Leu Thr Ile Ser Gly
    210                 215                 220

Leu Lys Thr Glu Asp Glu Ala Asp Tyr Tyr Cys Gln Ser Tyr Asp Ser

225                 230                 235                 240

Ser Asn Gln Val Val Phe Gly Gly Gly Thr Lys Leu Thr Val Leu Gly
                245                 250                 255

Gly Gly Gly Ser Gly Ser Asp Lys Thr His Thr Cys Pro Pro Cys Pro
            260                 265                 270

Ala Pro Glu Leu Leu Gly Gly Pro Ser Val Phe Leu Phe Pro Pro Lys
        275                 280                 285

Pro Lys Asp Thr Leu Met Ile Ser Arg Thr Pro Glu Val Thr Cys Val
    290                 295                 300

Val Val Ala Val Ser His Glu Asp Pro Glu Val Lys Phe Asn Trp Tyr
305                 310                 315                 320

Val Asp Gly Val Glu Val His Asn Ala Lys Thr Lys Pro Arg Glu Glu
                325                 330                 335

Gln Tyr Asn Ser Thr Tyr Arg Val Val Ser Val Leu Thr Val Leu His
            340                 345                 350

Gln Asp Trp Leu Asn Gly Lys Glu Tyr Lys Cys Lys Val Ser Asn Lys
        355                 360                 365

Ala Leu Ala Ala Pro Ile Glu Lys Thr Ile Ser Lys Ala Lys Gly Gln
    370                 375                 380

Pro Arg Glu Pro Gln Val Tyr Thr Leu Pro Pro Cys Arg Asp Glu Leu
385                 390                 395                 400

Thr Lys Asn Gln Val Ser Leu Trp Cys Leu Val Lys Gly Phe Tyr Pro
                405                 410                 415

Ser Asp Ile Ala Val Glu Trp Glu Ser Asn Gly Gln Pro Glu Asn Asn
            420                 425                 430

Tyr Lys Thr Thr Pro Pro Val Leu Asp Ser Asp Gly Ser Phe Phe Leu
        435                 440                 445

Tyr Ser Lys Leu Thr Val Asp Lys Ser Arg Trp Gln Gln Gly Asn Val
    450                 455                 460

Phe Ser Cys Ser Val Met His Glu Ala Leu His Asn His Tyr Thr Gln
465                 470                 475                 480

Lys Ser Leu Ser Leu Ser Pro Gly Lys
                485

<210> 538
<211> 333
<212> DNA
<213> Artificial Sequence

<220>
<221> source
<223> /note="Description of Artificial Sequence: Synthetic
      polynucleotide"

<400> 538
aacttcatgc tgacccagcc tcactccgtg tccgaatccc cggggaaaac cgtgactatc      60

agctgcaccg gctccagcgg ctcgatcgcg tcgaactacg tgcagtggta tcaacagcgc     120

cccggttccg cccccaccac cgtgatctac gaagataacc agcggccttc cggagtcccg     180

gatagattct ccggttccat tgactcctca tccaactccg cctcgctcac tattagcggc     240

ctcaagacgg aggacgaagc cgattactac tgtcagtcct acgactcgag caatcaagtg     300

gtgttcggag ggggcaccaa gctgaccgtg ctg                                   333

<210> 539
<211> 387
<212> DNA
<213> Artificial Sequence

<220>
<221> source
<223> /note="Description of Artificial Sequence: Synthetic
      polynucleotide"

<400> 539
gaagtgcagc tcgtggagtc gggcggtgga ttggtcaagc cgggcggaag cctgcggctg      60

tcatgcgccg cttctgggtt caccttctcc tcctactcca tgaactgggt cagacaggcg     120

cccggaaagg gactggaatg ggtgtcctca atctcgtcgt cctcgtccta catctattac     180

gccgactcag tgaaggggcg ctttactatt tcgcgggaca acgctaagaa ctccctgtac     240

ctccaaatga acagcctgcg ggcggaggac accgccgtgt actactgcgc aaggacccca     300

agcagctccg gctcatacta catggaggac tcctactact acggaatgga cgtctgggga     360

cagggaacca ctgtgaccgt gtcatcc                                         387

<210> 540
<211> 765
<212> DNA
<213> Artificial Sequence

<220>
<221> source
<223> /note="Description of Artificial Sequence: Synthetic
      polynucleotide"

<400> 540
gaagtgcagc tcgtggagtc gggcggtgga ttggtcaagc cgggcggaag cctgcggctg      60

tcatgcgccg cttctgggtt caccttctcc tcctactcca tgaactgggt cagacaggcg     120

cccggaaagg gactggaatg ggtgtcctca atctcgtcgt cctcgtccta catctattac     180

gccgactcag tgaaggggcg ctttactatt tcgcgggaca acgctaagaa ctccctgtac     240

ctccaaatga acagcctgcg ggcggaggac accgccgtgt actactgcgc aagggaccca     300

agcagctccg gctcatacta catggaggac tcctactact acggaatgga cgtctgggga     360

cagggaacca ctgtgaccgt gtcatccggc ggcggtggta gcgggggcgg aggaagcggg     420

gggggaggct ccaacttcat gctgacccag cctcactccg tgtccgaatc cccggggaaa     480

accgtgacta tcagctgcac cggctccagc ggctcgatcg cgtcgaacta cgtgcagtgg     540

tatcaacagc gccccggttc cgcccccacc accgtgatct acgaagataa ccagcggcct     600

tccggagtcc cggatagatt ctccggttcc attgactcct catccaactc cgcctcgctc     660

actattagcg gcctcaagac ggaggacgaa gccgattact actgtcagtc ctacgactcg     720

agcaatcaag tggtgttcgg agggggcacc aagctgaccg tgctg                     765

<210> 541
<211> 1467
<212> DNA
<213> Artificial Sequence

<220>
<221> source
<223> /note="Description of Artificial Sequence: Synthetic
      polynucleotide"

<400> 541
gaagtgcagc tcgtggagtc gggcggtgga ttggtcaagc cgggcggaag cctgcggctg      60

tcatgcgccg cttctgggtt caccttctcc tcctactcca tgaactgggt cagacaggcg     120

cccggaaagg gactggaatg ggtgtcctca atctcgtcgt cctcgtccta catctattac     180

gccgactcag tgaaggggcg ctttactatt tcgcgggaca acgctaagaa ctccctgtac     240

ctccaaatga acagcctgcg ggcggaggac accgccgtgt actactgcgc aagggaccca     300

agcagctccg gctcatacta catggaggac tcctactact acggaatgga cgtctgggga     360

cagggaacca ctgtgaccgt gtcatccggc ggcggtggta gcgggggcgg aggaagcggg     420

gggggaggct ccaacttcat gctgacccag cctcactccg tgtccgaatc cccggggaaa     480

accgtgacta tcagctgcac cggctccagc ggctcgatcg cgtcgaacta cgtgcagtgg     540

tatcaacagc gccccggttc cgcccccacc accgtgatct acgaagataa ccagcggcct     600

tccggagtcc cggatagatt ctccggttcc attgactcct catccaactc cgcctcgctc     660

actattagcg gcctcaagac ggaggacgaa gccgattact actgtcagtc ctacgactcg     720

agcaatcaag tggtgttcgg agggggcacc aagctgaccg tgctgggcgg tggaggatcc 780

ggatccgata agacccacac ctgtccaccc tgccctgccc ccgaactgct tggtggtccg 840

tccgtgtttc tgttcccgcc caagcccaag gacaccctca tgatctcacg gactcctgaa 900

gtgacctgtg tggtggtcgc tgtgtcccac gaggaccccg aagtcaagtt caattggtac 960

gtggacggag tggaagtgca caacgctaag accaagcccc gcgaggaaca gtacaactcc 1020

acttaccgcg tcgtgtcggt gctgaccgtg ctgcatcagg attggctgaa cggaaaggag 1080

tacaagtgca aggtgtccaa caaggctctg gcggcaccca tcgaaaagac catcagcaag 1140

gccaaagggc aacctagaga accacaagtc tacaccctgc ctccttgccg ggatgagctc 1200

accaagaacc aggtgtccct gtggtgcctc gtgaagggct tctacccctc tgacatcgcg 1260

gtggaatggg agtcaaacgg ccagccagag aacaactaca agacaacccc ccctgtcctg 1320

gacagcgacg gctccttctt cctgtactcg aagctgactg tggataagag ccggtggcaa 1380

cagggcaacg tgttctcatg ttcggtcatg cacgaggccc tgcataacca ctacactcag 1440

aagtccctga gcctgtcccc tggaaag 1467

<210> 542
<211> 481
<212> PRT
<213> Artificial Sequence

<220>
<221> source
<223> /note="Description of Artificial Sequence: Synthetic
      polypeptide"

<400> 542
Gln Val Gln Leu Val Gln Ser Gly Ala Glu Val Lys Lys Ser Gly Ala
1               5                   10                  15

Ser Val Lys Val Ser Cys Lys Ala Ser Gly Tyr Pro Phe Thr Gly Tyr
            20                  25                  30

Tyr Ile Gln Trp Val Arg Gln Ala Pro Gly Gln Gly Leu Glu Trp Met
        35                  40                  45

Gly Trp Ile Asp Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Ser Asp Ser Tyr Gly Tyr Tyr Tyr Gly Met Asp Val Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Gly Gly Gly Gly Ser Asp Ile Gln Met
    130                 135                 140

Thr Gln Ser Pro Ser Ser Leu Ser Ala Ser Val Gly Asp Arg Val Thr
145                 150                 155                 160

Phe Thr Cys Arg Ala Ser Gln Gly Ile Ser Ser Ala Leu Ala Trp Tyr
                165                 170                 175

Gln Gln Lys Pro Gly Lys Pro Pro Lys Leu Leu Ile Tyr Asp Ala Ser
            180                 185                 190

Ser Leu Glu Ser Gly Val Pro Ser Arg Phe Ser Gly Ser Gly Ser Gly
        195                 200                 205

Thr Asp Phe Thr Leu Thr Ile Ser Ser Leu Gln Pro Glu Asp Phe Ala
    210                 215                 220

Thr Tyr Tyr Cys Gln Gln Phe Asn Asn Tyr Pro Leu Thr Phe Gly Gly
225                 230                 235                 240

Gly Thr Lys Val Glu Ile Lys Gly Gly Gly Gly Ser Gly Ser Asp Lys
                245                 250                 255

Thr His Thr Cys Pro Pro Cys Pro Ala Pro Glu Leu Leu Gly Gly Pro
            260                 265                 270

Ser Val Phe Leu Phe Pro Pro Lys Pro Lys Asp Thr Leu Met Ile Ser
        275                 280                 285

Arg Thr Pro Glu Val Thr Cys Val Val Val Ala Val Ser His Glu Asp
    290                 295                 300

Pro Glu Val Lys Phe Asn Trp Tyr Val Asp Gly Val Glu Val His Asn
305                 310                 315                 320

Ala Lys Thr Lys Pro Arg Glu Glu Gln Tyr Asn Ser Thr Tyr Arg Val
                325                 330                 335

Val Ser Val Leu Thr Val Leu His Gln Asp Trp Leu Asn Gly Lys Glu
            340                 345                 350

Tyr Lys Cys Lys Val Ser Asn Lys Ala Leu Ala Ala Pro Ile Glu Lys
        355                 360                 365

Thr Ile Ser Lys Ala Lys Gly Gln Pro Arg Glu Pro Gln Val Tyr Thr
    370                 375                 380

Leu Pro Pro Cys Arg Asp Glu Leu Thr Lys Asn Gln Val Ser Leu Trp
385                 390                 395                 400

Cys Leu Val Lys Gly Phe Tyr Pro Ser Asp Ile Ala Val Glu Trp Glu
                405                 410                 415

Ser Asn Gly Gln Pro Glu Asn Asn Tyr Lys Thr Thr Pro Pro Val Leu
            420                 425                 430

Asp Ser Asp Gly Ser Phe Phe Leu Tyr Ser Lys Leu Thr Val Asp Lys
        435                 440                 445

Ser Arg Trp Gln Gln Gly Asn Val Phe Ser Cys Ser Val Met His Glu
    450                 455                 460

Ala Leu His Asn His Tyr Thr Gln Lys Ser Leu Ser Leu Ser Pro Gly
465                 470                 475                 480

Lys

<210> 543
<211> 321
<212> DNA
<213> Artificial Sequence

<220>
<221> source
<223> /note="Description of Artificial Sequence: Synthetic
      polynucleotide"

<400> 543
gacattcaga tgacccagtc accatcctcc ctgtccgcct ccgtcgggga tagagtgacc      60

ttcacctgtc gggcctccca aggaatctca agcgctctgg cctggtacca gcagaagcct     120

ggaaagccgc ccaagctgtt gatctacgat gcctcgagcc tggaatccgg cgtgccgagc     180

cggttcagcg gtagcggctc gggaaccgac ttcacgctca ccatctcgtc cctgcaaccg     240

gaggacttcg cgacttacta ctgccagcaa ttcaacaact accctctgac ctttggtggt     300

ggtactaagg tcgagatcaa g                                               321

<210> 544
<211> 357

<212> DNA
<213> Artificial Sequence

<220>
<221> source
<223> /note="Description of Artificial Sequence: Synthetic
      polynucleotide"

<400> 544
caagtgcagc tggtgcagtc cggggccgaa gtgaaaaagt ccggtgcatc cgtgaaagtg      60

tcgtgcaagg cctcaggcta tcccttcacc ggatactaca ttcagtgggt ccgccaggct     120

ccgggacagg gcctcgaatg gatgggctgg atcgatccca actccggcaa tactggctac     180

gcgcagaagt tccagggacg cgtgaccatg actcggaaca cctccatttc caccgcctac     240

atggaactgt cgtcactgag gtccgaggac accgccgtgt attactgcgc gtcggacagc     300

tacggatact actacgggat ggacgtgtgg ggacagggaa ctctggtcac cgtgtcg        357


<210> 545
<211> 741
<212> DNA
<213> Artificial Sequence

<220>
<221> source
<223> /note="Description of Artificial Sequence: Synthetic
      polynucleotide"

<400> 545
caagtgcagc tggtgcagtc cggggccgaa gtgaaaaagt ccggtgcatc cgtgaaagtg      60

tcgtgcaagg cctcaggcta tcccttcacc ggatactaca ttcagtgggt ccgccaggct     120

ccgggacagg gcctcgaatg gatgggctgg atcgatccca actccggcaa tactggctac     180

gcgcagaagt tccagggacg cgtgaccatg actcggaaca cctccatttc caccgcctac     240

atggaactgt cgtcactgag gtccgaggac accgccgtgt attactgcgc gtcggacagc     300

tacggatact actacgggat ggacgtgtgg ggacagggaa ctctggtcac cgtgtcgtcc     360

ggaggcggag gcagcggcgg gggcggctcc gggggagggg ggtcgggcgg aggcggaagc     420

gacattcaga tgacccagtc accatcctcc ctgtccgcct ccgtcgggga tagagtgacc     480

ttcacctgtc gggcctccca aggaatctca agcgctctgg cctggtacca gcagaagcct     540

ggaaagccgc ccaagctgtt gatctacgat gcctcgagcc tggaatccgg cgtgccgagc     600

cggttcagcg gtagcggctc gggaaccgac ttcacgctca ccatctcgtc cctgcaaccg     660

gaggacttcg cgacttacta ctgccagcaa ttcaacaact accctctgac ctttggtggt     720

ggtactaagg tcgagatcaa g                                               741


<210> 546
<211> 1443
<212> DNA

<213> Artificial Sequence

<220>
<221> source
<223> /note="Description of Artificial Sequence: Synthetic
      polynucleotide"

<400> 546
caagtgcagc tggtgcagtc cggggccgaa gtgaaaaagt ccggtgcatc cgtgaaagtg      60

tcgtgcaagg cctcaggcta tcccttcacc ggatactaca ttcagtgggt ccgccaggct     120

ccgggacagg gcctcgaatg gatgggctgg atcgatccca actccggcaa tactggctac     180

gcgcagaagt tccagggacg cgtgaccatg actcggaaca cctccatttc caccgcctac     240

atggaactgt cgtcactgag gtccgaggac accgccgtgt attactgcgc gtcggacagc     300

tacggatact actacgggat ggacgtgtgg ggacagggaa ctctggtcac cgtgtcgtcc     360

ggaggcggag gcagcggcgg gggcggctcc gggggagggg ggtcgggcgg aggcggaagc     420

gacattcaga tgacccagtc accatcctcc ctgtccgcct ccgtcgggga tagagtgacc     480

ttcacctgtc gggcctccca aggaatctca agcgctctgg cctggtacca gcagaagcct     540

ggaaagccgc ccaagctgtt gatctacgat gcctcgagcc tggaatccgg cgtgccgagc     600

cggttcagcg gtagcggctc gggaaccgac ttcacgctca ccatctcgtc cctgcaaccg     660

gaggacttcg cgacttacta ctgccagcaa ttcaacaact accctctgac ctttggtggt     720

ggtactaagg tcgagatcaa gggtggagga ggatccggat ccgataagac ccacacctgt     780

ccaccctgcc ctgcccccga actgcttggt ggtccgtccg tgtttctgtt cccgcccaag     840

cccaaggaca ccctcatgat ctcacggact cctgaagtga cctgtgtggt ggtcgctgtg     900

tcccacgagg accccgaagt caagttcaat tggtacgtgg acggagtgga agtgcacaac     960

gctaagacca agccccgcga ggaacagtac aactccactt accgcgtcgt gtcggtgctg    1020

accgtgctgc atcaggattg gctgaacgga aaggagtaca agtgcaaggt gtccaacaag    1080

gctctggcgg cacccatcga aaagaccatc agcaaggcca aagggcaacc tagagaacca    1140

caagtctaca ccctgcctcc ttgccgggat gagctcacca agaaccaggt gtccctgtgg    1200

tgcctcgtga agggcttcta cccctctgac atcgcggtgg aatgggagtc aaacggccag    1260

ccagagaaca actacaagac aaccccccct gtcctggaca gcgacggctc cttcttcctg    1320

tactcgaagc tgactgtgga taagagccgg tggcaacagg gcaacgtgtt ctcatgttcg    1380

gtcatgcacg aggccctgca taaccactac actcagaagt ccctgagcct gtcccctgga    1440

aag                                                                  1443

<210> 547
<211> 471
<212> PRT
<213> Artificial Sequence

<220>
<221> source
<223> /note="Description of Artificial Sequence: Synthetic
      polypeptide"

<400> 547
Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Asn Glu Asp Gly Ser Ala Lys Phe Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Phe Cys
                85                  90                  95

Ala Arg Asp Leu Arg Ser Gly Arg Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Gly Arg Ala Thr Leu Ser Cys Arg Ala Ser Gln Ser Ile
145                 150                 155                 160

Ser Gly Ser Phe Leu Ala Trp Tyr Gln Gln Lys Pro Gly Gln Ala Pro
                165                 170                 175

Arg Leu Leu Ile Tyr Gly Ala Ser Ser Arg Ala Thr Gly Ile Pro Asp
            180                 185                 190

Arg Phe Ser Gly Ser Gly Ser Gly Thr Asp Phe Thr Leu Thr Ile Ser
        195                 200                 205

Arg Leu Glu Pro Glu Asp Phe Ala Val Tyr Tyr Cys Gln Gln Tyr Gly
    210                 215                 220

Ser Ser Pro Pro Thr Phe Gly Leu Gly Thr Lys Leu Glu Ile Lys Gly
225                 230                 235                 240

Gly Gly Gly Ser Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Ala Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Ala Ala Pro Ile Glu Lys Thr Ile Ser Lys Ala Lys Gly Gln Pro Arg
        355                 360                 365

Glu Pro Gln Val Tyr Thr Leu Pro Pro Cys Arg Asp Glu Leu Thr Lys
    370                 375                 380

Asn Gln Val Ser Leu Trp Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210> 548
<211> 324
<212> DNA
<213> Artificial Sequence

<220>
<221> source
<223> /note="Description of Artificial Sequence: Synthetic
      polynucleotide"

<400> 548
gaaattgtgt tgacccagtc gcctggaacc ctttccctgt cgcccggcgg acgggctacc      60

ctgtcgtgcc gcgctagcca gtcgatctcc ggatcttttc tcgcctggta ccagcagaag     120

cccggacagg cccctaggct gctgatctac ggggccagct cacgcgcaac cggtattccg     180

gatcggttct ccggttccgg gtcgggaact gacttcaccc tgactatctc ccggctggaa     240

ccagaggatt tcgcggtcta ctactgccag cagtatggaa gctcaccgcc gaccttcggc     300

ttgggaacca agctggaaat caag                                            324


<210> 549
<211> 348
<212> DNA
<213> Artificial Sequence

<220>
<221> source
<223> /note="Description of Artificial Sequence: Synthetic
      polynucleotide"

<400> 549
caagtgcagc tcgtcgaaag cggtggcggg ctggtgcagc cgggcggctc gctgagactg      60

tcctgcgccg cgagcggctt caccttctcc tcctactgga tgtcctgggt ccgccaagcc     120

cccggaaagg ggctggaatg ggtggccaac attaacgagg acggttccgc caagttctac     180

gtggattccg tgaaaggccg gtttaccatc tcgagggaca acgccaagaa ttccctctac      240

ctccaaatga actccctgag agcggaggac actgccgtgt acttctgtgc acgcgacctg     300

agatcaggcc ggtactgggg ccaggggaca ctcgtgaccg tgtcaagc                  348


<210> 550
<211> 717
<212> DNA
<213> Artificial Sequence

<220>
<221> source
<223> /note="Description of Artificial Sequence: Synthetic
      polynucleotide"

<400> 550
caagtgcagc tcgtcgaaag cggtggcggg ctggtgcagc cgggcggctc gctgagactg      60

tcctgcgccg cgagcggctt caccttctcc tcctactgga tgtcctgggt ccgccaagcc     120

cccggaaagg ggctggaatg ggtggccaac attaacgagg acggttccgc caagttctac 180

gtggattccg tgaaaggccg gtttaccatc tcgagggaca acgccaagaa ttccctctac 240

ctccaaatga actccctgag agcggaggac actgccgtgt acttctgtgc acgcgacctg 300

agatcaggcc ggtactgggg ccaggggaca ctcgtgaccg tgtcaagcgg aggcggtggc 360

tccggaggag gaggttccgg gggaggaggc agcgaaattg tgttgaccca gtcgcctgga 420

accctttccc tgtcgcccgg cggacgggct accctgtcgt gccgcgctag ccagtcgatc 480

tccggatctt ttctcgcctg gtaccagcag aagcccggac aggcccctag gctgctgatc 540

tacggggcca gctcacgcgc aaccggtatt ccggatcggt tctccggttc cgggtcggga 600

actgacttca ccctgactat ctcccggctg gaaccagagg atttcgcggt ctactactgc 660

cagcagtatg gaagctcacc gccgaccttc ggcttgggaa ccaagctgga aatcaag 717

<210> 551
<211> 1413
<212> DNA
<213> Artificial Sequence

<220>
<221> source
<223> /note="Description of Artificial Sequence: Synthetic
      polynucleotide"

<400> 551
caagtgcagc tcgtcgaaag cggtggcggg ctggtgcagc cgggcggctc gctgagactg 60

tcctgcgccg cgagcggctt caccttctcc tcctactgga tgtcctgggt ccgccaagcc 120

cccggaaagg ggctggaatg ggtggccaac attaacgagg acggttccgc caagttctac 180

gtggattccg tgaaaggccg gtttaccatc tcgagggaca acgccaagaa ttccctctac 240

ctccaaatga actccctgag agcggaggac actgccgtgt acttctgtgc acgcgacctg 300

agatcaggcc ggtactgggg ccaggggaca ctcgtgaccg tgtcaagcgg aggcggtggc 360

tccggaggag gaggttccgg gggaggaggc agcgaaattg tgttgaccca gtcgcctgga 420

accctttccc tgtcgcccgg cggacgggct accctgtcgt gccgcgctag ccagtcgatc 480

tccggatctt ttctcgcctg gtaccagcag aagcccggac aggcccctag gctgctgatc 540

tacggggcca gctcacgcgc aaccggtatt ccggatcggt tctccggttc cgggtcggga 600

actgacttca ccctgactat ctcccggctg gaaccagagg atttcgcggt ctactactgc 660

cagcagtatg gaagctcacc gccgaccttc ggcttgggaa ccaagctgga aatcaagggg 720

gggggcggat ccgataagac ccacacctgt ccaccctgcc ctgcccccga actgcttggt 780

ggtccgtccg tgtttctgtt cccgcccaag cccaaggaca ccctcatgat ctcacggact 840

cctgaagtga cctgtgtggt ggtcgctgtg tcccacgagg accccgaagt caagttcaat 900

tggtacgtgg acggagtgga agtgcacaac gctaagacca agccccgcga ggaacagtac 960

aactccactt accgcgtcgt gtcggtgctg accgtgctgc atcaggattg gctgaacgga 1020

aaggagtaca agtgcaaggt gtccaacaag gctctggcgg cacccatcga aaagaccatc 1080

agcaaggcca aagggcaacc tagagaacca caagtctaca ccctgcctcc ttgccgggat 1140

gagctcacca agaaccaggt gtccctgtgg tgcctcgtga agggcttcta cccctctgac 1200

atcgcggtgg aatgggagtc aaacggccag ccagagaaca actacaagac aaccccccct 1260

gtcctggaca gcgacggctc cttcttcctg tactcgaagc tgactgtgga taagagccgg 1320

tggcaacagg gcaacgtgtt ctcatgttcg gtcatgcacg aggccctgca taaccactac 1380

actcagaagt ccctgagcct gtcccctgga aag 1413

<210> 552
<211> 477
<212> PRT
<213> Artificial Sequence

<220>
<221> source
<223> /note="Description of Artificial Sequence: Synthetic
      polypeptide"

<400> 552
Glu Val Gln Leu Val Glu Ser Gly Gly Gly Val Val Gln Pro Gly Gly
1               5                   10                  15

Ser Leu Arg Leu Ser Cys Ala Ala Ser Gly Phe Thr Phe Asp Asp Tyr
            20                  25                  30

Ala Met His Trp Val Arg Gln Ala Pro Gly Lys Gly Leu Glu Trp Val
        35                  40                  45

Ser Leu Ile Ser Gly Asp Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Val Glu Asp Thr Ala Val Tyr Tyr Cys
                85                  90                  95

Ala Arg Val Phe Asp Ser Tyr Tyr Met Asp Val Trp Gly Lys Gly Thr
            100                 105                 110

Thr Val Thr Val Ser Ser Gly Gly Gly Gly Ser Gly Gly Gly Gly Ser
        115                 120                 125

Gly Ser Gly Gly Ser Glu Ile Val Leu Thr Gln Ser Pro Leu Ser Leu

130                 135                 140

Pro Val Thr Pro Gly Gln Pro Ala Ser Ile Ser Cys Arg Ser Ser Gln
145                 150                 155                 160

Ser Leu Val Tyr Thr Asp Gly Asn Thr Tyr Leu Asn Trp Phe Gln Gln
                165                 170                 175

Arg Pro Gly Gln Ser Pro Arg Arg Leu Ile Tyr Lys Val Ser Asn Arg
            180                 185                 190

Asp Ser Gly Val Pro Asp Arg Phe Ser Gly Ser Gly Ser Asp Thr Asp
        195                 200                 205

Phe Thr Leu Lys Ile Ser Arg Val Glu Ala Glu Asp Val Gly Ile Tyr
    210                 215                 220

Tyr Cys Met Gln Gly Thr His Trp Ser Phe Thr Phe Gly Gln Gly Thr
225                 230                 235                 240

Arg Leu Glu Ile Lys Gly Gly Gly Gly Ser Asp Lys Thr His Thr Cys
                245                 250                 255

Pro Pro Cys Pro Ala Pro Glu Leu Leu Gly Gly Pro Ser Val Phe Leu
            260                 265                 270

Phe Pro Pro Lys Pro Lys Asp Thr Leu Met Ile Ser Arg Thr Pro Glu
        275                 280                 285

Val Thr Cys Val Val Val Ala Val Ser His Glu Asp Pro Glu Val Lys
    290                 295                 300

Phe Asn Trp Tyr Val Asp Gly Val Glu Val His Asn Ala Lys Thr Lys
305                 310                 315                 320

Pro Arg Glu Glu Gln Tyr Asn Ser Thr Tyr Arg Val Val Ser Val Leu
                325                 330                 335

Thr Val Leu His Gln Asp Trp Leu Asn Gly Lys Glu Tyr Lys Cys Lys
            340                 345                 350

Val Ser Asn Lys Ala Leu Ala Ala Pro Ile Glu Lys Thr Ile Ser Lys
        355                 360                 365

Ala Lys Gly Gln Pro Arg Glu Pro Gln Val Tyr Thr Leu Pro Pro Cys
    370                 375                 380

Arg Asp Glu Leu Thr Lys Asn Gln Val Ser Leu Trp Cys Leu Val Lys
385                 390                 395                 400

Gly Phe Tyr Pro Ser Asp Ile Ala Val Glu Trp Glu Ser Asn Gly Gln
                405                 410                 415

Pro Glu Asn Asn Tyr Lys Thr Thr Pro Pro Val Leu Asp Ser Asp Gly
            420                 425                 430

Ser Phe Phe Leu Tyr Ser Lys Leu Thr Val Asp Lys Ser Arg Trp Gln
        435                 440                 445

Gln Gly Asn Val Phe Ser Cys Ser Val Met His Glu Ala Leu His Asn
    450                 455                 460

His Tyr Thr Gln Lys Ser Leu Ser Leu Ser Pro Gly Lys
465                 470                 475

<210> 553
<211> 336
<212> DNA
<213> Artificial Sequence

<220>
<221> source
<223> /note="Description of Artificial Sequence: Synthetic
      polynucleotide"

<400> 553
gaaattgtcc tcacccaatc cccgctgtca ctgcccgtga cccctggcca gccggcatcc      60

atcagctgcc ggagcagcca gtccctggtg tacactgacg gaaatactta cctgaactgg     120

ttccagcaac gcccggggca gagcccacgc agactgatct acaaggtgtc aaacagggac     180

tctggagtgc ccgataggtt ctcgggttcc gggtcggaca ccgattttac actgaagatc     240

tcccgggtgg aagcggagga cgtgggcatc tattactgta tgcaggggac ccattggtcc     300

ttcacgttcg gacagggcac tcggctggaa atcaag                               336

<210> 554
<211> 354
<212> DNA
<213> Artificial Sequence

<220>
<221> source
<223> /note="Description of Artificial Sequence: Synthetic
      polynucleotide"

<400> 554
gaagtgcagt tggtggagag cggaggcggc gtggtgcagc ccggaggttc cctgcggctg      60

tcgtgcgcgg cctcgggttt cacttttgat gactacgcca tgcactgggt cagacaggcc     120

cctggaaagg gcctcgaatg ggtgtcgctg atttccggag atggaggcag cacctactat 180

gccgattccg tcaaggggag attcaccatt tcccgcgaca acagcaaaaa caccttgtac 240

ctccaaatga actccctgcg ggtggaggac accgctgtgt actactgcgc ccgcgtgttc 300

gactcatact acatggacgt ctggggaaag gggaccactg tgaccgtgtc cagc 354

<210> 555
<211> 735
<212> DNA
<213> Artificial Sequence

<220>
<221> source
<223> /note="Description of Artificial Sequence: Synthetic
      polynucleotide"

<400> 555
gaagtgcagt tggtggagag cggaggcggc gtggtgcagc ccggaggttc cctgcggctg 60

tcgtgcgcgg cctcgggttt cacttttgat gactacgcca tgcactgggt cagacaggcc 120

cctggaaagg gcctcgaatg ggtgtcgctg atttccggag atggaggcag cacctactat 180

gccgattccg tcaaggggag attcaccatt tcccgcgaca acagcaaaaa caccttgtac 240

ctccaaatga actccctgcg ggtggaggac accgctgtgt actactgcgc ccgcgtgttc 300

gactcatact acatggacgt ctggggaaag gggaccactg tgaccgtgtc cagcggggga 360

ggcggctccg gcggcggcgg atcgggttca ggagggtccg aaattgtcct cacccaatcc 420

ccgctgtcac tgcccgtgac ccctggccag ccggcatcca tcagctgccg gagcagccag 480

tccctggtgt acactgacgg aaatacttac ctgaactggt tccagcaacg cccggggcag 540

agcccacgca gactgatcta caaggtgtca aacagggact ctggagtgcc cgataggttc 600

tcgggttccg ggtcggacac cgattttaca ctgaagatct cccgggtgga agcggaggac 660

gtgggcatct attactgtat gcaggggacc cattggtcct tcacgttcgg acagggcact 720

cggctggaaa tcaag 735

<210> 556
<211> 1431
<212> DNA
<213> Artificial Sequence

<220>
<221> source
<223> /note="Description of Artificial Sequence: Synthetic
      polynucleotide"

<400> 556
gaagtgcagt tggtggagag cggaggcggc gtggtgcagc ccggaggttc cctgcggctg 60

tcgtgcgcgg cctcgggttt cacttttgat gactacgcca tgcactgggt cagacaggcc 120

cctggaaagg gcctcgaatg ggtgtcgctg atttccggag atggaggcag cacctactat 180

gccgattccg tcaaggggag attcaccatt tcccgcgaca acagcaaaaa caccttgtac 240

ctccaaatga actccctgcg ggtggaggac accgctgtgt actactgcgc ccgcgtgttc 300

gactcatact acatggacgt ctggggaaag gggaccactg tgaccgtgtc cagcggggga 360

ggcggctccg gcggcggcgg atcgggttca ggagggtccg aaattgtcct cacccaatcc 420

ccgctgtcac tgcccgtgac ccctggccag ccggcatcca tcagctgccg gagcagccag 480

tccctggtgt acactgacgg aaatacttac ctgaactggt tccagcaacg cccggggcag 540

agcccacgca gactgatcta caaggtgtca aacagggact ctggagtgcc cgataggttc 600

tcgggttccg ggtcggacac cgattttaca ctgaagatct cccgggtgga agcggaggac 660

gtgggcatct attactgtat gcaggggacc cattggtcct tcacgttcgg acagggcact 720

cggctggaaa tcaagggagg aggcggatcc gataagaccc acacctgtcc accctgccct 780

gcccccgaac tgcttggtgg tccgtccgtg tttctgttcc cgcccaagcc caaggacacc 840

ctcatgatct cacggactcc tgaagtgacc tgtgtggtgg tcgctgtgtc ccacgaggac 900

cccgaagtca agttcaattg gtacgtggac ggagtggaag tgcacaacgc taagaccaag 960

ccccgcgagg aacagtacaa ctccacttac cgcgtcgtgt cggtgctgac cgtgctgcat 1020

caggattggc tgaacggaaa ggagtacaag tgcaaggtgt ccaacaaggc tctggcggca 1080

cccatcgaaa agaccatcag caaggccaaa gggcaaccta gagaaccaca agtctacacc 1140

ctgcctcctt gccgggatga gctcaccaag aaccaggtgt ccctgtggtg cctcgtgaag 1200

ggcttctacc cctctgacat cgcggtggaa tgggagtcaa acggccagcc agagaacaac 1260

tacaagacaa ccccccctgt cctggacagc gacggctcct tcttcctgta ctcgaagctg 1320

actgtggata agagccggtg gcaacagggc aacgtgttct catgttcggt catgcacgag 1380

gccctgcata accactacac tcagaagtcc ctgagcctgt cccctggaaa g 1431

<210> 557

<400> 557
000

<210> 558

<400> 558
000

<210> 559

<400> 559
000

<210> 560

<400> 560
000

<210> 561

<400> 561
000

<210> 562

<400> 562
000

<210> 563

<400> 563
000

<210> 564

<400> 564
000

<210> 565

<400> 565
000

<210> 566

<400> 566
000

<210> 567

<400> 567
000

<210> 568

<400> 568
000

<210> 569

<400> 569
000

<210> 570

<400> 570
000

<210> 571

<400> 571
000

<210> 572

<400> 572
000

<210> 573

<400> 573
000

<210> 574

<400> 574
000

<210> 575

<400> 575
000

<210> 576

<400> 576
000

<210> 577

<400> 577
000

<210> 578

<400> 578
000

<210> 579

<400> 579
000

<210> 580

<400> 580
000

<210> 581

<400> 581
000

<210> 582

<400> 582
000

<210> 583

<400> 583
000

<210> 584

<400> 584
000

<210> 585

<400> 585
000

<210> 586

<400> 586
000

<210> 587

<400> 587
000

<210> 588

<400> 588
000

<210> 589

<400> 589
000

<210> 590

<400> 590
000

<210> 591

<400> 591
000

<210> 592

<400> 592
000

<210> 593

<400> 593
000

<210> 594

<400> 594
000

<210> 595

<400> 595
000

<210> 596

<400> 596
000

<210> 597

<400> 597
000

<210> 598

<400> 598
000

<210> 599

<400> 599
000

<210> 600

<400> 600
000

<210> 601

<400> 601
000

<210> 602

<400> 602
000

<210> 603

<400> 603
000

<210> 604

<400> 604
000

<210> 605

<400> 605
000

<210> 606

<400> 606
000

<210> 607

<400> 607
000

<210> 608

<400> 608
000

<210> 609

<400> 609
000

<210> 610

<400> 610
000

<210> 611

<400> 611
000

<210> 612

<400> 612
000

<210> 613

<400> 613
000

<210> 614

<400> 614
000

<210> 615

<400> 615
000

<210> 616

<400> 616
000

<210> 617

<400> 617
000

<210> 618

<400> 618
000

<210> 619

<400> 619
000

<210> 620

<400> 620
000

<210> 621

<400> 621
000

<210> 622

<400> 622
000

<210> 623

<400> 623
000

<210> 624

<400> 624
000

<210> 625

<400> 625
000

<210> 626

<400> 626
000

<210> 627

<400> 627
000

<210> 628

<400> 628
000

<210> 629

<400> 629
000

<210> 630

<400> 630
000

<210> 631

<400> 631
000

<210> 632

<400> 632
000

<210> 633

<400> 633
000

<210> 634

<400> 634
000

<210> 635

<400> 635
000

<210> 636

<400> 636
000

<210> 637

<400> 637
000

<210> 638

<400> 638
000

<210> 639

<400> 639
000

<210> 640

<400> 640
000

<210> 641

<400> 641
000

<210> 642

<400> 642
000

<210> 643

<400> 643
000

<210> 644

<400> 644
000

<210> 645

<400> 645
000

<210> 646

<400> 646
000

<210> 647

<400> 647
000

<210> 648

<400> 648
000

<210> 649

<400> 649
000

<210> 650

<400> 650
000

<210> 651

<400> 651
000

<210> 652

<400> 652
000

<210> 653

<400> 653
000

<210> 654

<400> 654
000

<210> 655

<400> 655
000

<210> 656

<400> 656
000

<210> 657

<400> 657
000

<210> 658

<400> 658
000

<210> 659

<400> 659
000

<210> 660

<400> 660
000

<210> 661

<400> 661
000

<210> 662

<400> 662
000

<210> 663

<400> 663
000

<210> 664

<400> 664
000

<210> 665

<400> 665
000

<210> 666

<400> 666
000

<210> 667

<400> 667
000

<210> 668

<400> 668
000

<210> 669

<400> 669
000

<210> 670

<400> 670
000

<210> 671

<400> 671
000

<210> 672

<400> 672
000

<210> 673

<400> 673
000

<210> 674

<400> 674
000

<210> 675

<400> 675
000

<210> 676

<400> 676
000

<210> 677

<400> 677
000

<210> 678

<400> 678
000

<210> 679

<400> 679
000

<210> 680

<400> 680
000

<210> 681

<400> 681
000

<210> 682

<400> 682
000

<210> 683

<400> 683
000

<210> 684

<400> 684
000

<210> 685

<400> 685
000

<210> 686

<400> 686
000

<210> 687

<400> 687
000

<210> 688

<400> 688
000

<210> 689

<400> 689
000

<210> 690

<400> 690
000

<210> 691

<400> 691
000

<210> 692

<400> 692
000

<210> 693

<400> 693
000

<210> 694

<400> 694
000

<210> 695

<400> 695
000

<210> 696

<400> 696
000

<210> 697

<400> 697
000

<210> 698

<400> 698
000

<210> 699

<400> 699
000

<210> 700

<400> 700
000

<210> 701

<400> 701
000

<210> 702

<400> 702
000

<210> 703

<400> 703
000

<210> 704

<400> 704
000

<210> 705

<400> 705
000

<210> 706

<400> 706
000

<210> 707

<400> 707
000

<210> 708

<400> 708
000

<210> 709

<400> 709
000

<210> 710

<400> 710
000

<210> 711

<400> 711
000

<210> 712

<400> 712
000

<210> 713

<400> 713
000

<210> 714

<400> 714
000

<210> 715

<400> 715
000

<210> 716

<400> 716
000

<210> 717

<400> 717
000

<210> 718

<400> 718
000

<210> 719

<400> 719
000

<210> 720

<400> 720
000

<210> 721

<400> 721
000

<210> 722

<400> 722
000

<210> 723

<400> 723
000

<210> 724

<400> 724
000

<210> 725

<400> 725
000

<210> 726

<400> 726
000

<210> 727

<400> 727
000

<210> 728

<400> 728
000

<210> 729

<400> 729
000

<210> 730

<400> 730
000

<210> 731

<400> 731
000

<210> 732

<400> 732
000

<210> 733

<400> 733
000

<210> 734

<400> 734
000

<210> 735

<400> 735
000

<210> 736

<400> 736
000

<210> 737

<400> 737
000

<210> 738

<400> 738
000

<210> 739

<400> 739
000

<210> 740

<400> 740
000

<210> 741

<400> 741
000

<210> 742

<400> 742
000

<210> 743

<400> 743
000

<210> 744

<400> 744
000

<210> 745

<400> 745
000

<210> 746

<400> 746
000

<210> 747

<400> 747
000

<210> 748

<400> 748
000

<210> 749

<400> 749
000

<210> 750

<400> 750
000

<210> 751

<400> 751
000

<210> 752

<400> 752
000

<210> 753

<400> 753
000

<210> 754

<400> 754
000

<210> 755

<400> 755
000

<210> 756

<400> 756
000

<210> 757

<400> 757
000

<210> 758

<400> 758
000

<210> 759

<400> 759
000

<210> 760

<400> 760
000

<210> 761

<400> 761
000

<210> 762

<400> 762
000

<210> 763

<400> 763
000

<210> 764

<400> 764
000

<210> 765

<400> 765
000

<210> 766

<400> 766
000

<210> 767

<400> 767
000

<210> 768

<400> 768
000

<210> 769

<400> 769
000

<210> 770

<400> 770
000

<210> 771

<400> 771
000

<210> 772

<400> 772
000

<210> 773

<400> 773
000

<210> 774

<400> 774
000

<210> 775

<400> 775
000

<210> 776

<400> 776
000

<210> 777

<400> 777
000

<210> 778

<400> 778
000

<210> 779

<400> 779
000

<210> 780

<400> 780
000

<210> 781

<400> 781
000

<210> 782

<400> 782
000

<210> 783

<400> 783
000

<210> 784

<400> 784
000

<210> 785

<400> 785
000

<210> 786

<400> 786
000

<210> 787

<400> 787
000

<210> 788

<400> 788
000

<210> 789

<400> 789
000

<210> 790

<400> 790
000

<210> 791

<400> 791
000

<210> 792

<400> 792
000

<210> 793

<400> 793
000

<210> 794

<400> 794
000

<210> 795

<400> 795
000

<210> 796

<400> 796
000

<210> 797

<400> 797
000

<210> 798

<400> 798
000

<210> 799

<400> 799
000

<210> 800

<400> 800
000

<210> 801

<400> 801
000

<210> 802

<400> 802
000

<210> 803

<400> 803
000

<210> 804

<400> 804
000

<210> 805

<400> 805
000

<210> 806

<400> 806
000

<210> 807

<400> 807
000

<210> 808

<400> 808
000

<210> 809

<400> 809
000

<210> 810

<400> 810
000

<210> 811

<400> 811
000

<210> 812

<400> 812
000

<210> 813

<400> 813
000

<210> 814

<400> 814
000

<210> 815

<400> 815
000

<210> 816

<400> 816
000

<210> 817

<400> 817
000

<210> 818

<400> 818
000

<210> 819

<400> 819
000

<210> 820

<400> 820
000

<210> 821

<400> 821
000

<210> 822

<400> 822
000

<210> 823

<400> 823
000

<210> 824

<400> 824
000

<210> 825

<400> 825
000

<210> 826

<400> 826
000

<210> 827

<400> 827
000

<210> 828

<400> 828
000

<210> 829

<400> 829
000

<210> 830

<400> 830
000

<210> 831

<400> 831
000

<210> 832

<400> 832
000

<210> 833

<400> 833
000

<210> 834

<400> 834
000

<210> 835

<400> 835
000

<210> 836

<400> 836
000

<210> 837

<400> 837
000

<210> 838

<400> 838
000

<210> 839

<400> 839
000

<210> 840

<400> 840
000

<210> 841

<400> 841
000

<210> 842

<400> 842
000

<210> 843

<400> 843
000

<210> 844

<400> 844
000

<210> 845

<400> 845
000

<210> 846

<400> 846
000

<210> 847

<400> 847
000

<210> 848

<400> 848
000

<210> 849

<400> 849
000

<210> 850

<400> 850
000

<210> 851

<400> 851
000

<210> 852

<400> 852
000

<210> 853

<400> 853
000

<210> 854

<400> 854
000

<210> 855

<400> 855
000

<210> 856

<400> 856
000

<210> 857

<400> 857
000

<210> 858

<400> 858
000

<210> 859

<400> 859
000

<210> 860

<400> 860
000

<210> 861

<400> 861
000

<210> 862

<400> 862
000

<210> 863

<400> 863
000

<210> 864

<400> 864
000

<210> 865

<400> 865
000

<210> 866

<400> 866
000

<210> 867

<400> 867
000

<210> 868

<400> 868
000

<210> 869

<400> 869
000

<210> 870

<400> 870
000

<210> 871

<400> 871
000

<210> 872

<400> 872
000

<210> 873

<400> 873
000

<210> 874

<400> 874
000

<210> 875

<400> 875
000

<210> 876

<400> 876
000

<210> 877

<400> 877
000

<210> 878

<400> 878
000

<210> 879

<400> 879
000

<210> 880

<400> 880
000

<210> 881

<400> 881
000

<210> 882

<400> 882
000

<210> 883

<400> 883
000

<210> 884

<400> 884
000

<210> 885

<400> 885
000

<210> 886

<400> 886
000

<210> 887

<400> 887
000

<210> 888

<400> 888
000

<210> 889

<400> 889
000

<210> 890

<400> 890
000

<210> 891

<400> 891
000

<210> 892

<400> 892
000

<210> 893

<400> 893
000

<210> 894

<400> 894
000

<210> 895

<400> 895
000

<210> 896

<400> 896
000

<210> 897

<400> 897
000

<210> 898

<400> 898
000

<210> 899

<400> 899
000

<210> 900

<400> 900
000

<210> 901

<400> 901
000

<210> 902

<400> 902
000

<210> 903

<400> 903
000

<210> 904

<400> 904
000

<210> 905

<400> 905
000

<210> 906

<400> 906
000

<210> 907

<400> 907
000

<210> 908

<400> 908
000

<210> 909

<400> 909
000

<210> 910

<400> 910
000

<210> 911

<400> 911
000

<210> 912

<400> 912
000

<210> 913

<400> 913
000

<210> 914

<400> 914
000

<210> 915

<400> 915
000

<210> 916

<400> 916
000

<210> 917

<400> 917
000

<210> 918

<400> 918
000

<210> 919

<400> 919
000

<210> 920

<400> 920
000

<210> 921

<400> 921
000

<210> 922

<400> 922
000

<210> 923

<400> 923
000

<210> 924

<400> 924
000

<210> 925

<400> 925
000

<210> 926

<400> 926
000

<210> 927

<400> 927
000

<210> 928

<400> 928
000

<210> 929

<400> 929
000

<210> 930

<400> 930
000

<210> 931

<400> 931
000

<210> 932

<400> 932
000

<210> 933

<400> 933
000

<210> 934

<400> 934
000

<210> 935

<400> 935
000

<210> 936

<400> 936
000

<210> 937

<400> 937
000

<210> 938

<400> 938
000

<210> 939

<400> 939
000

<210> 940

<400> 940
000

<210> 941

<400> 941
000

<210> 942

<400> 942
000

<210> 943

<400> 943
000

<210> 944

<400> 944
000

<210> 945

<400> 945
000

<210> 946

<400> 946
000

<210> 947

<400> 947
000

<210> 948

<400> 948
000

<210> 949

<400> 949
000

<210> 950

<400> 950
000

<210> 951

<400> 951
000

<210> 952

<400> 952
000

<210> 953

<400> 953
000

<210> 954

<400> 954
000

<210> 955

<400> 955
000

<210> 956

<400> 956
000

<210> 957

<400> 957
000

<210> 958

<400> 958
000

<210> 959

<400> 959
000

<210> 960

<400> 960
000

<210> 961

<400> 961
000

<210> 962

<400> 962
000

<210> 963

<400> 963
000

<210> 964

<400> 964
000

<210> 965

<400> 965
000

<210> 966

<400> 966
000

<210> 967

<400> 967
000

<210> 968

<400> 968
000

<210> 969

<400> 969
000

<210> 970

<400> 970
000

<210> 971

<400> 971
000

<210> 972

<400> 972
000

<210> 973

<400> 973
000

<210> 974

<400> 974
000

<210> 975

<400> 975
000

<210> 976

<400> 976
000

<210> 977

<400> 977
000

<210> 978

<400> 978
000

<210> 979

<400> 979
000

<210> 980

<400> 980
000

<210> 981

<400> 981
000

<210> 982

<400> 982
000

<210> 983

<400> 983
000

<210> 984

<400> 984
000

<210> 985

<400> 985
000

<210> 986

<400> 986
000

<210> 987

<400> 987
000

<210> 988

<400> 988
000

<210> 989

<400> 989
000

<210> 990

<400> 990
000

<210> 991

<400> 991
000

<210> 992

<400> 992
000

<210> 993

<400> 993
000

<210> 994

<400> 994
000

<210> 995

<400> 995
000

<210> 996

<400> 996
000

<210> 997

<400> 997
000

<210> 998

<400> 998
000

<210> 999

<400> 999
000

<210> 1000

<400> 1000
000

<210> 1001

<400> 1001
000

<210> 1002

<400> 1002
000

<210> 1003

<400> 1003
000

<210> 1004

<400> 1004
000

<210> 1005

<400> 1005
000

<210> 1006

<400> 1006
000

<210> 1007

<400> 1007
000

<210> 1008

<400> 1008
000

<210> 1009

<400> 1009
000

<210> 1010

<400> 1010
000

<210> 1011

<400> 1011
000

<210> 1012

<400> 1012
000

<210> 1013

<400> 1013
000

<210> 1014

<400> 1014
000

<210> 1015

<400> 1015
000

<210> 1016

<400> 1016
000

<210> 1017

<400> 1017
000

<210> 1018

<400> 1018
000

<210> 1019

<400> 1019
000

<210> 1020

<400> 1020
000

<210> 1021

<400> 1021
000

<210> 1022

<400> 1022
000

<210> 1023

<400> 1023
000

<210> 1024

<400> 1024
000

<210> 1025

<400> 1025
000

<210> 1026

<400> 1026
000

<210> 1027

<400> 1027
000

<210> 1028

<400> 1028
000

<210> 1029

<400> 1029
000

<210> 1030

<400> 1030
000

<210> 1031

<400> 1031
000

<210> 1032

<400> 1032
000

<210> 1033

<400> 1033
000

<210> 1034

<400> 1034
000

<210> 1035

<400> 1035
000

<210> 1036

<400> 1036
000

<210> 1037

<400> 1037
000

<210> 1038

<400> 1038
000

<210> 1039

<400> 1039
000

<210> 1040

<400> 1040
000

<210> 1041

<400> 1041
000

<210> 1042

<400> 1042
000

<210> 1043

<400> 1043
000

<210> 1044

<400> 1044
000

<210> 1045

<400> 1045
000

<210> 1046

<400> 1046
000

<210> 1047

<400> 1047
000

<210> 1048

<400> 1048
000

<210> 1049

<400> 1049
000

<210> 1050

<400> 1050
000

<210> 1051

<400> 1051
000

<210> 1052

<400> 1052
000

<210> 1053

<400> 1053
000

<210> 1054

<400> 1054
000

<210> 1055

<400> 1055
000

<210> 1056

<400> 1056
000

<210> 1057

<400> 1057
000

<210> 1058

<400> 1058
000

<210> 1059

<400> 1059
000

<210> 1060

<400> 1060
000

<210> 1061

<400> 1061
000

<210> 1062

<400> 1062
000

<210> 1063

<400> 1063
000

<210> 1064

<400> 1064
000

<210> 1065

<400> 1065
000

<210> 1066

<400> 1066
000

<210> 1067

<400> 1067
000

<210> 1068

<400> 1068
000

<210> 1069

<400> 1069
000

<210> 1070

<400> 1070
000

<210> 1071

<400> 1071
000

<210> 1072

<400> 1072
000

<210> 1073

<400> 1073
000

<210> 1074

<400> 1074
000

<210> 1075

<400> 1075
000

<210> 1076

<400> 1076
000

<210> 1077

<400> 1077
000

<210> 1078

<400> 1078
000

<210> 1079

<400> 1079
000

<210> 1080

<400> 1080
000

<210> 1081

<400> 1081
000

<210> 1082

<400> 1082
000

<210> 1083

<400> 1083
000

<210> 1084

<400> 1084
000

<210> 1085

<400> 1085
000

<210> 1086

<400> 1086
000

<210> 1087

<400> 1087
000

<210> 1088

<400> 1088
000

<210> 1089

<400> 1089
000

<210> 1090

<400> 1090
000

<210> 1091

<400> 1091
000

<210> 1092

<400> 1092
000

<210> 1093

<400> 1093
000

<210> 1094

<400> 1094
000

<210> 1095

<400> 1095
000

<210> 1096

<400> 1096
000

<210> 1097

<400> 1097
000

<210> 1098

<400> 1098
000

<210> 1099

<400> 1099
000

<210> 1100

<400> 1100
000

<210> 1101

<400> 1101
000

<210> 1102

<400> 1102
000

<210> 1103

<400> 1103
000

<210> 1104

<400> 1104
000

<210> 1105

<400> 1105
000

<210> 1106

<400> 1106
000

<210> 1107

<400> 1107
000

<210> 1108
<211> 18
<212> PRT
<213> Artificial Sequence

<220>
<221> source
<223> /note="Description of Artificial Sequence: Synthetic
      peptide"

<400> 1108
Gly Ser Thr Ser Gly Ser Gly Lys Pro Gly Ser Gly Glu Gly Ser Thr
1               5                   10                  15

Lys Gly

<210> 1109

<400> 1109
000

<210> 1110

<400> 1110
000

<210> 1111

<400> 1111
000

<210> 1112

<400> 1112
000

<210> 1113

<400> 1113
000

<210> 1114

<400> 1114

000

<210> 1115

<400> 1115
000

<210> 1116

<400> 1116
000

<210> 1117

<400> 1117
000

<210> 1118

<400> 1118
000

<210> 1119

<400> 1119
000

<210> 1120

<400> 1120
000

<210> 1121

<400> 1121
000

<210> 1122

<400> 1122
000

<210> 1123

<400> 1123
000

<210> 1124

<400> 1124
000

<210> 1125

<400> 1125
000

<210> 1126

<400> 1126
000

<210> 1127

<400> 1127
000

<210> 1128

<400> 1128
000

<210> 1129

<400> 1129
000

<210> 1130

<400> 1130
000

<210> 1131

<400> 1131
000

<210> 1132

<400> 1132
000

<210> 1133

<400> 1133
000

<210> 1134

<400> 1134
000

<210> 1135

<400> 1135

000

<210> 1136

<400> 1136
000

<210> 1137

<400> 1137
000

<210> 1138

<400> 1138
000

<210> 1139

<400> 1139
000

<210> 1140

<400> 1140
000

<210> 1141

<400> 1141
000

<210> 1142

<400> 1142
000

<210> 1143

<400> 1143
000

<210> 1144

<400> 1144
000

<210> 1145

<400> 1145
000

<210> 1146

<400> 1146
000

<210> 1147

<400> 1147
000

<210> 1148

<400> 1148
000

<210> 1149

<400> 1149
000

<210> 1150

<400> 1150
000

<210> 1151

<400> 1151
000

<210> 1152

<400> 1152
000

<210> 1153

<400> 1153
000

<210> 1154

<400> 1154
000

<210> 1155

<400> 1155
000

<210> 1156

<400> 1156

000

<210> 1157

<400> 1157
000

<210> 1158

<400> 1158
000

<210> 1159

<400> 1159
000

<210> 1160

<400> 1160
000

<210> 1161

<400> 1161
000

<210> 1162

<400> 1162
000

<210> 1163

<400> 1163
000

<210> 1164

<400> 1164
000

<210> 1165

<400> 1165
000

<210> 1166

<400> 1166
000

<210> 1167

<400> 1167
000

<210> 1168

<400> 1168
000

<210> 1169

<400> 1169
000

<210> 1170

<400> 1170
000

<210> 1171

<400> 1171
000

<210> 1172

<400> 1172
000

<210> 1173

<400> 1173
000

<210> 1174

<400> 1174
000

<210> 1175

<400> 1175
000

<210> 1176

<400> 1176
000

<210> 1177

<400> 1177

000

<210> 1178

<400> 1178
000

<210> 1179

<400> 1179
000

<210> 1180

<400> 1180
000

<210> 1181

<400> 1181
000

<210> 1182

<400> 1182
000

<210> 1183

<400> 1183
000

<210> 1184

<400> 1184
000

<210> 1185

<400> 1185
000

<210> 1186

<400> 1186
000

<210> 1187

<400> 1187
000

<210> 1188

<400> 1188
000

<210> 1189

<400> 1189
000

<210> 1190

<400> 1190
000

<210> 1191

<400> 1191
000

<210> 1192

<400> 1192
000

<210> 1193

<400> 1193
000

<210> 1194

<400> 1194
000

<210> 1195

<400> 1195
000

<210> 1196

<400> 1196
000

<210> 1197

<400> 1197
000

<210> 1198

<400> 1198

000

<210> 1199

<400> 1199
000

<210> 1200
<211> 119
<212> PRT
<213> Artificial Sequence

<220>
<221> source
<223> /note="Description of Artificial Sequence: Synthetic
      polypeptide"

<400> 1200

<210> 1201
<211> 106
<212> PRT
<213> Artificial Sequence

<220>
<221> source
<223> /note="Description of Artificial Sequence: Synthetic
      polypeptide"

<400> 1201

<210> 1202
<211> 125
<212> PRT
<213> Artificial Sequence

<220>
<221> source
<223> /note="Description of Artificial Sequence: Synthetic
      polypeptide"

<400> 1202
Glu Val Gln Leu Val Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Gln Ser Ile
65                  70                  75                  80

Leu Tyr Leu Gln Met Asn Asn Leu Lys Thr Glu Asp Thr Ala Met Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ala
        115                 120                 125

<210> 1203
<211> 109
<212> PRT
<213> Artificial Sequence

<220>
<221> source
<223> /note="Description of Artificial Sequence: Synthetic
      polypeptide"

<400> 1203
Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Lys Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

Leu Trp Val Phe Gly Gly Gly Thr Lys Leu Thr Val Leu
            100                 105

<210> 1204
<211> 119
<212> PRT
<213> Artificial Sequence

<220>
<221> source
<223> /note="Description of Artificial Sequence: Synthetic
      polypeptide"

<400> 1204
Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr

20                  25                  30

Thr Met His Trp Val Lys Gln Arg Pro Gly Gln Gly Leu Glu Trp Ile
        35                  40                  45

Gly Tyr Ile Asn Pro Ser Ser Gly Tyr Thr Lys Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Trp Gln Asp Tyr Asp Val Tyr Phe Asp Tyr Trp Gly Gln Gly
            100                 105                 110

Thr Thr Leu Thr Val Ser Ser
        115

<210> 1205
<211> 106
<212> PRT
<213> Artificial Sequence

<220>
<221> source
<223> /note="Description of Artificial Sequence: Synthetic
      polypeptide"

<400> 1205
Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Met
            20                  25                  30

His Trp Tyr Gln Gln Lys Pro Gly Ser Ser Pro Lys Pro Trp Ile Tyr
        35                  40                  45

Ala Thr Ser Asn Leu Ala Ser Gly Val Pro Ala Arg Phe Ser Gly Ser
    50                  55                  60

Gly Ser Gly Thr Ser Tyr Ser Leu Thr Ile Ser Arg Val Glu Ala Glu
65                  70                  75                  80

Asp Ala Ala Thr Tyr Tyr Cys Gln Gln Trp Ser Ser Asn Pro Pro Thr
                85                  90                  95

Phe Gly Gly Gly Thr Lys Leu Glu Thr Lys

100                 105

<210> 1206
<211> 119
<212> PRT
<213> Artificial Sequence

<220>
<221> source
<223> /note="Description of Artificial Sequence: Synthetic
      polypeptide"

<400> 1206

<210> 1207
<211> 106
<212> PRT
<213> Artificial Sequence

<220>
<221> source
<223> /note="Description of Artificial Sequence: Synthetic
      polypeptide"

<400> 1207
Gln Ile Val Leu Thr Gln Ser Pro Ala Ile Met Ser Ala Ser Pro Gly
1               5                   10                  15

Glu Lys Val Thr Met Thr Cys Arg Ala Ser Ser Ser Val Ser Tyr Met
            20                  25                  30

Asn Trp Tyr Gln Gln Lys Ser Gly Thr Ser Pro Lys Arg Trp Ile Tyr
        35                  40                  45

Asp Thr Ser Lys Val Ala Ser Gly Val Pro Tyr Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Trp Ser Ser Asn Pro Leu Thr
                85                  90                  95

Phe Gly Ser Gly Thr Lys Leu Glu Ile Asn
            100                 105

<210> 1208
<211> 119
<212> PRT
<213> Artificial Sequence

<220>
<221> source
<223> /note="Description of Artificial Sequence: Synthetic
      polypeptide"

<400> 1208

<210> 1209
<211> 106
<212> PRT
<213> Artificial Sequence

<220>
<221> source
<223> /note="Description of Artificial Sequence: Synthetic
      polypeptide"

<400> 1209


<210> 1210
<211> 118
<212> PRT
<213> Artificial Sequence

<220>
<221> source
<223> /note="Description of Artificial Sequence: Synthetic
      polypeptide"

<400> 1210

<210> 1211
<211> 108
<212> PRT
<213> Artificial Sequence

<220>
<221> source
<223> /note="Description of Artificial Sequence: Synthetic
      polypeptide"

<400> 1211

<210> 1212
<211> 124
<212> PRT

<213> Artificial Sequence

<220>
<221> source
<223> /note="Description of Artificial Sequence: Synthetic
      polypeptide"

<400> 1212
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Gly Arg Ile Arg Ser Lys Tyr Asn Asn Tyr Ala Thr Tyr Tyr Ala Asp
    50                  55                  60

Ser Val Lys Asp Arg Phe Ile Ser Arg Asp Asp Ser Lys Asn Ser Leu
65                  70                  75                  80

Tyr Leu Gln Met Asn Ser Leu Lys Thr Glu Asp Thr Ala Val Tyr Tyr
                85                  90                  95

Cys Val Arg His Gly Asn Phe Gly Asn Ser Tyr Val Ser Trp Phe Ala
            100                 105                 110

Tyr Trp Gly Gln Gly Thr Leu Val Thr Val Ser Ser
        115                 120


<210> 1213
<211> 107
<212> PRT
<213> Artificial Sequence

<220>
<221> source
<223> /note="Description of Artificial Sequence: Synthetic
      polypeptide"

<400> 1213
Gln Ala Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Trp Thr Pro Ala Arg Phe
    50                  55                  60

Ser Gly Ser Leu Leu Gly Gly Lys Ala Ala Leu Ile Gly Ala Gln Ala
65                  70                  75                  80

Glu Asp Glu Ala Asp Tyr Tyr Cys Ala Leu Trp Tyr Ser Asn Leu Trp
                85                  90                  95

Val Phe Gly Gly Gly Thr Lys Leu Thr Val Leu
            100                 105

<210> 1214
<211> 119
<212> PRT
<213> Artificial Sequence

<220>
<221> source
<223> /note="Description of Artificial Sequence: Synthetic
      polypeptide"

<400> 1214
Asp Ile Lys Leu Gln Gln Ser Gly Ala Glu Leu Ala Arg Pro Gly Ala
1               5                   10                  15

Ser Val Lys Met Ser Cys Lys Thr Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser
        115

<210> 1215
<211> 105
<212> PRT
<213> Artificial Sequence

<220>
<221> source
<223> /note="Description of Artificial Sequence: Synthetic
      polypeptide"

<400> 1215
Asp Ile Gln Leu Thr Gln Ser Pro Ala Ile Met Ser Ala Ser Pro Gly
1               5                   10                  15

Glu Lys Val Thr Met Thr Cys Arg Ala Ser Ser Ser Val Ser Tyr Met
            20                  25                  30

Asn Trp Tyr Gln Gln Lys Ser Gly Thr Ser Pro Lys Arg Trp Ile Tyr
        35                  40                  45

Asp Thr Ser Lys Val Ala Ser Gly Val Pro Tyr Arg Phe Ser Gly Ser
    50                  55                  60

Gly Ser Gly Thr Ser Tyr Ser Leu Ile Ser Ser Met Glu Ala Glu Asp
65                  70                  75                  80

Ala Ala Thr Tyr Tyr Cys Gln Gln Trp Ser Ser Asn Pro Leu Thr Phe
                85                  90                  95

Gly Ala Gly Thr Lys Leu Glu Leu Lys
            100                 105


<210> 1216
<211> 124
<212> PRT
<213> Artificial Sequence

<220>
<221> source
<223> /note="Description of Artificial Sequence: Synthetic
      polypeptide"

<400> 1216
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Ile Ser Arg Asp Asp Ser Lys Asn Ser Leu

65                  70                  75                  80

Tyr Leu Gln Met Asn Ser Leu Lys Thr Glu Asp Thr Ala Val Tyr Tyr
                85                  90                  95

Cys Val Arg His Gly Asn Phe Gly Asn Ser Tyr Val Ser Trp Phe Ala
            100                 105                 110

Tyr Trp Gly Gln Gly Thr Leu Val Thr Val Ser Ser
        115                 120

<210> 1217
<211> 107
<212> PRT
<213> Artificial Sequence

<220>
<221> source
<223> /note="Description of Artificial Sequence: Synthetic
      polypeptide"

<400> 1217
Gln Ala Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Trp Thr Pro Ala Arg Phe
    50                  55                  60

Ser Gly Ser Leu Leu Gly Gly Lys Ala Ala Leu Ile Gly Ala Gln Ala
65                  70                  75                  80

Glu Asp Glu Ala Asp Tyr Tyr Cys Ala Leu Trp Tyr Ser Asn Leu Trp
                85                  90                  95

Val Phe Gly Gly Gly Thr Lys Leu Thr Val Leu
            100                 105

<210> 1218
<211> 125
<212> PRT
<213> Artificial Sequence

<220>
<221> source
<223> /note="Description of Artificial Sequence: Synthetic
      polypeptide"

<400> 1218
Glu Val Lys Leu Leu Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Gln Ser Ile
65                  70                  75                  80

Leu Tyr Leu Gln Met Asn Asn Leu Lys Thr Glu Asp Thr Ala Met Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ala
        115                 120                 125

<210> 1219
<211> 109
<212> PRT
<213> Artificial Sequence

<220>
<221> source
<223> /note="Description of Artificial Sequence: Synthetic
      polypeptide"

<400> 1219
Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Lys Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

Leu Trp Val Phe Gly Gly Gly Thr Lys Leu Thr Val Leu
            100                 105

<210> 1220
<211> 125
<212> PRT
<213> Artificial Sequence

<220>
<221> source
<223> /note="Description of Artificial Sequence: Synthetic
      polypeptide"

<400> 1220
Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Asn Ser Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Gly Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Val Ser Trp Trp
            100                 105                 110

Ala Tyr Trp Gly Gln Gly Thr Leu Val Thr Val Ser Ser
        115                 120                 125

<210> 1221
<211> 109
<212> PRT
<213> Artificial Sequence

<220>
<221> source
<223> /note="Description of Artificial Sequence: Synthetic
      polypeptide"

<400> 1221
Gln Thr Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

Asn Tyr Pro Asn Trp Val Gln Gln Lys Pro Gly Gln Ala Pro Arg Gly
        35                  40                  45

Leu Ile Gly Gly Thr Lys Phe Leu Ala Pro Gly Thr Pro Gln Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Val Leu Trp Tyr Ser Asn
                85                  90                  95

Arg Trp Val Phe Gly Gly Gly Thr Lys Leu Thr Val Leu
            100                 105


<210> 1222
<211> 125
<212> PRT
<213> Artificial Sequence

<220>
<221> source
<223> /note="Description of Artificial Sequence: Synthetic
      polypeptide"

<400> 1222
Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Asn Lys Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Ile Ser Tyr Trp
                100                 105                 110

Ala Tyr Trp Gly Gln Gly Thr Leu Val Thr Val Ser Ser
        115                 120                 125

<210> 1223
<211> 109
<212> PRT
<213> Artificial Sequence

<220>
<221> source
<223> /note="Description of Artificial Sequence: Synthetic
      polypeptide"

<400> 1223
Gln Thr Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

Asn Tyr Pro Asn Trp Val Gln Gln Lys Pro Gly Gln Ala Pro Arg Gly
        35                  40                  45

Leu Ile Gly Gly Thr Lys Phe Leu Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Val Leu Trp Tyr Ser Asn
                85                  90                  95

Arg Trp Val Phe Gly Gly Gly Thr Lys Leu Thr Val Leu
            100                 105

<210> 1224
<211> 119
<212> PRT
<213> Artificial Sequence

<220>
<221> source
<223> /note="Description of Artificial Sequence: Synthetic
      polypeptide"

<400> 1224
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Arg Tyr
            20                  25                  30

Thr Met His Trp Val Arg Gln Ala Pro Gly Gln Gly Leu Glu Trp Met
        35                  40                  45

Gly Tyr Ile Asn Pro Ser Arg Gly Tyr Thr Asn Tyr Asn Gln Lys Phe
    50                  55                  60

Lys Asp Arg Val Thr Met Thr Thr Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Leu Val Thr Val Ser Ser
        115

<210> 1225
<211> 106
<212> PRT
<213> Artificial Sequence

<220>
<221> source
<223> /note="Description of Artificial Sequence: Synthetic
polypeptide"

<400> 1225
Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

Asn Trp Tyr Gln Gln Lys Pro Gly Gln Ala Pro Arg Leu Leu Ile Tyr
        35                  40                  45

Asp Thr Ser Lys Leu Ala Ser Gly Val Pro Ala His Phe Arg Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Ser Asn Pro Phe Thr
                85                  90                  95

Phe Gly Gln Gly Thr Lys Val Glu Ile Lys
            100                 105

<210> 1226
<211> 125
<212> PRT
<213> Artificial Sequence

<220>
<221> source
<223> /note="Description of Artificial Sequence: Synthetic
      polypeptide"

<400> 1226
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Ser Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Ala Arg His Gly Asn Phe Gly Asn Ser Tyr Val Ser Trp Phe
            100                 105                 110

Ala Tyr Trp Gly Gln Gly Thr Met Val Thr Val Ser Ser
        115                 120                 125

<210> 1227
<211> 109
<212> PRT
<213> Artificial Sequence

<220>
<221> source
<223> /note="Description of Artificial Sequence: Synthetic
      polypeptide"

<400> 1227
Gln Ala Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser

                  20                          25                          30

Asn Tyr Ala Asn Trp Val Gln Gln Lys Pro Gly Gln Ala Pro Arg Gly
            35                          40                          45

Leu Ile Gly Gly Thr Asn Lys Arg Ala Pro Gly Thr Pro Ala Arg Phe
      50                          55                          60

Ser Gly Ser Leu Leu Gly Gly Lys Ala Ala Leu Thr Leu Ser Gly Val
65                          70                          75                          80

Gln Pro Glu Asp Glu Ala Glu Tyr Tyr Cys Ala Leu Trp Tyr Ser Asn
                        85                          90                          95

Leu Trp Val Phe Gly Gly Gly Thr Lys Leu Thr Val Leu
                  100                         105

<210> 1228
<211> 125
<212> PRT
<213> Artificial Sequence

<220>
<221> source
<223> /note="Description of Artificial Sequence: Synthetic
      polypeptide"

<400> 1228
Glu Val Gln Leu Val Glu Ser Gly Gly Gly Leu Val Gln Pro Gly Gly
1                       5                           10                          15

Ser Leu Arg Leu Ser Cys Ala Ala Ser Gly Phe Thr Phe Asn Thr Tyr
                  20                          25                          30

Ala Met Asn Trp Val Arg Gln Ala Pro Gly Lys Gly Leu Glu Trp Val
            35                          40                          45

Gly Arg Ile Arg Ser Lys Tyr Asn Asn Tyr Ala Thr Tyr Tyr Ala Asp
      50                          55                          60

Ser Val Lys Asp Arg Phe Thr Ile Ser Arg Asp Asp Ser Lys Asn Ser
65                          70                          75                          80

Leu Tyr Leu Gln Met Asn Ser Leu Lys Thr Glu Asp Thr Ala Val Tyr
                        85                          90                          95

Tyr Cys Val Arg His Gly Asn Phe Gly Asn Ser Tyr Val Ser Trp Phe
                  100                         105                         110

Ala Tyr Trp Gly Gln Gly Thr Leu Val Thr Val Ser Ser

115                 120                 125

<210> 1229
<211> 109
<212> PRT
<213> Artificial Sequence

<220>
<221> source
<223> /note="Description of Artificial Sequence: Synthetic
      polypeptide"

<400> 1229
Gln Ala Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Gly Gln Ala Pro Arg Gly
        35                  40                  45

Leu Ile Gly Gly Thr Asn Lys Arg Ala Pro Trp Thr Pro Ala Arg Phe
    50                  55                  60

Ser Gly Ser Leu Leu Gly Gly Lys Ala Ala Leu Thr Ile Thr Gly Ala
65                  70                  75                  80

Gln Ala Glu Asp Glu Ala Asp Tyr Tyr Cys Ala Leu Trp Tyr Ser Asn
                85                  90                  95

Leu Trp Val Phe Gly Gly Gly Thr Lys Leu Thr Val Leu
            100                 105

<210> 1230
<211> 125
<212> PRT
<213> Artificial Sequence

<220>
<221> source
<223> /note="Description of Artificial Sequence: Synthetic
      polypeptide"

<400> 1230
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Gly Arg Ile Arg Ser Lys Tyr Asn Asn Tyr Ala Thr Tyr Tyr Ala Asp
    50                  55                  60

Ser Val Lys Gly Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser
        115                 120                 125

<210> 1231
<211> 109
<212> PRT
<213> Artificial Sequence

<220>
<221> source
<223> /note="Description of Artificial Sequence: Synthetic
      polypeptide"

<400> 1231
Gln Ala Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Gly Val Pro Ala Arg Phe
    50                  55                  60

Ser Gly Ser Leu Leu Gly Gly Lys Ala Ala Leu Thr Leu Ser Gly Ala
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu
            100                 105

<210> 1232
<211> 125

<212> PRT
<213> Artificial Sequence

<220>
<221> source
<223> /note="Description of Artificial Sequence: Synthetic
      polypeptide"

<400> 1232
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Gly Arg Ile Arg Ser Lys Tyr Asn Asn Tyr Ala Thr Tyr Tyr Ala Asp
    50                  55                  60

Ser Val Lys Gly Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Val Arg His Gly Asn Phe Gly Asp Ser Tyr Val Ser Trp Phe
            100                 105                 110

Ala Tyr Trp Gly Gln Gly Thr Leu Val Thr Val Ser Ser
        115                 120                 125


<210> 1233
<211> 109
<212> PRT
<213> Artificial Sequence

<220>
<221> source
<223> /note="Description of Artificial Sequence: Synthetic
      polypeptide"

<400> 1233
Gln Ala Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Thr Ser
            20                  25                  30

Asn Tyr Ala Asn Trp Val Gln Gln Lys Pro Gly Lys Ser Pro Arg Gly
        35                  40                  45

Leu Ile Gly Gly Thr Asn Lys Arg Ala Pro Gly Val Pro Ala Arg Phe
    50                  55                  60

Ser Gly Ser Leu Leu Gly Gly Lys Ala Ala Leu Thr Ile Ser Gly Ala
65                  70                  75                  80

Gln Pro Glu Asp Glu Ala Asp Tyr Tyr Cys Ala Leu Trp Tyr Ser Asn
                85                  90                  95

His Trp Val Phe Gly Gly Gly Thr Lys Leu Thr Val Leu
            100                 105

<210> 1234
<211> 119
<212> PRT
<213> Artificial Sequence

<220>
<221> source
<223> /note="Description of Artificial Sequence: Synthetic
      polypeptide"

<400> 1234

<210> 1235
<211> 101
<212> PRT

<213> Artificial Sequence

<220>
<221> source
<223> /note="Description of Artificial Sequence: Synthetic
      polypeptide"

<400> 1235
Asp Ile Gln Met Thr Gln Ser Pro Ser Ser Leu Ser Ala Ser Val Gly
1               5                   10                  15

Asp Arg Val Thr Ile Thr Cys Ser Ala Ser Ser Ser Val Ser Tyr Met
            20                  25                  30

Asn Trp Tyr Gln Gln Thr Pro Gly Lys Ala Pro Lys Arg Trp Ile Tyr
        35                  40                  45

Asp Thr Ser Lys Leu Ala Ser Gly Val Pro Ser Arg Phe Ser Gly Ser
    50                  55                  60

Gly Ser Gly Thr Asp Tyr Thr Phe Thr Ile Ser Ser Leu Gln Pro Glu
65                  70                  75                  80

Asp Ile Ala Thr Tyr Tyr Cys Gln Gln Trp Ser Ser Asn Pro Phe Thr
                85                  90                  95

Phe Gly Gln Gly Thr
            100


<210> 1236
<211> 119
<212> PRT
<213> Artificial Sequence

<220>
<221> source
<223> /note="Description of Artificial Sequence: Synthetic
      polypeptide"

<400> 1236
Gln Val Gln Leu Val Gln Ser Gly Gly Gly Val Val Gln Pro Gly Arg
1               5                   10                  15

Ser Leu Arg Leu Ser Cys Lys Ala Ser Gly Tyr Thr Phe Thr Arg Tyr
            20                  25                  30

Thr Met His Trp Val Arg Gln Ala Pro Gly Lys Gly Leu Glu Trp Ile
        35                  40                  45

Gly Tyr Ile Asn Pro Ser Arg Gly Tyr Thr Asn Tyr Asn Gln Lys Val
    50                  55                  60

Lys Asp Arg Phe Thr Ile Ser Arg Asp Asn Ser Lys Asn Thr Ala Phe
65                  70                  75                  80

Leu Gln Met Asp Ser Leu Arg Pro Glu Asp Thr Gly Val Tyr Phe Cys
                85                  90                  95

Ala Arg Tyr Tyr Asp Asp His Tyr Ser Leu Asp Tyr Trp Gly Gln Gly
            100                 105                 110

Thr Pro Val Thr Val Ser Ser
        115

<210> 1237
<211> 101
<212> PRT
<213> Artificial Sequence

<220>
<221> source
<223> /note="Description of Artificial Sequence: Synthetic
      polypeptide"

<400> 1237
Asp Ile Gln Met Thr Gln Ser Pro Ser Ser Leu Ser Ala Ser Val Gly
1               5                   10                  15

Asp Arg Val Thr Ile Thr Cys Ser Ala Ser Ser Ser Val Ser Tyr Met
            20                  25                  30

Asn Trp Tyr Gln Gln Thr Pro Gly Lys Ala Pro Lys Arg Trp Ile Tyr
        35                  40                  45

Asp Thr Ser Lys Leu Ala Ser Gly Val Pro Ser Arg Phe Ser Gly Ser
    50                  55                  60

Gly Ser Gly Thr Asp Tyr Thr Phe Thr Ile Ser Ser Leu Gln Pro Glu
65                  70                  75                  80

Asp Ile Ala Thr Tyr Tyr Cys Gln Gln Trp Ser Ser Asn Pro Phe Thr
                85                  90                  95

Phe Gly Gln Gly Thr
            100

<210> 1238

<400> 1238
000

<210> 1239

<400> 1239
000

<210> 1240

<400> 1240
000

<210> 1241

<400> 1241
000

<210> 1242

<400> 1242
000

<210> 1243

<400> 1243
000

<210> 1244

<400> 1244
000

<210> 1245

<400> 1245
000

<210> 1246

<400> 1246
000

<210> 1247

<400> 1247
000

<210> 1248

<400> 1248
000

<210> 1249

<400> 1249
000

<210> 1250

<400> 1250
000

<210> 1251

<400> 1251
000

<210> 1252

<400> 1252
000

<210> 1253

<400> 1253
000

<210> 1254

<400> 1254
000

<210> 1255

<400> 1255
000

<210> 1256

<400> 1256
000

<210> 1257

<400> 1257
000

<210> 1258

<400> 1258
000

<210> 1259

<400> 1259
000

<210> 1260

<400> 1260
000

<210> 1261

<400> 1261
000

<210> 1262

<400> 1262
000

<210> 1263

<400> 1263
000

<210> 1264

<400> 1264
000

<210> 1265

<400> 1265
000

<210> 1266

<400> 1266
000

<210> 1267

<400> 1267
000

<210> 1268

<400> 1268
000

<210> 1269

<400> 1269
000

<210> 1270

<400> 1270
000

<210> 1271

<400> 1271
000

<210> 1272

<400> 1272
000

<210> 1273

<400> 1273
000

<210> 1274

<400> 1274
000

<210> 1275

<400> 1275
000

<210> 1276

<400> 1276
000

<210> 1277

<400> 1277
000

<210> 1278

<400> 1278
000

<210> 1279

<400> 1279
000

<210> 1280

<400> 1280
000

<210> 1281

<400> 1281
000

<210> 1282

<400> 1282
000

<210> 1283

<400> 1283
000

<210> 1284

<400> 1284
000

<210> 1285

<400> 1285
000

<210> 1286

<400> 1286
000

<210> 1287

<400> 1287
000

<210> 1288

<400> 1288
000

<210> 1289

<400> 1289
000

<210> 1290

<400> 1290
000

<210> 1291

<400> 1291
000

<210> 1292

<400> 1292
000


<210> 1293

<400> 1293
000


<210> 1294

<400> 1294
000


<210> 1295

<400> 1295
000


<210> 1296

<400> 1296
000


<210> 1297

<400> 1297
000


<210> 1298

<400> 1298
000


<210> 1299

<400> 1299
000


<210> 1300
<211> 10
<212> PRT
<213> Artificial Sequence

<220>
<221> source
<223> /note="Description of Artificial Sequence: Synthetic
      peptide"

<400> 1300
Gly Tyr Thr Phe Thr Arg Tyr Thr Met His
1               5                   10

<210> 1301
<211> 10
<212> PRT
<213> Artificial Sequence

<220>
<221> source
<223> /note="Description of Artificial Sequence: Synthetic
      peptide"

<400> 1301
Ser Ala Ser Ser Ser Val Ser Tyr Met Asn
1               5                   10


<210> 1302
<211> 10
<212> PRT
<213> Artificial Sequence

<220>
<221> source
<223> /note="Description of Artificial Sequence: Synthetic
      peptide"

<400> 1302
Gly Phe Thr Phe Asn Thr Tyr Ala Met Asn
1               5                   10


<210> 1303
<211> 14
<212> PRT
<213> Artificial Sequence

<220>
<221> source
<223> /note="Description of Artificial Sequence: Synthetic
      peptide"

<400> 1303
Arg Ser Ser Thr Gly Ala Val Thr Thr Ser Asn Tyr Ala Asn
1               5                   10


<210> 1304
<211> 10
<212> PRT
<213> Artificial Sequence

<220>
<221> source
<223> /note="Description of Artificial Sequence: Synthetic
      peptide"

<400> 1304
Gly Tyr Thr Phe Thr Ser Tyr Thr Met His
1               5                   10


<210> 1305
<211> 10

<212> PRT
<213> Artificial Sequence

<220>
<221> source
<223> /note="Description of Artificial Sequence: Synthetic
      peptide"

<400> 1305
Arg Ala Ser Ser Ser Val Ser Tyr Met His
1               5                   10


<210> 1306
<211> 10
<212> PRT
<213> Artificial Sequence

<220>
<221> source
<223> /note="Description of Artificial Sequence: Synthetic
      peptide"

<400> 1306
Gly Tyr Thr Phe Thr Arg Tyr Thr Met His
1               5                   10


<210> 1307
<211> 10
<212> PRT
<213> Artificial Sequence

<220>
<221> source
<223> /note="Description of Artificial Sequence: Synthetic
      peptide"

<400> 1307
Arg Ala Ser Ser Ser Val Ser Tyr Met Asn
1               5                   10


<210> 1308
<211> 10
<212> PRT
<213> Artificial Sequence

<220>
<221> source
<223> /note="Description of Artificial Sequence: Synthetic
      peptide"

<400> 1308
Gly Tyr Thr Phe Thr Arg Tyr Thr Met His
1               5                   10


<210> 1309
<211> 10
<212> PRT
<213> Artificial Sequence

<220>
<221> source
<223> /note="Description of Artificial Sequence: Synthetic
      peptide"

<400> 1309
Ser Ala Ser Ser Ser Val Ser Tyr Met Asn
1               5                   10


<210> 1310
<211> 10
<212> PRT
<213> Artificial Sequence

<220>
<221> source
<223> /note="Description of Artificial Sequence: Synthetic
      peptide"

<400> 1310
Gly Phe Lys Phe Ser Gly Tyr Gly Met His
1               5                   10


<210> 1311
<211> 11
<212> PRT
<213> Artificial Sequence

<220>
<221> source
<223> /note="Description of Artificial Sequence: Synthetic
      peptide"

<400> 1311
Arg Ala Ser Gln Ser Val Ser Ser Tyr Leu Ala
1               5                   10


<210> 1312
<211> 10
<212> PRT
<213> Artificial Sequence

<220>
<221> source
<223> /note="Description of Artificial Sequence: Synthetic
      peptide"

<400> 1312
Gly Phe Thr Phe Ser Thr Tyr Ala Met Asn
1               5                   10


<210> 1313
<211> 14
<212> PRT
<213> Artificial Sequence

<220>
<221> source
<223> /note="Description of Artificial Sequence: Synthetic

peptide"

<400> 1313
Arg Ser Ser Thr Gly Ala Val Thr Thr Ser Asn Tyr Ala Asn
1               5                   10


<210> 1314
<211> 10
<212> PRT
<213> Artificial Sequence

<220>
<221> source
<223> /note="Description of Artificial Sequence: Synthetic
      peptide"

<400> 1314
Gly Tyr Thr Phe Thr Arg Tyr Thr Met His
1               5                   10


<210> 1315
<211> 10
<212> PRT
<213> Artificial Sequence

<220>
<221> source
<223> /note="Description of Artificial Sequence: Synthetic
      peptide"

<400> 1315
Arg Ala Ser Ser Ser Val Ser Tyr Met Asn
1               5                   10


<210> 1316
<211> 10
<212> PRT
<213> Artificial Sequence

<220>
<221> source
<223> /note="Description of Artificial Sequence: Synthetic
      peptide"

<400> 1316
Gly Phe Thr Phe Asn Thr Tyr Ala Met Asn
1               5                   10


<210> 1317
<211> 14
<212> PRT
<213> Artificial Sequence

<220>
<221> source
<223> /note="Description of Artificial Sequence: Synthetic
      peptide"

<400> 1317

Arg Ser Ser Thr Gly Ala Val Thr Thr Ser Asn Tyr Ala Asn
1               5                   10

<210> 1318
<211> 10
<212> PRT
<213> Artificial Sequence

<220>
<221> source
<223> /note="Description of Artificial Sequence: Synthetic
      peptide"

<400> 1318
Gly Phe Thr Phe Asn Thr Tyr Ala Met Asn
1               5                   10

<210> 1319
<211> 14
<212> PRT
<213> Artificial Sequence

<220>
<221> source
<223> /note="Description of Artificial Sequence: Synthetic
      peptide"

<400> 1319
Arg Ser Ser Thr Gly Ala Val Thr Thr Ser Asn Tyr Ala Asn
1               5                   10

<210> 1320
<211> 10
<212> PRT
<213> Artificial Sequence

<220>
<221> source
<223> /note="Description of Artificial Sequence: Synthetic
      peptide"

<400> 1320
Gly Phe Thr Phe Asn Ser Tyr Ala Met Asn
1               5                   10

<210> 1321
<211> 14
<212> PRT
<213> Artificial Sequence

<220>
<221> source
<223> /note="Description of Artificial Sequence: Synthetic
      peptide"

<400> 1321
Gly Ser Ser Thr Gly Ala Val Thr Ser Gly Asn Tyr Pro Asn
1               5                   10

<210> 1322
<211> 10
<212> PRT
<213> Artificial Sequence

<220>
<221> source
<223> /note="Description of Artificial Sequence: Synthetic
      peptide"

<400> 1322
Gly Phe Thr Phe Asn Lys Tyr Ala Met Asn
1               5                   10


<210> 1323
<211> 14
<212> PRT
<213> Artificial Sequence

<220>
<221> source
<223> /note="Description of Artificial Sequence: Synthetic
      peptide"

<400> 1323
Gly Ser Ser Thr Gly Ala Val Thr Ser Gly Asn Tyr Pro Asn
1               5                   10


<210> 1324
<211> 10
<212> PRT
<213> Artificial Sequence

<220>
<221> source
<223> /note="Description of Artificial Sequence: Synthetic
      peptide"

<400> 1324
Gly Tyr Thr Phe Thr Arg Tyr Thr Met His
1               5                   10


<210> 1325
<211> 10
<212> PRT
<213> Artificial Sequence

<220>
<221> source
<223> /note="Description of Artificial Sequence: Synthetic
      peptide"

<400> 1325
Ser Ala Ser Ser Ser Val Ser Tyr Met Asn
1               5                   10


<210> 1326
<211> 10

<212> PRT
<213> Artificial Sequence

<220>
<221> source
<223> /note="Description of Artificial Sequence: Synthetic
      peptide"

<400> 1326
Gly Phe Thr Phe Ser Thr Tyr Ala Met Asn
1               5                   10


<210> 1327
<211> 14
<212> PRT
<213> Artificial Sequence

<220>
<221> source
<223> /note="Description of Artificial Sequence: Synthetic
      peptide"

<400> 1327
Arg Ser Ser Thr Gly Ala Val Thr Thr Ser Asn Tyr Ala Asn
1               5                   10


<210> 1328
<211> 10
<212> PRT
<213> Artificial Sequence

<220>
<221> source
<223> /note="Description of Artificial Sequence: Synthetic
      peptide"

<400> 1328
Gly Phe Thr Phe Asn Thr Tyr Ala Met Asn
1               5                   10


<210> 1329
<211> 14
<212> PRT
<213> Artificial Sequence

<220>
<221> source
<223> /note="Description of Artificial Sequence: Synthetic
      peptide"

<400> 1329
Arg Ser Ser Thr Gly Ala Val Thr Thr Ser Asn Tyr Ala Asn
1               5                   10


<210> 1330
<211> 10
<212> PRT
<213> Artificial Sequence

<220>
<221> source
<223> /note="Description of Artificial Sequence: Synthetic
      peptide"

<400> 1330
Gly Phe Thr Phe Asn Thr Tyr Ala Met Asn
1               5                   10


<210> 1331
<211> 14
<212> PRT
<213> Artificial Sequence

<220>
<221> source
<223> /note="Description of Artificial Sequence: Synthetic
      peptide"

<400> 1331
Gly Ser Ser Thr Gly Ala Val Thr Thr Ser Asn Tyr Ala Asn
1               5                   10


<210> 1332
<211> 10
<212> PRT
<213> Artificial Sequence

<220>
<221> source
<223> /note="Description of Artificial Sequence: Synthetic
      peptide"

<400> 1332
Gly Phe Thr Phe Ser Thr Tyr Ala Met Asn
1               5                   10


<210> 1333
<211> 14
<212> PRT
<213> Artificial Sequence

<220>
<221> source
<223> /note="Description of Artificial Sequence: Synthetic
      peptide"

<400> 1333
Gly Ser Ser Thr Gly Ala Val Thr Thr Ser Asn Tyr Ala Asn
1               5                   10


<210> 1334
<211> 10
<212> PRT
<213> Artificial Sequence

<220>
<221> source
<223> /note="Description of Artificial Sequence: Synthetic

peptide"

<400> 1334
Gly Tyr Thr Phe Thr Arg Tyr Thr Met His
1               5                   10


<210> 1335
<211> 10
<212> PRT
<213> Artificial Sequence

<220>
<221> source
<223> /note="Description of Artificial Sequence: Synthetic
      peptide"

<400> 1335
Ser Ala Ser Ser Ser Val Ser Tyr Met Asn
1               5                   10


<210> 1336
<211> 10
<212> PRT
<213> Artificial Sequence

<220>
<221> source
<223> /note="Description of Artificial Sequence: Synthetic
      peptide"

<400> 1336
Gly Tyr Thr Phe Thr Arg Tyr Thr Met His
1               5                   10


<210> 1337
<211> 10
<212> PRT
<213> Artificial Sequence

<220>
<221> source
<223> /note="Description of Artificial Sequence: Synthetic
      peptide"

<400> 1337
Ser Ala Ser Ser Ser Val Ser Tyr Met Asn
1               5                   10


<210> 1338
<211> 17
<212> PRT
<213> Artificial Sequence

<220>
<221> source
<223> /note="Description of Artificial Sequence: Synthetic
      peptide"

<400> 1338

Tyr Ile Asn Pro Ser Arg Gly Tyr Thr Asn Tyr Asn Gln Lys Phe Lys
1               5                   10                  15

Asp

<210> 1339
<211> 7
<212> PRT
<213> Artificial Sequence

<220>
<221> source
<223> /note="Description of Artificial Sequence: Synthetic
      peptide"

<400> 1339
Asp Thr Ser Lys Leu Ala Ser
1               5

<210> 1340
<211> 19
<212> PRT
<213> Artificial Sequence

<220>
<221> source
<223> /note="Description of Artificial Sequence: Synthetic
      peptide"

<400> 1340
Arg Ile Arg Ser Lys Tyr Asn Asn Tyr Ala Thr Tyr Tyr Ala Asp Ser
1               5                   10                  15

Val Lys Asp

<210> 1341
<211> 7
<212> PRT
<213> Artificial Sequence

<220>
<221> source
<223> /note="Description of Artificial Sequence: Synthetic
      peptide"

<400> 1341
Gly Thr Asn Lys Arg Ala Pro
1               5

<210> 1342
<211> 17
<212> PRT
<213> Artificial Sequence

<220>

<221> source
<223> /note="Description of Artificial Sequence: Synthetic
      peptide"

<400> 1342
Tyr Ile Asn Pro Ser Ser Gly Tyr Thr Lys Tyr Asn Gln Lys Phe Lys
1               5                   10                  15

Asp

<210> 1343
<211> 7
<212> PRT
<213> Artificial Sequence

<220>
<221> source
<223> /note="Description of Artificial Sequence: Synthetic
      peptide"

<400> 1343
Ala Thr Ser Asn Leu Ala Ser
1               5

<210> 1344
<211> 17
<212> PRT
<213> Artificial Sequence

<220>
<221> source
<223> /note="Description of Artificial Sequence: Synthetic
      peptide"

<400> 1344
Tyr Ile Asn Pro Ser Arg Gly Tyr Thr Asn Tyr Asn Gln Lys Phe Lys
1               5                   10                  15

Asp

<210> 1345
<211> 7
<212> PRT
<213> Artificial Sequence

<220>
<221> source
<223> /note="Description of Artificial Sequence: Synthetic
      peptide"

<400> 1345
Asp Thr Ser Lys Val Ala Ser
1               5

<210> 1346

<211> 17
<212> PRT
<213> Artificial Sequence

<220>
<221> source
<223> /note="Description of Artificial Sequence: Synthetic
      peptide"

<400> 1346
Tyr Ile Asn Pro Ser Arg Gly Tyr Thr Asn Tyr Asn Gln Lys Val Lys
1               5                   10                  15

Asp

<210> 1347
<211> 7
<212> PRT
<213> Artificial Sequence

<220>
<221> source
<223> /note="Description of Artificial Sequence: Synthetic
      peptide"

<400> 1347
Asp Thr Ser Lys Leu Ala Ser
1               5

<210> 1348
<211> 17
<212> PRT
<213> Artificial Sequence

<220>
<221> source
<223> /note="Description of Artificial Sequence: Synthetic
      peptide"

<400> 1348
Val Ile Trp Tyr Asp Gly Ser Lys Lys Tyr Tyr Val Asp Ser Val Lys
1               5                   10                  15

Gly

<210> 1349
<211> 7
<212> PRT
<213> Artificial Sequence

<220>
<221> source
<223> /note="Description of Artificial Sequence: Synthetic
      peptide"

<400> 1349

Asp Ala Ser Asn Arg Ala Thr
1               5

<210> 1350
<211> 18
<212> PRT
<213> Artificial Sequence

<220>
<221> source
<223> /note="Description of Artificial Sequence: Synthetic
      peptide"

<400> 1350
Arg Ile Arg Ser Lys Tyr Asn Asn Tyr Ala Thr Tyr Tyr Ala Asp Ser
1               5                   10                  15

Val Lys

<210> 1351
<211> 7
<212> PRT
<213> Artificial Sequence

<220>
<221> source
<223> /note="Description of Artificial Sequence: Synthetic
      peptide"

<400> 1351
Gly Thr Asn Lys Arg Ala Pro
1               5

<210> 1352
<211> 17
<212> PRT
<213> Artificial Sequence

<220>
<221> source
<223> /note="Description of Artificial Sequence: Synthetic
      peptide"

<400> 1352
Tyr Ile Asn Pro Ser Arg Gly Tyr Thr Asn Tyr Asn Gln Lys Phe Lys
1               5                   10                  15

Asp

<210> 1353
<211> 7
<212> PRT
<213> Artificial Sequence

<220>

<221> source
<223> /note="Description of Artificial Sequence: Synthetic
      peptide"

<400> 1353
Asp Thr Ser Lys Val Ala Ser
1               5


<210> 1354
<211> 18
<212> PRT
<213> Artificial Sequence

<220>
<221> source
<223> /note="Description of Artificial Sequence: Synthetic
      peptide"

<400> 1354
Arg Ile Arg Ser Lys Tyr Asn Asn Tyr Ala Thr Tyr Tyr Ala Asp Ser
1               5                   10                  15


Val Lys



<210> 1355
<211> 7
<212> PRT
<213> Artificial Sequence

<220>
<221> source
<223> /note="Description of Artificial Sequence: Synthetic
      peptide"

<400> 1355
Gly Thr Asn Lys Arg Ala Pro
1               5


<210> 1356
<211> 19
<212> PRT
<213> Artificial Sequence

<220>
<221> source
<223> /note="Description of Artificial Sequence: Synthetic
      peptide"

<400> 1356
Arg Ile Arg Ser Lys Tyr Asn Asn Tyr Ala Thr Tyr Tyr Ala Asp Ser
1               5                   10                  15


Val Lys Asp



<210> 1357

<211> 7
<212> PRT
<213> Artificial Sequence

<220>
<221> source
<223> /note="Description of Artificial Sequence: Synthetic
      peptide"

<400> 1357
Gly Thr Asn Lys Arg Ala Pro
1               5


<210> 1358
<211> 19
<212> PRT
<213> Artificial Sequence

<220>
<221> source
<223> /note="Description of Artificial Sequence: Synthetic
      peptide"

<400> 1358
Arg Ile Arg Ser Lys Tyr Asn Asn Tyr Ala Thr Tyr Tyr Ala Asp Ser
1               5                   10                  15


Val Lys Gly



<210> 1359
<211> 7
<212> PRT
<213> Artificial Sequence

<220>
<221> source
<223> /note="Description of Artificial Sequence: Synthetic
      peptide"

<400> 1359
Gly Thr Lys Phe Leu Ala Pro
1               5


<210> 1360
<211> 19
<212> PRT
<213> Artificial Sequence

<220>
<221> source
<223> /note="Description of Artificial Sequence: Synthetic
      peptide"

<400> 1360
Arg Ile Arg Ser Lys Tyr Asn Asn Tyr Ala Thr Tyr Tyr Ala Asp Ser
1               5                   10                  15

Val Lys Asp

<210> 1361
<211> 7
<212> PRT
<213> Artificial Sequence

<220>
<221> source
<223> /note="Description of Artificial Sequence: Synthetic
      peptide"

<400> 1361
Gly Thr Lys Phe Leu Ala Pro
1               5

<210> 1362
<211> 17
<212> PRT
<213> Artificial Sequence

<220>
<221> source
<223> /note="Description of Artificial Sequence: Synthetic
      peptide"

<400> 1362
Tyr Ile Asn Pro Ser Arg Gly Tyr Thr Asn Tyr Asn Gln Lys Phe Lys
1               5                   10                  15

Asp

<210> 1363
<211> 7
<212> PRT
<213> Artificial Sequence

<220>
<221> source
<223> /note="Description of Artificial Sequence: Synthetic
      peptide"

<400> 1363
Asp Thr Ser Lys Leu Ala Ser
1               5

<210> 1364
<211> 19
<212> PRT
<213> Artificial Sequence

<220>
<221> source
<223> /note="Description of Artificial Sequence: Synthetic
      peptide"

<400> 1364
Arg Ile Arg Ser Lys Tyr Asn Asn Tyr Ala Thr Tyr Tyr Ala Asp Ser
1               5                   10                  15

Val Lys Asp

<210> 1365
<211> 7
<212> PRT
<213> Artificial Sequence

<220>
<221> source
<223> /note="Description of Artificial Sequence: Synthetic
      peptide"

<400> 1365
Gly Thr Asn Lys Arg Ala Pro
1               5

<210> 1366
<211> 19
<212> PRT
<213> Artificial Sequence

<220>
<221> source
<223> /note="Description of Artificial Sequence: Synthetic
      peptide"

<400> 1366
Arg Ile Arg Ser Lys Tyr Asn Asn Tyr Ala Thr Tyr Tyr Ala Asp Ser
1               5                   10                  15

Val Lys Asp

<210> 1367
<211> 7
<212> PRT
<213> Artificial Sequence

<220>
<221> source
<223> /note="Description of Artificial Sequence: Synthetic
      peptide"

<400> 1367
Gly Thr Asn Lys Arg Ala Pro
1               5

<210> 1368
<211> 19
<212> PRT
<213> Artificial Sequence

<220>
<221> source
<223> /note="Description of Artificial Sequence: Synthetic
      peptide"

<400> 1368
Arg Ile Arg Ser Lys Tyr Asn Asn Tyr Ala Thr Tyr Tyr Ala Asp Ser
1               5                   10                  15

Val Lys Gly

<210> 1369
<211> 7
<212> PRT
<213> Artificial Sequence

<220>
<221> source
<223> /note="Description of Artificial Sequence: Synthetic
      peptide"

<400> 1369
Gly Thr Asn Lys Arg Ala Pro
1               5

<210> 1370
<211> 19
<212> PRT
<213> Artificial Sequence

<220>
<221> source
<223> /note="Description of Artificial Sequence: Synthetic
      peptide"

<400> 1370
Arg Ile Arg Ser Lys Tyr Asn Asn Tyr Ala Thr Tyr Tyr Ala Asp Ser
1               5                   10                  15

Val Lys Gly

<210> 1371
<211> 7
<212> PRT
<213> Artificial Sequence

<220>
<221> source
<223> /note="Description of Artificial Sequence: Synthetic
      peptide"

<400> 1371
Gly Thr Asn Lys Arg Ala Pro
1               5

<210> 1372
<211> 17
<212> PRT
<213> Artificial Sequence

<220>
<221> source
<223> /note="Description of Artificial Sequence: Synthetic
      peptide"

<400> 1372
Tyr Ile Asn Pro Ser Arg Gly Tyr Thr Asn Tyr Asn Gln Lys Val Lys
1               5                   10                  15

Asp

<210> 1373
<211> 7
<212> PRT
<213> Artificial Sequence

<220>
<221> source
<223> /note="Description of Artificial Sequence: Synthetic
      peptide"

<400> 1373
Asp Thr Ser Lys Leu Ala Ser
1               5

<210> 1374
<211> 17
<212> PRT
<213> Artificial Sequence

<220>
<221> source
<223> /note="Description of Artificial Sequence: Synthetic
      peptide"

<400> 1374
Tyr Ile Asn Pro Ser Arg Gly Tyr Thr Asn Tyr Asn Gln Lys Val Lys
1               5                   10                  15

Asp

<210> 1375
<211> 7
<212> PRT
<213> Artificial Sequence

<220>
<221> source
<223> /note="Description of Artificial Sequence: Synthetic
      peptide"

<400> 1375
Asp Thr Ser Lys Leu Ala Ser
1               5

<210> 1376
<211> 10
<212> PRT
<213> Artificial Sequence

<220>
<221> source
<223> /note="Description of Artificial Sequence: Synthetic
      peptide"

<400> 1376
Tyr Tyr Asp Asp His Tyr Cys Leu Asp Tyr
1               5                   10

<210> 1377
<211> 9
<212> PRT
<213> Artificial Sequence

<220>
<221> source
<223> /note="Description of Artificial Sequence: Synthetic
      peptide"

<400> 1377
Gln Gln Trp Ser Ser Asn Pro Phe Thr
1               5

<210> 1378
<211> 14
<212> PRT
<213> Artificial Sequence

<220>
<221> source
<223> /note="Description of Artificial Sequence: Synthetic
      peptide"

<400> 1378
His Gly Asn Phe Gly Asn Ser Tyr Val Ser Trp Phe Ala Tyr
1               5                   10

<210> 1379
<211> 9
<212> PRT
<213> Artificial Sequence

<220>
<221> source
<223> /note="Description of Artificial Sequence: Synthetic
      peptide"

<400> 1379
Ala Leu Trp Tyr Ser Asn Leu Trp Val
1               5

<210> 1380
<211> 10
<212> PRT
<213> Artificial Sequence

<220>
<221> source
<223> /note="Description of Artificial Sequence: Synthetic
      peptide"

<400> 1380
Trp Gln Asp Tyr Asp Val Tyr Phe Asp Tyr
1               5                   10


<210> 1381
<211> 9
<212> PRT
<213> Artificial Sequence

<220>
<221> source
<223> /note="Description of Artificial Sequence: Synthetic
      peptide"

<400> 1381
Gln Gln Trp Ser Ser Asn Pro Pro Thr
1               5


<210> 1382
<211> 10
<212> PRT
<213> Artificial Sequence

<220>
<221> source
<223> /note="Description of Artificial Sequence: Synthetic
      peptide"

<400> 1382
Tyr Tyr Asp Asp His Tyr Cys Leu Asp Tyr
1               5                   10


<210> 1383
<211> 9
<212> PRT
<213> Artificial Sequence

<220>
<221> source
<223> /note="Description of Artificial Sequence: Synthetic
      peptide"

<400> 1383
Gln Gln Trp Ser Ser Asn Pro Leu Thr
1               5


<210> 1384

<211> 10
<212> PRT
<213> Artificial Sequence

<220>
<221> source
<223> /note="Description of Artificial Sequence: Synthetic
      peptide"

<400> 1384
Tyr Tyr Asp Asp His Tyr Cys Leu Asp Tyr
1               5                   10


<210> 1385
<211> 9
<212> PRT
<213> Artificial Sequence

<220>
<221> source
<223> /note="Description of Artificial Sequence: Synthetic
      peptide"

<400> 1385
Gln Gln Trp Ser Ser Asn Pro Phe Thr
1               5


<210> 1386
<211> 9
<212> PRT
<213> Artificial Sequence

<220>
<221> source
<223> /note="Description of Artificial Sequence: Synthetic
      peptide"

<400> 1386
Gln Met Gly Tyr Trp His Phe Asp Leu
1               5


<210> 1387
<211> 10
<212> PRT
<213> Artificial Sequence

<220>
<221> source
<223> /note="Description of Artificial Sequence: Synthetic
      peptide"

<400> 1387
Gln Gln Arg Ser Asn Trp Pro Pro Leu Thr
1               5                   10


<210> 1388
<211> 14
<212> PRT
<213> Artificial Sequence

<220>
<221> source
<223> /note="Description of Artificial Sequence: Synthetic
      peptide"

<400> 1388
His Gly Asn Phe Gly Asn Ser Tyr Val Ser Trp Phe Ala Tyr
1               5                   10


<210> 1389
<211> 9
<212> PRT
<213> Artificial Sequence

<220>
<221> source
<223> /note="Description of Artificial Sequence: Synthetic
      peptide"

<400> 1389
Ala Leu Trp Tyr Ser Asn Leu Trp Val
1               5


<210> 1390
<211> 10
<212> PRT
<213> Artificial Sequence

<220>
<221> source
<223> /note="Description of Artificial Sequence: Synthetic
      peptide"

<400> 1390
Tyr Tyr Asp Asp His Tyr Cys Leu Asp Tyr
1               5                   10


<210> 1391
<211> 9
<212> PRT
<213> Artificial Sequence

<220>
<221> source
<223> /note="Description of Artificial Sequence: Synthetic
      peptide"

<400> 1391
Gln Gln Trp Ser Ser Asn Pro Leu Thr
1               5


<210> 1392
<211> 14
<212> PRT
<213> Artificial Sequence

<220>
<221> source

<223> /note="Description of Artificial Sequence: Synthetic
      peptide"

<400> 1392
His Gly Asn Phe Gly Asn Ser Tyr Val Ser Trp Phe Ala Tyr
1               5                   10


<210> 1393
<211> 9
<212> PRT
<213> Artificial Sequence

<220>
<221> source
<223> /note="Description of Artificial Sequence: Synthetic
      peptide"

<400> 1393
Ala Leu Trp Tyr Ser Asn Leu Trp Val
1               5


<210> 1394
<211> 14
<212> PRT
<213> Artificial Sequence

<220>
<221> source
<223> /note="Description of Artificial Sequence: Synthetic
      peptide"

<400> 1394
His Gly Asn Phe Gly Asn Ser Tyr Val Ser Trp Phe Ala Tyr
1               5                   10


<210> 1395
<211> 9
<212> PRT
<213> Artificial Sequence

<220>
<221> source
<223> /note="Description of Artificial Sequence: Synthetic
      peptide"

<400> 1395
Ala Leu Trp Tyr Ser Asn Leu Trp Val
1               5


<210> 1396
<211> 14
<212> PRT
<213> Artificial Sequence

<220>
<221> source
<223> /note="Description of Artificial Sequence: Synthetic
      peptide"

<400> 1396
His Gly Asn Phe Gly Asn Ser Tyr Val Ser Trp Trp Ala Tyr
1               5                   10


<210> 1397
<211> 9
<212> PRT
<213> Artificial Sequence

<220>
<221> source
<223> /note="Description of Artificial Sequence: Synthetic
      peptide"

<400> 1397
Val Leu Trp Tyr Ser Asn Arg Trp Val
1               5


<210> 1398
<211> 14
<212> PRT
<213> Artificial Sequence

<220>
<221> source
<223> /note="Description of Artificial Sequence: Synthetic
      peptide"

<400> 1398
His Gly Asn Phe Gly Asn Ser Tyr Ile Ser Tyr Trp Ala Tyr
1               5                   10


<210> 1399
<211> 9
<212> PRT
<213> Artificial Sequence

<220>
<221> source
<223> /note="Description of Artificial Sequence: Synthetic
      peptide"

<400> 1399
Val Leu Trp Tyr Ser Asn Arg Trp Val
1               5


<210> 1400
<211> 10
<212> PRT
<213> Artificial Sequence

<220>
<221> source
<223> /note="Description of Artificial Sequence: Synthetic
      peptide"

<400> 1400
Tyr Tyr Asp Asp His Tyr Cys Leu Asp Tyr
1               5                   10

<210> 1401
<211> 9
<212> PRT
<213> Artificial Sequence

<220>
<221> source
<223> /note="Description of Artificial Sequence: Synthetic
      peptide"

<400> 1401
Gln Gln Trp Ser Ser Asn Pro Phe Thr
1               5


<210> 1402
<211> 14
<212> PRT
<213> Artificial Sequence

<220>
<221> source
<223> /note="Description of Artificial Sequence: Synthetic
      peptide"

<400> 1402
His Gly Asn Phe Gly Asn Ser Tyr Val Ser Trp Phe Ala Tyr
1               5                   10


<210> 1403
<211> 9
<212> PRT
<213> Artificial Sequence

<220>
<221> source
<223> /note="Description of Artificial Sequence: Synthetic
      peptide"

<400> 1403
Ala Leu Trp Tyr Ser Asn Leu Trp Val
1               5


<210> 1404
<211> 14
<212> PRT
<213> Artificial Sequence

<220>
<221> source
<223> /note="Description of Artificial Sequence: Synthetic
      peptide"

<400> 1404
His Gly Asn Phe Gly Asn Ser Tyr Val Ser Trp Phe Ala Tyr
1               5                   10


<210> 1405

<211> 9
<212> PRT
<213> Artificial Sequence

<220>
<221> source
<223> /note="Description of Artificial Sequence: Synthetic
      peptide"

<400> 1405
Ala Leu Trp Tyr Ser Asn Leu Trp Val
1               5


<210> 1406
<211> 14
<212> PRT
<213> Artificial Sequence

<220>
<221> source
<223> /note="Description of Artificial Sequence: Synthetic
      peptide"

<400> 1406
His Gly Asn Phe Gly Asn Ser Tyr Val Ser Trp Phe Ala Tyr
1               5                   10


<210> 1407
<211> 9
<212> PRT
<213> Artificial Sequence

<220>
<221> source
<223> /note="Description of Artificial Sequence: Synthetic
      peptide"

<400> 1407
Ala Leu Trp Tyr Ser Asn Leu Trp Val
1               5


<210> 1408
<211> 14
<212> PRT
<213> Artificial Sequence

<220>
<221> source
<223> /note="Description of Artificial Sequence: Synthetic
      peptide"

<400> 1408
His Gly Asn Phe Gly Asp Ser Tyr Val Ser Trp Phe Ala Tyr
1               5                   10


<210> 1409
<211> 9
<212> PRT
<213> Artificial Sequence

<220>
<221> source
<223> /note="Description of Artificial Sequence: Synthetic
      peptide"

<400> 1409
Ala Leu Trp Tyr Ser Asn His Trp Val
1               5


<210> 1410
<211> 10
<212> PRT
<213> Artificial Sequence

<220>
<221> source
<223> /note="Description of Artificial Sequence: Synthetic
      peptide"

<400> 1410
Tyr Tyr Asp Asp His Tyr Cys Leu Asp Tyr
1               5                   10


<210> 1411
<211> 9
<212> PRT
<213> Artificial Sequence

<220>
<221> source
<223> /note="Description of Artificial Sequence: Synthetic
      peptide"

<400> 1411
Gln Gln Trp Ser Ser Asn Pro Phe Thr
1               5


<210> 1412
<211> 10
<212> PRT
<213> Artificial Sequence

<220>
<221> source
<223> /note="Description of Artificial Sequence: Synthetic
      peptide"

<400> 1412
Tyr Tyr Asp Asp His Tyr Ser Leu Asp Tyr
1               5                   10


<210> 1413
<211> 9
<212> PRT
<213> Artificial Sequence

<220>
<221> source

<223> /note="Description of Artificial Sequence: Synthetic
      peptide"

<400> 1413
Gln Gln Trp Ser Ser Asn Pro Phe Thr
1               5


<210> 1414

<400> 1414
000


<210> 1415

<400> 1415
000


<210> 1416

<400> 1416
000


<210> 1417

<400> 1417
000


<210> 1418

<400> 1418
000


<210> 1419

<400> 1419
000


<210> 1420

<400> 1420
000


<210> 1421

<400> 1421
000


<210> 1422

<400> 1422
000


<210> 1423

<400> 1423
000

<210> 1424

<400> 1424
000

<210> 1425

<400> 1425
000

<210> 1426

<400> 1426
000

<210> 1427

<400> 1427
000

<210> 1428

<400> 1428
000

<210> 1429

<400> 1429
000

<210> 1430

<400> 1430
000

<210> 1431

<400> 1431
000

<210> 1432

<400> 1432
000

<210> 1433

<400> 1433
000

<210> 1434

<400> 1434
000

<210> 1435

<400> 1435
000

<210> 1436

<400> 1436
000

<210> 1437

<400> 1437
000

<210> 1438

<400> 1438
000

<210> 1439

<400> 1439
000

<210> 1440

<400> 1440
000

<210> 1441

<400> 1441
000

<210> 1442

<400> 1442
000

<210> 1443

<400> 1443
000

<210> 1444

<400> 1444
000

<210> 1445

<400> 1445
000

<210> 1446

<400> 1446
000

<210> 1447

<400> 1447
000

<210> 1448

<400> 1448
000

<210> 1449

<400> 1449
000

<210> 1450

<400> 1450
000

<210> 1451

<400> 1451
000

<210> 1452

<400> 1452
000

<210> 1453

<400> 1453
000

<210> 1454

<400> 1454
000

<210> 1455

<400> 1455
000

<210> 1456

<400> 1456
000

<210> 1457

<400> 1457
000

<210> 1458

<400> 1458
000

<210> 1459

<400> 1459
000

<210> 1460

<400> 1460
000

<210> 1461

<400> 1461
000

<210> 1462

<400> 1462
000

<210> 1463

<400> 1463
000

<210> 1464

<400> 1464
000

<210> 1465

<400> 1465
000

<210> 1466

<400> 1466
000

<210> 1467

<400> 1467
000

<210> 1468

<400> 1468
000

<210> 1469

<400> 1469
000

<210> 1470

<400> 1470
000

<210> 1471

<400> 1471
000

<210> 1472

<400> 1472
000

<210> 1473

<400> 1473
000

<210> 1474

<400> 1474
000

<210> 1475

<400> 1475
000

<210> 1476

<400> 1476
000

<210> 1477

<400> 1477
000

<210> 1478

<400> 1478
000

<210> 1479

<400> 1479
000

<210> 1480

<400> 1480
000

<210> 1481

<400> 1481
000

<210> 1482

<400> 1482
000

<210> 1483

<400> 1483
000

<210> 1484

<400> 1484
000

<210> 1485

<400> 1485
000

<210> 1486

<400> 1486
000

<210> 1487

<400> 1487
000

<210> 1488

<400> 1488
000

<210> 1489

<400> 1489
000

<210> 1490

<400> 1490
000

<210> 1491

<400> 1491
000

<210> 1492

<400> 1492
000

<210> 1493

<400> 1493
000

<210> 1494

<400> 1494
000

<210> 1495

<400> 1495
000

<210> 1496

<400> 1496
000

<210> 1497

<400> 1497
000


<210> 1498

<400> 1498
000


<210> 1499

<400> 1499
000


<210> 1500
<211> 7
<212> PRT
<213> Artificial Sequence

<220>
<221> source
<223> /note="Description of Artificial Sequence: Synthetic
      peptide"

<400> 1500
Gly Tyr Thr Phe Thr Arg Tyr
1               5


<210> 1501
<211> 6
<212> PRT
<213> Artificial Sequence

<220>
<221> source
<223> /note="Description of Artificial Sequence: Synthetic
      peptide"

<400> 1501
Ser Ser Ser Val Ser Tyr
1               5


<210> 1502
<211> 7
<212> PRT
<213> Artificial Sequence

<220>
<221> source
<223> /note="Description of Artificial Sequence: Synthetic
      peptide"

<400> 1502
Gly Phe Thr Phe Asn Thr Tyr
1               5

<210> 1503
<211> 10
<212> PRT
<213> Artificial Sequence

<220>
<221> source
<223> /note="Description of Artificial Sequence: Synthetic
      peptide"

<400> 1503
Ser Thr Gly Ala Val Thr Thr Ser Asn Tyr
1               5                   10


<210> 1504
<211> 7
<212> PRT
<213> Artificial Sequence

<220>
<221> source
<223> /note="Description of Artificial Sequence: Synthetic
      peptide"

<400> 1504
Gly Tyr Thr Phe Thr Ser Tyr
1               5


<210> 1505
<211> 6
<212> PRT
<213> Artificial Sequence

<220>
<221> source
<223> /note="Description of Artificial Sequence: Synthetic
      peptide"

<400> 1505
Ser Ser Ser Val Ser Tyr
1               5


<210> 1506
<211> 7
<212> PRT
<213> Artificial Sequence

<220>
<221> source
<223> /note="Description of Artificial Sequence: Synthetic
      peptide"

<400> 1506
Gly Tyr Thr Phe Thr Arg Tyr
1               5


<210> 1507

<211> 6
<212> PRT
<213> Artificial Sequence

<220>
<221> source
<223> /note="Description of Artificial Sequence: Synthetic
      peptide"

<400> 1507
Ser Ser Ser Val Ser Tyr
1               5


<210> 1508
<211> 7
<212> PRT
<213> Artificial Sequence

<220>
<221> source
<223> /note="Description of Artificial Sequence: Synthetic
      peptide"

<400> 1508
Gly Tyr Thr Phe Thr Arg Tyr
1               5


<210> 1509
<211> 6
<212> PRT
<213> Artificial Sequence

<220>
<221> source
<223> /note="Description of Artificial Sequence: Synthetic
      peptide"

<400> 1509
Ser Ser Ser Val Ser Tyr
1               5


<210> 1510
<211> 7
<212> PRT
<213> Artificial Sequence

<220>
<221> source
<223> /note="Description of Artificial Sequence: Synthetic
      peptide"

<400> 1510
Gly Phe Lys Phe Ser Gly Tyr
1               5


<210> 1511
<211> 7
<212> PRT
<213> Artificial Sequence

<220>
<221> source
<223> /note="Description of Artificial Sequence: Synthetic
      peptide"

<400> 1511
Ser Gln Ser Val Ser Ser Tyr
1               5


<210> 1512
<211> 7
<212> PRT
<213> Artificial Sequence

<220>
<221> source
<223> /note="Description of Artificial Sequence: Synthetic
      peptide"

<400> 1512
Gly Phe Thr Phe Ser Thr Tyr
1               5


<210> 1513
<211> 10
<212> PRT
<213> Artificial Sequence

<220>
<221> source
<223> /note="Description of Artificial Sequence: Synthetic
      peptide"

<400> 1513
Ser Thr Gly Ala Val Thr Thr Ser Asn Tyr
1               5                   10


<210> 1514
<211> 7
<212> PRT
<213> Artificial Sequence

<220>
<221> source
<223> /note="Description of Artificial Sequence: Synthetic
      peptide"

<400> 1514
Gly Tyr Thr Phe Thr Arg Tyr
1               5


<210> 1515
<211> 6
<212> PRT
<213> Artificial Sequence

<220>
<221> source

<223> /note="Description of Artificial Sequence: Synthetic
      peptide"

<400> 1515
Ser Ser Ser Val Ser Tyr
1               5


<210> 1516
<211> 7
<212> PRT
<213> Artificial Sequence

<220>
<221> source
<223> /note="Description of Artificial Sequence: Synthetic
      peptide"

<400> 1516
Gly Phe Thr Phe Asn Thr Tyr
1               5


<210> 1517
<211> 10
<212> PRT
<213> Artificial Sequence

<220>
<221> source
<223> /note="Description of Artificial Sequence: Synthetic
      peptide"

<400> 1517
Ser Thr Gly Ala Val Thr Thr Ser Asn Tyr
1               5                   10


<210> 1518
<211> 7
<212> PRT
<213> Artificial Sequence

<220>
<221> source
<223> /note="Description of Artificial Sequence: Synthetic
      peptide"

<400> 1518
Gly Phe Thr Phe Asn Thr Tyr
1               5


<210> 1519
<211> 10
<212> PRT
<213> Artificial Sequence

<220>
<221> source
<223> /note="Description of Artificial Sequence: Synthetic
      peptide"

<400> 1519
Ser Thr Gly Ala Val Thr Thr Ser Asn Tyr
1               5                   10


<210> 1520
<211> 7
<212> PRT
<213> Artificial Sequence

<220>
<221> source
<223> /note="Description of Artificial Sequence: Synthetic
      peptide"

<400> 1520
Gly Phe Thr Phe Asn Ser Tyr
1               5


<210> 1521
<211> 10
<212> PRT
<213> Artificial Sequence

<220>
<221> source
<223> /note="Description of Artificial Sequence: Synthetic
      peptide"

<400> 1521
Ser Thr Gly Ala Val Thr Ser Gly Asn Tyr
1               5                   10


<210> 1522
<211> 7
<212> PRT
<213> Artificial Sequence

<220>
<221> source
<223> /note="Description of Artificial Sequence: Synthetic
      peptide"

<400> 1522
Gly Phe Thr Phe Asn Lys Tyr
1               5


<210> 1523
<211> 10
<212> PRT
<213> Artificial Sequence

<220>
<221> source
<223> /note="Description of Artificial Sequence: Synthetic
      peptide"

<400> 1523
Ser Thr Gly Ala Val Thr Ser Gly Asn Tyr
1               5                   10

<210> 1524
<211> 7
<212> PRT
<213> Artificial Sequence

<220>
<221> source
<223> /note="Description of Artificial Sequence: Synthetic
      peptide"

<400> 1524
Gly Tyr Thr Phe Thr Arg Tyr
1               5


<210> 1525
<211> 6
<212> PRT
<213> Artificial Sequence

<220>
<221> source
<223> /note="Description of Artificial Sequence: Synthetic
      peptide"

<400> 1525
Ser Ser Ser Val Ser Tyr
1               5


<210> 1526
<211> 7
<212> PRT
<213> Artificial Sequence

<220>
<221> source
<223> /note="Description of Artificial Sequence: Synthetic
      peptide"

<400> 1526
Gly Phe Thr Phe Ser Thr Tyr
1               5


<210> 1527
<211> 10
<212> PRT
<213> Artificial Sequence

<220>
<221> source
<223> /note="Description of Artificial Sequence: Synthetic
      peptide"

<400> 1527
Ser Thr Gly Ala Val Thr Thr Ser Asn Tyr
1               5                   10


<210> 1528

<211> 7
<212> PRT
<213> Artificial Sequence

<220>
<221> source
<223> /note="Description of Artificial Sequence: Synthetic
      peptide"

<400> 1528
Gly Phe Thr Phe Asn Thr Tyr
1               5


<210> 1529
<211> 10
<212> PRT
<213> Artificial Sequence

<220>
<221> source
<223> /note="Description of Artificial Sequence: Synthetic
      peptide"

<400> 1529
Ser Thr Gly Ala Val Thr Thr Ser Asn Tyr
1               5                   10


<210> 1530
<211> 7
<212> PRT
<213> Artificial Sequence

<220>
<221> source
<223> /note="Description of Artificial Sequence: Synthetic
      peptide"

<400> 1530
Gly Phe Thr Phe Asn Thr Tyr
1               5


<210> 1531
<211> 10
<212> PRT
<213> Artificial Sequence

<220>
<221> source
<223> /note="Description of Artificial Sequence: Synthetic
      peptide"

<400> 1531
Ser Thr Gly Ala Val Thr Thr Ser Asn Tyr
1               5                   10


<210> 1532
<211> 7
<212> PRT
<213> Artificial Sequence

<220>
<221> source
<223> /note="Description of Artificial Sequence: Synthetic
      peptide"

<400> 1532
Gly Phe Thr Phe Ser Thr Tyr
1               5


<210> 1533
<211> 10
<212> PRT
<213> Artificial Sequence

<220>
<221> source
<223> /note="Description of Artificial Sequence: Synthetic
      peptide"

<400> 1533
Ser Thr Gly Ala Val Thr Thr Ser Asn Tyr
1               5                   10


<210> 1534
<211> 7
<212> PRT
<213> Artificial Sequence

<220>
<221> source
<223> /note="Description of Artificial Sequence: Synthetic
      peptide"

<400> 1534
Gly Tyr Thr Phe Thr Arg Tyr
1               5


<210> 1535
<211> 6
<212> PRT
<213> Artificial Sequence

<220>
<221> source
<223> /note="Description of Artificial Sequence: Synthetic
      peptide"

<400> 1535
Ser Ser Ser Val Ser Tyr
1               5


<210> 1536
<211> 7
<212> PRT
<213> Artificial Sequence

<220>
<221> source

<223> /note="Description of Artificial Sequence: Synthetic
      peptide"

<400> 1536
Gly Tyr Thr Phe Thr Arg Tyr
1               5


<210> 1537
<211> 6
<212> PRT
<213> Artificial Sequence

<220>
<221> source
<223> /note="Description of Artificial Sequence: Synthetic
      peptide"

<400> 1537
Ser Ser Ser Val Ser Tyr
1               5


<210> 1538
<211> 6
<212> PRT
<213> Artificial Sequence

<220>
<221> source
<223> /note="Description of Artificial Sequence: Synthetic
      peptide"

<400> 1538
Asn Pro Ser Arg Gly Tyr
1               5


<210> 1539
<211> 3
<212> PRT
<213> Artificial Sequence

<220>
<221> source
<223> /note="Description of Artificial Sequence: Synthetic
      peptide"

<400> 1539
Asp Thr Ser
1


<210> 1540
<211> 8
<212> PRT
<213> Artificial Sequence

<220>
<221> source
<223> /note="Description of Artificial Sequence: Synthetic
      peptide"

<400> 1540
Arg Ser Lys Tyr Asn Asn Tyr Ala
1               5


<210> 1541
<211> 3
<212> PRT
<213> Artificial Sequence

<220>
<221> source
<223> /note="Description of Artificial Sequence: Synthetic
      peptide"

<400> 1541
Gly Thr Asn
1


<210> 1542
<211> 6
<212> PRT
<213> Artificial Sequence

<220>
<221> source
<223> /note="Description of Artificial Sequence: Synthetic
      peptide"

<400> 1542
Asn Pro Ser Ser Gly Tyr
1               5


<210> 1543
<211> 3
<212> PRT
<213> Artificial Sequence

<220>
<221> source
<223> /note="Description of Artificial Sequence: Synthetic
      peptide"

<400> 1543
Ala Thr Ser
1


<210> 1544
<211> 6
<212> PRT
<213> Artificial Sequence

<220>
<221> source
<223> /note="Description of Artificial Sequence: Synthetic
      peptide"

<400> 1544
Asn Pro Ser Arg Gly Tyr
1               5

<210> 1545
<211> 3
<212> PRT
<213> Artificial Sequence

<220>
<221> source
<223> /note="Description of Artificial Sequence: Synthetic
      peptide"

<400> 1545
Asp Thr Ser
1


<210> 1546
<211> 6
<212> PRT
<213> Artificial Sequence

<220>
<221> source
<223> /note="Description of Artificial Sequence: Synthetic
      peptide"

<400> 1546
Asn Pro Ser Arg Gly Tyr
1               5


<210> 1547
<211> 3
<212> PRT
<213> Artificial Sequence

<220>
<221> source
<223> /note="Description of Artificial Sequence: Synthetic
      peptide"

<400> 1547
Asp Thr Ser
1


<210> 1548
<211> 6
<212> PRT
<213> Artificial Sequence

<220>
<221> source
<223> /note="Description of Artificial Sequence: Synthetic
      peptide"

<400> 1548
Trp Tyr Asp Gly Ser Lys
1               5


<210> 1549

<211> 3
<212> PRT
<213> Artificial Sequence

<220>
<221> source
<223> /note="Description of Artificial Sequence: Synthetic
      peptide"

<400> 1549
Asp Ala Ser
1


<210> 1550
<211> 9
<212> PRT
<213> Artificial Sequence

<220>
<221> source
<223> /note="Description of Artificial Sequence: Synthetic
      peptide"

<400> 1550
Arg Ser Lys Tyr Asn Asn Tyr Ala Thr
1               5


<210> 1551
<211> 3
<212> PRT
<213> Artificial Sequence

<220>
<221> source
<223> /note="Description of Artificial Sequence: Synthetic
      peptide"

<400> 1551
Gly Thr Asn
1


<210> 1552
<211> 6
<212> PRT
<213> Artificial Sequence

<220>
<221> source
<223> /note="Description of Artificial Sequence: Synthetic
      peptide"

<400> 1552
Asn Pro Ser Arg Gly Tyr
1               5


<210> 1553
<211> 3
<212> PRT
<213> Artificial Sequence

<220>
<221> source
<223> /note="Description of Artificial Sequence: Synthetic
      peptide"

<400> 1553
Asp Thr Ser
1


<210> 1554
<211> 9
<212> PRT
<213> Artificial Sequence

<220>
<221> source
<223> /note="Description of Artificial Sequence: Synthetic
      peptide"

<400> 1554
Arg Ser Lys Tyr Asn Asn Tyr Ala Thr
1               5


<210> 1555
<211> 3
<212> PRT
<213> Artificial Sequence

<220>
<221> source
<223> /note="Description of Artificial Sequence: Synthetic
      peptide"

<400> 1555
Gly Thr Asn
1


<210> 1556
<211> 8
<212> PRT
<213> Artificial Sequence

<220>
<221> source
<223> /note="Description of Artificial Sequence: Synthetic
      peptide"

<400> 1556
Arg Ser Lys Tyr Asn Asn Tyr Ala
1               5


<210> 1557
<211> 3
<212> PRT
<213> Artificial Sequence

<220>
<221> source

<223> /note="Description of Artificial Sequence: Synthetic
      peptide"

<400> 1557
Gly Thr Asn
1


<210> 1558
<211> 8
<212> PRT
<213> Artificial Sequence

<220>
<221> source
<223> /note="Description of Artificial Sequence: Synthetic
      peptide"

<400> 1558
Arg Ser Lys Tyr Asn Asn Tyr Ala
1               5


<210> 1559
<211> 3
<212> PRT
<213> Artificial Sequence

<220>
<221> source
<223> /note="Description of Artificial Sequence: Synthetic
      peptide"

<400> 1559
Gly Thr Lys
1


<210> 1560
<211> 8
<212> PRT
<213> Artificial Sequence

<220>
<221> source
<223> /note="Description of Artificial Sequence: Synthetic
      peptide"

<400> 1560
Arg Ser Lys Tyr Asn Asn Tyr Ala
1               5


<210> 1561
<211> 3
<212> PRT
<213> Artificial Sequence

<220>
<221> source
<223> /note="Description of Artificial Sequence: Synthetic
      peptide"

<400> 1561
Gly Thr Lys
1


<210> 1562
<211> 6
<212> PRT
<213> Artificial Sequence

<220>
<221> source
<223> /note="Description of Artificial Sequence: Synthetic
      peptide"

<400> 1562
Asn Pro Ser Arg Gly Tyr
1               5


<210> 1563
<211> 3
<212> PRT
<213> Artificial Sequence

<220>
<221> source
<223> /note="Description of Artificial Sequence: Synthetic
      peptide"

<400> 1563
Asp Thr Ser
1


<210> 1564
<211> 8
<212> PRT
<213> Artificial Sequence

<220>
<221> source
<223> /note="Description of Artificial Sequence: Synthetic
      peptide"

<400> 1564
Arg Ser Lys Tyr Asn Asn Tyr Ala
1               5


<210> 1565
<211> 3
<212> PRT
<213> Artificial Sequence

<220>
<221> source
<223> /note="Description of Artificial Sequence: Synthetic
      peptide"

<400> 1565
Gly Thr Asn
1

<210> 1566
<211> 8
<212> PRT
<213> Artificial Sequence

<220>
<221> source
<223> /note="Description of Artificial Sequence: Synthetic
      peptide"

<400> 1566
Arg Ser Lys Tyr Asn Asn Tyr Ala
1               5


<210> 1567
<211> 3
<212> PRT
<213> Artificial Sequence

<220>
<221> source
<223> /note="Description of Artificial Sequence: Synthetic
      peptide"

<400> 1567
Gly Thr Asn
1


<210> 1568
<211> 8
<212> PRT
<213> Artificial Sequence

<220>
<221> source
<223> /note="Description of Artificial Sequence: Synthetic
      peptide"

<400> 1568
Arg Ser Lys Tyr Asn Asn Tyr Ala
1               5


<210> 1569
<211> 3
<212> PRT
<213> Artificial Sequence

<220>
<221> source
<223> /note="Description of Artificial Sequence: Synthetic
      peptide"

<400> 1569
Gly Thr Asn
1


<210> 1570

<211> 8
<212> PRT
<213> Artificial Sequence

<220>
<221> source
<223> /note="Description of Artificial Sequence: Synthetic
      peptide"

<400> 1570
Arg Ser Lys Tyr Asn Asn Tyr Ala
1               5


<210> 1571
<211> 3
<212> PRT
<213> Artificial Sequence

<220>
<221> source
<223> /note="Description of Artificial Sequence: Synthetic
      peptide"

<400> 1571
Gly Thr Asn
1


<210> 1572
<211> 6
<212> PRT
<213> Artificial Sequence

<220>
<221> source
<223> /note="Description of Artificial Sequence: Synthetic
      peptide"

<400> 1572
Asn Pro Ser Arg Gly Tyr
1               5


<210> 1573
<211> 3
<212> PRT
<213> Artificial Sequence

<220>
<221> source
<223> /note="Description of Artificial Sequence: Synthetic
      peptide"

<400> 1573
Asp Thr Ser
1


<210> 1574
<211> 6
<212> PRT
<213> Artificial Sequence

<220>
<221> source
<223> /note="Description of Artificial Sequence: Synthetic
      peptide"

<400> 1574
Asn Pro Ser Arg Gly Tyr
1               5


<210> 1575
<211> 3
<212> PRT
<213> Artificial Sequence

<220>
<221> source
<223> /note="Description of Artificial Sequence: Synthetic
      peptide"

<400> 1575
Asp Thr Ser
1


<210> 1576
<211> 10
<212> PRT
<213> Artificial Sequence

<220>
<221> source
<223> /note="Description of Artificial Sequence: Synthetic
      peptide"

<400> 1576
Tyr Tyr Asp Asp His Tyr Cys Leu Asp Tyr
1               5                   10


<210> 1577
<211> 6
<212> PRT
<213> Artificial Sequence

<220>
<221> source
<223> /note="Description of Artificial Sequence: Synthetic
      peptide"

<400> 1577
Trp Ser Ser Asn Pro Phe
1               5


<210> 1578
<211> 14
<212> PRT
<213> Artificial Sequence

<220>
<221> source

<223> /note="Description of Artificial Sequence: Synthetic
      peptide"

<400> 1578
His Gly Asn Phe Gly Asn Ser Tyr Val Ser Trp Phe Ala Tyr
1               5                   10


<210> 1579
<211> 6
<212> PRT
<213> Artificial Sequence

<220>
<221> source
<223> /note="Description of Artificial Sequence: Synthetic
      peptide"

<400> 1579
Trp Tyr Ser Asn Leu Trp
1               5


<210> 1580
<211> 10
<212> PRT
<213> Artificial Sequence

<220>
<221> source
<223> /note="Description of Artificial Sequence: Synthetic
      peptide"

<400> 1580
Trp Gln Asp Tyr Asp Val Tyr Phe Asp Tyr
1               5                   10


<210> 1581
<211> 6
<212> PRT
<213> Artificial Sequence

<220>
<221> source
<223> /note="Description of Artificial Sequence: Synthetic
      peptide"

<400> 1581
Trp Ser Ser Asn Pro Pro
1               5


<210> 1582
<211> 10
<212> PRT
<213> Artificial Sequence

<220>
<221> source
<223> /note="Description of Artificial Sequence: Synthetic
      peptide"

<400> 1582
Tyr Tyr Asp Asp His Tyr Cys Leu Asp Tyr
1               5                   10


<210> 1583
<211> 6
<212> PRT
<213> Artificial Sequence

<220>
<221> source
<223> /note="Description of Artificial Sequence: Synthetic
      peptide"

<400> 1583
Trp Ser Ser Asn Pro Leu
1               5


<210> 1584
<211> 10
<212> PRT
<213> Artificial Sequence

<220>
<221> source
<223> /note="Description of Artificial Sequence: Synthetic
      peptide"

<400> 1584
Tyr Tyr Asp Asp His Tyr Cys Leu Asp Tyr
1               5                   10


<210> 1585
<211> 6
<212> PRT
<213> Artificial Sequence

<220>
<221> source
<223> /note="Description of Artificial Sequence: Synthetic
      peptide"

<400> 1585
Trp Ser Ser Asn Pro Phe
1               5


<210> 1586
<211> 9
<212> PRT
<213> Artificial Sequence

<220>
<221> source
<223> /note="Description of Artificial Sequence: Synthetic
      peptide"

<400> 1586
Gln Met Gly Tyr Trp His Phe Asp Leu
1               5

<210> 1587
<211> 7
<212> PRT
<213> Artificial Sequence

<220>
<221> source
<223> /note="Description of Artificial Sequence: Synthetic
      peptide"

<400> 1587
Arg Ser Asn Trp Pro Pro Leu
1               5


<210> 1588
<211> 13
<212> PRT
<213> Artificial Sequence

<220>
<221> source
<223> /note="Description of Artificial Sequence: Synthetic
      peptide"

<400> 1588
His Gly Asn Phe Gly Asn Ser Tyr Val Ser Trp Phe Ala
1               5                   10


<210> 1589
<211> 6
<212> PRT
<213> Artificial Sequence

<220>
<221> source
<223> /note="Description of Artificial Sequence: Synthetic
      peptide"

<400> 1589
Trp Tyr Ser Asn Leu Trp
1               5


<210> 1590
<211> 10
<212> PRT
<213> Artificial Sequence

<220>
<221> source
<223> /note="Description of Artificial Sequence: Synthetic
      peptide"

<400> 1590
Tyr Tyr Asp Asp His Tyr Cys Leu Asp Tyr
1               5                   10


<210> 1591

<211> 6
<212> PRT
<213> Artificial Sequence

<220>
<221> source
<223> /note="Description of Artificial Sequence: Synthetic
      peptide"

<400> 1591
Trp Ser Ser Asn Pro Leu
1               5


<210> 1592
<211> 13
<212> PRT
<213> Artificial Sequence

<220>
<221> source
<223> /note="Description of Artificial Sequence: Synthetic
      peptide"

<400> 1592
His Gly Asn Phe Gly Asn Ser Tyr Val Ser Trp Phe Ala
1               5                   10


<210> 1593
<211> 6
<212> PRT
<213> Artificial Sequence

<220>
<221> source
<223> /note="Description of Artificial Sequence: Synthetic
      peptide"

<400> 1593
Trp Tyr Ser Asn Leu Trp
1               5


<210> 1594
<211> 14
<212> PRT
<213> Artificial Sequence

<220>
<221> source
<223> /note="Description of Artificial Sequence: Synthetic
      peptide"

<400> 1594
His Gly Asn Phe Gly Asn Ser Tyr Val Ser Trp Phe Ala Tyr
1               5                   10


<210> 1595
<211> 6
<212> PRT
<213> Artificial Sequence

<220>
<221> source
<223> /note="Description of Artificial Sequence: Synthetic
      peptide"

<400> 1595
Trp Tyr Ser Asn Leu Trp
1               5


<210> 1596
<211> 14
<212> PRT
<213> Artificial Sequence

<220>
<221> source
<223> /note="Description of Artificial Sequence: Synthetic
      peptide"

<400> 1596
His Gly Asn Phe Gly Asn Ser Tyr Val Ser Trp Trp Ala Tyr
1               5                   10


<210> 1597
<211> 6
<212> PRT
<213> Artificial Sequence

<220>
<221> source
<223> /note="Description of Artificial Sequence: Synthetic
      peptide"

<400> 1597
Trp Tyr Ser Asn Arg Trp
1               5


<210> 1598
<211> 14
<212> PRT
<213> Artificial Sequence

<220>
<221> source
<223> /note="Description of Artificial Sequence: Synthetic
      peptide"

<400> 1598
His Gly Asn Phe Gly Asn Ser Tyr Ile Ser Tyr Trp Ala Tyr
1               5                   10


<210> 1599
<211> 6
<212> PRT
<213> Artificial Sequence

<220>
<221> source

<223> /note="Description of Artificial Sequence: Synthetic
      peptide"

<400> 1599
Trp Tyr Ser Asn Arg Trp
1               5


<210> 1600
<211> 10
<212> PRT
<213> Artificial Sequence

<220>
<221> source
<223> /note="Description of Artificial Sequence: Synthetic
      peptide"

<400> 1600
Tyr Tyr Asp Asp His Tyr Cys Leu Asp Tyr
1               5                   10


<210> 1601
<211> 6
<212> PRT
<213> Artificial Sequence

<220>
<221> source
<223> /note="Description of Artificial Sequence: Synthetic
      peptide"

<400> 1601
Trp Ser Ser Asn Pro Phe
1               5


<210> 1602
<211> 14
<212> PRT
<213> Artificial Sequence

<220>
<221> source
<223> /note="Description of Artificial Sequence: Synthetic
      peptide"

<400> 1602
His Gly Asn Phe Gly Asn Ser Tyr Val Ser Trp Phe Ala Tyr
1               5                   10


<210> 1603
<211> 6
<212> PRT
<213> Artificial Sequence

<220>
<221> source
<223> /note="Description of Artificial Sequence: Synthetic
      peptide"

<400> 1603
Trp Tyr Ser Asn Leu Trp
1               5


<210> 1604
<211> 14
<212> PRT
<213> Artificial Sequence

<220>
<221> source
<223> /note="Description of Artificial Sequence: Synthetic
      peptide"

<400> 1604
His Gly Asn Phe Gly Asn Ser Tyr Val Ser Trp Phe Ala Tyr
1               5                   10


<210> 1605
<211> 6
<212> PRT
<213> Artificial Sequence

<220>
<221> source
<223> /note="Description of Artificial Sequence: Synthetic
      peptide"

<400> 1605
Trp Tyr Ser Asn Leu Trp
1               5


<210> 1606
<211> 14
<212> PRT
<213> Artificial Sequence

<220>
<221> source
<223> /note="Description of Artificial Sequence: Synthetic
      peptide"

<400> 1606
His Gly Asn Phe Gly Asn Ser Tyr Val Ser Trp Phe Ala Tyr
1               5                   10


<210> 1607
<211> 6
<212> PRT
<213> Artificial Sequence

<220>
<221> source
<223> /note="Description of Artificial Sequence: Synthetic
      peptide"

<400> 1607
Trp Tyr Ser Asn Leu Trp
1               5

<210> 1608
<211> 14
<212> PRT
<213> Artificial Sequence

<220>
<221> source
<223> /note="Description of Artificial Sequence: Synthetic
      peptide"

<400> 1608
His Gly Asn Phe Gly Asp Ser Tyr Val Ser Trp Phe Ala Tyr
1               5                   10


<210> 1609
<211> 6
<212> PRT
<213> Artificial Sequence

<220>
<221> source
<223> /note="Description of Artificial Sequence: Synthetic
      peptide"

<400> 1609
Trp Tyr Ser Asn His Trp
1               5


<210> 1610
<211> 10
<212> PRT
<213> Artificial Sequence

<220>
<221> source
<223> /note="Description of Artificial Sequence: Synthetic
      peptide"

<400> 1610
Tyr Tyr Asp Asp His Tyr Cys Leu Asp Tyr
1               5                   10


<210> 1611
<211> 6
<212> PRT
<213> Artificial Sequence

<220>
<221> source
<223> /note="Description of Artificial Sequence: Synthetic
      peptide"

<400> 1611
Trp Ser Ser Asn Pro Phe
1               5


<210> 1612

<211> 10
<212> PRT
<213> Artificial Sequence

<220>
<221> source
<223> /note="Description of Artificial Sequence: Synthetic
      peptide"

<400> 1612
Tyr Tyr Asp Asp His Tyr Ser Leu Asp Tyr
1               5                   10


<210> 1613
<211> 6
<212> PRT
<213> Artificial Sequence

<220>
<221> source
<223> /note="Description of Artificial Sequence: Synthetic
      peptide"

<400> 1613
Trp Ser Ser Asn Pro Phe
1               5


<210> 1614

<400> 1614
000


<210> 1615

<400> 1615
000


<210> 1616

<400> 1616
000


<210> 1617

<400> 1617
000


<210> 1618

<400> 1618
000


<210> 1619

<400> 1619
000

<210> 1620

<400> 1620
000

<210> 1621

<400> 1621
000

<210> 1622

<400> 1622
000

<210> 1623

<400> 1623
000

<210> 1624

<400> 1624
000

<210> 1625

<400> 1625
000

<210> 1626

<400> 1626
000

<210> 1627

<400> 1627
000

<210> 1628

<400> 1628
000

<210> 1629

<400> 1629
000

<210> 1630

<400> 1630
000

<210> 1631

<400> 1631
000

<210> 1632

<400> 1632
000

<210> 1633

<400> 1633
000

<210> 1634

<400> 1634
000

<210> 1635

<400> 1635
000

<210> 1636

<400> 1636
000

<210> 1637

<400> 1637
000

<210> 1638

<400> 1638
000

<210> 1639

<400> 1639
000

<210> 1640

<400> 1640
000

<210> 1641

<400> 1641
000

<210> 1642

<400> 1642
000

<210> 1643

<400> 1643
000

<210> 1644

<400> 1644
000

<210> 1645

<400> 1645
000

<210> 1646

<400> 1646
000

<210> 1647

<400> 1647
000

<210> 1648

<400> 1648
000

<210> 1649

<400> 1649
000

<210> 1650

<400> 1650
000

<210> 1651

<400> 1651
000

<210> 1652

<400> 1652
000

<210> 1653

<400> 1653
000

<210> 1654

<400> 1654
000

<210> 1655

<400> 1655
000

<210> 1656

<400> 1656
000

<210> 1657

<400> 1657
000

<210> 1658

<400> 1658
000

<210> 1659

<400> 1659
000

<210> 1660

<400> 1660
000

<210> 1661

<400> 1661
000

<210> 1662

<400> 1662
000

<210> 1663

<400> 1663
000

<210> 1664

<400> 1664
000

<210> 1665

<400> 1665
000

<210> 1666

<400> 1666
000

<210> 1667

<400> 1667
000

<210> 1668

<400> 1668
000

<210> 1669

<400> 1669
000

<210> 1670

<400> 1670
000

<210> 1671

<400> 1671
000

<210> 1672

<400> 1672
000

<210> 1673

<400> 1673
000

<210> 1674

<400> 1674
000

<210> 1675

<400> 1675
000

<210> 1676

<400> 1676
000

<210> 1677

<400> 1677
000

<210> 1678

<400> 1678
000

<210> 1679

<400> 1679
000

<210> 1680

<400> 1680
000

<210> 1681

<400> 1681
000

<210> 1682

<400> 1682
000

<210> 1683

<400> 1683
000

<210> 1684

<400> 1684
000

<210> 1685

<400> 1685
000

<210> 1686

<400> 1686
000

<210> 1687

<400> 1687
000

<210> 1688

<400> 1688
000

<210> 1689

<400> 1689
000

<210> 1690

<400> 1690
000

<210> 1691

<400> 1691
000

<210> 1692

<400> 1692
000

<210> 1693

<400> 1693
000


<210> 1694

<400> 1694
000


<210> 1695

<400> 1695
000


<210> 1696

<400> 1696
000


<210> 1697

<400> 1697
000


<210> 1698

<400> 1698
000


<210> 1699

<400> 1699
000


<210> 1700
<211> 5
<212> PRT
<213> Artificial Sequence

<220>
<221> source
<223> /note="Description of Artificial Sequence: Synthetic
      peptide"

<400> 1700
Arg Tyr Thr Met His
1               5


<210> 1701
<211> 10
<212> PRT
<213> Artificial Sequence

<220>
<221> source

<223> /note="Description of Artificial Sequence: Synthetic
      peptide"

<400> 1701
Ser Ala Ser Ser Ser Val Ser Tyr Met Asn
1               5                   10


<210> 1702
<211> 5
<212> PRT
<213> Artificial Sequence

<220>
<221> source
<223> /note="Description of Artificial Sequence: Synthetic
      peptide"

<400> 1702
Thr Tyr Ala Met Asn
1               5


<210> 1703
<211> 14
<212> PRT
<213> Artificial Sequence

<220>
<221> source
<223> /note="Description of Artificial Sequence: Synthetic
      peptide"

<400> 1703
Arg Ser Ser Thr Gly Ala Val Thr Thr Ser Asn Tyr Ala Asn
1               5                   10


<210> 1704
<211> 5
<212> PRT
<213> Artificial Sequence

<220>
<221> source
<223> /note="Description of Artificial Sequence: Synthetic
      peptide"

<400> 1704
Ser Tyr Thr Met His
1               5


<210> 1705
<211> 10
<212> PRT
<213> Artificial Sequence

<220>
<221> source
<223> /note="Description of Artificial Sequence: Synthetic
      peptide"

<400> 1705
Arg Ala Ser Ser Ser Val Ser Tyr Met His
1               5                   10


<210> 1706
<211> 5
<212> PRT
<213> Artificial Sequence

<220>
<221> source
<223> /note="Description of Artificial Sequence: Synthetic
      peptide"

<400> 1706
Arg Tyr Thr Met His
1               5


<210> 1707
<211> 10
<212> PRT
<213> Artificial Sequence

<220>
<221> source
<223> /note="Description of Artificial Sequence: Synthetic
      peptide"

<400> 1707
Arg Ala Ser Ser Ser Val Ser Tyr Met Asn
1               5                   10


<210> 1708
<211> 5
<212> PRT
<213> Artificial Sequence

<220>
<221> source
<223> /note="Description of Artificial Sequence: Synthetic
      peptide"

<400> 1708
Arg Tyr Thr Met His
1               5


<210> 1709
<211> 10
<212> PRT
<213> Artificial Sequence

<220>
<221> source
<223> /note="Description of Artificial Sequence: Synthetic
      peptide"

<400> 1709
Ser Ala Ser Ser Ser Val Ser Tyr Met Asn
1               5                   10

<210> 1710
<211> 5
<212> PRT
<213> Artificial Sequence

<220>
<221> source
<223> /note="Description of Artificial Sequence: Synthetic
      peptide"

<400> 1710
Gly Tyr Gly Met His
1               5


<210> 1711
<211> 11
<212> PRT
<213> Artificial Sequence

<220>
<221> source
<223> /note="Description of Artificial Sequence: Synthetic
      peptide"

<400> 1711
Arg Ala Ser Gln Ser Val Ser Ser Tyr Leu Ala
1               5                   10


<210> 1712
<211> 5
<212> PRT
<213> Artificial Sequence

<220>
<221> source
<223> /note="Description of Artificial Sequence: Synthetic
      peptide"

<400> 1712
Thr Tyr Ala Met Asn
1               5


<210> 1713
<211> 14
<212> PRT
<213> Artificial Sequence

<220>
<221> source
<223> /note="Description of Artificial Sequence: Synthetic
      peptide"

<400> 1713
Arg Ser Ser Thr Gly Ala Val Thr Thr Ser Asn Tyr Ala Asn
1               5                   10


<210> 1714

<211> 5
<212> PRT
<213> Artificial Sequence

<220>
<221> source
<223> /note="Description of Artificial Sequence: Synthetic
      peptide"

<400> 1714
Arg Tyr Thr Met His
1               5


<210> 1715
<211> 10
<212> PRT
<213> Artificial Sequence

<220>
<221> source
<223> /note="Description of Artificial Sequence: Synthetic
      peptide"

<400> 1715
Arg Ala Ser Ser Ser Val Ser Tyr Met Asn
1               5                   10


<210> 1716
<211> 5
<212> PRT
<213> Artificial Sequence

<220>
<221> source
<223> /note="Description of Artificial Sequence: Synthetic
      peptide"

<400> 1716
Thr Tyr Ala Met Asn
1               5


<210> 1717
<211> 14
<212> PRT
<213> Artificial Sequence

<220>
<221> source
<223> /note="Description of Artificial Sequence: Synthetic
      peptide"

<400> 1717
Arg Ser Ser Thr Gly Ala Val Thr Thr Ser Asn Tyr Ala Asn
1               5                   10


<210> 1718
<211> 5
<212> PRT
<213> Artificial Sequence

<220>
<221> source
<223> /note="Description of Artificial Sequence: Synthetic
      peptide"

<400> 1718
Thr Tyr Ala Met Asn
1               5


<210> 1719
<211> 14
<212> PRT
<213> Artificial Sequence

<220>
<221> source
<223> /note="Description of Artificial Sequence: Synthetic
      peptide"

<400> 1719
Arg Ser Ser Thr Gly Ala Val Thr Thr Ser Asn Tyr Ala Asn
1               5                   10


<210> 1720
<211> 5
<212> PRT
<213> Artificial Sequence

<220>
<221> source
<223> /note="Description of Artificial Sequence: Synthetic
      peptide"

<400> 1720
Ser Tyr Ala Met Asn
1               5


<210> 1721
<211> 14
<212> PRT
<213> Artificial Sequence

<220>
<221> source
<223> /note="Description of Artificial Sequence: Synthetic
      peptide"

<400> 1721
Gly Ser Ser Thr Gly Ala Val Thr Ser Gly Asn Tyr Pro Asn
1               5                   10


<210> 1722
<211> 5
<212> PRT
<213> Artificial Sequence

<220>
<221> source

<223> /note="Description of Artificial Sequence: Synthetic
      peptide"

<400> 1722
Lys Tyr Ala Met Asn
1               5


<210> 1723
<211> 14
<212> PRT
<213> Artificial Sequence

<220>
<221> source
<223> /note="Description of Artificial Sequence: Synthetic
      peptide"

<400> 1723
Gly Ser Ser Thr Gly Ala Val Thr Ser Gly Asn Tyr Pro Asn
1               5                   10


<210> 1724
<211> 5
<212> PRT
<213> Artificial Sequence

<220>
<221> source
<223> /note="Description of Artificial Sequence: Synthetic
      peptide"

<400> 1724
Arg Tyr Thr Met His
1               5


<210> 1725
<211> 10
<212> PRT
<213> Artificial Sequence

<220>
<221> source
<223> /note="Description of Artificial Sequence: Synthetic
      peptide"

<400> 1725
Ser Ala Ser Ser Ser Val Ser Tyr Met Asn
1               5                   10


<210> 1726
<211> 5
<212> PRT
<213> Artificial Sequence

<220>
<221> source
<223> /note="Description of Artificial Sequence: Synthetic
      peptide"

<400> 1726
Thr Tyr Ala Met Asn
1               5


<210> 1727
<211> 14
<212> PRT
<213> Artificial Sequence

<220>
<221> source
<223> /note="Description of Artificial Sequence: Synthetic
      peptide"

<400> 1727
Arg Ser Ser Thr Gly Ala Val Thr Thr Ser Asn Tyr Ala Asn
1               5                   10


<210> 1728
<211> 5
<212> PRT
<213> Artificial Sequence

<220>
<221> source
<223> /note="Description of Artificial Sequence: Synthetic
      peptide"

<400> 1728
Thr Tyr Ala Met Asn
1               5


<210> 1729
<211> 14
<212> PRT
<213> Artificial Sequence

<220>
<221> source
<223> /note="Description of Artificial Sequence: Synthetic
      peptide"

<400> 1729
Arg Ser Ser Thr Gly Ala Val Thr Thr Ser Asn Tyr Ala Asn
1               5                   10


<210> 1730
<211> 5
<212> PRT
<213> Artificial Sequence

<220>
<221> source
<223> /note="Description of Artificial Sequence: Synthetic
      peptide"

<400> 1730
Thr Tyr Ala Met Asn
1               5

<210> 1731
<211> 14
<212> PRT
<213> Artificial Sequence

<220>
<221> source
<223> /note="Description of Artificial Sequence: Synthetic
      peptide"

<400> 1731
Gly Ser Ser Thr Gly Ala Val Thr Thr Ser Asn Tyr Ala Asn
1               5                   10


<210> 1732
<211> 5
<212> PRT
<213> Artificial Sequence

<220>
<221> source
<223> /note="Description of Artificial Sequence: Synthetic
      peptide"

<400> 1732
Thr Tyr Ala Met Asn
1               5


<210> 1733
<211> 14
<212> PRT
<213> Artificial Sequence

<220>
<221> source
<223> /note="Description of Artificial Sequence: Synthetic
      peptide"

<400> 1733
Gly Ser Ser Thr Gly Ala Val Thr Thr Ser Asn Tyr Ala Asn
1               5                   10


<210> 1734
<211> 5
<212> PRT
<213> Artificial Sequence

<220>
<221> source
<223> /note="Description of Artificial Sequence: Synthetic
      peptide"

<400> 1734
Arg Tyr Thr Met His
1               5


<210> 1735

<211> 10
<212> PRT
<213> Artificial Sequence

<220>
<221> source
<223> /note="Description of Artificial Sequence: Synthetic
      peptide"

<400> 1735
Ser Ala Ser Ser Ser Val Ser Tyr Met Asn
1               5                   10


<210> 1736
<211> 5
<212> PRT
<213> Artificial Sequence

<220>
<221> source
<223> /note="Description of Artificial Sequence: Synthetic
      peptide"

<400> 1736
Arg Tyr Thr Met His
1               5


<210> 1737
<211> 10
<212> PRT
<213> Artificial Sequence

<220>
<221> source
<223> /note="Description of Artificial Sequence: Synthetic
      peptide"

<400> 1737
Ser Ala Ser Ser Ser Val Ser Tyr Met Asn
1               5                   10


<210> 1738
<211> 17
<212> PRT
<213> Artificial Sequence

<220>
<221> source
<223> /note="Description of Artificial Sequence: Synthetic
      peptide"

<400> 1738
Tyr Ile Asn Pro Ser Arg Gly Tyr Thr Asn Tyr Asn Gln Lys Phe Lys
1               5                   10                  15


Asp

<210> 1739
<211> 7
<212> PRT
<213> Artificial Sequence

<220>
<221> source
<223> /note="Description of Artificial Sequence: Synthetic
      peptide"

<400> 1739
Asp Thr Ser Lys Leu Ala Ser
1               5


<210> 1740
<211> 19
<212> PRT
<213> Artificial Sequence

<220>
<221> source
<223> /note="Description of Artificial Sequence: Synthetic
      peptide"

<400> 1740
Arg Ile Arg Ser Lys Tyr Asn Asn Tyr Ala Thr Tyr Tyr Ala Asp Ser
1               5                   10                  15


Val Lys Asp




<210> 1741
<211> 7
<212> PRT
<213> Artificial Sequence

<220>
<221> source
<223> /note="Description of Artificial Sequence: Synthetic
      peptide"

<400> 1741
Gly Thr Asn Lys Arg Ala Pro
1               5


<210> 1742
<211> 17
<212> PRT
<213> Artificial Sequence

<220>
<221> source
<223> /note="Description of Artificial Sequence: Synthetic
      peptide"

<400> 1742
Tyr Ile Asn Pro Ser Ser Gly Tyr Thr Lys Tyr Asn Gln Lys Phe Lys
1               5                   10                  15

Asp

<210> 1743
<211> 7
<212> PRT
<213> Artificial Sequence

<220>
<221> source
<223> /note="Description of Artificial Sequence: Synthetic
      peptide"

<400> 1743
Ala Thr Ser Asn Leu Ala Ser
1               5

<210> 1744
<211> 17
<212> PRT
<213> Artificial Sequence

<220>
<221> source
<223> /note="Description of Artificial Sequence: Synthetic
      peptide"

<400> 1744
Tyr Ile Asn Pro Ser Arg Gly Tyr Thr Asn Tyr Asn Gln Lys Phe Lys
1               5                   10                  15

Asp

<210> 1745
<211> 7
<212> PRT
<213> Artificial Sequence

<220>
<221> source
<223> /note="Description of Artificial Sequence: Synthetic
      peptide"

<400> 1745
Asp Thr Ser Lys Val Ala Ser
1               5

<210> 1746
<211> 17
<212> PRT
<213> Artificial Sequence

<220>
<221> source
<223> /note="Description of Artificial Sequence: Synthetic
      peptide"

<400> 1746
Tyr Ile Asn Pro Ser Arg Gly Tyr Thr Asn Tyr Asn Gln Lys Val Lys
1               5                   10                  15

Asp

<210> 1747
<211> 7
<212> PRT
<213> Artificial Sequence

<220>
<221> source
<223> /note="Description of Artificial Sequence: Synthetic
      peptide"

<400> 1747
Asp Thr Ser Lys Leu Ala Ser
1               5

<210> 1748
<211> 17
<212> PRT
<213> Artificial Sequence

<220>
<221> source
<223> /note="Description of Artificial Sequence: Synthetic
      peptide"

<400> 1748
Val Ile Trp Tyr Asp Gly Ser Lys Lys Tyr Tyr Val Asp Ser Val Lys
1               5                   10                  15

Gly

<210> 1749
<211> 7
<212> PRT
<213> Artificial Sequence

<220>
<221> source
<223> /note="Description of Artificial Sequence: Synthetic
      peptide"

<400> 1749
Asp Ala Ser Asn Arg Ala Thr
1               5

<210> 1750
<211> 15
<212> PRT
<213> Artificial Sequence

<220>
<221> source
<223> /note="Description of Artificial Sequence: Synthetic
      peptide"

<400> 1750
Arg Ile Arg Ser Lys Tyr Asn Asn Tyr Ala Thr Tyr Tyr Ala Asp
1               5                   10                  15


<210> 1751
<211> 7
<212> PRT
<213> Artificial Sequence

<220>
<221> source
<223> /note="Description of Artificial Sequence: Synthetic
      peptide"

<400> 1751
Gly Thr Asn Lys Arg Ala Pro
1               5


<210> 1752
<211> 17
<212> PRT
<213> Artificial Sequence

<220>
<221> source
<223> /note="Description of Artificial Sequence: Synthetic
      peptide"

<400> 1752
Tyr Ile Asn Pro Ser Arg Gly Tyr Thr Asn Tyr Asn Gln Lys Phe Lys
1               5                   10                  15


Asp




<210> 1753
<211> 7
<212> PRT
<213> Artificial Sequence

<220>
<221> source
<223> /note="Description of Artificial Sequence: Synthetic
      peptide"

<400> 1753
Asp Thr Ser Lys Val Ala Ser
1               5


<210> 1754
<211> 15
<212> PRT

<213> Artificial Sequence

<220>
<221> source
<223> /note="Description of Artificial Sequence: Synthetic
      peptide"

<400> 1754
Arg Ile Arg Ser Lys Tyr Asn Asn Tyr Ala Thr Tyr Tyr Ala Asp
1               5                   10                  15


<210> 1755
<211> 7
<212> PRT
<213> Artificial Sequence

<220>
<221> source
<223> /note="Description of Artificial Sequence: Synthetic
      peptide"

<400> 1755
Gly Thr Asn Lys Arg Ala Pro
1               5


<210> 1756
<211> 19
<212> PRT
<213> Artificial Sequence

<220>
<221> source
<223> /note="Description of Artificial Sequence: Synthetic
      peptide"

<400> 1756
Arg Ile Arg Ser Lys Tyr Asn Asn Tyr Ala Thr Tyr Tyr Ala Asp Ser
1               5                   10                  15


Val Lys Asp



<210> 1757
<211> 7
<212> PRT
<213> Artificial Sequence

<220>
<221> source
<223> /note="Description of Artificial Sequence: Synthetic
      peptide"

<400> 1757
Gly Thr Asn Lys Arg Ala Pro
1               5


<210> 1758
<211> 19

<212> PRT
<213> Artificial Sequence

<220>
<221> source
<223> /note="Description of Artificial Sequence: Synthetic
      peptide"

<400> 1758
Arg Ile Arg Ser Lys Tyr Asn Asn Tyr Ala Thr Tyr Tyr Ala Asp Ser
1               5                   10                  15

Val Lys Gly

<210> 1759
<211> 7
<212> PRT
<213> Artificial Sequence

<220>
<221> source
<223> /note="Description of Artificial Sequence: Synthetic
      peptide"

<400> 1759
Gly Thr Lys Phe Leu Ala Pro
1               5

<210> 1760
<211> 19
<212> PRT
<213> Artificial Sequence

<220>
<221> source
<223> /note="Description of Artificial Sequence: Synthetic
      peptide"

<400> 1760
Arg Ile Arg Ser Lys Tyr Asn Asn Tyr Ala Thr Tyr Tyr Ala Asp Ser
1               5                   10                  15

Val Lys Asp

<210> 1761
<211> 7
<212> PRT
<213> Artificial Sequence

<220>
<221> source
<223> /note="Description of Artificial Sequence: Synthetic
      peptide"

<400> 1761
Gly Thr Lys Phe Leu Ala Pro

1               5


<210> 1762
<211> 17
<212> PRT
<213> Artificial Sequence

<220>
<221> source
<223> /note="Description of Artificial Sequence: Synthetic
      peptide"

<400> 1762
Tyr Ile Asn Pro Ser Arg Gly Tyr Thr Asn Tyr Asn Gln Lys Phe Lys
1               5                   10                  15


Asp




<210> 1763
<211> 7
<212> PRT
<213> Artificial Sequence

<220>
<221> source
<223> /note="Description of Artificial Sequence: Synthetic
      peptide"

<400> 1763
Asp Thr Ser Lys Leu Ala Ser
1               5


<210> 1764
<211> 19
<212> PRT
<213> Artificial Sequence

<220>
<221> source
<223> /note="Description of Artificial Sequence: Synthetic
      peptide"

<400> 1764
Arg Ile Arg Ser Lys Tyr Asn Asn Tyr Ala Thr Tyr Tyr Ala Asp Ser
1               5                   10                  15


Val Lys Asp




<210> 1765
<211> 7
<212> PRT
<213> Artificial Sequence

<220>
<221> source

<223> /note="Description of Artificial Sequence: Synthetic
      peptide"

<400> 1765
Gly Thr Asn Lys Arg Ala Pro
1               5


<210> 1766
<211> 19
<212> PRT
<213> Artificial Sequence

<220>
<221> source
<223> /note="Description of Artificial Sequence: Synthetic
      peptide"

<400> 1766
Arg Ile Arg Ser Lys Tyr Asn Asn Tyr Ala Thr Tyr Tyr Ala Asp Ser
1               5                   10                  15


Val Lys Asp



<210> 1767
<211> 7
<212> PRT
<213> Artificial Sequence

<220>
<221> source
<223> /note="Description of Artificial Sequence: Synthetic
      peptide"

<400> 1767
Gly Thr Asn Lys Arg Ala Pro
1               5


<210> 1768
<211> 19
<212> PRT
<213> Artificial Sequence

<220>
<221> source
<223> /note="Description of Artificial Sequence: Synthetic
      peptide"

<400> 1768
Arg Ile Arg Ser Lys Tyr Asn Asn Tyr Ala Thr Tyr Tyr Ala Asp Ser
1               5                   10                  15


Val Lys Gly



<210> 1769
<211> 7

<212> PRT
<213> Artificial Sequence

<220>
<221> source
<223> /note="Description of Artificial Sequence: Synthetic
      peptide"

<400> 1769
Gly Thr Asn Lys Arg Ala Pro
1               5


<210> 1770
<211> 19
<212> PRT
<213> Artificial Sequence

<220>
<221> source
<223> /note="Description of Artificial Sequence: Synthetic
      peptide"

<400> 1770
Arg Ile Arg Ser Lys Tyr Asn Asn Tyr Ala Thr Tyr Tyr Ala Asp Ser
1               5                   10                  15


Val Lys Gly



<210> 1771
<211> 7
<212> PRT
<213> Artificial Sequence

<220>
<221> source
<223> /note="Description of Artificial Sequence: Synthetic
      peptide"

<400> 1771
Gly Thr Asn Lys Arg Ala Pro
1               5


<210> 1772
<211> 17
<212> PRT
<213> Artificial Sequence

<220>
<221> source
<223> /note="Description of Artificial Sequence: Synthetic
      peptide"

<400> 1772
Tyr Ile Asn Pro Ser Arg Gly Tyr Thr Asn Tyr Asn Gln Lys Val Lys
1               5                   10                  15


Asp

<210> 1773
<211> 7
<212> PRT
<213> Artificial Sequence

<220>
<221> source
<223> /note="Description of Artificial Sequence: Synthetic
      peptide"

<400> 1773
Asp Thr Ser Lys Leu Ala Ser
1               5


<210> 1774
<211> 17
<212> PRT
<213> Artificial Sequence

<220>
<221> source
<223> /note="Description of Artificial Sequence: Synthetic
      peptide"

<400> 1774
Tyr Ile Asn Pro Ser Arg Gly Tyr Thr Asn Tyr Asn Gln Lys Val Lys
1               5                   10                  15


Asp



<210> 1775
<211> 7
<212> PRT
<213> Artificial Sequence

<220>
<221> source
<223> /note="Description of Artificial Sequence: Synthetic
      peptide"

<400> 1775
Asp Thr Ser Lys Leu Ala Ser
1               5


<210> 1776
<211> 10
<212> PRT
<213> Artificial Sequence

<220>
<221> source
<223> /note="Description of Artificial Sequence: Synthetic
      peptide"

<400> 1776

Tyr Tyr Asp Asp His Tyr Cys Leu Asp Tyr
1               5                   10

<210> 1777
<211> 9
<212> PRT
<213> Artificial Sequence

<220>
<221> source
<223> /note="Description of Artificial Sequence: Synthetic
      peptide"

<400> 1777
Gln Gln Trp Ser Ser Asn Pro Phe Thr
1               5

<210> 1778
<211> 14
<212> PRT
<213> Artificial Sequence

<220>
<221> source
<223> /note="Description of Artificial Sequence: Synthetic
      peptide"

<400> 1778
His Gly Asn Phe Gly Asn Ser Tyr Val Ser Trp Phe Ala Tyr
1               5                   10

<210> 1779
<211> 9
<212> PRT
<213> Artificial Sequence

<220>
<221> source
<223> /note="Description of Artificial Sequence: Synthetic
      peptide"

<400> 1779
Ala Leu Trp Tyr Ser Asn Leu Trp Val
1               5

<210> 1780
<211> 10
<212> PRT
<213> Artificial Sequence

<220>
<221> source
<223> /note="Description of Artificial Sequence: Synthetic
      peptide"

<400> 1780
Trp Gln Asp Tyr Asp Val Tyr Phe Asp Tyr
1               5                   10

<210> 1781
<211> 9
<212> PRT
<213> Artificial Sequence

<220>
<221> source
<223> /note="Description of Artificial Sequence: Synthetic
      peptide"

<400> 1781
Gln Gln Trp Ser Ser Asn Pro Pro Thr
1               5


<210> 1782
<211> 10
<212> PRT
<213> Artificial Sequence

<220>
<221> source
<223> /note="Description of Artificial Sequence: Synthetic
      peptide"

<400> 1782
Tyr Tyr Asp Asp His Tyr Cys Leu Asp Tyr
1               5                   10


<210> 1783
<211> 9
<212> PRT
<213> Artificial Sequence

<220>
<221> source
<223> /note="Description of Artificial Sequence: Synthetic
      peptide"

<400> 1783
Gln Gln Trp Ser Ser Asn Pro Leu Thr
1               5


<210> 1784
<211> 10
<212> PRT
<213> Artificial Sequence

<220>
<221> source
<223> /note="Description of Artificial Sequence: Synthetic
      peptide"

<400> 1784
Tyr Tyr Asp Asp His Tyr Cys Leu Asp Tyr
1               5                   10


<210> 1785
<211> 9

<212> PRT
<213> Artificial Sequence

<220>
<221> source
<223> /note="Description of Artificial Sequence: Synthetic
      peptide"

<400> 1785
Gln Gln Trp Ser Ser Asn Pro Phe Thr
1               5


<210> 1786
<211> 9
<212> PRT
<213> Artificial Sequence

<220>
<221> source
<223> /note="Description of Artificial Sequence: Synthetic
      peptide"

<400> 1786
Gln Met Gly Tyr Trp His Phe Asp Leu
1               5


<210> 1787
<211> 10
<212> PRT
<213> Artificial Sequence

<220>
<221> source
<223> /note="Description of Artificial Sequence: Synthetic
      peptide"

<400> 1787
Gln Gln Arg Ser Asn Trp Pro Pro Leu Thr
1               5                   10


<210> 1788
<211> 16
<212> PRT
<213> Artificial Sequence

<220>
<221> source
<223> /note="Description of Artificial Sequence: Synthetic
      peptide"

<400> 1788
Val Arg His Gly Asn Phe Gly Asn Ser Tyr Val Ser Trp Phe Ala Tyr
1               5                   10                  15


<210> 1789
<211> 9
<212> PRT
<213> Artificial Sequence

<220>
<221> source
<223> /note="Description of Artificial Sequence: Synthetic
      peptide"

<400> 1789
Ala Leu Trp Tyr Ser Asn Leu Trp Val
1               5


<210> 1790
<211> 10
<212> PRT
<213> Artificial Sequence

<220>
<221> source
<223> /note="Description of Artificial Sequence: Synthetic
      peptide"

<400> 1790
Tyr Tyr Asp Asp His Tyr Cys Leu Asp Tyr
1               5                   10


<210> 1791
<211> 9
<212> PRT
<213> Artificial Sequence

<220>
<221> source
<223> /note="Description of Artificial Sequence: Synthetic
      peptide"

<400> 1791
Gln Gln Trp Ser Ser Asn Pro Leu Thr
1               5


<210> 1792
<211> 16
<212> PRT
<213> Artificial Sequence

<220>
<221> source
<223> /note="Description of Artificial Sequence: Synthetic
      peptide"

<400> 1792
Val Arg His Gly Asn Phe Gly Asn Ser Tyr Val Ser Trp Phe Ala Tyr
1               5                   10                  15


<210> 1793
<211> 9
<212> PRT
<213> Artificial Sequence

<220>
<221> source
<223> /note="Description of Artificial Sequence: Synthetic

peptide"

<400> 1793
Ala Leu Trp Tyr Ser Asn Leu Trp Val
1               5


<210> 1794
<211> 14
<212> PRT
<213> Artificial Sequence

<220>
<221> source
<223> /note="Description of Artificial Sequence: Synthetic
      peptide"

<400> 1794
His Gly Asn Phe Gly Asn Ser Tyr Val Ser Trp Phe Ala Tyr
1               5                   10


<210> 1795
<211> 9
<212> PRT
<213> Artificial Sequence

<220>
<221> source
<223> /note="Description of Artificial Sequence: Synthetic
      peptide"

<400> 1795
Ala Leu Trp Tyr Ser Asn Leu Trp Val
1               5


<210> 1796
<211> 14
<212> PRT
<213> Artificial Sequence

<220>
<221> source
<223> /note="Description of Artificial Sequence: Synthetic
      peptide"

<400> 1796
His Gly Asn Phe Gly Asn Ser Tyr Val Ser Trp Trp Ala Tyr
1               5                   10


<210> 1797
<211> 9
<212> PRT
<213> Artificial Sequence

<220>
<221> source
<223> /note="Description of Artificial Sequence: Synthetic
      peptide"

<400> 1797

Val Leu Trp Tyr Ser Asn Arg Trp Val
1               5

<210> 1798
<211> 14
<212> PRT
<213> Artificial Sequence

<220>
<221> source
<223> /note="Description of Artificial Sequence: Synthetic
      peptide"

<400> 1798
His Gly Asn Phe Gly Asn Ser Tyr Ile Ser Tyr Trp Ala Tyr
1               5                   10

<210> 1799
<211> 9
<212> PRT
<213> Artificial Sequence

<220>
<221> source
<223> /note="Description of Artificial Sequence: Synthetic
      peptide"

<400> 1799
Val Leu Trp Tyr Ser Asn Arg Trp Val
1               5

<210> 1800
<211> 10
<212> PRT
<213> Artificial Sequence

<220>
<221> source
<223> /note="Description of Artificial Sequence: Synthetic
      peptide"

<400> 1800
Tyr Tyr Asp Asp His Tyr Cys Leu Asp Tyr
1               5                   10

<210> 1801
<211> 9
<212> PRT
<213> Artificial Sequence

<220>
<221> source
<223> /note="Description of Artificial Sequence: Synthetic
      peptide"

<400> 1801
Gln Gln Trp Ser Ser Asn Pro Phe Thr
1               5

<210> 1802
<211> 14
<212> PRT
<213> Artificial Sequence

<220>
<221> source
<223> /note="Description of Artificial Sequence: Synthetic
      peptide"

<400> 1802
His Gly Asn Phe Gly Asn Ser Tyr Val Ser Trp Phe Ala Tyr
1               5                   10


<210> 1803
<211> 9
<212> PRT
<213> Artificial Sequence

<220>
<221> source
<223> /note="Description of Artificial Sequence: Synthetic
      peptide"

<400> 1803
Ala Leu Trp Tyr Ser Asn Leu Trp Val
1               5


<210> 1804
<211> 14
<212> PRT
<213> Artificial Sequence

<220>
<221> source
<223> /note="Description of Artificial Sequence: Synthetic
      peptide"

<400> 1804
His Gly Asn Phe Gly Asn Ser Tyr Val Ser Trp Phe Ala Tyr
1               5                   10


<210> 1805
<211> 9
<212> PRT
<213> Artificial Sequence

<220>
<221> source
<223> /note="Description of Artificial Sequence: Synthetic
      peptide"

<400> 1805
Ala Leu Trp Tyr Ser Asn Leu Trp Val
1               5


<210> 1806
<211> 14

<212> PRT
<213> Artificial Sequence

<220>
<221> source
<223> /note="Description of Artificial Sequence: Synthetic
      peptide"

<400> 1806
His Gly Asn Phe Gly Asn Ser Tyr Val Ser Trp Phe Ala Tyr
1               5                   10


<210> 1807
<211> 9
<212> PRT
<213> Artificial Sequence

<220>
<221> source
<223> /note="Description of Artificial Sequence: Synthetic
      peptide"

<400> 1807
Ala Leu Trp Tyr Ser Asn Leu Trp Val
1               5


<210> 1808
<211> 14
<212> PRT
<213> Artificial Sequence

<220>
<221> source
<223> /note="Description of Artificial Sequence: Synthetic
      peptide"

<400> 1808
His Gly Asn Phe Gly Asp Ser Tyr Val Ser Trp Phe Ala Tyr
1               5                   10


<210> 1809
<211> 9
<212> PRT
<213> Artificial Sequence

<220>
<221> source
<223> /note="Description of Artificial Sequence: Synthetic
      peptide"

<400> 1809
Ala Leu Trp Tyr Ser Asn His Trp Val
1               5


<210> 1810
<211> 10
<212> PRT
<213> Artificial Sequence

<220>
<221> source
<223> /note="Description of Artificial Sequence: Synthetic
      peptide"

<400> 1810
Tyr Tyr Asp Asp His Tyr Cys Leu Asp Tyr
1               5                   10


<210> 1811
<211> 9
<212> PRT
<213> Artificial Sequence

<220>
<221> source
<223> /note="Description of Artificial Sequence: Synthetic
      peptide"

<400> 1811
Gln Gln Trp Ser Ser Asn Pro Phe Thr
1               5


<210> 1812
<211> 10
<212> PRT
<213> Artificial Sequence

<220>
<221> source
<223> /note="Description of Artificial Sequence: Synthetic
      peptide"

<400> 1812
Tyr Tyr Asp Asp His Tyr Ser Leu Asp Tyr
1               5                   10


<210> 1813
<211> 9
<212> PRT
<213> Artificial Sequence

<220>
<221> source
<223> /note="Description of Artificial Sequence: Synthetic
      peptide"

<400> 1813
Gln Gln Trp Ser Ser Asn Pro Phe Thr
1               5


<210> 1814

<400> 1814
000


<210> 1815

<400> 1815
000

<210> 1816

<400> 1816
000

<210> 1817

<400> 1817
000

<210> 1818

<400> 1818
000

<210> 1819

<400> 1819
000

<210> 1820

<400> 1820
000

<210> 1821

<400> 1821
000

<210> 1822

<400> 1822
000

<210> 1823

<400> 1823
000

<210> 1824

<400> 1824
000

<210> 1825

<400> 1825
000

<210> 1826

<400> 1826
000

<210> 1827

<400> 1827
000

<210> 1828

<400> 1828
000

<210> 1829

<400> 1829
000

<210> 1830

<400> 1830
000

<210> 1831

<400> 1831
000

<210> 1832

<400> 1832
000

<210> 1833

<400> 1833
000

<210> 1834

<400> 1834
000

<210> 1835

<400> 1835
000

<210> 1836

<400> 1836
000

<210> 1837

<400> 1837
000

<210> 1838

<400> 1838
000

<210> 1839

<400> 1839
000

<210> 1840

<400> 1840
000

<210> 1841

<400> 1841
000

<210> 1842

<400> 1842
000

<210> 1843

<400> 1843
000

<210> 1844

<400> 1844
000

<210> 1845

<400> 1845
000

<210> 1846

<400> 1846
000

<210> 1847

<400> 1847
000

<210> 1848

<400> 1848
000

<210> 1849

<400> 1849
000

<210> 1850

<400> 1850
000

<210> 1851

<400> 1851
000

<210> 1852

<400> 1852
000

<210> 1853

<400> 1853
000

<210> 1854

<400> 1854
000

<210> 1855

<400> 1855
000

<210> 1856

<400> 1856
000

<210> 1857

<400> 1857
000

<210> 1858

<400> 1858
000

<210> 1859

<400> 1859
000

<210> 1860

<400> 1860
000

<210> 1861

<400> 1861
000

<210> 1862

<400> 1862
000

<210> 1863

<400> 1863
000

<210> 1864

<400> 1864
000

<210> 1865

<400> 1865
000

<210> 1866

<400> 1866
000

<210> 1867

<400> 1867
000

<210> 1868

<400> 1868
000

<210> 1869

<400> 1869
000

<210> 1870

<400> 1870
000

<210> 1871

<400> 1871
000

<210> 1872

<400> 1872
000

<210> 1873

<400> 1873
000

<210> 1874

<400> 1874
000

<210> 1875

<400> 1875
000

<210> 1876

<400> 1876
000

<210> 1877

<400> 1877
000

<210> 1878

<400> 1878
000

<210> 1879

<400> 1879
000

<210> 1880

<400> 1880
000

<210> 1881

<400> 1881
000

<210> 1882

<400> 1882
000

<210> 1883

<400> 1883
000

<210> 1884

<400> 1884
000

<210> 1885

<400> 1885
000

<210> 1886

<400> 1886
000

<210> 1887

<400> 1887
000

<210> 1888

<400> 1888
000

<210> 1889

<400> 1889
000

<210> 1890

<400> 1890
000

<210> 1891

<400> 1891
000

<210> 1892

<400> 1892
000

<210> 1893

<400> 1893
000

<210> 1894

<400> 1894
000

<210> 1895

<400> 1895
000

<210> 1896

<400> 1896
000

<210> 1897

<400> 1897
000

<210> 1898

<400> 1898
000

<210> 1899

<400> 1899
000

<210> 1900
<211> 119
<212> PRT
<213> Artificial Sequence

<220>
<221> source
<223> /note="Description of Artificial Sequence: Synthetic
      polypeptide"

<400> 1900

<210> 1901
<211> 106
<212> PRT
<213> Artificial Sequence

<220>
<221> source
<223> /note="Description of Artificial Sequence: Synthetic
      polypeptide"

<400> 1901

<210> 1902
<211> 105
<212> PRT
<213> Artificial Sequence

<220>
<221> source
<223> /note="Description of Artificial Sequence: Synthetic
      polypeptide"

<400> 1902
Asp Ile Gln Leu Thr Gln Ser Pro Ala Ile Met Ser Ala Ser Pro Gly
1               5                   10                  15

Glu Lys Val Thr Met Thr Cys Arg Ala Ser Ser Ser Val Ser Tyr Met
            20                  25                  30

Asn Trp Tyr Gln Gln Lys Ser Gly Thr Ser Pro Lys Arg Trp Ile Tyr
        35                  40                  45

Asp Thr Ser Lys Val Ala Ser Gly Val Pro Tyr Arg Phe Ser Gly Ser
    50                  55                  60

Gly Ser Gly Thr Ser Tyr Ser Leu Ile Ser Ser Met Glu Ala Glu Asp
65                  70                  75                  80

Ala Ala Thr Tyr Tyr Cys Gln Gln Trp Ser Ser Asn Pro Leu Thr Phe
                85                  90                  95

Gly Ala Gly Thr Lys Leu Glu Leu Lys
            100                 105

<210> 1903

<211> 119
<212> PRT
<213> Artificial Sequence

<220>
<221> source
<223> /note="Description of Artificial Sequence: Synthetic
      polypeptide"

<400> 1903
Asp Ile Lys Leu Gln Gln Ser Gly Ala Glu Leu Ala Arg Pro Gly Ala
1               5                   10                  15

Ser Val Lys Met Ser Cys Lys Thr Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser
        115


<210> 1904
<211> 109
<212> PRT
<213> Artificial Sequence

<220>
<221> source
<223> /note="Description of Artificial Sequence: Synthetic
      polypeptide"

<400> 1904
Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Lys Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ala Leu Trp Tyr Ser Asn
                85                  90                  95

Leu Trp Val Phe Gly Gly Gly Thr Lys Leu Thr Val Leu
            100                 105

<210> 1905
<211> 125
<212> PRT
<213> Artificial Sequence

<220>
<221> source
<223> /note="Description of Artificial Sequence: Synthetic
      polypeptide"

<400> 1905
Glu Val Gln Leu Val Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Gln Ser Ile
65                  70                  75                  80

Leu Tyr Leu Gln Met Asn Asn Leu Lys Thr Glu Asp Thr Ala Met Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ala
        115                 120                 125

<210> 1906
<211> 107

<212> PRT
<213> Artificial Sequence

<220>
<221> source
<223> /note="Description of Artificial Sequence: Synthetic
      polypeptide"

<400> 1906
Gln Ala Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Trp Thr Pro Ala Arg Phe
    50                  55                  60

Ser Gly Ser Leu Leu Gly Gly Lys Ala Ala Leu Ile Gly Ala Gln Ala
65                  70                  75                  80

Glu Asp Glu Ala Asp Tyr Tyr Cys Ala Leu Trp Tyr Ser Asn Leu Trp
                85                  90                  95

Val Phe Gly Gly Gly Thr Lys Leu Thr Val Leu
            100                 105


<210> 1907
<211> 124
<212> PRT
<213> Artificial Sequence

<220>
<221> source
<223> /note="Description of Artificial Sequence: Synthetic
      polypeptide"

<400> 1907
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Gly Arg Ile Arg Ser Lys Tyr Asn Asn Tyr Ala Thr Tyr Tyr Ala Asp
    50                  55                  60

Ser Val Lys Asp Arg Phe Ile Ser Arg Asp Asp Ser Lys Asn Ser Leu
65                  70                  75                  80

Tyr Leu Gln Met Asn Ser Leu Lys Thr Glu Asp Thr Ala Val Tyr Tyr
                85                  90                  95

Cys Val Arg His Gly Asn Phe Gly Asn Ser Tyr Val Ser Trp Phe Ala
            100                 105                 110

Tyr Trp Gly Gln Gly Thr Leu Val Thr Val Ser Ser
        115                 120

<210> 1908
<211> 107
<212> PRT
<213> Artificial Sequence

<220>
<221> source
<223> /note="Description of Artificial Sequence: Synthetic
      polypeptide"

<400> 1908
Gln Ala Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Trp Thr Pro Ala Arg Phe
    50                  55                  60

Ser Gly Ser Leu Leu Gly Gly Lys Ala Ala Leu Ile Gly Ala Gln Ala
65                  70                  75                  80

Glu Asp Glu Ala Asp Tyr Tyr Cys Ala Leu Trp Tyr Ser Asn Leu Trp
                85                  90                  95

Val Phe Gly Gly Gly Thr Lys Leu Thr Val Leu
            100                 105

<210> 1909
<211> 124
<212> PRT
<213> Artificial Sequence

<220>
<221> source

<223> /note="Description of Artificial Sequence: Synthetic
      polypeptide"

<400> 1909
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Ile Ser Arg Asp Asp Ser Lys Asn Ser Leu
65                  70                  75                  80

Tyr Leu Gln Met Asn Ser Leu Lys Thr Glu Asp Thr Ala Val Tyr Tyr
                85                  90                  95

Cys Val Arg His Gly Asn Phe Gly Asn Ser Tyr Val Ser Trp Phe Ala
            100                 105                 110

Tyr Trp Gly Gln Gly Thr Leu Val Thr Val Ser Ser
        115                 120


<210> 1910
<211> 107
<212> PRT
<213> Artificial Sequence

<220>
<221> source
<223> /note="Description of Artificial Sequence: Synthetic
      polypeptide"

<400> 1910
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Pro
                85                  90                  95

Thr Phe Gly Gln Gly Thr Lys Val Glu Ile Lys
            100                 105

<210> 1911
<211> 118
<212> PRT
<213> Artificial Sequence

<220>
<221> source
<223> /note="Description of Artificial Sequence: Synthetic
      polypeptide"

<400> 1911
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Arg Ser Tyr
            20                  25                  30

Gly Met His Trp Val Arg Gln Ala Pro Gly Lys Gly Leu Glu Trp Val
        35                  40                  45

Ala Ile Ile Trp Tyr Ser Gly Ser Lys Lys Asn Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Thr Gly Tyr Asn Trp Phe Asp Pro Trp Gly Gln Gly Thr
            100                 105                 110

Leu Val Thr Val Ser Ser
        115